# EUROPEAN PATENT APPLICATION

(11) **EP 1 908 839 A2**
(43) Date of publication of application: **09.04.2008**
(21) Application number: 07114502.3
(22) Date of filing: 08.05.2001
(51) Int. Cl.: C12N 15/31, C07K 14/22, A61K 38/00, A61K 39/095, C12N 1/36, C12N 1/21, C07K 16/12, C12Q 1/02

(54) **Virulence genes, proteins, and their use**

(30) Priority: 08.05.2000 GB 0011108
(62) Divisional of application: 01925742.7
(71) Applicant: Microscience Limited, Wokingham, Berkshire RG41 5TU (GB)
(72) Inventor: Tang, Christoph Centre for Molecular Microbiology & Infection, London SW7 2AZ (GB)
(74) Representative: Jappy, John William Graham

(57) **Abstract**

A series of genes from *Neisseria meningitidis* are shown to encode products which are implicated in virulence. The identification of these genes therefore allows attenuated microorganisms to be produced. Furthermore, the genes or their encoded products can be used in the manufacture of vaccines for therapeutic application.

SEQUENCE LISTING

<110> Microscience Limited

<120> Virulence Gene and Protein, and Their Use

<130> REP06436WO

<140> not yet known
<141> 2001-05-08

<150> 0011108.8
<151> 2000-05-08

<160> 214

<170> PatentIn Ver. 2.1

<210> 1
<211> 1674
<212> DNA
<213> Neisseria meningitidis

<220>
<221> CDS
<222> (1)..(1674)

<400> 1
   atg ctg acc tat acc ccg ccc gat gcc cgc ccg ccc gcc aaa acc cac    48
   Met Leu Thr Tyr Thr Pro Pro Asp Ala Arg Pro Pro Ala Lys Thr His
     1               5                  10                  15
   gaa aag ccg tgg ctg ttg ctg ttg atg gcg ttt gcc tgg ttg tgg ccc    96
   Glu Lys Pro Trp Leu Leu Leu Leu Met Ala Phe Ala Trp Leu Trp Pro
                20                  25                  30
   ggc gtg ttt tcc cac gat ttg tgg aat cct gac gaa cct gcc gtc tat    144
   Gly Val Phe Ser His Asp Leu Trp Asn Pro Asp Glu Pro Ala Val Tyr
            35                  40                  45
   acc gcc gtc gaa gca ctg gca ggc agc ccc acc cct ttg gtt gcc cat    192
   Thr Ala Val Glu Ala Leu Ala Gly Ser Pro Thr Pro Leu Val Ala His
        50                  55                  60
   ctg ttc ggt caa atc gat ttc ggc ata ccg ccc gtg tat ctt tgg gtt    240
   Leu Phe Gly Gln Ile Asp Phe Gly Ile Pro Pro Val Tyr Leu Trp Val
    65                  70                  75                  80
   gcc gcc gcg ttc aaa cat ttg ctg tcg ccg tgg gct gcc gac ccg tat    288
   Ala Ala Ala Phe Lys His Leu Leu Ser Pro Trp Ala Ala Asp Pro Tyr
                   85                  90                  95
   gat gcc gca cgc ttt gcc ggc gtg ttt ttc gcc gtt gtc gga ctg act    336
   Asp Ala Ala Arg Phe Ala Gly Val Phe Phe Ala Val Val Gly Leu Thr
               100                 105                 110
   tcc tgc ggc ttt gcc ggt ttc aac ttt ttg ggc aga cac cac ggg cgc    384
   Ser Cys Gly Phe Ala Gly Phe Asn Phe Leu Gly Arg His His Gly Arg
           115                 120                 125
   agc gtc gtc ctg att ctc atc ggc tgt atc ggg ctg att ccg acc gta    432
   Ser Val Val Leu Ile Leu Ile Gly Cys Ile Gly Leu Ile Pro Thr Val
       130                 135                 140
   cac ttt ctc aac ccc gct gcc gcc gcc ttt gcc gcc gcc gga ctg gtg 480
   His Phe Leu Asn Pro Ala Ala Ala Ala Phe Ala Ala Ala Gly Leu Val
   145 150 155 160
   ctg cac ggt tat tct ttg gct cgc cgg cgc gtg att gcc gcc tct ttt    528
   Leu His Gly Tyr Ser Leu Ala Arg Arg Arg Val Ile Ala Ala Ser Phe
                   165                 170                 175
   ctg ctc ggt acg ggt tgg acg ctg atg tcg ttg gca gca gct tat ccg    576
   Leu Leu Gly Thr Gly Trp Thr Leu Met Ser Leu Ala Ala Ala Tyr Pro
               180                 185                 190
   gcg gca ttt gcc ctg atg ctg ccc ctg ccc gtg ctg atg ttt ttc cgt    624
   Ala Ala Phe Ala Leu Met Leu Pro Leu Pro Val Leu Met Phe Phe Arg
           195                 200                 205
   ccg tgg caa agc agg cgt ttg atg ttg acg gca gtc gcc tcg ctt gcc    672
   Pro Trp Gln Ser Arg Arg Leu Met Leu Thr Ala Val Ala Ser Leu Ala
       210                 215                 220
   ttt gcc ctg ccg ctt atg acc gtt tac ccg ctg ctc ttg gca aaa acg    720
   Phe Ala Leu Pro Leu Met Thr Val Tyr Pro Leu Leu Leu Ala Lys Thr
   225                 230                 235                 240
   cag ccc gcg ctg ttc gcg caa tgg ctc gac gat cac gtt ttc ggt acg    768
   Gln Pro Ala Leu Phe Ala Gln Trp Leu Asp Asp His Val Phe Gly Thr
                   245                 250                 255
   ttc ggc ggc gtg cgg cac att cag acg gca ttc agt ttg ttt tac tat    816
   Phe Gly Gly Val Arg His Ile Gln Thr Ala Phe Ser Leu Phe Tyr Tyr
               260                 265                 270
   ctg aaa aac ctg ctt tgg ttt gca ttg cct gcg ctg ccg ctg gcg gtt 864
   Leu Lys Asn Leu Leu Trp Phe Ala Leu Pro Ala Leu Pro Leu Ala Val
   275 280 285
   tgg acg gtt tgc cgc acg cgc ctg ttt tcg acc gac tgg ggg att ttg 912
   Trp Thr Val Cys Arg Thr Arg Leu Phe Ser Thr Asp Trp Gly Ile Leu
   290 295 300
   ggc gtc gtc tgg atg ctt gcc gtt ttg gtg ctg ctt gcc gtc aat ccg 960
   Gly Val Val Trp Met Leu Ala Val Leu Val Leu Leu Ala Val Asn Pro
   305 310 315 320
   cag cgt ttt cag gat aac ctc gtc tgg ctg ctt ccg ccg ctt gcc ctg 1008
   Gln Arg Phe Gln Asp Asn Leu Val Trp Leu Leu Pro Pro Leu Ala Leu
   325 330 335
   ttc ggc gcg gcg caa ctg gac agc ctg aga cgc ggc gcg gcg gcg ttt 1056
   Phe Gly Ala Ala Gln Leu Asp Ser Leu Arg Arg Gly Ala Ala Ala Phe
   340 345 350
   gtc aac tgg ttc ggc att atg gcg ttc gga ctg ttt gcc gtg ttc ctg 1104
   Val Asn Trp Phe Gly Ile Met Ala Phe Gly Leu Phe Ala Val Phe Leu
   355 360 365
   tgg acg ggc ttt ttc gcc atg aat tac ggc tgg ccc gcc aag ctt gcc 1152
   Trp Thr Gly Phe Phe Ala Met Asn Tyr Gly Trp Pro Ala Lys Leu Ala
   370 375 380
   gaa cgc gcc gcc tat ttc agc ccg tat tat gtt cct gat atc gat ccc 1200
   Glu Arg Ala Ala Tyr Phe Ser Pro Tyr Tyr Val Pro Asp Ile Asp Pro
   385 390 395 400
   att ccg atg gcg gtt gcc gta ctg ttc aca ccc ttg tgg ctg tgg gcg 1248
   Ile Pro Met Ala Val Ala Val Leu Phe Thr Pro Leu Trp Leu Trp Ala
   405 410 415
   att acc cgc aaa aac ata cgc ggc agg cag gcg gtt acc aac tgg gcg 1296
   Ile Thr Arg Lys Asn Ile Arg Gly Arg Gln Ala Val Thr Asn Trp Ala
   420 425 430
   gca ggc gtt acc ctg acc tgg gct ttg ctg atg acg ctg ttc ctg ccg 1344
   Ala Gly Val Thr Leu Thr Trp Ala Leu Leu Met Thr Leu Phe Leu Pro
   435 440 445
   tgg ctg gac gcg gcg aaa agc cac gcg ccc gtc gtc cgg agt atg gag 1392
   Trp Leu Asp Ala Ala Lys Ser His Ala Pro Val Val Arg Ser Met Glu
   450 455 460
   gca tcg ctt tcc ccg gaa tta aaa cgg gag ctt tca gac ggc atc gag     1440
   Ala Ser Leu Ser Pro Glu Leu Lys Arg Glu Leu Ser Asp Gly Ile Glu
   465                 470                 475                 480
   tgt atc gac ata ggc ggc ggc gac cta cac acg cgg att gtt tgg acg     1488
   Cys Ile Asp Ile Gly Gly Gly Asp Leu His Thr Arg Ile Val Trp Thr
                   485                 490                 495
   cag tac ggc aca ttg ccg cac cgc gtc ggc gat gta caa tgc cgc tac     1536
   Gln Tyr Gly Thr Leu Pro His Arg Val Gly Asp Val Gln Cys Arg Tyr
               500                 505                 510
   cgc atc gtc cgc ttg ccc caa aac gcg gat gcg ccg caa ggc tgg cag     1584
   Arg Ile Val Arg Leu Pro Gln Asn Ala Asp Ala Pro Gln Gly Trp Gln
           515                 520                 525
   acg gtc tgg cag ggt gcg cgc ccg cgc aac aaa gac agt aag ttc gca     1632
   Thr Val Trp Gln Gly Ala Arg Pro Arg Asn Lys Asp Ser Lys Phe Ala
       530                 535                 540
   ctg ata cgg aaa acc ggg gaa aat ata tta aaa aca aca gat             1674
   Leu Ile Arg Lys Thr Gly Glu Asn Ile Leu Lys Thr Thr Asp
   545                 550                 555

<210> 2
<211> 558
<212> PRT
<213> Neisseria meningitidis

<400> 2
Met Leu Thr Tyr Thr Pro Pro Asp Ala Arg Pro Pro Ala Lys Thr His
  1               5                  10                  15

Glu Lys Pro Trp Leu Leu Leu Leu Met Ala Phe Ala Trp Leu Trp Pro
             20                  25                  30

Gly Val Phe Ser His Asp Leu Trp Asn Pro Asp Glu Pro Ala Val Tyr
         35                  40                  45

Thr Ala Val Glu Ala Leu Ala Gly Ser Pro Thr Pro Leu Val Ala His
     50                  55                  60

Leu Phe Gly Gln Ile Asp Phe Gly Ile Pro Pro Val Tyr Leu Trp Val
 65                  70                  75                  80

Ala Ala Ala Phe Lys His Leu Leu Ser Pro Trp Ala Ala Asp Pro Tyr
                 85                  90                  95

Asp Ala Ala Arg Phe Ala Gly Val Phe Phe Ala Val Val Gly Leu Thr
            100                 105                 110

Ser Cys Gly Phe Ala Gly Phe Asn Phe Leu Gly Arg His His Gly Arg
        115                 120                 125

Ser Val Val Leu Ile Leu Ile Gly Cys Ile Gly Leu Ile Pro Thr Val
    130                 135                 140

His Phe Leu Asn Pro Ala Ala Ala Ala Phe Ala Ala Ala Gly Leu Val
145                 150                 155                 160

Leu His Gly Tyr Ser Leu Ala Arg Arg Arg Val Ile Ala Ala Ser Phe
                165                 170                 175

Leu Leu Gly Thr Gly Trp Thr Leu Met Ser Leu Ala Ala Ala Tyr Pro
            180                 185                 190

Ala Ala Phe Ala Leu Met Leu Pro Leu Pro Val Leu Met Phe Phe Arg
        195                 200                 205

Pro Trp Gln Ser Arg Arg Leu Met Leu Thr Ala Val Ala Ser Leu Ala
    210                 215                 220

Phe Ala Leu Pro Leu Met Thr Val Tyr Pro Leu Leu Leu Ala Lys Thr
225                 230                 235                 240

Gln Pro Ala Leu Phe Ala Gln Trp Leu Asp Asp His Val Phe Gly Thr
                245                 250                 255

Phe Gly Gly Val Arg His Ile Gln Thr Ala Phe Ser Leu Phe Tyr Tyr
            260                 265                 270

Leu Lys Asn Leu Leu Trp Phe Ala Leu Pro Ala Leu Pro Leu Ala Val
        275                 280                 285

Trp Thr Val Cys Arg Thr Arg Leu Phe Ser Thr Asp Trp Gly Ile Leu
    290                 295                 300

Gly Val Val Trp Met Leu Ala Val Leu Val Leu Leu Ala Val Asn Pro
305                 310                 315                 320

Gln Arg Phe Gln Asp Asn Leu Val Trp Leu Leu Pro Pro Leu Ala Leu
                325                 330                 335

Phe Gly Ala Ala Gln Leu Asp Ser Leu Arg Arg Gly Ala Ala Ala Phe
            340                 345                 350

Val Asn Trp Phe Gly Ile Met Ala Phe Gly Leu Phe Ala Val Phe Leu
        355                 360                 365

Trp Thr Gly Phe Phe Ala Met Asn Tyr Gly Trp Pro Ala Lys Leu Ala
    370                 375                 380

Glu Arg Ala Ala Tyr Phe Ser Pro Tyr Tyr Val Pro Asp Ile Asp Pro
385                 390                 395                 400

Ile Pro Met Ala Val Ala Val Leu Phe Thr Pro Leu Trp Leu Trp Ala
                405                 410                 415

Ile Thr Arg Lys Asn Ile Arg Gly Arg Gln Ala Val Thr Asn Trp Ala
            420                 425                 430

Ala Gly Val Thr Leu Thr Trp Ala Leu Leu Met Thr Leu Phe Leu Pro
        435                 440                 445

Trp Leu Asp Ala Ala Lys Ser His Ala Pro Val Val Arg Ser Met Glu
    450                 455                 460

Ala Ser Leu Ser Pro Glu Leu Lys Arg Glu Leu Ser Asp Gly Ile Glu
465                 470                 475                 480

Cys Ile Asp Ile Gly Gly Gly Asp Leu His Thr Arg Ile Val Trp Thr
                485                 490                 495

Gln Tyr Gly Thr Leu Pro His Arg Val Gly Asp Val Gln Cys Arg Tyr
            500                 505                 510

Arg Ile Val Arg Leu Pro Gln Asn Ala Asp Ala Pro Gln Gly Trp Gln
        515                 520                 525

Thr Val Trp Gln Gly Ala Arg Pro Arg Asn Lys Asp Ser Lys Phe Ala
    530                 535                 540

Leu Ile Arg Lys Thr Gly Glu Asn Ile Leu Lys Thr Thr Asp
545                 550                 555

<210> 3
<211> 1077
<212> DNA
<213> Neisseria meningitidis

<220>

<221> CDS
<222> (1)..(1077)

<400> 3
atg aaa aca ctg acc gta cac acc ccg tcc cac agc tac ccc atc ttt 48
Met Lys Thr Leu Thr Val His Thr Pro Ser His Ser Tyr Pro Ile Phe
1               5                   10                  15

atc ggc aac gga ctg ctg ccg cag gca gga agc ctg ctc aaa ccg cat 96
Ile Gly Asn Gly Leu Leu Pro Gln Ala Gly Ser Leu Leu Lys Pro His
            20                  25                  30

ttg ggc aaa cgc gcc gcc atc atc acc aac gaa acc gtc gcc ccg ctc 144
Leu Gly Lys Arg Ala Ala Ile Ile Thr Asn Glu Thr Val Ala Pro Leu
        35                  40                  45

tac ctc ggc acg ctt cag acg gca ttg gat gcg gca ggc gta tcc cat 192
Tyr Leu Gly Thr Leu Gln Thr Ala Leu Asp Ala Ala Gly Val Ser His
    50                  55                  60

ttc agc atc atc ctg ccc gac ggc gag gcg cac aaa aac tgg cag acg 240
Phe Ser Ile Ile Leu Pro Asp Gly Glu Ala His Lys Asn Trp Gln Thr
65                  70                  75                  80

ctc aac ctc atc ttt gac ggg ctg atg caa aac cgc gcc gaa cgc aaa 288
Leu Asn Leu Ile Phe Asp Gly Leu Met Gln Asn Arg Ala Glu Arg Lys
                85                  90                  95

acc acg ctc atc gca ctg ggc ggc ggc gtg atc ggc gat atg acg ggt 336
Thr Thr Leu Ile Ala Leu Gly Gly Gly Val Ile Gly Asp Met Thr Gly
            100                 105                 110

ttt gcg gcg gct act tat caa cgc ggc gcg cca ttt gtt caa att ccc 384
Phe Ala Ala Ala Thr Tyr Gln Arg Gly Ala Pro Phe Val Gln Ile Pro
        115                 120                 125

act act tta tta agt cag gtc gat tcc tcc gtc ggc ggc aaa acc gcc 432
Thr Thr Leu Leu Ser Gln Val Asp Ser Ser Val Gly Gly Lys Thr Ala
    130                 135                 140

atc aac cac ccg ctc ggc aaa aat atg atc ggc gcg ttc tac cag ccg 480
Ile Asn His Pro Leu Gly Lys Asn Met Ile Gly Ala Phe Tyr Gln Pro
145                 150                 155                 160

cag gcg gtg ctt gcc gac ttg gac acg ctg cac acc ctg ccc gcc cgc 528
Gln Ala Val Leu Ala Asp Leu Asp Thr Leu His Thr Leu Pro Ala Arg
                165                 170                 175

gaa ctg tcc gcc ggt atg gcg gaa gtc atc aaa tac ggc acg ctc ggc 576
Glu Leu Ser Ala Gly Met Ala Glu Val Ile Lys Tyr Gly Thr Leu Gly
            180                 185                 190

gac att agt ttt ttt gaa tgg ctg gaa cag cat atg ccc gaa ctg atg 624
Asp Ile Ser Phe Phe Glu Trp Leu Glu Gln His Met Pro Glu Leu Met
        195                 200                 205

gca ctc gaa cgc gcg ccc ctc atc caa gcc gtg tac cgc tgt tgc caa 672
Ala Leu Glu Arg Ala Pro Leu Ile Gln Ala Val Tyr Arg Cys Cys Gln
    210                 215                 220

atg aag gca gac atc gtc gcc caa gac gaa acc gaa cag ggc ata cgc 720
Met Lys Ala Asp Ile Val Ala Gln Asp Glu Thr Glu Gln Gly Ile Arg
225                 230                 235                 240

gca tgg ctc aac ctc gga cac acc ttc gga cac gcc att gaa acc gag 768
Ala Trp Leu Asn Leu Gly His Thr Phe Gly His Ala Ile Glu Thr Glu
                245                 250                 255

atg ggt tac ggt act tgg ctg cac ggc gag gcc gtc gcc gcc ggc tgc 816
Met Gly Tyr Gly Thr Trp Leu His Gly Glu Ala Val Ala Ala Gly Cys
            260                 265                 270

gtg ttg gcg gcg cgt ttg tcc gaa caa ctg ggc aaa atc tcc gcc gca 864
Val Leu Ala Ala Arg Leu Ser Glu Gln Leu Gly Lys Ile Ser Ala Ala
        275                 280                 285

gat acc gcg cgg ctc gcc gcc ctg ctc gaa gcc gcc gga ctg ccg tcc 912
Asp Thr Ala Arg Leu Ala Ala Leu Leu Glu Ala Ala Gly Leu Pro Ser
    290                 295                 300

gcg cca ccc gtg ttt gcc ttt gaa aaa tgg ctg gaa cac atg agc cac 960
Ala Pro Pro Val Phe Ala Phe Glu Lys Trp Leu Glu His Met Ser His
305                 310                 315                 320

gat aaa aaa gtc agc ggc ggc atc atg cgc ttt atc ggt ctg aac cgg 1008
Asp Lys Lys Val Ser Gly Gly Ile Met Arg Phe Ile Gly Leu Asn Arg
                325                 330                 335

ctg ggc gaa gcc aac atc acc gaa att acc gac acg gac atc ctc cgc 1056
Leu Gly Glu Ala Asn Ile Thr Glu Ile Thr Asp Thr Asp Ile Leu Arg
            340                 345                 350

cgc acc ctg caa ccg tat ctc 1077
Arg Thr Leu Gln Pro Tyr Leu
        355

<210> 4
<211> 359
<212> PRT
<213> Neisseria meningitidis

<400> 4
Met Lys Thr Leu Thr Val His Thr Pro Ser His Ser Tyr Pro Ile Phe
1               5                   10                  15

Ile Gly Asn Gly Leu Leu Pro Gln Ala Gly Ser Leu Leu Lys Pro His
            20                  25                  30

Leu Gly Lys Arg Ala Ala Ile Ile Thr Asn Glu Thr Val Ala Pro Leu
        35                  40                  45

Tyr Leu Gly Thr Leu Gln Thr Ala Leu Asp Ala Ala Gly Val Ser His
    50                  55                  60

Phe Ser Ile Ile Leu Pro Asp Gly Glu Ala His Lys Asn Trp Gln Thr
65                  70                  75                  80

Leu Asn Leu Ile Phe Asp Gly Leu Met Gln Asn Arg Ala Glu Arg Lys
                85                  90                  95

Thr Thr Leu Ile Ala Leu Gly Gly Gly Val Ile Gly Asp Met Thr Gly
            100                 105                 110

Phe Ala Ala Ala Thr Tyr Gln Arg Gly Ala Pro Phe Val Gln Ile Pro
        115                 120                 125

Thr Thr Leu Leu Ser Gln Val Asp Ser Ser Val Gly Gly Lys Thr Ala
    130                 135                 140

Ile Asn His Pro Leu Gly Lys Asn Met Ile Gly Ala Phe Tyr Gln Pro
145                 150                 155                 160

Gln Ala Val Leu Ala Asp Leu Asp Thr Leu His Thr Leu Pro Ala Arg
                165                 170                 175

Glu Leu Ser Ala Gly Met Ala Glu Val Ile Lys Tyr Gly Thr Leu Gly
            180                 185                 190

Asp Ile Ser Phe Phe Glu Trp Leu Glu Gln His Met Pro Glu Leu Met
        195                 200                 205

Ala Leu Glu Arg Ala Pro Leu Ile Gln Ala Val Tyr Arg Cys Cys Gln
    210                 215                 220

Met Lys Ala Asp Ile Val Ala Gln Asp Glu Thr Glu Gln Gly Ile Arg
225                 230                 235                 240

Ala Trp Leu Asn Leu Gly His Thr Phe Gly His Ala Ile Glu Thr Glu
                245                 250                 255

Met Gly Tyr Gly Thr Trp Leu His Gly Glu Ala Val Ala Ala Gly Cys
            260                 265                 270

Val Leu Ala Ala Arg Leu Ser Glu Gln Leu Gly Lys Ile Ser Ala Ala
        275                 280                 285

Asp Thr Ala Arg Leu Ala Ala Leu Leu Glu Ala Ala Gly Leu Pro Ser
    290                 295                 300

Ala Pro Pro Val Phe Ala Phe Glu Lys Trp Leu Glu His Met Ser His
305                 310                 315                 320

Asp Lys Lys Val Ser Gly Gly Ile Met Arg Phe Ile Gly Leu Asn Arg
                325                 330                 335

Leu Gly Glu Ala Asn Ile Thr Glu Ile Thr Asp Thr Asp Ile Leu Arg
            340                 345                 350

Arg Thr Leu Gln Pro Tyr Leu
        355

<210> 5
<211> 1053
<212> DNA
<213> Neisseria meningitidis

<220>
<221> CDS
<222> (1)..(1053)

<400> 5
atg aca cac cat tac ccc aca gac gat att aag att aaa gaa gtt aaa     48
Met Thr His His Tyr Pro Thr Asp Asp Ile Lys Ile Lys Glu Val Lys
  1               5                  10                  15

gag ttg ttg ccg ccg att gcc cat ctt tac gag ctg ccg att tcc aaa     96
Glu Leu Leu Pro Pro Ile Ala His Leu Tyr Glu Leu Pro Ile Ser Lys
             20                  25                  30

gag gct tcg ggc ttg gtt cac cgc acc cgt cag gaa att tcc gat ttg 144
Glu Ala Ser Gly Leu Val His Arg Thr Arg Gln Glu Ile Ser Asp Leu
        35                  40                  45

gtt cac ggc agg gac aag cgg ctg ttg gtt att atc ggg ccg tgt tcg 192
Val His Gly Arg Asp Lys Arg Leu Leu Val Ile Ile Gly Pro Cys Ser
    50                  55                  60

att cac gat ccg aaa gcg gcg ttg gaa tat gcg gag cgt ttg ttg aaa 240
Ile His Asp Pro Lys Ala Ala Leu Glu Tyr Ala Glu Arg Leu Leu Lys
65                  70                  75                  80

ctc cgc aag cag tat gaa aac gag ctt ttg att gtg atg cgc gtt tat 288
Leu Arg Lys Gln Tyr Glu Asn Glu Leu Leu Ile Val Met Arg Val Tyr
                85                  90                  95

ttc gag aag ccg agg acg acg gtg ggt tgg aaa ggt ttg att aac gac 336
Phe Glu Lys Pro Arg Thr Thr Val Gly Trp Lys Gly Leu Ile Asn Asp
            100                 105                 110

ccg cat ttg gac ggt acg ttt gac atc aat ttc ggt ttg cgt cag gca 384
Pro His Leu Asp Gly Thr Phe Asp Ile Asn Phe Gly Leu Arg Gln Ala
        115                 120                 125

cgc agc ctg ttg ttg tcg ctg aac aat atg ggt atg cct gcc tct acc 432
Arg Ser Leu Leu Leu Ser Leu Asn Asn Met Gly Met Pro Ala Ser Thr
    130                 135                 140

gag ttt ttg gat atg att acg ccg caa tat tat gcg gac ttg att tct 480
Glu Phe Leu Asp Met Ile Thr Pro Gln Tyr Tyr Ala Asp Leu Ile Ser
145                 150                 155                 160

tgg ggg gca atc ggc gcg cgg acg acc gaa agc caa gtt cac cgc gaa 528
Trp Gly Ala Ile Gly Ala Arg Thr Thr Glu Ser Gln Val His Arg Glu
                165                 170                 175

ttg gca agc ggg ctg tcc tgc ccc gtc ggc ttt aaa aac ggt acg gat 576
Leu Ala Ser Gly Leu Ser Cys Pro Val Gly Phe Lys Asn Gly Thr Asp
            180                 185                 190

ggc aat ttg aag att gcc atc gac gca atc ggt gcg gcg agc cat tcg 624
Gly Asn Leu Lys Ile Ala Ile Asp Ala Ile Gly Ala Ala Ser His Ser
        195                 200                 205

cat cat ttc ctg tct gta acc aag gcc ggg cat tcc gcc att gtc cat 672
His His Phe Leu Ser Val Thr Lys Ala Gly His Ser Ala Ile Val His
    210                 215                 220

acc ggc ggc aat cct gac tgt cat gtc att ttg cgc ggc gga aaa gag    720
Thr Gly Gly Asn Pro Asp Cys His Val Ile Leu Arg Gly Gly Lys Glu
225                 230                 235                 240

ccg aat tat gat gcg gga cac gtc agc gag gcg gcg gaa caa ctg cgt    768
Pro Asn Tyr Asp Ala Gly His Val Ser Glu Ala Ala Glu Gln Leu Arg
                245                 250                 255

gcg gca ggc gta acc gac aag ctg atg att gat tgc agc cac gcc aac    816
Ala Ala Gly Val Thr Asp Lys Leu Met Ile Asp Cys Ser His Ala Asn
            260                 265                 270

agc cgc aag gat tac acc cga cag atg gaa gtg gcg caa gac att gcc    864
Ser Arg Lys Asp Tyr Thr Arg Gln Met Glu Val Ala Gln Asp Ile Ala
        275                 280                 285

gcc caa ttg gaa cag gac ggc ggc aat att atg ggc gtg atg gtg gaa    912
Ala Gln Leu Glu Gln Asp Gly Gly Asn Ile Met Gly Val Met Val Glu
    290                 295                 300

agc cat ttg gtc gaa ggc agg cag gac aag ccg gaa gtg tac ggc aaa    960
Ser His Leu Val Glu Gly Arg Gln Asp Lys Pro Glu Val Tyr Gly Lys
305                 310                 315                 320

agc att acc gat gcg tgt att ggt tgg gac gcg act gaa gaa ctg ttg    1008
Ser Ile Thr Asp Ala Cys Ile Gly Trp Asp Ala Thr Glu Glu Leu Leu
                325                 330                 335

gca ttg ttg gca ggt gca aac aaa aaa cgt atg gcg cgc gcc ggt        1053
Ala Leu Leu Ala Gly Ala Asn Lys Lys Arg Met Ala Arg Ala Gly
            340                 345                 350

<210> 6
<211> 351
<212> PRT
<213> Neisseria meningitidis

<400> 6
Met Thr His His Tyr Pro Thr Asp Asp Ile Lys Ile Lys Glu Val Lys
  1               5                  10                  15

Glu Leu Leu Pro Pro Ile Ala His Leu Tyr Glu Leu Pro Ile Ser Lys
             20                  25                  30

Glu Ala Ser Gly Leu Val His Arg Thr Arg Gln Glu Ile Ser Asp Leu
         35                  40                  45

Val His Gly Arg Asp Lys Arg Leu Leu Val Ile Ile Gly Pro Cys Ser
    50                  55                  60

Ile His Asp Pro Lys Ala Ala Leu Glu Tyr Ala Glu Arg Leu Leu Lys
65                  70                  75                  80

Leu Arg Lys Gln Tyr Glu Asn Glu Leu Leu Ile Val Met Arg Val Tyr
                85                  90                  95

Phe Glu Lys Pro Arg Thr Thr Val Gly Trp Lys Gly Leu Ile Asn Asp
            100                 105                 110

Pro His Leu Asp Gly Thr Phe Asp Ile Asn Phe Gly Leu Arg Gln Ala
        115                 120                 125

Arg Ser Leu Leu Leu Ser Leu Asn Asn Met Gly Met Pro Ala Ser Thr
    130                 135                 140

Glu Phe Leu Asp Met Ile Thr Pro Gln Tyr Tyr Ala Asp Leu Ile Ser
145                 150                 155                 160

Trp Gly Ala Ile Gly Ala Arg Thr Thr Glu Ser Gln Val His Arg Glu
                165                 170                 175

Leu Ala Ser Gly Leu Ser Cys Pro Val Gly Phe Lys Asn Gly Thr Asp
            180                 185                 190

Gly Asn Leu Lys Ile Ala Ile Asp Ala Ile Gly Ala Ala Ser His Ser
        195                 200                 205

His His Phe Leu Ser Val Thr Lys Ala Gly His Ser Ala Ile Val His
    210                 215                 220

Thr Gly Gly Asn Pro Asp Cys His Val Ile Leu Arg Gly Gly Lys Glu
225                 230                 235                 240

Pro Asn Tyr Asp Ala Gly His Val Ser Glu Ala Ala Glu Gln Leu Arg
                245                 250                 255

Ala Ala Gly Val Thr Asp Lys Leu Met Ile Asp Cys Ser His Ala Asn
            260                 265                 270

Ser Arg Lys Asp Tyr Thr Arg Gln Met Glu Val Ala Gln Asp Ile Ala
        275                 280                 285

Ala Gln Leu Glu Gln Asp Gly Gly Asn Ile Met Gly Val Met Val Glu
    290                 295                 300

Ser His Leu Val Glu Gly Arg Gln Asp Lys Pro Glu Val Tyr Gly Lys
305                 310                 315                 320

Ser Ile Thr Asp Ala Cys Ile Gly Trp Asp Ala Thr Glu Glu Leu Leu
                325                 330                 335

Ala Leu Leu Ala Gly Ala Asn Lys Lys Arg Met Ala Arg Ala Gly
            340                 345                 350

<210> 7
<211> 1686
<212> DNA
<213> Neisseria meningitidis

<220>
<221> CDS
<222> (1)..(1686)

<400> 7
atg tta caa tcc gaa aat tcg aaa aat tta atc tct tgt tca ata aag    48
Met Leu Gln Ser Glu Asn Ser Lys Asn Leu Ile Ser Cys Ser Ile Lys
1               5                   10                  15

gct tta cca atc atg att tct acc aac ggc atc acc atg cag ttc ggc    96
Ala Leu Pro Ile Met Ile Ser Thr Asn Gly Ile Thr Met Gln Phe Gly
            20                  25                  30

gcg aag ccg ctg ttt gaa aac gta tcc gtt aaa ttc ggc gaa ggc aac    144
Ala Lys Pro Leu Phe Glu Asn Val Ser Val Lys Phe Gly Glu Gly Asn
        35                  40                  45

cgc tac ggt ttg atc ggc gcg aac ggc tca ggc aaa tcc acc ttc atg    192
Arg Tyr Gly Leu Ile Gly Ala Asn Gly Ser Gly Lys Ser Thr Phe Met
    50                  55                  60

aaa atc ctc ggc ggc gat ttg gaa cag aca gcc ggc gaa gtg gcg att    240
Lys Ile Leu Gly Gly Asp Leu Glu Gln Thr Ala Gly Glu Val Ala Ile
65                  70                  75                  80

gaa aac ggc gtg cgt ttg ggt aaa ttg cgc caa gac cag ttt gcc tac    288
Glu Asn Gly Val Arg Leu Gly Lys Leu Arg Gln Asp Gln Phe Ala Tyr
                85                  90                  95

gaa gat atg cgc gtg ctg gac gtg gta atg atg ggt cac acc gaa atg    336
Glu Asp Met Arg Val Leu Asp Val Val Met Met Gly His Thr Glu Met
            100                 105                 110

tgg gcg gcg atg acc gaa cgt gat gcg att tac gcc aat ccc gaa gcc 384
Trp Ala Ala Met Thr Glu Arg Asp Ala Ile Tyr Ala Asn Pro Glu Ala
        115                 120                 125

acc gaa gac gac tac atg aaa gcc gca gaa ctg gaa gcc aag ttc gcc 432
Thr Glu Asp Asp Tyr Met Lys Ala Ala Glu Leu Glu Ala Lys Phe Ala
    130                 135                 140

gaa tac gac ggc tac acc gcc gaa gcg cgt gcc gcc gag ttg ttg agc 480
Glu Tyr Asp Gly Tyr Thr Ala Glu Ala Arg Ala Ala Glu Leu Leu Ser
145                 150                 155                 160

ggc gtg ggc att tcc gaa gat ttg cac aat gca acc atg gca gaa gtt 528
Gly Val Gly Ile Ser Glu Asp Leu His Asn Ala Thr Met Ala Glu Val
                165                 170                 175

gcc ccg ggc ttc aaa ctg cgc gta ttg ctg gcg caa gcc ctg ttc tcc 576
Ala Pro Gly Phe Lys Leu Arg Val Leu Leu Ala Gln Ala Leu Phe Ser
            180                 185                 190

aag ccg gat gta ttg ctc ttg gac gaa ccg acc aat aac ttg gac att 624
Lys Pro Asp Val Leu Leu Leu Asp Glu Pro Thr Asn Asn Leu Asp Ile
        195                 200                 205

aat acc atc cgt tgg ttg gaa ggc gtg ttg aac cag tac gac tcc acg 672
Asn Thr Ile Arg Trp Leu Glu Gly Val Leu Asn Gln Tyr Asp Ser Thr
    210                 215                 220

atg att atc atc tcg cac gac cgc cac ttc ttg aac gaa gtc tgc acg 720
Met Ile Ile Ile Ser His Asp Arg His Phe Leu Asn Glu Val Cys Thr
225                 230                 235                 240

cat atg gca gat ttg gat tac aac acc atc acc atc tat ccg ggc aac 768
His Met Ala Asp Leu Asp Tyr Asn Thr Ile Thr Ile Tyr Pro Gly Asn
                245                 250                 255

tac gac gac tat atg ctc gcc tcc gcc caa tcg cgc gaa cgc gcc ctg 816
Tyr Asp Asp Tyr Met Leu Ala Ser Ala Gln Ser Arg Glu Arg Ala Leu
            260                 265                 270

aaa gac aat gcc aag gcg aag gaa aaa ttg cag gaa ttg caa gaa ttt 864
Lys Asp Asn Ala Lys Ala Lys Glu Lys Leu Gln Glu Leu Gln Glu Phe
        275                 280                 285

gtg gcg cgg ttc agt gcc aac aaa tcc aaa gcc cgt cag gca acc agc 912
Val Ala Arg Phe Ser Ala Asn Lys Ser Lys Ala Arg Gln Ala Thr Ser
    290                 295                 300

cgt ctg aaa cag gca gac aaa atc aaa tcg gag atg gtc gag gtc aaa 960
Arg Leu Lys Gln Ala Asp Lys Ile Lys Ser Glu Met Val Glu Val Lys
305                 310                 315                 320

ccc tct acc cgt caa aac ccg tat atc cgt ttt gaa gcc gat gaa aaa 1008
Pro Ser Thr Arg Gln Asn Pro Tyr Ile Arg Phe Glu Ala Asp Glu Lys
                325                 330                 335

gcc aag ctg cac cgt cag gct gtg gaa gtt gaa aaa ctg gcg aaa cgc 1056
Ala Lys Leu His Arg Gln Ala Val Glu Val Glu Lys Leu Ala Lys Arg
            340                 345                 350

ttt gaa acc cag ttg ttt aaa aac ctg aac ttt atc ctt gaa gcg gga 1104
Phe Glu Thr Gln Leu Phe Lys Asn Leu Asn Phe Ile Leu Glu Ala Gly
        355                 360                 365

caa cgc ctc gcc atc atc ggc ccg aac ggc gcg ggc aaa tcc acc ctg 1152
Gln Arg Leu Ala Ile Ile Gly Pro Asn Gly Ala Gly Lys Ser Thr Leu
    370                 375                 380

ctg aaa ctc ttg gcc ggc gcg tac aac ccc gaa tat tca gac ggt ctg 1200
Leu Lys Leu Leu Ala Gly Ala Tyr Asn Pro Glu Tyr Ser Asp Gly Leu
385                 390                 395                 400

ctg ccg gac gaa ggc agc atc aaa tgg gcg gaa aaa gcc agc gtc ggc 1248
Leu Pro Asp Glu Gly Ser Ile Lys Trp Ala Glu Lys Ala Ser Val Gly
                405                 410                 415

tac tat ccg caa gat cac gaa aac gac ttc gat gtc gat atg gat ttg 1296
Tyr Tyr Pro Gln Asp His Glu Asn Asp Phe Asp Val Asp Met Asp Leu
            420                 425                 430

agc gaa tgg atg cgc caa tgg ggg cag gac ggc gac gac gaa caa gtc 1344
Ser Glu Trp Met Arg Gln Trp Gly Gln Asp Gly Asp Asp Glu Gln Val
        435                 440                 445

atc cgc ggc act ttg ggg cgt ttg ctc ttc ggc agt aac gat gtc gtg 1392
Ile Arg Gly Thr Leu Gly Arg Leu Leu Phe Gly Ser Asn Asp Val Val
    450                 455                 460

aaa aaa gtg aag gtt ctc tcc ggt ggt gaa aaa ggc cgt atg ctt tac 1440
Lys Lys Val Lys Val Leu Ser Gly Gly Glu Lys Gly Arg Met Leu Tyr
465                 470                 475                 480

ggc aaa ctg ttg ctg ttg aaa ccc aat gtc tta gtc atg gat gaa ccg 1488
Gly Lys Leu Leu Leu Leu Lys Pro Asn Val Leu Val Met Asp Glu Pro
                485                 490                 495

acc aac cac atg gac atg gaa agc atc gaa tcc ttg aac atg gcg ctg    1536
Thr Asn His Met Asp Met Glu Ser Ile Glu Ser Leu Asn Met Ala Leu
            500                 505                 510

gaa aaa tac aac ggc acg ctg att ttc gtc tcc cac gac cgt cag ttc    1584
Glu Lys Tyr Asn Gly Thr Leu Ile Phe Val Ser His Asp Arg Gln Phe
        515                 520                 525

gtt tct tcc ctg gct acc caa atc att gaa ttg gac ggc aaa ggc gga    1632
Val Ser Ser Leu Ala Thr Gln Ile Ile Glu Leu Asp Gly Lys Gly Gly
    530                 535                 540

tat gaa cac tac ttg ggc gat tac gaa agc tat ctc gag aaa aaa ggc    1680
Tyr Glu His Tyr Leu Gly Asp Tyr Glu Ser Tyr Leu Glu Lys Lys Gly
545                 550                 555                 560

gta gca                                                            1686
Val Ala

<210> 8
<211> 562
<212> PRT
<213> Neisseria meningitidis

<400> 8
Met Leu Gln Ser Glu Asn Ser Lys Asn Leu Ile Ser Cys Ser Ile Lys
1               5                   10                  15

Ala Leu Pro Ile Met Ile Ser Thr Asn Gly Ile Thr Met Gln Phe Gly
            20                  25                  30

Ala Lys Pro Leu Phe Glu Asn Val Ser Val Lys Phe Gly Glu Gly Asn
        35                  40                  45

Arg Tyr Gly Leu Ile Gly Ala Asn Gly Ser Gly Lys Ser Thr Phe Met
    50                  55                  60

Lys Ile Leu Gly Gly Asp Leu Glu Gln Thr Ala Gly Glu Val Ala Ile
65                  70                  75                  80

Glu Asn Gly Val Arg Leu Gly Lys Leu Arg Gln Asp Gln Phe Ala Tyr
                85                  90                  95

Glu Asp Met Arg Val Leu Asp Val Val Met Met Gly His Thr Glu Met
            100                 105                 110

Trp Ala Ala Met Thr Glu Arg Asp Ala Ile Tyr Ala Asn Pro Glu Ala
        115                 120                 125

Thr Glu Asp Asp Tyr Met Lys Ala Ala Glu Leu Glu Ala Lys Phe Ala
    130                 135                 140

Glu Tyr Asp Gly Tyr Thr Ala Glu Ala Arg Ala Ala Glu Leu Leu Ser
145                 150                 155                 160

Gly Val Gly Ile Ser Glu Asp Leu His Asn Ala Thr Met Ala Glu Val
                165                 170                 175

Ala Pro Gly Phe Lys Leu Arg Val Leu Leu Ala Gln Ala Leu Phe Ser
            180                 185                 190

Lys Pro Asp Val Leu Leu Leu Asp Glu Pro Thr Asn Asn Leu Asp Ile
        195                 200                 205

Asn Thr Ile Arg Trp Leu Glu Gly Val Leu Asn Gln Tyr Asp Ser Thr
    210                 215                 220

Met Ile Ile Ile Ser His Asp Arg His Phe Leu Asn Glu Val Cys Thr
225                 230                 235                 240

His Met Ala Asp Leu Asp Tyr Asn Thr Ile Thr Ile Tyr Pro Gly Asn
                245                 250                 255

Tyr Asp Asp Tyr Met Leu Ala Ser Ala Gln Ser Arg Glu Arg Ala Leu
            260                 265                 270

Lys Asp Asn Ala Lys Ala Lys Glu Lys Leu Gln Glu Leu Gln Glu Phe
        275                 280                 285

Val Ala Arg Phe Ser Ala Asn Lys Ser Lys Ala Arg Gln Ala Thr Ser
    290                 295                 300

Arg Leu Lys Gln Ala Asp Lys Ile Lys Ser Glu Met Val Glu Val Lys
305                 310                 315                 320

Pro Ser Thr Arg Gln Asn Pro Tyr Ile Arg Phe Glu Ala Asp Glu Lys
                325                 330                 335

Ala Lys Leu His Arg Gln Ala Val Glu Val Glu Lys Leu Ala Lys Arg
            340                 345                 350

Phe Glu Thr Gln Leu Phe Lys Asn Leu Asn Phe Ile Leu Glu Ala Gly
        355                 360                 365

Gln Arg Leu Ala Ile Ile Gly Pro Asn Gly Ala Gly Lys Ser Thr Leu
    370                 375                 380

Leu Lys Leu Leu Ala Gly Ala Tyr Asn Pro Glu Tyr Ser Asp Gly Leu
385                 390                 395                 400

Leu Pro Asp Glu Gly Ser Ile Lys Trp Ala Glu Lys Ala Ser Val Gly
                405                 410                 415

Tyr Tyr Pro Gln Asp His Glu Asn Asp Phe Asp Val Asp Met Asp Leu
            420                 425                 430

Ser Glu Trp Met Arg Gln Trp Gly Gln Asp Gly Asp Asp Glu Gln Val
        435                 440                 445

Ile Arg Gly Thr Leu Gly Arg Leu Leu Phe Gly Ser Asn Asp Val Val
    450                 455                 460

Lys Lys Val Lys Val Leu Ser Gly Gly Glu Lys Gly Arg Met Leu Tyr
465                 470                 475                 480

Gly Lys Leu Leu Leu Leu Lys Pro Asn Val Leu Val Met Asp Glu Pro
                485                 490                 495

Thr Asn His Met Asp Met Glu Ser Ile Glu Ser Leu Asn Met Ala Leu
            500                 505                 510

Glu Lys Tyr Asn Gly Thr Leu Ile Phe Val Ser His Asp Arg Gln Phe
        515                 520                 525

Val Ser Ser Leu Ala Thr Gln Ile Ile Glu Leu Asp Gly Lys Gly Gly
    530                 535                 540

Tyr Glu His Tyr Leu Gly Asp Tyr Glu Ser Tyr Leu Glu Lys Lys Gly
545                 550                 555                 560

Val Ala

<210> 9
<211> 1632
<212> DNA
<213> Neisseria meningitidis

<220>
<221> CDS

<222> (1)..(1632)

<400> 9
atg ata aaa ccg aac ctg agg ccg aag ctc ggc tct tcc gcg ctg att    48
Met Ile Lys Pro Asn Leu Arg Pro Lys Leu Gly Ser Ser Ala Leu Ile
1               5                   10                  15

gcc ttc ctt tcc ctg tat tcc tcg ctg gta ttg aat tac gcc ttt ttt    96
Ala Phe Leu Ser Leu Tyr Ser Ser Leu Val Leu Asn Tyr Ala Phe Phe
            20                  25                  30

gcc aaa gtt gtc gag ctt cat cct ttt aac ggc acc ggg gcg gat atc    144
Ala Lys Val Val Glu Leu His Pro Phe Asn Gly Thr Gly Ala Asp Ile
        35                  40                  45

ttc ctc tat acg atg ccg gtg gtg ctg ttt ttt tta agt aat ttc gtt    192
Phe Leu Tyr Thr Met Pro Val Val Leu Phe Phe Leu Ser Asn Phe Val
    50                  55                  60

ttt cac gtc att gcc ctg cct ttc gtg cat aag gta ttg att ccg ttg    240
Phe His Val Ile Ala Leu Pro Phe Val His Lys Val Leu Ile Pro Leu
65                  70                  75                  80

ata ttg gtt atc agt gcg gcg gtg tct tac caa gaa ata ttt ttc aat    288
Ile Leu Val Ile Ser Ala Ala Val Ser Tyr Gln Glu Ile Phe Phe Asn
                85                  90                  95

atc tat ttc aac aag tcg atg ttg aat aat gtc ttg caa act acg gct    336
Ile Tyr Phe Asn Lys Ser Met Leu Asn Asn Val Leu Gln Thr Thr Ala
            100                 105                 110

gcc gaa agc gcg cgc ctg att acg ccg ggc tat gtg ctg tgg att gta    384
Ala Glu Ser Ala Arg Leu Ile Thr Pro Gly Tyr Val Leu Trp Ile Val
        115                 120                 125

tgt ttg ggc gta ttg ccc gcg ctg gcg tat atc gcc gtc aag gtt aaa    432
Cys Leu Gly Val Leu Pro Ala Leu Ala Tyr Ile Ala Val Lys Val Lys
    130                 135                 140

tac cgc gtt tgg tat aag gag ctt ttg acg cgc ctt gtg ctt gcc gcc    480
Tyr Arg Val Trp Tyr Lys Glu Leu Leu Thr Arg Leu Val Leu Ala Ala
145                 150                 155                 160

gtt tcc ttt ttg tgc gcg ttg ggc atc gca atg ttg caa tat cag gat    528
Val Ser Phe Leu Cys Ala Leu Gly Ile Ala Met Leu Gln Tyr Gln Asp
                165                 170                 175

tac gcc tcg ttt ttc cgc aac aat aaa tca gta acc cat ctg att gtg    576

Tyr Ala Ser Phe Phe Arg Asn Asn Lys Ser Val Thr His Leu Ile Val
            180                 185                 190

cca tct aat ttc atc ggc gcg ggc gtg tcg aaa tac aaa gat tgg aag     624
Pro Ser Asn Phe Ile Gly Ala Gly Val Ser Lys Tyr Lys Asp Trp Lys
        195                 200                 205

cgt tcc aat att cct tat acg caa ttg gat atg gcg gtc gtg caa aac     672
Arg Ser Asn Ile Pro Tyr Thr Gln Leu Asp Met Ala Val Val Gln Asn
    210                 215                 220

cgc ccg gcc ggc agc ctg cgc cgt ttc gtg gtg ctg gtc gtg ggc gag     720
Arg Pro Ala Gly Ser Leu Arg Arg Phe Val Val Leu Val Val Gly Glu
225                 230                 235                 240

acc acg cgt gcc gcc aac tgg ggt ttg aac ggt tac agc cgc caa act     768
Thr Thr Arg Ala Ala Asn Trp Gly Leu Asn Gly Tyr Ser Arg Gln Thr
                245                 250                 255

acg ccg ctg ctt gcc gcg cgc ggc gat gaa att gtc aat ttc ccg cag     816
Thr Pro Leu Leu Ala Ala Arg Gly Asp Glu Ile Val Asn Phe Pro Gln
            260                 265                 270

gtc aga agc tgc ggt aca tcg acc gcg cac tcc ctg ccg tgt atg ttc     864
Val Arg Ser Cys Gly Thr Ser Thr Ala His Ser Leu Pro Cys Met Phe
        275                 280                 285

tca acc ttc gac cgc acg gat tat gac gaa atc aaa gcc gaa cac caa     912
Ser Thr Phe Asp Arg Thr Asp Tyr Asp Glu Ile Lys Ala Glu His Gln
    290                 295                 300

gac aac ctg ctg gac atc gtg cag cgc gcc ggc gtg gaa gtt acc tgg     960
Asp Asn Leu Leu Asp Ile Val Gln Arg Ala Gly Val Glu Val Thr Trp
305                 310                 315                 320

ttg gaa aac gat tcc ggc tgc aag ggc gtg tgc ggc aaa gtg ccg aat     1008
Leu Glu Asn Asp Ser Gly Cys Lys Gly Val Cys Gly Lys Val Pro Asn
                325                 330                 335

acc gac gtt acc tcg ctc aac ctg ccc gaa tac tgc cgc aac ggc gag     1056
Thr Asp Val Thr Ser Leu Asn Leu Pro Glu Tyr Cys Arg Asn Gly Glu
            340                 345                 350

tgc ctc gac aat atc ctg ctg acc aag ttc gac gaa gtc ctc aac aaa     1104
Cys Leu Asp Asn Ile Leu Leu Thr Lys Phe Asp Glu Val Leu Asn Lys
        355                 360                 365

aac gat aaa gac gcg gtt tta atc ctg cat acc atc ggc agc cac ggg     1152

Asn Asp Lys Asp Ala Val Leu Ile Leu His Thr Ile Gly Ser His Gly
    370                 375                 380

ccg acg tat tac gaa cgc tat acc gaa gcc gaa cgc aaa ttc acg ccg     1200
Pro Thr Tyr Tyr Glu Arg Tyr Thr Glu Ala Glu Arg Lys Phe Thr Pro
385                 390                 395                 400

acc tgc gac acc aac gaa atc aac aaa tgc acc cgc gcc acg ctg gtc     1248
Thr Cys Asp Thr Asn Glu Ile Asn Lys Cys Thr Arg Ala Thr Leu Val
                405                 410                 415

aac act tac gac aat acg gtt ttg tat gtg gac cag ttt atc gac aag     1296
Asn Thr Tyr Asp Asn Thr Val Leu Tyr Val Asp Gln Phe Ile Asp Lys
            420                 425                 430

gtt atc cgc aaa ctt gaa aac cgc gac gat ttg gaa agc gtg gtg cat     1344
Val Ile Arg Lys Leu Glu Asn Arg Asp Asp Leu Glu Ser Val Val His
        435                 440                 445

tat gtt tcc gac cac ggc gaa agt ttg ggc gaa aac ggg atg tac ctg     1392
Tyr Val Ser Asp His Gly Glu Ser Leu Gly Glu Asn Gly Met Tyr Leu
    450                 455                 460

cac gcc gcg cct tac gcc atc gcg cct tcc ggg cag acg cat atc ccg     1440
His Ala Ala Pro Tyr Ala Ile Ala Pro Ser Gly Gln Thr His Ile Pro
465                 470                 475                 480

atg gtt atg tgg ttt tcc aaa gcc ttc cgc caa cac ggg ggc ata gat     1488
Met Val Met Trp Phe Ser Lys Ala Phe Arg Gln His Gly Gly Ile Asp
                485                 490                 495

ttc caa tgc ctc aaa caa aaa gcg gcg gaa aac gaa tat tcg cac gac     1536
Phe Gln Cys Leu Lys Gln Lys Ala Ala Glu Asn Glu Tyr Ser His Asp
            500                 505                 510

cac tat ttc agc acg gta ttg ggg ctg atg gac att tcc aac agc caa     1584
His Tyr Phe Ser Thr Val Leu Gly Leu Met Asp Ile Ser Asn Ser Gln
        515                 520                 525

acc tat cgg aag gaa atg gat ata ttg gca gcc tgc cgc cgt ccg cgc     1632
Thr Tyr Arg Lys Glu Met Asp Ile Leu Ala Ala Cys Arg Arg Pro Arg
    530                 535                 540

<210> 10
<211> 544
<212> PRT
<213> Neisseria meningitidis

<400> 10
Met Ile Lys Pro Asn Leu Arg Pro Lys Leu Gly Ser Ser Ala Leu Ile
1               5                   10                  15

Ala Phe Leu Ser Leu Tyr Ser Ser Leu Val Leu Asn Tyr Ala Phe Phe
            20                  25                  30

Ala Lys Val Val Glu Leu His Pro Phe Asn Gly Thr Gly Ala Asp Ile
        35                  40                  45

Phe Leu Tyr Thr Met Pro Val Val Leu Phe Phe Leu Ser Asn Phe Val
    50                  55                  60

Phe His Val Ile Ala Leu Pro Phe Val His Lys Val Leu Ile Pro Leu
65                  70                  75                  80

Ile Leu Val Ile Ser Ala Ala Val Ser Tyr Gln Glu Ile Phe Phe Asn
                85                  90                  95

Ile Tyr Phe Asn Lys Ser Met Leu Asn Asn Val Leu Gln Thr Thr Ala
            100                 105                 110

Ala Glu Ser Ala Arg Leu Ile Thr Pro Gly Tyr Val Leu Trp Ile Val
        115                 120                 125

Cys Leu Gly Val Leu Pro Ala Leu Ala Tyr Ile Ala Val Lys Val Lys
    130                 135                 140

Tyr Arg Val Trp Tyr Lys Glu Leu Leu Thr Arg Leu Val Leu Ala Ala
145                 150                 155                 160

Val Ser Phe Leu Cys Ala Leu Gly Ile Ala Met Leu Gln Tyr Gln Asp
                165                 170                 175

Tyr Ala Ser Phe Phe Arg Asn Asn Lys Ser Val Thr His Leu Ile Val
            180                 185                 190

Pro Ser Asn Phe Ile Gly Ala Gly Val Ser Lys Tyr Lys Asp Trp Lys
        195                 200                 205

Arg Ser Asn Ile Pro Tyr Thr Gln Leu Asp Met Ala Val Val Gln Asn
    210                 215                 220

Arg Pro Ala Gly Ser Leu Arg Arg Phe Val Val Leu Val Val Gly Glu
225                 230                 235                 240

Thr Thr Arg Ala Ala Asn Trp Gly Leu Asn Gly Tyr Ser Arg Gln Thr

245                 250                 255

Thr Pro Leu Leu Ala Ala Arg Gly Asp Glu Ile Val Asn Phe Pro Gln
            260                 265                 270

Val Arg Ser Cys Gly Thr Ser Thr Ala His Ser Leu Pro Cys Met Phe
        275                 280                 285

Ser Thr Phe Asp Arg Thr Asp Tyr Asp Glu Ile Lys Ala Glu His Gln
    290                 295                 300

Asp Asn Leu Leu Asp Ile Val Gln Arg Ala Gly Val Glu Val Thr Trp
305                 310                 315                 320

Leu Glu Asn Asp Ser Gly Cys Lys Gly Val Cys Gly Lys Val Pro Asn
                325                 330                 335

Thr Asp Val Thr Ser Leu Asn Leu Pro Glu Tyr Cys Arg Asn Gly Glu
            340                 345                 350

Cys Leu Asp Asn Ile Leu Leu Thr Lys Phe Asp Glu Val Leu Asn Lys
        355                 360                 365

Asn Asp Lys Asp Ala Val Leu Ile Leu His Thr Ile Gly Ser His Gly
    370                 375                 380

Pro Thr Tyr Tyr Glu Arg Tyr Thr Glu Ala Glu Arg Lys Phe Thr Pro
385                 390                 395                 400

Thr Cys Asp Thr Asn Glu Ile Asn Lys Cys Thr Arg Ala Thr Leu Val
                405                 410                 415

Asn Thr Tyr Asp Asn Thr Val Leu Tyr Val Asp Gln Phe Ile Asp Lys
            420                 425                 430

Val Ile Arg Lys Leu Glu Asn Arg Asp Asp Leu Glu Ser Val Val His
        435                 440                 445

Tyr Val Ser Asp His Gly Glu Ser Leu Gly Glu Asn Gly Met Tyr Leu
    450                 455                 460

His Ala Ala Pro Tyr Ala Ile Ala Pro Ser Gly Gln Thr His Ile Pro
465                 470                 475                 480

Met Val Met Trp Phe Ser Lys Ala Phe Arg Gln His Gly Gly Ile Asp
                485                 490                 495

Phe Gln Cys Leu Lys Gln Lys Ala Ala Glu Asn Glu Tyr Ser His Asp

500                 505                 510

His Tyr Phe Ser Thr Val Leu Gly Leu Met Asp Ile Ser Asn Ser Gln
        515                 520                 525

Thr Tyr Arg Lys Glu Met Asp Ile Leu Ala Ala Cys Arg Arg Pro Arg
    530                 535                 540

<210> 11
<211> 1052
<212> DNA
<213> Neisseria meningitidis

<220>
<221> CDS
<222> (1)..(1050)

<400> 11
atg aca cac cat tac ccc aca gac gat att aag att aaa gaa gtt aaa      48
Met Thr His His Tyr Pro Thr Asp Asp Ile Lys Ile Lys Glu Val Lys
1               5                   10                  15

gag ttg ttg ccg ccg att gcc cat ctt tac gag ctg ccg att tcc aaa      96
Glu Leu Leu Pro Pro Ile Ala His Leu Tyr Glu Leu Pro Ile Ser Lys
            20                  25                  30

gag gct tcg ggc ttg gtt cac cgc acc cgt cag gaa att tcc gat ttg      144
Glu Ala Ser Gly Leu Val His Arg Thr Arg Gln Glu Ile Ser Asp Leu
        35                  40                  45

gtt cac ggc agg gac aag cgg ctg ttg gtt att atc ggg ccg tgt tcg      192
Val His Gly Arg Asp Lys Arg Leu Leu Val Ile Ile Gly Pro Cys Ser
    50                  55                  60

att cac gat ccg aaa gcg gcg ttg gaa tat gcg gag cgt ttg ttg aaa      240
Ile His Asp Pro Lys Ala Ala Leu Glu Tyr Ala Glu Arg Leu Leu Lys
65                  70                  75                  80

ctc cgc aag cag tat gaa aac gag ctt ttg att gtg atg cgc gtt tat      288
Leu Arg Lys Gln Tyr Glu Asn Glu Leu Leu Ile Val Met Arg Val Tyr
                85                  90                  95

ttc gag aag ccg agg acg acg gtg ggt tgg aaa ggt ttg att aac gac      336
Phe Glu Lys Pro Arg Thr Thr Val Gly Trp Lys Gly Leu Ile Asn Asp
            100                 105                 110

ccg cat ttg gac ggt acg ttt gac atc aat ttc ggt ttg cgt cag gca 384
Pro His Leu Asp Gly Thr Phe Asp Ile Asn Phe Gly Leu Arg Gln Ala
        115                 120                 125

cgc agc ctg ttg ttg tcg ctg aac aat atg ggt atg cct gcc tct acc 432
Arg Ser Leu Leu Leu Ser Leu Asn Asn Met Gly Met Pro Ala Ser Thr
    130                 135                 140

gag ttt ttg gat atg att acg ccg caa tat tat gcg gac ttg att tct 480
Glu Phe Leu Asp Met Ile Thr Pro Gln Tyr Tyr Ala Asp Leu Ile Ser
145                 150                 155                 160

tgg ggg gca atc ggc gcg cgg acg acc gaa agc caa gtt cac cgc gaa 528
Trp Gly Ala Ile Gly Ala Arg Thr Thr Glu Ser Gln Val His Arg Glu
                165                 170                 175

ttg gca agc ggg ctg tcc tgc ccc gtc ggc ttt aaa aac ggt acg gat 576
Leu Ala Ser Gly Leu Ser Cys Pro Val Gly Phe Lys Asn Gly Thr Asp
            180                 185                 190

ggc aat ttg aag att gcc atc gac gca atc ggt gcg gcg agc cat tcg 624
Gly Asn Leu Lys Ile Ala Ile Asp Ala Ile Gly Ala Ala Ser His Ser
        195                 200                 205

cat cat ttc ctg tct gta acc aag gcc ggg cat tcc gcc att gtc cat 672
His His Phe Leu Ser Val Thr Lys Ala Gly His Ser Ala Ile Val His
    210                 215                 220

acc ggc ggc aat cct gac tgt cat gtc att ttg cgc ggc gga aaa gag 720
Thr Gly Gly Asn Pro Asp Cys His Val Ile Leu Arg Gly Gly Lys Glu
225                 230                 235                 240

ccg aat tat gat gcg gga cac gtc agc gag gcg gcg gaa caa ctg cgt 768
Pro Asn Tyr Asp Ala Gly His Val Ser Glu Ala Ala Glu Gln Leu Arg
                245                 250                 255

gcg gca ggc gta acc gac aag ctg atg att gat tgc agc cac gcc aac 816
Ala Ala Gly Val Thr Asp Lys Leu Met Ile Asp Cys Ser His Ala Asn
            260                 265                 270

agc cgc aag gat tac acc cga cag atg gaa gtg gcg caa gac att gcc 864
Ser Arg Lys Asp Tyr Thr Arg Gln Met Glu Val Ala Gln Asp Ile Ala
        275                 280                 285

gcc caa ttg gaa cag gac ggc ggc aat att atg ggc gtg atg gtg gaa 912
Ala Gln Leu Glu Gln Asp Gly Gly Asn Ile Met Gly Val Met Val Glu
    290                 295                 300

agc cat ttg gtc gaa ggc agg cag gac aag ccg gaa gtg tac ggc aaa     960
Ser His Leu Val Glu Gly Arg Gln Asp Lys Pro Glu Val Tyr Gly Lys
305                 310                 315                 320

agc att acc gat gcg tgt att ggt tgg gac gcg act gaa gaa ctg ttg     1008
Ser Ile Thr Asp Ala Cys Ile Gly Trp Asp Ala Thr Glu Glu Leu Leu
                325                 330                 335

gca ttg ttg gca ggt gca aac aaa aaa cgt atg gcg cgc gcc gg          1052
Ala Leu Leu Ala Gly Ala Asn Lys Lys Arg Met Ala Arg Ala
            340                 345                 350

<210> 12
<211> 350
<212> PRT
<213> Neisseria meningitidis

<400> 12
Met Thr His His Tyr Pro Thr Asp Asp Ile Lys Ile Lys Glu Val Lys
1               5                   10                  15

Glu Leu Leu Pro Pro Ile Ala His Leu Tyr Glu Leu Pro Ile Ser Lys
            20                  25                  30

Glu Ala Ser Gly Leu Val His Arg Thr Arg Gln Glu Ile Ser Asp Leu
        35                  40                  45

Val His Gly Arg Asp Lys Arg Leu Leu Val Ile Ile Gly Pro Cys Ser
    50                  55                  60

Ile His Asp Pro Lys Ala Ala Leu Glu Tyr Ala Glu Arg Leu Leu Lys
65                  70                  75                  80

Leu Arg Lys Gln Tyr Glu Asn Glu Leu Leu Ile Val Met Arg Val Tyr
                85                  90                  95

Phe Glu Lys Pro Arg Thr Thr Val Gly Trp Lys Gly Leu Ile Asn Asp
            100                 105                 110

Pro His Leu Asp Gly Thr Phe Asp Ile Asn Phe Gly Leu Arg Gln Ala
        115                 120                 125

Arg Ser Leu Leu Leu Ser Leu Asn Asn Met Gly Met Pro Ala Ser Thr
    130                 135                 140

Glu Phe Leu Asp Met Ile Thr Pro Gln Tyr Tyr Ala Asp Leu Ile Ser
145                 150                 155                 160

Trp Gly Ala Ile Gly Ala Arg Thr Thr Glu Ser Gln Val His Arg Glu
                165                 170                 175

Leu Ala Ser Gly Leu Ser Cys Pro Val Gly Phe Lys Asn Gly Thr Asp
            180                 185                 190

Gly Asn Leu Lys Ile Ala Ile Asp Ala Ile Gly Ala Ala Ser His Ser
        195                 200                 205

His His Phe Leu Ser Val Thr Lys Ala Gly His Ser Ala Ile Val His
    210                 215                 220

Thr Gly Gly Asn Pro Asp Cys His Val Ile Leu Arg Gly Gly Lys Glu
225                 230                 235                 240

Pro Asn Tyr Asp Ala Gly His Val Ser Glu Ala Ala Glu Gln Leu Arg
                245                 250                 255

Ala Ala Gly Val Thr Asp Lys Leu Met Ile Asp Cys Ser His Ala Asn
            260                 265                 270

Ser Arg Lys Asp Tyr Thr Arg Gln Met Glu Val Ala Gln Asp Ile Ala
        275                 280                 285

Ala Gln Leu Glu Gln Asp Gly Gly Asn Ile Met Gly Val Met Val Glu
    290                 295                 300

Ser His Leu Val Glu Gly Arg Gln Asp Lys Pro Glu Val Tyr Gly Lys
305                 310                 315                 320

Ser Ile Thr Asp Ala Cys Ile Gly Trp Asp Ala Thr Glu Glu Leu Leu
                325                 330                 335

Ala Leu Leu Ala Gly Ala Asn Lys Lys Arg Met Ala Arg Ala
            340                 345                 350

<210> 13
<211> 432
<212> DNA
<213> Neisseria meningitidis

<220>
<221> CDS
<222> (1)..(432)

<400> 13
atg gca ttt ggt tcg atg aat tcc ggc gac gat tct ccg atg tcc gac    48
Met Ala Phe Gly Ser Met Asn Ser Gly Asp Asp Ser Pro Met Ser Asp
1               5                   10                  15

atc aac gtt acg ccg ttg gtg gac gtg atg ctg gtg ttg ctg att gtg    96
Ile Asn Val Thr Pro Leu Val Asp Val Met Leu Val Leu Leu Ile Val
            20                  25                  30

ttt atg att act atg ccg gtg ctg acg cat tcc atc cct ttg gaa ctg    144
Phe Met Ile Thr Met Pro Val Leu Thr His Ser Ile Pro Leu Glu Leu
        35                  40                  45

ccg acc gcg tcc gag cag aca aac aag cag gac aaa cag cct aaa gac    192
Pro Thr Ala Ser Glu Gln Thr Asn Lys Gln Asp Lys Gln Pro Lys Asp
    50                  55                  60

ccc ctg cgc ctg acg att gat gcg aac ggc ggc tat tat gtc ggc ggg    240
Pro Leu Arg Leu Thr Ile Asp Ala Asn Gly Gly Tyr Tyr Val Gly Gly
65                  70                  75                  80

gat tct gca agc aaa gtg gaa atc ggg gaa gtg gaa agc cgt ctg aaa    288
Asp Ser Ala Ser Lys Val Glu Ile Gly Glu Val Glu Ser Arg Leu Lys
                85                  90                  95

gcc gcc aag gag cag aat gaa aac gtg att gtg gcg att gcg gca gac    336
Ala Ala Lys Glu Gln Asn Glu Asn Val Ile Val Ala Ile Ala Ala Asp
            100                 105                 110

aag gcg gtg gaa tac gat tat gta aac aaa gct tta gaa gcc gcc cgt    384
Lys Ala Val Glu Tyr Asp Tyr Val Asn Lys Ala Leu Glu Ala Ala Arg
        115                 120                 125

cag gca gga atc acc aaa atc ggt ttt gta acc gaa acc aag gcg caa    432
Gln Ala Gly Ile Thr Lys Ile Gly Phe Val Thr Glu Thr Lys Ala Gln
    130                 135                 140

<210> 14
<211> 144
<212> PRT
<213> Neisseria meningitidis

<400> 14
Met Ala Phe Gly Ser Met Asn Ser Gly Asp Asp Ser Pro Met Ser Asp
1               5                   10                  15

Ile Asn Val Thr Pro Leu Val Asp Val Met Leu Val Leu Leu Ile Val

20                  25                  30

Phe Met Ile Thr Met Pro Val Leu Thr His Ser Ile Pro Leu Glu Leu
        35                  40                  45

Pro Thr Ala Ser Glu Gln Thr Asn Lys Gln Asp Lys Gln Pro Lys Asp
    50                  55                  60

Pro Leu Arg Leu Thr Ile Asp Ala Asn Gly Gly Tyr Tyr Val Gly Gly
65                  70                  75                  80

Asp Ser Ala Ser Lys Val Glu Ile Gly Glu Val Glu Ser Arg Leu Lys
                85                  90                  95

Ala Ala Lys Glu Gln Asn Glu Asn Val Ile Val Ala Ile Ala Ala Asp
            100                 105                 110

Lys Ala Val Glu Tyr Asp Tyr Val Asn Lys Ala Leu Glu Ala Ala Arg
        115                 120                 125

Gln Ala Gly Ile Thr Lys Ile Gly Phe Val Thr Glu Thr Lys Ala Gln
    130                 135                 140

<210> 15
<211> 267
<212> DNA
<213> Neisseria meningitidis

<220>
<221> CDS
<222> (1)..(267)

<400> 15
atg ctc aaa caa tcc atc gaa atc atc aac aaa ctc gga ctc cac gcc    48
Met Leu Lys Gln Ser Ile Glu Ile Ile Asn Lys Leu Gly Leu His Ala
1               5                   10                  15

cgc gcg tcc aac aag ttc acc caa acc gcg tcc caa ttc aaa agc gaa    96
Arg Ala Ser Asn Lys Phe Thr Gln Thr Ala Ser Gln Phe Lys Ser Glu
            20                  25                  30

gtc tgg gtt acg aaa aac gac agc cgc gtc aac ggc aaa agc att atg    144
Val Trp Val Thr Lys Asn Asp Ser Arg Val Asn Gly Lys Ser Ile Met
        35                  40                  45

ggg ctg atg atg ctc gcc gcc gcc aag ggt acg gtc atc gaa ctg gag    192

Gly Leu Met Met Leu Ala Ala Ala Lys Gly Thr Val Ile Glu Leu Glu
     50                  55                  60

acg gac ggc gcg gac gag gcg gaa gcg atg cgc gcc ctg acc gac tta     240
Thr Asp Gly Ala Asp Glu Ala Glu Ala Met Arg Ala Leu Thr Asp Leu
 65                  70                  75                  80

atc aac ggc tac ttc ggc gag ggc gaa                                 267
Ile Asn Gly Tyr Phe Gly Glu Gly Glu
                 85

<210> 16
<211> 89
<212> PRT
<213> Neisseria meningitidis

<400> 16
Met Leu Lys Gln Ser Ile Glu Ile Ile Asn Lys Leu Gly Leu His Ala
  1               5                  10                  15

Arg Ala Ser Asn Lys Phe Thr Gln Thr Ala Ser Gln Phe Lys Ser Glu
             20                  25                  30

Val Trp Val Thr Lys Asn Asp Ser Arg Val Asn Gly Lys Ser Ile Met
         35                  40                  45

Gly Leu Met Met Leu Ala Ala Ala Lys Gly Thr Val Ile Glu Leu Glu
     50                  55                  60

Thr Asp Gly Ala Asp Glu Ala Glu Ala Met Arg Ala Leu Thr Asp Leu
 65                  70                  75                  80

Ile Asn Gly Tyr Phe Gly Glu Gly Glu
                 85

<210> 17
<211> 633
<212> DNA
<213> Neisseria meningitidis

<220>
<221> CDS
<222> (1)..(633)

<400> 17

atg ccc atg aac ctt ttt caa aac gcc aaa ttc ttc acg acg gta aac     48
Met Pro Met Asn Leu Phe Gln Asn Ala Lys Phe Phe Thr Thr Val Asn
1               5                   10                  15

cat ctc aaa gac ctg ccg gac aca ccg ctc gaa att gcc ttt gtc ggc     96
His Leu Lys Asp Leu Pro Asp Thr Pro Leu Glu Ile Ala Phe Val Gly
            20                  25                  30

agg agc aat gcc gga aaa tcc agt gcc atc aat acc ctg acc aac cat     144
Arg Ser Asn Ala Gly Lys Ser Ser Ala Ile Asn Thr Leu Thr Asn His
        35                  40                  45

gtc cgt ctt gcc tac gtt tca aaa aca ccc gga cgg acg cag cat atc     192
Val Arg Leu Ala Tyr Val Ser Lys Thr Pro Gly Arg Thr Gln His Ile
    50                  55                  60

aac ttc ttc gag ctg cag aac ggc aat ttt atg gtc gat ttg ccc ggc     240
Asn Phe Phe Glu Leu Gln Asn Gly Asn Phe Met Val Asp Leu Pro Gly
65                  70                  75                  80

tac ggt tat gcc caa gtc ccc gaa gca gta cgc gca cat tgg gtc aat     288
Tyr Gly Tyr Ala Gln Val Pro Glu Ala Val Arg Ala His Trp Val Asn
                85                  90                  95

ctg ctc ggc gac tat ctg caa cag cgc aaa cag ctt atc ggg ctg gtt     336
Leu Leu Gly Asp Tyr Leu Gln Gln Arg Lys Gln Leu Ile Gly Leu Val
            100                 105                 110

ttg att atg gat gcc cgc cat cct tta aaa gaa ctc gac atc cgt atg     384
Leu Ile Met Asp Ala Arg His Pro Leu Lys Glu Leu Asp Ile Arg Met
        115                 120                 125

ctg gac ttt ttc cac acg acc ggc aga ccg gtt cac atc ctg ctg tca     432
Leu Asp Phe Phe His Thr Thr Gly Arg Pro Val His Ile Leu Leu Ser
    130                 135                 140

aaa gcc gac aaa tta tcc aaa aac gaa cag ata aaa acc ctg tcc caa     480
Lys Ala Asp Lys Leu Ser Lys Asn Glu Gln Ile Lys Thr Leu Ser Gln
145                 150                 155                 160

gtc aaa aaa ctg ctc aaa cct tat tcc gac agg caa aac atc agc gta     528
Val Lys Lys Leu Leu Lys Pro Tyr Ser Asp Arg Gln Asn Ile Ser Val
                165                 170                 175

cag ctg ttt tcc agc ctg aaa aaa caa ggt att gac gag gcc aac cga     576
Gln Leu Phe Ser Ser Leu Lys Lys Gln Gly Ile Asp Glu Ala Asn Arg
            180                 185                 190

act gtc gga agc tgg ttt gac gca gca gat gcc gcc gct tcc tct cca 624
Thr Val Gly Ser Trp Phe Asp Ala Ala Asp Ala Ala Ala Ser Ser Pro
        195                 200                 205

aag gaa aac 633
Lys Glu Asn
    210

<210> 18
<211> 211
<212> PRT
<213> Neisseria meningitidis

<400> 18
Met Pro Met Asn Leu Phe Gln Asn Ala Lys Phe Phe Thr Thr Val Asn
1               5                   10                  15

His Leu Lys Asp Leu Pro Asp Thr Pro Leu Glu Ile Ala Phe Val Gly
            20                  25                  30

Arg Ser Asn Ala Gly Lys Ser Ser Ala Ile Asn Thr Leu Thr Asn His
        35                  40                  45

Val Arg Leu Ala Tyr Val Ser Lys Thr Pro Gly Arg Thr Gln His Ile
    50                  55                  60

Asn Phe Phe Glu Leu Gln Asn Gly Asn Phe Met Val Asp Leu Pro Gly
65                  70                  75                  80

Tyr Gly Tyr Ala Gln Val Pro Glu Ala Val Arg Ala His Trp Val Asn
                85                  90                  95

Leu Leu Gly Asp Tyr Leu Gln Gln Arg Lys Gln Leu Ile Gly Leu Val
            100                 105                 110

Leu Ile Met Asp Ala Arg His Pro Leu Lys Glu Leu Asp Ile Arg Met
        115                 120                 125

Leu Asp Phe Phe His Thr Thr Gly Arg Pro Val His Ile Leu Leu Ser
    130                 135                 140

Lys Ala Asp Lys Leu Ser Lys Asn Glu Gln Ile Lys Thr Leu Ser Gln
145                 150                 155                 160

Val Lys Lys Leu Leu Lys Pro Tyr Ser Asp Arg Gln Asn Ile Ser Val
                165                 170                 175

Gln Leu Phe Ser Ser Leu Lys Lys Gln Gly Ile Asp Glu Ala Asn Arg
            180                 185                 190

Thr Val Gly Ser Trp Phe Asp Ala Ala Asp Ala Ala Ala Ser Ser Pro
        195                 200                 205

Lys Glu Asn
    210

<210> 19
<211> 621
<212> DNA
<213> Neisseria meningitidis

<220>
<221> CDS
<222> (1)..(621)

<400> 19
atg aaa cga ttg act tta ttg gcc ttt gtt ttg gct gcc ggt gcg gtt      48
Met Lys Arg Leu Thr Leu Leu Ala Phe Val Leu Ala Ala Gly Ala Val
  1               5                  10                  15

tcc gca tct ccc aaa gca gac gtg gaa aaa ggc aaa cag gtt gcc gca      96
Ser Ala Ser Pro Lys Ala Asp Val Glu Lys Gly Lys Gln Val Ala Ala
            20                  25                  30

acg gtt tgt gcg gct tgc cat gca gca gac ggt aac agc ggc att gcg     144
Thr Val Cys Ala Ala Cys His Ala Ala Asp Gly Asn Ser Gly Ile Ala
        35                  40                  45

atg tat ccg cgt ttg gcg gca cag cat act gct tac atc tat cat caa     192
Met Tyr Pro Arg Leu Ala Ala Gln His Thr Ala Tyr Ile Tyr His Gln
     50                  55                  60

acc atc ggc atc cgc gac ggt aaa cgc acc cac ggt tcg gca gct gtg     240
Thr Ile Gly Ile Arg Asp Gly Lys Arg Thr His Gly Ser Ala Ala Val
 65                  70                  75                  80

atg aaa ccg gtg gta atg aat ttg agc gat cag gat att ttg aac gta     288
Met Lys Pro Val Val Met Asn Leu Ser Asp Gln Asp Ile Leu Asn Val
                 85                  90                  95

tcc gca ttc tat gcc aaa cag cag ccc aaa tcc ggt gaa gcc aat cct     336
Ser Ala Phe Tyr Ala Lys Gln Gln Pro Lys Ser Gly Glu Ala Asn Pro
            100                 105                 110

aag gaa aat ccc gaa ttg ggt gcg aaa atc tat cgc ggc ggt ttg agc 384
Lys Glu Asn Pro Glu Leu Gly Ala Lys Ile Tyr Arg Gly Gly Leu Ser
        115                 120                 125

gat aaa aaa gtg ccg gcg tgt atg tcc tgc cac ggt ccg agc ggt gcg 432
Asp Lys Lys Val Pro Ala Cys Met Ser Cys His Gly Pro Ser Gly Ala
    130                 135                 140

ggt atg ccg ggg ggc gga agc gaa att cag gct tat ccg cgt ttg ggc 480
Gly Met Pro Gly Gly Gly Ser Glu Ile Gln Ala Tyr Pro Arg Leu Gly
145                 150                 155                 160

ggt cag cat cag gca tat att gtt gaa cag atg aat gcc tac aag tcc 528
Gly Gln His Gln Ala Tyr Ile Val Glu Gln Met Asn Ala Tyr Lys Ser
                165                 170                 175

ggt cag cgt aaa aat acc atc atg gaa gat att gca aac cgt atg tct 576
Gly Gln Arg Lys Asn Thr Ile Met Glu Asp Ile Ala Asn Arg Met Ser
            180                 185                 190

gaa gaa gat ttg aaa gcg gtc gcc aac ttt atc caa ggt ttg cgt 621
Glu Glu Asp Leu Lys Ala Val Ala Asn Phe Ile Gln Gly Leu Arg
        195                 200                 205

<210> 20
<211> 207
<212> PRT
<213> Neisseria meningitidis

<400> 20
Met Lys Arg Leu Thr Leu Leu Ala Phe Val Leu Ala Ala Gly Ala Val
1               5                   10                  15

Ser Ala Ser Pro Lys Ala Asp Val Glu Lys Gly Lys Gln Val Ala Ala
            20                  25                  30

Thr Val Cys Ala Ala Cys His Ala Ala Asp Gly Asn Ser Gly Ile Ala
        35                  40                  45

Met Tyr Pro Arg Leu Ala Ala Gln His Thr Ala Tyr Ile Tyr His Gln
    50                  55                  60

Thr Ile Gly Ile Arg Asp Gly Lys Arg Thr His Gly Ser Ala Ala Val
65                  70                  75                  80

Met Lys Pro Val Val Met Asn Leu Ser Asp Gln Asp Ile Leu Asn Val

85                  90                  95

Ser Ala Phe Tyr Ala Lys Gln Gln Pro Lys Ser Gly Glu Ala Asn Pro
            100                 105                 110

Lys Glu Asn Pro Glu Leu Gly Ala Lys Ile Tyr Arg Gly Gly Leu Ser
        115                 120                 125

Asp Lys Lys Val Pro Ala Cys Met Ser Cys His Gly Pro Ser Gly Ala
    130                 135                 140

Gly Met Pro Gly Gly Gly Ser Glu Ile Gln Ala Tyr Pro Arg Leu Gly
145                 150                 155                 160

Gly Gln His Gln Ala Tyr Ile Val Glu Gln Met Asn Ala Tyr Lys Ser
                165                 170                 175

Gly Gln Arg Lys Asn Thr Ile Met Glu Asp Ile Ala Asn Arg Met Ser
            180                 185                 190

Glu Glu Asp Leu Lys Ala Val Ala Asn Phe Ile Gln Gly Leu Arg
        195                 200                 205

<210> 21
<211> 765
<212> DNA
<213> Neisseria meningitidis

<220>
<221> CDS
<222> (1)..(765)

<400> 21
atg ccg tct gaa gcc cga ttt ttc agg ctt cag acg gca ttt ccc cgt    48
Met Pro Ser Glu Ala Arg Phe Phe Arg Leu Gln Thr Ala Phe Pro Arg
1               5                   10                  15

ttg att tgc ggt ata atc cgc ctt tac cca ttg ttt gca aag cac aat    96
Leu Ile Cys Gly Ile Ile Arg Leu Tyr Pro Leu Phe Ala Lys His Asn
            20                  25                  30

atg aca cgc aaa atc ctc gtt acc tcc gcc ctg ccc tat gcc aac ggc    144
Met Thr Arg Lys Ile Leu Val Thr Ser Ala Leu Pro Tyr Ala Asn Gly
        35                  40                  45

agc atc cac ctc ggc cac atg gtc gaa cac atc caa acc gac gtt tgg    192

Ser Ile His Leu Gly His Met Val Glu His Ile Gln Thr Asp Val Trp
    50                  55                  60

gtg cgc ttt caa aaa ctg cgc ggc aac gaa tgc cac tac tgc tgc gcc 240
Val Arg Phe Gln Lys Leu Arg Gly Asn Glu Cys His Tyr Cys Cys Ala
65                  70                  75                  80

gac gac acc cac ggc acg ccc gtg atg ctt gcc gcg caa aaa caa ggc 288
Asp Asp Thr His Gly Thr Pro Val Met Leu Ala Ala Gln Lys Gln Gly
                85                  90                  95

atc gcg ccc gaa gac atg att gcc aaa gtg cgc gaa gag cac ctc gcc 336
Ile Ala Pro Glu Asp Met Ile Ala Lys Val Arg Glu Glu His Leu Ala
            100                 105                 110

gac ttt act ggc ttt ttc atc ggt tac gac aat tat tac agc acc cat 384
Asp Phe Thr Gly Phe Phe Ile Gly Tyr Asp Asn Tyr Tyr Ser Thr His
        115                 120                 125

tcc cct gaa aac aaa cag ttt tcc caa gac att tac cgc gcg ctg aaa 432
Ser Pro Glu Asn Lys Gln Phe Ser Gln Asp Ile Tyr Arg Ala Leu Lys
    130                 135                 140

gcc aac ggc aag att gag agc cgc gtc atc gag cag ctt ttc gac ccc 480
Ala Asn Gly Lys Ile Glu Ser Arg Val Ile Glu Gln Leu Phe Asp Pro
145                 150                 155                 160

gaa aaa caa atg ttc ctg ccc gac cgc ttc gtt aaa ggc gaa tgc ccg 528
Glu Lys Gln Met Phe Leu Pro Asp Arg Phe Val Lys Gly Glu Cys Pro
                165                 170                 175

aaa tgc cac gcc caa gac caa tac ggc gac aac tgc gaa gtc tgc ggc 576
Lys Cys His Ala Gln Asp Gln Tyr Gly Asp Asn Cys Glu Val Cys Gly
            180                 185                 190

acg acc tat tcc ccg acc gaa ctg att aac ccg tat tcc gcc gtt tcc 624
Thr Thr Tyr Ser Pro Thr Glu Leu Ile Asn Pro Tyr Ser Ala Val Ser
        195                 200                 205

ggc gcg aaa ccc gaa ttg cgc gaa tcc gaa cac ttc ttc ttc aaa ctg 672
Gly Ala Lys Pro Glu Leu Arg Glu Ser Glu His Phe Phe Phe Lys Leu
    210                 215                 220

gga gaa tgc gcc gac ttc ctc aaa gca tgg act tcc ggc aac aac ccg 720
Gly Glu Cys Ala Asp Phe Leu Lys Ala Trp Thr Ser Gly Asn Asn Pro
225                 230                 235                 240

cac gac ggc aag ccc cat ctg caa gcc gaa gcc ctc aac aaa atg 765

His Asp Gly Lys Pro His Leu Gln Ala Glu Ala Leu Asn Lys Met
                245                 250                 255

<210> 22
<211> 255
<212> PRT
<213> Neisseria meningitidis

<400> 22
Met Pro Ser Glu Ala Arg Phe Phe Arg Leu Gln Thr Ala Phe Pro Arg
1               5                   10                  15

Leu Ile Cys Gly Ile Ile Arg Leu Tyr Pro Leu Phe Ala Lys His Asn
            20                  25                  30

Met Thr Arg Lys Ile Leu Val Thr Ser Ala Leu Pro Tyr Ala Asn Gly
        35                  40                  45

Ser Ile His Leu Gly His Met Val Glu His Ile Gln Thr Asp Val Trp
    50                  55                  60

Val Arg Phe Gln Lys Leu Arg Gly Asn Glu Cys His Tyr Cys Cys Ala
65                  70                  75                  80

Asp Asp Thr His Gly Thr Pro Val Met Leu Ala Ala Gln Lys Gln Gly
                85                  90                  95

Ile Ala Pro Glu Asp Met Ile Ala Lys Val Arg Glu Glu His Leu Ala
            100                 105                 110

Asp Phe Thr Gly Phe Phe Ile Gly Tyr Asp Asn Tyr Tyr Ser Thr His
        115                 120                 125

Ser Pro Glu Asn Lys Gln Phe Ser Gln Asp Ile Tyr Arg Ala Leu Lys
    130                 135                 140

Ala Asn Gly Lys Ile Glu Ser Arg Val Ile Glu Gln Leu Phe Asp Pro
145                 150                 155                 160

Glu Lys Gln Met Phe Leu Pro Asp Arg Phe Val Lys Gly Glu Cys Pro
                165                 170                 175

Lys Cys His Ala Gln Asp Gln Tyr Gly Asp Asn Cys Glu Val Cys Gly
            180                 185                 190

Thr Thr Tyr Ser Pro Thr Glu Leu Ile Asn Pro Tyr Ser Ala Val Ser
        195                 200                 205

Gly Ala Lys Pro Glu Leu Arg Glu Ser Glu His Phe Phe Phe Lys Leu
    210                 215                 220

Gly Glu Cys Ala Asp Phe Leu Lys Ala Trp Thr Ser Gly Asn Asn Pro
225                 230                 235                 240

His Asp Gly Lys Pro His Leu Gln Ala Glu Ala Leu Asn Lys Met
                245                 250                 255

<210> 23
<211> 1539
<212> DNA
<213> Neisseria meningitidis

<220>
<221> CDS
<222> (1)..(1539)

<400> 23
atg aag ttt tta gac cgt gag gca agc att gcc aag ccg ggt ttc aac      48
Met Lys Phe Leu Asp Arg Glu Ala Ser Ile Ala Lys Pro Gly Phe Asn
  1               5                  10                  15

cgt tgg ctc gta ccg ccc gca gca ttg gcg gta cac ctt gcc atc ggg      96
Arg Trp Leu Val Pro Pro Ala Ala Leu Ala Val His Leu Ala Ile Gly
             20                  25                  30

cag att tac gcc tat tcc gta ttc aac gcg ccg ctg acc aaa ctt atc     144
Gln Ile Tyr Ala Tyr Ser Val Phe Asn Ala Pro Leu Thr Lys Leu Ile
         35                  40                  45

ggc ata acc gaa tcg gcg gcc gga gat tgg aag ctg acg acc gta ggt     192
Gly Ile Thr Glu Ser Ala Ala Gly Asp Trp Lys Leu Thr Thr Val Gly
     50                  55                  60

tgg att ttc agt atc gca ctc gcg atg ctg ggc gcg tcg gcc gcc ctg     240
Trp Ile Phe Ser Ile Ala Leu Ala Met Leu Gly Ala Ser Ala Ala Leu
 65                  70                  75                  80

ttc ggt acg tgg atg gaa cgg gta ggg ccg cgc aaa gcc ata ttt gcc     288
Phe Gly Thr Trp Met Glu Arg Val Gly Pro Arg Lys Ala Ile Phe Ala
                 85                  90                  95

gcc gcc tgc tgt ttc agc ctg ggc ttc ttc gta tcc gca ttc ggc gtg     336
Ala Ala Cys Cys Phe Ser Leu Gly Phe Phe Val Ser Ala Phe Gly Val

                100                 105                 110

cgt acg cac aac ctc ttc ctg ctc tat ttg ggc aac ggc gtc atc ggc    384
Arg Thr His Asn Leu Phe Leu Leu Tyr Leu Gly Asn Gly Val Ile Gly
        115                 120                 125

ggc gta ggc ttg ggt ttg ggc tac atc ggg ccg gtt tcc aca ctg atg    432
Gly Val Gly Leu Gly Leu Gly Tyr Ile Gly Pro Val Ser Thr Leu Met
    130                 135                 140

aaa tgg ttt ccc gac aag ccg ggt atg gcg acc ggt ttg gcg att atg    480
Lys Trp Phe Pro Asp Lys Pro Gly Met Ala Thr Gly Leu Ala Ile Met
145                 150                 155                 160

ggt ttc ggt ggc ggc gca atg ctg gcc tcg ccg ctt tcc gta tca ctg    528
Gly Phe Gly Gly Gly Ala Met Leu Ala Ser Pro Leu Ser Val Ser Leu
                165                 170                 175

atg aac acc ttt tca aac gct act tcg gtt ggg gtt gcc gaa acc ttc    576
Met Asn Thr Phe Ser Asn Ala Thr Ser Val Gly Val Ala Glu Thr Phe
            180                 185                 190

gcc gta ttg ggt ctg ctt tac ctc gca ctg atg atg ttc ggc gcg ttt    624
Ala Val Leu Gly Leu Leu Tyr Leu Ala Leu Met Met Phe Gly Ala Phe
        195                 200                 205

acc atc cgc gta cct gcc gac ggc tgg aaa cct gaa ggc tat acc gcg    672
Thr Ile Arg Val Pro Ala Asp Gly Trp Lys Pro Glu Gly Tyr Thr Ala
    210                 215                 220

ccg aaa acg caa aac aag ctg gtc agc agc aat cat gtc aac gtg tcc    720
Pro Lys Thr Gln Asn Lys Leu Val Ser Ser Asn His Val Asn Val Ser
225                 230                 235                 240

caa gcc atg aaa acg ccg cag ttt tgg ctg ttg ttc tgg gta ttg tgc    768
Gln Ala Met Lys Thr Pro Gln Phe Trp Leu Leu Phe Trp Val Leu Cys
                245                 250                 255

ctg aac gta act gcc ggc atc ggc gta ttg ggt cag gca tcc gtg atg    816
Leu Asn Val Thr Ala Gly Ile Gly Val Leu Gly Gln Ala Ser Val Met
            260                 265                 270

att cag gaa ctc ttt tcc gaa acc tcc ata ggc agg cag gcg gca gtc    864
Ile Gln Glu Leu Phe Ser Glu Thr Ser Ile Gly Arg Gln Ala Ala Val
        275                 280                 285

ggc gca ggt gcg gcg gca ggc ttc gtc agc ctc ctg agc ctg ttt aac    912
Gly Ala Gly Ala Ala Ala Gly Phe Val Ser Leu Leu Ser Leu Phe Asn

290                 295                 300

atg ggc gga cgc ttt ttg tgg tcg agc gtt tcc gac aaa atc gga cgc     960
Met Gly Gly Arg Phe Leu Trp Ser Ser Val Ser Asp Lys Ile Gly Arg
305                 310                 315                 320

aaa aac acc tac acc atc ttc ttc gta ctc ggt tcg ctg ctg tat ttc    1008
Lys Asn Thr Tyr Thr Ile Phe Phe Val Leu Gly Ser Leu Leu Tyr Phe
                325                 330                 335

gcc gtt ccc tcc atc ggc gag ggc ggc agc aaa gcc ctg ttc atc atc    1056
Ala Val Pro Ser Ile Gly Glu Gly Gly Ser Lys Ala Leu Phe Ile Ile
            340                 345                 350

ggc ttt tgc gtg att att tcc atg tac ggc ggc gga ttt gcc gcc att    1104
Gly Phe Cys Val Ile Ile Ser Met Tyr Gly Gly Gly Phe Ala Ala Ile
        355                 360                 365

ccc gcc tac ctg aaa gac ctg ttc ggc acc tat cag gtc ggc gcg att    1152
Pro Ala Tyr Leu Lys Asp Leu Phe Gly Thr Tyr Gln Val Gly Ala Ile
    370                 375                 380

cac gga cgc atc ctg ctg gcc tgg tca acc gcc gcc gtc atc ggc ccg    1200
His Gly Arg Ile Leu Leu Ala Trp Ser Thr Ala Ala Val Ile Gly Pro
385                 390                 395                 400

gta ctg gtc aat tac ata cgc caa agc caa atc gac agc ggc ata ccg    1248
Val Leu Val Asn Tyr Ile Arg Gln Ser Gln Ile Asp Ser Gly Ile Pro
                405                 410                 415

gcg gca cag gca tac agc gta acc atg tac atc atg gcg ggt ctg ctg    1296
Ala Ala Gln Ala Tyr Ser Val Thr Met Tyr Ile Met Ala Gly Leu Leu
            420                 425                 430

att atc ggt ttg ttg tgc aac ctc gcc gtc aaa tcc gta cac gaa aaa    1344
Ile Ile Gly Leu Leu Cys Asn Leu Ala Val Lys Ser Val His Glu Lys
        435                 440                 445

cac cac gaa aaa gac atc aaa acc gcc gca cac agc ggc aac ccc gat    1392
His His Glu Lys Asp Ile Lys Thr Ala Ala His Ser Gly Asn Pro Asp
    450                 455                 460

gac gaa acc gcc ata tcc gac gct tac ctt gtg ggc gaa aaa gtt tcc    1440
Asp Glu Thr Ala Ile Ser Asp Ala Tyr Leu Val Gly Glu Lys Val Ser
465                 470                 475                 480

ggc ggc ggc ata tcg gtc tgg tgg cgt tgg gca ttg gcc gtc ata ccg    1488
Gly Gly Gly Ile Ser Val Trp Trp Arg Trp Ala Leu Ala Val Ile Pro

485                 490                 495

ctc gcc tac ggc gtg gtg atg atc ttt gtc aaa gca ctc gac ctt ttc    1536
Leu Ala Tyr Gly Val Val Met Ile Phe Val Lys Ala Leu Asp Leu Phe
            500                 505                 510

tcc                                                                1539
Ser

<210> 24
<211> 513
<212> PRT
<213> Neisseria meningitidis

<400> 24
Met Lys Phe Leu Asp Arg Glu Ala Ser Ile Ala Lys Pro Gly Phe Asn
1               5                   10                  15

Arg Trp Leu Val Pro Pro Ala Ala Leu Ala Val His Leu Ala Ile Gly
            20                  25                  30

Gln Ile Tyr Ala Tyr Ser Val Phe Asn Ala Pro Leu Thr Lys Leu Ile
        35                  40                  45

Gly Ile Thr Glu Ser Ala Ala Gly Asp Trp Lys Leu Thr Thr Val Gly
    50                  55                  60

Trp Ile Phe Ser Ile Ala Leu Ala Met Leu Gly Ala Ser Ala Ala Leu
65                  70                  75                  80

Phe Gly Thr Trp Met Glu Arg Val Gly Pro Arg Lys Ala Ile Phe Ala
                85                  90                  95

Ala Ala Cys Cys Phe Ser Leu Gly Phe Phe Val Ser Ala Phe Gly Val
            100                 105                 110

Arg Thr His Asn Leu Phe Leu Leu Tyr Leu Gly Asn Gly Val Ile Gly
        115                 120                 125

Gly Val Gly Leu Gly Leu Gly Tyr Ile Gly Pro Val Ser Thr Leu Met
    130                 135                 140

Lys Trp Phe Pro Asp Lys Pro Gly Met Ala Thr Gly Leu Ala Ile Met
145                 150                 155                 160

Gly Phe Gly Gly Gly Ala Met Leu Ala Ser Pro Leu Ser Val Ser Leu
                165                 170                 175

Met Asn Thr Phe Ser Asn Ala Thr Ser Val Gly Val Ala Glu Thr Phe
            180                 185                 190

Ala Val Leu Gly Leu Leu Tyr Leu Ala Leu Met Met Phe Gly Ala Phe
        195                 200                 205

Thr Ile Arg Val Pro Ala Asp Gly Trp Lys Pro Glu Gly Tyr Thr Ala
    210                 215                 220

Pro Lys Thr Gln Asn Lys Leu Val Ser Ser Asn His Val Asn Val Ser
225                 230                 235                 240

Gln Ala Met Lys Thr Pro Gln Phe Trp Leu Leu Phe Trp Val Leu Cys
                245                 250                 255

Leu Asn Val Thr Ala Gly Ile Gly Val Leu Gly Gln Ala Ser Val Met
            260                 265                 270

Ile Gln Glu Leu Phe Ser Glu Thr Ser Ile Gly Arg Gln Ala Ala Val
        275                 280                 285

Gly Ala Gly Ala Ala Ala Gly Phe Val Ser Leu Leu Ser Leu Phe Asn
    290                 295                 300

Met Gly Gly Arg Phe Leu Trp Ser Ser Val Ser Asp Lys Ile Gly Arg
305                 310                 315                 320

Lys Asn Thr Tyr Thr Ile Phe Phe Val Leu Gly Ser Leu Leu Tyr Phe
                325                 330                 335

Ala Val Pro Ser Ile Gly Glu Gly Gly Ser Lys Ala Leu Phe Ile Ile
            340                 345                 350

Gly Phe Cys Val Ile Ile Ser Met Tyr Gly Gly Gly Phe Ala Ala Ile
        355                 360                 365

Pro Ala Tyr Leu Lys Asp Leu Phe Gly Thr Tyr Gln Val Gly Ala Ile
    370                 375                 380

His Gly Arg Ile Leu Leu Ala Trp Ser Thr Ala Ala Val Ile Gly Pro
385                 390                 395                 400

Val Leu Val Asn Tyr Ile Arg Gln Ser Gln Ile Asp Ser Gly Ile Pro
                405                 410                 415

Ala Ala Gln Ala Tyr Ser Val Thr Met Tyr Ile Met Ala Gly Leu Leu
            420                 425                 430

Ile Ile Gly Leu Leu Cys Asn Leu Ala Val Lys Ser Val His Glu Lys
        435                 440                 445

His His Glu Lys Asp Ile Lys Thr Ala Ala His Ser Gly Asn Pro Asp
    450                 455                 460

Asp Glu Thr Ala Ile Ser Asp Ala Tyr Leu Val Gly Glu Lys Val Ser
465                 470                 475                 480

Gly Gly Gly Ile Ser Val Trp Trp Arg Trp Ala Leu Ala Val Ile Pro
                485                 490                 495

Leu Ala Tyr Gly Val Val Met Ile Phe Val Lys Ala Leu Asp Leu Phe
            500                 505                 510

Ser

<210> 25
<211> 1038
<212> DNA
<213> Neisseria meningitidis

<220>
<221> CDS
<222> (1)..(1038)

<400> 25
ttg cag cct tta gtc agc gta ttg att tgc gcc tac aac gta gaa aaa      48
Leu Gln Pro Leu Val Ser Val Leu Ile Cys Ala Tyr Asn Val Glu Lys
1               5                   10                  15

tat ttc gcc caa tca tta gcc acc gtc gtg aat cag act tgg cgc aac      96
Tyr Phe Ala Gln Ser Leu Ala Thr Val Val Asn Gln Thr Trp Arg Asn
            20                  25                  30

ttg gag att ttg att gtc gat gac ggc tcg aca gac ggt acg ctt gcc      144
Leu Glu Ile Leu Ile Val Asp Asp Gly Ser Thr Asp Gly Thr Leu Ala
        35                  40                  45

att gcc aag gat ttt caa aag cgg gac agc cgt atc aaa atc ctt gca      192
Ile Ala Lys Asp Phe Gln Lys Arg Asp Ser Arg Ile Lys Ile Leu Ala
    50                  55                  60

caa gct caa aat tcc ggc ctg att ccc tct tta aac atc ggg ctg gac      240

Gln Ala Gln Asn Ser Gly Leu Ile Pro Ser Leu Asn Ile Gly Leu Asp
65                  70                  75                  80

gaa ttg gca aag tca gga atg ggg gaa tat att gca cgc acc gat gcc     288
Glu Leu Ala Lys Ser Gly Met Gly Glu Tyr Ile Ala Arg Thr Asp Ala
                85                  90                  95

gac gat att gcc gcc ccc gac tgg att gag aaa atc gtg ggc gag atg     336
Asp Asp Ile Ala Ala Pro Asp Trp Ile Glu Lys Ile Val Gly Glu Met
            100                 105                 110

gaa aaa gac cgc agc atc atc gcg atg ggc gcg tgg ctg gaa gtt ttg     384
Glu Lys Asp Arg Ser Ile Ile Ala Met Gly Ala Trp Leu Glu Val Leu
        115                 120                 125

tcg gaa gaa aag gac ggc aac cgg ctg gcg cgg cat cac agg cac ggc     432
Ser Glu Glu Lys Asp Gly Asn Arg Leu Ala Arg His His Arg His Gly
    130                 135                 140

aaa att tgg aaa aag ccg acc cgg cac gaa gat att gcc gac ttt ttc     480
Lys Ile Trp Lys Lys Pro Thr Arg His Glu Asp Ile Ala Asp Phe Phe
145                 150                 155                 160

cct ttc ggc aac ccc ata cac aac aac acg atg att atg cgg cgc agc     528
Pro Phe Gly Asn Pro Ile His Asn Asn Thr Met Ile Met Arg Arg Ser
                165                 170                 175

gtc att gac ggc gga ttg cgt tac aac acc gag cgg gat tgg gcg gaa     576
Val Ile Asp Gly Gly Leu Arg Tyr Asn Thr Glu Arg Asp Trp Ala Glu
            180                 185                 190

gat tac caa ttt tgg tac gat gtc agc aaa ttg ggc agg ctg gct tat     624
Asp Tyr Gln Phe Trp Tyr Asp Val Ser Lys Leu Gly Arg Leu Ala Tyr
        195                 200                 205

tat ccc gaa gcc ttg gtc aaa tac cgc ctt cac gcc aat cag gtt tca     672
Tyr Pro Glu Ala Leu Val Lys Tyr Arg Leu His Ala Asn Gln Val Ser
    210                 215                 220

tcc aaa tac agc atc cgc caa cac gaa atc gcg caa ggc atc caa aaa     720
Ser Lys Tyr Ser Ile Arg Gln His Glu Ile Ala Gln Gly Ile Gln Lys
225                 230                 235                 240

acc gcc aga aac gat ttt ttg cag tct atg ggt ttt aaa acc cgg ttc     768
Thr Ala Arg Asn Asp Phe Leu Gln Ser Met Gly Phe Lys Thr Arg Phe
                245                 250                 255

gac agc ctt gaa tac cgc caa ata aaa gca gta gcg tat gaa ttg ctg     816

Asp Ser Leu Glu Tyr Arg Gln Ile Lys Ala Val Ala Tyr Glu Leu Leu
            260                 265                 270

gag aaa cat ttg ccg gaa gaa gat ttt gaa cgc gcc cgc cgg ttt ttg 864
Glu Lys His Leu Pro Glu Glu Asp Phe Glu Arg Ala Arg Arg Phe Leu
        275                 280                 285

tac caa tgc ttc aaa cgg acg gac acg ccg ccc gcc ggc gcg tgg ctg 912
Tyr Gln Cys Phe Lys Arg Thr Asp Thr Pro Pro Ala Gly Ala Trp Leu
    290                 295                 300

gat ttc gcg gca gac ggc aag atg agg cgg ctg ttt acc atg agg caa 960
Asp Phe Ala Ala Asp Gly Lys Met Arg Arg Leu Phe Thr Met Arg Gln
305                 310                 315                 320

tac ttc ggc att ttg cac cgg ctg att aaa aac cgc cgg cag gcg cgg 1008
Tyr Phe Gly Ile Leu His Arg Leu Ile Lys Asn Arg Arg Gln Ala Arg
                325                 330                 335

tcg gat tcg gca ggg aaa gaa cag gag att 1038
Ser Asp Ser Ala Gly Lys Glu Gln Glu Ile
            340                 345

<210> 26
<211> 346
<212> PRT
<213> Neisseria meningitidis

<400> 26
Leu Gln Pro Leu Val Ser Val Leu Ile Cys Ala Tyr Asn Val Glu Lys
1               5                   10                  15

Tyr Phe Ala Gln Ser Leu Ala Thr Val Val Asn Gln Thr Trp Arg Asn
            20                  25                  30

Leu Glu Ile Leu Ile Val Asp Asp Gly Ser Thr Asp Gly Thr Leu Ala
        35                  40                  45

Ile Ala Lys Asp Phe Gln Lys Arg Asp Ser Arg Ile Lys Ile Leu Ala
    50                  55                  60

Gln Ala Gln Asn Ser Gly Leu Ile Pro Ser Leu Asn Ile Gly Leu Asp
65                  70                  75                  80

Glu Leu Ala Lys Ser Gly Met Gly Glu Tyr Ile Ala Arg Thr Asp Ala
                85                  90                  95

Asp Asp Ile Ala Ala Pro Asp Trp Ile Glu Lys Ile Val Gly Glu Met
            100                 105                 110

Glu Lys Asp Arg Ser Ile Ile Ala Met Gly Ala Trp Leu Glu Val Leu
        115                 120                 125

Ser Glu Glu Lys Asp Gly Asn Arg Leu Ala Arg His His Arg His Gly
    130                 135                 140

Lys Ile Trp Lys Lys Pro Thr Arg His Glu Asp Ile Ala Asp Phe Phe
145                 150                 155                 160

Pro Phe Gly Asn Pro Ile His Asn Asn Thr Met Ile Met Arg Arg Ser
                165                 170                 175

Val Ile Asp Gly Gly Leu Arg Tyr Asn Thr Glu Arg Asp Trp Ala Glu
            180                 185                 190

Asp Tyr Gln Phe Trp Tyr Asp Val Ser Lys Leu Gly Arg Leu Ala Tyr
        195                 200                 205

Tyr Pro Glu Ala Leu Val Lys Tyr Arg Leu His Ala Asn Gln Val Ser
    210                 215                 220

Ser Lys Tyr Ser Ile Arg Gln His Glu Ile Ala Gln Gly Ile Gln Lys
225                 230                 235                 240

Thr Ala Arg Asn Asp Phe Leu Gln Ser Met Gly Phe Lys Thr Arg Phe
                245                 250                 255

Asp Ser Leu Glu Tyr Arg Gln Ile Lys Ala Val Ala Tyr Glu Leu Leu
            260                 265                 270

Glu Lys His Leu Pro Glu Glu Asp Phe Glu Arg Ala Arg Arg Phe Leu
        275                 280                 285

Tyr Gln Cys Phe Lys Arg Thr Asp Thr Pro Pro Ala Gly Ala Trp Leu
    290                 295                 300

Asp Phe Ala Ala Asp Gly Lys Met Arg Arg Leu Phe Thr Met Arg Gln
305                 310                 315                 320

Tyr Phe Gly Ile Leu His Arg Leu Ile Lys Asn Arg Arg Gln Ala Arg
                325                 330                 335

Ser Asp Ser Ala Gly Lys Glu Gln Glu Ile
            340                 345

<210> 27
<211> 1856
<212> DNA
<213> Neisseria meningitidis

<220>
<221> CDS
<222> (1)..(1857)

<400> 27
atg cca gaa tac cgc tcc aaa acc tcc acc cac ggt cgc aat atg gcg     48
Met Pro Glu Tyr Arg Ser Lys Thr Ser Thr His Gly Arg Asn Met Ala
1               5                   10                  15

ggc gcg cgc gcg ttg tgg cgc gcc acc ggc gtg atg gaa acc gac ttc     96
Gly Ala Arg Ala Leu Trp Arg Ala Thr Gly Val Met Glu Thr Asp Phe
            20                  25                  30

ggc aaa ccg att atc gcc gtt gcc aac tcg ttc acc caa ttc gtg cca     144
Gly Lys Pro Ile Ile Ala Val Ala Asn Ser Phe Thr Gln Phe Val Pro
        35                  40                  45

ggt cat gtc cac ctg cac aat atg ggc cag ctg gtt gcc cgc gaa atc     192
Gly His Val His Leu His Asn Met Gly Gln Leu Val Ala Arg Glu Ile
    50                  55                  60

gaa aaa gcc ggc gcc atc gcc aaa gaa ttc aac acc atc gcc atc gac     240
Glu Lys Ala Gly Ala Ile Ala Lys Glu Phe Asn Thr Ile Ala Ile Asp
65                  70                  75                  80

gac ggc atc gct atg gga cac agc ggt atg ctg tat tcc ctg ccc agc     288
Asp Gly Ile Ala Met Gly His Ser Gly Met Leu Tyr Ser Leu Pro Ser
                85                  90                  95

cgc gat ttg att gcc gac tcc atc gaa tat atg gtc aat gcc cac tgc     336
Arg Asp Leu Ile Ala Asp Ser Ile Glu Tyr Met Val Asn Ala His Cys
            100                 105                 110

gcc gac gcg ctg gtg tgc att tcc aac tgc gac aaa atc acc ccg ggc     384
Ala Asp Ala Leu Val Cys Ile Ser Asn Cys Asp Lys Ile Thr Pro Gly
        115                 120                 125

atg ctg att gcc gcg atg cgc ctg aac atc ccg acc atc ttc gtt tcc     432
Met Leu Ile Ala Ala Met Arg Leu Asn Ile Pro Thr Ile Phe Val Ser
    130                 135                 140

ggc ggt ccg atg gaa gca ggt aaa gtt atc ggc gtg gca aac atc cag 480
Gly Gly Pro Met Glu Ala Gly Lys Val Ile Gly Val Ala Asn Ile Gln
145                 150                 155                 160

ccc gaa cgc cgt ttg gac ttg att gac gcg atg att gaa tcg gcg gac 528
Pro Glu Arg Arg Leu Asp Leu Ile Asp Ala Met Ile Glu Ser Ala Asp
                165                 170                 175

gac aat atc agc aac cgg caa gtt gaa gaa gtc gaa caa aac gcc tgc 576
Asp Asn Ile Ser Asn Arg Gln Val Glu Glu Val Glu Gln Asn Ala Cys
            180                 185                 190

ccg acc tgc ggc tcg tgt tcg ggt atg ttt acg gca aac tcg atg aac 624
Pro Thr Cys Gly Ser Cys Ser Gly Met Phe Thr Ala Asn Ser Met Asn
        195                 200                 205

tgc ctg acc gaa gca ctc ggg ctg tcc ctg ccg ggc aac ggt tct tac 672
Cys Leu Thr Glu Ala Leu Gly Leu Ser Leu Pro Gly Asn Gly Ser Tyr
    210                 215                 220

ctc gcc acc cac gtc ggc cgc aaa gaa ctg ttc ctc gaa gcc ggc cgt 720
Leu Ala Thr His Val Gly Arg Lys Glu Leu Phe Leu Glu Ala Gly Arg
225                 230                 235                 240

atg att gtc gaa atc acc aaa cgc tat tac gag caa aac gat gaa acc 768
Met Ile Val Glu Ile Thr Lys Arg Tyr Tyr Glu Gln Asn Asp Glu Thr
                245                 250                 255

gtg tta ccg cgc agc att gcc acc aaa aaa gcg ttt gaa aac gct atg 816
Val Leu Pro Arg Ser Ile Ala Thr Lys Lys Ala Phe Glu Asn Ala Met
            260                 265                 270

acc atg gac att gcc atg ggc ggc agc acc aac act att ttg cat tta 864
Thr Met Asp Ile Ala Met Gly Gly Ser Thr Asn Thr Ile Leu His Leu
        275                 280                 285

ctc gcc gtt gcc aac gaa gcg ggc gtg gat ttc aaa atg gca gac atc 912
Leu Ala Val Ala Asn Glu Ala Gly Val Asp Phe Lys Met Ala Asp Ile
    290                 295                 300

gac cgc tta agc cgc gtt gtg ccc tgc atc tgc aaa acc gca ccc aac 960
Asp Arg Leu Ser Arg Val Val Pro Cys Ile Cys Lys Thr Ala Pro Asn
305                 310                 315                 320

aac cac gac tac tat atg gaa gac gtg cac cga gcc ggc ggc atc ttc 1008
Asn His Asp Tyr Tyr Met Glu Asp Val His Arg Ala Gly Gly Ile Phe
                325                 330                 335

gcc atc ctg aaa gaa ttg gac aaa gcg ggc aag ctg cac acc gac gtg 1056
Ala Ile Leu Lys Glu Leu Asp Lys Ala Gly Lys Leu His Thr Asp Val
            340                 345                 350

cac act atc cac gcg ccg acg ctg aaa gac gcg att gaa aaa tgg gac 1104
His Thr Ile His Ala Pro Thr Leu Lys Asp Ala Ile Glu Lys Trp Asp
        355                 360                 365

gta acc aac cct gaa aac acc cac gcc atc gaa cgc ttc aaa gcc gcg 1152
Val Thr Asn Pro Glu Asn Thr His Ala Ile Glu Arg Phe Lys Ala Ala
    370                 375                 380

ccc ggc ggc gta cgc acc acc caa gcg ttc tcg caa aac cgt atg tgg 1200
Pro Gly Gly Val Arg Thr Thr Gln Ala Phe Ser Gln Asn Arg Met Trp
385                 390                 395                 400

aaa acc ctc gac ctc gac cgc gaa aaa ggc tgt atc cgc gac gtg gca 1248
Lys Thr Leu Asp Leu Asp Arg Glu Lys Gly Cys Ile Arg Asp Val Ala
                405                 410                 415

cac gcc tac tcg caa gac ggc ggt ttg gcg gtc ttg ttc ggc aac atc 1296
His Ala Tyr Ser Gln Asp Gly Gly Leu Ala Val Leu Phe Gly Asn Ile
            420                 425                 430

gcc gag cgc ggc tgc gtg gta aaa acc gca ggc gtg gac gag agc atc 1344
Ala Glu Arg Gly Cys Val Val Lys Thr Ala Gly Val Asp Glu Ser Ile
        435                 440                 445

ctc aaa ttc acc ggc cgc gcc cgc gtg ttt gaa agc caa gaa gac gca 1392
Leu Lys Phe Thr Gly Arg Ala Arg Val Phe Glu Ser Gln Glu Asp Ala
    450                 455                 460

gta gaa ggc att ttg ggc aac caa atc gtc gcc ggc gac atc gtc atc 1440
Val Glu Gly Ile Leu Gly Asn Gln Ile Val Ala Gly Asp Ile Val Ile
465                 470                 475                 480

atc cgc tac gaa ggc ccg aaa ggc ggc ccg ggc atg cag gaa atg ctg 1488
Ile Arg Tyr Glu Gly Pro Lys Gly Gly Pro Gly Met Gln Glu Met Leu
                485                 490                 495

tat ccg act tcc tac ctg aaa tct aaa ggc ctc ggc aaa gcc tgc gct 1536
Tyr Pro Thr Ser Tyr Leu Lys Ser Lys Gly Leu Gly Lys Ala Cys Ala
            500                 505                 510

ctc tta acc gac gga cgc ttc tcc ggc ggc aca tca ggt ttg tcc atc 1584
Leu Leu Thr Asp Gly Arg Phe Ser Gly Gly Thr Ser Gly Leu Ser Ile
        515                 520                 525

gga cac gcc tcg ccc gaa gcg gcg gaa ggt ggc gcg atc ggt ttg gta     1632
Gly His Ala Ser Pro Glu Ala Ala Glu Gly Gly Ala Ile Gly Leu Val
    530                 535                 540

cac gaa ggc gat acc gtc gaa atc gac atc ccc aac cgc agc atc cgc     1680
His Glu Gly Asp Thr Val Glu Ile Asp Ile Pro Asn Arg Ser Ile Arg
545                 550                 555                 560

ctt gtc att tcc gac gaa gaa ctc gcc gcc cgc cgt gcc gaa atg gaa     1728
Leu Val Ile Ser Asp Glu Glu Leu Ala Ala Arg Arg Ala Glu Met Glu
                565                 570                 575

gca cgc ggc agc aaa gcg tgg aaa ccg gaa aac cgc gac cgc tac gtc     1776
Ala Arg Gly Ser Lys Ala Trp Lys Pro Glu Asn Arg Asp Arg Tyr Val
            580                 585                 590

tcc gcc gca tta aga gct tac ggc gcg atg gcg act tcc gcc gac aaa     1824
Ser Ala Ala Leu Arg Ala Tyr Gly Ala Met Ala Thr Ser Ala Asp Lys
        595                 600                 605

ggc gcg gtg cgc gac gta gcg caa atc gaa aga                         1857
Gly Ala Val Arg Asp Val Ala Gln Ile Glu Arg
    610                 615

<210> 28
<211> 619
<212> PRT
<213> Neisseria meningitidis

<400> 28
Met Pro Glu Tyr Arg Ser Lys Thr Ser Thr His Gly Arg Asn Met Ala
1               5                   10                  15

Gly Ala Arg Ala Leu Trp Arg Ala Thr Gly Val Met Glu Thr Asp Phe
            20                  25                  30

Gly Lys Pro Ile Ile Ala Val Ala Asn Ser Phe Thr Gln Phe Val Pro
        35                  40                  45

Gly His Val His Leu His Asn Met Gly Gln Leu Val Ala Arg Glu Ile
    50                  55                  60

Glu Lys Ala Gly Ala Ile Ala Lys Glu Phe Asn Thr Ile Ala Ile Asp
65                  70                  75                  80

Asp Gly Ile Ala Met Gly His Ser Gly Met Leu Tyr Ser Leu Pro Ser
                85                  90                  95

Arg Asp Leu Ile Ala Asp Ser Ile Glu Tyr Met Val Asn Ala His Cys
            100                 105                 110

Ala Asp Ala Leu Val Cys Ile Ser Asn Cys Asp Lys Ile Thr Pro Gly
        115                 120                 125

Met Leu Ile Ala Ala Met Arg Leu Asn Ile Pro Thr Ile Phe Val Ser
    130                 135                 140

Gly Gly Pro Met Glu Ala Gly Lys Val Ile Gly Val Ala Asn Ile Gln
145                 150                 155                 160

Pro Glu Arg Arg Leu Asp Leu Ile Asp Ala Met Ile Glu Ser Ala Asp
                165                 170                 175

Asp Asn Ile Ser Asn Arg Gln Val Glu Glu Val Glu Gln Asn Ala Cys
            180                 185                 190

Pro Thr Cys Gly Ser Cys Ser Gly Met Phe Thr Ala Asn Ser Met Asn
        195                 200                 205

Cys Leu Thr Glu Ala Leu Gly Leu Ser Leu Pro Gly Asn Gly Ser Tyr
    210                 215                 220

Leu Ala Thr His Val Gly Arg Lys Glu Leu Phe Leu Glu Ala Gly Arg
225                 230                 235                 240

Met Ile Val Glu Ile Thr Lys Arg Tyr Tyr Glu Gln Asn Asp Glu Thr
                245                 250                 255

Val Leu Pro Arg Ser Ile Ala Thr Lys Lys Ala Phe Glu Asn Ala Met
            260                 265                 270

Thr Met Asp Ile Ala Met Gly Gly Ser Thr Asn Thr Ile Leu His Leu
        275                 280                 285

Leu Ala Val Ala Asn Glu Ala Gly Val Asp Phe Lys Met Ala Asp Ile
    290                 295                 300

Asp Arg Leu Ser Arg Val Val Pro Cys Ile Cys Lys Thr Ala Pro Asn
305                 310                 315                 320

Asn His Asp Tyr Tyr Met Glu Asp Val His Arg Ala Gly Gly Ile Phe
                325                 330                 335

Ala Ile Leu Lys Glu Leu Asp Lys Ala Gly Lys Leu His Thr Asp Val
            340                 345                 350

His Thr Ile His Ala Pro Thr Leu Lys Asp Ala Ile Glu Lys Trp Asp
        355                 360                 365

Val Thr Asn Pro Glu Asn Thr His Ala Ile Glu Arg Phe Lys Ala Ala
    370                 375                 380

Pro Gly Gly Val Arg Thr Thr Gln Ala Phe Ser Gln Asn Arg Met Trp
385                 390                 395                 400

Lys Thr Leu Asp Leu Asp Arg Glu Lys Gly Cys Ile Arg Asp Val Ala
                405                 410                 415

His Ala Tyr Ser Gln Asp Gly Gly Leu Ala Val Leu Phe Gly Asn Ile
            420                 425                 430

Ala Glu Arg Gly Cys Val Val Lys Thr Ala Gly Val Asp Glu Ser Ile
        435                 440                 445

Leu Lys Phe Thr Gly Arg Ala Arg Val Phe Glu Ser Gln Glu Asp Ala
    450                 455                 460

Val Glu Gly Ile Leu Gly Asn Gln Ile Val Ala Gly Asp Ile Val Ile
465                 470                 475                 480

Ile Arg Tyr Glu Gly Pro Lys Gly Gly Pro Gly Met Gln Glu Met Leu
                485                 490                 495

Tyr Pro Thr Ser Tyr Leu Lys Ser Lys Gly Leu Gly Lys Ala Cys Ala
            500                 505                 510

Leu Leu Thr Asp Gly Arg Phe Ser Gly Gly Thr Ser Gly Leu Ser Ile
        515                 520                 525

Gly His Ala Ser Pro Glu Ala Ala Glu Gly Gly Ala Ile Gly Leu Val
    530                 535                 540

His Glu Gly Asp Thr Val Glu Ile Asp Ile Pro Asn Arg Ser Ile Arg
545                 550                 555                 560

Leu Val Ile Ser Asp Glu Glu Leu Ala Ala Arg Arg Ala Glu Met Glu
                565                 570                 575

Ala Arg Gly Ser Lys Ala Trp Lys Pro Glu Asn Arg Asp Arg Tyr Val
            580                 585                 590

Ser Ala Ala Leu Arg Ala Tyr Gly Ala Met Ala Thr Ser Ala Asp Lys
        595                 600                 605

Gly Ala Val Arg Asp Val Ala Gln Ile Glu Arg
610                 615

<210> 29
<211> 1725
<212> DNA
<213> Neisseria meningitidis

<220>
<221> CDS
<222> (1)..(1725)

<400> 29
atg cag cta tca ggc gcg caa atc ata gtg cag agt ctc aaa gcc gaa     48
Met Gln Leu Ser Gly Ala Gln Ile Ile Val Gln Ser Leu Lys Ala Glu
1               5                   10                  15

ggt gtc gag tac gtt ttc ggt tat ccc ggc ggt gcg gtt atc gaa atc     96
Gly Val Glu Tyr Val Phe Gly Tyr Pro Gly Gly Ala Val Ile Glu Ile
            20                  25                  30

tac gat gcc ctt ttc caa ctc aat aaa ttc aag cac att ctg acc cgt     144
Tyr Asp Ala Leu Phe Gln Leu Asn Lys Phe Lys His Ile Leu Thr Arg
        35                  40                  45

cac gag cag gcg gca gta cac gcg gca gat gcg tat gcg cgc gtc agc     192
His Glu Gln Ala Ala Val His Ala Ala Asp Ala Tyr Ala Arg Val Ser
    50                  55                  60

ggt aag gtg ggc gtg gca ttg gtt aca tcc ggc ccg ggc gtt acc aat     240
Gly Lys Val Gly Val Ala Leu Val Thr Ser Gly Pro Gly Val Thr Asn
65                  70                  75                  80

gca ctg acc ggt att gct act gcc tat acg gat tcg att ccg atg gtg     288
Ala Leu Thr Gly Ile Ala Thr Ala Tyr Thr Asp Ser Ile Pro Met Val
                85                  90                  95

gtc atc agc ggg cag gta ggc aat tcc ctg att ggt acg gat gcg ttc     336
Val Ile Ser Gly Gln Val Gly Asn Ser Leu Ile Gly Thr Asp Ala Phe
            100                 105                 110

caa gaa gtt gat acg gtg ggt att acc cgt ccg tgc gtc aaa cac aat     384
Gln Glu Val Asp Thr Val Gly Ile Thr Arg Pro Cys Val Lys His Asn
        115                 120                 125

ttc ctg gtt acg gac atc aat gag ttg gcg gaa acc att aaa aag gcg 432
Phe Leu Val Thr Asp Ile Asn Glu Leu Ala Glu Thr Ile Lys Lys Ala
    130                 135                 140

ttc caa att gcc gca agc ggc cga ccg ggg ccc gtg gtg gtt gat gtc 480
Phe Gln Ile Ala Ala Ser Gly Arg Pro Gly Pro Val Val Val Asp Val
145                 150                 155                 160

ccg aaa gat gtt acg cag gcg atg gcg aaa ttc agc tat cct cag gaa 528
Pro Lys Asp Val Thr Gln Ala Met Ala Lys Phe Ser Tyr Pro Gln Glu
                165                 170                 175

gac att ttt atc cgt tcg tat caa ccg gtt gtt caa ggg cat atc ggt 576
Asp Ile Phe Ile Arg Ser Tyr Gln Pro Val Val Gln Gly His Ile Gly
            180                 185                 190

cag att aaa aag gcc gtg cag atg ttg gca tct gcc aaa cgt ccg gtc 624
Gln Ile Lys Lys Ala Val Gln Met Leu Ala Ser Ala Lys Arg Pro Val
        195                 200                 205

gtc tat ttt ggc ggc ggc gtg gtg ttg ggt aat gct tct gaa gag ctg 672
Val Tyr Phe Gly Gly Gly Val Val Leu Gly Asn Ala Ser Glu Glu Leu
    210                 215                 220

acc cga ttt gtc cga atg acg ggt gct ccg tgc acg ggt acg ctg atg 720
Thr Arg Phe Val Arg Met Thr Gly Ala Pro Cys Thr Gly Thr Leu Met
225                 230                 235                 240

ggc ttg ggc gct tat cct tcc ggc gac cgc caa ttc ctc ggt atg ctc 768
Gly Leu Gly Ala Tyr Pro Ser Gly Asp Arg Gln Phe Leu Gly Met Leu
                245                 250                 255

ggt atg cac ggt act tac gag gca aac ctt gcc atg cag aat gcg gat 816
Gly Met His Gly Thr Tyr Glu Ala Asn Leu Ala Met Gln Asn Ala Asp
            260                 265                 270

gtc gtt ctt gcc gta ggt gcg cgt ttt gac gac cgt gtg gtt tcc gta 864
Val Val Leu Ala Val Gly Ala Arg Phe Asp Asp Arg Val Val Ser Val
        275                 280                 285

ccg tcc aaa ttt ttc gag aag gcg aag aag gtt atc cat atc gat gtc 912
Pro Ser Lys Phe Phe Glu Lys Ala Lys Lys Val Ile His Ile Asp Val
    290                 295                 300

gat cct tcc agc atc gcc aaa cgc gtg aag gcg gac att ccg att gtc 960
Asp Pro Ser Ser Ile Ala Lys Arg Val Lys Ala Asp Ile Pro Ile Val
305                 310                 315                 320

ggc gac gtg aaa aac gtt ttg tcc gag atg gtt gcg ctg tgg caa aaa 1008
Gly Asp Val Lys Asn Val Leu Ser Glu Met Val Ala Leu Trp Gln Lys
                325                 330                 335

caa gag tcc gtg ccg tct gaa gat gct ttg ggc aaa tgg tgg aaa acc 1056
Gln Glu Ser Val Pro Ser Glu Asp Ala Leu Gly Lys Trp Trp Lys Thr
            340                 345                 350

ata gag gaa tgg cgt tcc cgc gat tgc ttg tgg ttt gac aac ggc agc 1104
Ile Glu Glu Trp Arg Ser Arg Asp Cys Leu Trp Phe Asp Asn Gly Ser
        355                 360                 365

gaa att atc aag cca caa tat gtg att cag aag ctt gcc gag att acc 1152
Glu Ile Ile Lys Pro Gln Tyr Val Ile Gln Lys Leu Ala Glu Ile Thr
    370                 375                 380

ggc aat tcg gca atc atc aca tcg gat gta ggg cag cat caa atg ttt 1200
Gly Asn Ser Ala Ile Ile Thr Ser Asp Val Gly Gln His Gln Met Phe
385                 390                 395                 400

gcg gct caa tat tat ccc ttc gaa cgt ccg cgc caa tgg ctc aac tcc 1248
Ala Ala Gln Tyr Tyr Pro Phe Glu Arg Pro Arg Gln Trp Leu Asn Ser
                405                 410                 415

ggc ggt ttg ggt acg atg ggc gtt ggt ctg cct tat gcg att ggt gca 1296
Gly Gly Leu Gly Thr Met Gly Val Gly Leu Pro Tyr Ala Ile Gly Ala
            420                 425                 430

aaa ctt gcc gcc ccg gat caa gac gta ttc tgt att acc ggc gac ggc 1344
Lys Leu Ala Ala Pro Asp Gln Asp Val Phe Cys Ile Thr Gly Asp Gly
        435                 440                 445

tcg atc cag atg aac atc caa gag ctg tcc acc tgt ttc caa tat cgg 1392
Ser Ile Gln Met Asn Ile Gln Glu Leu Ser Thr Cys Phe Gln Tyr Arg
    450                 455                 460

att ccg gtt aac gtc att acg ctg aac aac ggt tat ctc ggt atg gta 1440
Ile Pro Val Asn Val Ile Thr Leu Asn Asn Gly Tyr Leu Gly Met Val
465                 470                 475                 480

cgc cag tgg cag gaa ata tat tac ggc gga cga gag tcg gaa acc tat 1488
Arg Gln Trp Gln Glu Ile Tyr Tyr Gly Gly Arg Glu Ser Glu Thr Tyr
                485                 490                 495

ttc gat tct ttg ccc gat ttc gtc aaa ctt gcc gag gca tac ggc cat 1536
Phe Asp Ser Leu Pro Asp Phe Val Lys Leu Ala Glu Ala Tyr Gly His
            500                 505                 510

atc ggt atc cgc gtg gac aag aag tct gat gtg gaa ggt gcg ttg ttg     1584
Ile Gly Ile Arg Val Asp Lys Lys Ser Asp Val Glu Gly Ala Leu Leu
        515                 520                 525

gaa gca ttg aac caa aaa gac agg ctg gtg ttt atc gac ttc ctg acc     1632
Glu Ala Leu Asn Gln Lys Asp Arg Leu Val Phe Ile Asp Phe Leu Thr
    530                 535                 540

gac cag aaa cag aat gtg atg ccc atg gtc ggc aac ggc aaa ggt ttg     1680
Asp Gln Lys Gln Asn Val Met Pro Met Val Gly Asn Gly Lys Gly Leu
545                 550                 555                 560

gac gaa atg gta ctt ccg ccg cat atg cgt gcg gac gga aag gcg         1725
Asp Glu Met Val Leu Pro Pro His Met Arg Ala Asp Gly Lys Ala
                565                 570                 575

<210> 30
<211> 575
<212> PRT
<213> Neisseria meningitidis

<400> 30
Met Gln Leu Ser Gly Ala Gln Ile Ile Val Gln Ser Leu Lys Ala Glu
1               5                   10                  15

Gly Val Glu Tyr Val Phe Gly Tyr Pro Gly Gly Ala Val Ile Glu Ile
            20                  25                  30

Tyr Asp Ala Leu Phe Gln Leu Asn Lys Phe Lys His Ile Leu Thr Arg
        35                  40                  45

His Glu Gln Ala Ala Val His Ala Ala Asp Ala Tyr Ala Arg Val Ser
    50                  55                  60

Gly Lys Val Gly Val Ala Leu Val Thr Ser Gly Pro Gly Val Thr Asn
65                  70                  75                  80

Ala Leu Thr Gly Ile Ala Thr Ala Tyr Thr Asp Ser Ile Pro Met Val
                85                  90                  95

Val Ile Ser Gly Gln Val Gly Asn Ser Leu Ile Gly Thr Asp Ala Phe
            100                 105                 110

Gln Glu Val Asp Thr Val Gly Ile Thr Arg Pro Cys Val Lys His Asn
        115                 120                 125

Phe Leu Val Thr Asp Ile Asn Glu Leu Ala Glu Thr Ile Lys Lys Ala

130                 135                 140

Phe Gln Ile Ala Ala Ser Gly Arg Pro Gly Pro Val Val Val Asp Val
145                 150                 155                 160

Pro Lys Asp Val Thr Gln Ala Met Ala Lys Phe Ser Tyr Pro Gln Glu
                165                 170                 175

Asp Ile Phe Ile Arg Ser Tyr Gln Pro Val Val Gln Gly His Ile Gly
            180                 185                 190

Gln Ile Lys Lys Ala Val Gln Met Leu Ala Ser Ala Lys Arg Pro Val
        195                 200                 205

Val Tyr Phe Gly Gly Gly Val Val Leu Gly Asn Ala Ser Glu Glu Leu
    210                 215                 220

Thr Arg Phe Val Arg Met Thr Gly Ala Pro Cys Thr Gly Thr Leu Met
225                 230                 235                 240

Gly Leu Gly Ala Tyr Pro Ser Gly Asp Arg Gln Phe Leu Gly Met Leu
                245                 250                 255

Gly Met His Gly Thr Tyr Glu Ala Asn Leu Ala Met Gln Asn Ala Asp
            260                 265                 270

Val Val Leu Ala Val Gly Ala Arg Phe Asp Asp Arg Val Val Ser Val
        275                 280                 285

Pro Ser Lys Phe Phe Glu Lys Ala Lys Lys Val Ile His Ile Asp Val
    290                 295                 300

Asp Pro Ser Ser Ile Ala Lys Arg Val Lys Ala Asp Ile Pro Ile Val
305                 310                 315                 320

Gly Asp Val Lys Asn Val Leu Ser Glu Met Val Ala Leu Trp Gln Lys
                325                 330                 335

Gln Glu Ser Val Pro Ser Glu Asp Ala Leu Gly Lys Trp Trp Lys Thr
            340                 345                 350

Ile Glu Glu Trp Arg Ser Arg Asp Cys Leu Trp Phe Asp Asn Gly Ser
        355                 360                 365

Glu Ile Ile Lys Pro Gln Tyr Val Ile Gln Lys Leu Ala Glu Ile Thr
    370                 375                 380

Gly Asn Ser Ala Ile Ile Thr Ser Asp Val Gly Gln His Gln Met Phe

385                 390                 395                 400

Ala Ala Gln Tyr Tyr Pro Phe Glu Arg Pro Arg Gln Trp Leu Asn Ser
                405                 410                 415

Gly Gly Leu Gly Thr Met Gly Val Gly Leu Pro Tyr Ala Ile Gly Ala
            420                 425                 430

Lys Leu Ala Ala Pro Asp Gln Asp Val Phe Cys Ile Thr Gly Asp Gly
        435                 440                 445

Ser Ile Gln Met Asn Ile Gln Glu Leu Ser Thr Cys Phe Gln Tyr Arg
    450                 455                 460

Ile Pro Val Asn Val Ile Thr Leu Asn Asn Gly Tyr Leu Gly Met Val
465                 470                 475                 480

Arg Gln Trp Gln Glu Ile Tyr Tyr Gly Gly Arg Glu Ser Glu Thr Tyr
                485                 490                 495

Phe Asp Ser Leu Pro Asp Phe Val Lys Leu Ala Glu Ala Tyr Gly His
            500                 505                 510

Ile Gly Ile Arg Val Asp Lys Lys Ser Asp Val Glu Gly Ala Leu Leu
        515                 520                 525

Glu Ala Leu Asn Gln Lys Asp Arg Leu Val Phe Ile Asp Phe Leu Thr
    530                 535                 540

Asp Gln Lys Gln Asn Val Met Pro Met Val Gly Asn Gly Lys Gly Leu
545                 550                 555                 560

Asp Glu Met Val Leu Pro Pro His Met Arg Ala Asp Gly Lys Ala
                565                 570                 575

<210> 31
<211> 651
<212> DNA
<213> Neisseria meningitidis

<220>
<221> CDS
<222> (1)..(651)

<400> 31
atg ttt tcc gta ccg cgt tcc ttt ttg ccg ggc gtt ttc gta ctt gcc      48

Met Phe Ser Val Pro Arg Ser Phe Leu Pro Gly Val Phe Val Leu Ala
1               5                   10                  15

gcg ctt gcc gcc tgc aaa cct caa gac aac agt gcg gcg caa gtc gct    96
Ala Leu Ala Ala Cys Lys Pro Gln Asp Asn Ser Ala Ala Gln Val Ala
            20                  25                  30

tct tca agt gca tcc gcg tcg gct gcg gaa aat gcg gca aag ccg caa    144
Ser Ser Ser Ala Ser Ala Ser Ala Ala Glu Asn Ala Ala Lys Pro Gln
        35                  40                  45

acg cgc ggt acg gat atg cgt aag gaa gac atc ggc ggc gat ttc acg    192
Thr Arg Gly Thr Asp Met Arg Lys Glu Asp Ile Gly Gly Asp Phe Thr
    50                  55                  60

ctg acc gac ggc gaa ggc aag cct ttc aac ctg agc gat ttg aaa ggc    240
Leu Thr Asp Gly Glu Gly Lys Pro Phe Asn Leu Ser Asp Leu Lys Gly
65                  70                  75                  80

aag gtc gtg att ctg tct ttc ggc ttt acg cac tgt ccc gat gtc tgc    288
Lys Val Val Ile Leu Ser Phe Gly Phe Thr His Cys Pro Asp Val Cys
                85                  90                  95

ccg aca gag ctt ttg acg tac agc gac acg ttg aag cag ttg ggc ggg    336
Pro Thr Glu Leu Leu Thr Tyr Ser Asp Thr Leu Lys Gln Leu Gly Gly
            100                 105                 110

cag gct aag gac gtg aaa gtg gtg ttc gtc agc atc gat ccg gaa cgc    384
Gln Ala Lys Asp Val Lys Val Val Phe Val Ser Ile Asp Pro Glu Arg
        115                 120                 125

gac acg cct gaa atc atc ggc aag tat gcc aaa cag ttc aat ccg gac    432
Asp Thr Pro Glu Ile Ile Gly Lys Tyr Ala Lys Gln Phe Asn Pro Asp
    130                 135                 140

ttt atc ggt ctg acg gca acg ggc gac caa aac ctg ccg gtc atc aag    480
Phe Ile Gly Leu Thr Ala Thr Gly Asp Gln Asn Leu Pro Val Ile Lys
145                 150                 155                 160

cag caa tac cgc gtg gtt tct gcc aaa gtc aat caa aaa gac gac agc    528
Gln Gln Tyr Arg Val Val Ser Ala Lys Val Asn Gln Lys Asp Asp Ser
                165                 170                 175

gaa aac tat ttg gtc gac cac tct tcc ggt gcg tat ctc atc gac aaa    576
Glu Asn Tyr Leu Val Asp His Ser Ser Gly Ala Tyr Leu Ile Asp Lys
            180                 185                 190

aac ggt gag gtt gcc att ttc tcg cct tac gga agc gag ccg gaa acg    624

Asn Gly Glu Val Ala Ile Phe Ser Pro Tyr Gly Ser Glu Pro Glu Thr
        195                 200                 205

att gct gcc gat gta agg acc ctg ctc                                651
Ile Ala Ala Asp Val Arg Thr Leu Leu
    210                 215

<210> 32
<211> 217
<212> PRT
<213> Neisseria meningitidis

<400> 32
Met Phe Ser Val Pro Arg Ser Phe Leu Pro Gly Val Phe Val Leu Ala
 1               5                  10                  15

Ala Leu Ala Ala Cys Lys Pro Gln Asp Asn Ser Ala Ala Gln Val Ala
            20                  25                  30

Ser Ser Ser Ala Ser Ala Ser Ala Ala Glu Asn Ala Ala Lys Pro Gln
        35                  40                  45

Thr Arg Gly Thr Asp Met Arg Lys Glu Asp Ile Gly Gly Asp Phe Thr
    50                  55                  60

Leu Thr Asp Gly Glu Gly Lys Pro Phe Asn Leu Ser Asp Leu Lys Gly
65                  70                  75                  80

Lys Val Val Ile Leu Ser Phe Gly Phe Thr His Cys Pro Asp Val Cys
                85                  90                  95

Pro Thr Glu Leu Leu Thr Tyr Ser Asp Thr Leu Lys Gln Leu Gly Gly
            100                 105                 110

Gln Ala Lys Asp Val Lys Val Val Phe Val Ser Ile Asp Pro Glu Arg
        115                 120                 125

Asp Thr Pro Glu Ile Ile Gly Lys Tyr Ala Lys Gln Phe Asn Pro Asp
    130                 135                 140

Phe Ile Gly Leu Thr Ala Thr Gly Asp Gln Asn Leu Pro Val Ile Lys
145                 150                 155                 160

Gln Gln Tyr Arg Val Val Ser Ala Lys Val Asn Gln Lys Asp Asp Ser
                165                 170                 175

Glu Asn Tyr Leu Val Asp His Ser Ser Gly Ala Tyr Leu Ile Asp Lys

180                 185                 190

Asn Gly Glu Val Ala Ile Phe Ser Pro Tyr Gly Ser Glu Pro Glu Thr
        195                 200                 205

Ile Ala Ala Asp Val Arg Thr Leu Leu
    210                 215

<210> 33
<211> 330
<212> DNA
<213> Neisseria meningitidis

<220>
<221> CDS
<222> (1)..(330)

<400> 33
ttg ccg ctg ctt tgt tgt ctt tgg ttt tgg cag cct gcg gcg gtg aaa      48
Leu Pro Leu Leu Cys Cys Leu Trp Phe Trp Gln Pro Ala Ala Val Lys
  1               5                  10                  15

aag ccg ctg aag ctc ccg ctg ctg aag cac ctg ccg ccg aag ctc ccg      96
Lys Pro Leu Lys Leu Pro Leu Leu Lys His Leu Pro Pro Lys Leu Pro
             20                  25                  30

cta ctg aag cac ctg ccg ccg aag ctc ccg ctg ctg aag cac ctg ccg     144
Leu Leu Lys His Leu Pro Pro Lys Leu Pro Leu Leu Lys His Leu Pro
         35                  40                  45

ccg aag ctc ctg cta ctg aag cac ctg ccg ccg aag ctc ccg ctg ctg     192
Pro Lys Leu Leu Leu Leu Lys His Leu Pro Pro Lys Leu Pro Leu Leu
     50                  55                  60

aag ctg ccg cta ccg aag cac ctg ccg ctg aag ctg ccg cta ccg aag     240
Lys Leu Pro Leu Pro Lys His Leu Pro Leu Lys Leu Pro Leu Pro Lys
 65                  70                  75                  80

cac ctg ccg ctg aag ctc ctg ctg ccg aag ctg caa aat aag cat ttt     288
His Leu Pro Leu Lys Leu Leu Leu Pro Lys Leu Gln Asn Lys His Phe
                 85                  90                  95

ccg ctt gca aaa aag cag gat acg ttc agt atc ctg ctt ttt             330
Pro Leu Ala Lys Lys Gln Asp Thr Phe Ser Ile Leu Leu Phe
            100                 105                 110

<210> 34
<211> 110
<212> PRT
<213> Neisseria meningitidis

<400> 34
Leu Pro Leu Leu Cys Cys Leu Trp Phe Trp Gln Pro Ala Ala Val Lys
1               5                   10                  15

Lys Pro Leu Lys Leu Pro Leu Leu Lys His Leu Pro Pro Lys Leu Pro
            20                  25                  30

Leu Leu Lys His Leu Pro Pro Lys Leu Pro Leu Leu Lys His Leu Pro
        35                  40                  45

Pro Lys Leu Leu Leu Leu Lys His Leu Pro Pro Lys Leu Pro Leu Leu
    50                  55                  60

Lys Leu Pro Leu Pro Lys His Leu Pro Leu Lys Leu Pro Leu Pro Lys
65                  70                  75                  80

His Leu Pro Leu Lys Leu Leu Leu Pro Lys Leu Gln Asn Lys His Phe
                85                  90                  95

Pro Leu Ala Lys Lys Gln Asp Thr Phe Ser Ile Leu Leu Phe
            100                 105                 110

<210> 35
<211> 2118
<212> DNA
<213> Neisseria meningitidis

<220>
<221> CDS
<222> (1)..(2118)

<400> 35
atg ttc gac aaa cac gtt aag acc ttc caa tac ggt aat cag acc gtt     48
Met Phe Asp Lys His Val Lys Thr Phe Gln Tyr Gly Asn Gln Thr Val
1               5                   10                  15

act ttg gaa acc ggc gaa att gcc cgc caa gcc gcc gct gcc gtt aaa     96
Thr Leu Glu Thr Gly Glu Ile Ala Arg Gln Ala Ala Ala Ala Val Lys
            20                  25                  30

gtc tct atg ggc gac acc gtg gtt ttg gtt gcc gtt acc acc aac aaa 144
Val Ser Met Gly Asp Thr Val Val Leu Val Ala Val Thr Thr Asn Lys
        35                  40                  45

gaa gtg aaa gaa ggc caa gac ttc ttc ccc ctg acc gtc gat tat ttg 192
Glu Val Lys Glu Gly Gln Asp Phe Phe Pro Leu Thr Val Asp Tyr Leu
    50                  55                  60

gaa cgc act tac gcc gca ggt aaa atc ccc ggc ggt ttc ttc aaa cgc 240
Glu Arg Thr Tyr Ala Ala Gly Lys Ile Pro Gly Gly Phe Phe Lys Arg
65                  70                  75                  80

gaa ggc aaa caa agc gaa aaa gaa atc ctg acc agc cgt ctg atc gac 288
Glu Gly Lys Gln Ser Glu Lys Glu Ile Leu Thr Ser Arg Leu Ile Asp
                85                  90                  95

cgc ccg att cgt ccg ctg ttc ccc gaa ggt ttc tac cac gac atc caa 336
Arg Pro Ile Arg Pro Leu Phe Pro Glu Gly Phe Tyr His Asp Ile Gln
            100                 105                 110

atc gta gcg atg gtc gtg tcc gtc gat ccc gaa atc gat tcc gac att 384
Ile Val Ala Met Val Val Ser Val Asp Pro Glu Ile Asp Ser Asp Ile
        115                 120                 125

cct gca atg ttg ggc gcg tct gcc gcg ctg gtg ttg agc ggc gta ccg 432
Pro Ala Met Leu Gly Ala Ser Ala Ala Leu Val Leu Ser Gly Val Pro
    130                 135                 140

ttt gcc ggt ccg att ggt gcg gca cgc gtg ggt tat ata aac ggc gtg 480
Phe Ala Gly Pro Ile Gly Ala Ala Arg Val Gly Tyr Ile Asn Gly Val
145                 150                 155                 160

tac gtt ttg aac ccg act aaa gcc gaa ttg gcg aaa tcg caa ttg gac 528
Tyr Val Leu Asn Pro Thr Lys Ala Glu Leu Ala Lys Ser Gln Leu Asp
                165                 170                 175

ttg gtg gtc gcc ggt act tca aaa gcc gtg ttg atg gtg gaa tcc gaa 576
Leu Val Val Ala Gly Thr Ser Lys Ala Val Leu Met Val Glu Ser Glu
            180                 185                 190

gcc aaa atc ctg ccc gaa gac gtg atg ctg ggc gcg gtg gtt tac ggc 624
Ala Lys Ile Leu Pro Glu Asp Val Met Leu Gly Ala Val Val Tyr Gly
        195                 200                 205

cac gat caa atg cag gtt gcc atc aat gca atc aat gaa ttt gcc gac 672
His Asp Gln Met Gln Val Ala Ile Asn Ala Ile Asn Glu Phe Ala Asp
    210                 215                 220

gaa gtc aat ccg gaa ctt tgg gat tgg aaa gca cct gaa acc aat gag 720
Glu Val Asn Pro Glu Leu Trp Asp Trp Lys Ala Pro Glu Thr Asn Glu
225                 230                 235                 240

gaa ctg gtt gcc aaa gtc cgc ggg att gcc ggc gaa acc att aaa gaa 768
Glu Leu Val Ala Lys Val Arg Gly Ile Ala Gly Glu Thr Ile Lys Glu
                245                 250                 255

gcg ttc aaa atc cgt caa aaa caa gcg cgt tct gcc aaa ttg gac gaa 816
Ala Phe Lys Ile Arg Gln Lys Gln Ala Arg Ser Ala Lys Leu Asp Glu
            260                 265                 270

gct tgg agt gcg gta aaa gaa gcc ttg att acc gaa gaa acc gac acg 864
Ala Trp Ser Ala Val Lys Glu Ala Leu Ile Thr Glu Glu Thr Asp Thr
        275                 280                 285

ctg gca gcc aac gaa atc aaa ggc att ttc aaa cac ttg gaa gcc gat 912
Leu Ala Ala Asn Glu Ile Lys Gly Ile Phe Lys His Leu Glu Ala Asp
    290                 295                 300

gtc gtc cgc agc caa att ttg gac ggc caa ccg cgc atc gac ggc cgc 960
Val Val Arg Ser Gln Ile Leu Asp Gly Gln Pro Arg Ile Asp Gly Arg
305                 310                 315                 320

gac acc cgc acc gtc cgt ccg ctg aac atc caa acc ggc gta ttg ccg 1008
Asp Thr Arg Thr Val Arg Pro Leu Asn Ile Gln Thr Gly Val Leu Pro
                325                 330                 335

cgc acg cac ggt tcc gca ttg ttt acc cgt ggc gaa acc caa gcc ctt 1056
Arg Thr His Gly Ser Ala Leu Phe Thr Arg Gly Glu Thr Gln Ala Leu
            340                 345                 350

gcc gta gcg act ttg ggt act tcg cgc gac gag caa atc atc gac gcg 1104
Ala Val Ala Thr Leu Gly Thr Ser Arg Asp Glu Gln Ile Ile Asp Ala
        355                 360                 365

ctg tcc ggc gaa tac acc gac cgc ttt atg ctg cac tac aac ttt ccg 1152
Leu Ser Gly Glu Tyr Thr Asp Arg Phe Met Leu His Tyr Asn Phe Pro
    370                 375                 380

ccg tac tct acc ggc gaa gtg ggc cgc atg ggc gcg ccg aaa cgc cgt 1200
Pro Tyr Ser Thr Gly Glu Val Gly Arg Met Gly Ala Pro Lys Arg Arg
385                 390                 395                 400

gaa atc ggt cac ggt cgt ttg gct aaa cgt gca ttg ttg gcc gta ttg 1248
Glu Ile Gly His Gly Arg Leu Ala Lys Arg Ala Leu Leu Ala Val Leu
                405                 410                 415

ccg aaa cct gaa gat ttc agc tac acc atg cgc gtg gtc tcc gaa att 1296
Pro Lys Pro Glu Asp Phe Ser Tyr Thr Met Arg Val Val Ser Glu Ile
            420                 425                 430

acc gaa tcc aac ggc tct tcc tct atg gct tcc gtc tgc ggc ggc tgc 1344
Thr Glu Ser Asn Gly Ser Ser Ser Met Ala Ser Val Cys Gly Gly Cys
        435                 440                 445

ttg agc ctg ctg tcc gcc ggc gtg cct ttg aaa gca cac gtt gcc ggt 1392
Leu Ser Leu Leu Ser Ala Gly Val Pro Leu Lys Ala His Val Ala Gly
    450                 455                 460

atc gcg atg ggt ctg att ctg gaa ggc aat aag ttt gcc gtc ctg acc 1440
Ile Ala Met Gly Leu Ile Leu Glu Gly Asn Lys Phe Ala Val Leu Thr
465                 470                 475                 480

gac att ttg ggc gac gaa gac cac ttg ggc gat atg gac ttt aaa gtg 1488
Asp Ile Leu Gly Asp Glu Asp His Leu Gly Asp Met Asp Phe Lys Val
                485                 490                 495

gct ggt acg acc gaa ggc gtt acc gcg ctg caa atg gac atc aaa atc 1536
Ala Gly Thr Thr Glu Gly Val Thr Ala Leu Gln Met Asp Ile Lys Ile
            500                 505                 510

caa ggc att acc aaa gag att atg caa atc gct ttg gcg cag gcc aaa 1584
Gln Gly Ile Thr Lys Glu Ile Met Gln Ile Ala Leu Ala Gln Ala Lys
        515                 520                 525

gaa gcg cgt ctg cac atc ttg gat cag atg aaa gcc gcc gtt gcg ggc 1632
Glu Ala Arg Leu His Ile Leu Asp Gln Met Lys Ala Ala Val Ala Gly
    530                 535                 540

ccg caa gag ctg tcc gca cac gcg cca cgc ttg ttc acg atg aaa atc 1680
Pro Gln Glu Leu Ser Ala His Ala Pro Arg Leu Phe Thr Met Lys Ile
545                 550                 555                 560

aac caa gac aaa atc cgc gaa gtt atc ggt aag ggc ggt gaa acc atc 1728
Asn Gln Asp Lys Ile Arg Glu Val Ile Gly Lys Gly Gly Glu Thr Ile
                565                 570                 575

cgt gcg att acc gct gaa acc ggt acg gaa atc aat att gcc gaa gac 1776
Arg Ala Ile Thr Ala Glu Thr Gly Thr Glu Ile Asn Ile Ala Glu Asp
            580                 585                 590

ggt acg att acc att gcc gca acc act caa gaa gcc ggc gat gcg gcg 1824
Gly Thr Ile Thr Ile Ala Ala Thr Thr Gln Glu Ala Gly Asp Ala Ala
        595                 600                 605

aaa aaa cgc atc gag cag att act gcc gaa gtg gaa gtg ggc aaa gtg    1872
Lys Lys Arg Ile Glu Gln Ile Thr Ala Glu Val Glu Val Gly Lys Val
    610                 615                 620

tac gaa ggc act gtg gtg aaa atc ctc gac aac aat gtc ggc gcg att    1920
Tyr Glu Gly Thr Val Val Lys Ile Leu Asp Asn Asn Val Gly Ala Ile
625                 630                 635                 640

gtc agt gtg atg ccg ggc aaa gac ggt ttg gta cac atc agc caa atc    1968
Val Ser Val Met Pro Gly Lys Asp Gly Leu Val His Ile Ser Gln Ile
                645                 650                 655

gcc cac gag cgc gta cgc aat gtc ggc gac tac ctg caa gtc ggt cag    2016
Ala His Glu Arg Val Arg Asn Val Gly Asp Tyr Leu Gln Val Gly Gln
            660                 665                 670

gtg gtg aac gtg aaa gca ttg gaa gtg gac gac aga ggc cgt gtc cgt    2064
Val Val Asn Val Lys Ala Leu Glu Val Asp Asp Arg Gly Arg Val Arg
        675                 680                 685

ctg tcc atc aaa gcc ctg ctg gac gcg cct gcc cgt gag gaa aat gcc    2112
Leu Ser Ile Lys Ala Leu Leu Asp Ala Pro Ala Arg Glu Glu Asn Ala
    690                 695                 700

gcc gag                                                            2118
Ala Glu
705

<210> 36
<211> 706
<212> PRT
<213> Neisseria meningitidis

<400> 36
Met Phe Asp Lys His Val Lys Thr Phe Gln Tyr Gly Asn Gln Thr Val
1               5                   10                  15

Thr Leu Glu Thr Gly Glu Ile Ala Arg Gln Ala Ala Ala Ala Val Lys
            20                  25                  30

Val Ser Met Gly Asp Thr Val Val Leu Val Ala Val Thr Thr Asn Lys
        35                  40                  45

Glu Val Lys Glu Gly Gln Asp Phe Phe Pro Leu Thr Val Asp Tyr Leu
    50                  55                  60

Glu Arg Thr Tyr Ala Ala Gly Lys Ile Pro Gly Gly Phe Phe Lys Arg

65                  70                  75                  80

Glu Gly Lys Gln Ser Glu Lys Glu Ile Leu Thr Ser Arg Leu Ile Asp
                85                  90                  95

Arg Pro Ile Arg Pro Leu Phe Pro Glu Gly Phe Tyr His Asp Ile Gln
            100                 105                 110

Ile Val Ala Met Val Val Ser Val Asp Pro Glu Ile Asp Ser Asp Ile
        115                 120                 125

Pro Ala Met Leu Gly Ala Ser Ala Ala Leu Val Leu Ser Gly Val Pro
    130                 135                 140

Phe Ala Gly Pro Ile Gly Ala Ala Arg Val Gly Tyr Ile Asn Gly Val
145                 150                 155                 160

Tyr Val Leu Asn Pro Thr Lys Ala Glu Leu Ala Lys Ser Gln Leu Asp
                165                 170                 175

Leu Val Val Ala Gly Thr Ser Lys Ala Val Leu Met Val Glu Ser Glu
            180                 185                 190

Ala Lys Ile Leu Pro Glu Asp Val Met Leu Gly Ala Val Val Tyr Gly
        195                 200                 205

His Asp Gln Met Gln Val Ala Ile Asn Ala Ile Asn Glu Phe Ala Asp
    210                 215                 220

Glu Val Asn Pro Glu Leu Trp Asp Trp Lys Ala Pro Glu Thr Asn Glu
225                 230                 235                 240

Glu Leu Val Ala Lys Val Arg Gly Ile Ala Gly Glu Thr Ile Lys Glu
                245                 250                 255

Ala Phe Lys Ile Arg Gln Lys Gln Ala Arg Ser Ala Lys Leu Asp Glu
            260                 265                 270

Ala Trp Ser Ala Val Lys Glu Ala Leu Ile Thr Glu Glu Thr Asp Thr
        275                 280                 285

Leu Ala Ala Asn Glu Ile Lys Gly Ile Phe Lys His Leu Glu Ala Asp
    290                 295                 300

Val Val Arg Ser Gln Ile Leu Asp Gly Gln Pro Arg Ile Asp Gly Arg
305                 310                 315                 320

Asp Thr Arg Thr Val Arg Pro Leu Asn Ile Gln Thr Gly Val Leu Pro

                325                 330                 335

Arg Thr His Gly Ser Ala Leu Phe Thr Arg Gly Glu Thr Gln Ala Leu
            340                 345                 350

Ala Val Ala Thr Leu Gly Thr Ser Arg Asp Glu Gln Ile Ile Asp Ala
        355                 360                 365

Leu Ser Gly Glu Tyr Thr Asp Arg Phe Met Leu His Tyr Asn Phe Pro
    370                 375                 380

Pro Tyr Ser Thr Gly Glu Val Gly Arg Met Gly Ala Pro Lys Arg Arg
385                 390                 395                 400

Glu Ile Gly His Gly Arg Leu Ala Lys Arg Ala Leu Leu Ala Val Leu
                405                 410                 415

Pro Lys Pro Glu Asp Phe Ser Tyr Thr Met Arg Val Val Ser Glu Ile
            420                 425                 430

Thr Glu Ser Asn Gly Ser Ser Ser Met Ala Ser Val Cys Gly Gly Cys
        435                 440                 445

Leu Ser Leu Leu Ser Ala Gly Val Pro Leu Lys Ala His Val Ala Gly
    450                 455                 460

Ile Ala Met Gly Leu Ile Leu Glu Gly Asn Lys Phe Ala Val Leu Thr
465                 470                 475                 480

Asp Ile Leu Gly Asp Glu Asp His Leu Gly Asp Met Asp Phe Lys Val
                485                 490                 495

Ala Gly Thr Thr Glu Gly Val Thr Ala Leu Gln Met Asp Ile Lys Ile
            500                 505                 510

Gln Gly Ile Thr Lys Glu Ile Met Gln Ile Ala Leu Ala Gln Ala Lys
        515                 520                 525

Glu Ala Arg Leu His Ile Leu Asp Gln Met Lys Ala Ala Val Ala Gly
    530                 535                 540

Pro Gln Glu Leu Ser Ala His Ala Pro Arg Leu Phe Thr Met Lys Ile
545                 550                 555                 560

Asn Gln Asp Lys Ile Arg Glu Val Ile Gly Lys Gly Gly Glu Thr Ile
                565                 570                 575

Arg Ala Ile Thr Ala Glu Thr Gly Thr Glu Ile Asn Ile Ala Glu Asp

580                 585                 590

Gly Thr Ile Thr Ile Ala Ala Thr Thr Gln Glu Ala Gly Asp Ala Ala
        595                 600                 605

Lys Lys Arg Ile Glu Gln Ile Thr Ala Glu Val Glu Val Gly Lys Val
    610                 615                 620

Tyr Glu Gly Thr Val Val Lys Ile Leu Asp Asn Asn Val Gly Ala Ile
625                 630                 635                 640

Val Ser Val Met Pro Gly Lys Asp Gly Leu Val His Ile Ser Gln Ile
                645                 650                 655

Ala His Glu Arg Val Arg Asn Val Gly Asp Tyr Leu Gln Val Gly Gln
            660                 665                 670

Val Val Asn Val Lys Ala Leu Glu Val Asp Asp Arg Gly Arg Val Arg
        675                 680                 685

Leu Ser Ile Lys Ala Leu Leu Asp Ala Pro Ala Arg Glu Glu Asn Ala
    690                 695                 700

Ala Glu
705

<210> 37
<211> 3972
<212> DNA
<213> Neisseria meningitidis

<220>
<221> CDS
<222> (1)..(3972)

<400> 37
gtg agc cga att atg tct gtc gtt ttg ccc ttg cgc ggc gtt acc gcc      48
Val Ser Arg Ile Met Ser Val Val Leu Pro Leu Arg Gly Val Thr Ala
1               5                   10                  15

ctt tcc gat ttc cgt gtt gaa aaa ctc ttg caa aaa gcc gcc gca ctc      96
Leu Ser Asp Phe Arg Val Glu Lys Leu Leu Gln Lys Ala Ala Ala Leu
            20                  25                  30

ggt ctg ccc gaa gtc aaa tta agc agc gaa ttt tgg tat ttc gtc ggc      144
Gly Leu Pro Glu Val Lys Leu Ser Ser Glu Phe Trp Tyr Phe Val Gly

35                  40                  45

agc gag aaa gca ctt gat gcc gcg act gtc gaa aaa ctg caa gcc ttg     192
Ser Glu Lys Ala Leu Asp Ala Ala Thr Val Glu Lys Leu Gln Ala Leu
    50                  55                  60

ttg gcg gcg caa agc gtt gaa caa acg cca aaa gcg cgc gag ggc ttg     240
Leu Ala Ala Gln Ser Val Glu Gln Thr Pro Lys Ala Arg Glu Gly Leu
65                  70                  75                  80

cat ttg ttt ttg gtc acg ccc cgt ttg ggt acg att tcg ccg tgg gct     288
His Leu Phe Leu Val Thr Pro Arg Leu Gly Thr Ile Ser Pro Trp Ala
                85                  90                  95

tcc aag gcg acc aat atc gcg gaa aac tgc ggt ttg gca ggc atc gaa     336
Ser Lys Ala Thr Asn Ile Ala Glu Asn Cys Gly Leu Ala Gly Ile Glu
            100                 105                 110

cgc atc gag cgc ggc atg gcg gtg tgg ctg gaa ggt gcg ctt acc gat     384
Arg Ile Glu Arg Gly Met Ala Val Trp Leu Glu Gly Ala Leu Thr Asp
        115                 120                 125

gaa cag caa cag caa tgg gcg gct ttg ctg cac gac cgc atg act gaa     432
Glu Gln Gln Gln Gln Trp Ala Ala Leu Leu His Asp Arg Met Thr Glu
    130                 135                 140

tcc gtg ctg ccc gat ttt cag acg gca tcc aaa tta ttc cac cat ctc     480
Ser Val Leu Pro Asp Phe Gln Thr Ala Ser Lys Leu Phe His His Leu
145                 150                 155                 160

gaa tcc gaa acc ttt tcc acc gtc gat gtt ttg ggc ggc ggt aaa gag     528
Glu Ser Glu Thr Phe Ser Thr Val Asp Val Leu Gly Gly Gly Lys Glu
                165                 170                 175

gct ttg gtc aaa gcc aat acc gaa atg ggt ttg gca ctc tcc gcc gac     576
Ala Leu Val Lys Ala Asn Thr Glu Met Gly Leu Ala Leu Ser Ala Asp
            180                 185                 190

gaa atc gat tat ctg gtt gaa aac tat cag gct ttg cag cgc aat ccg     624
Glu Ile Asp Tyr Leu Val Glu Asn Tyr Gln Ala Leu Gln Arg Asn Pro
        195                 200                 205

tct gat gtg gag ctg atg atg ttc gcg cag gca aac agc gaa cac tgc     672
Ser Asp Val Glu Leu Met Met Phe Ala Gln Ala Asn Ser Glu His Cys
    210                 215                 220

cgc cac aaa atc ttc aac gcc gac ttc atc ctc aac ggc gaa aaa cag     720
Arg His Lys Ile Phe Asn Ala Asp Phe Ile Leu Asn Gly Glu Lys Gln

225                 230                 235                 240

ccg aaa tcg ctc ttc ggt atg ata cgc gac acg cac aac gcg cat ccc     768
Pro Lys Ser Leu Phe Gly Met Ile Arg Asp Thr His Asn Ala His Pro
                245                 250                 255

gaa ggc aca gtc gtt gcc tac aaa gac aac tca tcc gtg atc gaa ggc     816
Glu Gly Thr Val Val Ala Tyr Lys Asp Asn Ser Ser Val Ile Glu Gly
            260                 265                 270

gca aaa gtc gaa cgt ttc tat cca aat gcg gca gaa aac caa ggc tac     864
Ala Lys Val Glu Arg Phe Tyr Pro Asn Ala Ala Glu Asn Gln Gly Tyr
        275                 280                 285

cgt ttc cac gag gaa gac acg cat atc atc atg aaa gtg gaa acg cac     912
Arg Phe His Glu Glu Asp Thr His Ile Ile Met Lys Val Glu Thr His
    290                 295                 300

aac cac ccg acc gcc atc gcg ccg ttt gcg ggc gcg gca acg ggc gcg     960
Asn His Pro Thr Ala Ile Ala Pro Phe Ala Gly Ala Ala Thr Gly Ala
305                 310                 315                 320

ggc ggc gaa atc cgc gac gaa ggt gcg acg ggc aaa ggt tcg cgt ccg     1008
Gly Gly Glu Ile Arg Asp Glu Gly Ala Thr Gly Lys Gly Ser Arg Pro
                325                 330                 335

aaa gca ggc ttg acc ggc ttt acc gtg tcc aac ctc aac atc ccc ggc     1056
Lys Ala Gly Leu Thr Gly Phe Thr Val Ser Asn Leu Asn Ile Pro Gly
            340                 345                 350

ctc aaa cag ccg tgg gaa caa gat tac ggc aag ccg gaa cat att tcc     1104
Leu Lys Gln Pro Trp Glu Gln Asp Tyr Gly Lys Pro Glu His Ile Ser
        355                 360                 365

tcg ccg ctg gac att atg att gaa ggc ccc atc ggc ggc gcg gcg ttc     1152
Ser Pro Leu Asp Ile Met Ile Glu Gly Pro Ile Gly Gly Ala Ala Phe
    370                 375                 380

aac aac gaa ttc ggc cgc ccc aac ctc ttg ggc tac ttc cgc act ttt     1200
Asn Asn Glu Phe Gly Arg Pro Asn Leu Leu Gly Tyr Phe Arg Thr Phe
385                 390                 395                 400

gaa gaa aaa ttt gac ggt cag gtt cgc ggc tac cac aaa ccg att atg     1248
Glu Glu Lys Phe Asp Gly Gln Val Arg Gly Tyr His Lys Pro Ile Met
                405                 410                 415

att gcc ggc ggc ttg ggc agc att cag gcg cag cag acg cat aaa gac     1296
Ile Ala Gly Gly Leu Gly Ser Ile Gln Ala Gln Gln Thr His Lys Asp

            420                 425                 430

gaa atc ccc gaa ggc gcg ttg ctg atc caa ctg ggc ggc ccg ggt atg 1344
Glu Ile Pro Glu Gly Ala Leu Leu Ile Gln Leu Gly Gly Pro Gly Met
        435                 440                 445

ctt atc ggc ttg ggc ggc ggc gcg gct tct tcg atg gat acc ggc aca 1392
Leu Ile Gly Leu Gly Gly Gly Ala Ala Ser Ser Met Asp Thr Gly Thr
    450                 455                 460

aac gac gcg tct tta gac ttc aac tcc gta caa cgc ggc aac ccc gaa 1440
Asn Asp Ala Ser Leu Asp Phe Asn Ser Val Gln Arg Gly Asn Pro Glu
465                 470                 475                 480

atc gaa cgc cgc gcg cag gaa gtg atc gac cgc tgc tgg cag ctc ggc 1488
Ile Glu Arg Arg Ala Gln Glu Val Ile Asp Arg Cys Trp Gln Leu Gly
                485                 490                 495

gac aaa aac ccg att atc tcc atc cac gac gtt ggc gcg ggc ggc ttg 1536
Asp Lys Asn Pro Ile Ile Ser Ile His Asp Val Gly Ala Gly Gly Leu
            500                 505                 510

tcc aac gcc ttc ccc gaa ctc gtc aac gat gcc gga cgc ggc gcg gta 1584
Ser Asn Ala Phe Pro Glu Leu Val Asn Asp Ala Gly Arg Gly Ala Val
        515                 520                 525

ttc aag ctg cgc gaa gtg ccg ctg gaa gaa cac ggt ctc aac ccg ctg 1632
Phe Lys Leu Arg Glu Val Pro Leu Glu Glu His Gly Leu Asn Pro Leu
    530                 535                 540

caa atc tgg tgc aac gaa tcg caa gag cgt tat gta ttg tcg att ttg 1680
Gln Ile Trp Cys Asn Glu Ser Gln Glu Arg Tyr Val Leu Ser Ile Leu
545                 550                 555                 560

gaa aaa gat ttg gac atc ttc cgc tcc atc tgc gaa cgt gaa cgc tgc 1728
Glu Lys Asp Leu Asp Ile Phe Arg Ser Ile Cys Glu Arg Glu Arg Cys
                565                 570                 575

ccg ttt gcg gta gtc ggt acg gcg acc gac gac ggc cat ttg aaa gta 1776
Pro Phe Ala Val Val Gly Thr Ala Thr Asp Asp Gly His Leu Lys Val
            580                 585                 590

cgc gac gat ttg ttc tcc aac aac cct gtc gat ttg ccg ttg aac gtc 1824
Arg Asp Asp Leu Phe Ser Asn Asn Pro Val Asp Leu Pro Leu Asn Val
        595                 600                 605

ttg ctc ggc aaa ccg ccc aaa acc acg cgt acc gac aaa acg gtt gcg 1872
Leu Leu Gly Lys Pro Pro Lys Thr Thr Arg Thr Asp Lys Thr Val Ala

610                 615                 620

ccg tcc aaa aaa ccg ttt cac gcg ggc gat atc gac att act gaa gcc     1920
Pro Ser Lys Lys Pro Phe His Ala Gly Asp Ile Asp Ile Thr Glu Ala
625                 630                 635                 640

gcc tac cgc gtt ctg cgc ctg cct gcc gta gcc gcc aaa aac ttc ctg     1968
Ala Tyr Arg Val Leu Arg Leu Pro Ala Val Ala Ala Lys Asn Phe Leu
                645                 650                 655

att acc atc ggc gac cgc agc gtc ggc ggc atg acc cac cgc gac caa     2016
Ile Thr Ile Gly Asp Arg Ser Val Gly Gly Met Thr His Arg Asp Gln
            660                 665                 670

atg gtc ggc aaa tac caa acc ccc gta gcc gac tgc gcc gtt acc atg     2064
Met Val Gly Lys Tyr Gln Thr Pro Val Ala Asp Cys Ala Val Thr Met
        675                 680                 685

atg ggc ttc aac act tat cgc ggc gaa gcg atg agc atg ggc gaa aaa     2112
Met Gly Phe Asn Thr Tyr Arg Gly Glu Ala Met Ser Met Gly Glu Lys
    690                 695                 700

ccg acc gtc gcc ctg ttt gac gcg cct gct tcg ggc aga atg tgc gtc     2160
Pro Thr Val Ala Leu Phe Asp Ala Pro Ala Ser Gly Arg Met Cys Val
705                 710                 715                 720

ggc gaa gcc atc acc aac atc gcg gca gcc aat atc ggc gac atc ggc     2208
Gly Glu Ala Ile Thr Asn Ile Ala Ala Ala Asn Ile Gly Asp Ile Gly
                725                 730                 735

aac atc aaa ctc tcc gcc aac tgg atg gcg gcg tgc ggc aac gaa ggc     2256
Asn Ile Lys Leu Ser Ala Asn Trp Met Ala Ala Cys Gly Asn Glu Gly
            740                 745                 750

gaa gac gaa aaa ctc tac cgc acc gtc gaa gcg gtt tcc aaa gcc tgt     2304
Glu Asp Glu Lys Leu Tyr Arg Thr Val Glu Ala Val Ser Lys Ala Cys
        755                 760                 765

cag gca ttg gat ttg agc att ccc gtg ggc aaa gac agc ctg tcg atg     2352
Gln Ala Leu Asp Leu Ser Ile Pro Val Gly Lys Asp Ser Leu Ser Met
    770                 775                 780

aaa acc gtg tgg cag gac ggc gaa gag aaa aaa tcc gtc gtt tcg ccg     2400
Lys Thr Val Trp Gln Asp Gly Glu Glu Lys Lys Ser Val Val Ser Pro
785                 790                 795                 800

ttg agc ctg att atc tcc gcg ttc gcg ccg gtt caa gac gta cgc aaa     2448
Leu Ser Leu Ile Ile Ser Ala Phe Ala Pro Val Gln Asp Val Arg Lys

                805                 810                 815

acc gtt acg ccc gaa ttg aaa aac gtc gag gac agc gta ttg ctg ttt 2496
Thr Val Thr Pro Glu Leu Lys Asn Val Glu Asp Ser Val Leu Leu Phe
            820                 825                 830

gtc gat ttg ggc ttc ggc aaa gcg cgt atg ggc ggc tcg gcg ttt ggt 2544
Val Asp Leu Gly Phe Gly Lys Ala Arg Met Gly Gly Ser Ala Phe Gly
        835                 840                 845

cag gtg tac aac aat atg agc ggc gac gcg ccc gat ttg gac gat aca 2592
Gln Val Tyr Asn Asn Met Ser Gly Asp Ala Pro Asp Leu Asp Asp Thr
    850                 855                 860

agt cgt ctg aaa gcg ttt tac aac gtg att cag cag ctt gtc gcc gaa 2640
Ser Arg Leu Lys Ala Phe Tyr Asn Val Ile Gln Gln Leu Val Ala Glu
865                 870                 875                 880

gac aaa ctc ttg gcg tat cac gac cgc agc gac ggc ggc ttg ttt gcc 2688
Asp Lys Leu Leu Ala Tyr His Asp Arg Ser Asp Gly Gly Leu Phe Ala
                885                 890                 895

gtt ttg gta gaa atg gcg ttt gca ggg cgg tgc ggc ttg gat ata gat 2736
Val Leu Val Glu Met Ala Phe Ala Gly Arg Cys Gly Leu Asp Ile Asp
            900                 905                 910

tta aat tta ttg ctt gca caa aca ttt att acc aac cat acc gct ctg 2784
Leu Asn Leu Leu Leu Ala Gln Thr Phe Ile Thr Asn His Thr Ala Leu
        915                 920                 925

tct caa tca ttg cgg act gaa gag gta aaa gcg ttg gct gaa tgg caa 2832
Ser Gln Ser Leu Arg Thr Glu Glu Val Lys Ala Leu Ala Glu Trp Gln
    930                 935                 940

gaa acc att gcc cgc aca tta ttt aat gaa gag ttg ggt gct gtt atc 2880
Glu Thr Ile Ala Arg Thr Leu Phe Asn Glu Glu Leu Gly Ala Val Ile
945                 950                 955                 960

caa gtt aga aaa caa gat gtt gcc gat att atc aat tta ttc tat caa 2928
Gln Val Arg Lys Gln Asp Val Ala Asp Ile Ile Asn Leu Phe Tyr Gln
                965                 970                 975

caa cag ctg cat cat aat gtc ttt gaa atc ggt acg tta act gat gag 2976
Gln Gln Leu His His Asn Val Phe Glu Ile Gly Thr Leu Thr Asp Glu
            980                 985                 990

aac acg tta atc atc cgc gac ggg caa acg cac ctt att tct gac aac 3024
Asn Thr Leu Ile Ile Arg Asp Gly Gln Thr His Leu Ile Ser Asp Asn

995                 1000                1005

cta atc aaa ctg caa caa acc tgg caa gaa acc agc cat caa atc caa    3072
Leu Ile Lys Leu Gln Gln Thr Trp Gln Glu Thr Ser His Gln Ile Gln
    1010                1015                1020

cgc ctg cgc gac aac cct gcc tgc gcc gac agc gag ttt gcc ctg att    3120
Arg Leu Arg Asp Asn Pro Ala Cys Ala Asp Ser Glu Phe Ala Leu Ile
1025                1030                1035                1040

ggc gac aac gga cgc agc gca ttg ttt gcc aac ctg aaa ttc gac gtg    3168
Gly Asp Asn Gly Arg Ser Ala Leu Phe Ala Asn Leu Lys Phe Asp Val
                1045                1050                1055

aac gaa gac atc gcc gcg ccg ttt atc aac agc ggc gcg aaa ccc aaa    3216
Asn Glu Asp Ile Ala Ala Pro Phe Ile Asn Ser Gly Ala Lys Pro Lys
            1060                1065                1070

atc gcc atc ctg cgc gaa cag ggc gta aac ggg caa atc gaa atg gcc    3264
Ile Ala Ile Leu Arg Glu Gln Gly Val Asn Gly Gln Ile Glu Met Ala
        1075                1080                1085

gcc gcc ttc acc cgt gcc ggt ttc gat gcc tac gac gtg cat atg tcc    3312
Ala Ala Phe Thr Arg Ala Gly Phe Asp Ala Tyr Asp Val His Met Ser
    1090                1095                1100

gac ctg atg gca ggc cgc gtc cac ctt gcc gac ttc aaa atg ctg gcg    3360
Asp Leu Met Ala Gly Arg Val His Leu Ala Asp Phe Lys Met Leu Ala
1105                1110                1115                1120

gcg tgc ggc ggc ttc agc tac ggc gac gta ctc ggc gcg ggc aaa ggc    3408
Ala Cys Gly Gly Phe Ser Tyr Gly Asp Val Leu Gly Ala Gly Lys Gly
                1125                1130                1135

tgg gcg aaa tcc atc ctg ttc cac ccc gcc tta cgc gac cag ttc gcc    3456
Trp Ala Lys Ser Ile Leu Phe His Pro Ala Leu Arg Asp Gln Phe Ala
            1140                1145                1150

gcc ttc ttc acc gac ccg aac acg ctg aca ttg ggc gtg tgc aac ggc    3504
Ala Phe Phe Thr Asp Pro Asn Thr Leu Thr Leu Gly Val Cys Asn Gly
        1155                1160                1165

tgc cag atg gtc agc aac ctc gcc gaa atc atc ccc ggc acg gca ggc    3552
Cys Gln Met Val Ser Asn Leu Ala Glu Ile Ile Pro Gly Thr Ala Gly
    1170                1175                1180

tgg ccg aag ttc aag cgc aac ctg agc gaa cag ttc gaa gcg cgc ctg    3600
Trp Pro Lys Phe Lys Arg Asn Leu Ser Glu Gln Phe Glu Ala Arg Leu

1185                1190                1195                1200

agc atg gtt cac gtc ccc aaa tca gcc tcc ctg att ctg aac gaa atg      3648
Ser Met Val His Val Pro Lys Ser Ala Ser Leu Ile Leu Asn Glu Met
                1205                1210                1215

caa ggc tcc agc ctg ccc gtc gtc gtc agc cac ggc gaa ggc cgc gcc      3696
Gln Gly Ser Ser Leu Pro Val Val Val Ser His Gly Glu Gly Arg Ala
            1220                1225                1230

gac ttc gcg ctt cac ggc ggc aac att tct gcc gat ttg ggc att gcg      3744
Asp Phe Ala Leu His Gly Gly Asn Ile Ser Ala Asp Leu Gly Ile Ala
        1235                1240                1245

ttg caa tat gtg gac gga caa aac caa att acc caa acc tac ccg ctc      3792
Leu Gln Tyr Val Asp Gly Gln Asn Gln Ile Thr Gln Thr Tyr Pro Leu
    1250                1255                1260

aac ccc aac ggc tcg ccg caa ggc atc gcc ggc gtg acc aac gcc gac      3840
Asn Pro Asn Gly Ser Pro Gln Gly Ile Ala Gly Val Thr Asn Ala Asp
1265                1270                1275                1280

ggc cgc gtt acc atc atg atg ccg cat cct gaa cgt gta tac cgc gcc      3888
Gly Arg Val Thr Ile Met Met Pro His Pro Glu Arg Val Tyr Arg Ala
                1285                1290                1295

gca caa atg agc tgg aaa ccg gaa gac tgg acg gaa ttg tcc ggc tgg      3936
Ala Gln Met Ser Trp Lys Pro Glu Asp Trp Thr Glu Leu Ser Gly Trp
            1300                1305                1310

tac cgc ctc ttc gcc ggc gca cgt aaa gcc ttg ggc                      3972
Tyr Arg Leu Phe Ala Gly Ala Arg Lys Ala Leu Gly
        1315                1320

<210> 38
<211> 1324
<212> PRT
<213> Neisseria meningitidis

<400> 38
Val Ser Arg Ile Met Ser Val Val Leu Pro Leu Arg Gly Val Thr Ala
1               5                   10                  15

Leu Ser Asp Phe Arg Val Glu Lys Leu Leu Gln Lys Ala Ala Ala Leu
            20                  25                  30

Gly Leu Pro Glu Val Lys Leu Ser Ser Glu Phe Trp Tyr Phe Val Gly

        35                  40                  45

Ser Glu Lys Ala Leu Asp Ala Ala Thr Val Glu Lys Leu Gln Ala Leu
    50                  55                  60

Leu Ala Ala Gln Ser Val Glu Gln Thr Pro Lys Ala Arg Glu Gly Leu
65                  70                  75                  80

His Leu Phe Leu Val Thr Pro Arg Leu Gly Thr Ile Ser Pro Trp Ala
                85                  90                  95

Ser Lys Ala Thr Asn Ile Ala Glu Asn Cys Gly Leu Ala Gly Ile Glu
            100                 105                 110

Arg Ile Glu Arg Gly Met Ala Val Trp Leu Glu Gly Ala Leu Thr Asp
        115                 120                 125

Glu Gln Gln Gln Gln Trp Ala Ala Leu Leu His Asp Arg Met Thr Glu
    130                 135                 140

Ser Val Leu Pro Asp Phe Gln Thr Ala Ser Lys Leu Phe His His Leu
145                 150                 155                 160

Glu Ser Glu Thr Phe Ser Thr Val Asp Val Leu Gly Gly Gly Lys Glu
                165                 170                 175

Ala Leu Val Lys Ala Asn Thr Glu Met Gly Leu Ala Leu Ser Ala Asp
            180                 185                 190

Glu Ile Asp Tyr Leu Val Glu Asn Tyr Gln Ala Leu Gln Arg Asn Pro
        195                 200                 205

Ser Asp Val Glu Leu Met Met Phe Ala Gln Ala Asn Ser Glu His Cys
    210                 215                 220

Arg His Lys Ile Phe Asn Ala Asp Phe Ile Leu Asn Gly Glu Lys Gln
225                 230                 235                 240

Pro Lys Ser Leu Phe Gly Met Ile Arg Asp Thr His Asn Ala His Pro
                245                 250                 255

Glu Gly Thr Val Val Ala Tyr Lys Asp Asn Ser Ser Val Ile Glu Gly
            260                 265                 270

Ala Lys Val Glu Arg Phe Tyr Pro Asn Ala Ala Glu Asn Gln Gly Tyr
        275                 280                 285

Arg Phe His Glu Glu Asp Thr His Ile Ile Met Lys Val Glu Thr His

290                 295                 300

Asn His Pro Thr Ala Ile Ala Pro Phe Ala Gly Ala Ala Thr Gly Ala
305                 310                 315                 320

Gly Gly Glu Ile Arg Asp Glu Gly Ala Thr Gly Lys Gly Ser Arg Pro
                325                 330                 335

Lys Ala Gly Leu Thr Gly Phe Thr Val Ser Asn Leu Asn Ile Pro Gly
            340                 345                 350

Leu Lys Gln Pro Trp Glu Gln Asp Tyr Gly Lys Pro Glu His Ile Ser
        355                 360                 365

Ser Pro Leu Asp Ile Met Ile Glu Gly Pro Ile Gly Gly Ala Ala Phe
    370                 375                 380

Asn Asn Glu Phe Gly Arg Pro Asn Leu Leu Gly Tyr Phe Arg Thr Phe
385                 390                 395                 400

Glu Glu Lys Phe Asp Gly Gln Val Arg Gly Tyr His Lys Pro Ile Met
                405                 410                 415

Ile Ala Gly Gly Leu Gly Ser Ile Gln Ala Gln Gln Thr His Lys Asp
            420                 425                 430

Glu Ile Pro Glu Gly Ala Leu Leu Ile Gln Leu Gly Gly Pro Gly Met
        435                 440                 445

Leu Ile Gly Leu Gly Gly Gly Ala Ala Ser Ser Met Asp Thr Gly Thr
    450                 455                 460

Asn Asp Ala Ser Leu Asp Phe Asn Ser Val Gln Arg Gly Asn Pro Glu
465                 470                 475                 480

Ile Glu Arg Arg Ala Gln Glu Val Ile Asp Arg Cys Trp Gln Leu Gly
                485                 490                 495

Asp Lys Asn Pro Ile Ile Ser Ile His Asp Val Gly Ala Gly Gly Leu
            500                 505                 510

Ser Asn Ala Phe Pro Glu Leu Val Asn Asp Ala Gly Arg Gly Ala Val
        515                 520                 525

Phe Lys Leu Arg Glu Val Pro Leu Glu Glu His Gly Leu Asn Pro Leu
    530                 535                 540

Gln Ile Trp Cys Asn Glu Ser Gln Glu Arg Tyr Val Leu Ser Ile Leu

545                 550                 555                 560

Glu Lys Asp Leu Asp Ile Phe Arg Ser Ile Cys Glu Arg Glu Arg Cys
                565                 570                 575

Pro Phe Ala Val Val Gly Thr Ala Thr Asp Asp Gly His Leu Lys Val
            580                 585                 590

Arg Asp Asp Leu Phe Ser Asn Asn Pro Val Asp Leu Pro Leu Asn Val
        595                 600                 605

Leu Leu Gly Lys Pro Pro Lys Thr Thr Arg Thr Asp Lys Thr Val Ala
    610                 615                 620

Pro Ser Lys Lys Pro Phe His Ala Gly Asp Ile Asp Ile Thr Glu Ala
625                 630                 635                 640

Ala Tyr Arg Val Leu Arg Leu Pro Ala Val Ala Ala Lys Asn Phe Leu
                645                 650                 655

Ile Thr Ile Gly Asp Arg Ser Val Gly Gly Met Thr His Arg Asp Gln
            660                 665                 670

Met Val Gly Lys Tyr Gln Thr Pro Val Ala Asp Cys Ala Val Thr Met
        675                 680                 685

Met Gly Phe Asn Thr Tyr Arg Gly Glu Ala Met Ser Met Gly Glu Lys
    690                 695                 700

Pro Thr Val Ala Leu Phe Asp Ala Pro Ala Ser Gly Arg Met Cys Val
705                 710                 715                 720

Gly Glu Ala Ile Thr Asn Ile Ala Ala Ala Asn Ile Gly Asp Ile Gly
                725                 730                 735

Asn Ile Lys Leu Ser Ala Asn Trp Met Ala Ala Cys Gly Asn Glu Gly
            740                 745                 750

Glu Asp Glu Lys Leu Tyr Arg Thr Val Glu Ala Val Ser Lys Ala Cys
        755                 760                 765

Gln Ala Leu Asp Leu Ser Ile Pro Val Gly Lys Asp Ser Leu Ser Met
    770                 775                 780

Lys Thr Val Trp Gln Asp Gly Glu Glu Lys Lys Ser Val Val Ser Pro
785                 790                 795                 800

Leu Ser Leu Ile Ile Ser Ala Phe Ala Pro Val Gln Asp Val Arg Lys

                805                 810                 815

Thr Val Thr Pro Glu Leu Lys Asn Val Glu Asp Ser Val Leu Leu Phe
            820                 825                 830

Val Asp Leu Gly Phe Gly Lys Ala Arg Met Gly Gly Ser Ala Phe Gly
        835                 840                 845

Gln Val Tyr Asn Asn Met Ser Gly Asp Ala Pro Asp Leu Asp Asp Thr
    850                 855                 860

Ser Arg Leu Lys Ala Phe Tyr Asn Val Ile Gln Gln Leu Val Ala Glu
865                 870                 875                 880

Asp Lys Leu Leu Ala Tyr His Asp Arg Ser Asp Gly Gly Leu Phe Ala
                885                 890                 895

Val Leu Val Glu Met Ala Phe Ala Gly Arg Cys Gly Leu Asp Ile Asp
            900                 905                 910

Leu Asn Leu Leu Leu Ala Gln Thr Phe Ile Thr Asn His Thr Ala Leu
        915                 920                 925

Ser Gln Ser Leu Arg Thr Glu Glu Val Lys Ala Leu Ala Glu Trp Gln
    930                 935                 940

Glu Thr Ile Ala Arg Thr Leu Phe Asn Glu Glu Leu Gly Ala Val Ile
945                 950                 955                 960

Gln Val Arg Lys Gln Asp Val Ala Asp Ile Ile Asn Leu Phe Tyr Gln
                965                 970                 975

Gln Gln Leu His His Asn Val Phe Glu Ile Gly Thr Leu Thr Asp Glu
            980                 985                 990

Asn Thr Leu Ile Ile Arg Asp Gly Gln Thr His Leu Ile Ser Asp Asn
        995                 1000                1005

Leu Ile Lys Leu Gln Gln Thr Trp Gln Glu Thr Ser His Gln Ile Gln
    1010                1015                1020

Arg Leu Arg Asp Asn Pro Ala Cys Ala Asp Ser Glu Phe Ala Leu Ile
025 1030 1035 1040

Gly Asp Asn Gly Arg Ser Ala Leu Phe Ala Asn Leu Lys Phe Asp Val
                1045                1050                1055

Asn Glu Asp Ile Ala Ala Pro Phe Ile Asn Ser Gly Ala Lys Pro Lys

1060                 1065                 1070

Ile Ala Ile Leu Arg Glu Gln Gly Val Asn Gly Gln Ile Glu Met Ala
        1075                 1080                 1085

Ala Ala Phe Thr Arg Ala Gly Phe Asp Ala Tyr Asp Val His Met Ser
    1090                 1095                 1100

Asp Leu Met Ala Gly Arg Val His Leu Ala Asp Phe Lys Met Leu Ala
105                 1110                 1115                 1120

Ala Cys Gly Gly Phe Ser Tyr Gly Asp Val Leu Gly Ala Gly Lys Gly
                1125                 1130                 1135

Trp Ala Lys Ser Ile Leu Phe His Pro Ala Leu Arg Asp Gln Phe Ala
            1140                 1145                 1150

Ala Phe Phe Thr Asp Pro Asn Thr Leu Thr Leu Gly Val Cys Asn Gly
        1155                 1160                 1165

Cys Gln Met Val Ser Asn Leu Ala Glu Ile Ile Pro Gly Thr Ala Gly
    1170                 1175                 1180

Trp Pro Lys Phe Lys Arg Asn Leu Ser Glu Gln Phe Glu Ala Arg Leu
185                 1190                 1195                 1200

Ser Met Val His Val Pro Lys Ser Ala Ser Leu Ile Leu Asn Glu Met
                1205                 1210                 1215

Gln Gly Ser Ser Leu Pro Val Val Val Ser His Gly Glu Gly Arg Ala
            1220                 1225                 1230

Asp Phe Ala Leu His Gly Gly Asn Ile Ser Ala Asp Leu Gly Ile Ala
        1235                 1240                 1245

Leu Gln Tyr Val Asp Gly Gln Asn Gln Ile Thr Gln Thr Tyr Pro Leu
    1250                 1255                 1260

Asn Pro Asn Gly Ser Pro Gln Gly Ile Ala Gly Val Thr Asn Ala Asp
265                 1270                 1275                 1280

Gly Arg Val Thr Ile Met Met Pro His Pro Glu Arg Val Tyr Arg Ala
                1285                 1290                 1295

Ala Gln Met Ser Trp Lys Pro Glu Asp Trp Thr Glu Leu Ser Gly Trp
            1300                 1305                 1310

Tyr Arg Leu Phe Ala Gly Ala Arg Lys Ala Leu Gly

1315                1320

<210> 39
<211> 471
<212> DNA
<213> Neisseria meningitidis

<220>
<221> CDS
<222> (1)..(471)

<400> 39
atg tcc cgc aat acc gaa gag ctg caa ggc atc tcg ctt ttg ggt aat     48
Met Ser Arg Asn Thr Glu Glu Leu Gln Gly Ile Ser Leu Leu Gly Asn
1               5                   10                  15

cag aaa acc cga tat ccg acc ggc tac gcg cct gaa att ctc gaa gca     96
Gln Lys Thr Arg Tyr Pro Thr Gly Tyr Ala Pro Glu Ile Leu Glu Ala
            20                  25                  30

ttc gac aac aaa cat ccc gac aac gac tat ttc gtc aaa ttc gtc tgc     144
Phe Asp Asn Lys His Pro Asp Asn Asp Tyr Phe Val Lys Phe Val Cys
        35                  40                  45

cca gag ttc acc agc ctc tgc ccc atg acc ggg cag ccc gac ttc gcc     192
Pro Glu Phe Thr Ser Leu Cys Pro Met Thr Gly-Gln Pro Asp Phe Ala
    50                  55                  60

acc atc tac atc cgc tac att ccg cac atc aaa atg gtg gaa agc aaa     240
Thr Ile Tyr Ile Arg Tyr Ile Pro His Ile Lys Met Val Glu Ser Lys
65                  70                  75                  80

tcc ctg aaa ctc tac ctc ttc agc ttc cgc aac cac ggc gat ttt cat     288
Ser Leu Lys Leu Tyr Leu Phe Ser Phe Arg Asn His Gly Asp Phe His
                85                  90                  95

gaa gac tgc gtc aac atc atc atg aaa gac ctc att gcc ctg atg gat     336
Glu Asp Cys Val Asn Ile Ile Met Lys Asp Leu Ile Ala Leu Met Asp
            100                 105                 110

ccg aaa tac atc gaa gta ttc ggc gag ttc aca ccg cgc ggc ggc atc     384
Pro Lys Tyr Ile Glu Val Phe Gly Glu Phe Thr Pro Arg Gly Gly Ile
        115                 120                 125

gcc att cat cct ttc gcc aat tac ggc aaa gca ggc acc gag ttt gaa     432
Ala Ile His Pro Phe Ala Asn Tyr Gly Lys Ala Gly Thr Glu Phe Glu

130 135 140

gca ttg gcg cgt aaa cgc ctg ttt gag cac gac gca caa 471
Ala Leu Ala Arg Lys Arg Leu Phe Glu His Asp Ala Gln
145 150 155

<210> 40
<211> 157
<212> PRT
<213> Neisseria meningitidis

<400> 40
Met Ser Arg Asn Thr Glu Glu Leu Gln Gly Ile Ser Leu Leu Gly Asn
1               5                   10                  15

Gln Lys Thr Arg Tyr Pro Thr Gly Tyr Ala Pro Glu Ile Leu Glu Ala
            20                  25                  30

Phe Asp Asn Lys His Pro Asp Asn Asp Tyr Phe Val Lys Phe Val Cys
        35                  40                  45

Pro Glu Phe Thr Ser Leu Cys Pro Met Thr Gly Gln Pro Asp Phe Ala
    50                  55                  60

Thr Ile Tyr Ile Arg Tyr Ile Pro His Ile Lys Met Val Glu Ser Lys
65                  70                  75                  80

Ser Leu Lys Leu Tyr Leu Phe Ser Phe Arg Asn His Gly Asp Phe His
                85                  90                  95

Glu Asp Cys Val Asn Ile Ile Met Lys Asp Leu Ile Ala Leu Met Asp
            100                 105                 110

Pro Lys Tyr Ile Glu Val Phe Gly Glu Phe Thr Pro Arg Gly Gly Ile
        115                 120                 125

Ala Ile His Pro Phe Ala Asn Tyr Gly Lys Ala Gly Thr Glu Phe Glu
    130                 135                 140

Ala Leu Ala Arg Lys Arg Leu Phe Glu His Asp Ala Gln
145                 150                 155

<210> 41
<211> 504
<212> DNA

<213> Neisseria meningitidis

<220>
<221> CDS
<222> (1)..(504)

<400> 41
atg ccc cta cta gac agt ttc aaa gtc gat cac acc cgt atg cat gcc    48
Met Pro Leu Leu Asp Ser Phe Lys Val Asp His Thr Arg Met His Ala
1               5                   10                  15

ccc gcc gta cgc gtg gcg aaa acc atg acc acg ccc aaa ggc gac acc    96
Pro Ala Val Arg Val Ala Lys Thr Met Thr Thr Pro Lys Gly Asp Thr
            20                  25                  30

att acc gtg ttc gac ctg cgc ttt tgc gtt ccc aat aaa gaa atc ctg    144
Ile Thr Val Phe Asp Leu Arg Phe Cys Val Pro Asn Lys Glu Ile Leu
        35                  40                  45

cct gaa aaa ggc ata cac acg ctg gag cat ttg ttc gca ggt ttt atg    192
Pro Glu Lys Gly Ile His Thr Leu Glu His Leu Phe Ala Gly Phe Met
    50                  55                  60

cgc gac cac ttg aac ggc aac ggc gtg gaa atc atc gac att tcc ccg    240
Arg Asp His Leu Asn Gly Asn Gly Val Glu Ile Ile Asp Ile Ser Pro
65                  70                  75                  80

atg ggc tgc cgc acc ggt ttt tat atg agc ctt atc ggc acg cct tcc    288
Met Gly Cys Arg Thr Gly Phe Tyr Met Ser Leu Ile Gly Thr Pro Ser
                85                  90                  95

gaa cag cag gtc gcc gat gcg tgg ctg gct tcg atg cag gat gtt ttg    336
Glu Gln Gln Val Ala Asp Ala Trp Leu Ala Ser Met Gln Asp Val Leu
            100                 105                 110

aat gtc aaa gac caa agc aaa atc ccc gag ttg aac gaa tac caa tgc    384
Asn Val Lys Asp Gln Ser Lys Ile Pro Glu Leu Asn Glu Tyr Gln Cys
        115                 120                 125

ggc act tat caa atg cac tcg ctc gcc gaa gcg cag caa atc gcg caa    432
Gly Thr Tyr Gln Met His Ser Leu Ala Glu Ala Gln Gln Ile Ala Gln
    130                 135                 140

aac gtg ttg gcg cgc aaa gtg gcg gtg aac aaa aat gaa gag ctg acg    480
Asn Val Leu Ala Arg Lys Val Ala Val Asn Lys Asn Glu Glu Leu Thr
145                 150                 155                 160

ctg gat gaa ggg ctg ctg aac gcc                                    504

Leu Asp Glu Gly Leu Leu Asn Ala
                165

<210> 42
<211> 168
<212> PRT
<213> Neisseria meningitidis

<400> 42
Met Pro Leu Leu Asp Ser Phe Lys Val Asp His Thr Arg Met His Ala
1               5                   10                  15

Pro Ala Val Arg Val Ala Lys Thr Met Thr Thr Pro Lys Gly Asp Thr
            20                  25                  30

Ile Thr Val Phe Asp Leu Arg Phe Cys Val Pro Asn Lys Glu Ile Leu
        35                  40                  45

Pro Glu Lys Gly Ile His Thr Leu Glu His Leu Phe Ala Gly Phe Met
    50                  55                  60

Arg Asp His Leu Asn Gly Asn Gly Val Glu Ile Ile Asp Ile Ser Pro
65                  70                  75                  80

Met Gly Cys Arg Thr Gly Phe Tyr Met Ser Leu Ile Gly Thr Pro Ser
                85                  90                  95

Glu Gln Gln Val Ala Asp Ala Trp Leu Ala Ser Met Gln Asp Val Leu
            100                 105                 110

Asn Val Lys Asp Gln Ser Lys Ile Pro Glu Leu Asn Glu Tyr Gln Cys
        115                 120                 125

Gly Thr Tyr Gln Met His Ser Leu Ala Glu Ala Gln Gln Ile Ala Gln
    130                 135                 140

Asn Val Leu Ala Arg Lys Val Ala Val Asn Lys Asn Glu Glu Leu Thr
145                 150                 155                 160

Leu Asp Glu Gly Leu Leu Asn Ala
                165

<210> 43
<211> 813
<212> DNA

<213> Neisseria meningitidis

<220>
<221> CDS
<222> (1)..(813)

<400> 43
atg act atg cac gcc ctc ccc cgc tac gcc gtt ttt ggc aac ccc gtc     48
Met Thr Met His Ala Leu Pro Arg Tyr Ala Val Phe Gly Asn Pro Val
1               5                   10                  15

gcc cac agc aaa tcg ccg caa att cat caa caa ttt gcc ctt cag gaa     96
Ala His Ser Lys Ser Pro Gln Ile His Gln Gln Phe Ala Leu Gln Glu
            20                  25                  30

ggc gtt gac att gaa tac gaa cgc att tgc gcc gac atc ggc ggt ttc     144
Gly Val Asp Ile Glu Tyr Glu Arg Ile Cys Ala Asp Ile Gly Gly Phe
        35                  40                  45

gcg cag gcg gtt tcg aca ttt ttt gaa aca ggc ggt tgc ggg gca aac     192
Ala Gln Ala Val Ser Thr Phe Phe Glu Thr Gly Gly Cys Gly Ala Asn
    50                  55                  60

gtt acc gta ccg ttc aag cag gaa gcg ttt cat ctg gcg gac gag cat     240
Val Thr Val Pro Phe Lys Gln Glu Ala Phe His Leu Ala Asp Glu His
65                  70                  75                  80

tct gaa cgc gca ttg gct gcc ggc gcg gtc aac acg ctg att ttt ctg     288
Ser Glu Arg Ala Leu Ala Ala Gly Ala Val Asn Thr Leu Ile Phe Leu
                85                  90                  95

aaa aac gga aaa ctg cgc ggc gac aat acc gac ggt atc ggt ttg gcc     336
Lys Asn Gly Lys Leu Arg Gly Asp Asn Thr Asp Gly Ile Gly Leu Ala
            100                 105                 110

aac gac atc acg cag gtc aaa aac att gcc atc gaa ggc aaa acc atc     384
Asn Asp Ile Thr Gln Val Lys Asn Ile Ala Ile Glu Gly Lys Thr Ile
        115                 120                 125

ttg ctt ttg ggc gcg ggc ggc gcg gtg cgc ggc gtg att cct gtt ttg     432
Leu Leu Leu Gly Ala Gly Gly Ala Val Arg Gly Val Ile Pro Val Leu
    130                 135                 140

aaa gaa cac cgt cct gcc cgt atc gtc att gcc aac cgt acc cgc gcc     480
Lys Glu His Arg Pro Ala Arg Ile Val Ile Ala Asn Arg Thr Arg Ala
145                 150                 155                 160

aaa gcc gag gaa ttg gcg cag ctt ttc ggc att gaa gcc gtc ccg atg     528

Lys Ala Glu Glu Leu Ala Gln Leu Phe Gly Ile Glu Ala Val Pro Met
                165                 170                 175

gcg gat gtg aac ggc ggt ttt gat atc atc atc aac ggc acg tcg ggc     576
Ala Asp Val Asn Gly Gly Phe Asp Ile Ile Ile Asn Gly Thr Ser Gly
            180                 185                 190

ggt cta aac ggt cag att ccc gat att ccg ccc gat att ttt caa aac     624
Gly Leu Asn Gly Gln Ile Pro Asp Ile Pro Pro Asp Ile Phe Gln Asn
        195                 200                 205

tgc gcg ctt gcc tac gat atg gtg tac ggc tgc gcg gca aaa ccg ttt     672
Cys Ala Leu Ala Tyr Asp Met Val Tyr Gly Cys Ala Ala Lys Pro Phe
    210                 215                 220

tta gat ttt gca cga caa tcg ggt gcg aaa aaa act gcc gac gga ctg     720
Leu Asp Phe Ala Arg Gln Ser Gly Ala Lys Lys Thr Ala Asp Gly Leu
225                 230                 235                 240

ggt atg cta gtc ggt caa gcg gcg gct tcc tac gcc ctc tgg cgc gga     768
Gly Met Leu Val Gly Gln Ala Ala Ala Ser Tyr Ala Leu Trp Arg Gly
                245                 250                 255

ttt acg ccc gat atc cgc ccc gtt atc gaa tac atg aaa gcc cta         813
Phe Thr Pro Asp Ile Arg Pro Val Ile Glu Tyr Met Lys Ala Leu
            260                 265                 270

<210> 44
<211> 271
<212> PRT
<213> Neisseria meningitidis

<400> 44
Met Thr Met His Ala Leu Pro Arg Tyr Ala Val Phe Gly Asn Pro Val
  1               5                  10                  15

Ala His Ser Lys Ser Pro Gln Ile His Gln Gln Phe Ala Leu Gln Glu
             20                  25                  30

Gly Val Asp Ile Glu Tyr Glu Arg Ile Cys Ala Asp Ile Gly Gly Phe
         35                  40                  45

Ala Gln Ala Val Ser Thr Phe Phe Glu Thr Gly Gly Cys Gly Ala Asn
     50                  55                  60

Val Thr Val Pro Phe Lys Gln Glu Ala Phe His Leu Ala Asp Glu His
 65                  70                  75                  80

Ser Glu Arg Ala Leu Ala Ala Gly Ala Val Asn Thr Leu Ile Phe Leu
                85                  90                  95

Lys Asn Gly Lys Leu Arg Gly Asp Asn Thr Asp Gly Ile Gly Leu Ala
            100                 105                 110

Asn Asp Ile Thr Gln Val Lys Asn Ile Ala Ile Glu Gly Lys Thr Ile
        115                 120                 125

Leu Leu Leu Gly Ala Gly Gly Ala Val Arg Gly Val Ile Pro Val Leu
    130                 135                 140

Lys Glu His Arg Pro Ala Arg Ile Val Ile Ala Asn Arg Thr Arg Ala
145                 150                 155                 160

Lys Ala Glu Glu Leu Ala Gln Leu Phe Gly Ile Glu Ala Val Pro Met
                165                 170                 175

Ala Asp Val Asn Gly Gly Phe Asp Ile Ile Ile Asn Gly Thr Ser Gly
            180                 185                 190

Gly Leu Asn Gly Gln Ile Pro Asp Ile Pro Pro Asp Ile Phe Gln Asn
        195                 200                 205

Cys Ala Leu Ala Tyr Asp Met Val Tyr Gly Cys Ala Ala Lys Pro Phe
    210                 215                 220

Leu Asp Phe Ala Arg Gln Ser Gly Ala Lys Lys Thr Ala Asp Gly Leu
225                 230                 235                 240

Gly Met Leu Val Gly Gln Ala Ala Ala Ser Tyr Ala Leu Trp Arg Gly
                245                 250                 255

Phe Thr Pro Asp Ile Arg Pro Val Ile Glu Tyr Met Lys Ala Leu
            260                 265                 270

<210> 45
<211> 546
<212> DNA
<213> Neisseria meningitidis

<220>
<221> CDS
<222> (1)..(546)

<400> 45
ttg ctt tct cat ttg gat atg aaa ttc gtc agc gac ctt ttg tct gtc    48
Leu Leu Ser His Leu Asp Met Lys Phe Val Ser Asp Leu Leu Ser Val
1               5                   10                  15

atc ttg ttt ttt gct act tat acc gtt acc aaa aat atg att gcc gct    96
Ile Leu Phe Phe Ala Thr Tyr Thr Val Thr Lys Asn Met Ile Ala Ala
            20                  25                  30

acg gcg gtt gcc ttg gtt gcc ggt gtg gtt cag gcg gct ttt ctg tat    144
Thr Ala Val Ala Leu Val Ala Gly Val Val Gln Ala Ala Phe Leu Tyr
        35                  40                  45

tgg aaa tat aaa aag ctg gat acg atg cag tgg gtc gga ctg gtg ctg    192
Trp Lys Tyr Lys Lys Leu Asp Thr Met Gln Trp Val Gly Leu Val Leu
    50                  55                  60

att gtc gta ttc ggc ggc gca acc att gtt ttg ggc gac agc cgc ttc    240
Ile Val Val Phe Gly Gly Ala Thr Ile Val Leu Gly Asp Ser Arg Phe
65                  70                  75                  80

att atg tgg aag ccg agc gtt ttg ttt tgg ctg ggc gcg ctg ttc ctg    288
Ile Met Trp Lys Pro Ser Val Leu Phe Trp Leu Gly Ala Leu Phe Leu
                85                  90                  95

tgg ggc agc cac ctc gcc ggt aaa aac ggc ttg aag gcg agt atc ggc    336
Trp Gly Ser His Leu Ala Gly Lys Asn Gly Leu Lys Ala Ser Ile Gly
            100                 105                 110

agg gag att cag ctt ccg gat gcc gta tgg gcg aaa ttg acg tat atg    384
Arg Glu Ile Gln Leu Pro Asp Ala Val Trp Ala Lys Leu Thr Tyr Met
        115                 120                 125

tgg gtc ggt ttc ctg att ttt atg ggt atc gcc aac tgg ttt gtg ttt    432
Trp Val Gly Phe Leu Ile Phe Met Gly Ile Ala Asn Trp Phe Val Phe
    130                 135                 140

acc cgg ttc gag tcg caa tgg gtc aac tat aaa atg ttc ggc tcg act    480
Thr Arg Phe Glu Ser Gln Trp Val Asn Tyr Lys Met Phe Gly Ser Thr
145                 150                 155                 160

gca ctg atg ctt gtt ttc ttt att att cag ggt att tat ctg agt acc    528
Ala Leu Met Leu Val Phe Phe Ile Ile Gln Gly Ile Tyr Leu Ser Thr
                165                 170                 175

tgt ctg aaa aag gag gat                                            546
Cys Leu Lys Lys Glu Asp
            180

<210> 46
<211> 182
<212> PRT
<213> Neisseria meningitidis

<400> 46
Leu Leu Ser His Leu Asp Met Lys Phe Val Ser Asp Leu Leu Ser Val
1               5                   10                  15

Ile Leu Phe Phe Ala Thr Tyr Thr Val Thr Lys Asn Met Ile Ala Ala
            20                  25                  30

Thr Ala Val Ala Leu Val Ala Gly Val Val Gln Ala Ala Phe Leu Tyr
        35                  40                  45

Trp Lys Tyr Lys Lys Leu Asp Thr Met Gln Trp Val Gly Leu Val Leu
    50                  55                  60

Ile Val Val Phe Gly Gly Ala Thr Ile Val Leu Gly Asp Ser Arg Phe
65                  70                  75                  80

Ile Met Trp Lys Pro Ser Val Leu Phe Trp Leu Gly Ala Leu Phe Leu
                85                  90                  95

Trp Gly Ser His Leu Ala Gly Lys Asn Gly Leu Lys Ala Ser Ile Gly
            100                 105                 110

Arg Glu Ile Gln Leu Pro Asp Ala Val Trp Ala Lys Leu Thr Tyr Met
        115                 120                 125

Trp Val Gly Phe Leu Ile Phe Met Gly Ile Ala Asn Trp Phe Val Phe
    130                 135                 140

Thr Arg Phe Glu Ser Gln Trp Val Asn Tyr Lys Met Phe Gly Ser Thr
145                 150                 155                 160

Ala Leu Met Leu Val Phe Phe Ile Ile Gln Gly Ile Tyr Leu Ser Thr
                165                 170                 175

Cys Leu Lys Lys Glu Asp
            180

<210> 47
<211> 585

<212> DNA
<213> Neisseria meningitidis

<220>
<221> CDS
<222> (1)..(585)

<400> 47
ttg aat att aaa ctg aaa acc ttg tta ttg ccc ttc gcc acg ctg gca    48
Leu Asn Ile Lys Leu Lys Thr Leu Leu Leu Pro Phe Ala Thr Leu Ala
1               5                   10                  15

ttg tgc acc aat gct ttt gcc gcc ccg ccc agc gac gcg tcg ttg gcg    96
Leu Cys Thr Asn Ala Phe Ala Ala Pro Pro Ser Asp Ala Ser Leu Ala
            20                  25                  30

cgt tgg ctg gat acg cag aat ttt gac cgg gat ata gaa aaa aat atg    144
Arg Trp Leu Asp Thr Gln Asn Phe Asp Arg Asp Ile Glu Lys Asn Met
        35                  40                  45

att gag ggc ttt aat gcc gga ttt aaa ccg tat gcg gac aaa gcc ctt    192
Ile Glu Gly Phe Asn Ala Gly Phe Lys Pro Tyr Ala Asp Lys Ala Leu
    50                  55                  60

gcc gaa atg ccg gaa gcg aaa aaa gat cag gcg gca gaa gcc ttt aac    240
Ala Glu Met Pro Glu Ala Lys Lys Asp Gln Ala Ala Glu Ala Phe Asn
65                  70                  75                  80

cgt tat cgt gag aat gtt ttg aaa gat ttg att acg ccc gaa gtg aaa    288
Arg Tyr Arg Glu Asn Val Leu Lys Asp Leu Ile Thr Pro Glu Val Lys
                85                  90                  95

cag gct gtc cgc aat act tta ttg aag aat gcc cgt gag ata tac acg    336
Gln Ala Val Arg Asn Thr Leu Leu Lys Asn Ala Arg Glu Ile Tyr Thr
            100                 105                 110

caa gaa gaa att gac ggc atg att gcc ttt tac ggt tcg cct gtc ggt    384
Gln Glu Glu Ile Asp Gly Met Ile Ala Phe Tyr Gly Ser Pro Val Gly
        115                 120                 125

cag tcc gtc gtt gcc aaa aat ccg cgc tta atc aag aaa tcg atg agt    432
Gln Ser Val Val Ala Lys Asn Pro Arg Leu Ile Lys Lys Ser Met Ser
    130                 135                 140

gaa ata gcg gta tct tgg act gca ttg tca ggg aaa atc gcg caa cat    480
Glu Ile Ala Val Ser Trp Thr Ala Leu Ser Gly Lys Ile Ala Gln His
145                 150                 155                 160

cat ctg ccc gag ttt acg gaa gag ttg cgg cgc atc atc tgc ggc ggt     528
His Leu Pro Glu Phe Thr Glu Glu Leu Arg Arg Ile Ile Cys Gly Gly
                165                 170                 175

aaa aat ccc gat gcg ggc tgt aaa caa gcc gga cag gtt ggg aaa agg     576
Lys Asn Pro Asp Ala Gly Cys Lys Gln Ala Gly Gln Val Gly Lys Arg
            180                 185                 190

cat cag aaa                                                         585
His Gln Lys
        195

<210> 48
<211> 195
<212> PRT
<213> Neisseria meningitidis

<400> 48
Leu Asn Ile Lys Leu Lys Thr Leu Leu Leu Pro Phe Ala Thr Leu Ala
  1               5                  10                  15

Leu Cys Thr Asn Ala Phe Ala Ala Pro Pro Ser Asp Ala Ser Leu Ala
             20                  25                  30

Arg Trp Leu Asp Thr Gln Asn Phe Asp Arg Asp Ile Glu Lys Asn Met
         35                  40                  45

Ile Glu Gly Phe Asn Ala Gly Phe Lys Pro Tyr Ala Asp Lys Ala Leu
     50                  55                  60

Ala Glu Met Pro Glu Ala Lys Lys Asp Gln Ala Ala Glu Ala Phe Asn
 65                  70                  75                  80

Arg Tyr Arg Glu Asn Val Leu Lys Asp Leu Ile Thr Pro Glu Val Lys
                 85                  90                  95

Gln Ala Val Arg Asn Thr Leu Leu Lys Asn Ala Arg Glu Ile Tyr Thr
            100                 105                 110

Gln Glu Glu Ile Asp Gly Met Ile Ala Phe Tyr Gly Ser Pro Val Gly
        115                 120                 125

Gln Ser Val Val Ala Lys Asn Pro Arg Leu Ile Lys Lys Ser Met Ser
    130                 135                 140

Glu Ile Ala Val Ser Trp Thr Ala Leu Ser Gly Lys Ile Ala Gln His
145                 150                 155                 160

His Leu Pro Glu Phe Thr Glu Glu Leu Arg Arg Ile Ile Cys Gly Gly
                165                 170                 175

Lys Asn Pro Asp Ala Gly Cys Lys Gln Ala Gly Gln Val Gly Lys Arg
            180                 185                 190

His Gln Lys
        195

<210> 49
<211> 462
<212> DNA
<213> Neisseria meningitidis

<220>
<221> CDS
<222> (1)..(462)

<400> 49
ttg ctt tgc ccg gaa aaa atg tcg ggg atg gcg gga cag tat ccg tac      48
Leu Leu Cys Pro Glu Lys Met Ser Gly Met Ala Gly Gln Tyr Pro Tyr
  1               5                  10                  15

ggc gtc cgg tcg ggt ttg cgg agg aac ggc ttg aaa ctt tgg gat att      96
Gly Val Arg Ser Gly Leu Arg Arg Asn Gly Leu Lys Leu Trp Asp Ile
             20                  25                  30

cat ttt aga atg acc cgt ttt atc gtc gca aga tgc ggt tta ttg ttt     144
His Phe Arg Met Thr Arg Phe Ile Val Ala Arg Cys Gly Leu Leu Phe
         35                  40                  45

gca acc ctt aaa gga aaa acc atg aag aaa atg ttc gtg ctg ttc tgt     192
Ala Thr Leu Lys Gly Lys Thr Met Lys Lys Met Phe Val Leu Phe Cys
     50                  55                  60

atg ctg ttc tcc tgc gcc ttc tcc ctt gcg gcg gta aac atc aat gcg     240
Met Leu Phe Ser Cys Ala Phe Ser Leu Ala Ala Val Asn Ile Asn Ala
 65                  70                  75                  80

gct tcg cag cag gag ctg gag gcg ctg ccg ggc ata ggc cct gcg aag     288
Ala Ser Gln Gln Glu Leu Glu Ala Leu Pro Gly Ile Gly Pro Ala Lys
                 85                  90                  95

gcg aag gcc att gcg gaa tac cgt gcg caa aac ggt gcg ttc aag tct     336
Ala Lys Ala Ile Ala Glu Tyr Arg Ala Gln Asn Gly Ala Phe Lys Ser

            100                 105                 110

gta gac gat ttg acc aag gta aag ggc atc ggc cct gcg gtg ctg gcg 384
Val Asp Asp Leu Thr Lys Val Lys Gly Ile Gly Pro Ala Val Leu Ala
        115                 120                 125

aag ctg aag gat cag gct tct gtc ggt gcg ccc gca cca aaa ggc cca 432
Lys Leu Lys Asp Gln Ala Ser Val Gly Ala Pro Ala Pro Lys Gly Pro
    130                 135                 140

gct aaa cca gtg ctg ccc gcg gat aaa aaa 462
Ala Lys Pro Val Leu Pro Ala Asp Lys Lys
145                 150

<210> 50
<211> 154
<212> PRT
<213> Neisseria meningitidis

<400> 50
Leu Leu Cys Pro Glu Lys Met Ser Gly Met Ala Gly Gln Tyr Pro Tyr
1               5                   10                  15

Gly Val Arg Ser Gly Leu Arg Arg Asn Gly Leu Lys Leu Trp Asp Ile
            20                  25                  30

His Phe Arg Met Thr Arg Phe Ile Val Ala Arg Cys Gly Leu Leu Phe
        35                  40                  45

Ala Thr Leu Lys Gly Lys Thr Met Lys Lys Met Phe Val Leu Phe Cys
    50                  55                  60

Met Leu Phe Ser Cys Ala Phe Ser Leu Ala Ala Val Asn Ile Asn Ala
65                  70                  75                  80

Ala Ser Gln Gln Glu Leu Glu Ala Leu Pro Gly Ile Gly Pro Ala Lys
                85                  90                  95

Ala Lys Ala Ile Ala Glu Tyr Arg Ala Gln Asn Gly Ala Phe Lys Ser
            100                 105                 110

Val Asp Asp Leu Thr Lys Val Lys Gly Ile Gly Pro Ala Val Leu Ala
        115                 120                 125

Lys Leu Lys Asp Gln Ala Ser Val Gly Ala Pro Ala Pro Lys Gly Pro
    130                 135                 140

Ala Lys Pro Val Leu Pro Ala Asp Lys Lys
145                 150

<210> 51
<211> 969
<212> DNA
<213> Neisseria meningitidis

<220>
<221> CDS
<222> (1)..(969)

<400> 51
atg tcc gcc aag ttc caa caa gaa acc ctc aaa tcc cgt ttc gcg caa    48
Met Ser Ala Lys Phe Gln Gln Glu Thr Leu Lys Ser Arg Phe Ala Gln
1               5                   10                  15

gcc aaa gtc ctg gtt gtc ggc gac gtg atg ctc gac cgc tat tgg ttc    96
Ala Lys Val Leu Val Val Gly Asp Val Met Leu Asp Arg Tyr Trp Phe
            20                  25                  30

ggc gat gtg tcc cgt att tcg ccc gaa gcc ccc gtg ccg gtg gcg aaa    144
Gly Asp Val Ser Arg Ile Ser Pro Glu Ala Pro Val Pro Val Ala Lys
        35                  40                  45

atc gga cga atc gac caa cgc gcg ggc gga gcg gca aat gtc gcg cgc    192
Ile Gly Arg Ile Asp Gln Arg Ala Gly Gly Ala Ala Asn Val Ala Arg
    50                  55                  60

aac atc gct tcg ctg ggc ggc aaa gta ggg ctg ttg tcg gta acc ggt    240
Asn Ile Ala Ser Leu Gly Gly Lys Val Gly Leu Leu Ser Val Thr Gly
65                  70                  75                  80

aac gac gaa gcc gcc gac gcg ctc gac gcg ctg atg gtg cag gac ggc    288
Asn Asp Glu Ala Ala Asp Ala Leu Asp Ala Leu Met Val Gln Asp Gly
                85                  90                  95

gtc gcc tcc tat ctg atg cgc gac aaa caa atc gcc acc acc gtc aaa    336
Val Ala Ser Tyr Leu Met Arg Asp Lys Gln Ile Ala Thr Thr Val Lys
            100                 105                 110

ctg cgc gtc gtc gcc cgc aac cag cag ctt atc cgc ctt gat ttt gaa    384
Leu Arg Val Val Ala Arg Asn Gln Gln Leu Ile Arg Leu Asp Phe Glu
        115                 120                 125

gaa cat ccc aac cgc gaa gtg ttg gaa caa atc aag cgg aaa tac cgc    432

Glu His Pro Asn Arg Glu Val Leu Glu Gln Ile Lys Arg Lys Tyr Arg
    130                 135                 140

gaa atc ttg ccc gaa tac gac gca atc att ttt tca gac tac ggc aaa 480
Glu Ile Leu Pro Glu Tyr Asp Ala Ile Ile Phe Ser Asp Tyr Gly Lys
145                 150                 155                 160

ggc ggc ctg tcg cac atc tcc gat atg atc gat tgg gcg aaa cac gaa 528
Gly Gly Leu Ser His Ile Ser Asp Met Ile Asp Trp Ala Lys His Glu
                165                 170                 175

ggc aaa acc gta tta atc gac ccc aaa ggc gac gat tac gaa aaa tac 576
Gly Lys Thr Val Leu Ile Asp Pro Lys Gly Asp Asp Tyr Glu Lys Tyr
            180                 185                 190

gcc ggc gca acc ctg att acg ccc aac cgc gcc gaa ttg aaa gaa gtg 624
Ala Gly Ala Thr Leu Ile Thr Pro Asn Arg Ala Glu Leu Lys Glu Val
        195                 200                 205

gtc ggc agc tgg aaa aac gaa aat gat ttg acc gaa aaa gcc caa aac 672
Val Gly Ser Trp Lys Asn Glu Asn Asp Leu Thr Glu Lys Ala Gln Asn
    210                 215                 220

ctg cgc cgc cac ctc gac ttg acc gct att tta ctg acc cga agc gaa 720
Leu Arg Arg His Leu Asp Leu Thr Ala Ile Leu Leu Thr Arg Ser Glu
225                 230                 235                 240

gaa ggc atg acc ttg ttc agc gaa ggc gaa ccc att tac cag ccc acc 768
Glu Gly Met Thr Leu Phe Ser Glu Gly Glu Pro Ile Tyr Gln Pro Thr
                245                 250                 255

cgc gcc caa gaa gtt tac gac gta tcc ggt gcg ggc gac acc gtc att 816
Arg Ala Gln Glu Val Tyr Asp Val Ser Gly Ala Gly Asp Thr Val Ile
            260                 265                 270

gcc gga atg ggc ttg ggt ttg gcg gca ggc tgc acc atg ccc gaa gcc 864
Ala Gly Met Gly Leu Gly Leu Ala Ala Gly Cys Thr Met Pro Glu Ala
        275                 280                 285

atg tac ctt gcc aat act gcg gcc ggg gtt gtc gtg gcg aaa ctc ggt 912
Met Tyr Leu Ala Asn Thr Ala Ala Gly Val Val Val Ala Lys Leu Gly
    290                 295                 300

acg gcg gtt tgc tcg ttt gcc gaa ttg acc aag gca ttg tca ggg caa 960
Thr Ala Val Cys Ser Phe Ala Glu Leu Thr Lys Ala Leu Ser Gly Gln
305                 310                 315                 320

tca aca atg 969

Ser Thr Met

<210> 52
<211> 323
<212> PRT
<213> Neisseria meningitidis

<400> 52
Met Ser Ala Lys Phe Gln Gln Glu Thr Leu Lys Ser Arg Phe Ala Gln
1               5                   10                  15

Ala Lys Val Leu Val Val Gly Asp Val Met Leu Asp Arg Tyr Trp Phe
            20                  25                  30

Gly Asp Val Ser Arg Ile Ser Pro Glu Ala Pro Val Pro Val Ala Lys
        35                  40                  45

Ile Gly Arg Ile Asp Gln Arg Ala Gly Gly Ala Ala Asn Val Ala Arg
    50                  55                  60

Asn Ile Ala Ser Leu Gly Gly Lys Val Gly Leu Leu Ser Val Thr Gly
65                  70                  75                  80

Asn Asp Glu Ala Ala Asp Ala Leu Asp Ala Leu Met Val Gln Asp Gly
                85                  90                  95

Val Ala Ser Tyr Leu Met Arg Asp Lys Gln Ile Ala Thr Thr Val Lys
            100                 105                 110

Leu Arg Val Val Ala Arg Asn Gln Gln Leu Ile Arg Leu Asp Phe Glu
        115                 120                 125

Glu His Pro Asn Arg Glu Val Leu Glu Gln Ile Lys Arg Lys Tyr Arg
    130                 135                 140

Glu Ile Leu Pro Glu Tyr Asp Ala Ile Ile Phe Ser Asp Tyr Gly Lys
145                 150                 155                 160

Gly Gly Leu Ser His Ile Ser Asp Met Ile Asp Trp Ala Lys His Glu
                165                 170                 175

Gly Lys Thr Val Leu Ile Asp Pro Lys Gly Asp Asp Tyr Glu Lys Tyr
            180                 185                 190

Ala Gly Ala Thr Leu Ile Thr Pro Asn Arg Ala Glu Leu Lys Glu Val
        195                 200                 205

Val Gly Ser Trp Lys Asn Glu Asn Asp Leu Thr Glu Lys Ala Gln Asn
    210                 215                 220

Leu Arg Arg His Leu Asp Leu Thr Ala Ile Leu Leu Thr Arg Ser Glu
225                 230                 235                 240

Glu Gly Met Thr Leu Phe Ser Glu Gly Glu Pro Ile Tyr Gln Pro Thr
                245                 250                 255

Arg Ala Gln Glu Val Tyr Asp Val Ser Gly Ala Gly Asp Thr Val Ile
            260                 265                 270

Ala Gly Met Gly Leu Gly Leu Ala Ala Gly Cys Thr Met Pro Glu Ala
        275                 280                 285

Met Tyr Leu Ala Asn Thr Ala Ala Gly Val Val Val Ala Lys Leu Gly
    290                 295                 300

Thr Ala Val Cys Ser Phe Ala Glu Leu Thr Lys Ala Leu Ser Gly Gln
305                 310                 315                 320

Ser Thr Met

<210> 53
<211> 864
<212> DNA
<213> Neisseria meningitidis

<220>
<221> CDS
<222> (1)..(864)

<400> 53
atg aaa gca aaa atc ctg act tcc gtt gca ctg ctt gcc tgt tcc ggc    48
Met Lys Ala Lys Ile Leu Thr Ser Val Ala Leu Leu Ala Cys Ser Gly
  1               5                  10                  15

agc ctg ttt gcc caa acg ctg gca acc gtc aac ggt cag aaa atc gac    96
Ser Leu Phe Ala Gln Thr Leu Ala Thr Val Asn Gly Gln Lys Ile Asp
             20                  25                  30

agt tcc gtc att gat gcg cag gtt gcc gca ttc cgt gcg gaa aac agc    144
Ser Ser Val Ile Asp Ala Gln Val Ala Ala Phe Arg Ala Glu Asn Ser
         35                  40                  45

cgt gcc gaa gac acg ccg caa ctg cgc caa tcc ctg ctg gaa aac gaa    192
Arg Ala Glu Asp Thr Pro Gln Leu Arg Gln Ser Leu Leu Glu Asn Glu
     50                  55                  60

gtg gtc aac acc gtg gtc gca cag gaa gtg aaa cgc ctg aaa ctc gac    240
Val Val Asn Thr Val Val Ala Gln Glu Val Lys Arg Leu Lys Leu Asp
 65                  70                  75                  80

cgg tcg gca gag ttt aaa aat gcg ctt gcc aaa ttg cgt gcc gaa gcg    288
Arg Ser Ala Glu Phe Lys Asn Ala Leu Ala Lys Leu Arg Ala Glu Ala
                 85                  90                  95

aaa aag tcg ggc gac gac aag aaa ccg tcc ttc aaa acc gtt tgg cag    336
Lys Lys Ser Gly Asp Asp Lys Lys Pro Ser Phe Lys Thr Val Trp Gln
            100                 105                 110

gcg gta aaa tat ggc ttg aac ggc gag gca tac gcg ctg cat atc gcc    384
Ala Val Lys Tyr Gly Leu Asn Gly Glu Ala Tyr Ala Leu His Ile Ala
        115                 120                 125

aaa acc caa ccg gtt tcc gag cag gaa gta aaa gcc gca tat gac aat    432
Lys Thr Gln Pro Val Ser Glu Gln Glu Val Lys Ala Ala Tyr Asp Asn
    130                 135                 140

atc agc ggt ttt tac aaa ggt acg cag gaa gtc cag ttg ggc gaa atc    480
Ile Ser Gly Phe Tyr Lys Gly Thr Gln Glu Val Gln Leu Gly Glu Ile
145                 150                 155                 160

ctg acc gac aag gaa gaa aat gca aaa aaa gcg gtt gcc gac ttg aag    528
Leu Thr Asp Lys Glu Glu Asn Ala Lys Lys Ala Val Ala Asp Leu Lys
                165                 170                 175

gcg aaa aaa ggt ttc gat gcc gtc ttg aaa caa tat tcc ctc aac gac    576
Ala Lys Lys Gly Phe Asp Ala Val Leu Lys Gln Tyr Ser Leu Asn Asp
            180                 185                 190

cgt acc aaa cag acc ggt gcg ccg gtc gga tat gtg ccg ctg aaa gat    624
Arg Thr Lys Gln Thr Gly Ala Pro Val Gly Tyr Val Pro Leu Lys Asp
        195                 200                 205

ttg gaa cag ggt gtt ccg ccg ctt tat cag gca att aag gac ttg aaa    672
Leu Glu Gln Gly Val Pro Pro Leu Tyr Gln Ala Ile Lys Asp Leu Lys
    210                 215                 220

aaa ggc gaa ttt acg gca acg ccg ctg aaa aac ggc gat ttc tac ggc    720
Lys Gly Glu Phe Thr Ala Thr Pro Leu Lys Asn Gly Asp Phe Tyr Gly
225                 230                 235                 240

gtt tat tat gtc aac gac agc cgc gag gta aaa gtg cct tct ttt gat    768
Val Tyr Tyr Val Asn Asp Ser Arg Glu Val Lys Val Pro Ser Phe Asp
                245                 250                 255

gaa atg aaa gga cag att gcg ggc aac ctt cag gcg gaa cgg att gac    816
Glu Met Lys Gly Gln Ile Ala Gly Asn Leu Gln Ala Glu Arg Ile Asp
            260                 265                 270

cgt gcc gtc ggt gca ctg ttg ggc aag gca aac atc aaa cct gca aaa    864
Arg Ala Val Gly Ala Leu Leu Gly Lys Ala Asn Ile Lys Pro Ala Lys
        275                 280                 285

<210> 54
<211> 288
<212> PRT
<213> Neisseria meningitidis

<400> 54
Met Lys Ala Lys Ile Leu Thr Ser Val Ala Leu Leu Ala Cys Ser Gly
1               5                   10                  15

Ser Leu Phe Ala Gln Thr Leu Ala Thr Val Asn Gly Gln Lys Ile Asp
            20                  25                  30

Ser Ser Val Ile Asp Ala Gln Val Ala Ala Phe Arg Ala Glu Asn Ser
        35                  40                  45

Arg Ala Glu Asp Thr Pro Gln Leu Arg Gln Ser Leu Leu Glu Asn Glu
    50                  55                  60

Val Val Asn Thr Val Val Ala Gln Glu Val Lys Arg Leu Lys Leu Asp
65                  70                  75                  80

Arg Ser Ala Glu Phe Lys Asn Ala Leu Ala Lys Leu Arg Ala Glu Ala
                85                  90                  95

Lys Lys Ser Gly Asp Asp Lys Lys Pro Ser Phe Lys Thr Val Trp Gln
            100                 105                 110

Ala Val Lys Tyr Gly Leu Asn Gly Glu Ala Tyr Ala Leu His Ile Ala
        115                 120                 125

Lys Thr Gln Pro Val Ser Glu Gln Glu Val Lys Ala Ala Tyr Asp Asn
    130                 135                 140

Ile Ser Gly Phe Tyr Lys Gly Thr Gln Glu Val Gln Leu Gly Glu Ile
145                 150                 155                 160

Leu Thr Asp Lys Glu Glu Asn Ala Lys Lys Ala Val Ala Asp Leu Lys
                165                 170                 175

Ala Lys Lys Gly Phe Asp Ala Val Leu Lys Gln Tyr Ser Leu Asn Asp
            180                 185                 190

Arg Thr Lys Gln Thr Gly Ala Pro Val Gly Tyr Val Pro Leu Lys Asp
        195                 200                 205

Leu Glu Gln Gly Val Pro Pro Leu Tyr Gln Ala Ile Lys Asp Leu Lys
    210                 215                 220

Lys Gly Glu Phe Thr Ala Thr Pro Leu Lys Asn Gly Asp Phe Tyr Gly
225                 230                 235                 240

Val Tyr Tyr Val Asn Asp Ser Arg Glu Val Lys Val Pro Ser Phe Asp
                245                 250                 255

Glu Met Lys Gly Gln Ile Ala Gly Asn Leu Gln Ala Glu Arg Ile Asp
            260                 265                 270

Arg Ala Val Gly Ala Leu Leu Gly Lys Ala Asn Ile Lys Pro Ala Lys
        275                 280                 285

<210> 55
<211> 1257
<212> DNA
<213> Neisseria meningitidis

<220>
<221> CDS
<222> (1)..(1257)

<400> 55
atg aaa cag acc gtc ctc aaa aat aac ctg caa aac ctg ctt gaa agc    48
Met Lys Gln Thr Val Leu Lys Asn Asn Leu Gln Asn Leu Leu Glu Ser
  1               5                  10                  15

gca gaa aat atc ctg ctg ctt caa ggc cct gtc ggc gat ttt ttt ctg    96
Ala Glu Asn Ile Leu Leu Leu Gln Gly Pro Val Gly Asp Phe Phe Leu
             20                  25                  30

cgc ctt gcc gac tgg ctg act gca aac ggc aaa acc gta cat aaa ttc   144
Arg Leu Ala Asp Trp Leu Thr Ala Asn Gly Lys Thr Val His Lys Phe
         35                  40                  45

aac ttt aat gca ggc gac gac tat ttt tat ccg ccc act caa gcg cat 192
Asn Phe Asn Ala Gly Asp Asp Tyr Phe Tyr Pro Pro Thr Gln Ala His
    50                  55                  60

acc gtt gtt ttt aac gac aac tac gat gcc ttt cct gag ttt ttg caa 240
Thr Val Val Phe Asn Asp Asn Tyr Asp Ala Phe Pro Glu Phe Leu Gln
65                  70                  75                  80

gaa tac atc act caa cat cac atc cag gcc gtt gtc tgc ttt ggc gac 288
Glu Tyr Ile Thr Gln His His Ile Gln Ala Val Val Cys Phe Gly Asp
                85                  90                  95

aca cgc cct tat cac gtc att gca aaa cgc att gca aac gaa aac caa 336
Thr Arg Pro Tyr His Val Ile Ala Lys Arg Ile Ala Asn Glu Asn Gln
            100                 105                 110

gcc agt ttc tgg gcg ttt gaa gaa ggc tat ttc cgc ccc tac tac atc 384
Ala Ser Phe Trp Ala Phe Glu Glu Gly Tyr Phe Arg Pro Tyr Tyr Ile
        115                 120                 125

acc tta gaa aaa gac ggc gtc aac gca ttt tcc ccg ttg ccg cgc cgt 432
Thr Leu Glu Lys Asp Gly Val Asn Ala Phe Ser Pro Leu Pro Arg Arg
    130                 135                 140

gcc gac ttt ttt ctt gaa caa ttc cct aag ctt gcc cag caa gaa tat 480
Ala Asp Phe Phe Leu Glu Gln Phe Pro Lys Leu Ala Gln Gln Glu Tyr
145                 150                 155                 160

aaa gcg cca acg ccg gta cac ggc ggt ttt acg ccc atg gca aaa aac 528
Lys Ala Pro Thr Pro Val His Gly Gly Phe Thr Pro Met Ala Lys Asn
                165                 170                 175

gct atc cgt tac tat atc gag ttg ttc cgc aat cta cgc aaa tac ccc 576
Ala Ile Arg Tyr Tyr Ile Glu Leu Phe Arg Asn Leu Arg Lys Tyr Pro
            180                 185                 190

gac tac atc cac cac cgc gca ccc aat gcc ggc cat tac ctc aaa ccg 624
Asp Tyr Ile His His Arg Ala Pro Asn Ala Gly His Tyr Leu Lys Pro
        195                 200                 205

tgg tcg ctc tcc atc ctc aag cgt ttg aac tac tat att gaa gac atc 672
Trp Ser Leu Ser Ile Leu Lys Arg Leu Asn Tyr Tyr Ile Glu Asp Ile
    210                 215                 220

caa atc gcc aaa cgt gtg gaa gca ggc aaa tac ggc aag ttt ttt att 720
Gln Ile Ala Lys Arg Val Glu Ala Gly Lys Tyr Gly Lys Phe Phe Ile
225                 230                 235                 240

gtt ccc ttg cag gta ttc aac gac agc caa gtc cgt atc cat tgc gac 768
Val Pro Leu Gln Val Phe Asn Asp Ser Gln Val Arg Ile His Cys Asp
                245                 250                 255

ttt ccc agc gtc cgc agc ttc ctg ctc cat gtt ttg agt tca ttt gcc 816
Phe Pro Ser Val Arg Ser Phe Leu Leu His Val Leu Ser Ser Phe Ala
            260                 265                 270

gaa cac gcg cct gcc gat acc aac atc atc atc aag cat cat cct atg 864
Glu His Ala Pro Ala Asp Thr Asn Ile Ile Ile Lys His His Pro Met
        275                 280                 285

gac cgc ggc ttt atc gac tac tgg cgc gac att aaa cgc ttt atc aaa 912
Asp Arg Gly Phe Ile Asp Tyr Trp Arg Asp Ile Lys Arg Phe Ile Lys
    290                 295                 300

gaa cac ccc gaa ctc aaa ggc cgt gtg att tat gtc cat gat gtc ccc 960
Glu His Pro Glu Leu Lys Gly Arg Val Ile Tyr Val His Asp Val Pro
305                 310                 315                 320

ctg ccc gtt ttc ctg cgc cac ggt ctc ggc atg gtt acc atc aac agc 1008
Leu Pro Val Phe Leu Arg His Gly Leu Gly Met Val Thr Ile Asn Ser
                325                 330                 335

acc agc ggc ctg tcc gga ctg att cac aat atg cca gtt aag gtt ctc 1056
Thr Ser Gly Leu Ser Gly Leu Ile His Asn Met Pro Val Lys Val Leu
            340                 345                 350

ggc cgt gcc tat tat gat att ccc ggc att act gac caa aat acc ttg 1104
Gly Arg Ala Tyr Tyr Asp Ile Pro Gly Ile Thr Asp Gln Asn Thr Leu
        355                 360                 365

gca gaa ttt tgg aat cat ccg aca ccg cct gac aaa gag ctg ttc cat 1152
Ala Glu Phe Trp Asn His Pro Thr Pro Pro Asp Lys Glu Leu Phe His
    370                 375                 380

gcc tac cga atg tac cac ctc aac gtg acc caa att aac ggc aac ttc 1200
Ala Tyr Arg Met Tyr His Leu Asn Val Thr Gln Ile Asn Gly Asn Phe
385                 390                 395                 400

tac agt cag gtc ttt ttt cct aac aaa aac aca tcc gac tct tcc aca 1248
Tyr Ser Gln Val Phe Phe Pro Asn Lys Asn Thr Ser Asp Ser Ser Thr
                405                 410                 415

cca aca acc 1257
Pro Thr Thr

<210> 56
<211> 419
<212> PRT
<213> Neisseria meningitidis

<400> 56
Met Lys Gln Thr Val Leu Lys Asn Asn Leu Gln Asn Leu Leu Glu Ser
1               5                   10                  15

Ala Glu Asn Ile Leu Leu Leu Gln Gly Pro Val Gly Asp Phe Phe Leu
            20                  25                  30

Arg Leu Ala Asp Trp Leu Thr Ala Asn Gly Lys Thr Val His Lys Phe
        35                  40                  45

Asn Phe Asn Ala Gly Asp Asp Tyr Phe Tyr Pro Pro Thr Gln Ala His
    50                  55                  60

Thr Val Val Phe Asn Asp Asn Tyr Asp Ala Phe Pro Glu Phe Leu Gln
65                  70                  75                  80

Glu Tyr Ile Thr Gln His His Ile Gln Ala Val Val Cys Phe Gly Asp
                85                  90                  95

Thr Arg Pro Tyr His Val Ile Ala Lys Arg Ile Ala Asn Glu Asn Gln
            100                 105                 110

Ala Ser Phe Trp Ala Phe Glu Glu Gly Tyr Phe Arg Pro Tyr Tyr Ile
        115                 120                 125

Thr Leu Glu Lys Asp Gly Val Asn Ala Phe Ser Pro Leu Pro Arg Arg
    130                 135                 140

Ala Asp Phe Phe Leu Glu Gln Phe Pro Lys Leu Ala Gln Gln Glu Tyr
145                 150                 155                 160

Lys Ala Pro Thr Pro Val His Gly Gly Phe Thr Pro Met Ala Lys Asn
                165                 170                 175

Ala Ile Arg Tyr Tyr Ile Glu Leu Phe Arg Asn Leu Arg Lys Tyr Pro
            180                 185                 190

Asp Tyr Ile His His Arg Ala Pro Asn Ala Gly His Tyr Leu Lys Pro
        195                 200                 205

Trp Ser Leu Ser Ile Leu Lys Arg Leu Asn Tyr Tyr Ile Glu Asp Ile
    210                 215                 220

Gln Ile Ala Lys Arg Val Glu Ala Gly Lys Tyr Gly Lys Phe Phe Ile
225                 230                 235                 240

Val Pro Leu Gln Val Phe Asn Asp Ser Gln Val Arg Ile His Cys Asp
                245                 250                 255

Phe Pro Ser Val Arg Ser Phe Leu Leu His Val Leu Ser Ser Phe Ala
            260                 265                 270

Glu His Ala Pro Ala Asp Thr Asn Ile Ile Ile Lys His His Pro Met
        275                 280                 285

Asp Arg Gly Phe Ile Asp Tyr Trp Arg Asp Ile Lys Arg Phe Ile Lys
    290                 295                 300

Glu His Pro Glu Leu Lys Gly Arg Val Ile Tyr Val His Asp Val Pro
305                 310                 315                 320

Leu Pro Val Phe Leu Arg His Gly Leu Gly Met Val Thr Ile Asn Ser
                325                 330                 335

Thr Ser Gly Leu Ser Gly Leu Ile His Asn Met Pro Val Lys Val Leu
            340                 345                 350

Gly Arg Ala Tyr Tyr Asp Ile Pro Gly Ile Thr Asp Gln Asn Thr Leu
        355                 360                 365

Ala Glu Phe Trp Asn His Pro Thr Pro Pro Asp Lys Glu Leu Phe His
    370                 375                 380

Ala Tyr Arg Met Tyr His Leu Asn Val Thr Gln Ile Asn Gly Asn Phe
385                 390                 395                 400

Tyr Ser Gln Val Phe Phe Pro Asn Lys Asn Thr Ser Asp Ser Ser Thr
                405                 410                 415

Pro Thr Thr

<210> 57
<211> 1407
<212> DNA
<213> Neisseria meningitidis

<220>

<221> CDS
<222> (1)..(1407)

<400> 57
atg acc tcc aca ttc ccc cgc cgc ctc gcc cgc aaa atc cgc caa acc     48
Met Thr Ser Thr Phe Pro Arg Arg Leu Ala Arg Lys Ile Arg Gln Thr
1               5                   10                  15

cgc cgc ctg tcg cgc aaa agc atc gcc ttt ctg ttc ctt ttg gca ggt     96
Arg Arg Leu Ser Arg Lys Ser Ile Ala Phe Leu Phe Leu Leu Ala Gly
            20                  25                  30

tcg gca ctc gtc gcc ctg acc gcg ctg ttt ttt gcc cat ctt gcc gat     144
Ser Ala Leu Val Ala Leu Thr Ala Leu Phe Phe Ala His Leu Ala Asp
        35                  40                  45

ttt gcg ctg gaa ctg aac gcc aaa ctg gtt caa caa tac ccg tgg ttc     192
Phe Ala Leu Glu Leu Asn Ala Lys Leu Val Gln Gln Tyr Pro Trp Phe
    50                  55                  60

gcg tgg gtc gcg ctt cct ttg ggt tta ccg ctt att gcg tgg ctc aca     240
Ala Trp Val Ala Leu Pro Leu Gly Leu Pro Leu Ile Ala Trp Leu Thr
65                  70                  75                  80

cgc aaa ttc gcc ccc ttc acc gcc ggc agc ggc atc ccg cag gtc atc     288
Arg Lys Phe Ala Pro Phe Thr Ala Gly Ser Gly Ile Pro Gln Val Ile
                85                  90                  95

gcc tca ctg tcg ctg ccc tac ggc gca cag aaa acg cgg ctg atc cgc     336
Ala Ser Leu Ser Leu Pro Tyr Gly Ala Gln Lys Thr Arg Leu Ile Arg
            100                 105                 110

ctc ggg cag acg ctg ctg aag att ccg cta acc ttt ttg ggt atg ctg     384
Leu Gly Gln Thr Leu Leu Lys Ile Pro Leu Thr Phe Leu Gly Met Leu
        115                 120                 125

ttc ggc gcg tcc atc gga cgc gaa ggt ccg tcc gtg cag gtc ggc gcg     432
Phe Gly Ala Ser Ile Gly Arg Glu Gly Pro Ser Val Gln Val Gly Ala
    130                 135                 140

gca gtg atg ggc gcg tgg ggc gcg tgg tgc aag aaa cac ggc ttg gca     480
Ala Val Met Gly Ala Trp Gly Ala Trp Cys Lys Lys His Gly Leu Ala
145                 150                 155                 160

ttc aaa ggg atg cag gaa aac gat ttg atg gcg gcg ggc gcg gcg ggc     528
Phe Lys Gly Met Gln Glu Asn Asp Leu Met Ala Ala Gly Ala Ala Gly
                165                 170                 175

ggt ttg gca gcc gcg ttc aac gcg ccg ctg gcg ggc gtg att ttc gcc 576
Gly Leu Ala Ala Ala Phe Asn Ala Pro Leu Ala Gly Val Ile Phe Ala
            180                 185                 190

att gag gaa ctc ggg cgc ggc atc atg ttg cgc tgg gag agg caa att 624
Ile Glu Glu Leu Gly Arg Gly Ile Met Leu Arg Trp Glu Arg Gln Ile
        195                 200                 205

ctt ttg ggc gtg ctc gcc tcc ggt ttc ata cag gtc gcc att cag ggc 672
Leu Leu Gly Val Leu Ala Ser Gly Phe Ile Gln Val Ala Ile Gln Gly
    210                 215                 220

aac aac ccg tat ttt tcc ggc ttc aac ggc ggc gta ttg gaa cat atc 720
Asn Asn Pro Tyr Phe Ser Gly Phe Asn Gly Gly Val Leu Glu His Ile
225                 230                 235                 240

ttt ctg tgg gtc gca ctg tcc ggc ctg gtt tgc ggc gcg gcg ggc ggg 768
Phe Leu Trp Val Ala Leu Ser Gly Leu Val Cys Gly Ala Ala Gly Gly
                245                 250                 255

ctg ttc gga cgt ttg ctc tat cgc ggt gcg gcg gcg ttt gca ccg cgc 816
Leu Phe Gly Arg Leu Leu Tyr Arg Gly Ala Ala Ala Phe Ala Pro Arg
            260                 265                 270

aag ata cgc ggc ttc atc cgc aac cgt ccg ctg ctg ctg gcg gca ctg 864
Lys Ile Arg Gly Phe Ile Arg Asn Arg Pro Leu Leu Leu Ala Ala Leu
        275                 280                 285

atg ggg ctg ctg ctc gcc ctg ctc ggc acg ttc tac caa ggc aaa acc 912
Met Gly Leu Leu Leu Ala Leu Leu Gly Thr Phe Tyr Gln Gly Lys Thr
    290                 295                 300

tac ggc acc ggc tac cac gaa gcc gcc caa gcc ctg cac ggc atc tac 960
Tyr Gly Thr Gly Tyr His Glu Ala Ala Gln Ala Leu His Gly Ile Tyr
305                 310                 315                 320

gaa gcc ccc ttc gga ctc gcc gcc gcc aaa tgg ctc gcc acc gta ttc 1008
Glu Ala Pro Phe Gly Leu Ala Ala Ala Lys Trp Leu Ala Thr Val Phe
                325                 330                 335

agc tat tgg gca ggc gtt ccg ggc ggc att ttc act ccc tcg ctg acc 1056
Ser Tyr Trp Ala Gly Val Pro Gly Gly Ile Phe Thr Pro Ser Leu Thr
            340                 345                 350

ata ggc gcg gtt ttg ggc gag cat atc gcc gcc atc gcc gac ata tcg 1104
Ile Gly Ala Val Leu Gly Glu His Ile Ala Ala Ile Ala Asp Ile Ser
        355                 360                 365

cag ggt gca aac atc atc gtc ctc atc tgc atg gcg gca ttt ctg gcg 1152
Gln Gly Ala Asn Ile Ile Val Leu Ile Cys Met Ala Ala Phe Leu Ala
    370                 375                 380

ggc gcg aca caa tcc ccg att act tcc gcc gtc gtc gtc atg gaa atg 1200
Gly Ala Thr Gln Ser Pro Ile Thr Ser Ala Val Val Val Met Glu Met
385                 390                 395                 400

acg ggc gga caa agc ctg ctg ttt tgg atg ctg att gcc tgc att ttc 1248
Thr Gly Gly Gln Ser Leu Leu Phe Trp Met Leu Ile Ala Cys Ile Phe
                405                 410                 415

gcc tcg cag gtt tcg cgc cag ttt tcg ccg cgt ccg ttc tac cac gca 1296
Ala Ser Gln Val Ser Arg Gln Phe Ser Pro Arg Pro Phe Tyr His Ala
            420                 425                 430

tcg gga atg cgc ttc cgc cag cgc gtg ctt caa gaa acc gcc gcc caa 1344
Ser Gly Met Arg Phe Arg Gln Arg Val Leu Gln Glu Thr Ala Ala Gln
        435                 440                 445

acc ggc aat gcg ccc gca aga ccg caa aca gca aac agc aaa acg gga 1392
Thr Gly Asn Ala Pro Ala Arg Pro Gln Thr Ala Asn Ser Lys Thr Gly
    450                 455                 460

atg ccg tct gaa aat 1407
Met Pro Ser Glu Asn
465

<210> 58
<211> 469
<212> PRT
<213> Neisseria meningitidis

<400> 58
Met Thr Ser Thr Phe Pro Arg Arg Leu Ala Arg Lys Ile Arg Gln Thr
1               5                   10                  15

Arg Arg Leu Ser Arg Lys Ser Ile Ala Phe Leu Phe Leu Leu Ala Gly
            20                  25                  30

Ser Ala Leu Val Ala Leu Thr Ala Leu Phe Phe Ala His Leu Ala Asp
        35                  40                  45

Phe Ala Leu Glu Leu Asn Ala Lys Leu Val Gln Gln Tyr Pro Trp Phe
    50                  55                  60

Ala Trp Val Ala Leu Pro Leu Gly Leu Pro Leu Ile Ala Trp Leu Thr

65                  70                  75                  80

Arg Lys Phe Ala Pro Phe Thr Ala Gly Ser Gly Ile Pro Gln Val Ile
                85                  90                  95

Ala Ser Leu Ser Leu Pro Tyr Gly Ala Gln Lys Thr Arg Leu Ile Arg
            100                 105                 110

Leu Gly Gln Thr Leu Leu Lys Ile Pro Leu Thr Phe Leu Gly Met Leu
        115                 120                 125

Phe Gly Ala Ser Ile Gly Arg Glu Gly Pro Ser Val Gln Val Gly Ala
    130                 135                 140

Ala Val Met Gly Ala Trp Gly Ala Trp Cys Lys Lys His Gly Leu Ala
145                 150                 155                 160

Phe Lys Gly Met Gln Glu Asn Asp Leu Met Ala Ala Gly Ala Ala Gly
                165                 170                 175

Gly Leu Ala Ala Ala Phe Asn Ala Pro Leu Ala Gly Val Ile Phe Ala
            180                 185                 190

Ile Glu Glu Leu Gly Arg Gly Ile Met Leu Arg Trp Glu Arg Gln Ile
        195                 200                 205

Leu Leu Gly Val Leu Ala Ser Gly Phe Ile Gln Val Ala Ile Gln Gly
    210                 215                 220

Asn Asn Pro Tyr Phe Ser Gly Phe Asn Gly Gly Val Leu Glu His Ile
225                 230                 235                 240

Phe Leu Trp Val Ala Leu Ser Gly Leu Val Cys Gly Ala Ala Gly Gly
                245                 250                 255

Leu Phe Gly Arg Leu Leu Tyr Arg Gly Ala Ala Ala Phe Ala Pro Arg
            260                 265                 270

Lys Ile Arg Gly Phe Ile Arg Asn Arg Pro Leu Leu Leu Ala Ala Leu
        275                 280                 285

Met Gly Leu Leu Leu Ala Leu Leu Gly Thr Phe Tyr Gln Gly Lys Thr
    290                 295                 300

Tyr Gly Thr Gly Tyr His Glu Ala Ala Gln Ala Leu His Gly Ile Tyr
305                 310                 315                 320

Glu Ala Pro Phe Gly Leu Ala Ala Ala Lys Trp Leu Ala Thr Val Phe

                325                 330                 335

Ser Tyr Trp Ala Gly Val Pro Gly Gly Ile Phe Thr Pro Ser Leu Thr
            340                 345                 350

Ile Gly Ala Val Leu Gly Glu His Ile Ala Ala Ile Ala Asp Ile Ser
        355                 360                 365

Gln Gly Ala Asn Ile Ile Val Leu Ile Cys Met Ala Ala Phe Leu Ala
    370                 375                 380

Gly Ala Thr Gln Ser Pro Ile Thr Ser Ala Val Val Val Met Glu Met
385                 390                 395                 400

Thr Gly Gly Gln Ser Leu Leu Phe Trp Met Leu Ile Ala Cys Ile Phe
                405                 410                 415

Ala Ser Gln Val Ser Arg Gln Phe Ser Pro Arg Pro Phe Tyr His Ala
            420                 425                 430

Ser Gly Met Arg Phe Arg Gln Arg Val Leu Gln Glu Thr Ala Ala Gln
        435                 440                 445

Thr Gly Asn Ala Pro Ala Arg Pro Gln Thr Ala Asn Ser Lys Thr Gly
    450                 455                 460

Met Pro Ser Glu Asn
465

<210> 59
<211> 423
<212> DNA
<213> Neisseria meningitidis

<220>
<221> CDS
<222> (1)..(423)

<400> 59
atg aca caa gaa acc gct ttg ggc gca gca ctg aaa tcc gcc gtc caa 48
Met Thr Gln Glu Thr Ala Leu Gly Ala Ala Leu Lys Ser Ala Val Gln
1               5                   10                  15

act atg agc aaa aag aaa cag aca gaa atg att gcc gac cac atc tac 96
Thr Met Ser Lys Lys Lys Gln Thr Glu Met Ile Ala Asp His Ile Tyr
            20                  25                  30

ggc aaa tat gat gta ttc aaa cga ttt aaa ccg ttg gca ctc ggt atc     144
Gly Lys Tyr Asp Val Phe Lys Arg Phe Lys Pro Leu Ala Leu Gly Ile
        35                  40                  45

gat cag gat ttg att gcc gct ttg ccc caa tac gat gcc gca ctg att     192
Asp Gln Asp Leu Ile Ala Ala Leu Pro Gln Tyr Asp Ala Ala Leu Ile
    50                  55                  60

gca cgc gtc ctc gcc aac cac tgc cgc cgt ccg cgc tat ctg aaa gcc     240
Ala Arg Val Leu Ala Asn His Cys Arg Arg Pro Arg Tyr Leu Lys Ala
65                  70                  75                  80

ttg gcg cgc ggt ggc aaa cgt ttc gat ttg aac aac cgt ttc aaa ggc     288
Leu Ala Arg Gly Gly Lys Arg Phe Asp Leu Asn Asn Arg Phe Lys Gly
                85                  90                  95

gaa gtt acc ccc gaa gaa cag gca atc gcg caa aac cat cct ttc gtg     336
Glu Val Thr Pro Glu Glu Gln Ala Ile Ala Gln Asn His Pro Phe Val
            100                 105                 110

cag cag gct ttg caa cag cag tcc gcc caa gct gtc gcc gaa acg ccg     384
Gln Gln Ala Leu Gln Gln Gln Ser Ala Gln Ala Val Ala Glu Thr Pro
        115                 120                 125

tct gtt gaa gcc gaa gca gcc gaa tct tcc aca aca gaa                 423
Ser Val Glu Ala Glu Ala Ala Glu Ser Ser Thr Thr Glu
    130                 135                 140


<210> 60
<211> 141
<212> PRT
<213> Neisseria meningitidis

<400> 60
Met Thr Gln Glu Thr Ala Leu Gly Ala Ala Leu Lys Ser Ala Val Gln
1               5                   10                  15

Thr Met Ser Lys Lys Lys Gln Thr Glu Met Ile Ala Asp His Ile Tyr
            20                  25                  30

Gly Lys Tyr Asp Val Phe Lys Arg Phe Lys Pro Leu Ala Leu Gly Ile
        35                  40                  45

Asp Gln Asp Leu Ile Ala Ala Leu Pro Gln Tyr Asp Ala Ala Leu Ile
    50                  55                  60

Ala Arg Val Leu Ala Asn His Cys Arg Arg Pro Arg Tyr Leu Lys Ala
65                  70                  75                  80

Leu Ala Arg Gly Gly Lys Arg Phe Asp Leu Asn Asn Arg Phe Lys Gly
                85                  90                  95

Glu Val Thr Pro Glu Glu Gln Ala Ile Ala Gln Asn His Pro Phe Val
            100                 105                 110

Gln Gln Ala Leu Gln Gln Gln Ser Ala Gln Ala Val Ala Glu Thr Pro
        115                 120                 125

Ser Val Glu Ala Glu Ala Ala Glu Ser Ser Thr Thr Glu
    130                 135                 140

<210> 61
<211> 1377
<212> DNA
<213> Neisseria meningitidis

<220>
<221> CDS
<222> (1)..(1377)

<400> 61
atg ttc gct ttc aaa tcc tta ctc gat atg ccg cgc ggt gag gca ctt     48
Met Phe Ala Phe Lys Ser Leu Leu Asp Met Pro Arg Gly Glu Ala Leu
  1               5                  10                  15

gcc gtc gtc gtc gct ctg att gcc gcg atg ggc tat acc atc att tca     96
Ala Val Val Val Ala Leu Ile Ala Ala Met Gly Tyr Thr Ile Ile Ser
             20                  25                  30

ttg gag tgg ctg ccg cat atg tcc att att gcc gcc atc gtc gtg ctg    144
Leu Glu Trp Leu Pro His Met Ser Ile Ile Ala Ala Ile Val Val Leu
         35                  40                  45

att ttg tac ggc ttg gcg cgc ggt ttg aaa tac aac gat atg cag cag    192
Ile Leu Tyr Gly Leu Ala Arg Gly Leu Lys Tyr Asn Asp Met Gln Gln
     50                  55                  60

ggc atg ata ggc gcg ttg aat cag ggt atg ggc gcg att tac ctg ttt    240
Gly Met Ile Gly Ala Leu Asn Gln Gly Met Gly Ala Ile Tyr Leu Phe
 65                  70                  75                  80

ttc ttc atc ggg ctg atg gtc agc gcg ctg atg atg agc ggc gcg att    288

Phe Phe Ile Gly Leu Met Val Ser Ala Leu Met Met Ser Gly Ala Ile
                85                  90                  95

ccg acg ctg atg tat tac ggt ttc gga ctg att tcc ccg act tat ttt 336
Pro Thr Leu Met Tyr Tyr Gly Phe Gly Leu Ile Ser Pro Thr Tyr Phe
            100                 105                 110

tat ttt tcc gcc ttc gcg ctg tgt tcc gtc atc ggc gtg tcc atc ggc 384
Tyr Phe Ser Ala Phe Ala Leu Cys Ser Val Ile Gly Val Ser Ile Gly
        115                 120                 125

agc agc ctg acc acc tgc gcc act gtc ggc gtt gcc ttt atg ggt atg 432
Ser Ser Leu Thr Thr Cys Ala Thr Val Gly Val Ala Phe Met Gly Met
    130                 135                 140

gcg gcg gcg ttt cag gcc gat atg gcg atg acg gcg ggc gcg att gtt 480
Ala Ala Ala Phe Gln Ala Asp Met Ala Met Thr Ala Gly Ala Ile Val
145                 150                 155                 160

tcg ggc gca ttt ttt ggc gac aaa atg tcc ccg ctt tcc gat acg acg 528
Ser Gly Ala Phe Phe Gly Asp Lys Met Ser Pro Leu Ser Asp Thr Thr
                165                 170                 175

ggc att tcc gcg tcc att gtc ggt atc gac ctg ttt gaa cac atc aaa 576
Gly Ile Ser Ala Ser Ile Val Gly Ile Asp Leu Phe Glu His Ile Lys
            180                 185                 190

aat atg atg tac acc acc att ccc gcg tgg ctc atc agt gcg gca ctg 624
Asn Met Met Tyr Thr Thr Ile Pro Ala Trp Leu Ile Ser Ala Ala Leu
        195                 200                 205

atg ctg tgg ctt ctt ccc agc gtc gcc gcg cag gat ttg aac agc gtc 672
Met Leu Trp Leu Leu Pro Ser Val Ala Ala Gln Asp Leu Asn Ser Val
    210                 215                 220

gaa tcc ttc cgc agc cag ctt gaa gcc acg gga ttg gtg cac tgc tat 720
Glu Ser Phe Arg Ser Gln Leu Glu Ala Thr Gly Leu Val His Cys Tyr
225                 230                 235                 240

tcg ctg att ccg ttt gcg ctg ttg gtc gtt ttg gca ttg atg cgc gtc 768
Ser Leu Ile Pro Phe Ala Leu Leu Val Val Leu Ala Leu Met Arg Val
                245                 250                 255

aat gcc gtg gtc gct atg ctc ttt acc gtc att gcc gcc gtt gcc gta 816
Asn Ala Val Val Ala Met Leu Phe Thr Val Ile Ala Ala Val Ala Val
            260                 265                 270

acg tat ctg cac agc acg ccc gat ctg cgt cag ctc ggc gcg tgg ttt 864

Thr Tyr Leu His Ser Thr Pro Asp Leu Arg Gln Leu Gly Ala Trp Phe
        275                 280                 285

tac ggc ggc tac aaa ctc gaa ggc gaa gcg ttt aaa gac att gcc aaa 912
Tyr Gly Gly Tyr Lys Leu Glu Gly Glu Ala Phe Lys Asp Ile Ala Lys
    290                 295                 300

ctc att tct cgc ggc ggt ttg gaa agt atg ttt ttc acg cag acc atc 960
Leu Ile Ser Arg Gly Gly Leu Glu Ser Met Phe Phe Thr Gln Thr Ile
305                 310                 315                 320

gtg att ctt ggg atg agc ctt ggc ggg ctg ctg ttt gca ctg ggc gcg 1008
Val Ile Leu Gly Met Ser Leu Gly Gly Leu Leu Phe Ala Leu Gly Ala
                325                 330                 335

att cct tcc ctg ctg gat gcc gtc cgc agc ttt ttg acg aat gcc ggg 1056
Ile Pro Ser Leu Leu Asp Ala Val Arg Ser Phe Leu Thr Asn Ala Gly
            340                 345                 350

cgt gcc aca ttc agc gtt gcc atg act tcg gtc ggg gtt aat ttc ctg 1104
Arg Ala Thr Phe Ser Val Ala Met Thr Ser Val Gly Val Asn Phe Leu
        355                 360                 365

atc ggc gag caa tat ttg agt att ttg ttg tcg ggt gaa acg ttc aaa 1152
Ile Gly Glu Gln Tyr Leu Ser Ile Leu Leu Ser Gly Glu Thr Phe Lys
    370                 375                 380

cct gtt tac gat aag ctc ggt ctg cat tcg cgc aat ctg tcg cgg acg 1200
Pro Val Tyr Asp Lys Leu Gly Leu His Ser Arg Asn Leu Ser Arg Thr
385                 390                 395                 400

ctg gaa gat gcg ggg acg gtc atc aac ccg ctc gta ccg tgg agc gta 1248
Leu Glu Asp Ala Gly Thr Val Ile Asn Pro Leu Val Pro Trp Ser Val
                405                 410                 415

tgc ggc gtg ttc atc agc cac gcg ctg ggc gtg ccg gtt tgg gaa tat 1296
Cys Gly Val Phe Ile Ser His Ala Leu Gly Val Pro Val Trp Glu Tyr
            420                 425                 430

ctg ccg tat gcc ttt ttc tgc tat ttg agt ttg gct ttg acc ctg tta 1344
Leu Pro Tyr Ala Phe Phe Cys Tyr Leu Ser Leu Ala Leu Thr Leu Leu
        435                 440                 445

ttc ggt tgg acg ggg ctg act ttg agc aaa aaa 1377
Phe Gly Trp Thr Gly Leu Thr Leu Ser Lys Lys
    450                 455

<210> 62
<211> 459
<212> PRT
<213> Neisseria meningitidis

<400> 62
Met Phe Ala Phe Lys Ser Leu Leu Asp Met Pro Arg Gly Glu Ala Leu
1               5                   10                  15

Ala Val Val Val Ala Leu Ile Ala Ala Met Gly Tyr Thr Ile Ile Ser
            20                  25                  30

Leu Glu Trp Leu Pro His Met Ser Ile Ile Ala Ala Ile Val Val Leu
        35                  40                  45

Ile Leu Tyr Gly Leu Ala Arg Gly Leu Lys Tyr Asn Asp Met Gln Gln
    50                  55                  60

Gly Met Ile Gly Ala Leu Asn Gln Gly Met Gly Ala Ile Tyr Leu Phe
65                  70                  75                  80

Phe Phe Ile Gly Leu Met Val Ser Ala Leu Met Met Ser Gly Ala Ile
                85                  90                  95

Pro Thr Leu Met Tyr Tyr Gly Phe Gly Leu Ile Ser Pro Thr Tyr Phe
            100                 105                 110

Tyr Phe Ser Ala Phe Ala Leu Cys Ser Val Ile Gly Val Ser Ile Gly
        115                 120                 125

Ser Ser Leu Thr Thr Cys Ala Thr Val Gly Val Ala Phe Met Gly Met
    130                 135                 140

Ala Ala Ala Phe Gln Ala Asp Met Ala Met Thr Ala Gly Ala Ile Val
145                 150                 155                 160

Ser Gly Ala Phe Phe Gly Asp Lys Met Ser Pro Leu Ser Asp Thr Thr
                165                 170                 175

Gly Ile Ser Ala Ser Ile Val Gly Ile Asp Leu Phe Glu His Ile Lys
            180                 185                 190

Asn Met Met Tyr Thr Thr Ile Pro Ala Trp Leu Ile Ser Ala Ala Leu
        195                 200                 205

Met Leu Trp Leu Leu Pro Ser Val Ala Ala Gln Asp Leu Asn Ser Val
    210                 215                 220

Glu Ser Phe Arg Ser Gln Leu Glu Ala Thr Gly Leu Val His Cys Tyr
225                 230                 235                 240

Ser Leu Ile Pro Phe Ala Leu Leu Val Val Leu Ala Leu Met Arg Val
                245                 250                 255

Asn Ala Val Val Ala Met Leu Phe Thr Val Ile Ala Ala Val Ala Val
            260                 265                 270

Thr Tyr Leu His Ser Thr Pro Asp Leu Arg Gln Leu Gly Ala Trp Phe
        275                 280                 285

Tyr Gly Gly Tyr Lys Leu Glu Gly Glu Ala Phe Lys Asp Ile Ala Lys
    290                 295                 300

Leu Ile Ser Arg Gly Gly Leu Glu Ser Met Phe Phe Thr Gln Thr Ile
305                 310                 315                 320

Val Ile Leu Gly Met Ser Leu Gly Gly Leu Leu Phe Ala Leu Gly Ala
                325                 330                 335

Ile Pro Ser Leu Leu Asp Ala Val Arg Ser Phe Leu Thr Asn Ala Gly
            340                 345                 350

Arg Ala Thr Phe Ser Val Ala Met Thr Ser Val Gly Val Asn Phe Leu
        355                 360                 365

Ile Gly Glu Gln Tyr Leu Ser Ile Leu Leu Ser Gly Glu Thr Phe Lys
    370                 375                 380

Pro Val Tyr Asp Lys Leu Gly Leu His Ser Arg Asn Leu Ser Arg Thr
385                 390                 395                 400

Leu Glu Asp Ala Gly Thr Val Ile Asn Pro Leu Val Pro Trp Ser Val
                405                 410                 415

Cys Gly Val Phe Ile Ser His Ala Leu Gly Val Pro Val Trp Glu Tyr
            420                 425                 430

Leu Pro Tyr Ala Phe Phe Cys Tyr Leu Ser Leu Ala Leu Thr Leu Leu
        435                 440                 445

Phe Gly Trp Thr Gly Leu Thr Leu Ser Lys Lys
    450                 455

<210> 63

<211> 1098
<212> DNA
<213> Neisseria meningitidis

<220>
<221> CDS
<222> (1)..(1098)

<400> 63
atg gca ggc aac act ttc gga caa ctc ttc acc gtt acc acc ttc ggc     48
Met Ala Gly Asn Thr Phe Gly Gln Leu Phe Thr Val Thr Thr Phe Gly
1               5                   10                  15

gaa agc cac ggc gcg ggt ttg ggc tgt atc atc gac ggc tgc ccg ccc     96
Glu Ser His Gly Ala Gly Leu Gly Cys Ile Ile Asp Gly Cys Pro Pro
            20                  25                  30

ggc ttg gaa tta agc gaa gcg gat atc caa ttt gac ctc gac cga cgc     144
Gly Leu Glu Leu Ser Glu Ala Asp Ile Gln Phe Asp Leu Asp Arg Arg
        35                  40                  45

aaa ccc ggc acc agc cgc cac gtt acc caa cgc cgc gaa gcc gac caa     192
Lys Pro Gly Thr Ser Arg His Val Thr Gln Arg Arg Glu Ala Asp Gln
    50                  55                  60

gtc gaa atc ctc tcc ggc gta ttc gaa ggc aaa acc acc ggc acg ccc     240
Val Glu Ile Leu Ser Gly Val Phe Glu Gly Lys Thr Thr Gly Thr Pro
65                  70                  75                  80

atc gcc ctc tta atc cgc aat acc gac cag cgc agc aaa gac tac ggc     288
Ile Ala Leu Leu Ile Arg Asn Thr Asp Gln Arg Ser Lys Asp Tyr Gly
                85                  90                  95

aac atc gcc acc agc ttc cgc ccc ggc cac gcc gac tat acc tat tgg     336
Asn Ile Ala Thr Ser Phe Arg Pro Gly His Ala Asp Tyr Thr Tyr Trp
            100                 105                 110

cac aaa tac ggc acg cgc gac tac cgg ggc ggc ggc agg agt tcc gcc     384
His Lys Tyr Gly Thr Arg Asp Tyr Arg Gly Gly Gly Arg Ser Ser Ala
        115                 120                 125

cgc gaa acc gcc gcc cgc gtt gcc gcc gga gcc gtt gcc aaa aaa tgg     432
Arg Glu Thr Ala Ala Arg Val Ala Ala Gly Ala Val Ala Lys Lys Trp
    130                 135                 140

ttg aaa gaa aaa ttc ggc acg gaa atc acc gcc tac gtt acc caa gtc     480
Leu Lys Glu Lys Phe Gly Thr Glu Ile Thr Ala Tyr Val Thr Gln Val
145                 150                 155                 160

ggc gaa aaa gaa atc cgg ttt gaa ggc tgc gaa cac att tcc caa aat 528
Gly Glu Lys Glu Ile Arg Phe Glu Gly Cys Glu His Ile Ser Gln Asn
                165                 170                 175

cct ttt ttt gcc gcc aac cat agc caa att gcc gag ctg gaa aac tat 576
Pro Phe Phe Ala Ala Asn His Ser Gln Ile Ala Glu Leu Glu Asn Tyr
            180                 185                 190

atg gac agc gtg cgc aaa tcc ttg gat tcc gtc ggc gcg aag ctg cat 624
Met Asp Ser Val Arg Lys Ser Leu Asp Ser Val Gly Ala Lys Leu His
        195                 200                 205

att gaa gcc gcc aac gtc ccc gtc ggc ttg ggc gaa ccc gta ttc gac 672
Ile Glu Ala Ala Asn Val Pro Val Gly Leu Gly Glu Pro Val Phe Asp
    210                 215                 220

cgc ctc gat gcc gaa atc gcc tac gcc atg atg ggc atc aac gcc gtc 720
Arg Leu Asp Ala Glu Ile Ala Tyr Ala Met Met Gly Ile Asn Ala Val
225                 230                 235                 240

aaa ggc gtg gaa atc ggc gca ggt ttt gac agc gta acg caa cgc ggc 768
Lys Gly Val Glu Ile Gly Ala Gly Phe Asp Ser Val Thr Gln Arg Gly
                245                 250                 255

agc gaa cac ggc gac gaa ctc acc ccg caa ggc ttc ctg tcc aac cac 816
Ser Glu His Gly Asp Glu Leu Thr Pro Gln Gly Phe Leu Ser Asn His
            260                 265                 270

tca ggc ggc atc ctc ggc ggc atc agc acc gga caa gac atc cac gtc 864
Ser Gly Gly Ile Leu Gly Gly Ile Ser Thr Gly Gln Asp Ile His Val
        275                 280                 285

aat atc gcc atc aaa ccc acc agc tcc atc gcc acc ccg cgc cgc agt 912
Asn Ile Ala Ile Lys Pro Thr Ser Ser Ile Ala Thr Pro Arg Arg Ser
    290                 295                 300

atc gac atc aac ggc aac ccc atc gaa ctc gcc acg cac ggc agg cac 960
Ile Asp Ile Asn Gly Asn Pro Ile Glu Leu Ala Thr His Gly Arg His
305                 310                 315                 320

gac ccc tgc gtc ggc ttg cgc gcc gcg ccg att gca gaa gct atg ctg 1008
Asp Pro Cys Val Gly Leu Arg Ala Ala Pro Ile Ala Glu Ala Met Leu
                325                 330                 335

gca ttg gtt tta atc gac cac gcc ctg cgc cat cgc gcg caa aat gcg 1056
Ala Leu Val Leu Ile Asp His Ala Leu Arg His Arg Ala Gln Asn Ala
            340                 345                 350

gat gtt cag gtt aat acg ccc gac att acc ctt tca aac aaa          1098
Asp Val Gln Val Asn Thr Pro Asp Ile Thr Leu Ser Asn Lys
        355                 360                 365

<210> 64
<211> 366
<212> PRT
<213> Neisseria meningitidis

<400> 64
Met Ala Gly Asn Thr Phe Gly Gln Leu Phe Thr Val Thr Thr Phe Gly
1               5                   10                  15

Glu Ser His Gly Ala Gly Leu Gly Cys Ile Ile Asp Gly Cys Pro Pro
            20                  25                  30

Gly Leu Glu Leu Ser Glu Ala Asp Ile Gln Phe Asp Leu Asp Arg Arg
        35                  40                  45

Lys Pro Gly Thr Ser Arg His Val Thr Gln Arg Arg Glu Ala Asp Gln
    50                  55                  60

Val Glu Ile Leu Ser Gly Val Phe Glu Gly Lys Thr Thr Gly Thr Pro
65                  70                  75                  80

Ile Ala Leu Leu Ile Arg Asn Thr Asp Gln Arg Ser Lys Asp Tyr Gly
                85                  90                  95

Asn Ile Ala Thr Ser Phe Arg Pro Gly His Ala Asp Tyr Thr Tyr Trp
            100                 105                 110

His Lys Tyr Gly Thr Arg Asp Tyr Arg Gly Gly Gly Arg Ser Ser Ala
        115                 120                 125

Arg Glu Thr Ala Ala Arg Val Ala Ala Gly Ala Val Ala Lys Lys Trp
    130                 135                 140

Leu Lys Glu Lys Phe Gly Thr Glu Ile Thr Ala Tyr Val Thr Gln Val
145                 150                 155                 160

Gly Glu Lys Glu Ile Arg Phe Glu Gly Cys Glu His Ile Ser Gln Asn
                165                 170                 175

Pro Phe Phe Ala Ala Asn His Ser Gln Ile Ala Glu Leu Glu Asn Tyr
            180                 185                 190

Met Asp Ser Val Arg Lys Ser Leu Asp Ser Val Gly Ala Lys Leu His
        195                 200                 205

Ile Glu Ala Ala Asn Val Pro Val Gly Leu Gly Glu Pro Val Phe Asp
    210                 215                 220

Arg Leu Asp Ala Glu Ile Ala Tyr Ala Met Met Gly Ile Asn Ala Val
225                 230                 235                 240

Lys Gly Val Glu Ile Gly Ala Gly Phe Asp Ser Val Thr Gln Arg Gly
                245                 250                 255

Ser Glu His Gly Asp Glu Leu Thr Pro Gln Gly Phe Leu Ser Asn His
            260                 265                 270

Ser Gly Gly Ile Leu Gly Gly Ile Ser Thr Gly Gln Asp Ile His Val
        275                 280                 285

Asn Ile Ala Ile Lys Pro Thr Ser Ser Ile Ala Thr Pro Arg Arg Ser
    290                 295                 300

Ile Asp Ile Asn Gly Asn Pro Ile Glu Leu Ala Thr His Gly Arg His
305                 310                 315                 320

Asp Pro Cys Val Gly Leu Arg Ala Ala Pro Ile Ala Glu Ala Met Leu
                325                 330                 335

Ala Leu Val Leu Ile Asp His Ala Leu Arg His Arg Ala Gln Asn Ala
            340                 345                 350

Asp Val Gln Val Asn Thr Pro Asp Ile Thr Leu Ser Asn Lys
        355                 360                 365

<210> 65
<211> 461
<212> DNA
<213> Neisseria meningitidis

<220>
<221> CDS
<222> (1)..(459)

<400> 65
atg caa agc ggt ttt aac gct att ttc agg aat gac acc atg cag gtt     48
Met Gln Ser Gly Phe Asn Ala Ile Phe Arg Asn Asp Thr Met Gln Val
1               5                   10                  15

aca tca aaa tgg ata gac gga atg tgc ttt gtc gga aca acg gaa ggc     96
Thr Ser Lys Trp Ile Asp Gly Met Cys Phe Val Gly Thr Thr Glu Gly
            20                  25                  30

ggg cac agc gtc gtt atg gag ggg tcg gcg gca gaa ggt gcg gcc aag     144
Gly His Ser Val Val Met Glu Gly Ser Ala Ala Glu Gly Ala Ala Lys
        35                  40                  45

cgc ggg ccc agc cct ttg gaa atg ctg ctg ttg ggc gtg gcg ggc tgt     192
Arg Gly Pro Ser Pro Leu Glu Met Leu Leu Leu Gly Val Ala Gly Cys
    50                  55                  60

tcg agc atc gat gtg gtg atg att gcc gaa aaa cag cgt cag aaa gtg     240
Ser Ser Ile Asp Val Val Met Ile Ala Glu Lys Gln Arg Gln Lys Val
65                  70                  75                  80

act gac tgc cgt gcg acg gtt acg gcg aaa cgg gcg gac gat gcg ccg     288
Thr Asp Cys Arg Ala Thr Val Thr Ala Lys Arg Ala Asp Asp Ala Pro
                85                  90                  95

cgc gtg ttt acc gaa atc cac atc cat ttc aaa gta ttc ggg cat gat     336
Arg Val Phe Thr Glu Ile His Ile His Phe Lys Val Phe Gly His Asp
            100                 105                 110

ttg aaa gaa tcg gcc att gag cgc gcc gtt cag atg tct gcc gaa aaa     384
Leu Lys Glu Ser Ala Ile Glu Arg Ala Val Gln Met Ser Ala Glu Lys
        115                 120                 125

tac tgt tcg gct tcg att atg ttg ggc aaa gcg gca aag att acc cac     432
Tyr Cys Ser Ala Ser Ile Met Leu Gly Lys Ala Ala Lys Ile Thr His
    130                 135                 140

agt ttt gaa att gcc ggg gca gat aaa ta                              461
Ser Phe Glu Ile Ala Gly Ala Asp Lys
145                 150

<210> 66
<211> 153
<212> PRT
<213> Neisseria meningitidis

<400> 66
Met Gln Ser Gly Phe Asn Ala Ile Phe Arg Asn Asp Thr Met Gln Val
1               5                   10                  15

Thr Ser Lys Trp Ile Asp Gly Met Cys Phe Val Gly Thr Thr Glu Gly

20                  25                  30

Gly His Ser Val Val Met Glu Gly Ser Ala Ala Glu Gly Ala Ala Lys
        35                  40                  45

Arg Gly Pro Ser Pro Leu Glu Met Leu Leu Leu Gly Val Ala Gly Cys
    50                  55                  60

Ser Ser Ile Asp Val Val Met Ile Ala Glu Lys Gln Arg Gln Lys Val
65                  70                  75                  80

Thr Asp Cys Arg Ala Thr Val Thr Ala Lys Arg Ala Asp Asp Ala Pro
                85                  90                  95

Arg Val Phe Thr Glu Ile His Ile His Phe Lys Val Phe Gly His Asp
            100                 105                 110

Leu Lys Glu Ser Ala Ile Glu Arg Ala Val Gln Met Ser Ala Glu Lys
        115                 120                 125

Tyr Cys Ser Ala Ser Ile Met Leu Gly Lys Ala Ala Lys Ile Thr His
    130                 135                 140

Ser Phe Glu Ile Ala Gly Ala Asp Lys
145                 150

<210> 67
<211> 1659
<212> DNA
<213> Neisseria meningitidis

<220>
<221> CDS
<222> (1)..(1659)

<400> 67
atg act gac aac agc cct cct cca aac gga cac gcc caa gca cgc gtc      48
Met Thr Asp Asn Ser Pro Pro Pro Asn Gly His Ala Gln Ala Arg Val
1               5                   10                  15

cgc aaa aac aac acc ttc ctc tct gcc gtc tgg ctg gtt ccg ctg atc      96
Arg Lys Asn Asn Thr Phe Leu Ser Ala Val Trp Leu Val Pro Leu Ile
            20                  25                  30

gcg ctg att gcc ggc ggc tgg ctt tgg gtt aag gaa atc cgc aac agg      144
Ala Leu Ile Ala Gly Gly Trp Leu Trp Val Lys Glu Ile Arg Asn Arg

        35                  40                  45

ggg cct gtg gtt acg ctc ttg atg gac agc gcg gaa ggc att gag gtc 192
Gly Pro Val Val Thr Leu Leu Met Asp Ser Ala Glu Gly Ile Glu Val
    50                  55                  60

aac aat acg gtc atc aaa gta ttg agc atc gat gtc gga cgc gtt acc 240
Asn Asn Thr Val Ile Lys Val Leu Ser Ile Asp Val Gly Arg Val Thr
65                  70                  75                  80

cga atc aaa ctg cgc gac gac caa aaa ggc gtg gaa gta acc gcc caa 288
Arg Ile Lys Leu Arg Asp Asp Gln Lys Gly Val Glu Val Thr Ala Gln
                85                  90                  95

ctc aat gcg gac gta tcc ggc ctc atc cgc agc gat acc cag ttt tgg 336
Leu Asn Ala Asp Val Ser Gly Leu Ile Arg Ser Asp Thr Gln Phe Trp
            100                 105                 110

gtg gtc aag ccg cgt atc gac caa agc ggc gta acc ggt ttg ggt acg 384
Val Val Lys Pro Arg Ile Asp Gln Ser Gly Val Thr Gly Leu Gly Thr
        115                 120                 125

ctg ctt tcg ggt tcg tac atc gcc ttt aca ccc ggc aaa agc gac gag 432
Leu Leu Ser Gly Ser Tyr Ile Ala Phe Thr Pro Gly Lys Ser Asp Glu
    130                 135                 140

gca aaa gac gtg ttc caa gtg cag gac att ccg ccc gtt acc gcc atc 480
Ala Lys Asp Val Phe Gln Val Gln Asp Ile Pro Pro Val Thr Ala Ile
145                 150                 155                 160

ggg caa agc ggg ctg cgc ttg aat ttg att ggt aaa aac gac cgc atc 528
Gly Gln Ser Gly Leu Arg Leu Asn Leu Ile Gly Lys Asn Asp Arg Ile
                165                 170                 175

ctc aac gtc aac agc cct gtt ttg tat gaa aac ttt atg gtc ggg caa 576
Leu Asn Val Asn Ser Pro Val Leu Tyr Glu Asn Phe Met Val Gly Gln
            180                 185                 190

gtc gaa agc gcg cat ttc gac ccg tcc gac caa agc gtg cat tac acc 624
Val Glu Ser Ala His Phe Asp Pro Ser Asp Gln Ser Val His Tyr Thr
        195                 200                 205

atc ttc atc caa agc ccc aac gac aaa ctg att cat tcc gcc agc cgt 672
Ile Phe Ile Gln Ser Pro Asn Asp Lys Leu Ile His Ser Ala Ser Arg
    210                 215                 220

ttc tgg ctg gaa agc ggc atc aat atc gaa acc aca ggc agc ggc atc 720
Phe Trp Leu Glu Ser Gly Ile Asn Ile Glu Thr Thr Gly Ser Gly Ile

225                 230                 235                 240

aaa ctc aat tcc gcc cct ctg cct gcc ctg ctg tcg ggc gcg att tca     768
Lys Leu Asn Ser Ala Pro Leu Pro Ala Leu Leu Ser Gly Ala Ile Ser
                245                 250                 255

ttt gat tcg ccg aaa acc aaa aac agt aaa aac gtc aaa agc gaa gac     816
Phe Asp Ser Pro Lys Thr Lys Asn Ser Lys Asn Val Lys Ser Glu Asp
            260                 265                 270

agc ttc acg ctt tac gac agc cgc agc gaa gtc gcc aac ctg cct gat     864
Ser Phe Thr Leu Tyr Asp Ser Arg Ser Glu Val Ala Asn Leu Pro Asp
        275                 280                 285

gac cgt tcg ctg tac tac acc gcg ttt ttc aaa caa tcc gtg cgc gga     912
Asp Arg Ser Leu Tyr Tyr Thr Ala Phe Phe Lys Gln Ser Val Arg Gly
    290                 295                 300

ctg acc gtc ggt tcg cct gtc gag tac aaa ggg ctg aat gtc ggc gtg     960
Leu Thr Val Gly Ser Pro Val Glu Tyr Lys Gly Leu Asn Val Gly Val
305                 310                 315                 320

gtt tcc gat gtt cct tat ttc gac cgc aac gac agc ctg cac ctg ttt     1008
Val Ser Asp Val Pro Tyr Phe Asp Arg Asn Asp Ser Leu His Leu Phe
                325                 330                 335

gaa aac ggc tgg att ccc gta cgc atc cgt att gag cct tcc cgt ttg     1056
Glu Asn Gly Trp Ile Pro Val Arg Ile Arg Ile Glu Pro Ser Arg Leu
            340                 345                 350

gaa atc aat gcc gac gaa caa agc aaa gaa cat tgg aaa caa caa ttt     1104
Glu Ile Asn Ala Asp Glu Gln Ser Lys Glu His Trp Lys Gln Gln Phe
        355                 360                 365

cag acg gcc tta aac aaa ggc ctg acc gcc acc atc tcc agc aac aac     1152
Gln Thr Ala Leu Asn Lys Gly Leu Thr Ala Thr Ile Ser Ser Asn Asn
    370                 375                 380

ctg ctg acc ggc agc aaa atg att gag ttg aac gat cag cct tcc gcc     1200
Leu Leu Thr Gly Ser Lys Met Ile Glu Leu Asn Asp Gln Pro Ser Ala
385                 390                 395                 400

tcg ccc aag ctg cga ccg cat acc gtt tat gca ggc gat acc gtt atc     1248
Ser Pro Lys Leu Arg Pro His Thr Val Tyr Ala Gly Asp Thr Val Ile
                405                 410                 415

gcg acc cag ggc ggc ggt ttg gac gat ttg cag gtc aaa ttg gcg gat     1296
Ala Thr Gln Gly Gly Gly Leu Asp Asp Leu Gln Val Lys Leu Ala Asp

420                    425                    430

ttg ctg gac aag ttc gac aaa ctg cct tta gat aag acg gtt gcc gaa    1344
Leu Leu Asp Lys Phe Asp Lys Leu Pro Leu Asp Lys Thr Val Ala Glu
        435                 440                 445

ttg aac ggt tcg ctt gcc gag ctc aaa tcc aca ctc aaa tct gcc aat    1392
Leu Asn Gly Ser Leu Ala Glu Leu Lys Ser Thr Leu Lys Ser Ala Asn
    450                 455                 460

gcc gcc cta agc tcc atc gac aaa ctg gtc ggc aaa ccg cag aca caa    1440
Ala Ala Leu Ser Ser Ile Asp Lys Leu Val Gly Lys Pro Gln Thr Gln
465                 470                 475                 480

aac att ccg aac gaa ctg aac caa acc ctg aaa gag ttg cgc aca acc    1488
Asn Ile Pro Asn Glu Leu Asn Gln Thr Leu Lys Glu Leu Arg Thr Thr
                485                 490                 495

ctg caa ggc gta tcg cct caa tcg cct atc tac ggc gac gta caa aat    1536
Leu Gln Gly Val Ser Pro Gln Ser Pro Ile Tyr Gly Asp Val Gln Asn
            500                 505                 510

acg ctg caa agt ttg gac aaa acc tta aaa gac gtt caa ccc gtc att    1584
Thr Leu Gln Ser Leu Asp Lys Thr Leu Lys Asp Val Gln Pro Val Ile
        515                 520                 525

aac act ttg aaa gaa aaa ccc aac gcg ctg att ttc aac agc agc agc    1632
Asn Thr Leu Lys Glu Lys Pro Asn Ala Leu Ile Phe Asn Ser Ser Ser
    530                 535                 540

aaa gac cct atc ccg aaa gga agc cga                                1659
Lys Asp Pro Ile Pro Lys Gly Ser Arg
545                 550

<210> 68
<211> 553
<212> PRT
<213> Neisseria meningitidis

<400> 68
Met Thr Asp Asn Ser Pro Pro Pro Asn Gly His Ala Gln Ala Arg Val
1               5                   10                  15

Arg Lys Asn Asn Thr Phe Leu Ser Ala Val Trp Leu Val Pro Leu Ile
            20                  25                  30

Ala Leu Ile Ala Gly Gly Trp Leu Trp Val Lys Glu Ile Arg Asn Arg

        35                  40                  45

Gly Pro Val Val Thr Leu Leu Met Asp Ser Ala Glu Gly Ile Glu Val
    50                  55                  60

Asn Asn Thr Val Ile Lys Val Leu Ser Ile Asp Val Gly Arg Val Thr
65                  70                  75                  80

Arg Ile Lys Leu Arg Asp Asp Gln Lys Gly Val Glu Val Thr Ala Gln
                85                  90                  95

Leu Asn Ala Asp Val Ser Gly Leu Ile Arg Ser Asp Thr Gln Phe Trp
            100                 105                 110

Val Val Lys Pro Arg Ile Asp Gln Ser Gly Val Thr Gly Leu Gly Thr
        115                 120                 125

Leu Leu Ser Gly Ser Tyr Ile Ala Phe Thr Pro Gly Lys Ser Asp Glu
    130                 135                 140

Ala Lys Asp Val Phe Gln Val Gln Asp Ile Pro Pro Val Thr Ala Ile
145                 150                 155                 160

Gly Gln Ser Gly Leu Arg Leu Asn Leu Ile Gly Lys Asn Asp Arg Ile
                165                 170                 175

Leu Asn Val Asn Ser Pro Val Leu Tyr Glu Asn Phe Met Val Gly Gln
            180                 185                 190

Val Glu Ser Ala His Phe Asp Pro Ser Asp Gln Ser Val His Tyr Thr
        195                 200                 205

Ile Phe Ile Gln Ser Pro Asn Asp Lys Leu Ile His Ser Ala Ser Arg
    210                 215                 220

Phe Trp Leu Glu Ser Gly Ile Asn Ile Glu Thr Thr Gly Ser Gly Ile
225                 230                 235                 240

Lys Leu Asn Ser Ala Pro Leu Pro Ala Leu Leu Ser Gly Ala Ile Ser
                245                 250                 255

Phe Asp Ser Pro Lys Thr Lys Asn Ser Lys Asn Val Lys Ser Glu Asp
            260                 265                 270

Ser Phe Thr Leu Tyr Asp Ser Arg Ser Glu Val Ala Asn Leu Pro Asp
        275                 280                 285

Asp Arg Ser Leu Tyr Tyr Thr Ala Phe Phe Lys Gln Ser Val Arg Gly

    290                 295                 300

Leu Thr Val Gly Ser Pro Val Glu Tyr Lys Gly Leu Asn Val Gly Val
305                 310                 315                 320

Val Ser Asp Val Pro Tyr Phe Asp Arg Asn Asp Ser Leu His Leu Phe
                325                 330                 335

Glu Asn Gly Trp Ile Pro Val Arg Ile Arg Ile Glu Pro Ser Arg Leu
            340                 345                 350

Glu Ile Asn Ala Asp Glu Gln Ser Lys Glu His Trp Lys Gln Gln Phe
        355                 360                 365

Gln Thr Ala Leu Asn Lys Gly Leu Thr Ala Thr Ile Ser Ser Asn Asn
    370                 375                 380

Leu Leu Thr Gly Ser Lys Met Ile Glu Leu Asn Asp Gln Pro Ser Ala
385                 390                 395                 400

Ser Pro Lys Leu Arg Pro His Thr Val Tyr Ala Gly Asp Thr Val Ile
                405                 410                 415

Ala Thr Gln Gly Gly Gly Leu Asp Asp Leu Gln Val Lys Leu Ala Asp
            420                 425                 430

Leu Leu Asp Lys Phe Asp Lys Leu Pro Leu Asp Lys Thr Val Ala Glu
        435                 440                 445

Leu Asn Gly Ser Leu Ala Glu Leu Lys Ser Thr Leu Lys Ser Ala Asn
    450                 455                 460

Ala Ala Leu Ser Ser Ile Asp Lys Leu Val Gly Lys Pro Gln Thr Gln
465                 470                 475                 480

Asn Ile Pro Asn Glu Leu Asn Gln Thr Leu Lys Glu Leu Arg Thr Thr
                485                 490                 495

Leu Gln Gly Val Ser Pro Gln Ser Pro Ile Tyr Gly Asp Val Gln Asn
            500                 505                 510

Thr Leu Gln Ser Leu Asp Lys Thr Leu Lys Asp Val Gln Pro Val Ile
        515                 520                 525

Asn Thr Leu Lys Glu Lys Pro Asn Ala Leu Ile Phe Asn Ser Ser Ser
    530                 535                 540

Lys Asp Pro Ile Pro Lys Gly Ser Arg

545                550

<210> 69
<211> 330
<212> DNA
<213> Neisseria meningitidis

<220>
<221> CDS
<222> (1)..(330)

<400> 69
ttg ccg ctg ctt tgt tgt ctt tgg ttt tgg cag cct gcg gcg gtg aaa     48
Leu Pro Leu Leu Cys Cys Leu Trp Phe Trp Gln Pro Ala Ala Val Lys
1               5                   10                  15

aag ccg ctg aag ctc ccg ctg ctg aag cac ctg ccg ccg aag ctc ccg     96
Lys Pro Leu Lys Leu Pro Leu Leu Lys His Leu Pro Pro Lys Leu Pro
            20                  25                  30

cta ctg aag cac ctg ccg ccg aag ctc ccg ctg ctg aag cac ctg ccg     144
Leu Leu Lys His Leu Pro Pro Lys Leu Pro Leu Leu Lys His Leu Pro
        35                  40                  45

ccg aag ctc ctg cta ctg aag cac ctg ccg ccg aag ctc ccg ctg ctg     192
Pro Lys Leu Leu Leu Leu Lys His Leu Pro Pro Lys Leu Pro Leu Leu
    50                  55                  60

aag ctg ccg cta ccg aag cac ctg ccg ctg aag ctg ccg cta ccg aag     240
Lys Leu Pro Leu Pro Lys His Leu Pro Leu Lys Leu Pro Leu Pro Lys
65                  70                  75                  80

cac ctg ccg ctg aag ctc ctg ctg ccg aag ctg caa aat aag cat ttt     288
His Leu Pro Leu Lys Leu Leu Leu Pro Lys Leu Gln Asn Lys His Phe
                85                  90                  95

ccg ctt gca aaa aag cag gat acg ttc agt atc ctg ctt ttt             330
Pro Leu Ala Lys Lys Gln Asp Thr Phe Ser Ile Leu Leu Phe
            100                 105                 110

<210> 70
<211> 110
<212> PRT
<213> Neisseria meningitidis

<400> 70
Leu Pro Leu Leu Cys Cys Leu Trp Phe Trp Gln Pro Ala Ala Val Lys
1               5                   10                  15

Lys Pro Leu Lys Leu Pro Leu Leu Lys His Leu Pro Pro Lys Leu Pro
            20                  25                  30

Leu Leu Lys His Leu Pro Pro Lys Leu Pro Leu Leu Lys His Leu Pro
        35                  40                  45

Pro Lys Leu Leu Leu Leu Lys His Leu Pro Pro Lys Leu Pro Leu Leu
    50                  55                  60

Lys Leu Pro Leu Pro Lys His Leu Pro Leu Lys Leu Pro Leu Pro Lys
65                  70                  75                  80

His Leu Pro Leu Lys Leu Leu Leu Pro Lys Leu Gln Asn Lys His Phe
                85                  90                  95

Pro Leu Ala Lys Lys Gln Asp Thr Phe Ser Ile Leu Leu Phe
            100                 105                 110

<210> 71
<211> 2274
<212> DNA
<213> Neisseria meningitidis

<220>
<221> CDS
<222> (1)..(2274)

<400> 71
atg aca aca tta cat ttc tca ggc ttc ccg cgt gtc ggt gcc ttc cgc    48
Met Thr Thr Leu His Phe Ser Gly Phe Pro Arg Val Gly Ala Phe Arg
1               5                   10                  15

gaa ttg aaa ttc gca caa gaa aaa tac tgg cgc aaa gaa atc agc gag    96
Glu Leu Lys Phe Ala Gln Glu Lys Tyr Trp Arg Lys Glu Ile Ser Glu
            20                  25                  30

caa gaa ttg ctg gct gtt gct aaa gac ttg cgc gag aaa aac tgg aaa    144
Gln Glu Leu Leu Ala Val Ala Lys Asp Leu Arg Glu Lys Asn Trp Lys
        35                  40                  45

cac cag gcc gct gcc aac gcc gat tac gtt gcc gta ggc gat ttc act    192
His Gln Ala Ala Ala Asn Ala Asp Tyr Val Ala Val Gly Asp Phe Thr

    50                  55                  60

ttc tac gac cac atc ctc gac ctg caa gtc gcc acc ggc gcg att ccc 240
Phe Tyr Asp His Ile Leu Asp Leu Gln Val Ala Thr Gly Ala Ile Pro
65                  70                  75                  80

gcc cgc ttc ggc ttc gac agc caa aac cta tct ttg gaa caa ttc ttc 288
Ala Arg Phe Gly Phe Asp Ser Gln Asn Leu Ser Leu Glu Gln Phe Phe
                85                  90                  95

caa ctg gcg cgc ggt aac aaa gac caa ttc gct atc gaa atg acc aaa 336
Gln Leu Ala Arg Gly Asn Lys Asp Gln Phe Ala Ile Glu Met Thr Lys
            100                 105                 110

tgg ttc gac acc aac tac cac tac ttg gtg cct gaa ttc cac gcc gat 384
Trp Phe Asp Thr Asn Tyr His Tyr Leu Val Pro Glu Phe His Ala Asp
        115                 120                 125

acc gaa ttt aaa gcc aat gcc aaa cac tac gtt caa cag ctg caa gaa 432
Thr Glu Phe Lys Ala Asn Ala Lys His Tyr Val Gln Gln Leu Gln Glu
    130                 135                 140

gcc caa gct ttg ggc ctg aaa gcc aaa ccg acc gtt gtg ggt ccg ttg 480
Ala Gln Ala Leu Gly Leu Lys Ala Lys Pro Thr Val Val Gly Pro Leu
145                 150                 155                 160

act ttc ctg tgg gtc ggt aaa gaa aaa ggc gcc gtt gaa ttc gac cgt 528
Thr Phe Leu Trp Val Gly Lys Glu Lys Gly Ala Val Glu Phe Asp Arg
                165                 170                 175

ctg agc ctg ttg cct aaa ctg ttg cct gtt tac gtt gaa atc ctg act 576
Leu Ser Leu Leu Pro Lys Leu Leu Pro Val Tyr Val Glu Ile Leu Thr
            180                 185                 190

gct ttg gtt gaa gcc ggt gcc gag tgg att caa atc gac gag cct gct 624
Ala Leu Val Glu Ala Gly Ala Glu Trp Ile Gln Ile Asp Glu Pro Ala
        195                 200                 205

ttg act gtt gac ctg cct aaa gaa tgg gta gaa gca tac aaa gac gtt 672
Leu Thr Val Asp Leu Pro Lys Glu Trp Val Glu Ala Tyr Lys Asp Val
    210                 215                 220

tac gcc act ttg agc aaa gta agt gcc aaa atc ctg ctg agc act tac 720
Tyr Ala Thr Leu Ser Lys Val Ser Ala Lys Ile Leu Leu Ser Thr Tyr
225                 230                 235                 240

ttc ggt tct gtt gcc gaa cac gcc gca ttg ttg aaa tcc ctg cct gtt 768
Phe Gly Ser Val Ala Glu His Ala Ala Leu Leu Lys Ser Leu Pro Val

                245                 250                 255

gac ggc ctg cac atc gac ttg gta cgc gct cct gaa caa ctg gat gcg 816
Asp Gly Leu His Ile Asp Leu Val Arg Ala Pro Glu Gln Leu Asp Ala
            260                 265                 270

ttc gcc gac tac gac aaa gtt ctg tct gcc ggc gtc att gac ggc cgc 864
Phe Ala Asp Tyr Asp Lys Val Leu Ser Ala Gly Val Ile Asp Gly Arg
        275                 280                 285

aat att tgg cgc gcc aac ctg aac aaa gtt ttg gaa act gtt gag ctt 912
Asn Ile Trp Arg Ala Asn Leu Asn Lys Val Leu Glu Thr Val Glu Leu
    290                 295                 300

ctg caa gcc aaa ctg ggc gac cgt ttg tgg att tcc agc tct tgc tcg 960
Leu Gln Ala Lys Leu Gly Asp Arg Leu Trp Ile Ser Ser Ser Cys Ser
305                 310                 315                 320

ctg ctg cac act ccg ttt gac ttg tca gtt gaa gaa aaa ctg aaa gcc 1008
Leu Leu His Thr Pro Phe Asp Leu Ser Val Glu Glu Lys Leu Lys Ala
                325                 330                 335

aac aaa ccc gac ctg tac tct tgg ttg gca ttc acc ctg caa aaa acc 1056
Asn Lys Pro Asp Leu Tyr Ser Trp Leu Ala Phe Thr Leu Gln Lys Thr
            340                 345                 350

caa gaa ttg cgc gtc ctg aaa gcc gca ttg aac gaa ggc cgt gat tct 1104
Gln Glu Leu Arg Val Leu Lys Ala Ala Leu Asn Glu Gly Arg Asp Ser
        355                 360                 365

gtt gcc gaa gaa ctg gct gcc agc caa gct gct gcc gac tcc cgt gcc 1152
Val Ala Glu Glu Leu Ala Ala Ser Gln Ala Ala Ala Asp Ser Arg Ala
    370                 375                 380

aac agc agc gaa atc cac cgt gca gac gtt gcc aaa cgc ctg gcc gat 1200
Asn Ser Ser Glu Ile His Arg Ala Asp Val Ala Lys Arg Leu Ala Asp
385                 390                 395                 400

ttg cct gcc aac gca gac caa cgc aaa tcc cca ttt gcc gac cgt atc 1248
Leu Pro Ala Asn Ala Asp Gln Arg Lys Ser Pro Phe Ala Asp Arg Ile
                405                 410                 415

aaa gcg caa caa gca tgg ttg aac ctg cct ctg ctg ccg acg acc aac 1296
Lys Ala Gln Gln Ala Trp Leu Asn Leu Pro Leu Leu Pro Thr Thr Asn
            420                 425                 430

atc ggt tct ttc ccg caa act acc gaa atc cgc caa gca cgc gca gcc 1344
Ile Gly Ser Phe Pro Gln Thr Thr Glu Ile Arg Gln Ala Arg Ala Ala

435                 440                 445

ttc aaa aaa ggc gaa ctg tct gcc gcc gat tac gaa gcc gcg atg aaa     1392
Phe Lys Lys Gly Glu Leu Ser Ala Ala Asp Tyr Glu Ala Ala Met Lys
    450                 455                 460

aaa gaa atc gcc ttg gtg gtt gaa gag caa gaa aaa ctg gac ttg gac     1440
Lys Glu Ile Ala Leu Val Val Glu Glu Gln Glu Lys Leu Asp Leu Asp
465                 470                 475                 480

gta ctg gta cac ggc gaa gcc gag cgt aac gac atg gtt gaa tac ttc     1488
Val Leu Val His Gly Glu Ala Glu Arg Asn Asp Met Val Glu Tyr Phe
                485                 490                 495

ggc gaa ttg ttg agc ggt ttt gca ttc acc caa tat ggc tgg gta caa     1536
Gly Glu Leu Leu Ser Gly Phe Ala Phe Thr Gln Tyr Gly Trp Val Gln
            500                 505                 510

agc tac ggt tct cgc tgc gtt aaa cca ccg att atc ttc ggt gac gta     1584
Ser Tyr Gly Ser Arg Cys Val Lys Pro Pro Ile Ile Phe Gly Asp Val
        515                 520                 525

agc cgt cct gaa gcc atg acc gta gct tgg tct act tac gca caa agc     1632
Ser Arg Pro Glu Ala Met Thr Val Ala Trp Ser Thr Tyr Ala Gln Ser
    530                 535                 540

ctg acc aaa cgc ccg atg aaa ggt atg ttg acc ggc cct gta acc att     1680
Leu Thr Lys Arg Pro Met Lys Gly Met Leu Thr Gly Pro Val Thr Ile
545                 550                 555                 560

ctg caa tgg tct ttc gtc cgc aac gat att cct cgc tct acc gtg tgt     1728
Leu Gln Trp Ser Phe Val Arg Asn Asp Ile Pro Arg Ser Thr Val Cys
                565                 570                 575

aaa caa atc gca ctg gct ctg aac gac gaa gta ttg gat ctg gaa aaa     1776
Lys Gln Ile Ala Leu Ala Leu Asn Asp Glu Val Leu Asp Leu Glu Lys
            580                 585                 590

gca ggc atc aaa gtc atc caa atc gac gaa cct gcc atc cgc gaa ggc     1824
Ala Gly Ile Lys Val Ile Gln Ile Asp Glu Pro Ala Ile Arg Glu Gly
        595                 600                 605

ctg cct ttg aaa cgt gcc gat tgg gat gcc tac ctg aac tgg gcc ggc     1872
Leu Pro Leu Lys Arg Ala Asp Trp Asp Ala Tyr Leu Asn Trp Ala Gly
    610                 615                 620

gaa tct ttc cgc ctg tcc tct acc ggt tgc gaa gac agc act caa atc     1920
Glu Ser Phe Arg Leu Ser Ser Thr Gly Cys Glu Asp Ser Thr Gln Ile

625                 630                 635                 640

cac acc cat atg tgc tac tct gag ttc aac gac atc ctg cct gcg att     1968
His Thr His Met Cys Tyr Ser Glu Phe Asn Asp Ile Leu Pro Ala Ile
                645                 650                 655

gct gca atg gat gcg gac gtg atc acc atc gag act tca cgt tcc gac     2016
Ala Ala Met Asp Ala Asp Val Ile Thr Ile Glu Thr Ser Arg Ser Asp
            660                 665                 670

atg gaa ctc ttg acc gcg ttc ggc gaa ttc aaa tac ccg aac gac atc     2064
Met Glu Leu Leu Thr Ala Phe Gly Glu Phe Lys Tyr Pro Asn Asp Ile
        675                 680                 685

ggc ccg ggg gtt tac gac atc cac agc ccg cgc gta ccg aca gaa gcc     2112
Gly Pro Gly Val Tyr Asp Ile His Ser Pro Arg Val Pro Thr Glu Ala
    690                 695                 700

gaa gtg gag cac ctg ttg cgc aaa gcc atc gag gtt gta ccg gtt gaa     2160
Glu Val Glu His Leu Leu Arg Lys Ala Ile Glu Val Val Pro Val Glu
705                 710                 715                 720

cgt ctg tgg gtt aac ccg gac tgc ggc ctg aaa aca cgc ggc tgg aaa     2208
Arg Leu Trp Val Asn Pro Asp Cys Gly Leu Lys Thr Arg Gly Trp Lys
                725                 730                 735

gaa act ctg gaa caa ctc caa gtg atg atg aac gta acc cac aaa ttg     2256
Glu Thr Leu Glu Gln Leu Gln Val Met Met Asn Val Thr His Lys Leu
            740                 745                 750

cgt gcc gaa ttg gcg aaa                                             2274
Arg Ala Glu Leu Ala Lys
        755

<210> 72
<211> 758
<212> PRT
<213> Neisseria meningitidis

<400> 72
Met Thr Thr Leu His Phe Ser Gly Phe Pro Arg Val Gly Ala Phe Arg
1               5                   10                  15

Glu Leu Lys Phe Ala Gln Glu Lys Tyr Trp Arg Lys Glu Ile Ser Glu
            20                  25                  30

Gln Glu Leu Leu Ala Val Ala Lys Asp Leu Arg Glu Lys Asn Trp Lys

        35                  40                  45

His Gln Ala Ala Ala Asn Ala Asp Tyr Val Ala Val Gly Asp Phe Thr
    50                  55                  60

Phe Tyr Asp His Ile Leu Asp Leu Gln Val Ala Thr Gly Ala Ile Pro
65                  70                  75                  80

Ala Arg Phe Gly Phe Asp Ser Gln Asn Leu Ser Leu Glu Gln Phe Phe
                85                  90                  95

Gln Leu Ala Arg Gly Asn Lys Asp Gln Phe Ala Ile Glu Met Thr Lys
            100                 105                 110

Trp Phe Asp Thr Asn Tyr His Tyr Leu Val Pro Glu Phe His Ala Asp
        115                 120                 125

Thr Glu Phe Lys Ala Asn Ala Lys His Tyr Val Gln Gln Leu Gln Glu
    130                 135                 140

Ala Gln Ala Leu Gly Leu Lys Ala Lys Pro Thr Val Val Gly Pro Leu
145                 150                 155                 160

Thr Phe Leu Trp Val Gly Lys Glu Lys Gly Ala Val Glu Phe Asp Arg
                165                 170                 175

Leu Ser Leu Leu Pro Lys Leu Leu Pro Val Tyr Val Glu Ile Leu Thr
            180                 185                 190

Ala Leu Val Glu Ala Gly Ala Glu Trp Ile Gln Ile Asp Glu Pro Ala
        195                 200                 205

Leu Thr Val Asp Leu Pro Lys Glu Trp Val Glu Ala Tyr Lys Asp Val
    210                 215                 220

Tyr Ala Thr Leu Ser Lys Val Ser Ala Lys Ile Leu Leu Ser Thr Tyr
225                 230                 235                 240

Phe Gly Ser Val Ala Glu His Ala Ala Leu Leu Lys Ser Leu Pro Val
                245                 250                 255

Asp Gly Leu His Ile Asp Leu Val Arg Ala Pro Glu Gln Leu Asp Ala
            260                 265                 270

Phe Ala Asp Tyr Asp Lys Val Leu Ser Ala Gly Val Ile Asp Gly Arg
        275                 280                 285

Asn Ile Trp Arg Ala Asn Leu Asn Lys Val Leu Glu Thr Val Glu Leu

290                 295                 300

Leu Gln Ala Lys Leu Gly Asp Arg Leu Trp Ile Ser Ser Ser Cys Ser
305                 310                 315                 320

Leu Leu His Thr Pro Phe Asp Leu Ser Val Glu Glu Lys Leu Lys Ala
                325                 330                 335

Asn Lys Pro Asp Leu Tyr Ser Trp Leu Ala Phe Thr Leu Gln Lys Thr
            340                 345                 350

Gln Glu Leu Arg Val Leu Lys Ala Ala Leu Asn Glu Gly Arg Asp Ser
        355                 360                 365

Val Ala Glu Glu Leu Ala Ala Ser Gln Ala Ala Ala Asp Ser Arg Ala
    370                 375                 380

Asn Ser Ser Glu Ile His Arg Ala Asp Val Ala Lys Arg Leu Ala Asp
385                 390                 395                 400

Leu Pro Ala Asn Ala Asp Gln Arg Lys Ser Pro Phe Ala Asp Arg Ile
                405                 410                 415

Lys Ala Gln Gln Ala Trp Leu Asn Leu Pro Leu Leu Pro Thr Thr Asn
            420                 425                 430

Ile Gly Ser Phe Pro Gln Thr Thr Glu Ile Arg Gln Ala Arg Ala Ala
        435                 440                 445

Phe Lys Lys Gly Glu Leu Ser Ala Ala Asp Tyr Glu Ala Ala Met Lys
    450                 455                 460

Lys Glu Ile Ala Leu Val Val Glu Glu Gln Glu Lys Leu Asp Leu Asp
465                 470                 475                 480

Val Leu Val His Gly Glu Ala Glu Arg Asn Asp Met Val Glu Tyr Phe
                485                 490                 495

Gly Glu Leu Leu Ser Gly Phe Ala Phe Thr Gln Tyr Gly Trp Val Gln
            500                 505                 510

Ser Tyr Gly Ser Arg Cys Val Lys Pro Pro Ile Ile Phe Gly Asp Val
        515                 520                 525

Ser Arg Pro Glu Ala Met Thr Val Ala Trp Ser Thr Tyr Ala Gln Ser
    530                 535                 540

Leu Thr Lys Arg Pro Met Lys Gly Met Leu Thr Gly Pro Val Thr Ile

545                 550                 555                 560

Leu Gln Trp Ser Phe Val Arg Asn Asp Ile Pro Arg Ser Thr Val Cys
                565                 570                 575

Lys Gln Ile Ala Leu Ala Leu Asn Asp Glu Val Leu Asp Leu Glu Lys
            580                 585                 590

Ala Gly Ile Lys Val Ile Gln Ile Asp Glu Pro Ala Ile Arg Glu Gly
        595                 600                 605

Leu Pro Leu Lys Arg Ala Asp Trp Asp Ala Tyr Leu Asn Trp Ala Gly
    610                 615                 620

Glu Ser Phe Arg Leu Ser Ser Thr Gly Cys Glu Asp Ser Thr Gln Ile
625                 630                 635                 640

His Thr His Met Cys Tyr Ser Glu Phe Asn Asp Ile Leu Pro Ala Ile
                645                 650                 655

Ala Ala Met Asp Ala Asp Val Ile Thr Ile Glu Thr Ser Arg Ser Asp
            660                 665                 670

Met Glu Leu Leu Thr Ala Phe Gly Glu Phe Lys Tyr Pro Asn Asp Ile
        675                 680                 685

Gly Pro Gly Val Tyr Asp Ile His Ser Pro Arg Val Pro Thr Glu Ala
    690                 695                 700

Glu Val Glu His Leu Leu Arg Lys Ala Ile Glu Val Val Pro Val Glu
705                 710                 715                 720

Arg Leu Trp Val Asn Pro Asp Cys Gly Leu Lys Thr Arg Gly Trp Lys
                725                 730                 735

Glu Thr Leu Glu Gln Leu Gln Val Met Met Asn Val Thr His Lys Leu
            740                 745                 750

Arg Ala Glu Leu Ala Lys
        755

<210> 73
<211> 2118
<212> DNA
<213> Neisseria meningitidis

<220>
<221> CDS
<222> (1)..(2118)

<400> 73
atg cgc cgt ttt cta ccg atc gca gcc ata tgc gcc gtc gtc ctg ttg     48
Met Arg Arg Phe Leu Pro Ile Ala Ala Ile Cys Ala Val Val Leu Leu
1               5                   10                  15

tac gga ctg acg gcg gca acc ggc agc acc agt tcg ctg gcg gat tat     96
Tyr Gly Leu Thr Ala Ala Thr Gly Ser Thr Ser Ser Leu Ala Asp Tyr
            20                  25                  30

ttc tgg tgg att gtt gcg ttc agc gca atg ctg ctg ctg gtg ttg tcc     144
Phe Trp Trp Ile Val Ala Phe Ser Ala Met Leu Leu Leu Val Leu Ser
        35                  40                  45

gcc gtt ttg gca cgt tat gtc ata ttg ctg ttg aaa gac agg cgc gac     192
Ala Val Leu Ala Arg Tyr Val Ile Leu Leu Leu Lys Asp Arg Arg Asp
    50                  55                  60

ggc gta ttc ggt tcg cag att gcc aaa cgc ctt tcc ggg atg ttt acg     240
Gly Val Phe Gly Ser Gln Ile Ala Lys Arg Leu Ser Gly Met Phe Thr
65                  70                  75                  80

ctg gtt gcc gta ctg ccc ggc gtg ttt ctg ttc ggc gtt tcc gca cag     288
Leu Val Ala Val Leu Pro Gly Val Phe Leu Phe Gly Val Ser Ala Gln
                85                  90                  95

ttt atc aac ggc acg att aat tcg tgg ttc ggc aac gat acc cac gag     336
Phe Ile Asn Gly Thr Ile Asn Ser Trp Phe Gly Asn Asp Thr His Glu
            100                 105                 110

gcg ctt gaa cgc agc ctc aat ttg agc aag tcc gca ttg aat ctg gcg     384
Ala Leu Glu Arg Ser Leu Asn Leu Ser Lys Ser Ala Leu Asn Leu Ala
        115                 120                 125

gca gac aac gcc ctt ggc aac gcc atc ccc gtg cag ata gac ctc atc     432
Ala Asp Asn Ala Leu Gly Asn Ala Ile Pro Val Gln Ile Asp Leu Ile
    130                 135                 140

ggc gcg gct tcc ctg ccc ggg gat atg ggc agg gtg ctg gaa cat tac     480
Gly Ala Ala Ser Leu Pro Gly Asp Met Gly Arg Val Leu Glu His Tyr
145                 150                 155                 160

gcc ggc agc ggt ttt gcc cag ctt gcc ctg tac aat gcc gca agc ggc     528
Ala Gly Ser Gly Phe Ala Gln Leu Ala Leu Tyr Asn Ala Ala Ser Gly
                165                 170                 175

aaa atc gaa aaa agc atc aac ccg cac aag ctc gat cag ccg ttt cca    576
Lys Ile Glu Lys Ser Ile Asn Pro His Lys Leu Asp Gln Pro Phe Pro
            180                 185                 190

ggt aag gcg cgt tgg gaa aaa atc caa cag gcg ggt tcg gtc agg gat    624
Gly Lys Ala Arg Trp Glu Lys Ile Gln Gln Ala Gly Ser Val Arg Asp
        195                 200                 205

ttg gaa agc ata ggc ggc gta ttg tac gcg cag ggc tgg ctg tcg gca    672
Leu Glu Ser Ile Gly Gly Val Leu Tyr Ala Gln Gly Trp Leu Ser Ala
    210                 215                 220

ggt acg cac aac ggg cgc gat tac gcc ttg ttt ttc cgt cag ccg gtt    720
Gly Thr His Asn Gly Arg Asp Tyr Ala Leu Phe Phe Arg Gln Pro Val
225                 230                 235                 240

ccc aaa ggc gtg gca gag gat gcc gtc tta atc gaa aag gca agg gcg    768
Pro Lys Gly Val Ala Glu Asp Ala Val Leu Ile Glu Lys Ala Arg Ala
                245                 250                 255

aaa tat gct gag ttg agt tac agc aaa aaa ggt ttg cag acc ttt ttc    816
Lys Tyr Ala Glu Leu Ser Tyr Ser Lys Lys Gly Leu Gln Thr Phe Phe
            260                 265                 270

ctg gca acc ctg ctg att gcc tcg ctg ctg tcg att ttt ctt gca ctg    864
Leu Ala Thr Leu Leu Ile Ala Ser Leu Leu Ser Ile Phe Leu Ala Leu
        275                 280                 285

gtc atg gca ctg tat ttc gcc cgc cgt ttc gtc gaa ccc gtc cta tcg    912
Val Met Ala Leu Tyr Phe Ala Arg Arg Phe Val Glu Pro Val Leu Ser
    290                 295                 300

ctt gcc gag ggg gcg aag gcg gtg gcg caa ggc gat ttc agc cag acg    960
Leu Ala Glu Gly Ala Lys Ala Val Ala Gln Gly Asp Phe Ser Gln Thr
305                 310                 315                 320

cgc ccc gtg ttg cgc aac gac gag ttc gga cgc ttg acc aag ttg ttc    1008
Arg Pro Val Leu Arg Asn Asp Glu Phe Gly Arg Leu Thr Lys Leu Phe
                325                 330                 335

aac cac atg acc gag cag ctt tcc atc gcc aaa gaa gca gac gag cgc    1056
Asn His Met Thr Glu Gln Leu Ser Ile Ala Lys Glu Ala Asp Glu Arg
            340                 345                 350

aac cgc cgg cgc gag gaa gcc gcc aga cat tat ctc gaa tgc gtg ttg    1104
Asn Arg Arg Arg Glu Glu Ala Ala Arg His Tyr Leu Glu Cys Val Leu
        355                 360                 365

gag ggg ctg acc acg ggc gtg gtg gtg ttt gac gaa caa ggc tgt ctg 1152
Glu Gly Leu Thr Thr Gly Val Val Val Phe Asp Glu Gln Gly Cys Leu
    370                 375                 380

aaa acc ttc aac aaa gcg gcg gaa cag att ttg ggg atg ccg ctt acc 1200
Lys Thr Phe Asn Lys Ala Ala Glu Gln Ile Leu Gly Met Pro Leu Thr
385                 390                 395                 400

ccc ctg tgg ggc agc agc cgg cac ggt tgg cac ggc gtt tcg gcg cag 1248
Pro Leu Trp Gly Ser Ser Arg His Gly Trp His Gly Val Ser Ala Gln
                405                 410                 415

cag tcc ctg ctt gcc gaa gtg ttt gcc gcc atc ggc gcg gcg gca ggt 1296
Gln Ser Leu Leu Ala Glu Val Phe Ala Ala Ile Gly Ala Ala Ala Gly
            420                 425                 430

acg gac aaa ccg gtc cat gtg aaa tat gcc gcg ccg gac gat gcc aaa 1344
Thr Asp Lys Pro Val His Val Lys Tyr Ala Ala Pro Asp Asp Ala Lys
        435                 440                 445

atc ctg ctg ggc aag gca acc gtc ctg ccc gaa gac aac ggc aac ggc 1392
Ile Leu Leu Gly Lys Ala Thr Val Leu Pro Glu Asp Asn Gly Asn Gly
    450                 455                 460

gtg gta atg gtg att gac gac atc acc gtt ttg ata cac gcg caa aaa 1440
Val Val Met Val Ile Asp Asp Ile Thr Val Leu Ile His Ala Gln Lys
465                 470                 475                 480

gaa gcc gcg tgg ggc gaa gtg gca aaa cgg ctg gca cac gaa atc cgc 1488
Glu Ala Ala Trp Gly Glu Val Ala Lys Arg Leu Ala His Glu Ile Arg
                485                 490                 495

aat ccg ctc acg ccc atc cag ctt tct gcc gaa cgg ctg gcg tgg aaa 1536
Asn Pro Leu Thr Pro Ile Gln Leu Ser Ala Glu Arg Leu Ala Trp Lys
            500                 505                 510

ttg ggc ggg aag ctg gac gag cag gac gcg caa atc ctg aca cgt tcg 1584
Leu Gly Gly Lys Leu Asp Glu Gln Asp Ala Gln Ile Leu Thr Arg Ser
        515                 520                 525

acc gac acc atc atc aaa caa gtg gcg gca tta aaa gaa atg gtc gag 1632
Thr Asp Thr Ile Ile Lys Gln Val Ala Ala Leu Lys Glu Met Val Glu
    530                 535                 540

gca ttc cgc aat tac gcg cgt tcc cct tcg ctc aaa ttg gaa aat cag 1680
Ala Phe Arg Asn Tyr Ala Arg Ser Pro Ser Leu Lys Leu Glu Asn Gln
545                 550                 555                 560

gat ttg aac gcc tta atc ggc gat gtg ttg gca ttg tac gaa gct ggt 1728
Asp Leu Asn Ala Leu Ile Gly Asp Val Leu Ala Leu Tyr Glu Ala Gly
                565                 570                 575

ccg tgc cgg ttt gcg gcg gaa ctt gcc ggc gaa ccg ctg atg atg gcg 1776
Pro Cys Arg Phe Ala Ala Glu Leu Ala Gly Glu Pro Leu Met Met Ala
            580                 585                 590

gcg gat acg acc gcc atg cgg cag gtg ctg cac aat att ttc aaa aat 1824
Ala Asp Thr Thr Ala Met Arg Gln Val Leu His Asn Ile Phe Lys Asn
        595                 600                 605

gcc gcc gaa gcg gcg gaa gaa gcc gat gtg ccc gaa gtc agg gta aaa 1872
Ala Ala Glu Ala Ala Glu Glu Ala Asp Val Pro Glu Val Arg Val Lys
    610                 615                 620

tcg gaa gcg ggg cag gac gga cgg att gtc ctg aca gtt tgc gac aac 1920
Ser Glu Ala Gly Gln Asp Gly Arg Ile Val Leu Thr Val Cys Asp Asn
625                 630                 635                 640

ggc aag ggg ttc ggc agg gaa atg ctg cac aat gcc ttc gag ccg tat 1968
Gly Lys Gly Phe Gly Arg Glu Met Leu His Asn Ala Phe Glu Pro Tyr
                645                 650                 655

gta acg gac aaa ccg gct gga acg gga ttg gga ctg ccc gtg gtg aaa 2016
Val Thr Asp Lys Pro Ala Gly Thr Gly Leu Gly Leu Pro Val Val Lys
            660                 665                 670

aaa atc att gaa gaa cac ggc ggc cgc atc agc ctg agc aat cag gat 2064
Lys Ile Ile Glu Glu His Gly Gly Arg Ile Ser Leu Ser Asn Gln Asp
        675                 680                 685

gcg ggc ggc gcg tgt gtc aga atc atc ttg cca aaa acg gta gaa act 2112
Ala Gly Gly Ala Cys Val Arg Ile Ile Leu Pro Lys Thr Val Glu Thr
    690                 695                 700

tat gcg 2118
Tyr Ala
705

<210> 74
<211> 706
<212> PRT
<213> Neisseria meningitidis

<400> 74

Met Arg Arg Phe Leu Pro Ile Ala Ala Ile Cys Ala Val Val Leu Leu
1               5                   10                  15

Tyr Gly Leu Thr Ala Ala Thr Gly Ser Thr Ser Ser Leu Ala Asp Tyr
            20                  25                  30

Phe Trp Trp Ile Val Ala Phe Ser Ala Met Leu Leu Leu Val Leu Ser
        35                  40                  45

Ala Val Leu Ala Arg Tyr Val Ile Leu Leu Leu Lys Asp Arg Arg Asp
    50                  55                  60

Gly Val Phe Gly Ser Gln Ile Ala Lys Arg Leu Ser Gly Met Phe Thr
65                  70                  75                  80

Leu Val Ala Val Leu Pro Gly Val Phe Leu Phe Gly Val Ser Ala Gln
                85                  90                  95

Phe Ile Asn Gly Thr Ile Asn Ser Trp Phe Gly Asn Asp Thr His Glu
            100                 105                 110

Ala Leu Glu Arg Ser Leu Asn Leu Ser Lys Ser Ala Leu Asn Leu Ala
        115                 120                 125

Ala Asp Asn Ala Leu Gly Asn Ala Ile Pro Val Gln Ile Asp Leu Ile
    130                 135                 140

Gly Ala Ala Ser Leu Pro Gly Asp Met Gly Arg Val Leu Glu His Tyr
145                 150                 155                 160

Ala Gly Ser Gly Phe Ala Gln Leu Ala Leu Tyr Asn Ala Ala Ser Gly
                165                 170                 175

Lys Ile Glu Lys Ser Ile Asn Pro His Lys Leu Asp Gln Pro Phe Pro
            180                 185                 190

Gly Lys Ala Arg Trp Glu Lys Ile Gln Gln Ala Gly Ser Val Arg Asp
        195                 200                 205

Leu Glu Ser Ile Gly Gly Val Leu Tyr Ala Gln Gly Trp Leu Ser Ala
    210                 215                 220

Gly Thr His Asn Gly Arg Asp Tyr Ala Leu Phe Phe Arg Gln Pro Val
225                 230                 235                 240

Pro Lys Gly Val Ala Glu Asp Ala Val Leu Ile Glu Lys Ala Arg Ala
                245                 250                 255

Lys Tyr Ala Glu Leu Ser Tyr Ser Lys Lys Gly Leu Gln Thr Phe Phe
            260                 265                 270

Leu Ala Thr Leu Leu Ile Ala Ser Leu Leu Ser Ile Phe Leu Ala Leu
        275                 280                 285

Val Met Ala Leu Tyr Phe Ala Arg Arg Phe Val Glu Pro Val Leu Ser
    290                 295                 300

Leu Ala Glu Gly Ala Lys Ala Val Ala Gln Gly Asp Phe Ser Gln Thr
305                 310                 315                 320

Arg Pro Val Leu Arg Asn Asp Glu Phe Gly Arg Leu Thr Lys Leu Phe
                325                 330                 335

Asn His Met Thr Glu Gln Leu Ser Ile Ala Lys Glu Ala Asp Glu Arg
            340                 345                 350

Asn Arg Arg Arg Glu Glu Ala Ala Arg His Tyr Leu Glu Cys Val Leu
        355                 360                 365

Glu Gly Leu Thr Thr Gly Val Val Val Phe Asp Glu Gln Gly Cys Leu
    370                 375                 380

Lys Thr Phe Asn Lys Ala Ala Glu Gln Ile Leu Gly Met Pro Leu Thr
385                 390                 395                 400

Pro Leu Trp Gly Ser Ser Arg His Gly Trp His Gly Val Ser Ala Gln
                405                 410                 415

Gln Ser Leu Leu Ala Glu Val Phe Ala Ala Ile Gly Ala Ala Ala Gly
            420                 425                 430

Thr Asp Lys Pro Val His Val Lys Tyr Ala Ala Pro Asp Asp Ala Lys
        435                 440                 445

Ile Leu Leu Gly Lys Ala Thr Val Leu Pro Glu Asp Asn Gly Asn Gly
    450                 455                 460

Val Val Met Val Ile Asp Asp Ile Thr Val Leu Ile His Ala Gln Lys
465                 470                 475                 480

Glu Ala Ala Trp Gly Glu Val Ala Lys Arg Leu Ala His Glu Ile Arg
                485                 490                 495

Asn Pro Leu Thr Pro Ile Gln Leu Ser Ala Glu Arg Leu Ala Trp Lys
            500                 505                 510

Leu Gly Gly Lys Leu Asp Glu Gln Asp Ala Gln Ile Leu Thr Arg Ser
        515                 520                 525

Thr Asp Thr Ile Ile Lys Gln Val Ala Ala Leu Lys Glu Met Val Glu
    530                 535                 540

Ala Phe Arg Asn Tyr Ala Arg Ser Pro Ser Leu Lys Leu Glu Asn Gln
545                 550                 555                 560

Asp Leu Asn Ala Leu Ile Gly Asp Val Leu Ala Leu Tyr Glu Ala Gly
                565                 570                 575

Pro Cys Arg Phe Ala Ala Glu Leu Ala Gly Glu Pro Leu Met Met Ala
            580                 585                 590

Ala Asp Thr Thr Ala Met Arg Gln Val Leu His Asn Ile Phe Lys Asn
        595                 600                 605

Ala Ala Glu Ala Ala Glu Glu Ala Asp Val Pro Glu Val Arg Val Lys
    610                 615                 620

Ser Glu Ala Gly Gln Asp Gly Arg Ile Val Leu Thr Val Cys Asp Asn
625                 630                 635                 640

Gly Lys Gly Phe Gly Arg Glu Met Leu His Asn Ala Phe Glu Pro Tyr
                645                 650                 655

Val Thr Asp Lys Pro Ala Gly Thr Gly Leu Gly Leu Pro Val Val Lys
            660                 665                 670

Lys Ile Ile Glu Glu His Gly Gly Arg Ile Ser Leu Ser Asn Gln Asp
        675                 680                 685

Ala Gly Gly Ala Cys Val Arg Ile Ile Leu Pro Lys Thr Val Glu Thr
    690                 695                 700

Tyr Ala
705

<210> 75
<211> 1686
<212> DNA
<213> Neisseria meningitidis

<220>
<221> CDS

<222> (1)..(1686)

<400> 75
atg tta caa tcc gaa aat tcg aaa aat tta atc tct tgt tca ata aag     48
Met Leu Gln Ser Glu Asn Ser Lys Asn Leu Ile Ser Cys Ser Ile Lys
1               5                   10                  15

gct tta cca atc atg att tct acc aac ggc atc acc atg cag ttc ggc     96
Ala Leu Pro Ile Met Ile Ser Thr Asn Gly Ile Thr Met Gln Phe Gly
            20                  25                  30

gcg aag ccg ctg ttt gaa aac gta tcc gtt aaa ttc ggc gaa ggc aac     144
Ala Lys Pro Leu Phe Glu Asn Val Ser Val Lys Phe Gly Glu Gly Asn
        35                  40                  45

cgc tac ggt ttg atc ggc gcg aac ggc tca ggc aaa tcc acc ttc atg     192
Arg Tyr Gly Leu Ile Gly Ala Asn Gly Ser Gly Lys Ser Thr Phe Met
    50                  55                  60

aaa atc ctc ggc ggc gat ttg gaa cag aca gcc ggc gaa gtg gcg att     240
Lys Ile Leu Gly Gly Asp Leu Glu Gln Thr Ala Gly Glu Val Ala Ile
65                  70                  75                  80

gaa aac ggc gtg cgt ttg ggt aaa ttg cgc caa gac cag ttt gcc tac     288
Glu Asn Gly Val Arg Leu Gly Lys Leu Arg Gln Asp Gln Phe Ala Tyr
                85                  90                  95

gaa gat atg cgc gtg ctg gac gtg gta atg atg ggt cac acc gaa atg     336
Glu Asp Met Arg Val Leu Asp Val Val Met Met Gly His Thr Glu Met
            100                 105                 110

tgg gcg gcg atg acc gaa cgt gat gcg att tac gcc aat ccc gaa gcc     384
Trp Ala Ala Met Thr Glu Arg Asp Ala Ile Tyr Ala Asn Pro Glu Ala
        115                 120                 125

acc gaa gac gac tac atg aaa gcc gca gaa ctg gaa gcc aag ttc gcc     432
Thr Glu Asp Asp Tyr Met Lys Ala Ala Glu Leu Glu Ala Lys Phe Ala
    130                 135                 140

gaa tac gac ggc tac acc gcc gaa gcg cgt gcc gcc gag ttg ttg agc     480
Glu Tyr Asp Gly Tyr Thr Ala Glu Ala Arg Ala Ala Glu Leu Leu Ser
145                 150                 155                 160

ggc gtg ggc att tcc gaa gat ttg cac aat gca acc atg gca gaa gtt     528
Gly Val Gly Ile Ser Glu Asp Leu His Asn Ala Thr Met Ala Glu Val
                165                 170                 175

gcc ccg ggc ttc aaa ctg cgc gta ttg ctg gcg caa gcc ctg ttc tcc     576

Ala Pro Gly Phe Lys Leu Arg Val Leu Leu Ala Gln Ala Leu Phe Ser
            180                 185                 190

aag ccg gat gta ttg ctc ttg gac gaa ccg acc aat aac ttg gac att 624
Lys Pro Asp Val Leu Leu Leu Asp Glu Pro Thr Asn Asn Leu Asp Ile
        195                 200                 205

aat acc atc cgt tgg ttg gaa ggc gtg ttg aac cag tac gac tcc acg 672
Asn Thr Ile Arg Trp Leu Glu Gly Val Leu Asn Gln Tyr Asp Ser Thr
    210                 215                 220

atg att atc atc tcg cac gac cgc cac ttc ttg aac gaa gtc tgc acg 720
Met Ile Ile Ile Ser His Asp Arg His Phe Leu Asn Glu Val Cys Thr
225                 230                 235                 240

cat atg gca gat ttg gat tac aac acc atc acc atc tat ccg ggc aac 768
His Met Ala Asp Leu Asp Tyr Asn Thr Ile Thr Ile Tyr Pro Gly Asn
                245                 250                 255

tac gac gac tat atg ctc gcc tcc gcc caa tcg cgc gaa cgc gcc ctg 816
Tyr Asp Asp Tyr Met Leu Ala Ser Ala Gln Ser Arg Glu Arg Ala Leu
            260                 265                 270

aaa gac aat gcc aag gcg aag gaa aaa ttg cag gaa ttg caa gaa ttt 864
Lys Asp Asn Ala Lys Ala Lys Glu Lys Leu Gln Glu Leu Gln Glu Phe
        275                 280                 285

gtg gcg cgg ttc agt gcc aac aaa tcc aaa gcc cgt cag gca acc agc 912
Val Ala Arg Phe Ser Ala Asn Lys Ser Lys Ala Arg Gln Ala Thr Ser
    290                 295                 300

cgt ctg aaa cag gca gac aaa atc aaa tcg gag atg gtc gag gtc aaa 960
Arg Leu Lys Gln Ala Asp Lys Ile Lys Ser Glu Met Val Glu Val Lys
305                 310                 315                 320

ccc tct acc cgt caa aac ccg tat atc cgt ttt gaa gcc gat gaa aaa 1008
Pro Ser Thr Arg Gln Asn Pro Tyr Ile Arg Phe Glu Ala Asp Glu Lys
                325                 330                 335

gcc aag ctg cac cgt cag gct gtg gaa gtt gaa aaa ctg gcg aaa cgc 1056
Ala Lys Leu His Arg Gln Ala Val Glu Val Glu Lys Leu Ala Lys Arg
            340                 345                 350

ttt gaa acc cag ttg ttt aaa aac ctg aac ttt atc ctt gaa gcg gga 1104
Phe Glu Thr Gln Leu Phe Lys Asn Leu Asn Phe Ile Leu Glu Ala Gly
        355                 360                 365

caa cgc ctc gcc atc atc ggc ccg aac ggc gcg ggc aaa tcc acc ctg 1152

Gln Arg Leu Ala Ile Ile Gly Pro Asn Gly Ala Gly Lys Ser Thr Leu
    370                 375                 380

ctg aaa ctc ttg gcc ggc gcg tac aac ccc gaa tat tca gac ggt ctg    1200
Leu Lys Leu Leu Ala Gly Ala Tyr Asn Pro Glu Tyr Ser Asp Gly Leu
385                 390                 395                 400

ctg ccg gac gaa ggc agc atc aaa tgg gcg gaa aaa gcc agc gtc ggc    1248
Leu Pro Asp Glu Gly Ser Ile Lys Trp Ala Glu Lys Ala Ser Val Gly
                405                 410                 415

tac tat ccg caa gat cac gaa aac gac ttc gat gtc gat atg gat ttg    1296
Tyr Tyr Pro Gln Asp His Glu Asn Asp Phe Asp Val Asp Met Asp Leu
            420                 425                 430

agc gaa tgg atg cgc caa tgg ggg cag gac ggc gac gac gaa caa gtc    1344
Ser Glu Trp Met Arg Gln Trp Gly Gln Asp Gly Asp Asp Glu Gln Val
        435                 440                 445

atc cgc ggc act ttg ggg cgt ttg ctc ttc ggc agt aac gat gtc gtg    1392
Ile Arg Gly Thr Leu Gly Arg Leu Leu Phe Gly Ser Asn Asp Val Val
    450                 455                 460

aaa aaa gtg aag gtt ctc tcc ggt ggt gaa aaa ggc cgt atg ctt tac    1440
Lys Lys Val Lys Val Leu Ser Gly Gly Glu Lys Gly Arg Met Leu Tyr
465                 470                 475                 480

ggc aaa ctg ttg ctg ttg aaa ccc aat gtc tta gtc atg gat gaa ccg    1488
Gly Lys Leu Leu Leu Leu Lys Pro Asn Val Leu Val Met Asp Glu Pro
                485                 490                 495

acc aac cac atg gac atg gaa agc atc gaa tcc ttg aac atg gcg ctg    1536
Thr Asn His Met Asp Met Glu Ser Ile Glu Ser Leu Asn Met Ala Leu
            500                 505                 510

gaa aaa tac aac ggc acg ctg att ttc gtc tcc cac gac cgt cag ttc    1584
Glu Lys Tyr Asn Gly Thr Leu Ile Phe Val Ser His Asp Arg Gln Phe
        515                 520                 525

gtt tct tcc ctg gct acc caa atc att gaa ttg gac ggc aaa ggc gga    1632
Val Ser Ser Leu Ala Thr Gln Ile Ile Glu Leu Asp Gly Lys Gly Gly
    530                 535                 540

tat gaa cac tac ttg ggc gat tac gaa agc tat ctc gag aaa aaa ggc    1680
Tyr Glu His Tyr Leu Gly Asp Tyr Glu Ser Tyr Leu Glu Lys Lys Gly
545                 550                 555                 560

gta gca                                                            1686

Val Ala

<210> 76
<211> 562
<212> PRT
<213> Neisseria meningitidis

<400> 76
Met Leu Gln Ser Glu Asn Ser Lys Asn Leu Ile Ser Cys Ser Ile Lys
1               5                   10                  15

Ala Leu Pro Ile Met Ile Ser Thr Asn Gly Ile Thr Met Gln Phe Gly
            20                  25                  30

Ala Lys Pro Leu Phe Glu Asn Val Ser Val Lys Phe Gly Glu Gly Asn
        35                  40                  45

Arg Tyr Gly Leu Ile Gly Ala Asn Gly Ser Gly Lys Ser Thr Phe Met
    50                  55                  60

Lys Ile Leu Gly Gly Asp Leu Glu Gln Thr Ala Gly Glu Val Ala Ile
65                  70                  75                  80

Glu Asn Gly Val Arg Leu Gly Lys Leu Arg Gln Asp Gln Phe Ala Tyr
                85                  90                  95

Glu Asp Met Arg Val Leu Asp Val Val Met Met Gly His Thr Glu Met
            100                 105                 110

Trp Ala Ala Met Thr Glu Arg Asp Ala Ile Tyr Ala Asn Pro Glu Ala
        115                 120                 125

Thr Glu Asp Asp Tyr Met Lys Ala Ala Glu Leu Glu Ala Lys Phe Ala
    130                 135                 140

Glu Tyr Asp Gly Tyr Thr Ala Glu Ala Arg Ala Ala Glu Leu Leu Ser
145                 150                 155                 160

Gly Val Gly Ile Ser Glu Asp Leu His Asn Ala Thr Met Ala Glu Val
                165                 170                 175

Ala Pro Gly Phe Lys Leu Arg Val Leu Leu Ala Gln Ala Leu Phe Ser
            180                 185                 190

Lys Pro Asp Val Leu Leu Leu Asp Glu Pro Thr Asn Asn Leu Asp Ile
        195                 200                 205

Asn Thr Ile Arg Trp Leu Glu Gly Val Leu Asn Gln Tyr Asp Ser Thr
    210                 215                 220

Met Ile Ile Ile Ser His Asp Arg His Phe Leu Asn Glu Val Cys Thr
225                 230                 235                 240

His Met Ala Asp Leu Asp Tyr Asn Thr Ile Thr Ile Tyr Pro Gly Asn
                245                 250                 255

Tyr Asp Asp Tyr Met Leu Ala Ser Ala Gln Ser Arg Glu Arg Ala Leu
            260                 265                 270

Lys Asp Asn Ala Lys Ala Lys Glu Lys Leu Gln Glu Leu Gln Glu Phe
        275                 280                 285

Val Ala Arg Phe Ser Ala Asn Lys Ser Lys Ala Arg Gln Ala Thr Ser
    290                 295                 300

Arg Leu Lys Gln Ala Asp Lys Ile Lys Ser Glu Met Val Glu Val Lys
305                 310                 315                 320

Pro Ser Thr Arg Gln Asn Pro Tyr Ile Arg Phe Glu Ala Asp Glu Lys
                325                 330                 335

Ala Lys Leu His Arg Gln Ala Val Glu Val Glu Lys Leu Ala Lys Arg
            340                 345                 350

Phe Glu Thr Gln Leu Phe Lys Asn Leu Asn Phe Ile Leu Glu Ala Gly
        355                 360                 365

Gln Arg Leu Ala Ile Ile Gly Pro Asn Gly Ala Gly Lys Ser Thr Leu
    370                 375                 380

Leu Lys Leu Leu Ala Gly Ala Tyr Asn Pro Glu Tyr Ser Asp Gly Leu
385                 390                 395                 400

Leu Pro Asp Glu Gly Ser Ile Lys Trp Ala Glu Lys Ala Ser Val Gly
                405                 410                 415

Tyr Tyr Pro Gln Asp His Glu Asn Asp Phe Asp Val Asp Met Asp Leu
            420                 425                 430

Ser Glu Trp Met Arg Gln Trp Gly Gln Asp Gly Asp Asp Glu Gln Val
        435                 440                 445

Ile Arg Gly Thr Leu Gly Arg Leu Leu Phe Gly Ser Asn Asp Val Val
    450                 455                 460

Lys Lys Val Lys Val Leu Ser Gly Gly Glu Lys Gly Arg Met Leu Tyr
465                 470                 475                 480

Gly Lys Leu Leu Leu Leu Lys Pro Asn Val Leu Val Met Asp Glu Pro
                485                 490                 495

Thr Asn His Met Asp Met Glu Ser Ile Glu Ser Leu Asn Met Ala Leu
            500                 505                 510

Glu Lys Tyr Asn Gly Thr Leu Ile Phe Val Ser His Asp Arg Gln Phe
        515                 520                 525

Val Ser Ser Leu Ala Thr Gln Ile Ile Glu Leu Asp Gly Lys Gly Gly
    530                 535                 540

Tyr Glu His Tyr Leu Gly Asp Tyr Glu Ser Tyr Leu Glu Lys Lys Gly
545                 550                 555                 560

Val Ala

<210> 77
<211> 1773
<212> DNA
<213> Neisseria meningitidis

<220>
<221> CDS
<222> (1)..(1773)

<400> 77
atg agt atc gtg ctg cac ggc gtg gcg gcg ggc aaa ggc att gcc gtc    48
Met Ser Ile Val Leu His Gly Val Ala Ala Gly Lys Gly Ile Ala Val
1               5                   10                  15

ggt tgc gcc cac ctg att gcg cgc ggt acg gag gaa gtg ccg cag tat    96
Gly Cys Ala His Leu Ile Ala Arg Gly Thr Glu Glu Val Pro Gln Tyr
            20                  25                  30

gat gtt gcg gag gcg gac acc gat gcc gaa gcc gaa cgt ttc gat gcc    144
Asp Val Ala Glu Ala Asp Thr Asp Ala Glu Ala Glu Arg Phe Asp Ala
        35                  40                  45

gcc gtc aaa gcc acg cgc aaa gag ttg gaa cag ctc cgc agc gcg att    192
Ala Val Lys Ala Thr Arg Lys Glu Leu Glu Gln Leu Arg Ser Ala Ile
    50                  55                  60

ccc gaa aac gcc ccg acc gag ttg ggc gcg ttc atc tcg ctg cac ctg 240
Pro Glu Asn Ala Pro Thr Glu Leu Gly Ala Phe Ile Ser Leu His Leu
65                  70                  75                  80

atg ctc ttg acc gat gtt acc ttg tcg cgc gaa ccc gtc gat att tta 288
Met Leu Leu Thr Asp Val Thr Leu Ser Arg Glu Pro Val Asp Ile Leu
                85                  90                  95

agg gaa caa aaa atc aac gcc gag tgg gca ttg aag cag cag agc gac 336
Arg Glu Gln Lys Ile Asn Ala Glu Trp Ala Leu Lys Gln Gln Ser Asp
            100                 105                 110

aaa ctc gcc gcc caa ttc gac aat atg gac gat gcc tat ttg cgc gaa 384
Lys Leu Ala Ala Gln Phe Asp Asn Met Asp Asp Ala Tyr Leu Arg Glu
        115                 120                 125

cgc aag cag gat atg ctg caa gtc gtc cgc cgc atc cac aac aac ctg 432
Arg Lys Gln Asp Met Leu Gln Val Val Arg Arg Ile His Asn Asn Leu
    130                 135                 140

atc ggg cag ggc aac gag ttg gaa gtt gcc gac aac ctg ttt gac gaa 480
Ile Gly Gln Gly Asn Glu Leu Glu Val Ala Asp Asn Leu Phe Asp Glu
145                 150                 155                 160

acc gtt ctg att gca aac gac ctt tcg ccc gcc gac acg gtt ttg ttt 528
Thr Val Leu Ile Ala Asn Asp Leu Ser Pro Ala Asp Thr Val Leu Phe
                165                 170                 175

aaa gag cag cgc att gcc gcc ttc gtt acc gat gcc ggc ggc ccc acc 576
Lys Glu Gln Arg Ile Ala Ala Phe Val Thr Asp Ala Gly Gly Pro Thr
            180                 185                 190

ggg cat acg gcg att ttg ggc agg agc ttg gac atc ccg tcc gtc gtc 624
Gly His Thr Ala Ile Leu Gly Arg Ser Leu Asp Ile Pro Ser Val Val
        195                 200                 205

ggg ctg cac aac gcg cgc aaa ctg att acc gag ggc gaa acg gtc att 672
Gly Leu His Asn Ala Arg Lys Leu Ile Thr Glu Gly Glu Thr Val Ile
    210                 215                 220

gtg gac ggt atc aac ggc gtg ttg att atc gcg ccg gat gag tcg gtg 720
Val Asp Gly Ile Asn Gly Val Leu Ile Ile Ala Pro Asp Glu Ser Val
225                 230                 235                 240

ttg aac gaa tac cgc cgc cgt gcc cgc gaa tac cgc agc cac aaa cgc 768
Leu Asn Glu Tyr Arg Arg Arg Ala Arg Glu Tyr Arg Ser His Lys Arg
                245                 250                 255

gat ttg aac aag ctc aaa aaa acc gcc gcc gcc acc gct gac ggg gtc 816
Asp Leu Asn Lys Leu Lys Lys Thr Ala Ala Ala Thr Ala Asp Gly Val
            260                 265                 270

tgc atc gag ctt gtg ggc aat ata gaa tcc gcc gaa gac gtg aaa ccg 864
Cys Ile Glu Leu Val Gly Asn Ile Glu Ser Ala Glu Asp Val Lys Pro
        275                 280                 285

ctg cac aac ctc ggc gca gac ggc atc ggg ctg ttc cgc agc gag ttt 912
Leu His Asn Leu Gly Ala Asp Gly Ile Gly Leu Phe Arg Ser Glu Phe
    290                 295                 300

ctt tac ctg aac cgc gat acg atg ccg tct gaa gac gag cag tac gaa 960
Leu Tyr Leu Asn Arg Asp Thr Met Pro Ser Glu Asp Glu Gln Tyr Glu
305                 310                 315                 320

gtg tac agc gcg att gtc aaa aaa atg aaa ggc aaa agc gta acg ata 1008
Val Tyr Ser Ala Ile Val Lys Lys Met Lys Gly Lys Ser Val Thr Ile
                325                 330                 335

cgg aca gtc gat ttg ggt gtg gac aaa aac ccg cgc tgg ttc ggg aaa 1056
Arg Thr Val Asp Leu Gly Val Asp Lys Asn Pro Arg Trp Phe Gly Lys
            340                 345                 350

aac agc acg ccc aac ggc agc ctc aac ccc gcg ctg ggc atg acc ggc 1104
Asn Ser Thr Pro Asn Gly Ser Leu Asn Pro Ala Leu Gly Met Thr Gly
        355                 360                 365

atc cgc ctg tgc ctt gcc gaa ccg gtc atg ttc cgc acc cag atg cgc 1152
Ile Arg Leu Cys Leu Ala Glu Pro Val Met Phe Arg Thr Gln Met Arg
    370                 375                 380

gcc atc ctc cgc gcc gcc gcc cac ggc ccc gtg cgg atg atg tgg ccg 1200
Ala Ile Leu Arg Ala Ala Ala His Gly Pro Val Arg Met Met Trp Pro
385                 390                 395                 400

atg att acc tcc gta tcc gaa gtg cgc cag tgc ctc atc cac ctc gac 1248
Met Ile Thr Ser Val Ser Glu Val Arg Gln Cys Leu Ile His Leu Asp
                405                 410                 415

acc gcg caa cgc cag ctt gcc gaa cgc ggc gat gcc ttc ggt aaa gtc 1296
Thr Ala Gln Arg Gln Leu Ala Glu Arg Gly Asp Ala Phe Gly Lys Val
            420                 425                 430

ggc atc ggc tgt atg att gaa att ccg tct gcc gcg ctg acc gtc ggc 1344
Gly Ile Gly Cys Met Ile Glu Ile Pro Ser Ala Ala Leu Thr Val Gly
        435                 440                 445

agt att ttg aaa ctg gtc gat ttc atc tcc gtc ggt acc aac gac ctg    1392
Ser Ile Leu Lys Leu Val Asp Phe Ile Ser Val Gly Thr Asn Asp Leu
    450                 455                 460

att caa tac atc ttg tcc gtc gat cgc ggc gac gac agc gtc agc cac    1440
Ile Gln Tyr Ile Leu Ser Val Asp Arg Gly Asp Asp Ser Val Ser His
465                 470                 475                 480

ctc tac cag ccc ggc cat ccc gcc gtg ctg aaa atg ctg caa cac gtc    1488
Leu Tyr Gln Pro Gly His Pro Ala Val Leu Lys Met Leu Gln His Val
                485                 490                 495

atc cgt acc gcc aac cgc atg gac aaa gac gta tcc gta tgc ggc gag    1536
Ile Arg Thr Ala Asn Arg Met Asp Lys Asp Val Ser Val Cys Gly Glu
            500                 505                 510

atg gcg ggc gat acc gcg ttt acc cgc gtt tta ttg ggt atg ggg ctg    1584
Met Ala Gly Asp Thr Ala Phe Thr Arg Val Leu Leu Gly Met Gly Leu
        515                 520                 525

cgc cgt ttt tcc atg aac ccc aac aac atc ctg ccc gtc aaa aac atc    1632
Arg Arg Phe Ser Met Asn Pro Asn Asn Ile Leu Pro Val Lys Asn Ile
    530                 535                 540

att ctg cac agc aat gtc gga cag ctc gaa agc gat att gtg aaa gtc    1680
Ile Leu His Ser Asn Val Gly Gln Leu Glu Ser Asp Ile Val Lys Val
545                 550                 555                 560

atc cgc tgc gaa gac gaa gag aag tcg gaa aag ctg atc aaa cag atg    1728
Ile Arg Cys Glu Asp Glu Glu Lys Ser Glu Lys Leu Ile Lys Gln Met
                565                 570                 575

aac agc gtg tct gtc gag gaa gaa gcc gac ttc aag ggg cgg aaa        1773
Asn Ser Val Ser Val Glu Glu Glu Ala Asp Phe Lys Gly Arg Lys
            580                 585                 590

<210> 78
<211> 591
<212> PRT
<213> Neisseria meningitidis

<400> 78
Met Ser Ile Val Leu His Gly Val Ala Ala Gly Lys Gly Ile Ala Val
  1               5                  10                  15

Gly Cys Ala His Leu Ile Ala Arg Gly Thr Glu Glu Val Pro Gln Tyr

            20                  25                  30

Asp Val Ala Glu Ala Asp Thr Asp Ala Glu Ala Glu Arg Phe Asp Ala
        35                  40                  45

Ala Val Lys Ala Thr Arg Lys Glu Leu Glu Gln Leu Arg Ser Ala Ile
    50                  55                  60

Pro Glu Asn Ala Pro Thr Glu Leu Gly Ala Phe Ile Ser Leu His Leu
65                  70                  75                  80

Met Leu Leu Thr Asp Val Thr Leu Ser Arg Glu Pro Val Asp Ile Leu
                85                  90                  95

Arg Glu Gln Lys Ile Asn Ala Glu Trp Ala Leu Lys Gln Gln Ser Asp
            100                 105                 110

Lys Leu Ala Ala Gln Phe Asp Asn Met Asp Asp Ala Tyr Leu Arg Glu
        115                 120                 125

Arg Lys Gln Asp Met Leu Gln Val Val Arg Arg Ile His Asn Asn Leu
    130                 135                 140

Ile Gly Gln Gly Asn Glu Leu Glu Val Ala Asp Asn Leu Phe Asp Glu
145                 150                 155                 160

Thr Val Leu Ile Ala Asn Asp Leu Ser Pro Ala Asp Thr Val Leu Phe
                165                 170                 175

Lys Glu Gln Arg Ile Ala Ala Phe Val Thr Asp Ala Gly Gly Pro Thr
            180                 185                 190

Gly His Thr Ala Ile Leu Gly Arg Ser Leu Asp Ile Pro Ser Val Val
        195                 200                 205

Gly Leu His Asn Ala Arg Lys Leu Ile Thr Glu Gly Glu Thr Val Ile
    210                 215                 220

Val Asp Gly Ile Asn Gly Val Leu Ile Ile Ala Pro Asp Glu Ser Val
225                 230                 235                 240

Leu Asn Glu Tyr Arg Arg Arg Ala Arg Glu Tyr Arg Ser His Lys Arg
                245                 250                 255

Asp Leu Asn Lys Leu Lys Lys Thr Ala Ala Ala Thr Ala Asp Gly Val
            260                 265                 270

Cys Ile Glu Leu Val Gly Asn Ile Glu Ser Ala Glu Asp Val Lys Pro

        275                 280                 285

Leu His Asn Leu Gly Ala Asp Gly Ile Gly Leu Phe Arg Ser Glu Phe
    290                 295                 300

Leu Tyr Leu Asn Arg Asp Thr Met Pro Ser Glu Asp Glu Gln Tyr Glu
305                 310                 315                 320

Val Tyr Ser Ala Ile Val Lys Lys Met Lys Gly Lys Ser Val Thr Ile
                325                 330                 335

Arg Thr Val Asp Leu Gly Val Asp Lys Asn Pro Arg Trp Phe Gly Lys
            340                 345                 350

Asn Ser Thr Pro Asn Gly Ser Leu Asn Pro Ala Leu Gly Met Thr Gly
        355                 360                 365

Ile Arg Leu Cys Leu Ala Glu Pro Val Met Phe Arg Thr Gln Met Arg
    370                 375                 380

Ala Ile Leu Arg Ala Ala Ala His Gly Pro Val Arg Met Met Trp Pro
385                 390                 395                 400

Met Ile Thr Ser Val Ser Glu Val Arg Gln Cys Leu Ile His Leu Asp
                405                 410                 415

Thr Ala Gln Arg Gln Leu Ala Glu Arg Gly Asp Ala Phe Gly Lys Val
            420                 425                 430

Gly Ile Gly Cys Met Ile Glu Ile Pro Ser Ala Ala Leu Thr Val Gly
        435                 440                 445

Ser Ile Leu Lys Leu Val Asp Phe Ile Ser Val Gly Thr Asn Asp Leu
    450                 455                 460

Ile Gln Tyr Ile Leu Ser Val Asp Arg Gly Asp Asp Ser Val Ser His
465                 470                 475                 480

Leu Tyr Gln Pro Gly His Pro Ala Val Leu Lys Met Leu Gln His Val
                485                 490                 495

Ile Arg Thr Ala Asn Arg Met Asp Lys Asp Val Ser Val Cys Gly Glu
            500                 505                 510

Met Ala Gly Asp Thr Ala Phe Thr Arg Val Leu Leu Gly Met Gly Leu
        515                 520                 525

Arg Arg Phe Ser Met Asn Pro Asn Asn Ile Leu Pro Val Lys Asn Ile

530                 535                 540

Ile Leu His Ser Asn Val Gly Gln Leu Glu Ser Asp Ile Val Lys Val
545                 550                 555                 560

Ile Arg Cys Glu Asp Glu Glu Lys Ser Glu Lys Leu Ile Lys Gln Met
                565                 570                 575

Asn Ser Val Ser Val Glu Glu Glu Ala Asp Phe Lys Gly Arg Lys
            580                 585                 590

<210> 79
<211> 1062
<212> DNA
<213> Neisseria meningitidis

<220>
<221> CDS
<222> (1)..(1062)

<400> 79
atg gaa aaa gaa ttc agg ata tta aat atc gta tcg gcc aag att tgg      48
Met Glu Lys Glu Phe Arg Ile Leu Asn Ile Val Ser Ala Lys Ile Trp
  1               5                  10                  15

ggt gga ggc gaa caa tat gtc tat gat gtt tca aaa gca ttg ggg ctt      96
Gly Gly Gly Glu Gln Tyr Val Tyr Asp Val Ser Lys Ala Leu Gly Leu
             20                  25                  30

cgg gac tgc aca atg ttt acc gcc gtc aat aaa aat aat gaa ttg atg     144
Arg Asp Cys Thr Met Phe Thr Ala Val Asn Lys Asn Asn Glu Leu Met
         35                  40                  45

cac agg cga ttt tcc gaa gtt tct tcc gtt ttc acg aca cgc ctt cac     192
His Arg Arg Phe Ser Glu Val Ser Ser Val Phe Thr Thr Arg Leu His
     50                  55                  60

acg ctc aac ggg ctg ttt tcg ctc tac gca ctt acc cgc ttt atc cgg     240
Thr Leu Asn Gly Leu Phe Ser Leu Tyr Ala Leu Thr Arg Phe Ile Arg
 65                  70                  75                  80

aaa aac cgc att tcc cac ctg atg ata cac acc ggc aaa att gcc gcc     288
Lys Asn Arg Ile Ser His Leu Met Ile His Thr Gly Lys Ile Ala Ala
                 85                  90                  95

tta tcc ata ctt ttg aaa aaa ctg acc ggg gtg cgc ctg ata ttt gtc     336

Leu Ser Ile Leu Leu Lys Lys Leu Thr Gly Val Arg Leu Ile Phe Val
            100                 105                 110

aaa cat aat gtt gtc gcc aac aaa act gat ttt tat cac cgc ctg ata 384
Lys His Asn Val Val Ala Asn Lys Thr Asp Phe Tyr His Arg Leu Ile
        115                 120                 125

cag aaa aac acc gac cgc ttt att tgc gtt tcc cgt ctg gtt tac gat 432
Gln Lys Asn Thr Asp Arg Phe Ile Cys Val Ser Arg Leu Val Tyr Asp
    130                 135                 140

gtg caa acc gcc gac aat ccc ttt aaa gaa aaa tac cgg att gtt cat 480
Val Gln Thr Ala Asp Asn Pro Phe Lys Glu Lys Tyr Arg Ile Val His
145                 150                 155                 160

aac ggt atc gat acc ggc cgt ttc cct ccc cct caa gaa aaa ccc gac 528
Asn Gly Ile Asp Thr Gly Arg Phe Pro Pro Pro Gln Glu Lys Pro Asp
                165                 170                 175

agc cgt ttt ttt acc gtc gcc tac gcc ggc agg atc agt ccg gaa aaa 576
Ser Arg Phe Phe Thr Val Ala Tyr Ala Gly Arg Ile Ser Pro Glu Lys
            180                 185                 190

gga ttg gaa aac ctg att gaa gcc tgt gtg ata ctg cat cgg aaa tat 624
Gly Leu Glu Asn Leu Ile Glu Ala Cys Val Ile Leu His Arg Lys Tyr
        195                 200                 205

cct caa atc agg ctc aaa ttg gca ggg gac gga cat ccg gat tat atg 672
Pro Gln Ile Arg Leu Lys Leu Ala Gly Asp Gly His Pro Asp Tyr Met
    210                 215                 220

tgt cgc ctg aag cgg gac gta tct gct tcg gga gca gaa cca ttt gtt 720
Cys Arg Leu Lys Arg Asp Val Ser Ala Ser Gly Ala Glu Pro Phe Val
225                 230                 235                 240

tct ttt gaa ggg ttt acc gaa aaa ctt gct tcg ttt tac cgc caa agc 768
Ser Phe Glu Gly Phe Thr Glu Lys Leu Ala Ser Phe Tyr Arg Gln Ser
                245                 250                 255

gat gtc gtg gtt ttg ccc agc ctc gtc ccg gag gca ttc ggt ttg tca 816
Asp Val Val Val Leu Pro Ser Leu Val Pro Glu Ala Phe Gly Leu Ser
            260                 265                 270

tta tgc gag gcg atg tac tgc cga acg gcg gtg att tcc aat act ttg 864
Leu Cys Glu Ala Met Tyr Cys Arg Thr Ala Val Ile Ser Asn Thr Leu
        275                 280                 285

ggc gcg caa aag gaa att atc gaa cat cat caa tcg ggg att ctg ctg 912

Gly Ala Gln Lys Glu Ile Ile Glu His His Gln Ser Gly Ile Leu Leu
    290                 295                 300

gat agg ctg aca cct gaa tct ttg gcg gac gaa atc gaa cgc ctc gtc     960
Asp Arg Leu Thr Pro Glu Ser Leu Ala Asp Glu Ile Glu Arg Leu Val
305                 310                 315                 320

tta aac cct gaa acg aaa aac gca ctg gca acg gca gga cat caa tgc     1008
Leu Asn Pro Glu Thr Lys Asn Ala Leu Ala Thr Ala Gly His Gln Cys
                325                 330                 335

gtc gcc aac cgt ttt acc atc aac cat acc gcc gac aaa tta ttg gat     1056
Val Ala Asn Arg Phe Thr Ile Asn His Thr Ala Asp Lys Leu Leu Asp
            340                 345                 350

gca ata                                                             1062
Ala Ile

<210> 80
<211> 354
<212> PRT
<213> Neisseria meningitidis

<400> 80
Met Glu Lys Glu Phe Arg Ile Leu Asn Ile Val Ser Ala Lys Ile Trp
  1               5                  10                  15

Gly Gly Gly Glu Gln Tyr Val Tyr Asp Val Ser Lys Ala Leu Gly Leu
             20                  25                  30

Arg Asp Cys Thr Met Phe Thr Ala Val Asn Lys Asn Asn Glu Leu Met
         35                  40                  45

His Arg Arg Phe Ser Glu Val Ser Ser Val Phe Thr Thr Arg Leu His
     50                  55                  60

Thr Leu Asn Gly Leu Phe Ser Leu Tyr Ala Leu Thr Arg Phe Ile Arg
 65                  70                  75                  80

Lys Asn Arg Ile Ser His Leu Met Ile His Thr Gly Lys Ile Ala Ala
                 85                  90                  95

Leu Ser Ile Leu Leu Lys Lys Leu Thr Gly Val Arg Leu Ile Phe Val
            100                 105                 110

Lys His Asn Val Val Ala Asn Lys Thr Asp Phe Tyr His Arg Leu Ile
        115                 120                 125

Gln Lys Asn Thr Asp Arg Phe Ile Cys Val Ser Arg Leu Val Tyr Asp
    130                 135                 140

Val Gln Thr Ala Asp Asn Pro Phe Lys Glu Lys Tyr Arg Ile Val His
145                 150                 155                 160

Asn Gly Ile Asp Thr Gly Arg Phe Pro Pro Pro Gln Glu Lys Pro Asp
                165                 170                 175

Ser Arg Phe Phe Thr Val Ala Tyr Ala Gly Arg Ile Ser Pro Glu Lys
            180                 185                 190

Gly Leu Glu Asn Leu Ile Glu Ala Cys Val Ile Leu His Arg Lys Tyr
        195                 200                 205

Pro Gln Ile Arg Leu Lys Leu Ala Gly Asp Gly His Pro Asp Tyr Met
    210                 215                 220

Cys Arg Leu Lys Arg Asp Val Ser Ala Ser Gly Ala Glu Pro Phe Val
225                 230                 235                 240

Ser Phe Glu Gly Phe Thr Glu Lys Leu Ala Ser Phe Tyr Arg Gln Ser
                245                 250                 255

Asp Val Val Val Leu Pro Ser Leu Val Pro Glu Ala Phe Gly Leu Ser
            260                 265                 270

Leu Cys Glu Ala Met Tyr Cys Arg Thr Ala Val Ile Ser Asn Thr Leu
        275                 280                 285

Gly Ala Gln Lys Glu Ile Ile Glu His His Gln Ser Gly Ile Leu Leu
    290                 295                 300

Asp Arg Leu Thr Pro Glu Ser Leu Ala Asp Glu Ile Glu Arg Leu Val
305                 310                 315                 320

Leu Asn Pro Glu Thr Lys Asn Ala Leu Ala Thr Ala Gly His Gln Cys
                325                 330                 335

Val Ala Asn Arg Phe Thr Ile Asn His Thr Ala Asp Lys Leu Leu Asp
            340                 345                 350

Ala Ile

<210> 81
<211> 795
<212> DNA
<213> Neisseria meningitidis

<220>
<221> CDS
<222> (1)..(795)

<400> 81
atg gcg gct gtt ttt tct gta tgt gcc tgt ttt atg tgt tcc tgc ctt 48
Met Ala Ala Val Phe Ser Val Cys Ala Cys Phe Met Cys Ser Cys Leu
1               5                   10                  15

gtt gtc aaa aat acc gtt atc gga agc gga cgc acc aaa atc gcc gtg 96
Val Val Lys Asn Thr Val Ile Gly Ser Gly Arg Thr Lys Ile Ala Val
            20                  25                  30

ccg ctt gtc gcc cgc gat gcc gcc gta ctt tcc gcc gta ctt gac caa 144
Pro Leu Val Ala Arg Asp Ala Ala Val Leu Ser Ala Val Leu Asp Gln
        35                  40                  45

atc aaa aac ctg ccc ttc gat atc gtc gag ttc cgc gcc gat ttt ttg 192
Ile Lys Asn Leu Pro Phe Asp Ile Val Glu Phe Arg Ala Asp Phe Leu
    50                  55                  60

gaa tgc gcg ggc agc atc ggc gaa gta ttg cgc cac acg cag acc gtc 240
Glu Cys Ala Gly Ser Ile Gly Glu Val Leu Arg His Thr Gln Thr Val
65                  70                  75                  80

cgc gac gcg cta ccc gac aag ccg ctg ctg ttt acg ttc aga cgg cat 288
Arg Asp Ala Leu Pro Asp Lys Pro Leu Leu Phe Thr Phe Arg Arg His
                85                  90                  95

ggc gaa ggc ggc tcg ttc ccg tgt tcg gac gat tat tat ttt gaa ctg 336
Gly Glu Gly Gly Ser Phe Pro Cys Ser Asp Asp Tyr Tyr Phe Glu Leu
            100                 105                 110

ctc gac gcg ctg atc gaa agc cgc ctg ccc gac atc atc gac atc gag 384
Leu Asp Ala Leu Ile Glu Ser Arg Leu Pro Asp Ile Ile Asp Ile Glu
        115                 120                 125

ctg ttt tcc ggc gaa acc gcc gtc cgg tgc gcc gtg gca aat gct caa 432
Leu Phe Ser Gly Glu Thr Ala Val Arg Cys Ala Val Ala Asn Ala Gln
    130                 135                 140

aaa aac ggc atc gcc gcc ctg ctc tgc aat cat gag ttt cac cgc acg 480
Lys Asn Gly Ile Ala Ala Leu Leu Cys Asn His Glu Phe His Arg Thr

145                 150                 155                 160

ccg ccg caa gaa gaa atc gta tgc cgt ctg aaa cag atg gag gac tgc     528
Pro Pro Gln Glu Glu Ile Val Cys Arg Leu Lys Gln Met Glu Asp Cys
                165                 170                 175

ggc gcg gac atc tgc aaa att gcg gtg atg ccg caa agc gcg gaa gat     576
Gly Ala Asp Ile Cys Lys Ile Ala Val Met Pro Gln Ser Ala Glu Asp
            180                 185                 190

gtg ctg act ttg ctt tcc gcc acg ctc aaa gcg aaa gag ctt gcc gcc     624
Val Leu Thr Leu Leu Ser Ala Thr Leu Lys Ala Lys Glu Leu Ala Ala
        195                 200                 205

aaa ccg att gtt acg atg tcg atg ggg cag acg ggg gcg gtc agc cgg     672
Lys Pro Ile Val Thr Met Ser Met Gly Gln Thr Gly Ala Val Ser Arg
    210                 215                 220

ctt gcc gga cag gtg ttc ggc tca agc atc acg ttc ggt tcg gga acg     720
Leu Ala Gly Gln Val Phe Gly Ser Ser Ile Thr Phe Gly Ser Gly Thr
225                 230                 235                 240

caa aac tcc gcg ccg ggg caa atc ggc gta tcc gcc ctc cgt gcg aca     768
Gln Asn Ser Ala Pro Gly Gln Ile Gly Val Ser Ala Leu Arg Ala Thr
                245                 250                 255

ctc gac tgc ctc gaa aac gga gca gac                                 795
Leu Asp Cys Leu Glu Asn Gly Ala Asp
            260                 265

<210> 82
<211> 265
<212> PRT
<213> Neisseria meningitidis

<400> 82
Met Ala Ala Val Phe Ser Val Cys Ala Cys Phe Met Cys Ser Cys Leu
1               5                   10                  15

Val Val Lys Asn Thr Val Ile Gly Ser Gly Arg Thr Lys Ile Ala Val
            20                  25                  30

Pro Leu Val Ala Arg Asp Ala Ala Val Leu Ser Ala Val Leu Asp Gln
        35                  40                  45

Ile Lys Asn Leu Pro Phe Asp Ile Val Glu Phe Arg Ala Asp Phe Leu
    50                  55                  60

Glu Cys Ala Gly Ser Ile Gly Glu Val Leu Arg His Thr Gln Thr Val
65                  70                  75                  80

Arg Asp Ala Leu Pro Asp Lys Pro Leu Leu Phe Thr Phe Arg Arg His
                85                  90                  95

Gly Glu Gly Gly Ser Phe Pro Cys Ser Asp Asp Tyr Tyr Phe Glu Leu
            100                 105                 110

Leu Asp Ala Leu Ile Glu Ser Arg Leu Pro Asp Ile Ile Asp Ile Glu
        115                 120                 125

Leu Phe Ser Gly Glu Thr Ala Val Arg Cys Ala Val Ala Asn Ala Gln
    130                 135                 140

Lys Asn Gly Ile Ala Ala Leu Leu Cys Asn His Glu Phe His Arg Thr
145                 150                 155                 160

Pro Pro Gln Glu Glu Ile Val Cys Arg Leu Lys Gln Met Glu Asp Cys
                165                 170                 175

Gly Ala Asp Ile Cys Lys Ile Ala Val Met Pro Gln Ser Ala Glu Asp
            180                 185                 190

Val Leu Thr Leu Leu Ser Ala Thr Leu Lys Ala Lys Glu Leu Ala Ala
        195                 200                 205

Lys Pro Ile Val Thr Met Ser Met Gly Gln Thr Gly Ala Val Ser Arg
    210                 215                 220

Leu Ala Gly Gln Val Phe Gly Ser Ser Ile Thr Phe Gly Ser Gly Thr
225                 230                 235                 240

Gln Asn Ser Ala Pro Gly Gln Ile Gly Val Ser Ala Leu Arg Ala Thr
                245                 250                 255

Leu Asp Cys Leu Glu Asn Gly Ala Asp
            260                 265

<210> 83
<211> 876
<212> DNA
<213> Neisseria meningitidis

<220>

<221> CDS
<222> (1)..(876)

<400> 83
ttg acc gtc cga acg aaa aag acg gcg cat tat acc cta ttc cat tcc     48
Leu Thr Val Arg Thr Lys Lys Thr Ala His Tyr Thr Leu Phe His Ser
1               5                   10                  15

gac cga aaa ccg aac atg act act ctc aaa ccc gcc ctg ccc gct tat     96
Asp Arg Lys Pro Asn Met Thr Thr Leu Lys Pro Ala Leu Pro Ala Tyr
            20                  25                  30

ctg gac aac atc cgc atc atc ctc acg cgc acc agc cat ccc gcc aac     144
Leu Asp Asn Ile Arg Ile Ile Leu Thr Arg Thr Ser His Pro Ala Asn
        35                  40                  45

atc ggc tct gcc gcg cgc gcg atg aaa aca atg ggt ctg cac aaa ctg     192
Ile Gly Ser Ala Ala Arg Ala Met Lys Thr Met Gly Leu His Lys Leu
    50                  55                  60

acc atc gtc gcc cca aat ctg atg gca acg ccg atg acg gaa aac ccg     240
Thr Ile Val Ala Pro Asn Leu Met Ala Thr Pro Met Thr Glu Asn Pro
65                  70                  75                  80

ccc gtg ttt gac ccg gag cat cct caa tcg ttt aaa tta ccg gaa gaa     288
Pro Val Phe Asp Pro Glu His Pro Gln Ser Phe Lys Leu Pro Glu Glu
                85                  90                  95

agc ttc atc ctc gct tcc ggc gcg gca gac gtt ttg gaa aat gcc acc     336
Ser Phe Ile Leu Ala Ser Gly Ala Ala Asp Val Leu Glu Asn Ala Thr
            100                 105                 110

att gcc gct tct ttg gac gaa gcc ctt gcc gac acc acc atc gcc tgc     384
Ile Ala Ala Ser Leu Asp Glu Ala Leu Ala Asp Thr Thr Ile Ala Cys
        115                 120                 125

gcc ctg acc agc cgc cgc cgc gaa att act gcg ccg ctg caa acc ccg     432
Ala Leu Thr Ser Arg Arg Arg Glu Ile Thr Ala Pro Leu Gln Thr Pro
    130                 135                 140

cgc gat ttg gta tcc gaa tta ctg cag acc gca aac cga ggc gag aaa     480
Arg Asp Leu Val Ser Glu Leu Leu Gln Thr Ala Asn Arg Gly Glu Lys
145                 150                 155                 160

gtg gca ctg gtt ttc ggc aac gag act ttc ggc ttg agc atc gaa gaa     528
Val Ala Leu Val Phe Gly Asn Glu Thr Phe Gly Leu Ser Ile Glu Glu
                165                 170                 175

gtc caa gcc tgc aac cga ctg atg acc atc aac ggc aat ccc gac tat    576
Val Gln Ala Cys Asn Arg Leu Met Thr Ile Asn Gly Asn Pro Asp Tyr
            180                 185                 190

ttc tcg ctc aac ctc gcc caa gcc gtg cag gtc gtg tgc tac gaa atc    624
Phe Ser Leu Asn Leu Ala Gln Ala Val Gln Val Val Cys Tyr Glu Ile
        195                 200                 205

ttc agc caa acc ggt tcg ccc atg acc cat ctt caa caa gaa gac cac    672
Phe Ser Gln Thr Gly Ser Pro Met Thr His Leu Gln Gln Glu Asp His
    210                 215                 220

gct gcg acc cac gag caa atc aaa ggc atg gtc gcc cac atg gaa agc    720
Ala Ala Thr His Glu Gln Ile Lys Gly Met Val Ala His Met Glu Ser
225                 230                 235                 240

gtg atg aac gac atc ggc ttt ttc aac cgc cgc aac ggc gag cgt ctg    768
Val Met Asn Asp Ile Gly Phe Phe Asn Arg Arg Asn Gly Glu Arg Leu
                245                 250                 255

atg cgc cgt atg cag agc ctg ttc gga cgc gcc aac acg caa acc gaa    816
Met Arg Arg Met Gln Ser Leu Phe Gly Arg Ala Asn Thr Gln Thr Glu
            260                 265                 270

gac atc gat atc ctg cgc ggt ttt ttc aat acc gtc agc cat cgt atc    864
Asp Ile Asp Ile Leu Arg Gly Phe Phe Asn Thr Val Ser His Arg Ile
        275                 280                 285

cat aaa aaa gac                                                    876
His Lys Lys Asp
    290

<210> 84
<211> 292
<212> PRT
<213> Neisseria meningitidis

<400> 84
Leu Thr Val Arg Thr Lys Lys Thr Ala His Tyr Thr Leu Phe His Ser
  1               5                  10                  15

Asp Arg Lys Pro Asn Met Thr Thr Leu Lys Pro Ala Leu Pro Ala Tyr
             20                  25                  30

Leu Asp Asn Ile Arg Ile Ile Leu Thr Arg Thr Ser His Pro Ala Asn
         35                  40                  45

Ile Gly Ser Ala Ala Arg Ala Met Lys Thr Met Gly Leu His Lys Leu
    50                  55                  60

Thr Ile Val Ala Pro Asn Leu Met Ala Thr Pro Met Thr Glu Asn Pro
65                  70                  75                  80

Pro Val Phe Asp Pro Glu His Pro Gln Ser Phe Lys Leu Pro Glu Glu
                85                  90                  95

Ser Phe Ile Leu Ala Ser Gly Ala Ala Asp Val Leu Glu Asn Ala Thr
            100                 105                 110

Ile Ala Ala Ser Leu Asp Glu Ala Leu Ala Asp Thr Thr Ile Ala Cys
        115                 120                 125

Ala Leu Thr Ser Arg Arg Arg Glu Ile Thr Ala Pro Leu Gln Thr Pro
    130                 135                 140

Arg Asp Leu Val Ser Glu Leu Leu Gln Thr Ala Asn Arg Gly Glu Lys
145                 150                 155                 160

Val Ala Leu Val Phe Gly Asn Glu Thr Phe Gly Leu Ser Ile Glu Glu
                165                 170                 175

Val Gln Ala Cys Asn Arg Leu Met Thr Ile Asn Gly Asn Pro Asp Tyr
            180                 185                 190

Phe Ser Leu Asn Leu Ala Gln Ala Val Gln Val Val Cys Tyr Glu Ile
        195                 200                 205

Phe Ser Gln Thr Gly Ser Pro Met Thr His Leu Gln Gln Glu Asp His
    210                 215                 220

Ala Ala Thr His Glu Gln Ile Lys Gly Met Val Ala His Met Glu Ser
225                 230                 235                 240

Val Met Asn Asp Ile Gly Phe Phe Asn Arg Arg Asn Gly Glu Arg Leu
                245                 250                 255

Met Arg Arg Met Gln Ser Leu Phe Gly Arg Ala Asn Thr Gln Thr Glu
            260                 265                 270

Asp Ile Asp Ile Leu Arg Gly Phe Phe Asn Thr Val Ser His Arg Ile
        275                 280                 285

His Lys Lys Asp
    290

<210> 85
<211> 1545
<212> DNA
<213> Neisseria meningitidis

<220>
<221> CDS
<222> (1)..(1545)

<400> 85
gtg cgt ctc aat cat ttc ata atg ata gcg att att ata tat gtg att 48
Val Arg Leu Asn His Phe Ile Met Ile Ala Ile Ile Ile Tyr Val Ile
1               5                   10                  15

tcc cct gca aac aag ccg gcc cgc cgc cac agc gtt ccc act tat ccg 96
Ser Pro Ala Asn Lys Pro Ala Arg Arg His Ser Val Pro Thr Tyr Pro
            20                  25                  30

gct ttg cct tat aat tgc ttt ttt tat gta aca gat tta cct atg aat 144
Ala Leu Pro Tyr Asn Cys Phe Phe Tyr Val Thr Asp Leu Pro Met Asn
        35                  40                  45

ttc ccc aaa aca gcg gcc tcc ctg ctg ctg ctt ctc gcc tcc ctc gcc 192
Phe Pro Lys Thr Ala Ala Ser Leu Leu Leu Leu Leu Ala Ser Leu Ala
    50                  55                  60

gca cac gcg ctc gat aca ggt cgc att ccg caa aac gaa atc gcc gta 240
Ala His Ala Leu Asp Thr Gly Arg Ile Pro Gln Asn Glu Ile Ala Val
65                  70                  75                  80

tat gtc caa gag ctt gac agc gga aaa gtc atc att gac cac cgc tcg 288
Tyr Val Gln Glu Leu Asp Ser Gly Lys Val Ile Ile Asp His Arg Ser
                85                  90                  95

gat gtc ccc gtc aac ccc gcc tcc aca atg aaa ctc gtt acc gcg ttt 336
Asp Val Pro Val Asn Pro Ala Ser Thr Met Lys Leu Val Thr Ala Phe
            100                 105                 110

gcc gcc ttc aaa acc ttc ggc agc aat tac cgc tgg gcg acc gag ttt 384
Ala Ala Phe Lys Thr Phe Gly Ser Asn Tyr Arg Trp Ala Thr Glu Phe
        115                 120                 125

aaa agc aac ggt acg gta aac gac ggc acg ctt gac gga aac ctg tat 432
Lys Ser Asn Gly Thr Val Asn Asp Gly Thr Leu Asp Gly Asn Leu Tyr
    130                 135                 140

tgg gcg ggc agc ggc gac ccc gtt ttc aat cag gaa aac ctg ctt gcc    480
Trp Ala Gly Ser Gly Asp Pro Val Phe Asn Gln Glu Asn Leu Leu Ala
145                 150                 155                 160

gtc caa cgc cag ttg cgc gaa caa ggc ata cgc aat atc acg gga cac    528
Val Gln Arg Gln Leu Arg Glu Gln Gly Ile Arg Asn Ile Thr Gly His
                165                 170                 175

ctg atg ctc gac cac agc ctg tgg ggc gaa gtc ggc agc ccc gac gat    576
Leu Met Leu Asp His Ser Leu Trp Gly Glu Val Gly Ser Pro Asp Asp
            180                 185                 190

ttc gaa gcc gac agc ggt tcg ccg ttt atg acg ccc ccc aat cca act    624
Phe Glu Ala Asp Ser Gly Ser Pro Phe Met Thr Pro Pro Asn Pro Thr
        195                 200                 205

atg ctg tct gcc ggt atg gtt atg gtg cgc gcc gaa cgc aat gcc gcc    672
Met Leu Ser Ala Gly Met Val Met Val Arg Ala Glu Arg Asn Ala Ala
    210                 215                 220

gac agt acc gac atc ctc acc gat ccg cct ttg ccg cat att ttc gcc    720
Asp Ser Thr Asp Ile Leu Thr Asp Pro Pro Leu Pro His Ile Phe Ala
225                 230                 235                 240

caa aac aac ttg aaa att acc gcc tcc caa gct gcc tgc cct tcg atc    768
Gln Asn Asn Leu Lys Ile Thr Ala Ser Gln Ala Ala Cys Pro Ser Ile
                245                 250                 255

aaa aaa ctg atg cgt gca tct ttt tcg gac aat acg ctg aaa ttg cgc    816
Lys Lys Leu Met Arg Ala Ser Phe Ser Asp Asn Thr Leu Lys Leu Arg
            260                 265                 270

ggc aat att ccc gag agc tgt ttg ggc aag cct gtc ggt gtc cgg atg    864
Gly Asn Ile Pro Glu Ser Cys Leu Gly Lys Pro Val Gly Val Arg Met
        275                 280                 285

ttc gcg ctt gac gaa ctg atc cgg caa agt ttt acc aac cac tgg ctg    912
Phe Ala Leu Asp Glu Leu Ile Arg Gln Ser Phe Thr Asn His Trp Leu
    290                 295                 300

ctc ggc ggc gga cgg att tca gac ggc atc ggc ata tcc gac acg ccg    960
Leu Gly Gly Gly Arg Ile Ser Asp Gly Ile Gly Ile Ser Asp Thr Pro
305                 310                 315                 320

gaa ggc gcg cag acg ctt gcc gtt gca cac tca aag ccg atg aag gaa    1008
Glu Gly Ala Gln Thr Leu Ala Val Ala His Ser Lys Pro Met Lys Glu
                325                 330                 335

att ttg acg gac atg aac aag cgt tcg gac aat cta att gcg cgt tcc     1056
Ile Leu Thr Asp Met Asn Lys Arg Ser Asp Asn Leu Ile Ala Arg Ser
            340                 345                 350

gtc ttc ctc aaa ctc ggc ggc gac ggc aaa ctg ccc gcc gtt tcc gaa     1104
Val Phe Leu Lys Leu Gly Gly Asp Gly Lys Leu Pro Ala Val Ser Glu
        355                 360                 365

cag gca gcg tct gcc gtc cgg cgt gaa ctt gcc gtg tcg ggc atc gat     1152
Gln Ala Ala Ser Ala Val Arg Arg Glu Leu Ala Val Ser Gly Ile Asp
    370                 375                 380

gtt gcg gat ttg gtt ttg gaa aac ggt tca ggt ctg tcc aga aaa gaa     1200
Val Ala Asp Leu Val Leu Glu Asn Gly Ser Gly Leu Ser Arg Lys Glu
385                 390                 395                 400

agg gta acg gcg aga atg atg gcg caa atg ttg gaa acg gct tat ttc     1248
Arg Val Thr Ala Arg Met Met Ala Gln Met Leu Glu Thr Ala Tyr Phe
                405                 410                 415

agc ccg ttt gca caa gat ttc atc gat acg ctg ccc atc gcc ggc aca     1296
Ser Pro Phe Ala Gln Asp Phe Ile Asp Thr Leu Pro Ile Ala Gly Thr
            420                 425                 430

gac ggg act tta cgc aac cgc ttc aaa caa agc ggc ggg ctg ttg cgc     1344
Asp Gly Thr Leu Arg Asn Arg Phe Lys Gln Ser Gly Gly Leu Leu Arg
        435                 440                 445

tta aaa acc ggc acg ctc aac aat gtc cgc gcc ctt gca ggt tat tgg     1392
Leu Lys Thr Gly Thr Leu Asn Asn Val Arg Ala Leu Ala Gly Tyr Trp
    450                 455                 460

ctg ggc gac aaa ccg atg gcg gtg gtc gtc atc atc aac agc ggc cgc     1440
Leu Gly Asp Lys Pro Met Ala Val Val Val Ile Ile Asn Ser Gly Arg
465                 470                 475                 480

gcc gtt tcc ctg ctg ccc gac ttg gac aac ttc gtt gcc aac aac atc     1488
Ala Val Ser Leu Leu Pro Asp Leu Asp Asn Phe Val Ala Asn Asn Ile
                485                 490                 495

atc tcc ggc ggc gat ggc tgg ctg gat gcg aaa ctg atg tgc aaa gaa     1536
Ile Ser Gly Gly Asp Gly Trp Leu Asp Ala Lys Leu Met Cys Lys Glu
            500                 505                 510

cgc cga gcc                                                         1545
Arg Arg Ala
        515

<210> 86
<211> 515
<212> PRT
<213> Neisseria meningitidis

<400> 86
Val Arg Leu Asn His Phe Ile Met Ile Ala Ile Ile Ile Tyr Val Ile
1               5                   10                  15

Ser Pro Ala Asn Lys Pro Ala Arg Arg His Ser Val Pro Thr Tyr Pro
            20                  25                  30

Ala Leu Pro Tyr Asn Cys Phe Phe Tyr Val Thr Asp Leu Pro Met Asn
        35                  40                  45

Phe Pro Lys Thr Ala Ala Ser Leu Leu Leu Leu Leu Ala Ser Leu Ala
    50                  55                  60

Ala His Ala Leu Asp Thr Gly Arg Ile Pro Gln Asn Glu Ile Ala Val
65                  70                  75                  80

Tyr Val Gln Glu Leu Asp Ser Gly Lys Val Ile Ile Asp His Arg Ser
                85                  90                  95

Asp Val Pro Val Asn Pro Ala Ser Thr Met Lys Leu Val Thr Ala Phe
            100                 105                 110

Ala Ala Phe Lys Thr Phe Gly Ser Asn Tyr Arg Trp Ala Thr Glu Phe
        115                 120                 125

Lys Ser Asn Gly Thr Val Asn Asp Gly Thr Leu Asp Gly Asn Leu Tyr
    130                 135                 140

Trp Ala Gly Ser Gly Asp Pro Val Phe Asn Gln Glu Asn Leu Leu Ala
145                 150                 155                 160

Val Gln Arg Gln Leu Arg Glu Gln Gly Ile Arg Asn Ile Thr Gly His
                165                 170                 175

Leu Met Leu Asp His Ser Leu Trp Gly Glu Val Gly Ser Pro Asp Asp
            180                 185                 190

Phe Glu Ala Asp Ser Gly Ser Pro Phe Met Thr Pro Pro Asn Pro Thr
        195                 200                 205

Met Leu Ser Ala Gly Met Val Met Val Arg Ala Glu Arg Asn Ala Ala
    210                 215                 220

Asp Ser Thr Asp Ile Leu Thr Asp Pro Pro Leu Pro His Ile Phe Ala
225                 230                 235                 240

Gln Asn Asn Leu Lys Ile Thr Ala Ser Gln Ala Ala Cys Pro Ser Ile
                245                 250                 255

Lys Lys Leu Met Arg Ala Ser Phe Ser Asp Asn Thr Leu Lys Leu Arg
            260                 265                 270

Gly Asn Ile Pro Glu Ser Cys Leu Gly Lys Pro Val Gly Val Arg Met
        275                 280                 285

Phe Ala Leu Asp Glu Leu Ile Arg Gln Ser Phe Thr Asn His Trp Leu
    290                 295                 300

Leu Gly Gly Gly Arg Ile Ser Asp Gly Ile Gly Ile Ser Asp Thr Pro
305                 310                 315                 320

Glu Gly Ala Gln Thr Leu Ala Val Ala His Ser Lys Pro Met Lys Glu
                325                 330                 335

Ile Leu Thr Asp Met Asn Lys Arg Ser Asp Asn Leu Ile Ala Arg Ser
            340                 345                 350

Val Phe Leu Lys Leu Gly Gly Asp Gly Lys Leu Pro Ala Val Ser Glu
        355                 360                 365

Gln Ala Ala Ser Ala Val Arg Arg Glu Leu Ala Val Ser Gly Ile Asp
    370                 375                 380

Val Ala Asp Leu Val Leu Glu Asn Gly Ser Gly Leu Ser Arg Lys Glu
385                 390                 395                 400

Arg Val Thr Ala Arg Met Met Ala Gln Met Leu Glu Thr Ala Tyr Phe
                405                 410                 415

Ser Pro Phe Ala Gln Asp Phe Ile Asp Thr Leu Pro Ile Ala Gly Thr
            420                 425                 430

Asp Gly Thr Leu Arg Asn Arg Phe Lys Gln Ser Gly Gly Leu Leu Arg
        435                 440                 445

Leu Lys Thr Gly Thr Leu Asn Asn Val Arg Ala Leu Ala Gly Tyr Trp
    450                 455                 460

Leu Gly Asp Lys Pro Met Ala Val Val Val Ile Ile Asn Ser Gly Arg
465                 470                 475                 480

Ala Val Ser Leu Leu Pro Asp Leu Asp Asn Phe Val Ala Asn Asn Ile
                485                 490                 495

Ile Ser Gly Gly Asp Gly Trp Leu Asp Ala Lys Leu Met Cys Lys Glu
            500                 505                 510

Arg Arg Ala
        515

<210> 87
<211> 840
<212> DNA
<213> Neisseria meningitidis

<220>
<221> CDS
<222> (1)..(840)

<400> 87
atg gat aaa gaa cga att tta acc ccc gca gtc gtg ttt tcc gtc gca    48
Met Asp Lys Glu Arg Ile Leu Thr Pro Ala Val Val Phe Ser Val Ala
1               5                   10                  15

ctg ctg cat ttg gca atg gtg gca ttg ctc tgg cag gcg cac aag ctg    96
Leu Leu His Leu Ala Met Val Ala Leu Leu Trp Gln Ala His Lys Leu
            20                  25                  30

ccc gtg ata gag tca ggc aat gtt att gaa ttt gtc gat ttg ggc gat    144
Pro Val Ile Glu Ser Gly Asn Val Ile Glu Phe Val Asp Leu Gly Asp
        35                  40                  45

ttt ggc gga ggg gac ggc gca ccc gaa ggt gca ggc gcg cct gcc gcg    192
Phe Gly Gly Gly Asp Gly Ala Pro Glu Gly Ala Gly Ala Pro Ala Ala
    50                  55                  60

ccc gaa ccg caa ccc gtg ccc gag ccg ccc aaa cct gtc gag ccg ccc    240
Pro Glu Pro Gln Pro Val Pro Glu Pro Pro Lys Pro Val Glu Pro Pro
65                  70                  75                  80

aag ccg gtt ttg aag ccg gtg gtt acg aaa aag gcg gat gcg gat att    288
Lys Pro Val Leu Lys Pro Val Val Thr Lys Lys Ala Asp Ala Asp Ile
                85                  90                  95

cag cag cct aag gaa gag ccg aaa cct gaa gaa aag ccg aaa ccc gaa    336
Gln Gln Pro Lys Glu Glu Pro Lys Pro Glu Glu Lys Pro Lys Pro Glu

            100                 105                 110

gaa aaa ccg aaa cca gag cct aag ccg gaa gcg aag cct gtc ccg aaa 384
Glu Lys Pro Lys Pro Glu Pro Lys Pro Glu Ala Lys Pro Val Pro Lys
        115                 120                 125

ccg gcg gaa aaa ccg gtc gag aag ccg tct gaa aaa cct gcc gaa cat 432
Pro Ala Glu Lys Pro Val Glu Lys Pro Ser Glu Lys Pro Ala Glu His
    130                 135                 140

ccc ggc aat gct tct gcc aaa gca gac agc gag cag ggc aat ggg gaa 480
Pro Gly Asn Ala Ser Ala Lys Ala Asp Ser Glu Gln Gly Asn Gly Glu
145                 150                 155                 160

gat aag gga acc ggt atc aaa gga gac gga acg ggg cgc gga gaa ggc 528
Asp Lys Gly Thr Gly Ile Lys Gly Asp Gly Thr Gly Arg Gly Glu Gly
                165                 170                 175

agc ggt aaa ggt agc ggc ggt gtc aaa ggc gaa cac ggg gaa gga gcc 576
Ser Gly Lys Gly Ser Gly Gly Val Lys Gly Glu His Gly Glu Gly Ala
            180                 185                 190

ggc agc agc aaa ggc aat cct tta cgc gcc aac ggc agc att ccg cgc 624
Gly Ser Ser Lys Gly Asn Pro Leu Arg Ala Asn Gly Ser Ile Pro Arg
        195                 200                 205

ccg gct tat ccc acg ctt tct atg gag aat gat gag cag ggt acg gtt 672
Pro Ala Tyr Pro Thr Leu Ser Met Glu Asn Asp Glu Gln Gly Thr Val
    210                 215                 220

gtt ttg agc gtt ttg gtt tct ccg ggc ggt cat gtt gag tcc gtt aaa 720
Val Leu Ser Val Leu Val Ser Pro Gly Gly His Val Glu Ser Val Lys
225                 230                 235                 240

atc gtg aaa agc agt ggt ttt tcc cgg ttg gac aat gcg gca cgc aag 768
Ile Val Lys Ser Ser Gly Phe Ser Arg Leu Asp Asn Ala Ala Arg Lys
                245                 250                 255

gcg gcg caa aac ggg cat ttt caa gcc aat gcc tgg acg gag ttt aaa 816
Ala Ala Gln Asn Gly His Phe Gln Ala Asn Ala Trp Thr Glu Phe Lys
            260                 265                 270

gtc ccc gtc aag ttt gaa ttg aat 840
Val Pro Val Lys Phe Glu Leu Asn
        275                 280

<210> 88

<211> 280
<212> PRT
<213> Neisseria meningitidis

<400> 88
Met Asp Lys Glu Arg Ile Leu Thr Pro Ala Val Val Phe Ser Val Ala
1               5                   10                  15

Leu Leu His Leu Ala Met Val Ala Leu Leu Trp Gln Ala His Lys Leu
            20                  25                  30

Pro Val Ile Glu Ser Gly Asn Val Ile Glu Phe Val Asp Leu Gly Asp
        35                  40                  45

Phe Gly Gly Gly Asp Gly Ala Pro Glu Gly Ala Gly Ala Pro Ala Ala
    50                  55                  60

Pro Glu Pro Gln Pro Val Pro Glu Pro Pro Lys Pro Val Glu Pro Pro
65                  70                  75                  80

Lys Pro Val Leu Lys Pro Val Val Thr Lys Lys Ala Asp Ala Asp Ile
                85                  90                  95

Gln Gln Pro Lys Glu Glu Pro Lys Pro Glu Glu Lys Pro Lys Pro Glu
            100                 105                 110

Glu Lys Pro Lys Pro Glu Pro Lys Pro Glu Ala Lys Pro Val Pro Lys
        115                 120                 125

Pro Ala Glu Lys Pro Val Glu Lys Pro Ser Glu Lys Pro Ala Glu His
    130                 135                 140

Pro Gly Asn Ala Ser Ala Lys Ala Asp Ser Glu Gln Gly Asn Gly Glu
145                 150                 155                 160

Asp Lys Gly Thr Gly Ile Lys Gly Asp Gly Thr Gly Arg Gly Glu Gly
                165                 170                 175

Ser Gly Lys Gly Ser Gly Gly Val Lys Gly Glu His Gly Glu Gly Ala
            180                 185                 190

Gly Ser Ser Lys Gly Asn Pro Leu Arg Ala Asn Gly Ser Ile Pro Arg
        195                 200                 205

Pro Ala Tyr Pro Thr Leu Ser Met Glu Asn Asp Glu Gln Gly Thr Val
    210                 215                 220

Val Leu Ser Val Leu Val Ser Pro Gly Gly His Val Glu Ser Val Lys

225                 230                 235                 240

Ile Val Lys Ser Ser Gly Phe Ser Arg Leu Asp Asn Ala Ala Arg Lys
                245                 250                 255

Ala Ala Gln Asn Gly His Phe Gln Ala Asn Ala Trp Thr Glu Phe Lys
            260                 265                 270

Val Pro Val Lys Phe Glu Leu Asn
        275                 280

<210> 89
<211> 1584
<212> DNA
<213> Neisseria meningitidis

<220>
<221> CDS
<222> (1)..(1584)

<400> 89
atg gca ctt ttc ctc agc ata ttc ccc atc gtc ctg ctg att tgg ctg     48
Met Ala Leu Phe Leu Ser Ile Phe Pro Ile Val Leu Leu Ile Trp Leu
1               5                   10                  15

atg gtg aaa aaa aac agt atg ccc tcc tac gtc gcg ctg ccg att acc     96
Met Val Lys Lys Asn Ser Met Pro Ser Tyr Val Ala Leu Pro Ile Thr
            20                  25                  30

gca gtg ctg att tac gcc atc aaa ctt ttc tac ttc gac gat gcg ggc     144
Ala Val Leu Ile Tyr Ala Ile Lys Leu Phe Tyr Phe Asp Asp Ala Gly
        35                  40                  45

atg ctg ctc aac gcc acc gcc gct tcc ggc ctc gtc aaa acg ctc acg     192
Met Leu Leu Asn Ala Thr Ala Ala Ser Gly Leu Val Lys Thr Leu Thr
    50                  55                  60

ccg att acc gtg att ttc ggc gcg att atg ttc aac cgt atg atg gaa     240
Pro Ile Thr Val Ile Phe Gly Ala Ile Met Phe Asn Arg Met Met Glu
65                  70                  75                  80

acc acg ggc tgc atc gat gtc atc cgc aaa tgg ctg gcg acc atc agc     288
Thr Thr Gly Cys Ile Asp Val Ile Arg Lys Trp Leu Ala Thr Ile Ser
                85                  90                  95

ccc aac ccc gta gcg caa ctg atg att atc ggc tgg gct ttt gcc ttt     336

Pro Asn Pro Val Ala Gln Leu Met Ile Ile Gly Trp Ala Phe Ala Phe
            100                 105                 110

atg att gaa ggc gca tcc ggc ttc ggc acg cct gcc gcg att gcc gcg 384
Met Ile Glu Gly Ala Ser Gly Phe Gly Thr Pro Ala Ala Ile Ala Ala
        115                 120                 125

ccg att ctg atg agc ttg ggc ttc aac ccg ttg aaa gtg gcg att ttc 432
Pro Ile Leu Met Ser Leu Gly Phe Asn Pro Leu Lys Val Ala Ile Phe
    130                 135                 140

act ttg gtg atg aac tcc gtc ccc gtc tct ttc ggc gcg gtc ggt acg 480
Thr Leu Val Met Asn Ser Val Pro Val Ser Phe Gly Ala Val Gly Thr
145                 150                 155                 160

ccg acc tgg ttc ggt ttc gca ccg ctg aac ctg agt gcc gaa gac atc 528
Pro Thr Trp Phe Gly Phe Ala Pro Leu Asn Leu Ser Ala Glu Asp Ile
                165                 170                 175

ctc gcc atc ggc agg cag acc ggc gta atg cac ttc ttc gca ggt ttc 576
Leu Ala Ile Gly Arg Gln Thr Gly Val Met His Phe Phe Ala Gly Phe
            180                 185                 190

gtc atc ccc gtc atc ggc ttg ggc ttc atc gta cct tgg tct gaa atc 624
Val Ile Pro Val Ile Gly Leu Gly Phe Ile Val Pro Trp Ser Glu Ile
        195                 200                 205

cgc aaa aac ttg ggc ttc gtc gcc att gcc gtc ttc tcc tgc acc att 672
Arg Lys Asn Leu Gly Phe Val Ala Ile Ala Val Phe Ser Cys Thr Ile
    210                 215                 220

cct tat gtc gca ttg gcg atg gtc aac gaa gaa ttc ccg tcg ctc gtc 720
Pro Tyr Val Ala Leu Ala Met Val Asn Glu Glu Phe Pro Ser Leu Val
225                 230                 235                 240

gcc ggc gcg atc ggc ctg atg gtg tcc gta ttc gcc gcc aac caa ggc 768
Ala Gly Ala Ile Gly Leu Met Val Ser Val Phe Ala Ala Asn Gln Gly
                245                 250                 255

tgg ggc ttg agc aaa gac cac gcc aaa gac ccg aat gcc gaa aaa gtg 816
Trp Gly Leu Ser Lys Asp His Ala Lys Asp Pro Asn Ala Glu Lys Val
            260                 265                 270

ccg ttc gcc caa gtc gcc aaa gca ctc gcc cct ttg ggt atg ctg atc 864
Pro Phe Ala Gln Val Ala Lys Ala Leu Ala Pro Leu Gly Met Leu Ile
        275                 280                 285

ggc atg ctg gtg gtt acg cgc atc aaa cag ctc ggc atc aaa ggc att 912

Gly Met Leu Val Val Thr Arg Ile Lys Gln Leu Gly Ile Lys Gly Ile
    290                 295                 300

ttg acc agc aaa gaa gaa tgg ttc agc ttc caa ctg ccg ttt gat ttg     960
Leu Thr Ser Lys Glu Glu Trp Phe Ser Phe Gln Leu Pro Phe Asp Leu
305                 310                 315                 320

tcc aaa atc acc gtc agc gac tcc ctg acg att act ttc ggc aat att     1008
Ser Lys Ile Thr Val Ser Asp Ser Leu Thr Ile Thr Phe Gly Asn Ile
                325                 330                 335

ttc gga caa gat gtc agc gcg tct tac caa acg ctg tac gtc ccg gct     1056
Phe Gly Gln Asp Val Ser Ala Ser Tyr Gln Thr Leu Tyr Val Pro Ala
            340                 345                 350

tgg att ccg ttt gtg ctg acc gtt tgg att tgc atc ctg ctg tat aaa     1104
Trp Ile Pro Phe Val Leu Thr Val Trp Ile Cys Ile Leu Leu Tyr Lys
        355                 360                 365

acc aaa ttc aaa gat gcc tgg acg att tat tcc gta act ttc aat caa     1152
Thr Lys Phe Lys Asp Ala Trp Thr Ile Tyr Ser Val Thr Phe Asn Gln
    370                 375                 380

acc aaa aaa ccg ctg ctt gcc ctg atg ggc gcg ctg att atg gtt cag     1200
Thr Lys Lys Pro Leu Leu Ala Leu Met Gly Ala Leu Ile Met Val Gln
385                 390                 395                 400

ctg atg ctg gtc ggc ggc gac aat tcg atg gtg aaa atc atc ggt aag     1248
Leu Met Leu Val Gly Gly Asp Asn Ser Met Val Lys Ile Ile Gly Lys
                405                 410                 415

gaa ttt gcc gca atg gcg ggc gaa cac tgg gtt tat ttc tcg ccg tat     1296
Glu Phe Ala Ala Met Ala Gly Glu His Trp Val Tyr Phe Ser Pro Tyr
            420                 425                 430

ctg ggc gcg atc ggt gcg ttc ttc tcc ggt tcc aac acc gtg tcc aac     1344
Leu Gly Ala Ile Gly Ala Phe Phe Ser Gly Ser Asn Thr Val Ser Asn
        435                 440                 445

ctg acc ttc ggc ccg att cag cag caa atc gcc ttg gat acc ggc ctg     1392
Leu Thr Phe Gly Pro Ile Gln Gln Gln Ile Ala Leu Asp Thr Gly Leu
    450                 455                 460

tcc gtt acc ctg att ctg gcg ttg cag tcc gtc ggc ggc gcg atg ggc     1440
Ser Val Thr Leu Ile Leu Ala Leu Gln Ser Val Gly Gly Ala Met Gly
465                 470                 475                 480

aat atg gtg tgc ctc aac aac atc atc gcc gta tgt acc gta ttg gat     1488

Asn Met Val Cys Leu Asn Asn Ile Ile Ala Val Cys Thr Val Leu Asp
                485                 490                 495

gtg aaa aat tcc gaa ggt gcg att atc aag aaa acc gtt atc ccg atg     1536
Val Lys Asn Ser Glu Gly Ala Ile Ile Lys Lys Thr Val Ile Pro Met
            500                 505                 510

gcg att tac ggc gtg att gcc gtc gtc gcg gca atg att ttc ttc ctc     1584
Ala Ile Tyr Gly Val Ile Ala Val Val Ala Ala Met Ile Phe Phe Leu
        515                 520                 525

<210> 90
<211> 528
<212> PRT
<213> Neisseria meningitidis

<400> 90
Met Ala Leu Phe Leu Ser Ile Phe Pro Ile Val Leu Leu Ile Trp Leu
1               5                   10                  15

Met Val Lys Lys Asn Ser Met Pro Ser Tyr Val Ala Leu Pro Ile Thr
            20                  25                  30

Ala Val Leu Ile Tyr Ala Ile Lys Leu Phe Tyr Phe Asp Asp Ala Gly
        35                  40                  45

Met Leu Leu Asn Ala Thr Ala Ala Ser Gly Leu Val Lys Thr Leu Thr
    50                  55                  60

Pro Ile Thr Val Ile Phe Gly Ala Ile Met Phe Asn Arg Met Met Glu
65                  70                  75                  80

Thr Thr Gly Cys Ile Asp Val Ile Arg Lys Trp Leu Ala Thr Ile Ser
                85                  90                  95

Pro Asn Pro Val Ala Gln Leu Met Ile Ile Gly Trp Ala Phe Ala Phe
            100                 105                 110

Met Ile Glu Gly Ala Ser Gly Phe Gly Thr Pro Ala Ala Ile Ala Ala
        115                 120                 125

Pro Ile Leu Met Ser Leu Gly Phe Asn Pro Leu Lys Val Ala Ile Phe
    130                 135                 140

Thr Leu Val Met Asn Ser Val Pro Val Ser Phe Gly Ala Val Gly Thr
145                 150                 155                 160

Pro Thr Trp Phe Gly Phe Ala Pro Leu Asn Leu Ser Ala Glu Asp Ile
                165                 170                 175

Leu Ala Ile Gly Arg Gln Thr Gly Val Met His Phe Phe Ala Gly Phe
            180                 185                 190

Val Ile Pro Val Ile Gly Leu Gly Phe Ile Val Pro Trp Ser Glu Ile
        195                 200                 205

Arg Lys Asn Leu Gly Phe Val Ala Ile Ala Val Phe Ser Cys Thr Ile
    210                 215                 220

Pro Tyr Val Ala Leu Ala Met Val Asn Glu Glu Phe Pro Ser Leu Val
225                 230                 235                 240

Ala Gly Ala Ile Gly Leu Met Val Ser Val Phe Ala Ala Asn Gln Gly
                245                 250                 255

Trp Gly Leu Ser Lys Asp His Ala Lys Asp Pro Asn Ala Glu Lys Val
            260                 265                 270

Pro Phe Ala Gln Val Ala Lys Ala Leu Ala Pro Leu Gly Met Leu Ile
        275                 280                 285

Gly Met Leu Val Val Thr Arg Ile Lys Gln Leu Gly Ile Lys Gly Ile
    290                 295                 300

Leu Thr Ser Lys Glu Glu Trp Phe Ser Phe Gln Leu Pro Phe Asp Leu
305                 310                 315                 320

Ser Lys Ile Thr Val Ser Asp Ser Leu Thr Ile Thr Phe Gly Asn Ile
                325                 330                 335

Phe Gly Gln Asp Val Ser Ala Ser Tyr Gln Thr Leu Tyr Val Pro Ala
            340                 345                 350

Trp Ile Pro Phe Val Leu Thr Val Trp Ile Cys Ile Leu Leu Tyr Lys
        355                 360                 365

Thr Lys Phe Lys Asp Ala Trp Thr Ile Tyr Ser Val Thr Phe Asn Gln
    370                 375                 380

Thr Lys Lys Pro Leu Leu Ala Leu Met Gly Ala Leu Ile Met Val Gln
385                 390                 395                 400

Leu Met Leu Val Gly Gly Asp Asn Ser Met Val Lys Ile Ile Gly Lys
                405                 410                 415

Glu Phe Ala Ala Met Ala Gly Glu His Trp Val Tyr Phe Ser Pro Tyr
            420                 425                 430

Leu Gly Ala Ile Gly Ala Phe Phe Ser Gly Ser Asn Thr Val Ser Asn
        435                 440                 445

Leu Thr Phe Gly Pro Ile Gln Gln Gln Ile Ala Leu Asp Thr Gly Leu
    450                 455                 460

Ser Val Thr Leu Ile Leu Ala Leu Gln Ser Val Gly Gly Ala Met Gly
465                 470                 475                 480

Asn Met Val Cys Leu Asn Asn Ile Ile Ala Val Cys Thr Val Leu Asp
                485                 490                 495

Val Lys Asn Ser Glu Gly Ala Ile Ile Lys Lys Thr Val Ile Pro Met
            500                 505                 510

Ala Ile Tyr Gly Val Ile Ala Val Val Ala Ala Met Ile Phe Phe Leu
        515                 520                 525

<210> 91
<211> 1686
<212> DNA
<213> Neisseria meningitidis

<220>
<221> CDS
<222> (1)..(1686)

<400> 91
atg tta caa tcc gaa aat tcg aaa aat tta atc tct tgt tca ata aag 48
Met Leu Gln Ser Glu Asn Ser Lys Asn Leu Ile Ser Cys Ser Ile Lys
1               5                   10                  15

gct tta cca atc atg att tct acc aac ggc atc acc atg cag ttc ggc 96
Ala Leu Pro Ile Met Ile Ser Thr Asn Gly Ile Thr Met Gln Phe Gly
            20                  25                  30

gcg aag ccg ctg ttt gaa aac gta tcc gtt aaa ttc ggc gaa ggc aac 144
Ala Lys Pro Leu Phe Glu Asn Val Ser Val Lys Phe Gly Glu Gly Asn
        35                  40                  45

cgc tac ggt ttg atc ggc gcg aac ggc tca ggc aaa tcc acc ttc atg 192
Arg Tyr Gly Leu Ile Gly Ala Asn Gly Ser Gly Lys Ser Thr Phe Met
    50                  55                  60

aaa atc ctc ggc ggc gat ttg gaa cag aca gcc ggc gaa gtg gcg att 240
Lys Ile Leu Gly Gly Asp Leu Glu Gln Thr Ala Gly Glu Val Ala Ile
65                  70                  75                  80

gaa aac ggc gtg cgt ttg ggt aaa ttg cgc caa gac cag ttt gcc tac 288
Glu Asn Gly Val Arg Leu Gly Lys Leu Arg Gln Asp Gln Phe Ala Tyr
                85                  90                  95

gaa gat atg cgc gtg ctg gac gtg gta atg atg ggt cac acc gaa atg 336
Glu Asp Met Arg Val Leu Asp Val Val Met Met Gly His Thr Glu Met
            100                 105                 110

tgg gcg gcg atg acc gaa cgt gat gcg att tac gcc aat ccc gaa gcc 384
Trp Ala Ala Met Thr Glu Arg Asp Ala Ile Tyr Ala Asn Pro Glu Ala
        115                 120                 125

acc gaa gac gac tac atg aaa gcc gca gaa ctg gaa gcc aag ttc gcc 432
Thr Glu Asp Asp Tyr Met Lys Ala Ala Glu Leu Glu Ala Lys Phe Ala
    130                 135                 140

gaa tac gac ggc tac acc gcc gaa gcg cgt gcc gcc gag ttg ttg agc 480
Glu Tyr Asp Gly Tyr Thr Ala Glu Ala Arg Ala Ala Glu Leu Leu Ser
145                 150                 155                 160

ggc gtg ggc att tcc gaa gat ttg cac aat gca acc atg gca gaa gtt 528
Gly Val Gly Ile Ser Glu Asp Leu His Asn Ala Thr Met Ala Glu Val
                165                 170                 175

gcc ccg ggc ttc aaa ctg cgc gta ttg ctg gcg caa gcc ctg ttc tcc 576
Ala Pro Gly Phe Lys Leu Arg Val Leu Leu Ala Gln Ala Leu Phe Ser
            180                 185                 190

aag ccg gat gta ttg ctc ttg gac gaa ccg acc aat aac ttg gac att 624
Lys Pro Asp Val Leu Leu Leu Asp Glu Pro Thr Asn Asn Leu Asp Ile
        195                 200                 205

aat acc atc cgt tgg ttg gaa ggc gtg ttg aac cag tac gac tcc acg 672
Asn Thr Ile Arg Trp Leu Glu Gly Val Leu Asn Gln Tyr Asp Ser Thr
    210                 215                 220

atg att atc atc tcg cac gac cgc cac ttc ttg aac gaa gtc tgc acg 720
Met Ile Ile Ile Ser His Asp Arg His Phe Leu Asn Glu Val Cys Thr
225                 230                 235                 240

cat atg gca gat ttg gat tac aac acc atc acc atc tat ccg ggc aac 768
His Met Ala Asp Leu Asp Tyr Asn Thr Ile Thr Ile Tyr Pro Gly Asn
                245                 250                 255

tac gac gac tat atg ctc gcc tcc gcc caa tcg cgc gaa cgc gcc ctg 816
Tyr Asp Asp Tyr Met Leu Ala Ser Ala Gln Ser Arg Glu Arg Ala Leu
            260                 265                 270

aaa gac aat gcc aag gcg aag gaa aaa ttg cag gaa ttg caa gaa ttt 864
Lys Asp Asn Ala Lys Ala Lys Glu Lys Leu Gln Glu Leu Gln Glu Phe
        275                 280                 285

gtg gcg cgg ttc agt gcc aac aaa tcc aaa gcc cgt cag gca acc agc 912
Val Ala Arg Phe Ser Ala Asn Lys Ser Lys Ala Arg Gln Ala Thr Ser
    290                 295                 300

cgt ctg aaa cag gca gac aaa atc aaa tcg gag atg gtc gag gtc aaa 960
Arg Leu Lys Gln Ala Asp Lys Ile Lys Ser Glu Met Val Glu Val Lys
305                 310                 315                 320

ccc tct acc cgt caa aac ccg tat atc cgt ttt gaa gcc gat gaa aaa 1008
Pro Ser Thr Arg Gln Asn Pro Tyr Ile Arg Phe Glu Ala Asp Glu Lys
                325                 330                 335

gcc aag ctg cac cgt cag gct gtg gaa gtt gaa aaa ctg gcg aaa cgc 1056
Ala Lys Leu His Arg Gln Ala Val Glu Val Glu Lys Leu Ala Lys Arg
            340                 345                 350

ttt gaa acc cag ttg ttt aaa aac ctg aac ttt atc ctt gaa gcg gga 1104
Phe Glu Thr Gln Leu Phe Lys Asn Leu Asn Phe Ile Leu Glu Ala Gly
        355                 360                 365

caa cgc ctc gcc atc atc ggc ccg aac ggc gcg ggc aaa tcc acc ctg 1152
Gln Arg Leu Ala Ile Ile Gly Pro Asn Gly Ala Gly Lys Ser Thr Leu
    370                 375                 380

ctg aaa ctc ttg gcc ggc gcg tac aac ccc gaa tat tca gac ggt ctg 1200
Leu Lys Leu Leu Ala Gly Ala Tyr Asn Pro Glu Tyr Ser Asp Gly Leu
385                 390                 395                 400

ctg ccg gac gaa ggc agc atc aaa tgg gcg gaa aaa gcc agc gtc ggc 1248
Leu Pro Asp Glu Gly Ser Ile Lys Trp Ala Glu Lys Ala Ser Val Gly
                405                 410                 415

tac tat ccg caa gat cac gaa aac gac ttc gat gtc gat atg gat ttg 1296
Tyr Tyr Pro Gln Asp His Glu Asn Asp Phe Asp Val Asp Met Asp Leu
            420                 425                 430

agc gaa tgg atg cgc caa tgg ggg cag gac ggc gac gac gaa caa gtc 1344
Ser Glu Trp Met Arg Gln Trp Gly Gln Asp Gly Asp Asp Glu Gln Val
        435                 440                 445

atc cgc ggc act ttg ggg cgt ttg ctc ttc ggc agt aac gat gtc gtg     1392
Ile Arg Gly Thr Leu Gly Arg Leu Leu Phe Gly Ser Asn Asp Val Val
    450                 455                 460

aaa aaa gtg aag gtt ctc tcc ggt ggt gaa aaa ggc cgt atg ctt tac     1440
Lys Lys Val Lys Val Leu Ser Gly Gly Glu Lys Gly Arg Met Leu Tyr
465                 470                 475                 480

ggc aaa ctg ttg ctg ttg aaa ccc aat gtc tta gtc atg gat gaa ccg     1488
Gly Lys Leu Leu Leu Leu Lys Pro Asn Val Leu Val Met Asp Glu Pro
                485                 490                 495

acc aac cac atg gac atg gaa agc atc gaa tcc ttg aac atg gcg ctg     1536
Thr Asn His Met Asp Met Glu Ser Ile Glu Ser Leu Asn Met Ala Leu
            500                 505                 510

gaa aaa tac aac ggc acg ctg att ttc gtc tcc cac gac cgt cag ttc     1584
Glu Lys Tyr Asn Gly Thr Leu Ile Phe Val Ser His Asp Arg Gln Phe
        515                 520                 525

gtt tct tcc ctg gct acc caa atc att gaa ttg gac ggc aaa ggc gga     1632
Val Ser Ser Leu Ala Thr Gln Ile Ile Glu Leu Asp Gly Lys Gly Gly
    530                 535                 540

tat gaa cac tac ttg ggc gat tac gaa agc tat ctc gag aaa aaa ggc     1680
Tyr Glu His Tyr Leu Gly Asp Tyr Glu Ser Tyr Leu Glu Lys Lys Gly
545                 550                 555                 560

gta gca                                                             1686
Val Ala

<210> 92
<211> 562
<212> PRT
<213> Neisseria meningitidis

<400> 92
Met Leu Gln Ser Glu Asn Ser Lys Asn Leu Ile Ser Cys Ser Ile Lys
1               5                   10                  15

Ala Leu Pro Ile Met Ile Ser Thr Asn Gly Ile Thr Met Gln Phe Gly
            20                  25                  30

Ala Lys Pro Leu Phe Glu Asn Val Ser Val Lys Phe Gly Glu Gly Asn
        35                  40                  45

Arg Tyr Gly Leu Ile Gly Ala Asn Gly Ser Gly Lys Ser Thr Phe Met
    50                  55                  60

Lys Ile Leu Gly Gly Asp Leu Glu Gln Thr Ala Gly Glu Val Ala Ile
65                  70                  75                  80

Glu Asn Gly Val Arg Leu Gly Lys Leu Arg Gln Asp Gln Phe Ala Tyr
                85                  90                  95

Glu Asp Met Arg Val Leu Asp Val Val Met Met Gly His Thr Glu Met
            100                 105                 110

Trp Ala Ala Met Thr Glu Arg Asp Ala Ile Tyr Ala Asn Pro Glu Ala
        115                 120                 125

Thr Glu Asp Asp Tyr Met Lys Ala Ala Glu Leu Glu Ala Lys Phe Ala
    130                 135                 140

Glu Tyr Asp Gly Tyr Thr Ala Glu Ala Arg Ala Ala Glu Leu Leu Ser
145                 150                 155                 160

Gly Val Gly Ile Ser Glu Asp Leu His Asn Ala Thr Met Ala Glu Val
                165                 170                 175

Ala Pro Gly Phe Lys Leu Arg Val Leu Leu Ala Gln Ala Leu Phe Ser
            180                 185                 190

Lys Pro Asp Val Leu Leu Leu Asp Glu Pro Thr Asn Asn Leu Asp Ile
        195                 200                 205

Asn Thr Ile Arg Trp Leu Glu Gly Val Leu Asn Gln Tyr Asp Ser Thr
    210                 215                 220

Met Ile Ile Ile Ser His Asp Arg His Phe Leu Asn Glu Val Cys Thr
225                 230                 235                 240

His Met Ala Asp Leu Asp Tyr Asn Thr Ile Thr Ile Tyr Pro Gly Asn
                245                 250                 255

Tyr Asp Asp Tyr Met Leu Ala Ser Ala Gln Ser Arg Glu Arg Ala Leu
            260                 265                 270

Lys Asp Asn Ala Lys Ala Lys Glu Lys Leu Gln Glu Leu Gln Glu Phe
        275                 280                 285

Val Ala Arg Phe Ser Ala Asn Lys Ser Lys Ala Arg Gln Ala Thr Ser
    290                 295                 300

Arg Leu Lys Gln Ala Asp Lys Ile Lys Ser Glu Met Val Glu Val Lys
305                 310                 315                 320

Pro Ser Thr Arg Gln Asn Pro Tyr Ile Arg Phe Glu Ala Asp Glu Lys
                325                 330                 335

Ala Lys Leu His Arg Gln Ala Val Glu Val Glu Lys Leu Ala Lys Arg
            340                 345                 350

Phe Glu Thr Gln Leu Phe Lys Asn Leu Asn Phe Ile Leu Glu Ala Gly
        355                 360                 365

Gln Arg Leu Ala Ile Ile Gly Pro Asn Gly Ala Gly Lys Ser Thr Leu
    370                 375                 380

Leu Lys Leu Leu Ala Gly Ala Tyr Asn Pro Glu Tyr Ser Asp Gly Leu
385                 390                 395                 400

Leu Pro Asp Glu Gly Ser Ile Lys Trp Ala Glu Lys Ala Ser Val Gly
                405                 410                 415

Tyr Tyr Pro Gln Asp His Glu Asn Asp Phe Asp Val Asp Met Asp Leu
            420                 425                 430

Ser Glu Trp Met Arg Gln Trp Gly Gln Asp Gly Asp Asp Glu Gln Val
        435                 440                 445

Ile Arg Gly Thr Leu Gly Arg Leu Leu Phe Gly Ser Asn Asp Val Val
    450                 455                 460

Lys Lys Val Lys Val Leu Ser Gly Gly Glu Lys Gly Arg Met Leu Tyr
465                 470                 475                 480

Gly Lys Leu Leu Leu Leu Lys Pro Asn Val Leu Val Met Asp Glu Pro
                485                 490                 495

Thr Asn His Met Asp Met Glu Ser Ile Glu Ser Leu Asn Met Ala Leu
            500                 505                 510

Glu Lys Tyr Asn Gly Thr Leu Ile Phe Val Ser His Asp Arg Gln Phe
        515                 520                 525

Val Ser Ser Leu Ala Thr Gln Ile Ile Glu Leu Asp Gly Lys Gly Gly
    530                 535                 540

Tyr Glu His Tyr Leu Gly Asp Tyr Glu Ser Tyr Leu Glu Lys Lys Gly
545                 550                 555                 560

Val Ala

<210> 93
<211> 1126
<212> DNA
<213> Neisseria meningitidis

<220>
<221> CDS
<222> (1)..(1125)

<400> 93
atg aac acc tcg caa cgc aac cgc ctc gtc agc cgc tgg ctc aac tcc 48
Met Asn Thr Ser Gln Arg Asn Arg Leu Val Ser Arg Trp Leu Asn Ser
1               5                   10                  15

tac gaa cgc tac cgc tac cgc cgc ctc atc cac gcc gtc cgg ctc ggc 96
Tyr Glu Arg Tyr Arg Tyr Arg Arg Leu Ile His Ala Val Arg Leu Gly
            20                  25                  30

ggg gcc gtc ctg ttc gcc acc gcc tcc gcc cgg ctg ctc cac ctc caa 144
Gly Ala Val Leu Phe Ala Thr Ala Ser Ala Arg Leu Leu His Leu Gln
        35                  40                  45

cac ggc gag tgg ata ggg atg acc gtc ttc gtc gtc ctc ggc atg ctc 192
His Gly Glu Trp Ile Gly Met Thr Val Phe Val Val Leu Gly Met Leu
    50                  55                  60

cag ttt caa ggg gcg att tac tcc aag gcg gtg gaa cgt atg ctc ggc 240
Gln Phe Gln Gly Ala Ile Tyr Ser Lys Ala Val Glu Arg Met Leu Gly
65                  70                  75                  80

acg gtc atc ggg ctg ggc gcg ggt ttg ggc gtt tta tgg ctg aac cag 288
Thr Val Ile Gly Leu Gly Ala Gly Leu Gly Val Leu Trp Leu Asn Gln
                85                  90                  95

cat tat ttc cac ggc aac ctc ctc ttc tac ctc acc gtc ggc acg gca 336
His Tyr Phe His Gly Asn Leu Leu Phe Tyr Leu Thr Val Gly Thr Ala
            100                 105                 110

agc gca ctg gcc ggc tgg gcg gcg gtc ggc aaa aac ggc tac gtc cct 384
Ser Ala Leu Ala Gly Trp Ala Ala Val Gly Lys Asn Gly Tyr Val Pro
        115                 120                 125

atg ctg gcg ggg ctg acg atg tgc atg ctc atc ggc gac aac ggc agc 432

Met Leu Ala Gly Leu Thr Met Cys Met Leu Ile Gly Asp Asn Gly Ser
    130                 135                 140

gaa tgg ttc gac agc ggc ctg atg cgc gcg atg aac gtc ctc atc ggc    480
Glu Trp Phe Asp Ser Gly Leu Met Arg Ala Met Asn Val Leu Ile Gly
145                 150                 155                 160

gcg gcc atc gcc atc gcc gcc gcc aaa ctg ctg ccg ctg aaa tcc aca    528
Ala Ala Ile Ala Ile Ala Ala Ala Lys Leu Leu Pro Leu Lys Ser Thr
                165                 170                 175

ctg atg tgg cgt ttc atg ctt gcc gac aac ctg acc gac tgc agc aaa    576
Leu Met Trp Arg Phe Met Leu Ala Asp Asn Leu Thr Asp Cys Ser Lys
            180                 185                 190

atg att gcc gaa atc agc aac ggc agg cgc atg acc cgc gaa cgc ctc    624
Met Ile Ala Glu Ile Ser Asn Gly Arg Arg Met Thr Arg Glu Arg Leu
        195                 200                 205

gaa gag aac atg gcg aaa atg cgc caa atc aac gca cgc atg gtc aaa    672
Glu Glu Asn Met Ala Lys Met Arg Gln Ile Asn Ala Arg Met Val Lys
    210                 215                 220

agc cgc agc cac ctc gcc gcc aca tcg ggc gaa agc cgc atc agc ccc    720
Ser Arg Ser His Leu Ala Ala Thr Ser Gly Glu Ser Arg Ile Ser Pro
225                 230                 235                 240

gcc atg atg gaa gcc atg cag cac gcc cac cgt aaa att gtc aac acc    768
Ala Met Met Glu Ala Met Gln His Ala His Arg Lys Ile Val Asn Thr
                245                 250                 255

acc gag ctg ctc ctg acc acc gcc gcc aag ctg caa tct ccc aaa ctc    816
Thr Glu Leu Leu Leu Thr Thr Ala Ala Lys Leu Gln Ser Pro Lys Leu
            260                 265                 270

aac ggc agc gaa atc cgg ctg ctt gac cgc cac ttc aca ctg ctc caa    864
Asn Gly Ser Glu Ile Arg Leu Leu Asp Arg His Phe Thr Leu Leu Gln
        275                 280                 285

acc gac ctg caa caa acc gtc gcc ctt atc aac ggc aga cac gcc cgc    912
Thr Asp Leu Gln Gln Thr Val Ala Leu Ile Asn Gly Arg His Ala Arg
    290                 295                 300

cgc atc cgc atc gac acc gcc atc aac ccc gaa ctg gaa gcc ctc gcc    960
Arg Ile Arg Ile Asp Thr Ala Ile Asn Pro Glu Leu Glu Ala Leu Ala
305                 310                 315                 320

gaa cac ctc cac tac caa tgg cag ggc ttc ctc tgg ctc agc acc aat    1008

Glu His Leu His Tyr Gln Trp Gln Gly Phe Leu Trp Leu Ser Thr Asn
                325                 330                 335

atg cgt cag gaa att tcc gcc ctc gtc atc ctg ctg caa cgc acc cgc      1056
Met Arg Gln Glu Ile Ser Ala Leu Val Ile Leu Leu Gln Arg Thr Arg
            340                 345                 350

cgc aaa tgg ctg gat gcc cac gaa cgc caa cac ctg cgc caa agc ctg      1104
Arg Lys Trp Leu Asp Ala His Glu Arg Gln His Leu Arg Gln Ser Leu
        355                 360                 365

ctt gaa aca cgg gaa cac agt t                                        1126
Leu Glu Thr Arg Glu His Ser
    370                 375

<210> 94
<211> 375
<212> PRT
<213> Neisseria meningitidis

<400> 94
Met Asn Thr Ser Gln Arg Asn Arg Leu Val Ser Arg Trp Leu Asn Ser
  1               5                  10                  15

Tyr Glu Arg Tyr Arg Tyr Arg Arg Leu Ile His Ala Val Arg Leu Gly
             20                  25                  30

Gly Ala Val Leu Phe Ala Thr Ala Ser Ala Arg Leu Leu His Leu Gln
         35                  40                  45

His Gly Glu Trp Ile Gly Met Thr Val Phe Val Val Leu Gly Met Leu
     50                  55                  60

Gln Phe Gln Gly Ala Ile Tyr Ser Lys Ala Val Glu Arg Met Leu Gly
 65                  70                  75                  80

Thr Val Ile Gly Leu Gly Ala Gly Leu Gly Val Leu Trp Leu Asn Gln
                 85                  90                  95

His Tyr Phe His Gly Asn Leu Leu Phe Tyr Leu Thr Val Gly Thr Ala
            100                 105                 110

Ser Ala Leu Ala Gly Trp Ala Ala Val Gly Lys Asn Gly Tyr Val Pro
        115                 120                 125

Met Leu Ala Gly Leu Thr Met Cys Met Leu Ile Gly Asp Asn Gly Ser
    130                 135                 140

Glu Trp Phe Asp Ser Gly Leu Met Arg Ala Met Asn Val Leu Ile Gly
145                 150                 155                 160

Ala Ala Ile Ala Ile Ala Ala Ala Lys Leu Leu Pro Leu Lys Ser Thr
                165                 170                 175

Leu Met Trp Arg Phe Met Leu Ala Asp Asn Leu Thr Asp Cys Ser Lys
            180                 185                 190

Met Ile Ala Glu Ile Ser Asn Gly Arg Arg Met Thr Arg Glu Arg Leu
        195                 200                 205

Glu Glu Asn Met Ala Lys Met Arg Gln Ile Asn Ala Arg Met Val Lys
    210                 215                 220

Ser Arg Ser His Leu Ala Ala Thr Ser Gly Glu Ser Arg Ile Ser Pro
225                 230                 235                 240

Ala Met Met Glu Ala Met Gln His Ala His Arg Lys Ile Val Asn Thr
                245                 250                 255

Thr Glu Leu Leu Leu Thr Thr Ala Ala Lys Leu Gln Ser Pro Lys Leu
            260                 265                 270

Asn Gly Ser Glu Ile Arg Leu Leu Asp Arg His Phe Thr Leu Leu Gln
        275                 280                 285

Thr Asp Leu Gln Gln Thr Val Ala Leu Ile Asn Gly Arg His Ala Arg
    290                 295                 300

Arg Ile Arg Ile Asp Thr Ala Ile Asn Pro Glu Leu Glu Ala Leu Ala
305                 310                 315                 320

Glu His Leu His Tyr Gln Trp Gln Gly Phe Leu Trp Leu Ser Thr Asn
                325                 330                 335

Met Arg Gln Glu Ile Ser Ala Leu Val Ile Leu Leu Gln Arg Thr Arg
            340                 345                 350

Arg Lys Trp Leu Asp Ala His Glu Arg Gln His Leu Arg Gln Ser Leu
        355                 360                 365

Leu Glu Thr Arg Glu His Ser
    370                 375

<210> 95
<211> 1725
<212> DNA
<213> Neisseria meningitidis

<220>
<221> CDS
<222> (1)..(1725)

<400> 95
atg cag cta tca ggc gcg caa atc ata gtg cag agt ctc aaa gcc gaa     48
Met Gln Leu Ser Gly Ala Gln Ile Ile Val Gln Ser Leu Lys Ala Glu
1               5                   10                  15

ggt gtc gag tac gtt ttc ggt tat ccc ggc ggt gcg gtt atc gaa atc     96
Gly Val Glu Tyr Val Phe Gly Tyr Pro Gly Gly Ala Val Ile Glu Ile
            20                  25                  30

tac gat gcc ctt ttc caa ctc aat aaa ttc aag cac att ctg acc cgt     144
Tyr Asp Ala Leu Phe Gln Leu Asn Lys Phe Lys His Ile Leu Thr Arg
        35                  40                  45

cac gag cag gcg gca gta cac gcg gca gat gcg tat gcg cgc gtc agc     192
His Glu Gln Ala Ala Val His Ala Ala Asp Ala Tyr Ala Arg Val Ser
    50                  55                  60

ggt aag gtg ggc gtg gca ttg gtt aca tcc ggc ccg ggc gtt acc aat     240
Gly Lys Val Gly Val Ala Leu Val Thr Ser Gly Pro Gly Val Thr Asn
65                  70                  75                  80

gca ctg acc ggt att gct act gcc tat acg gat tcg att ccg atg gtg     288
Ala Leu Thr Gly Ile Ala Thr Ala Tyr Thr Asp Ser Ile Pro Met Val
                85                  90                  95

gtc atc agc ggg cag gta ggc aat tcc ctg att ggt acg gat gcg ttc     336
Val Ile Ser Gly Gln Val Gly Asn Ser Leu Ile Gly Thr Asp Ala Phe
            100                 105                 110

caa gaa gtt gat acg gtg ggt att acc cgt ccg tgc gtc aaa cac aat     384
Gln Glu Val Asp Thr Val Gly Ile Thr Arg Pro Cys Val Lys His Asn
        115                 120                 125

ttc ctg gtt acg gac atc aat gag ttg gcg gaa acc att aaa aag gcg     432
Phe Leu Val Thr Asp Ile Asn Glu Leu Ala Glu Thr Ile Lys Lys Ala
    130                 135                 140

ttc caa att gcc gca agc ggc cga ccg ggg ccc gtg gtg gtt gat gtc     480
Phe Gln Ile Ala Ala Ser Gly Arg Pro Gly Pro Val Val Val Asp Val

145                 150                 155                 160

ccg aaa gat gtt acg cag gcg atg gcg aaa ttc agc tat cct cag gaa     528
Pro Lys Asp Val Thr Gln Ala Met Ala Lys Phe Ser Tyr Pro Gln Glu
                165                 170                 175

gac att ttt atc cgt tcg tat caa ccg gtt gtt caa ggg cat atc ggt     576
Asp Ile Phe Ile Arg Ser Tyr Gln Pro Val Val Gln Gly His Ile Gly
            180                 185                 190

cag att aaa aag gcc gtg cag atg ttg gca tct gcc aaa cgt ccg gtc     624
Gln Ile Lys Lys Ala Val Gln Met Leu Ala Ser Ala Lys Arg Pro Val
        195                 200                 205

gtc tat ttt ggc ggc ggc gtg gtg ttg ggt aat gct tct gaa gag ctg     672
Val Tyr Phe Gly Gly Gly Val Val Leu Gly Asn Ala Ser Glu Glu Leu
    210                 215                 220

acc cga ttt gtc cga atg acg ggt gct ccg tgc acg ggt acg ctg atg     720
Thr Arg Phe Val Arg Met Thr Gly Ala Pro Cys Thr Gly Thr Leu Met
225                 230                 235                 240

ggc ttg ggc gct tat cct tcc ggc gac cgc caa ttc ctc ggt atg ctc     768
Gly Leu Gly Ala Tyr Pro Ser Gly Asp Arg Gln Phe Leu Gly Met Leu
                245                 250                 255

ggt atg cac ggt act tac gag gca aac ctt gcc atg cag aat gcg gat     816
Gly Met His Gly Thr Tyr Glu Ala Asn Leu Ala Met Gln Asn Ala Asp
            260                 265                 270

gtc gtt ctt gcc gta ggt gcg cgt ttt gac gac cgt gtg gtt tcc gta     864
Val Val Leu Ala Val Gly Ala Arg Phe Asp Asp Arg Val Val Ser Val
        275                 280                 285

ccg tcc aaa ttt ttc gag aag gcg aag aag gtt atc cat atc gat gtc     912
Pro Ser Lys Phe Phe Glu Lys Ala Lys Lys Val Ile His Ile Asp Val
    290                 295                 300

gat cct tcc agc atc gcc aaa cgc gtg aag gcg gac att ccg att gtc     960
Asp Pro Ser Ser Ile Ala Lys Arg Val Lys Ala Asp Ile Pro Ile Val
305                 310                 315                 320

ggc gac gtg aaa aac gtt ttg tcc gag atg gtt gcg ctg tgg caa aaa     1008
Gly Asp Val Lys Asn Val Leu Ser Glu Met Val Ala Leu Trp Gln Lys
                325                 330                 335

caa gag tcc gtg ccg tct gaa gat gct ttg ggc aaa tgg tgg aaa acc     1056
Gln Glu Ser Val Pro Ser Glu Asp Ala Leu Gly Lys Trp Trp Lys Thr

            340                 345                 350

ata gag gaa tgg cgt tcc cgc gat tgc ttg tgg ttt gac aac ggc agc 1104
Ile Glu Glu Trp Arg Ser Arg Asp Cys Leu Trp Phe Asp Asn Gly Ser
        355                 360                 365

gaa att atc aag cca caa tat gtg att cag aag ctt gcc gag att acc 1152
Glu Ile Ile Lys Pro Gln Tyr Val Ile Gln Lys Leu Ala Glu Ile Thr
    370                 375                 380

ggc aat tcg gca atc atc aca tcg gat gta ggg cag cat caa atg ttt 1200
Gly Asn Ser Ala Ile Ile Thr Ser Asp Val Gly Gln His Gln Met Phe
385                 390                 395                 400

gcg gct caa tat tat ccc ttc gaa cgt ccg cgc caa tgg ctc aac tcc 1248
Ala Ala Gln Tyr Tyr Pro Phe Glu Arg Pro Arg Gln Trp Leu Asn Ser
                405                 410                 415

ggc ggt ttg ggt acg atg ggc gtt ggt ctg cct tat gcg att ggt gca 1296
Gly Gly Leu Gly Thr Met Gly Val Gly Leu Pro Tyr Ala Ile Gly Ala
            420                 425                 430

aaa ctt gcc gcc ccg gat caa gac gta ttc tgt att acc ggc gac ggc 1344
Lys Leu Ala Ala Pro Asp Gln Asp Val Phe Cys Ile Thr Gly Asp Gly
        435                 440                 445

tcg atc cag atg aac atc caa gag ctg tcc acc tgt ttc caa tat cgg 1392
Ser Ile Gln Met Asn Ile Gln Glu Leu Ser Thr Cys Phe Gln Tyr Arg
    450                 455                 460

att ccg gtt aac gtc att acg ctg aac aac ggt tat ctc ggt atg gta 1440
Ile Pro Val Asn Val Ile Thr Leu Asn Asn Gly Tyr Leu Gly Met Val
465                 470                 475                 480

cgc cag tgg cag gaa ata tat tac ggc gga cga gag tcg gaa acc tat 1488
Arg Gln Trp Gln Glu Ile Tyr Tyr Gly Gly Arg Glu Ser Glu Thr Tyr
                485                 490                 495

ttc gat tct ttg ccc gat ttc gtc aaa ctt gcc gag gca tac ggc cat 1536
Phe Asp Ser Leu Pro Asp Phe Val Lys Leu Ala Glu Ala Tyr Gly His
            500                 505                 510

atc ggt atc cgc gtg gac aag aag tct gat gtg gaa ggt gcg ttg ttg 1584
Ile Gly Ile Arg Val Asp Lys Lys Ser Asp Val Glu Gly Ala Leu Leu
        515                 520                 525

gaa gca ttg aac caa aaa gac agg ctg gtg ttt atc gac ttc ctg acc 1632
Glu Ala Leu Asn Gln Lys Asp Arg Leu Val Phe Ile Asp Phe Leu Thr

530                 535                 540

gac cag aaa cag aat gtg atg ccc atg gtc ggc aac ggc aaa ggt ttg    1680
Asp Gln Lys Gln Asn Val Met Pro Met Val Gly Asn Gly Lys Gly Leu
545                 550                 555                 560

gac gaa atg gta ctt ccg ccg cat atg cgt gcg gac gga aag gcg        1725
Asp Glu Met Val Leu Pro Pro His Met Arg Ala Asp Gly Lys Ala
                565                 570                 575

<210> 96
<211> 575
<212> PRT
<213> Neisseria meningitidis

<400> 96
Met Gln Leu Ser Gly Ala Gln Ile Ile Val Gln Ser Leu Lys Ala Glu
1               5                   10                  15

Gly Val Glu Tyr Val Phe Gly Tyr Pro Gly Gly Ala Val Ile Glu Ile
            20                  25                  30

Tyr Asp Ala Leu Phe Gln Leu Asn Lys Phe Lys His Ile Leu Thr Arg
        35                  40                  45

His Glu Gln Ala Ala Val His Ala Ala Asp Ala Tyr Ala Arg Val Ser
    50                  55                  60

Gly Lys Val Gly Val Ala Leu Val Thr Ser Gly Pro Gly Val Thr Asn
65                  70                  75                  80

Ala Leu Thr Gly Ile Ala Thr Ala Tyr Thr Asp Ser Ile Pro Met Val
                85                  90                  95

Val Ile Ser Gly Gln Val Gly Asn Ser Leu Ile Gly Thr Asp Ala Phe
            100                 105                 110

Gln Glu Val Asp Thr Val Gly Ile Thr Arg Pro Cys Val Lys His Asn
        115                 120                 125

Phe Leu Val Thr Asp Ile Asn Glu Leu Ala Glu Thr Ile Lys Lys Ala
    130                 135                 140

Phe Gln Ile Ala Ala Ser Gly Arg Pro Gly Pro Val Val Val Asp Val
145                 150                 155                 160

Pro Lys Asp Val Thr Gln Ala Met Ala Lys Phe Ser Tyr Pro Gln Glu

                165                 170                 175

Asp Ile Phe Ile Arg Ser Tyr Gln Pro Val Val Gln Gly His Ile Gly
            180                 185                 190

Gln Ile Lys Lys Ala Val Gln Met Leu Ala Ser Ala Lys Arg Pro Val
        195                 200                 205

Val Tyr Phe Gly Gly Gly Val Val Leu Gly Asn Ala Ser Glu Glu Leu
    210                 215                 220

Thr Arg Phe Val Arg Met Thr Gly Ala Pro Cys Thr Gly Thr Leu Met
225                 230                 235                 240

Gly Leu Gly Ala Tyr Pro Ser Gly Asp Arg Gln Phe Leu Gly Met Leu
                245                 250                 255

Gly Met His Gly Thr Tyr Glu Ala Asn Leu Ala Met Gln Asn Ala Asp
            260                 265                 270

Val Val Leu Ala Val Gly Ala Arg Phe Asp Asp Arg Val Val Ser Val
        275                 280                 285

Pro Ser Lys Phe Phe Glu Lys Ala Lys Lys Val Ile His Ile Asp Val
    290                 295                 300

Asp Pro Ser Ser Ile Ala Lys Arg Val Lys Ala Asp Ile Pro Ile Val
305                 310                 315                 320

Gly Asp Val Lys Asn Val Leu Ser Glu Met Val Ala Leu Trp Gln Lys
                325                 330                 335

Gln Glu Ser Val Pro Ser Glu Asp Ala Leu Gly Lys Trp Trp Lys Thr
            340                 345                 350

Ile Glu Glu Trp Arg Ser Arg Asp Cys Leu Trp Phe Asp Asn Gly Ser
        355                 360                 365

Glu Ile Ile Lys Pro Gln Tyr Val Ile Gln Lys Leu Ala Glu Ile Thr
    370                 375                 380

Gly Asn Ser Ala Ile Ile Thr Ser Asp Val Gly Gln His Gln Met Phe
385                 390                 395                 400

Ala Ala Gln Tyr Tyr Pro Phe Glu Arg Pro Arg Gln Trp Leu Asn Ser
                405                 410                 415

Gly Gly Leu Gly Thr Met Gly Val Gly Leu Pro Tyr Ala Ile Gly Ala

            420                 425                 430

Lys Leu Ala Ala Pro Asp Gln Asp Val Phe Cys Ile Thr Gly Asp Gly
        435                 440                 445

Ser Ile Gln Met Asn Ile Gln Glu Leu Ser Thr Cys Phe Gln Tyr Arg
    450                 455                 460

Ile Pro Val Asn Val Ile Thr Leu Asn Asn Gly Tyr Leu Gly Met Val
465                 470                 475                 480

Arg Gln Trp Gln Glu Ile Tyr Tyr Gly Gly Arg Glu Ser Glu Thr Tyr
                485                 490                 495

Phe Asp Ser Leu Pro Asp Phe Val Lys Leu Ala Glu Ala Tyr Gly His
            500                 505                 510

Ile Gly Ile Arg Val Asp Lys Lys Ser Asp Val Glu Gly Ala Leu Leu
        515                 520                 525

Glu Ala Leu Asn Gln Lys Asp Arg Leu Val Phe Ile Asp Phe Leu Thr
    530                 535                 540

Asp Gln Lys Gln Asn Val Met Pro Met Val Gly Asn Gly Lys Gly Leu
545                 550                 555                 560

Asp Glu Met Val Leu Pro Pro His Met Arg Ala Asp Gly Lys Ala
                565                 570                 575

<210> 97
<211> 570
<212> DNA
<213> Neisseria meningitidis

<220>
<221> CDS
<222> (1)..(570)

<400> 97
atg gac aat cac gcc gaa gca cac tgg caa aac ggc tgg ctt caa agc 48
Met Asp Asn His Ala Glu Ala His Trp Gln Asn Gly Trp Leu Gln Ser
1               5                   10                  15

ata cgc cat acc ccg tcg ccc aat ttc agc ccg agg gaa acg ggg gaa 96
Ile Arg His Thr Pro Ser Pro Asn Phe Ser Pro Arg Glu Thr Gly Glu
            20                  25                  30

acg gtt tcc ctg atc gtg ttg cac aac att tca ctg ccg ccg ttc gaa 144
Thr Val Ser Leu Ile Val Leu His Asn Ile Ser Leu Pro Pro Phe Glu
        35                  40                  45

tac ggc acg gat gct gtg gaa aag ctg ttt gcc aac cgg ctc gac ccc 192
Tyr Gly Thr Asp Ala Val Glu Lys Leu Phe Ala Asn Arg Leu Asp Pro
    50                  55                  60

aac gga cat ccg ttc ttc agc ctg ata cac act ttg cgc gta tcc agc 240
Asn Gly His Pro Phe Phe Ser Leu Ile His Thr Leu Arg Val Ser Ser
65                  70                  75                  80

cat ttc tta atc aaa cgc gac ggc aaa acg gtg cag ttc gta tca tgc 288
His Phe Leu Ile Lys Arg Asp Gly Lys Thr Val Gln Phe Val Ser Cys
                85                  90                  95

ggc gat atg gcg tac cac gcg ggc gta tcc tcg ttt cgc gga cgg gaa 336
Gly Asp Met Ala Tyr His Ala Gly Val Ser Ser Phe Arg Gly Arg Glu
            100                 105                 110

aaa tgc aac gca ttt tcc atc ggc atc gaa ttg gaa ggc tgc gat ttc 384
Lys Cys Asn Ala Phe Ser Ile Gly Ile Glu Leu Glu Gly Cys Asp Phe
        115                 120                 125

gaa ccc ttt acc gaa gcg caa tac cgt tcg ctc gaa aca ttg ttg gaa 432
Glu Pro Phe Thr Glu Ala Gln Tyr Arg Ser Leu Glu Thr Leu Leu Glu
    130                 135                 140

gca ctc tgc cgc cgc tac ccc gtt acc gca gta acc gga cat cag gac 480
Ala Leu Cys Arg Arg Tyr Pro Val Thr Ala Val Thr Gly His Gln Asp
145                 150                 155                 160

atc gcg ccc ggc cgc aaa acc gac ccc ggc cac ttt ttc gac tgg cgg 528
Ile Ala Pro Gly Arg Lys Thr Asp Pro Gly His Phe Phe Asp Trp Arg
                165                 170                 175

cgg ata cgg gag aaa ggg ttt ccc gta gac aga aat gcc gtc 570
Arg Ile Arg Glu Lys Gly Phe Pro Val Asp Arg Asn Ala Val
            180                 185                 190

<210> 98
<211> 190
<212> PRT
<213> Neisseria meningitidis

<400> 98

Met Asp Asn His Ala Glu Ala His Trp Gln Asn Gly Trp Leu Gln Ser
1               5                   10                  15

Ile Arg His Thr Pro Ser Pro Asn Phe Ser Pro Arg Glu Thr Gly Glu
            20                  25                  30

Thr Val Ser Leu Ile Val Leu His Asn Ile Ser Leu Pro Pro Phe Glu
        35                  40                  45

Tyr Gly Thr Asp Ala Val Glu Lys Leu Phe Ala Asn Arg Leu Asp Pro
    50                  55                  60

Asn Gly His Pro Phe Phe Ser Leu Ile His Thr Leu Arg Val Ser Ser
65                  70                  75                  80

His Phe Leu Ile Lys Arg Asp Gly Lys Thr Val Gln Phe Val Ser Cys
                85                  90                  95

Gly Asp Met Ala Tyr His Ala Gly Val Ser Ser Phe Arg Gly Arg Glu
            100                 105                 110

Lys Cys Asn Ala Phe Ser Ile Gly Ile Glu Leu Glu Gly Cys Asp Phe
        115                 120                 125

Glu Pro Phe Thr Glu Ala Gln Tyr Arg Ser Leu Glu Thr Leu Leu Glu
    130                 135                 140

Ala Leu Cys Arg Arg Tyr Pro Val Thr Ala Val Thr Gly His Gln Asp
145                 150                 155                 160

Ile Ala Pro Gly Arg Lys Thr Asp Pro Gly His Phe Phe Asp Trp Arg
                165                 170                 175

Arg Ile Arg Glu Lys Gly Phe Pro Val Asp Arg Asn Ala Val
            180                 185                 190

<210> 99
<211> 1515
<212> DNA
<213> Neisseria meningitidis

<220>
<221> CDS
<222> (1)..(1515)

<400> 99

atg ttg tat ttc aga tac ggt ttt ttg gtt gtt tgg tgt gcg gca ggt 48
Met Leu Tyr Phe Arg Tyr Gly Phe Leu Val Val Trp Cys Ala Ala Gly
1               5                   10                  15

gtt tct gcc gcc tat ggg gcg gat gcg ccc gcg att ttg gat gac aag 96
Val Ser Ala Ala Tyr Gly Ala Asp Ala Pro Ala Ile Leu Asp Asp Lys
            20                  25                  30

gca ttg ttg cag gtg cag cgg tcg gtg tcg gat aag tgg gcg gaa tcg 144
Ala Leu Leu Gln Val Gln Arg Ser Val Ser Asp Lys Trp Ala Glu Ser
        35                  40                  45

gat tgg aaa gtt gac aat gat gcc ccg cgc gtg gtt gac ggg gat ttt 192
Asp Trp Lys Val Asp Asn Asp Ala Pro Arg Val Val Asp Gly Asp Phe
    50                  55                  60

ttg ttg gcg cat ccg aaa atg ttg gaa cat agt ttg cgc gac gtg ctc 240
Leu Leu Ala His Pro Lys Met Leu Glu His Ser Leu Arg Asp Val Leu
65                  70                  75                  80

aac ggc aat cag gcg gat ttg atc gct tcg ttg gcg gat ttg tat gcc 288
Asn Gly Asn Gln Ala Asp Leu Ile Ala Ser Leu Ala Asp Leu Tyr Ala
                85                  90                  95

aag ctg ccg gat tat gac gcg gtt ttg tac ggc agg gcg cgg gct ttg 336
Lys Leu Pro Asp Tyr Asp Ala Val Leu Tyr Gly Arg Ala Arg Ala Leu
            100                 105                 110

ctg gcg aaa ttg gcg gga agg ccg gcg gag gcg gtg gcg cgg tat cgg 384
Leu Ala Lys Leu Ala Gly Arg Pro Ala Glu Ala Val Ala Arg Tyr Arg
        115                 120                 125

gaa ctg cac ggg gaa aat gcg gca gac gag cgg att ttg ctg gat ttg 432
Glu Leu His Gly Glu Asn Ala Ala Asp Glu Arg Ile Leu Leu Asp Leu
    130                 135                 140

gcg gcg gcg gag ttt gac gat ttc cgg ctg aag tcg gca gaa agg cat 480
Ala Ala Ala Glu Phe Asp Asp Phe Arg Leu Lys Ser Ala Glu Arg His
145                 150                 155                 160

ttt gcc gag gcg gaa aaa ttg gat ttg ccg gcg ccg gtt ttg gaa aat 528
Phe Ala Glu Ala Glu Lys Leu Asp Leu Pro Ala Pro Val Leu Glu Asn
                165                 170                 175

gtg ggg cgt ttt cgg aaa aaa gcg gag ggg ctg acg ggc tgg cgt ttt 576
Val Gly Arg Phe Arg Lys Lys Ala Glu Gly Leu Thr Gly Trp Arg Phe
            180                 185                 190

tcg ggc ggc atc agt ccg gcg gtc aat aga aat gcc aat aat gcc gcg    624
Ser Gly Gly Ile Ser Pro Ala Val Asn Arg Asn Ala Asn Asn Ala Ala
        195                 200                 205

ccg cag tat tgc cgg caa aac gga ggc cgg cag ata tgc agt gtc agc    672
Pro Gln Tyr Cys Arg Gln Asn Gly Gly Arg Gln Ile Cys Ser Val Ser
    210                 215                 220

cgg gcg gag cgg gcg gca ggc ttg aat tat gaa atc gag gcg gaa aaa    720
Arg Ala Glu Arg Ala Ala Gly Leu Asn Tyr Glu Ile Glu Ala Glu Lys
225                 230                 235                 240

ctg acg gcg ttg gca gat aat cat tat ttg ttg ttc cgt tcc aat atc    768
Leu Thr Ala Leu Ala Asp Asn His Tyr Leu Leu Phe Arg Ser Asn Ile
                245                 250                 255

ggc ggc acg agc tat tat ttc agt aaa aaa tca gct tat gac gac ggg    816
Gly Gly Thr Ser Tyr Tyr Phe Ser Lys Lys Ser Ala Tyr Asp Asp Gly
            260                 265                 270

ttc ggc aga gcg tat ttg ggt tgg cag tat aaa aat gca cgg cag acg    864
Phe Gly Arg Ala Tyr Leu Gly Trp Gln Tyr Lys Asn Ala Arg Gln Thr
        275                 280                 285

gcg ggg att ttg ccg ttt tat cag gtg cag ttg tcg ggc agc gac ggc    912
Ala Gly Ile Leu Pro Phe Tyr Gln Val Gln Leu Ser Gly Ser Asp Gly
    290                 295                 300

ttt gat gcg aaa aca aaa cgg gta aac aac cgc cgc ctg ccg ccg tat    960
Phe Asp Ala Lys Thr Lys Arg Val Asn Asn Arg Arg Leu Pro Pro Tyr
305                 310                 315                 320

atg ctg gcg cac gga gtc ggc gtg cag ttg tcc cat act tac cgc cca    1008
Met Leu Ala His Gly Val Gly Val Gln Leu Ser His Thr Tyr Arg Pro
                325                 330                 335

aac ccg gga tgg caa ttt tcg gtc gcg ctg gaa cat tac cgc caa cgc    1056
Asn Pro Gly Trp Gln Phe Ser Val Ala Leu Glu His Tyr Arg Gln Arg
            340                 345                 350

tac cgc gaa cag gat agg gcg gaa tac aat aac ggt cgg cag gac ggg    1104
Tyr Arg Glu Gln Asp Arg Ala Glu Tyr Asn Asn Gly Arg Gln Asp Gly
        355                 360                 365

ttt tat gtt tcg tcg gca aaa cgt ttg ggc gaa tcg gca act gtg ttc    1152
Phe Tyr Val Ser Ser Ala Lys Arg Leu Gly Glu Ser Ala Thr Val Phe
    370                 375                 380

ggc ggc tgg cag ttt gtg cgg ttt gtg ccg aaa cgc gaa acg gtg ggc 1200
Gly Gly Trp Gln Phe Val Arg Phe Val Pro Lys Arg Glu Thr Val Gly
385                 390                 395                 400

ggc gcg gtc aat aat gcc gcc tac cgg cgc aac ggt gtt tat gcc ggc 1248
Gly Ala Val Asn Asn Ala Ala Tyr Arg Arg Asn Gly Val Tyr Ala Gly
                405                 410                 415

tgg gcg cag gag tgg cgg cag ttg ggc ggt ttg aac agt cgg gtt tcc 1296
Trp Ala Gln Glu Trp Arg Gln Leu Gly Gly Leu Asn Ser Arg Val Ser
            420                 425                 430

gcg tct tat gcc cgc cgc aac tat aag ggc gtt gcg gct ttc tcg aca 1344
Ala Ser Tyr Ala Arg Arg Asn Tyr Lys Gly Val Ala Ala Phe Ser Thr
        435                 440                 445

gag gcg caa cgc aac cgc gaa tgg aat gtc tcg ctg gct ttg agc cac 1392
Glu Ala Gln Arg Asn Arg Glu Trp Asn Val Ser Leu Ala Leu Ser His
    450                 455                 460

gac aag ttg tcg tac aaa ggt atc gtg ccc gcg ttg aat tat cgt ttc 1440
Asp Lys Leu Ser Tyr Lys Gly Ile Val Pro Ala Leu Asn Tyr Arg Phe
465                 470                 475                 480

ggc agg acg gaa agt aat gtg ccg tat gcg aaa cgc cgc aac agc gag 1488
Gly Arg Thr Glu Ser Asn Val Pro Tyr Ala Lys Arg Arg Asn Ser Glu
                485                 490                 495

gtg ttt gtg tcg gcg gat tgg cgg ttt 1515
Val Phe Val Ser Ala Asp Trp Arg Phe
            500                 505

<210> 100
<211> 505
<212> PRT
<213> Neisseria meningitidis

<400> 100
Met Leu Tyr Phe Arg Tyr Gly Phe Leu Val Val Trp Cys Ala Ala Gly
1               5                   10                  15

Val Ser Ala Ala Tyr Gly Ala Asp Ala Pro Ala Ile Leu Asp Asp Lys
            20                  25                  30

Ala Leu Leu Gln Val Gln Arg Ser Val Ser Asp Lys Trp Ala Glu Ser
        35                  40                  45

Asp Trp Lys Val Asp Asn Asp Ala Pro Arg Val Val Asp Gly Asp Phe
    50                  55                  60

Leu Leu Ala His Pro Lys Met Leu Glu His Ser Leu Arg Asp Val Leu
65                  70                  75                  80

Asn Gly Asn Gln Ala Asp Leu Ile Ala Ser Leu Ala Asp Leu Tyr Ala
                85                  90                  95

Lys Leu Pro Asp Tyr Asp Ala Val Leu Tyr Gly Arg Ala Arg Ala Leu
            100                 105                 110

Leu Ala Lys Leu Ala Gly Arg Pro Ala Glu Ala Val Ala Arg Tyr Arg
        115                 120                 125

Glu Leu His Gly Glu Asn Ala Ala Asp Glu Arg Ile Leu Leu Asp Leu
    130                 135                 140

Ala Ala Ala Glu Phe Asp Asp Phe Arg Leu Lys Ser Ala Glu Arg His
145                 150                 155                 160

Phe Ala Glu Ala Glu Lys Leu Asp Leu Pro Ala Pro Val Leu Glu Asn
                165                 170                 175

Val Gly Arg Phe Arg Lys Lys Ala Glu Gly Leu Thr Gly Trp Arg Phe
            180                 185                 190

Ser Gly Gly Ile Ser Pro Ala Val Asn Arg Asn Ala Asn Asn Ala Ala
        195                 200                 205

Pro Gln Tyr Cys Arg Gln Asn Gly Gly Arg Gln Ile Cys Ser Val Ser
    210                 215                 220

Arg Ala Glu Arg Ala Ala Gly Leu Asn Tyr Glu Ile Glu Ala Glu Lys
225                 230                 235                 240

Leu Thr Ala Leu Ala Asp Asn His Tyr Leu Leu Phe Arg Ser Asn Ile
                245                 250                 255

Gly Gly Thr Ser Tyr Tyr Phe Ser Lys Lys Ser Ala Tyr Asp Asp Gly
            260                 265                 270

Phe Gly Arg Ala Tyr Leu Gly Trp Gln Tyr Lys Asn Ala Arg Gln Thr
        275                 280                 285

Ala Gly Ile Leu Pro Phe Tyr Gln Val Gln Leu Ser Gly Ser Asp Gly
    290                 295                 300

Phe Asp Ala Lys Thr Lys Arg Val Asn Asn Arg Arg Leu Pro Pro Tyr
305                 310                 315                 320

Met Leu Ala His Gly Val Gly Val Gln Leu Ser His Thr Tyr Arg Pro
                325                 330                 335

Asn Pro Gly Trp Gln Phe Ser Val Ala Leu Glu His Tyr Arg Gln Arg
            340                 345                 350

Tyr Arg Glu Gln Asp Arg Ala Glu Tyr Asn Asn Gly Arg Gln Asp Gly
        355                 360                 365

Phe Tyr Val Ser Ser Ala Lys Arg Leu Gly Glu Ser Ala Thr Val Phe
    370                 375                 380

Gly Gly Trp Gln Phe Val Arg Phe Val Pro Lys Arg Glu Thr Val Gly
385                 390                 395                 400

Gly Ala Val Asn Asn Ala Ala Tyr Arg Arg Asn Gly Val Tyr Ala Gly
                405                 410                 415

Trp Ala Gln Glu Trp Arg Gln Leu Gly Gly Leu Asn Ser Arg Val Ser
            420                 425                 430

Ala Ser Tyr Ala Arg Arg Asn Tyr Lys Gly Val Ala Ala Phe Ser Thr
        435                 440                 445

Glu Ala Gln Arg Asn Arg Glu Trp Asn Val Ser Leu Ala Leu Ser His
    450                 455                 460

Asp Lys Leu Ser Tyr Lys Gly Ile Val Pro Ala Leu Asn Tyr Arg Phe
465                 470                 475                 480

Gly Arg Thr Glu Ser Asn Val Pro Tyr Ala Lys Arg Arg Asn Ser Glu
                485                 490                 495

Val Phe Val Ser Ala Asp Trp Arg Phe
            500                 505

<210> 101
<211> 1476
<212> DNA
<213> Neisseria meningitidis

<220>
<221> CDS

<222> (1)..(1476)

<400> 101
atg aaa tac aaa gac ctg cgc gac ttc atc gcc atg ctc gag cag cag 48
Met Lys Tyr Lys Asp Leu Arg Asp Phe Ile Ala Met Leu Glu Gln Gln
1               5                   10                  15

ggc aaa ctc aaa cgc atc gcg cac ccc gtt tcc ccg cat ttg gaa atg 96
Gly Lys Leu Lys Arg Ile Ala His Pro Val Ser Pro His Leu Glu Met
            20                  25                  30

acc gaa atc gcc gac cgc gtg ctg cgc gcc gaa ggg ccg gcg ttg ttg 144
Thr Glu Ile Ala Asp Arg Val Leu Arg Ala Glu Gly Pro Ala Leu Leu
        35                  40                  45

ttt gaa cac cca gtt aag ccc gac ggt acg cgc tat gat tat ccc gtg 192
Phe Glu His Pro Val Lys Pro Asp Gly Thr Arg Tyr Asp Tyr Pro Val
    50                  55                  60

ttg gca aac ctg ttc ggc acg ccc gaa cgt gtg gcg atg ggc atg ggc 240
Leu Ala Asn Leu Phe Gly Thr Pro Glu Arg Val Ala Met Gly Met Gly
65                  70                  75                  80

gcg gac agc gtg tcc aag ctg cgc gaa atc ggg cag acg ctg gcg tat 288
Ala Asp Ser Val Ser Lys Leu Arg Glu Ile Gly Gln Thr Leu Ala Tyr
                85                  90                  95

ttg aaa gaa ccc gaa ccg ccc aaa ggc att aaa gac gcg ttt tcc aaa 336
Leu Lys Glu Pro Glu Pro Pro Lys Gly Ile Lys Asp Ala Phe Ser Lys
            100                 105                 110

ctg ccg ctc ttg aaa gac att tgg agc atg gcg ccg aac gtg gtg aaa 384
Leu Pro Leu Leu Lys Asp Ile Trp Ser Met Ala Pro Asn Val Val Lys
        115                 120                 125

aat gcg ccg tgt cag gaa atc gta tgg gaa ggc gaa gac gtt gat ttg 432
Asn Ala Pro Cys Gln Glu Ile Val Trp Glu Gly Glu Asp Val Asp Leu
    130                 135                 140

tat caa ctt ccg att cag cat tgc tgg ccg gaa gac gtt gcg ccg ctg 480
Tyr Gln Leu Pro Ile Gln His Cys Trp Pro Glu Asp Val Ala Pro Leu
145                 150                 155                 160

gta acg tgg ggc ttg acc gtc acg cgc ggg ccg cac aaa aaa cgc caa 528
Val Thr Trp Gly Leu Thr Val Thr Arg Gly Pro His Lys Lys Arg Gln
                165                 170                 175

aat ctc ggc att tac cgc caa caa tta atc ggc ata aac aag ctg att 576

Asn Leu Gly Ile Tyr Arg Gln Gln Leu Ile Gly Ile Asn Lys Leu Ile
        180                 185                 190

atg cgc tgg ctg tcg cat cgc ggc ggc gcg ctg gat tat cag gaa ttc     624
Met Arg Trp Leu Ser His Arg Gly Gly Ala Leu Asp Tyr Gln Glu Phe
    195                 200                 205

cgc aaa ctc aat ccc gat acg ccg tat ccc gtc gcc gtc gtg ctc ggc     672
Arg Lys Leu Asn Pro Asp Thr Pro Tyr Pro Val Ala Val Val Leu Gly
    210                 215                 220

tgc gac cct gcc acc att ttg ggc gcg gta acg ccc gtt ccc gat acc     720
Cys Asp Pro Ala Thr Ile Leu Gly Ala Val Thr Pro Val Pro Asp Thr
225                 230                 235                 240

ttg agc gaa tac cag ttt gcc gga ctg ctg cgc ggt tcg cgg acg gaa     768
Leu Ser Glu Tyr Gln Phe Ala Gly Leu Leu Arg Gly Ser Arg Thr Glu
                245                 250                 255

ctg gtg aaa tgt atc ggc aac gat ttg caa gtg cct gcc cgc gca gaa     816
Leu Val Lys Cys Ile Gly Asn Asp Leu Gln Val Pro Ala Arg Ala Glu
            260                 265                 270

atc gtg ttg gaa ggc gtc atc cat cca aac gaa acc gcg ttg gaa ggt     864
Ile Val Leu Glu Gly Val Ile His Pro Asn Glu Thr Ala Leu Glu Gly
        275                 280                 285

cca tac ggc gac cac acg ggc tat tac aac gag cag gac cat ttc ccc     912
Pro Tyr Gly Asp His Thr Gly Tyr Tyr Asn Glu Gln Asp His Phe Pro
    290                 295                 300

gtg ttt acg gtc gaa cgc atc acc atg cgc gaa aac ccg att tac cac     960
Val Phe Thr Val Glu Arg Ile Thr Met Arg Glu Asn Pro Ile Tyr His
305                 310                 315                 320

tct acc tac aca ggc aaa ccg ccc gat gaa ccc gcc gtt ttg ggc gtg     1008
Ser Thr Tyr Thr Gly Lys Pro Pro Asp Glu Pro Ala Val Leu Gly Val
                325                 330                 335

gcg ttg aac gaa gtg ttc gta ccg ctt ttg caa aag cag ttc ccc gaa     1056
Ala Leu Asn Glu Val Phe Val Pro Leu Leu Gln Lys Gln Phe Pro Glu
            340                 345                 350

atc acc gat ttt tac ctg ccg ccc gaa ggc tgc tcc tac cgc atg gcg     1104
Ile Thr Asp Phe Tyr Leu Pro Pro Glu Gly Cys Ser Tyr Arg Met Ala
        355                 360                 365

gtg gtg agc atg aaa aaa cag tac gcc gga cac gcc aag cgc gtg atg     1152

Val Val Ser Met Lys Lys Gln Tyr Ala Gly His Ala Lys Arg Val Met
    370                 375                 380

atg ggc tgc tgg tcg ttc ctg cgc cag ttt atg tac acc aaa ttc atc      1200
Met Gly Cys Trp Ser Phe Leu Arg Gln Phe Met Tyr Thr Lys Phe Ile
385                 390                 395                 400

att gtg gtg gat gac gat gtg gat gtg cgc gac tgg aaa gaa gtc atc      1248
Ile Val Val Asp Asp Asp Val Asp Val Arg Asp Trp Lys Glu Val Ile
                405                 410                 415

tgg gcg gta acc acg cgc atg gac ccc gtg cgc gat acc gtt ttg atg      1296
Trp Ala Val Thr Thr Arg Met Asp Pro Val Arg Asp Thr Val Leu Met
            420                 425                 430

gaa aac acg ccc atc gac tac ctc gac ttc gcc agc ccc gtc agc gga      1344
Glu Asn Thr Pro Ile Asp Tyr Leu Asp Phe Ala Ser Pro Val Ser Gly
        435                 440                 445

ctt ggc ggc aaa atg ggt ttg gat gcg acc aac aag tgg ccg ggc gaa      1392
Leu Gly Gly Lys Met Gly Leu Asp Ala Thr Asn Lys Trp Pro Gly Glu
    450                 455                 460

acc gac cgc gaa tgg gga cgg gtg att aaa aaa gac cct gcg gtt acg      1440
Thr Asp Arg Glu Trp Gly Arg Val Ile Lys Lys Asp Pro Ala Val Thr
465                 470                 475                 480

gct aag att gat gag att tgg gag gaa ttg ggg ttg                      1476
Ala Lys Ile Asp Glu Ile Trp Glu Glu Leu Gly Leu
                485                 490

<210> 102
<211> 492
<212> PRT
<213> Neisseria meningitidis

<400> 102
Met Lys Tyr Lys Asp Leu Arg Asp Phe Ile Ala Met Leu Glu Gln Gln
  1               5                  10                  15

Gly Lys Leu Lys Arg Ile Ala His Pro Val Ser Pro His Leu Glu Met
             20                  25                  30

Thr Glu Ile Ala Asp Arg Val Leu Arg Ala Glu Gly Pro Ala Leu Leu
         35                  40                  45

Phe Glu His Pro Val Lys Pro Asp Gly Thr Arg Tyr Asp Tyr Pro Val

50                  55                  60

Leu Ala Asn Leu Phe Gly Thr Pro Glu Arg Val Ala Met Gly Met Gly
65                  70                  75                  80

Ala Asp Ser Val Ser Lys Leu Arg Glu Ile Gly Gln Thr Leu Ala Tyr
                85                  90                  95

Leu Lys Glu Pro Glu Pro Pro Lys Gly Ile Lys Asp Ala Phe Ser Lys
            100                 105                 110

Leu Pro Leu Leu Lys Asp Ile Trp Ser Met Ala Pro Asn Val Val Lys
        115                 120                 125

Asn Ala Pro Cys Gln Glu Ile Val Trp Glu Gly Glu Asp Val Asp Leu
    130                 135                 140

Tyr Gln Leu Pro Ile Gln His Cys Trp Pro Glu Asp Val Ala Pro Leu
145                 150                 155                 160

Val Thr Trp Gly Leu Thr Val Thr Arg Gly Pro His Lys Lys Arg Gln
                165                 170                 175

Asn Leu Gly Ile Tyr Arg Gln Gln Leu Ile Gly Ile Asn Lys Leu Ile
            180                 185                 190

Met Arg Trp Leu Ser His Arg Gly Gly Ala Leu Asp Tyr Gln Glu Phe
        195                 200                 205

Arg Lys Leu Asn Pro Asp Thr Pro Tyr Pro Val Ala Val Val Leu Gly
    210                 215                 220

Cys Asp Pro Ala Thr Ile Leu Gly Ala Val Thr Pro Val Pro Asp Thr
225                 230                 235                 240

Leu Ser Glu Tyr Gln Phe Ala Gly Leu Leu Arg Gly Ser Arg Thr Glu
                245                 250                 255

Leu Val Lys Cys Ile Gly Asn Asp Leu Gln Val Pro Ala Arg Ala Glu
            260                 265                 270

Ile Val Leu Glu Gly Val Ile His Pro Asn Glu Thr Ala Leu Glu Gly
        275                 280                 285

Pro Tyr Gly Asp His Thr Gly Tyr Tyr Asn Glu Gln Asp His Phe Pro
    290                 295                 300

Val Phe Thr Val Glu Arg Ile Thr Met Arg Glu Asn Pro Ile Tyr His

305                 310                 315                 320

Ser Thr Tyr Thr Gly Lys Pro Pro Asp Glu Pro Ala Val Leu Gly Val
                325                 330                 335

Ala Leu Asn Glu Val Phe Val Pro Leu Leu Gln Lys Gln Phe Pro Glu
            340                 345                 350

Ile Thr Asp Phe Tyr Leu Pro Pro Glu Gly Cys Ser Tyr Arg Met Ala
        355                 360                 365

Val Val Ser Met Lys Lys Gln Tyr Ala Gly His Ala Lys Arg Val Met
    370                 375                 380

Met Gly Cys Trp Ser Phe Leu Arg Gln Phe Met Tyr Thr Lys Phe Ile
385                 390                 395                 400

Ile Val Val Asp Asp Asp Val Asp Val Arg Asp Trp Lys Glu Val Ile
                405                 410                 415

Trp Ala Val Thr Thr Arg Met Asp Pro Val Arg Asp Thr Val Leu Met
            420                 425                 430

Glu Asn Thr Pro Ile Asp Tyr Leu Asp Phe Ala Ser Pro Val Ser Gly
        435                 440                 445

Leu Gly Gly Lys Met Gly Leu Asp Ala Thr Asn Lys Trp Pro Gly Glu
    450                 455                 460

Thr Asp Arg Glu Trp Gly Arg Val Ile Lys Lys Asp Pro Ala Val Thr
465                 470                 475                 480

Ala Lys Ile Asp Glu Ile Trp Glu Glu Leu Gly Leu
                485                 490

<210> 103
<211> 1089
<212> DNA
<213> Neisseria meningitidis

<220>
<221> CDS
<222> (1)..(1089)

<400> 103
atg agt ttg aaa tgc ggc atc gtc ggt ttg ccc aac gtc ggc aaa tcc     48

Met Ser Leu Lys Cys Gly Ile Val Gly Leu Pro Asn Val Gly Lys Ser
1               5                   10                  15

acc ctt ttt aac gcg ctg acc caa tcg ggt atc gaa gcg gca aac tat    96
Thr Leu Phe Asn Ala Leu Thr Gln Ser Gly Ile Glu Ala Ala Asn Tyr
            20                  25                  30

cct ttc tgt acc atc gaa ccc aac gtc ggc atc gtc gaa gtc ccc gat    144
Pro Phe Cys Thr Ile Glu Pro Asn Val Gly Ile Val Glu Val Pro Asp
        35                  40                  45

ccg cgt atg gcc gaa ttg gca aaa atc gtc aat ccg caa aaa atg cag    192
Pro Arg Met Ala Glu Leu Ala Lys Ile Val Asn Pro Gln Lys Met Gln
    50                  55                  60

cct gcc atc gtc gaa ttt gtc gat att gcc ggt ttg gtt gca ggc gcg    240
Pro Ala Ile Val Glu Phe Val Asp Ile Ala Gly Leu Val Ala Gly Ala
65                  70                  75                  80

agc aaa ggc gag ggc ttg ggc aac cag ttc ctt gcc aac atc cgc gaa    288
Ser Lys Gly Glu Gly Leu Gly Asn Gln Phe Leu Ala Asn Ile Arg Glu
                85                  90                  95

acc gat gcg att gtg aat gtc gtg cgc tgc ttt gac gac gac aac atc    336
Thr Asp Ala Ile Val Asn Val Val Arg Cys Phe Asp Asp Asp Asn Ile
            100                 105                 110

gtc cac gtt gca ggc cgc gtc gat ccg att gcc gac att gaa acc atc    384
Val His Val Ala Gly Arg Val Asp Pro Ile Ala Asp Ile Glu Thr Ile
        115                 120                 125

ggc aca gag ttg gca ctt gcc gac ctg gca agt gtc gaa aaa gcc atc    432
Gly Thr Glu Leu Ala Leu Ala Asp Leu Ala Ser Val Glu Lys Ala Ile
    130                 135                 140

gtc cgc gaa gaa aaa cgc gcc cgc tca ggc gac aaa gac gcg caa aag    480
Val Arg Glu Glu Lys Arg Ala Arg Ser Gly Asp Lys Asp Ala Gln Lys
145                 150                 155                 160

ctg gtc gat ttg tgc aaa aaa ctg ctg ccg cat ctg gac gaa ggc aaa    528
Leu Val Asp Leu Cys Lys Lys Leu Leu Pro His Leu Asp Glu Gly Lys
                165                 170                 175

ccc gtg cgt tcc ttc ggt ttg gac gcg gaa gaa cgc gcg atg ctc aaa    576
Pro Val Arg Ser Phe Gly Leu Asp Ala Glu Glu Arg Ala Met Leu Lys
            180                 185                 190

ccg ctg ttc ctg ctg acc gcc aaa ccg gcg atg tat gtg ggc aac gtc    624

Pro Leu Phe Leu Leu Thr Ala Lys Pro Ala Met Tyr Val Gly Asn Val
        195                 200                 205

gcc gaa gac ggt ttt gaa aac aat ccg cac ctc gac cgc ctg aaa gaa 672
Ala Glu Asp Gly Phe Glu Asn Asn Pro His Leu Asp Arg Leu Lys Glu
    210                 215                 220

ttg gcg gca aaa gaa aac gcc ccc gtc gtc gcc gtt tgc gcc gcg atg 720
Leu Ala Ala Lys Glu Asn Ala Pro Val Val Ala Val Cys Ala Ala Met
225                 230                 235                 240

gag agc gaa att gcc gaa ttg gaa gac gac gaa aaa gcc gag ttc ctc 768
Glu Ser Glu Ile Ala Glu Leu Glu Asp Asp Glu Lys Ala Glu Phe Leu
                245                 250                 255

gcc gaa atg ggc ttg gaa gaa ccg ggc ctg aac cgc ctg att cgt gcc 816
Ala Glu Met Gly Leu Glu Glu Pro Gly Leu Asn Arg Leu Ile Arg Ala
            260                 265                 270

ggt tac gac ctc ttg ggg ctg caa acc tat ttc acc gcc ggt gtg aaa 864
Gly Tyr Asp Leu Leu Gly Leu Gln Thr Tyr Phe Thr Ala Gly Val Lys
        275                 280                 285

gaa gtc cgc gcg tgg acg ata cac aaa ggc gat acc gcc ccg caa gcc 912
Glu Val Arg Ala Trp Thr Ile His Lys Gly Asp Thr Ala Pro Gln Ala
    290                 295                 300

gcc ggc gtg att cac acc gac ttt gag cgc ggc ttc atc cgc gcc caa 960
Ala Gly Val Ile His Thr Asp Phe Glu Arg Gly Phe Ile Arg Ala Gln
305                 310                 315                 320

gtc att tct tac gac gac ttt gtc tcg ctc ggc ggc gaa gcc aaa gcc 1008
Val Ile Ser Tyr Asp Asp Phe Val Ser Leu Gly Gly Glu Ala Lys Ala
                325                 330                 335

aaa gaa gcc ggc aaa atg cgt gtg gaa ggt aag gaa tac gtc gtg cag 1056
Lys Glu Ala Gly Lys Met Arg Val Glu Gly Lys Glu Tyr Val Val Gln
            340                 345                 350

gac ggc gat gta atg cac ttt ttg ttt aac gtg 1089
Asp Gly Asp Val Met His Phe Leu Phe Asn Val
        355                 360

<210> 104
<211> 363
<212> PRT
<213> Neisseria meningitidis

<400> 104
Met Ser Leu Lys Cys Gly Ile Val Gly Leu Pro Asn Val Gly Lys Ser
1               5                   10                  15

Thr Leu Phe Asn Ala Leu Thr Gln Ser Gly Ile Glu Ala Ala Asn Tyr
            20                  25                  30

Pro Phe Cys Thr Ile Glu Pro Asn Val Gly Ile Val Glu Val Pro Asp
        35                  40                  45

Pro Arg Met Ala Glu Leu Ala Lys Ile Val Asn Pro Gln Lys Met Gln
    50                  55                  60

Pro Ala Ile Val Glu Phe Val Asp Ile Ala Gly Leu Val Ala Gly Ala
65                  70                  75                  80

Ser Lys Gly Glu Gly Leu Gly Asn Gln Phe Leu Ala Asn Ile Arg Glu
                85                  90                  95

Thr Asp Ala Ile Val Asn Val Val Arg Cys Phe Asp Asp Asp Asn Ile
            100                 105                 110

Val His Val Ala Gly Arg Val Asp Pro Ile Ala Asp Ile Glu Thr Ile
        115                 120                 125

Gly Thr Glu Leu Ala Leu Ala Asp Leu Ala Ser Val Glu Lys Ala Ile
    130                 135                 140

Val Arg Glu Glu Lys Arg Ala Arg Ser Gly Asp Lys Asp Ala Gln Lys
145                 150                 155                 160

Leu Val Asp Leu Cys Lys Lys Leu Leu Pro His Leu Asp Glu Gly Lys
                165                 170                 175

Pro Val Arg Ser Phe Gly Leu Asp Ala Glu Glu Arg Ala Met Leu Lys
            180                 185                 190

Pro Leu Phe Leu Leu Thr Ala Lys Pro Ala Met Tyr Val Gly Asn Val
        195                 200                 205

Ala Glu Asp Gly Phe Glu Asn Asn Pro His Leu Asp Arg Leu Lys Glu
    210                 215                 220

Leu Ala Ala Lys Glu Asn Ala Pro Val Val Ala Val Cys Ala Ala Met
225                 230                 235                 240

Glu Ser Glu Ile Ala Glu Leu Glu Asp Asp Glu Lys Ala Glu Phe Leu

245                 250                 255

Ala Glu Met Gly Leu Glu Glu Pro Gly Leu Asn Arg Leu Ile Arg Ala
            260                 265                 270

Gly Tyr Asp Leu Leu Gly Leu Gln Thr Tyr Phe Thr Ala Gly Val Lys
        275                 280                 285

Glu Val Arg Ala Trp Thr Ile His Lys Gly Asp Thr Ala Pro Gln Ala
    290                 295                 300

Ala Gly Val Ile His Thr Asp Phe Glu Arg Gly Phe Ile Arg Ala Gln
305                 310                 315                 320

Val Ile Ser Tyr Asp Asp Phe Val Ser Leu Gly Gly Glu Ala Lys Ala
                325                 330                 335

Lys Glu Ala Gly Lys Met Arg Val Glu Gly Lys Glu Tyr Val Val Gln
            340                 345                 350

Asp Gly Asp Val Met His Phe Leu Phe Asn Val
        355                 360

<210> 105
<211> 1177
<212> DNA
<213> Neisseria meningitidis

<220>
<221> CDS
<222> (1)..(1176)

<400> 105
atg gca aaa atg atg aaa tgg gcg gct gtt gcg gcg gtc gcg gcg gca      48
Met Ala Lys Met Met Lys Trp Ala Ala Val Ala Ala Val Ala Ala Ala
1               5                   10                  15

gcg gtt tgg ggc gga tgg tct tat ctg aag ccc gag ccg cag gct gct      96
Ala Val Trp Gly Gly Trp Ser Tyr Leu Lys Pro Glu Pro Gln Ala Ala
            20                  25                  30

tat att acg gaa acg gtc agg cgc ggc gac atc agc cgg acg gtt tct      144
Tyr Ile Thr Glu Thr Val Arg Arg Gly Asp Ile Ser Arg Thr Val Ser
        35                  40                  45

gca aca ggg gag att tcg ccg tcc aac ctg gta tcg gtc ggc gcg cag      192

Ala Thr Gly Glu Ile Ser Pro Ser Asn Leu Val Ser Val Gly Ala Gln
    50                  55                  60

gca tcg ggg cag att aag aaa ctt tat gtc aaa ctc ggg caa cag gtt 240
Ala Ser Gly Gln Ile Lys Lys Leu Tyr Val Lys Leu Gly Gln Gln Val
65                  70                  75                  80

aaa aag ggc gat ttg att gcg gaa atc aat tcg acc tcg cag acc aat 288
Lys Lys Gly Asp Leu Ile Ala Glu Ile Asn Ser Thr Ser Gln Thr Asn
                85                  90                  95

acg ctc aat acg gaa aaa tcc aaa ttg gaa acg tat cag gcg aag ctg 336
Thr Leu Asn Thr Glu Lys Ser Lys Leu Glu Thr Tyr Gln Ala Lys Leu
            100                 105                 110

gtg tcg gca cag att gca ttg ggc agc gcg gag aag aaa tat aag cgt 384
Val Ser Ala Gln Ile Ala Leu Gly Ser Ala Glu Lys Lys Tyr Lys Arg
        115                 120                 125

cag gcg gcg ttg tgg aag gat gat gcg acc gct aaa gaa gat ttg gaa 432
Gln Ala Ala Leu Trp Lys Asp Asp Ala Thr Ala Lys Glu Asp Leu Glu
    130                 135                 140

agc gca cag gat gcg ctt gcc gcc gcc aaa gcc aat gtt gcc gag ctg 480
Ser Ala Gln Asp Ala Leu Ala Ala Ala Lys Ala Asn Val Ala Glu Leu
145                 150                 155                 160

aag gct cta atc aga cag agc aaa att tcc atc aat acc gcc gag tcg 528
Lys Ala Leu Ile Arg Gln Ser Lys Ile Ser Ile Asn Thr Ala Glu Ser
                165                 170                 175

gaa ttg ggc tac acg cgc att acc gca acg atg gac ggc acg gtg gtg 576
Glu Leu Gly Tyr Thr Arg Ile Thr Ala Thr Met Asp Gly Thr Val Val
            180                 185                 190

gcg att ctc gtg gaa gag ggg cag act gtg aac gcg gcg cag tct acg 624
Ala Ile Leu Val Glu Glu Gly Gln Thr Val Asn Ala Ala Gln Ser Thr
        195                 200                 205

ccg acg att gtc caa ttg gcg aat ctg gat atg atg ttg aac aaa atg 672
Pro Thr Ile Val Gln Leu Ala Asn Leu Asp Met Met Leu Asn Lys Met
    210                 215                 220

cag att gcc gag ggc gat att acc aag gtg aag gcg ggg cag gat att 720
Gln Ile Ala Glu Gly Asp Ile Thr Lys Val Lys Ala Gly Gln Asp Ile
225                 230                 235                 240

tcg ttt acg att ttg tcc gaa ccg gat acg ccg att aag gcg aag ctc 768

Ser Phe Thr Ile Leu Ser Glu Pro Asp Thr Pro Ile Lys Ala Lys Leu
                245                 250                 255

gac agc gtc gac ccc ggg ctg acc acg atg tcg tcg ggc ggc tac aac 816
Asp Ser Val Asp Pro Gly Leu Thr Thr Met Ser Ser Gly Gly Tyr Asn
            260                 265                 270

agc agt acg gat acg gct tcc aat gcg gtc tac tat tat gcc cgt tcg 864
Ser Ser Thr Asp Thr Ala Ser Asn Ala Val Tyr Tyr Tyr Ala Arg Ser
        275                 280                 285

ttt gtg ccg aat ccg gac ggc aaa ctc gcc acg ggg atg acg acg cag 912
Phe Val Pro Asn Pro Asp Gly Lys Leu Ala Thr Gly Met Thr Thr Gln
    290                 295                 300

aat acg gtt gaa atc gac ggt gtg aaa aat gtg ctg att att ccg tcg 960
Asn Thr Val Glu Ile Asp Gly Val Lys Asn Val Leu Ile Ile Pro Ser
305                 310                 315                 320

ctg acc gtg aaa aat cgc ggc ggc agg gcg ttt gtg cgc gtg ttg ggt 1008
Leu Thr Val Lys Asn Arg Gly Gly Arg Ala Phe Val Arg Val Leu Gly
                325                 330                 335

gca gac ggc aag gcg gcg gaa cgc gaa atc cgg acc ggt atg aga gac 1056
Ala Asp Gly Lys Ala Ala Glu Arg Glu Ile Arg Thr Gly Met Arg Asp
            340                 345                 350

agt atg aat acc gaa gta aaa agc ggg ttg aaa gag ggg gac aaa gtg 1104
Ser Met Asn Thr Glu Val Lys Ser Gly Leu Lys Glu Gly Asp Lys Val
        355                 360                 365

gtc atc tcc gaa ata acc gcc gcc gag cag cag gaa agc ggc gaa cgc 1152
Val Ile Ser Glu Ile Thr Ala Ala Glu Gln Gln Glu Ser Gly Glu Arg
    370                 375                 380

gcc cta ggc ggc ccg ccg cgc cga t 1177
Ala Leu Gly Gly Pro Pro Arg Arg
385                 390

<210> 106
<211> 392
<212> PRT
<213> Neisseria meningitidis

<400> 106
Met Ala Lys Met Met Lys Trp Ala Ala Val Ala Ala Val Ala Ala Ala
1               5                   10                  15

Ala Val Trp Gly Gly Trp Ser Tyr Leu Lys Pro Glu Pro Gln Ala Ala
            20                  25                  30

Tyr Ile Thr Glu Thr Val Arg Arg Gly Asp Ile Ser Arg Thr Val Ser
        35                  40                  45

Ala Thr Gly Glu Ile Ser Pro Ser Asn Leu Val Ser Val Gly Ala Gln
    50                  55                  60

Ala Ser Gly Gln Ile Lys Lys Leu Tyr Val Lys Leu Gly Gln Gln Val
65                  70                  75                  80

Lys Lys Gly Asp Leu Ile Ala Glu Ile Asn Ser Thr Ser Gln Thr Asn
                85                  90                  95

Thr Leu Asn Thr Glu Lys Ser Lys Leu Glu Thr Tyr Gln Ala Lys Leu
            100                 105                 110

Val Ser Ala Gln Ile Ala Leu Gly Ser Ala Glu Lys Lys Tyr Lys Arg
        115                 120                 125

Gln Ala Ala Leu Trp Lys Asp Asp Ala Thr Ala Lys Glu Asp Leu Glu
    130                 135                 140

Ser Ala Gln Asp Ala Leu Ala Ala Ala Lys Ala Asn Val Ala Glu Leu
145                 150                 155                 160

Lys Ala Leu Ile Arg Gln Ser Lys Ile Ser Ile Asn Thr Ala Glu Ser
                165                 170                 175

Glu Leu Gly Tyr Thr Arg Ile Thr Ala Thr Met Asp Gly Thr Val Val
            180                 185                 190

Ala Ile Leu Val Glu Glu Gly Gln Thr Val Asn Ala Ala Gln Ser Thr
        195                 200                 205

Pro Thr Ile Val Gln Leu Ala Asn Leu Asp Met Met Leu Asn Lys Met
    210                 215                 220

Gln Ile Ala Glu Gly Asp Ile Thr Lys Val Lys Ala Gly Gln Asp Ile
225                 230                 235                 240

Ser Phe Thr Ile Leu Ser Glu Pro Asp Thr Pro Ile Lys Ala Lys Leu
                245                 250                 255

Asp Ser Val Asp Pro Gly Leu Thr Thr Met Ser Ser Gly Gly Tyr Asn
            260                 265                 270

Ser Ser Thr Asp Thr Ala Ser Asn Ala Val Tyr Tyr Tyr Ala Arg Ser
        275                 280                 285

Phe Val Pro Asn Pro Asp Gly Lys Leu Ala Thr Gly Met Thr Thr Gln
    290                 295                 300

Asn Thr Val Glu Ile Asp Gly Val Lys Asn Val Leu Ile Ile Pro Ser
305                 310                 315                 320

Leu Thr Val Lys Asn Arg Gly Gly Arg Ala Phe Val Arg Val Leu Gly
                325                 330                 335

Ala Asp Gly Lys Ala Ala Glu Arg Glu Ile Arg Thr Gly Met Arg Asp
            340                 345                 350

Ser Met Asn Thr Glu Val Lys Ser Gly Leu Lys Glu Gly Asp Lys Val
        355                 360                 365

Val Ile Ser Glu Ile Thr Ala Ala Glu Gln Gln Glu Ser Gly Glu Arg
    370                 375                 380

Ala Leu Gly Gly Pro Pro Arg Arg
385                 390

<210> 107
<211> 1185
<212> DNA
<213> Neisseria meningitidis

<220>
<221> CDS
<222> (1)..(1185)

<400> 107
atg aca gag gac gaa cgt ttc gcg tgg ctg caa ttg gcg ttt acg ccc      48
Met Thr Glu Asp Glu Arg Phe Ala Trp Leu Gln Leu Ala Phe Thr Pro
  1               5                  10                  15

tat atc ggc gcg gaa agt ttc ctg ctg ctg atg cgc cgt ttc ggc agc      96
Tyr Ile Gly Ala Glu Ser Phe Leu Leu Leu Met Arg Arg Phe Gly Ser
             20                  25                  30

gcg caa aat gcc ctg tcc gca ccg gcg gaa cag gtg gcg gca ctg ata     144
Ala Gln Asn Ala Leu Ser Ala Pro Ala Glu Gln Val Ala Ala Leu Ile
         35                  40                  45

cgg cac aaa cag gcg ctt gag gct tgg cgc aat gcg gaa aaa cgc gct 192
Arg His Lys Gln Ala Leu Glu Ala Trp Arg Asn Ala Glu Lys Arg Ala
    50                  55                  60

ctg gcg cgg cag gcg gca gaa gcg gcg ttg gaa tgg gaa atg cgg gac 240
Leu Ala Arg Gln Ala Ala Glu Ala Ala Leu Glu Trp Glu Met Arg Asp
65                  70                  75                  80

gga tgc cgc ctg atg ctg ctt cag gat gaa gat ttc ccc gaa atg ctg 288
Gly Cys Arg Leu Met Leu Leu Gln Asp Glu Asp Phe Pro Glu Met Leu
                85                  90                  95

acg cag ggg ctg acc gcg ccg ccg gtt ttg ttt ttg cgc ggc aac gtg 336
Thr Gln Gly Leu Thr Ala Pro Pro Val Leu Phe Leu Arg Gly Asn Val
            100                 105                 110

cgg ctg ctg cac aag cct tcc gcc gcc atc gtc ggc agc cgc cat gcc 384
Arg Leu Leu His Lys Pro Ser Ala Ala Ile Val Gly Ser Arg His Ala
        115                 120                 125

acg ccg cag gcg atg cgg att gcc aaa gat ttc ggc aag tcg ttg ggc 432
Thr Pro Gln Ala Met Arg Ile Ala Lys Asp Phe Gly Lys Ser Leu Gly
    130                 135                 140

ggg caa aat atc ccc gtt gtg tcg ggt atg gct tcc ggt atc gat act 480
Gly Gln Asn Ile Pro Val Val Ser Gly Met Ala Ser Gly Ile Asp Thr
145                 150                 155                 160

gcc gcc cat cag ggc gca ctg gag gca gaa ggc ggc acc atc gcc gtg 528
Ala Ala His Gln Gly Ala Leu Glu Ala Glu Gly Gly Thr Ile Ala Val
                165                 170                 175

tgg ggg acg ggt ata gac cgc att tac ccg ccg tcc aat aaa aac ctt 576
Trp Gly Thr Gly Ile Asp Arg Ile Tyr Pro Pro Ser Asn Lys Asn Leu
            180                 185                 190

gcc tat gaa atc gcc gaa aaa gga ttg att gtc agc gag ttt ccc atc 624
Ala Tyr Glu Ile Ala Glu Lys Gly Leu Ile Val Ser Glu Phe Pro Ile
        195                 200                 205

ggt acg cgc ccg tat gcc ggc aat ttt ccg cgc cgc aac cgc ctg att 672
Gly Thr Arg Pro Tyr Ala Gly Asn Phe Pro Arg Arg Asn Arg Leu Ile
    210                 215                 220

gcc gcc ctg tcg caa gta acg ctg gtg gtt gaa gcc gcc ttg gaa tcc 720
Ala Ala Leu Ser Gln Val Thr Leu Val Val Glu Ala Ala Leu Glu Ser
225                 230                 235                 240

ggt tcg ctg att acc gcc ggc ctg gcg gca gaa atg ggg cgc gaa gtg 768
Gly Ser Leu Ile Thr Ala Gly Leu Ala Ala Glu Met Gly Arg Glu Val
                245                 250                 255

atg gcg gta ccc ggt tcg ata gac aat ccg cac agc aaa ggc tgc cac 816
Met Ala Val Pro Gly Ser Ile Asp Asn Pro His Ser Lys Gly Cys His
            260                 265                 270

aaa ctg att aaa gac ggc gca aaa ttg gtg gaa tgc ctg gac gac atc 864
Lys Leu Ile Lys Asp Gly Ala Lys Leu Val Glu Cys Leu Asp Asp Ile
        275                 280                 285

ctg aac gaa tgc ccg ggg cta ttg caa aat acg ggt gct tca tca tat 912
Leu Asn Glu Cys Pro Gly Leu Leu Gln Asn Thr Gly Ala Ser Ser Tyr
    290                 295                 300

tct ata aat aag gat acg ccc gac acg gga cgc cgc gcc gtt cag acg 960
Ser Ile Asn Lys Asp Thr Pro Asp Thr Gly Arg Arg Ala Val Gln Thr
305                 310                 315                 320

gca tac gcc ccg ccg cct gcc gca aaa atg ccg tct gaa gcg gcg gca 1008
Ala Tyr Ala Pro Pro Pro Ala Ala Lys Met Pro Ser Glu Ala Ala Ala
                325                 330                 335

ggc ggc aca gcc gtc ggc ggc ata ctg gat aaa atg ggt ttc gac ccc 1056
Gly Gly Thr Ala Val Gly Gly Ile Leu Asp Lys Met Gly Phe Asp Pro
            340                 345                 350

atc cat ccc gac gtg ctt gcc gga cag ttg gct atg cct gcc gca gat 1104
Ile His Pro Asp Val Leu Ala Gly Gln Leu Ala Met Pro Ala Ala Asp
        355                 360                 365

ttg tat gcc gca ctg ttg gaa ttg gaa ttg gac ggc agc gtt gcc gca 1152
Leu Tyr Ala Ala Leu Leu Glu Leu Glu Leu Asp Gly Ser Val Ala Ala
    370                 375                 380

atg ccc agc ggc aga tac cag cgt atc cga act 1185
Met Pro Ser Gly Arg Tyr Gln Arg Ile Arg Thr
385                 390                 395

<210> 108
<211> 395
<212> PRT
<213> Neisseria meningitidis

<400> 108

Met Thr Glu Asp Glu Arg Phe Ala Trp Leu Gln Leu Ala Phe Thr Pro
1               5                   10                  15

Tyr Ile Gly Ala Glu Ser Phe Leu Leu Leu Met Arg Arg Phe Gly Ser
            20                  25                  30

Ala Gln Asn Ala Leu Ser Ala Pro Ala Glu Gln Val Ala Ala Leu Ile
        35                  40                  45

Arg His Lys Gln Ala Leu Glu Ala Trp Arg Asn Ala Glu Lys Arg Ala
    50                  55                  60

Leu Ala Arg Gln Ala Ala Glu Ala Ala Leu Glu Trp Glu Met Arg Asp
65                  70                  75                  80

Gly Cys Arg Leu Met Leu Leu Gln Asp Glu Asp Phe Pro Glu Met Leu
                85                  90                  95

Thr Gln Gly Leu Thr Ala Pro Pro Val Leu Phe Leu Arg Gly Asn Val
            100                 105                 110

Arg Leu Leu His Lys Pro Ser Ala Ala Ile Val Gly Ser Arg His Ala
        115                 120                 125

Thr Pro Gln Ala Met Arg Ile Ala Lys Asp Phe Gly Lys Ser Leu Gly
    130                 135                 140

Gly Gln Asn Ile Pro Val Val Ser Gly Met Ala Ser Gly Ile Asp Thr
145                 150                 155                 160

Ala Ala His Gln Gly Ala Leu Glu Ala Glu Gly Gly Thr Ile Ala Val
                165                 170                 175

Trp Gly Thr Gly Ile Asp Arg Ile Tyr Pro Pro Ser Asn Lys Asn Leu
            180                 185                 190

Ala Tyr Glu Ile Ala Glu Lys Gly Leu Ile Val Ser Glu Phe Pro Ile
        195                 200                 205

Gly Thr Arg Pro Tyr Ala Gly Asn Phe Pro Arg Arg Asn Arg Leu Ile
    210                 215                 220

Ala Ala Leu Ser Gln Val Thr Leu Val Val Glu Ala Ala Leu Glu Ser
225                 230                 235                 240

Gly Ser Leu Ile Thr Ala Gly Leu Ala Ala Glu Met Gly Arg Glu Val
                245                 250                 255

Met Ala Val Pro Gly Ser Ile Asp Asn Pro His Ser Lys Gly Cys His
            260                 265                 270

Lys Leu Ile Lys Asp Gly Ala Lys Leu Val Glu Cys Leu Asp Asp Ile
        275                 280                 285

Leu Asn Glu Cys Pro Gly Leu Leu Gln Asn Thr Gly Ala Ser Ser Tyr
    290                 295                 300

Ser Ile Asn Lys Asp Thr Pro Asp Thr Gly Arg Arg Ala Val Gln Thr
305                 310                 315                 320

Ala Tyr Ala Pro Pro Pro Ala Ala Lys Met Pro Ser Glu Ala Ala Ala
                325                 330                 335

Gly Gly Thr Ala Val Gly Gly Ile Leu Asp Lys Met Gly Phe Asp Pro
            340                 345                 350

Ile His Pro Asp Val Leu Ala Gly Gln Leu Ala Met Pro Ala Ala Asp
        355                 360                 365

Leu Tyr Ala Ala Leu Leu Glu Leu Glu Leu Asp Gly Ser Val Ala Ala
    370                 375                 380

Met Pro Ser Gly Arg Tyr Gln Arg Ile Arg Thr
385                 390                 395

<210> 109
<211> 1257
<212> DNA
<213> Neisseria meningitidis

<220>
<221> CDS
<222> (1)..(1257)

<400> 109
atg aaa cag acc gtc ctc aaa aat aac ctg caa aac ctg ctt gaa agc 48
Met Lys Gln Thr Val Leu Lys Asn Asn Leu Gln Asn Leu Leu Glu Ser
1               5                   10                  15

gca gaa aat atc ctg ctg ctt caa ggc cct gtc ggc gat ttt ttt ctg 96
Ala Glu Asn Ile Leu Leu Leu Gln Gly Pro Val Gly Asp Phe Phe Leu
            20                  25                  30

cgc ctt gcc gac tgg ctg act gca aac ggc aaa acc gta cat aaa ttc 144

Arg Leu Ala Asp Trp Leu Thr Ala Asn Gly Lys Thr Val His Lys Phe
        35                  40                  45

aac ttt aat gca ggc gac gac tat ttt tat ccg ccc act caa gcg cat 192
Asn Phe Asn Ala Gly Asp Asp Tyr Phe Tyr Pro Pro Thr Gln Ala His
    50                  55                  60

acc gtt gtt ttt aac gac aac tac gat gcc ttt cct gag ttt ttg caa 240
Thr Val Val Phe Asn Asp Asn Tyr Asp Ala Phe Pro Glu Phe Leu Gln
65                  70                  75                  80

gaa tac atc act caa cat cac atc cag gcc gtt gtc tgc ttt ggc gac 288
Glu Tyr Ile Thr Gln His His Ile Gln Ala Val Val Cys Phe Gly Asp
                85                  90                  95

aca cgc cct tat cac gtc att gca aaa cgc att gca aac gaa aac caa 336
Thr Arg Pro Tyr His Val Ile Ala Lys Arg Ile Ala Asn Glu Asn Gln
            100                 105                 110

gcc agt ttc tgg gcg ttt gaa gaa ggc tat ttc cgc ccc tac tac atc 384
Ala Ser Phe Trp Ala Phe Glu Glu Gly Tyr Phe Arg Pro Tyr Tyr Ile
        115                 120                 125

acc tta gaa aaa gac ggc gtc aac gca ttt tcc ccg ttg ccg cgc cgt 432
Thr Leu Glu Lys Asp Gly Val Asn Ala Phe Ser Pro Leu Pro Arg Arg
    130                 135                 140

gcc gac ttt ttt ctt gaa caa ttc cct aag ctt gcc cag caa gaa tat 480
Ala Asp Phe Phe Leu Glu Gln Phe Pro Lys Leu Ala Gln Gln Glu Tyr
145                 150                 155                 160

aaa gcg cca acg ccg gta cac ggc ggt ttt acg ccc atg gca aaa aac 528
Lys Ala Pro Thr Pro Val His Gly Gly Phe Thr Pro Met Ala Lys Asn
                165                 170                 175

gct atc cgt tac tat atc gag ttg ttc cgc aat cta cgc aaa tac ccc 576
Ala Ile Arg Tyr Tyr Ile Glu Leu Phe Arg Asn Leu Arg Lys Tyr Pro
            180                 185                 190

gac tac atc cac cac cgc gca ccc aat gcc ggc cat tac ctc aaa ccg 624
Asp Tyr Ile His His Arg Ala Pro Asn Ala Gly His Tyr Leu Lys Pro
        195                 200                 205

tgg tcg ctc tcc atc ctc aag cgt ttg aac tac tat att gaa gac atc 672
Trp Ser Leu Ser Ile Leu Lys Arg Leu Asn Tyr Tyr Ile Glu Asp Ile
    210                 215                 220

caa atc gcc aaa cgt gtg gaa gca ggc aaa tac ggc aag ttt ttt att 720

Gln Ile Ala Lys Arg Val Glu Ala Gly Lys Tyr Gly Lys Phe Phe Ile
225                 230                 235                 240

gtt ccc ttg cag gta ttc aac gac agc caa gtc cgt atc cat tgc gac     768
Val Pro Leu Gln Val Phe Asn Asp Ser Gln Val Arg Ile His Cys Asp
                245                 250                 255

ttt ccc agc gtc cgc agc ttc ctg ctc cat gtt ttg agt tca ttt gcc     816
Phe Pro Ser Val Arg Ser Phe Leu Leu His Val Leu Ser Ser Phe Ala
            260                 265                 270

gaa cac gcg cct gcc gat acc aac atc atc atc aag cat cat cct atg     864
Glu His Ala Pro Ala Asp Thr Asn Ile Ile Ile Lys His His Pro Met
        275                 280                 285

gac cgc ggc ttt atc gac tac tgg cgc gac att aaa cgc ttt atc aaa     912
Asp Arg Gly Phe Ile Asp Tyr Trp Arg Asp Ile Lys Arg Phe Ile Lys
    290                 295                 300

gaa cac ccc gaa ctc aaa ggc cgt gtg att tat gtc cat gat gtc ccc     960
Glu His Pro Glu Leu Lys Gly Arg Val Ile Tyr Val His Asp Val Pro
305                 310                 315                 320

ctg ccc gtt ttc ctg cgc cac ggt ctc ggc atg gtt acc atc aac agc     1008
Leu Pro Val Phe Leu Arg His Gly Leu Gly Met Val Thr Ile Asn Ser
                325                 330                 335

acc agc ggc ctg tcc gga ctg att cac aat atg cca gtt aag gtt ctc     1056
Thr Ser Gly Leu Ser Gly Leu Ile His Asn Met Pro Val Lys Val Leu
            340                 345                 350

ggc cgt gcc tat tat gat att ccc ggc att act gac caa aat acc ttg     1104
Gly Arg Ala Tyr Tyr Asp Ile Pro Gly Ile Thr Asp Gln Asn Thr Leu
        355                 360                 365

gca gaa ttt tgg aat cat ccg aca ccg cct gac aaa gag ctg ttc cat     1152
Ala Glu Phe Trp Asn His Pro Thr Pro Pro Asp Lys Glu Leu Phe His
    370                 375                 380

gcc tac cga atg tac cac ctc aac gtg acc caa att aac ggc aac ttc     1200
Ala Tyr Arg Met Tyr His Leu Asn Val Thr Gln Ile Asn Gly Asn Phe
385                 390                 395                 400

tac agt cag gtc ttt ttt cct aac aaa aac aca tcc gac tct tcc aca     1248
Tyr Ser Gln Val Phe Phe Pro Asn Lys Asn Thr Ser Asp Ser Ser Thr
                405                 410                 415

cca aca acc                                                         1257

Pro Thr Thr

<210> 110
<211> 419
<212> PRT
<213> Neisseria meningitidis

<400> 110
Met Lys Gln Thr Val Leu Lys Asn Asn Leu Gln Asn Leu Leu Glu Ser
1               5                   10                  15

Ala Glu Asn Ile Leu Leu Leu Gln Gly Pro Val Gly Asp Phe Phe Leu
            20                  25                  30

Arg Leu Ala Asp Trp Leu Thr Ala Asn Gly Lys Thr Val His Lys Phe
        35                  40                  45

Asn Phe Asn Ala Gly Asp Asp Tyr Phe Tyr Pro Pro Thr Gln Ala His
    50                  55                  60

Thr Val Val Phe Asn Asp Asn Tyr Asp Ala Phe Pro Glu Phe Leu Gln
65                  70                  75                  80

Glu Tyr Ile Thr Gln His His Ile Gln Ala Val Val Cys Phe Gly Asp
                85                  90                  95

Thr Arg Pro Tyr His Val Ile Ala Lys Arg Ile Ala Asn Glu Asn Gln
            100                 105                 110

Ala Ser Phe Trp Ala Phe Glu Glu Gly Tyr Phe Arg Pro Tyr Tyr Ile
        115                 120                 125

Thr Leu Glu Lys Asp Gly Val Asn Ala Phe Ser Pro Leu Pro Arg Arg
    130                 135                 140

Ala Asp Phe Phe Leu Glu Gln Phe Pro Lys Leu Ala Gln Gln Glu Tyr
145                 150                 155                 160

Lys Ala Pro Thr Pro Val His Gly Gly Phe Thr Pro Met Ala Lys Asn
                165                 170                 175

Ala Ile Arg Tyr Tyr Ile Glu Leu Phe Arg Asn Leu Arg Lys Tyr Pro
            180                 185                 190

Asp Tyr Ile His His Arg Ala Pro Asn Ala Gly His Tyr Leu Lys Pro
        195                 200                 205

Trp Ser Leu Ser Ile Leu Lys Arg Leu Asn Tyr Tyr Ile Glu Asp Ile
    210                 215                 220

Gln Ile Ala Lys Arg Val Glu Ala Gly Lys Tyr Gly Lys Phe Phe Ile
225                 230                 235                 240

Val Pro Leu Gln Val Phe Asn Asp Ser Gln Val Arg Ile His Cys Asp
                245                 250                 255

Phe Pro Ser Val Arg Ser Phe Leu Leu His Val Leu Ser Ser Phe Ala
            260                 265                 270

Glu His Ala Pro Ala Asp Thr Asn Ile Ile Ile Lys His His Pro Met
        275                 280                 285

Asp Arg Gly Phe Ile Asp Tyr Trp Arg Asp Ile Lys Arg Phe Ile Lys
    290                 295                 300

Glu His Pro Glu Leu Lys Gly Arg Val Ile Tyr Val His Asp Val Pro
305                 310                 315                 320

Leu Pro Val Phe Leu Arg His Gly Leu Gly Met Val Thr Ile Asn Ser
                325                 330                 335

Thr Ser Gly Leu Ser Gly Leu Ile His Asn Met Pro Val Lys Val Leu
            340                 345                 350

Gly Arg Ala Tyr Tyr Asp Ile Pro Gly Ile Thr Asp Gln Asn Thr Leu
        355                 360                 365

Ala Glu Phe Trp Asn His Pro Thr Pro Pro Asp Lys Glu Leu Phe His
    370                 375                 380

Ala Tyr Arg Met Tyr His Leu Asn Val Thr Gln Ile Asn Gly Asn Phe
385                 390                 395                 400

Tyr Ser Gln Val Phe Phe Pro Asn Lys Asn Thr Ser Asp Ser Ser Thr
                405                 410                 415

Pro Thr Thr

<210> 111
<211> 1674
<212> DNA
<213> Neisseria meningitidis

<220>
<221> CDS
<222> (1)..(1674)

<400> 111
atg agt ttc aaa acc gat gcc gaa atc gcc caa tcc tcc acc atg cgc     48
Met Ser Phe Lys Thr Asp Ala Glu Ile Ala Gln Ser Ser Thr Met Arg
1               5                   10                  15

ccg att ggc gaa att gcc gcc aag ctg ggt ttg aac gtt gac aac att     96
Pro Ile Gly Glu Ile Ala Ala Lys Leu Gly Leu Asn Val Asp Asn Ile
            20                  25                  30

gag cct tac ggt cat tac aaa gcc aaa atc aat cct gcc gaa gcg ttc     144
Glu Pro Tyr Gly His Tyr Lys Ala Lys Ile Asn Pro Ala Glu Ala Phe
        35                  40                  45

aaa ctg ccg caa aaa cag ggc agg ctg att ttg gtt acc gcc atc aac     192
Lys Leu Pro Gln Lys Gln Gly Arg Leu Ile Leu Val Thr Ala Ile Asn
    50                  55                  60

ccg act ccg gcg ggc gaa ggt aaa acc acc gta acc atc ggt ttg gcg     240
Pro Thr Pro Ala Gly Glu Gly Lys Thr Thr Val Thr Ile Gly Leu Ala
65                  70                  75                  80

gac gca ttg cgc cat atc ggc aaa gac tct gtg att gct ttg cgc gag     288
Asp Ala Leu Arg His Ile Gly Lys Asp Ser Val Ile Ala Leu Arg Glu
                85                  90                  95

cct tct ttg ggt ccg gtg ttc ggc gtg aaa ggc ggc gcg gca ggc ggc     336
Pro Ser Leu Gly Pro Val Phe Gly Val Lys Gly Gly Ala Ala Gly Gly
            100                 105                 110

ggc tat gcc caa gtt ttg ccg atg gaa gac atc aac ctg cac ttc acc     384
Gly Tyr Ala Gln Val Leu Pro Met Glu Asp Ile Asn Leu His Phe Thr
        115                 120                 125

gga gat ttt cac gcc atc ggt gcg gca aat aat ctg ctt gcc gcg atg     432
Gly Asp Phe His Ala Ile Gly Ala Ala Asn Asn Leu Leu Ala Ala Met
    130                 135                 140

ctc gac aac cat atc tac caa ggc aac gag ttg aac atc gac ccc aaa     480
Leu Asp Asn His Ile Tyr Gln Gly Asn Glu Leu Asn Ile Asp Pro Lys
145                 150                 155                 160

cgc gtg ctg tgg cgg cgc gtg gtc gat atg aac gac cgc cag ttg cgc     528
Arg Val Leu Trp Arg Arg Val Val Asp Met Asn Asp Arg Gln Leu Arg

                165                 170                 175

aac atc atc gac ggc atg ggc aag cct gtt gac ggc gtg atg cgt cct 576
Asn Ile Ile Asp Gly Met Gly Lys Pro Val Asp Gly Val Met Arg Pro
            180                 185                 190

gac ggt ttc gat att acc gtt gct tcc gaa gtg atg gcg gta ttc tgt 624
Asp Gly Phe Asp Ile Thr Val Ala Ser Glu Val Met Ala Val Phe Cys
        195                 200                 205

ctt gcc aaa gac atc agc gat ttg aaa gag cgt ttg ggc aac atc ctt 672
Leu Ala Lys Asp Ile Ser Asp Leu Lys Glu Arg Leu Gly Asn Ile Leu
    210                 215                 220

gtc gcc tac gcc aaa gac ggc agc ccc gtt tac gcc aaa gat ttg aaa 720
Val Ala Tyr Ala Lys Asp Gly Ser Pro Val Tyr Ala Lys Asp Leu Lys
225                 230                 235                 240

gcg aat ggc gcg atg gcg gca ttg ctt aaa gat gcg att aag ccc aac 768
Ala Asn Gly Ala Met Ala Ala Leu Leu Lys Asp Ala Ile Lys Pro Asn
                245                 250                 255

ttg gtg caa acc atc gaa ggc acg ccc gcc ttc gta cac ggc ggc ccg 816
Leu Val Gln Thr Ile Glu Gly Thr Pro Ala Phe Val His Gly Gly Pro
            260                 265                 270

ttc gcc aac atc gcc cac ggc tgc aac tcc gta acc gca acc cgt ctg 864
Phe Ala Asn Ile Ala His Gly Cys Asn Ser Val Thr Ala Thr Arg Leu
        275                 280                 285

gcg aaa cac ctt gcc gat tac gcc gta acc gaa gca ggc ttc ggc gcg 912
Ala Lys His Leu Ala Asp Tyr Ala Val Thr Glu Ala Gly Phe Gly Ala
    290                 295                 300

gac ttg ggc gcg gaa aaa ttc tgc gac atc aaa tgc cgc ctt gcc ggt 960
Asp Leu Gly Ala Glu Lys Phe Cys Asp Ile Lys Cys Arg Leu Ala Gly
305                 310                 315                 320

ttg aaa cct gat gcg gct gtt gtc gtg gcg act gtc cgc gcg ttg aaa 1008
Leu Lys Pro Asp Ala Ala Val Val Val Ala Thr Val Arg Ala Leu Lys
                325                 330                 335

tat aac ggc ggc gtg gaa cgc gcc aac ctc ggc gaa gaa aat tta gac 1056
Tyr Asn Gly Gly Val Glu Arg Ala Asn Leu Gly Glu Glu Asn Leu Asp
            340                 345                 350

gct ttg gaa aaa ggt ttg ccc aac ctg ctg aaa cac att tcc aac ctg 1104
Ala Leu Glu Lys Gly Leu Pro Asn Leu Leu Lys His Ile Ser Asn Leu

355                 360                 365

aaa aac gta ttc gga ctg ccc gtc gtc gtt gcg ctc aac cgc ttc gtg     1152
Lys Asn Val Phe Gly Leu Pro Val Val Val Ala Leu Asn Arg Phe Val
    370                 375                 380

tcc gac tcc gat gcc gag ttg gcg atg att gaa aaa gcc tgt gcc gaa     1200
Ser Asp Ser Asp Ala Glu Leu Ala Met Ile Glu Lys Ala Cys Ala Glu
385                 390                 395                 400

cac ggc gtt gaa gtt tcc ctg acc gaa gtg tgg ggc aaa ggt ggt gcg     1248
His Gly Val Glu Val Ser Leu Thr Glu Val Trp Gly Lys Gly Gly Ala
                405                 410                 415

ggc ggc gcg gat ttg gcg cgc aaa gtc gtc aac gcc att gaa agt caa     1296
Gly Gly Ala Asp Leu Ala Arg Lys Val Val Asn Ala Ile Glu Ser Gln
            420                 425                 430

acc aat aac ttc ggt ttc gcc tac gat gtc gag ttg ggc atc aaa gac     1344
Thr Asn Asn Phe Gly Phe Ala Tyr Asp Val Glu Leu Gly Ile Lys Asp
        435                 440                 445

aaa atc cgt gcg att gcc caa aaa gtg tac ggc gcg gaa gat gtt gat     1392
Lys Ile Arg Ala Ile Ala Gln Lys Val Tyr Gly Ala Glu Asp Val Asp
    450                 455                 460

ttc agc gcg gaa gcg tct gcc gaa atc gct tca ctg gaa aaa ctg ggc     1440
Phe Ser Ala Glu Ala Ser Ala Glu Ile Ala Ser Leu Glu Lys Leu Gly
465                 470                 475                 480

ttg gac aaa atg ccg atc tgc atg gcg aaa acc caa tac tct ttg agc     1488
Leu Asp Lys Met Pro Ile Cys Met Ala Lys Thr Gln Tyr Ser Leu Ser
                485                 490                 495

gac aac gcc aaa ctg ttg ggc tgc ccc gaa gac ttc cgc atc gcc gtg     1536
Asp Asn Ala Lys Leu Leu Gly Cys Pro Glu Asp Phe Arg Ile Ala Val
            500                 505                 510

cgc ggc atc acc gtt tcc gca ggc gca ggt ttc atc gtc gcc ctg tgc     1584
Arg Gly Ile Thr Val Ser Ala Gly Ala Gly Phe Ile Val Ala Leu Cys
        515                 520                 525

ggc aac atg atg aaa atg ccc ggc ctg ccc aaa gtt ccg gct gcc gag     1632
Gly Asn Met Met Lys Met Pro Gly Leu Pro Lys Val Pro Ala Ala Glu
    530                 535                 540

aaa atc gat gtg gac gca gaa ggc gtg att cac ggc ttg ttc             1674
Lys Ile Asp Val Asp Ala Glu Gly Val Ile His Gly Leu Phe

545                550                555

<210> 112
<211> 558
<212> PRT
<213> Neisseria meningitidis

<400> 112
Met Ser Phe Lys Thr Asp Ala Glu Ile Ala Gln Ser Ser Thr Met Arg
1               5                   10                  15

Pro Ile Gly Glu Ile Ala Ala Lys Leu Gly Leu Asn Val Asp Asn Ile
            20                  25                  30

Glu Pro Tyr Gly His Tyr Lys Ala Lys Ile Asn Pro Ala Glu Ala Phe
        35                  40                  45

Lys Leu Pro Gln Lys Gln Gly Arg Leu Ile Leu Val Thr Ala Ile Asn
    50                  55                  60

Pro Thr Pro Ala Gly Glu Gly Lys Thr Thr Val Thr Ile Gly Leu Ala
65                  70                  75                  80

Asp Ala Leu Arg His Ile Gly Lys Asp Ser Val Ile Ala Leu Arg Glu
                85                  90                  95

Pro Ser Leu Gly Pro Val Phe Gly Val Lys Gly Gly Ala Ala Gly Gly
            100                 105                 110

Gly Tyr Ala Gln Val Leu Pro Met Glu Asp Ile Asn Leu His Phe Thr
        115                 120                 125

Gly Asp Phe His Ala Ile Gly Ala Ala Asn Asn Leu Leu Ala Ala Met
    130                 135                 140

Leu Asp Asn His Ile Tyr Gln Gly Asn Glu Leu Asn Ile Asp Pro Lys
145                 150                 155                 160

Arg Val Leu Trp Arg Arg Val Val Asp Met Asn Asp Arg Gln Leu Arg
                165                 170                 175

Asn Ile Ile Asp Gly Met Gly Lys Pro Val Asp Gly Val Met Arg Pro
            180                 185                 190

Asp Gly Phe Asp Ile Thr Val Ala Ser Glu Val Met Ala Val Phe Cys
        195                 200                 205

Leu Ala Lys Asp Ile Ser Asp Leu Lys Glu Arg Leu Gly Asn Ile Leu
    210                 215                 220

Val Ala Tyr Ala Lys Asp Gly Ser Pro Val Tyr Ala Lys Asp Leu Lys
225                 230                 235                 240

Ala Asn Gly Ala Met Ala Ala Leu Leu Lys Asp Ala Ile Lys Pro Asn
                245                 250                 255

Leu Val Gln Thr Ile Glu Gly Thr Pro Ala Phe Val His Gly Gly Pro
            260                 265                 270

Phe Ala Asn Ile Ala His Gly Cys Asn Ser Val Thr Ala Thr Arg Leu
        275                 280                 285

Ala Lys His Leu Ala Asp Tyr Ala Val Thr Glu Ala Gly Phe Gly Ala
    290                 295                 300

Asp Leu Gly Ala Glu Lys Phe Cys Asp Ile Lys Cys Arg Leu Ala Gly
305                 310                 315                 320

Leu Lys Pro Asp Ala Ala Val Val Val Ala Thr Val Arg Ala Leu Lys
                325                 330                 335

Tyr Asn Gly Gly Val Glu Arg Ala Asn Leu Gly Glu Glu Asn Leu Asp
            340                 345                 350

Ala Leu Glu Lys Gly Leu Pro Asn Leu Leu Lys His Ile Ser Asn Leu
        355                 360                 365

Lys Asn Val Phe Gly Leu Pro Val Val Val Ala Leu Asn Arg Phe Val
    370                 375                 380

Ser Asp Ser Asp Ala Glu Leu Ala Met Ile Glu Lys Ala Cys Ala Glu
385                 390                 395                 400

His Gly Val Glu Val Ser Leu Thr Glu Val Trp Gly Lys Gly Gly Ala
                405                 410                 415

Gly Gly Ala Asp Leu Ala Arg Lys Val Val Asn Ala Ile Glu Ser Gln
            420                 425                 430

Thr Asn Asn Phe Gly Phe Ala Tyr Asp Val Glu Leu Gly Ile Lys Asp
        435                 440                 445

Lys Ile Arg Ala Ile Ala Gln Lys Val Tyr Gly Ala Glu Asp Val Asp
    450                 455                 460

Phe Ser Ala Glu Ala Ser Ala Glu Ile Ala Ser Leu Glu Lys Leu Gly
465                 470                 475                 480

Leu Asp Lys Met Pro Ile Cys Met Ala Lys Thr Gln Tyr Ser Leu Ser
                485                 490                 495

Asp Asn Ala Lys Leu Leu Gly Cys Pro Glu Asp Phe Arg Ile Ala Val
            500                 505                 510

Arg Gly Ile Thr Val Ser Ala Gly Ala Gly Phe Ile Val Ala Leu Cys
        515                 520                 525

Gly Asn Met Met Lys Met Pro Gly Leu Pro Lys Val Pro Ala Ala Glu
    530                 535                 540

Lys Ile Asp Val Asp Ala Glu Gly Val Ile His Gly Leu Phe
545                 550                 555

<210> 113
<211> 475
<212> DNA
<213> Neisseria meningitidis

<220>
<221> CDS
<222> (1)..(474)

<400> 113
atg gaa cac cta ttt gag gaa tgg ttg ccc gac ctg ccc gcc gac gtt    48
Met Glu His Leu Phe Glu Glu Trp Leu Pro Asp Leu Pro Ala Asp Val
  1               5                  10                  15

tca gac ggc atc ggc ctg ccg atg agc cgg tta ttg aaa gcc cgg tcg    96
Ser Asp Gly Ile Gly Leu Pro Met Ser Arg Leu Leu Lys Ala Arg Ser
             20                  25                  30

ctg act gcc gca ttg cgc gcc ttg ccg cat ctg ttt tcg gta gaa ctg    144
Leu Thr Ala Ala Leu Arg Ala Leu Pro His Leu Phe Ser Val Glu Leu
         35                  40                  45

ctg aaa ctg ggc gaa ttg gaa acg gaa tgc gga ggg cgg ctg gtg cgc    192
Leu Lys Leu Gly Glu Leu Glu Thr Glu Cys Gly Gly Arg Leu Val Arg
     50                  55                  60

gaa gtt ttg ttg aag ctg gac ggt atc cct gtt gtc gcg gca agg agc    240
Glu Val Leu Leu Lys Leu Asp Gly Ile Pro Val Val Ala Ala Arg Ser

65                  70                  75                  80

gaa tgc cgt atc ggt tcg gcg ttt tgg caa aac att ttg gac tgc ggc      288
Glu Cys Arg Ile Gly Ser Ala Phe Trp Gln Asn Ile Leu Asp Cys Gly
                85                  90                  95

acg cgt cct ttg ggc gaa cgt ctg ttt caa gcc gat ttg gaa ggg gcg      336
Thr Arg Pro Leu Gly Glu Arg Leu Phe Gln Ala Asp Leu Glu Gly Ala
            100                 105                 110

cgt tcg gcg ttt gag ttt gcc gtt ttc ggc gaa gga tgc gga cgg tac      384
Arg Ser Ala Phe Glu Phe Ala Val Phe Gly Glu Gly Cys Gly Arg Tyr
        115                 120                 125

ttt gcc gct cgg cgt tcg cgg ttt tcc cat cac ggc gag gaa atg ctg      432
Phe Ala Ala Arg Arg Ser Arg Phe Ser His His Gly Glu Glu Met Leu
    130                 135                 140

ctg acc gag tat ttt ctg ccc gaa ctg aaa cgc ttt atc gga t            475
Leu Thr Glu Tyr Phe Leu Pro Glu Leu Lys Arg Phe Ile Gly
145                 150                 155

<210> 114
<211> 158
<212> PRT
<213> Neisseria meningitidis

<400> 114
Met Glu His Leu Phe Glu Glu Trp Leu Pro Asp Leu Pro Ala Asp Val
1               5                   10                  15

Ser Asp Gly Ile Gly Leu Pro Met Ser Arg Leu Leu Lys Ala Arg Ser
            20                  25                  30

Leu Thr Ala Ala Leu Arg Ala Leu Pro His Leu Phe Ser Val Glu Leu
        35                  40                  45

Leu Lys Leu Gly Glu Leu Glu Thr Glu Cys Gly Gly Arg Leu Val Arg
    50                  55                  60

Glu Val Leu Leu Lys Leu Asp Gly Ile Pro Val Val Ala Ala Arg Ser
65                  70                  75                  80

Glu Cys Arg Ile Gly Ser Ala Phe Trp Gln Asn Ile Leu Asp Cys Gly
                85                  90                  95

Thr Arg Pro Leu Gly Glu Arg Leu Phe Gln Ala Asp Leu Glu Gly Ala

            100                 105                 110

Arg Ser Ala Phe Glu Phe Ala Val Phe Gly Glu Gly Cys Gly Arg Tyr
        115                 120                 125

Phe Ala Ala Arg Arg Ser Arg Phe Ser His His Gly Glu Glu Met Leu
    130                 135                 140

Leu Thr Glu Tyr Phe Leu Pro Glu Leu Lys Arg Phe Ile Gly
145                 150                 155

<210> 115
<211> 1476
<212> DNA
<213> Neisseria meningitidis

<220>
<221> CDS
<222> (1)..(1476)

<400> 115
atg aaa tac aaa gac ctg cgc gac ttc atc gcc atg ctc gag cag cag 48
Met Lys Tyr Lys Asp Leu Arg Asp Phe Ile Ala Met Leu Glu Gln Gln
1               5                   10                  15

ggc aaa ctc aaa cgc atc gcg cac ccc gtt tcc ccg cat ttg gaa atg 96
Gly Lys Leu Lys Arg Ile Ala His Pro Val Ser Pro His Leu Glu Met
            20                  25                  30

acc gaa atc gcc gac cgc gtg ctg cgc gcc gaa ggg ccg gcg ttg ttg 144
Thr Glu Ile Ala Asp Arg Val Leu Arg Ala Glu Gly Pro Ala Leu Leu
        35                  40                  45

ttt gaa cac cca gtt aag ccc gac ggt acg cgc tat gat tat ccc gtg 192
Phe Glu His Pro Val Lys Pro Asp Gly Thr Arg Tyr Asp Tyr Pro Val
    50                  55                  60

ttg gca aac ctg ttc ggc acg ccc gaa cgt gtg gcg atg ggc atg ggc 240
Leu Ala Asn Leu Phe Gly Thr Pro Glu Arg Val Ala Met Gly Met Gly
65                  70                  75                  80

gcg gac agc gtg tcc aag ctg cgc gaa atc ggg cag acg ctg gcg tat 288
Ala Asp Ser Val Ser Lys Leu Arg Glu Ile Gly Gln Thr Leu Ala Tyr
                85                  90                  95

ttg aaa gaa ccc gaa ccg ccc aaa ggc att aaa gac gcg ttt tcc aaa 336

Leu Lys Glu Pro Glu Pro Pro Lys Gly Ile Lys Asp Ala Phe Ser Lys
            100                 105                 110

ctg ccg ctc ttg aaa gac att tgg agc atg gcg ccg aac gtg gtg aaa 384
Leu Pro Leu Leu Lys Asp Ile Trp Ser Met Ala Pro Asn Val Val Lys
        115                 120                 125

aat gcg ccg tgt cag gaa atc gta tgg gaa ggc gaa gac gtt gat ttg 432
Asn Ala Pro Cys Gln Glu Ile Val Trp Glu Gly Glu Asp Val Asp Leu
    130                 135                 140

tat caa ctt ccg att cag cat tgc tgg ccg gaa gac gtt gcg ccg ctg 480
Tyr Gln Leu Pro Ile Gln His Cys Trp Pro Glu Asp Val Ala Pro Leu
145                 150                 155                 160

gta acg tgg ggc ttg acc gtc acg cgc ggg ccg cac aaa aaa cgc caa 528
Val Thr Trp Gly Leu Thr Val Thr Arg Gly Pro His Lys Lys Arg Gln
                165                 170                 175

aat ctc ggc att tac cgc caa caa tta atc ggc ata aac aag ctg att 576
Asn Leu Gly Ile Tyr Arg Gln Gln Leu Ile Gly Ile Asn Lys Leu Ile
            180                 185                 190

atg cgc tgg ctg tcg cat cgc ggc ggc gcg ctg gat tat cag gaa ttc 624
Met Arg Trp Leu Ser His Arg Gly Gly Ala Leu Asp Tyr Gln Glu Phe
        195                 200                 205

cgc aaa ctc aat ccc gat acg ccg tat ccc gtc gcc gtc gtg ctc ggc 672
Arg Lys Leu Asn Pro Asp Thr Pro Tyr Pro Val Ala Val Val Leu Gly
    210                 215                 220

tgc gac cct gcc acc att ttg ggc gcg gta acg ccc gtt ccc gat acc 720
Cys Asp Pro Ala Thr Ile Leu Gly Ala Val Thr Pro Val Pro Asp Thr
225                 230                 235                 240

ttg agc gaa tac cag ttt gcc gga ctg ctg cgc ggt tcg cgg acg gaa 768
Leu Ser Glu Tyr Gln Phe Ala Gly Leu Leu Arg Gly Ser Arg Thr Glu
                245                 250                 255

ctg gtg aaa tgt atc ggc aac gat ttg caa gtg cct gcc cgc gca gaa 816
Leu Val Lys Cys Ile Gly Asn Asp Leu Gln Val Pro Ala Arg Ala Glu
            260                 265                 270

atc gtg ttg gaa ggc gtc atc cat cca aac gaa acc gcg ttg gaa ggt 864
Ile Val Leu Glu Gly Val Ile His Pro Asn Glu Thr Ala Leu Glu Gly
        275                 280                 285

cca tac ggc gac cac acg ggc tat tac aac gag cag gac cat ttc ccc 912

Pro Tyr Gly Asp His Thr Gly Tyr Tyr Asn Glu Gln Asp His Phe Pro
    290                 295                 300

gtg ttt acg gtc gaa cgc atc acc atg cgc gaa aac ccg att tac cac     960
Val Phe Thr Val Glu Arg Ile Thr Met Arg Glu Asn Pro Ile Tyr His
305                 310                 315                 320

tct acc tac aca ggc aaa ccg ccc gat gaa ccc gcc gtt ttg ggc gtg     1008
Ser Thr Tyr Thr Gly Lys Pro Pro Asp Glu Pro Ala Val Leu Gly Val
                325                 330                 335

gcg ttg aac gaa gtg ttc gta ccg ctt ttg caa aag cag ttc ccc gaa     1056
Ala Leu Asn Glu Val Phe Val Pro Leu Leu Gln Lys Gln Phe Pro Glu
            340                 345                 350

atc acc gat ttt tac ctg ccg ccc gaa ggc tgc tcc tac cgc atg gcg     1104
Ile Thr Asp Phe Tyr Leu Pro Pro Glu Gly Cys Ser Tyr Arg Met Ala
        355                 360                 365

gtg gtg agc atg aaa aaa cag tac gcc gga cac gcc aag cgc gtg atg     1152
Val Val Ser Met Lys Lys Gln Tyr Ala Gly His Ala Lys Arg Val Met
    370                 375                 380

atg ggc tgc tgg tcg ttc ctg cgc cag ttt atg tac acc aaa ttc atc     1200
Met Gly Cys Trp Ser Phe Leu Arg Gln Phe Met Tyr Thr Lys Phe Ile
385                 390                 395                 400

att gtg gtg gat gac gat gtg gat gtg cgc gac tgg aaa gaa gtc atc     1248
Ile Val Val Asp Asp Asp Val Asp Val Arg Asp Trp Lys Glu Val Ile
                405                 410                 415

tgg gcg gta acc acg cgc atg gac ccc gtg cgc gat acc gtt ttg atg     1296
Trp Ala Val Thr Thr Arg Met Asp Pro Val Arg Asp Thr Val Leu Met
            420                 425                 430

gaa aac acg ccc atc gac tac ctc gac ttc gcc agc ccc gtc agc gga     1344
Glu Asn Thr Pro Ile Asp Tyr Leu Asp Phe Ala Ser Pro Val Ser Gly
        435                 440                 445

ctt ggc ggc aaa atg ggt ttg gat gcg acc aac aag tgg ccg ggc gaa     1392
Leu Gly Gly Lys Met Gly Leu Asp Ala Thr Asn Lys Trp Pro Gly Glu
    450                 455                 460

acc gac cgc gaa tgg gga cgg gtg att aaa aaa gac cct gcg gtt acg     1440
Thr Asp Arg Glu Trp Gly Arg Val Ile Lys Lys Asp Pro Ala Val Thr
465                 470                 475                 480

gct aag att gat gag att tgg gag gaa ttg ggg ttg                     1476

Ala Lys Ile Asp Glu Ile Trp Glu Glu Leu Gly Leu
                485                 490

<210> 116
<211> 492
<212> PRT
<213> Neisseria meningitidis

<400> 116
Met Lys Tyr Lys Asp Leu Arg Asp Phe Ile Ala Met Leu Glu Gln Gln
1               5                   10                  15

Gly Lys Leu Lys Arg Ile Ala His Pro Val Ser Pro His Leu Glu Met
            20                  25                  30

Thr Glu Ile Ala Asp Arg Val Leu Arg Ala Glu Gly Pro Ala Leu Leu
        35                  40                  45

Phe Glu His Pro Val Lys Pro Asp Gly Thr Arg Tyr Asp Tyr Pro Val
    50                  55                  60

Leu Ala Asn Leu Phe Gly Thr Pro Glu Arg Val Ala Met Gly Met Gly
65                  70                  75                  80

Ala Asp Ser Val Ser Lys Leu Arg Glu Ile Gly Gln Thr Leu Ala Tyr
                85                  90                  95

Leu Lys Glu Pro Glu Pro Pro Lys Gly Ile Lys Asp Ala Phe Ser Lys
            100                 105                 110

Leu Pro Leu Leu Lys Asp Ile Trp Ser Met Ala Pro Asn Val Val Lys
        115                 120                 125

Asn Ala Pro Cys Gln Glu Ile Val Trp Glu Gly Glu Asp Val Asp Leu
    130                 135                 140

Tyr Gln Leu Pro Ile Gln His Cys Trp Pro Glu Asp Val Ala Pro Leu
145                 150                 155                 160

Val Thr Trp Gly Leu Thr Val Thr Arg Gly Pro His Lys Lys Arg Gln
                165                 170                 175

Asn Leu Gly Ile Tyr Arg Gln Gln Leu Ile Gly Ile Asn Lys Leu Ile
            180                 185                 190

Met Arg Trp Leu Ser His Arg Gly Gly Ala Leu Asp Tyr Gln Glu Phe
        195                 200                 205

Arg Lys Leu Asn Pro Asp Thr Pro Tyr Pro Val Ala Val Val Leu Gly
    210                 215                 220

Cys Asp Pro Ala Thr Ile Leu Gly Ala Val Thr Pro Val Pro Asp Thr
225                 230                 235                 240

Leu Ser Glu Tyr Gln Phe Ala Gly Leu Leu Arg Gly Ser Arg Thr Glu
                245                 250                 255

Leu Val Lys Cys Ile Gly Asn Asp Leu Gln Val Pro Ala Arg Ala Glu
            260                 265                 270

Ile Val Leu Glu Gly Val Ile His Pro Asn Glu Thr Ala Leu Glu Gly
        275                 280                 285

Pro Tyr Gly Asp His Thr Gly Tyr Tyr Asn Glu Gln Asp His Phe Pro
    290                 295                 300

Val Phe Thr Val Glu Arg Ile Thr Met Arg Glu Asn Pro Ile Tyr His
305                 310                 315                 320

Ser Thr Tyr Thr Gly Lys Pro Pro Asp Glu Pro Ala Val Leu Gly Val
                325                 330                 335

Ala Leu Asn Glu Val Phe Val Pro Leu Leu Gln Lys Gln Phe Pro Glu
            340                 345                 350

Ile Thr Asp Phe Tyr Leu Pro Pro Glu Gly Cys Ser Tyr Arg Met Ala
        355                 360                 365

Val Val Ser Met Lys Lys Gln Tyr Ala Gly His Ala Lys Arg Val Met
    370                 375                 380

Met Gly Cys Trp Ser Phe Leu Arg Gln Phe Met Tyr Thr Lys Phe Ile
385                 390                 395                 400

Ile Val Val Asp Asp Asp Val Asp Val Arg Asp Trp Lys Glu Val Ile
                405                 410                 415

Trp Ala Val Thr Thr Arg Met Asp Pro Val Arg Asp Thr Val Leu Met
            420                 425                 430

Glu Asn Thr Pro Ile Asp Tyr Leu Asp Phe Ala Ser Pro Val Ser Gly
        435                 440                 445

Leu Gly Gly Lys Met Gly Leu Asp Ala Thr Asn Lys Trp Pro Gly Glu
    450                 455                 460

Thr Asp Arg Glu Trp Gly Arg Val Ile Lys Lys Asp Pro Ala Val Thr
465                 470                 475                 480

Ala Lys Ile Asp Glu Ile Trp Glu Glu Leu Gly Leu
                485                 490

<210> 117
<211> 1515
<212> DNA
<213> Neisseria meningitidis

<220>
<221> CDS
<222> (1)..(1515)

<400> 117
atg ttg tat ttc aga tac ggt ttt ttg gtt gtt tgg tgt gcg gca ggt     48
Met Leu Tyr Phe Arg Tyr Gly Phe Leu Val Val Trp Cys Ala Ala Gly
1               5                   10                  15

gtt tct gcc gcc tat ggg gcg gat gcg ccc gcg att ttg gat gac aag     96
Val Ser Ala Ala Tyr Gly Ala Asp Ala Pro Ala Ile Leu Asp Asp Lys
            20                  25                  30

gca ttg ttg cag gtg cag cgg tcg gtg tcg gat aag tgg gcg gaa tcg     144
Ala Leu Leu Gln Val Gln Arg Ser Val Ser Asp Lys Trp Ala Glu Ser
        35                  40                  45

gat tgg aaa gtt gac aat gat gcc ccg cgc gtg gtt gac ggg gat ttt     192
Asp Trp Lys Val Asp Asn Asp Ala Pro Arg Val Val Asp Gly Asp Phe
    50                  55                  60

ttg ttg gcg cat ccg aaa atg ttg gaa cat agt ttg cgc gac gtg ctc     240
Leu Leu Ala His Pro Lys Met Leu Glu His Ser Leu Arg Asp Val Leu
65                  70                  75                  80

aac ggc aat cag gcg gat ttg atc gct tcg ttg gcg gat ttg tat gcc     288
Asn Gly Asn Gln Ala Asp Leu Ile Ala Ser Leu Ala Asp Leu Tyr Ala
                85                  90                  95

aag ctg ccg gat tat gac gcg gtt ttg tac ggc agg gcg cgg gct ttg     336
Lys Leu Pro Asp Tyr Asp Ala Val Leu Tyr Gly Arg Ala Arg Ala Leu
            100                 105                 110

ctg gcg aaa ttg gcg gga agg ccg gcg gag gcg gtg gcg cgg tat cgg     384

Leu Ala Lys Leu Ala Gly Arg Pro Ala Glu Ala Val Ala Arg Tyr Arg
      115                 120                 125

gaa ctg cac ggg gaa aat gcg gca gac gag cgg att ttg ctg gat ttg     432
Glu Leu His Gly Glu Asn Ala Ala Asp Glu Arg Ile Leu Leu Asp Leu
    130                 135                 140

gcg gcg gcg gag ttt gac gat ttc cgg ctg aag tcg gca gaa agg cat     480
Ala Ala Ala Glu Phe Asp Asp Phe Arg Leu Lys Ser Ala Glu Arg His
145                 150                 155                 160

ttt gcc gag gcg gaa aaa ttg gat ttg ccg gcg ccg gtt ttg gaa aat     528
Phe Ala Glu Ala Glu Lys Leu Asp Leu Pro Ala Pro Val Leu Glu Asn
                165                 170                 175

gtg ggg cgt ttt cgg aaa aaa gcg gag ggg ctg acg ggc tgg cgt ttt     576
Val Gly Arg Phe Arg Lys Lys Ala Glu Gly Leu Thr Gly Trp Arg Phe
            180                 185                 190

tcg ggc ggc atc agt ccg gcg gtc aat aga aat gcc aat aat gcc gcg     624
Ser Gly Gly Ile Ser Pro Ala Val Asn Arg Asn Ala Asn Asn Ala Ala
        195                 200                 205

ccg cag tat tgc cgg caa aac gga ggc cgg cag ata tgc agt gtc agc     672
Pro Gln Tyr Cys Arg Gln Asn Gly Gly Arg Gln Ile Cys Ser Val Ser
    210                 215                 220

cgg gcg gag cgg gcg gca ggc ttg aat tat gaa atc gag gcg gaa aaa     720
Arg Ala Glu Arg Ala Ala Gly Leu Asn Tyr Glu Ile Glu Ala Glu Lys
225                 230                 235                 240

ctg acg gcg ttg gca gat aat cat tat ttg ttg ttc cgt tcc aat atc     768
Leu Thr Ala Leu Ala Asp Asn His Tyr Leu Leu Phe Arg Ser Asn Ile
                245                 250                 255

ggc ggc acg agc tat tat ttc agt aaa aaa tca gct tat gac gac ggg     816
Gly Gly Thr Ser Tyr Tyr Phe Ser Lys Lys Ser Ala Tyr Asp Asp Gly
            260                 265                 270

ttc ggc aga gcg tat ttg ggt tgg cag tat aaa aat gca cgg cag acg     864
Phe Gly Arg Ala Tyr Leu Gly Trp Gln Tyr Lys Asn Ala Arg Gln Thr
        275                 280                 285

gcg ggg att ttg ccg ttt tat cag gtg cag ttg tcg ggc agc gac ggc     912
Ala Gly Ile Leu Pro Phe Tyr Gln Val Gln Leu Ser Gly Ser Asp Gly
    290                 295                 300

ttt gat gcg aaa aca aaa cgg gta aac aac cgc cgc ctg ccg ccg tat     960

Phe Asp Ala Lys Thr Lys Arg Val Asn Asn Arg Arg Leu Pro Pro Tyr
305                 310                 315                 320

atg ctg gcg cac gga gtc ggc gtg cag ttg tcc cat act tac cgc cca     1008
Met Leu Ala His Gly Val Gly Val Gln Leu Ser His Thr Tyr Arg Pro
                325                 330                 335

aac ccg gga tgg caa ttt tcg gtc gcg ctg gaa cat tac cgc caa cgc     1056
Asn Pro Gly Trp Gln Phe Ser Val Ala Leu Glu His Tyr Arg Gln Arg
            340                 345                 350

tac cgc gaa cag gat agg gcg gaa tac aat aac ggt cgg cag gac ggg     1104
Tyr Arg Glu Gln Asp Arg Ala Glu Tyr Asn Asn Gly Arg Gln Asp Gly
        355                 360                 365

ttt tat gtt tcg tcg gca aaa cgt ttg ggc gaa tcg gca act gtg ttc     1152
Phe Tyr Val Ser Ser Ala Lys Arg Leu Gly Glu Ser Ala Thr Val Phe
    370                 375                 380

ggc ggc tgg cag ttt gtg cgg ttt gtg ccg aaa cgc gaa acg gtg ggc     1200
Gly Gly Trp Gln Phe Val Arg Phe Val Pro Lys Arg Glu Thr Val Gly
385                 390                 395                 400

ggc gcg gtc aat aat gcc gcc tac cgg cgc aac ggt gtt tat gcc ggc     1248
Gly Ala Val Asn Asn Ala Ala Tyr Arg Arg Asn Gly Val Tyr Ala Gly
                405                 410                 415

tgg gcg cag gag tgg cgg cag ttg ggc ggt ttg aac agt cgg gtt tcc     1296
Trp Ala Gln Glu Trp Arg Gln Leu Gly Gly Leu Asn Ser Arg Val Ser
            420                 425                 430

gcg tct tat gcc cgc cgc aac tat aag ggc gtt gcg gct ttc tcg aca     1344
Ala Ser Tyr Ala Arg Arg Asn Tyr Lys Gly Val Ala Ala Phe Ser Thr
        435                 440                 445

gag gcg caa cgc aac cgc gaa tgg aat gtc tcg ctg gct ttg agc cac     1392
Glu Ala Gln Arg Asn Arg Glu Trp Asn Val Ser Leu Ala Leu Ser His
    450                 455                 460

gac aag ttg tcg tac aaa ggt atc gtg ccc gcg ttg aat tat cgt ttc     1440
Asp Lys Leu Ser Tyr Lys Gly Ile Val Pro Ala Leu Asn Tyr Arg Phe
465                 470                 475                 480

ggc agg acg gaa agt aat gtg ccg tat gcg aaa cgc cgc aac agc gag     1488
Gly Arg Thr Glu Ser Asn Val Pro Tyr Ala Lys Arg Arg Asn Ser Glu
                485                 490                 495

gtg ttt gtg tcg gcg gat tgg cgg ttt                                 1515

Val Phe Val Ser Ala Asp Trp Arg Phe
            500                 505

<210> 118
<211> 505
<212> PRT
<213> Neisseria meningitidis

<400> 118
Met Leu Tyr Phe Arg Tyr Gly Phe Leu Val Val Trp Cys Ala Ala Gly
1               5                   10                  15

Val Ser Ala Ala Tyr Gly Ala Asp Ala Pro Ala Ile Leu Asp Asp Lys
            20                  25                  30

Ala Leu Leu Gln Val Gln Arg Ser Val Ser Asp Lys Trp Ala Glu Ser
        35                  40                  45

Asp Trp Lys Val Asp Asn Asp Ala Pro Arg Val Val Asp Gly Asp Phe
    50                  55                  60

Leu Leu Ala His Pro Lys Met Leu Glu His Ser Leu Arg Asp Val Leu
65                  70                  75                  80

Asn Gly Asn Gln Ala Asp Leu Ile Ala Ser Leu Ala Asp Leu Tyr Ala
                85                  90                  95

Lys Leu Pro Asp Tyr Asp Ala Val Leu Tyr Gly Arg Ala Arg Ala Leu
            100                 105                 110

Leu Ala Lys Leu Ala Gly Arg Pro Ala Glu Ala Val Ala Arg Tyr Arg
        115                 120                 125

Glu Leu His Gly Glu Asn Ala Ala Asp Glu Arg Ile Leu Leu Asp Leu
    130                 135                 140

Ala Ala Ala Glu Phe Asp Asp Phe Arg Leu Lys Ser Ala Glu Arg His
145                 150                 155                 160

Phe Ala Glu Ala Glu Lys Leu Asp Leu Pro Ala Pro Val Leu Glu Asn
                165                 170                 175

Val Gly Arg Phe Arg Lys Lys Ala Glu Gly Leu Thr Gly Trp Arg Phe
            180                 185                 190

Ser Gly Gly Ile Ser Pro Ala Val Asn Arg Asn Ala Asn Asn Ala Ala
        195                 200                 205

Pro Gln Tyr Cys Arg Gln Asn Gly Gly Arg Gln Ile Cys Ser Val Ser
    210                 215                 220

Arg Ala Glu Arg Ala Ala Gly Leu Asn Tyr Glu Ile Glu Ala Glu Lys
225                 230                 235                 240

Leu Thr Ala Leu Ala Asp Asn His Tyr Leu Leu Phe Arg Ser Asn Ile
                245                 250                 255

Gly Gly Thr Ser Tyr Tyr Phe Ser Lys Lys Ser Ala Tyr Asp Asp Gly
            260                 265                 270

Phe Gly Arg Ala Tyr Leu Gly Trp Gln Tyr Lys Asn Ala Arg Gln Thr
        275                 280                 285

Ala Gly Ile Leu Pro Phe Tyr Gln Val Gln Leu Ser Gly Ser Asp Gly
    290                 295                 300

Phe Asp Ala Lys Thr Lys Arg Val Asn Asn Arg Arg Leu Pro Pro Tyr
305                 310                 315                 320

Met Leu Ala His Gly Val Gly Val Gln Leu Ser His Thr Tyr Arg Pro
                325                 330                 335

Asn Pro Gly Trp Gln Phe Ser Val Ala Leu Glu His Tyr Arg Gln Arg
            340                 345                 350

Tyr Arg Glu Gln Asp Arg Ala Glu Tyr Asn Asn Gly Arg Gln Asp Gly
        355                 360                 365

Phe Tyr Val Ser Ser Ala Lys Arg Leu Gly Glu Ser Ala Thr Val Phe
    370                 375                 380

Gly Gly Trp Gln Phe Val Arg Phe Val Pro Lys Arg Glu Thr Val Gly
385                 390                 395                 400

Gly Ala Val Asn Asn Ala Ala Tyr Arg Arg Asn Gly Val Tyr Ala Gly
                405                 410                 415

Trp Ala Gln Glu Trp Arg Gln Leu Gly Gly Leu Asn Ser Arg Val Ser
            420                 425                 430

Ala Ser Tyr Ala Arg Arg Asn Tyr Lys Gly Val Ala Ala Phe Ser Thr
        435                 440                 445

Glu Ala Gln Arg Asn Arg Glu Trp Asn Val Ser Leu Ala Leu Ser His
    450                 455                 460

Asp Lys Leu Ser Tyr Lys Gly Ile Val Pro Ala Leu Asn Tyr Arg Phe
465                 470                 475                 480

Gly Arg Thr Glu Ser Asn Val Pro Tyr Ala Lys Arg Arg Asn Ser Glu
                485                 490                 495

Val Phe Val Ser Ala Asp Trp Arg Phe
            500                 505

<210> 119
<211> 756
<212> DNA
<213> Neisseria meningitidis

<220>
<221> CDS
<222> (1)..(756)

<400> 119
atg gaa acg cat tcg ggc tgt ttt ttg cgg aag acg gta atg aaa gac    48
Met Glu Thr His Ser Gly Cys Phe Leu Arg Lys Thr Val Met Lys Asp
  1               5                  10                  15

gat gtt ttg aaa cag cag gca cac gcg gcg ata cag aag aaa ctg ggc    96
Asp Val Leu Lys Gln Gln Ala His Ala Ala Ile Gln Lys Lys Leu Gly
             20                  25                  30

tac gcg ttc cgc gat att tcg ctt ttg cgg cag gct ttg acg cac agg    144
Tyr Ala Phe Arg Asp Ile Ser Leu Leu Arg Gln Ala Leu Thr His Arg
         35                  40                  45

agc cat cat gcg aag cac aac gag cgg ttc gag ttt gtc ggc gat tcg    192
Ser His His Ala Lys His Asn Glu Arg Phe Glu Phe Val Gly Asp Ser
     50                  55                  60

att ttg aat tat acg gtg gcg cgg atg ctg ttt gac gcg ttt ccg aag    240
Ile Leu Asn Tyr Thr Val Ala Arg Met Leu Phe Asp Ala Phe Pro Lys
 65                  70                  75                  80

ttg acc gag ggc gag ttg tcg cgg ttg cgg gca agt ctg gtc aat gag    288
Leu Thr Glu Gly Glu Leu Ser Arg Leu Arg Ala Ser Leu Val Asn Glu
                 85                  90                  95

ggc gtg ctg gcg gaa atg gcg gcg gaa atg aat gtc ggc gac ggc ctg    336
Gly Val Leu Ala Glu Met Ala Ala Glu Met Asn Val Gly Asp Gly Leu

            100                 105                 110

tac ttg ggg gcg ggc gag ttg aag agc ggc ggc ttc aga cgg cct tcg 384
Tyr Leu Gly Ala Gly Glu Leu Lys Ser Gly Gly Phe Arg Arg Pro Ser
        115                 120                 125

ata ctg gcg gac gcg atg gag gcg atg ttt gcg gcg gtc agc ttc gat 432
Ile Leu Ala Asp Ala Met Glu Ala Met Phe Ala Ala Val Ser Phe Asp
    130                 135                 140

gcg gat ttc aac acg gcg gaa aag gtg gtg cgc cat ttg ttt gca gaa 480
Ala Asp Phe Asn Thr Ala Glu Lys Val Val Arg His Leu Phe Ala Glu
145                 150                 155                 160

cgc gtc cgg cga gtc gat ttc caa aat cag gca aaa gac ggc aaa act 528
Arg Val Arg Arg Val Asp Phe Gln Asn Gln Ala Lys Asp Gly Lys Thr
                165                 170                 175

gct ttg cag gag gcg ttg cag gcg cgc cgt ttc gcc ttg ccg aaa tac 576
Ala Leu Gln Glu Ala Leu Gln Ala Arg Arg Phe Ala Leu Pro Lys Tyr
            180                 185                 190

cgt atc gaa gag caa atc ggc cat gcc aac gac agt atg ttt gtc att 624
Arg Ile Glu Glu Gln Ile Gly His Ala Asn Asp Ser Met Phe Val Ile
        195                 200                 205

tcc tgc gat ttg ggc gaa ctg ggt ttc gtg tgc cgt gcc aaa ggg acg 672
Ser Cys Asp Leu Gly Glu Leu Gly Phe Val Cys Arg Ala Lys Gly Thr
    210                 215                 220

agc cgc aag gcg gcg gag cag gaa gcg gcg aaa gag gct ttg aaa tgg 720
Ser Arg Lys Ala Ala Glu Gln Glu Ala Ala Lys Glu Ala Leu Lys Trp
225                 230                 235                 240

ctg gaa gag aag ctg ccg ctg aag aag aaa aag aaa 756
Leu Glu Glu Lys Leu Pro Leu Lys Lys Lys Lys Lys
                245                 250

<210> 120
<211> 252
<212> PRT
<213> Neisseria meningitidis

<400> 120
Met Glu Thr His Ser Gly Cys Phe Leu Arg Lys Thr Val Met Lys Asp
1               5                   10                  15

Asp Val Leu Lys Gln Gln Ala His Ala Ala Ile Gln Lys Lys Leu Gly
            20                  25                  30

Tyr Ala Phe Arg Asp Ile Ser Leu Leu Arg Gln Ala Leu Thr His Arg
        35                  40                  45

Ser His His Ala Lys His Asn Glu Arg Phe Glu Phe Val Gly Asp Ser
    50                  55                  60

Ile Leu Asn Tyr Thr Val Ala Arg Met Leu Phe Asp Ala Phe Pro Lys
65                  70                  75                  80

Leu Thr Glu Gly Glu Leu Ser Arg Leu Arg Ala Ser Leu Val Asn Glu
                85                  90                  95

Gly Val Leu Ala Glu Met Ala Ala Glu Met Asn Val Gly Asp Gly Leu
            100                 105                 110

Tyr Leu Gly Ala Gly Glu Leu Lys Ser Gly Gly Phe Arg Arg Pro Ser
        115                 120                 125

Ile Leu Ala Asp Ala Met Glu Ala Met Phe Ala Ala Val Ser Phe Asp
    130                 135                 140

Ala Asp Phe Asn Thr Ala Glu Lys Val Val Arg His Leu Phe Ala Glu
145                 150                 155                 160

Arg Val Arg Arg Val Asp Phe Gln Asn Gln Ala Lys Asp Gly Lys Thr
                165                 170                 175

Ala Leu Gln Glu Ala Leu Gln Ala Arg Arg Phe Ala Leu Pro Lys Tyr
            180                 185                 190

Arg Ile Glu Glu Gln Ile Gly His Ala Asn Asp Ser Met Phe Val Ile
        195                 200                 205

Ser Cys Asp Leu Gly Glu Leu Gly Phe Val Cys Arg Ala Lys Gly Thr
    210                 215                 220

Ser Arg Lys Ala Ala Glu Gln Glu Ala Ala Lys Glu Ala Leu Lys Trp
225                 230                 235                 240

Leu Glu Glu Lys Leu Pro Leu Lys Lys Lys Lys Lys
                245                 250

<210> 121

<211> 570
<212> DNA
<213> Neisseria meningitidis

<220>
<221> CDS
<222> (1)..(570)

<400> 121
atg gac aat cac gcc gaa gca cac tgg caa aac ggc tgg ctt caa agc 48
Met Asp Asn His Ala Glu Ala His Trp Gln Asn Gly Trp Leu Gln Ser
1               5                   10                  15

ata cgc cat acc ccg tcg ccc aat ttc agc ccg agg gaa acg ggg gaa 96
Ile Arg His Thr Pro Ser Pro Asn Phe Ser Pro Arg Glu Thr Gly Glu
            20                  25                  30

acg gtt tcc ctg atc gtg ttg cac aac att tca ctg ccg ccg ttc gaa 144
Thr Val Ser Leu Ile Val Leu His Asn Ile Ser Leu Pro Pro Phe Glu
        35                  40                  45

tac ggc acg gat gct gtg gaa aag ctg ttt gcc aac cgg ctc gac ccc 192
Tyr Gly Thr Asp Ala Val Glu Lys Leu Phe Ala Asn Arg Leu Asp Pro
    50                  55                  60

aac gga cat ccg ttc ttc agc ctg ata cac act ttg cgc gta tcc agc 240
Asn Gly His Pro Phe Phe Ser Leu Ile His Thr Leu Arg Val Ser Ser
65                  70                  75                  80

cat ttc tta atc aaa cgc gac ggc aaa acg gtg cag ttc gta tca tgc 288
His Phe Leu Ile Lys Arg Asp Gly Lys Thr Val Gln Phe Val Ser Cys
                85                  90                  95

ggc gat atg gcg tac cac gcg ggc gta tcc tcg ttt cgc gga cgg gaa 336
Gly Asp Met Ala Tyr His Ala Gly Val Ser Ser Phe Arg Gly Arg Glu
            100                 105                 110

aaa tgc aac gca ttt tcc atc ggc atc gaa ttg gaa ggc tgc gat ttc 384
Lys Cys Asn Ala Phe Ser Ile Gly Ile Glu Leu Glu Gly Cys Asp Phe
        115                 120                 125

gaa ccc ttt acc gaa gcg caa tac cgt tcg ctc gaa aca ttg ttg gaa 432
Glu Pro Phe Thr Glu Ala Gln Tyr Arg Ser Leu Glu Thr Leu Leu Glu
    130                 135                 140

gca ctc tgc cgc cgc tac ccc gtt acc gca gta acc gga cat cag gac 480
Ala Leu Cys Arg Arg Tyr Pro Val Thr Ala Val Thr Gly His Gln Asp
145                 150                 155                 160

atc gcg ccc ggc cgc aaa acc gac ccc ggc cac ttt ttc gac tgg cgg     528
Ile Ala Pro Gly Arg Lys Thr Asp Pro Gly His Phe Phe Asp Trp Arg
                165                 170                 175

cgg ata cgg gag aaa ggg ttt ccc gta gac aga aat gcc gtc             570
Arg Ile Arg Glu Lys Gly Phe Pro Val Asp Arg Asn Ala Val
            180                 185                 190

<210> 122
<211> 190
<212> PRT
<213> Neisseria meningitidis

<400> 122
Met Asp Asn His Ala Glu Ala His Trp Gln Asn Gly Trp Leu Gln Ser
1               5                   10                  15

Ile Arg His Thr Pro Ser Pro Asn Phe Ser Pro Arg Glu Thr Gly Glu
            20                  25                  30

Thr Val Ser Leu Ile Val Leu His Asn Ile Ser Leu Pro Pro Phe Glu
        35                  40                  45

Tyr Gly Thr Asp Ala Val Glu Lys Leu Phe Ala Asn Arg Leu Asp Pro
    50                  55                  60

Asn Gly His Pro Phe Phe Ser Leu Ile His Thr Leu Arg Val Ser Ser
65                  70                  75                  80

His Phe Leu Ile Lys Arg Asp Gly Lys Thr Val Gln Phe Val Ser Cys
                85                  90                  95

Gly Asp Met Ala Tyr His Ala Gly Val Ser Ser Phe Arg Gly Arg Glu
            100                 105                 110

Lys Cys Asn Ala Phe Ser Ile Gly Ile Glu Leu Glu Gly Cys Asp Phe
        115                 120                 125

Glu Pro Phe Thr Glu Ala Gln Tyr Arg Ser Leu Glu Thr Leu Leu Glu
    130                 135                 140

Ala Leu Cys Arg Arg Tyr Pro Val Thr Ala Val Thr Gly His Gln Asp
145                 150                 155                 160

Ile Ala Pro Gly Arg Lys Thr Asp Pro Gly His Phe Phe Asp Trp Arg
                165                 170                 175

Arg Ile Arg Glu Lys Gly Phe Pro Val Asp Arg Asn Ala Val
            180                 185                 190

<210> 123
<211> 2274
<212> DNA
<213> Neisseria meningitidis

<220>
<221> CDS
<222> (1)..(2274)

<400> 123
atg aca aca tta cat ttc tca ggc ttc ccg cgt gtc ggt gcc ttc cgc      48
Met Thr Thr Leu His Phe Ser Gly Phe Pro Arg Val Gly Ala Phe Arg
1               5                   10                  15

gaa ttg aaa ttc gca caa gaa aaa tac tgg cgc aaa gaa atc agc gag      96
Glu Leu Lys Phe Ala Gln Glu Lys Tyr Trp Arg Lys Glu Ile Ser Glu
            20                  25                  30

caa gaa ttg ctg gct gtt gct aaa gac ttg cgc gag aaa aac tgg aaa     144
Gln Glu Leu Leu Ala Val Ala Lys Asp Leu Arg Glu Lys Asn Trp Lys
        35                  40                  45

cac cag gcc gct gcc aac gcc gat tac gtt gcc gta ggc gat ttc act     192
His Gln Ala Ala Ala Asn Ala Asp Tyr Val Ala Val Gly Asp Phe Thr
    50                  55                  60

ttc tac gac cac atc ctc gac ctg caa gtc gcc acc ggc gcg att ccc     240
Phe Tyr Asp His Ile Leu Asp Leu Gln Val Ala Thr Gly Ala Ile Pro
65                  70                  75                  80

gcc cgc ttc ggc ttc gac agc caa aac cta tct ttg gaa caa ttc ttc     288
Ala Arg Phe Gly Phe Asp Ser Gln Asn Leu Ser Leu Glu Gln Phe Phe
                85                  90                  95

caa ctg gcg cgc ggt aac aaa gac caa ttc gct atc gaa atg acc aaa     336
Gln Leu Ala Arg Gly Asn Lys Asp Gln Phe Ala Ile Glu Met Thr Lys
            100                 105                 110

tgg ttc gac acc aac tac cac tac ttg gtg cct gaa ttc cac gcc gat     384
Trp Phe Asp Thr Asn Tyr His Tyr Leu Val Pro Glu Phe His Ala Asp
        115                 120                 125

acc gaa ttt aaa gcc aat gcc aaa cac tac gtt caa cag ctg caa gaa 432
Thr Glu Phe Lys Ala Asn Ala Lys His Tyr Val Gln Gln Leu Gln Glu
    130                 135                 140

gcc caa gct ttg ggc ctg aaa gcc aaa ccg acc gtt gtg ggt ccg ttg 480
Ala Gln Ala Leu Gly Leu Lys Ala Lys Pro Thr Val Val Gly Pro Leu
145                 150                 155                 160

act ttc ctg tgg gtc ggt aaa gaa aaa ggc gcc gtt gaa ttc gac cgt 528
Thr Phe Leu Trp Val Gly Lys Glu Lys Gly Ala Val Glu Phe Asp Arg
                165                 170                 175

ctg agc ctg ttg cct aaa ctg ttg cct gtt tac gtt gaa atc ctg act 576
Leu Ser Leu Leu Pro Lys Leu Leu Pro Val Tyr Val Glu Ile Leu Thr
            180                 185                 190

gct ttg gtt gaa gcc ggt gcc gag tgg att caa atc gac gag cct gct 624
Ala Leu Val Glu Ala Gly Ala Glu Trp Ile Gln Ile Asp Glu Pro Ala
        195                 200                 205

ttg act gtt gac ctg cct aaa gaa tgg gta gaa gca tac aaa gac gtt 672
Leu Thr Val Asp Leu Pro Lys Glu Trp Val Glu Ala Tyr Lys Asp Val
    210                 215                 220

tac gcc act ttg agc aaa gta agt gcc aaa atc ctg ctg agc act tac 720
Tyr Ala Thr Leu Ser Lys Val Ser Ala Lys Ile Leu Leu Ser Thr Tyr
225                 230                 235                 240

ttc ggt tct gtt gcc gaa cac gcc gca ttg ttg aaa tcc ctg cct gtt 768
Phe Gly Ser Val Ala Glu His Ala Ala Leu Leu Lys Ser Leu Pro Val
                245                 250                 255

gac ggc ctg cac atc gac ttg gta cgc gct cct gaa caa ctg gat gcg 816
Asp Gly Leu His Ile Asp Leu Val Arg Ala Pro Glu Gln Leu Asp Ala
            260                 265                 270

ttc gcc gac tac gac aaa gtt ctg tct gcc ggc gtc att gac ggc cgc 864
Phe Ala Asp Tyr Asp Lys Val Leu Ser Ala Gly Val Ile Asp Gly Arg
        275                 280                 285

aat att tgg cgc gcc aac ctg aac aaa gtt ttg gaa act gtt gag ctt 912
Asn Ile Trp Arg Ala Asn Leu Asn Lys Val Leu Glu Thr Val Glu Leu
    290                 295                 300

ctg caa gcc aaa ctg ggc gac cgt ttg tgg att tcc agc tct tgc tcg 960
Leu Gln Ala Lys Leu Gly Asp Arg Leu Trp Ile Ser Ser Ser Cys Ser
305                 310                 315                 320

ctg ctg cac act ccg ttt gac ttg tca gtt gaa gaa aaa ctg aaa gcc 1008
Leu Leu His Thr Pro Phe Asp Leu Ser Val Glu Glu Lys Leu Lys Ala
                325                 330                 335

aac aaa ccc gac ctg tac tct tgg ttg gca ttc acc ctg caa aaa acc 1056
Asn Lys Pro Asp Leu Tyr Ser Trp Leu Ala Phe Thr Leu Gln Lys Thr
            340                 345                 350

caa gaa ttg cgc gtc ctg aaa gcc gca ttg aac gaa ggc cgt gat tct 1104
Gln Glu Leu Arg Val Leu Lys Ala Ala Leu Asn Glu Gly Arg Asp Ser
        355                 360                 365

gtt gcc gaa gaa ctg gct gcc agc caa gct gct gcc gac tcc cgt gcc 1152
Val Ala Glu Glu Leu Ala Ala Ser Gln Ala Ala Ala Asp Ser Arg Ala
    370                 375                 380

aac agc agc gaa atc cac cgt gca gac gtt gcc aaa cgc ctg gcc gat 1200
Asn Ser Ser Glu Ile His Arg Ala Asp Val Ala Lys Arg Leu Ala Asp
385                 390                 395                 400

ttg cct gcc aac gca gac caa cgc aaa tcc cca ttt gcc gac cgt atc 1248
Leu Pro Ala Asn Ala Asp Gln Arg Lys Ser Pro Phe Ala Asp Arg Ile
                405                 410                 415

aaa gcg caa caa gca tgg ttg aac ctg cct ctg ctg ccg acg acc aac 1296
Lys Ala Gln Gln Ala Trp Leu Asn Leu Pro Leu Leu Pro Thr Thr Asn
            420                 425                 430

atc ggt tct ttc ccg caa act acc gaa atc cgc caa gca cgc gca gcc 1344
Ile Gly Ser Phe Pro Gln Thr Thr Glu Ile Arg Gln Ala Arg Ala Ala
        435                 440                 445

ttc aaa aaa ggc gaa ctg tct gcc gcc gat tac gaa gcc gcg atg aaa 1392
Phe Lys Lys Gly Glu Leu Ser Ala Ala Asp Tyr Glu Ala Ala Met Lys
    450                 455                 460

aaa gaa atc gcc ttg gtg gtt gaa gag caa gaa aaa ctg gac ttg gac 1440
Lys Glu Ile Ala Leu Val Val Glu Glu Gln Glu Lys Leu Asp Leu Asp
465                 470                 475                 480

gta ctg gta cac ggc gaa gcc gag cgt aac gac atg gtt gaa tac ttc 1488
Val Leu Val His Gly Glu Ala Glu Arg Asn Asp Met Val Glu Tyr Phe
                485                 490                 495

ggc gaa ttg ttg agc ggt ttt gca ttc acc caa tat ggc tgg gta caa 1536
Gly Glu Leu Leu Ser Gly Phe Ala Phe Thr Gln Tyr Gly Trp Val Gln
            500                 505                 510

agc tac ggt tct cgc tgc gtt aaa cca ccg att atc ttc ggt gac gta    1584
Ser Tyr Gly Ser Arg Cys Val Lys Pro Pro Ile Ile Phe Gly Asp Val
        515                 520                 525

agc cgt cct gaa gcc atg acc gta gct tgg tct act tac gca caa agc    1632
Ser Arg Pro Glu Ala Met Thr Val Ala Trp Ser Thr Tyr Ala Gln Ser
    530                 535                 540

ctg acc aaa cgc ccg atg aaa ggt atg ttg acc ggc cct gta acc att    1680
Leu Thr Lys Arg Pro Met Lys Gly Met Leu Thr Gly Pro Val Thr Ile
545                 550                 555                 560

ctg caa tgg tct ttc gtc cgc aac gat att cct cgc tct acc gtg tgt    1728
Leu Gln Trp Ser Phe Val Arg Asn Asp Ile Pro Arg Ser Thr Val Cys
                565                 570                 575

aaa caa atc gca ctg gct ctg aac gac gaa gta ttg gat ctg gaa aaa    1776
Lys Gln Ile Ala Leu Ala Leu Asn Asp Glu Val Leu Asp Leu Glu Lys
            580                 585                 590

gca ggc atc aaa gtc atc caa atc gac gaa cct gcc atc cgc gaa ggc    1824
Ala Gly Ile Lys Val Ile Gln Ile Asp Glu Pro Ala Ile Arg Glu Gly
        595                 600                 605

ctg cct ttg aaa cgt gcc gat tgg gat gcc tac ctg aac tgg gcc ggc    1872
Leu Pro Leu Lys Arg Ala Asp Trp Asp Ala Tyr Leu Asn Trp Ala Gly
    610                 615                 620

gaa tct ttc cgc ctg tcc tct acc ggt tgc gaa gac agc act caa atc    1920
Glu Ser Phe Arg Leu Ser Ser Thr Gly Cys Glu Asp Ser Thr Gln Ile
625                 630                 635                 640

cac acc cat atg tgc tac tct gag ttc aac gac atc ctg cct gcg att    1968
His Thr His Met Cys Tyr Ser Glu Phe Asn Asp Ile Leu Pro Ala Ile
                645                 650                 655

gct gca atg gat gcg gac gtg atc acc atc gag act tca cgt tcc gac    2016
Ala Ala Met Asp Ala Asp Val Ile Thr Ile Glu Thr Ser Arg Ser Asp
            660                 665                 670

atg gaa ctc ttg acc gcg ttc ggc gaa ttc aaa tac ccg aac gac atc    2064
Met Glu Leu Leu Thr Ala Phe Gly Glu Phe Lys Tyr Pro Asn Asp Ile
        675                 680                 685

ggc ccg ggg gtt tac gac atc cac agc ccg cgc gta ccg aca gaa gcc    2112
Gly Pro Gly Val Tyr Asp Ile His Ser Pro Arg Val Pro Thr Glu Ala
    690                 695                 700

gaa gtg gag cac ctg ttg cgc aaa gcc atc gag gtt gta ccg gtt gaa    2160
Glu Val Glu His Leu Leu Arg Lys Ala Ile Glu Val Val Pro Val Glu
705                 710                 715                 720

cgt ctg tgg gtt aac ccg gac tgc ggc ctg aaa aca cgc ggc tgg aaa    2208
Arg Leu Trp Val Asn Pro Asp Cys Gly Leu Lys Thr Arg Gly Trp Lys
                725                 730                 735

gaa act ctg gaa caa ctc caa gtg atg atg aac gta acc cac aaa ttg    2256
Glu Thr Leu Glu Gln Leu Gln Val Met Met Asn Val Thr His Lys Leu
            740                 745                 750

cgt gcc gaa ttg gcg aaa                                            2274
Arg Ala Glu Leu Ala Lys
        755

<210> 124
<211> 758
<212> PRT
<213> Neisseria meningitidis

<400> 124
Met Thr Thr Leu His Phe Ser Gly Phe Pro Arg Val Gly Ala Phe Arg
  1               5                  10                  15

Glu Leu Lys Phe Ala Gln Glu Lys Tyr Trp Arg Lys Glu Ile Ser Glu
             20                  25                  30

Gln Glu Leu Leu Ala Val Ala Lys Asp Leu Arg Glu Lys Asn Trp Lys
         35                  40                  45

His Gln Ala Ala Ala Asn Ala Asp Tyr Val Ala Val Gly Asp Phe Thr
     50                  55                  60

Phe Tyr Asp His Ile Leu Asp Leu Gln Val Ala Thr Gly Ala Ile Pro
 65                  70                  75                  80

Ala Arg Phe Gly Phe Asp Ser Gln Asn Leu Ser Leu Glu Gln Phe Phe
                 85                  90                  95

Gln Leu Ala Arg Gly Asn Lys Asp Gln Phe Ala Ile Glu Met Thr Lys
            100                 105                 110

Trp Phe Asp Thr Asn Tyr His Tyr Leu Val Pro Glu Phe His Ala Asp
        115                 120                 125

Thr Glu Phe Lys Ala Asn Ala Lys His Tyr Val Gln Gln Leu Gln Glu

130                 135                 140

Ala Gln Ala Leu Gly Leu Lys Ala Lys Pro Thr Val Val Gly Pro Leu
145                 150                 155                 160

Thr Phe Leu Trp Val Gly Lys Glu Lys Gly Ala Val Glu Phe Asp Arg
                165                 170                 175

Leu Ser Leu Leu Pro Lys Leu Leu Pro Val Tyr Val Glu Ile Leu Thr
            180                 185                 190

Ala Leu Val Glu Ala Gly Ala Glu Trp Ile Gln Ile Asp Glu Pro Ala
        195                 200                 205

Leu Thr Val Asp Leu Pro Lys Glu Trp Val Glu Ala Tyr Lys Asp Val
    210                 215                 220

Tyr Ala Thr Leu Ser Lys Val Ser Ala Lys Ile Leu Leu Ser Thr Tyr
225                 230                 235                 240

Phe Gly Ser Val Ala Glu His Ala Ala Leu Leu Lys Ser Leu Pro Val
                245                 250                 255

Asp Gly Leu His Ile Asp Leu Val Arg Ala Pro Glu Gln Leu Asp Ala
            260                 265                 270

Phe Ala Asp Tyr Asp Lys Val Leu Ser Ala Gly Val Ile Asp Gly Arg
        275                 280                 285

Asn Ile Trp Arg Ala Asn Leu Asn Lys Val Leu Glu Thr Val Glu Leu
    290                 295                 300

Leu Gln Ala Lys Leu Gly Asp Arg Leu Trp Ile Ser Ser Ser Cys Ser
305                 310                 315                 320

Leu Leu His Thr Pro Phe Asp Leu Ser Val Glu Glu Lys Leu Lys Ala
                325                 330                 335

Asn Lys Pro Asp Leu Tyr Ser Trp Leu Ala Phe Thr Leu Gln Lys Thr
            340                 345                 350

Gln Glu Leu Arg Val Leu Lys Ala Ala Leu Asn Glu Gly Arg Asp Ser
        355                 360                 365

Val Ala Glu Glu Leu Ala Ala Ser Gln Ala Ala Ala Asp Ser Arg Ala
    370                 375                 380

Asn Ser Ser Glu Ile His Arg Ala Asp Val Ala Lys Arg Leu Ala Asp

385                 390                 395                 400

Leu Pro Ala Asn Ala Asp Gln Arg Lys Ser Pro Phe Ala Asp Arg Ile
                405                 410                 415

Lys Ala Gln Gln Ala Trp Leu Asn Leu Pro Leu Leu Pro Thr Thr Asn
            420                 425                 430

Ile Gly Ser Phe Pro Gln Thr Thr Glu Ile Arg Gln Ala Arg Ala Ala
        435                 440                 445

Phe Lys Lys Gly Glu Leu Ser Ala Ala Asp Tyr Glu Ala Ala Met Lys
    450                 455                 460

Lys Glu Ile Ala Leu Val Val Glu Glu Gln Glu Lys Leu Asp Leu Asp
465                 470                 475                 480

Val Leu Val His Gly Glu Ala Glu Arg Asn Asp Met Val Glu Tyr Phe
                485                 490                 495

Gly Glu Leu Leu Ser Gly Phe Ala Phe Thr Gln Tyr Gly Trp Val Gln
            500                 505                 510

Ser Tyr Gly Ser Arg Cys Val Lys Pro Pro Ile Ile Phe Gly Asp Val
        515                 520                 525

Ser Arg Pro Glu Ala Met Thr Val Ala Trp Ser Thr Tyr Ala Gln Ser
    530                 535                 540

Leu Thr Lys Arg Pro Met Lys Gly Met Leu Thr Gly Pro Val Thr Ile
545                 550                 555                 560

Leu Gln Trp Ser Phe Val Arg Asn Asp Ile Pro Arg Ser Thr Val Cys
                565                 570                 575

Lys Gln Ile Ala Leu Ala Leu Asn Asp Glu Val Leu Asp Leu Glu Lys
            580                 585                 590

Ala Gly Ile Lys Val Ile Gln Ile Asp Glu Pro Ala Ile Arg Glu Gly
        595                 600                 605

Leu Pro Leu Lys Arg Ala Asp Trp Asp Ala Tyr Leu Asn Trp Ala Gly
    610                 615                 620

Glu Ser Phe Arg Leu Ser Ser Thr Gly Cys Glu Asp Ser Thr Gln Ile
625                 630                 635                 640

His Thr His Met Cys Tyr Ser Glu Phe Asn Asp Ile Leu Pro Ala Ile

645                 650                 655

Ala Ala Met Asp Ala Asp Val Ile Thr Ile Glu Thr Ser Arg Ser Asp
            660                 665                 670

Met Glu Leu Leu Thr Ala Phe Gly Glu Phe Lys Tyr Pro Asn Asp Ile
        675                 680                 685

Gly Pro Gly Val Tyr Asp Ile His Ser Pro Arg Val Pro Thr Glu Ala
    690                 695                 700

Glu Val Glu His Leu Leu Arg Lys Ala Ile Glu Val Val Pro Val Glu
705                 710                 715                 720

Arg Leu Trp Val Asn Pro Asp Cys Gly Leu Lys Thr Arg Gly Trp Lys
                725                 730                 735

Glu Thr Leu Glu Gln Leu Gln Val Met Met Asn Val Thr His Lys Leu
            740                 745                 750

Arg Ala Glu Leu Ala Lys
        755

<210> 125
<211> 1137
<212> DNA
<213> Neisseria meningitidis

<220>
<221> CDS
<222> (1)..(1137)

<400> 125
atg agc cgt tta tgg ttt ttt gcc gca aaa aac att ata atc cgc ctt    48
Met Ser Arg Leu Trp Phe Phe Ala Ala Lys Asn Ile Ile Ile Arg Leu
  1               5                  10                  15

att tac cta ttg ccc aag gag aca caa atg gca ctc gta tcc atg cgc    96
Ile Tyr Leu Leu Pro Lys Glu Thr Gln Met Ala Leu Val Ser Met Arg
             20                  25                  30

caa ctg ctt gat cat gct gcc gaa aac agc tac ggc ctg ccc gcg ttc    144
Gln Leu Leu Asp His Ala Ala Glu Asn Ser Tyr Gly Leu Pro Ala Phe
         35                  40                  45

aac gtc aac aac ctc gaa caa atg cgc gcc att atg gaa gcc gcc gac    192

Asn Val Asn Asn Leu Glu Gln Met Arg Ala Ile Met Glu Ala Ala Asp
    50                  55                  60

caa gtc aac gcg ccc gtc atc gta cag gcg agc gca ggt gcg cgc aaa 240
Gln Val Asn Ala Pro Val Ile Val Gln Ala Ser Ala Gly Ala Arg Lys
65                  70                  75                  80

tac gcg ggc gcg ccg ttt ttg cgc cac ctg att ttg gcg gct gtc gaa 288
Tyr Ala Gly Ala Pro Phe Leu Arg His Leu Ile Leu Ala Ala Val Glu
                85                  90                  95

gaa ttt ccg cac atc ccc gtc gtg atg cac caa gac cac ggc gca tcg 336
Glu Phe Pro His Ile Pro Val Val Met His Gln Asp His Gly Ala Ser
            100                 105                 110

ccc gac gtg tgc caa cgc tcc atc caa ctg ggc ttt tcc tcc gtg atg 384
Pro Asp Val Cys Gln Arg Ser Ile Gln Leu Gly Phe Ser Ser Val Met
        115                 120                 125

atg gac ggc tcg ctg atg gaa gac ggc aaa acc cct tct tct tat gaa 432
Met Asp Gly Ser Leu Met Glu Asp Gly Lys Thr Pro Ser Ser Tyr Glu
    130                 135                 140

tac aac gtc aac gcc acc cgt acc gtg gtt aat ttc tcc cac gcc tgc 480
Tyr Asn Val Asn Ala Thr Arg Thr Val Val Asn Phe Ser His Ala Cys
145                 150                 155                 160

ggc gta tcc gtt gaa ggc gaa atc ggc gta ttg ggc aac ctc gaa act 528
Gly Val Ser Val Glu Gly Glu Ile Gly Val Leu Gly Asn Leu Glu Thr
                165                 170                 175

ggc gaa gcc ggc gaa gaa gac ggt gta ggc gca gtg ggc aaa ctt tcc 576
Gly Glu Ala Gly Glu Glu Asp Gly Val Gly Ala Val Gly Lys Leu Ser
            180                 185                 190

cac gac caa atg ctc acc agc gtc gaa gat gcc gtg cgt ttc gtt aaa 624
His Asp Gln Met Leu Thr Ser Val Glu Asp Ala Val Arg Phe Val Lys
        195                 200                 205

gat acc ggc gtt gac gca ttg gcg att gcc gtc ggc acc agc cac ggc 672
Asp Thr Gly Val Asp Ala Leu Ala Ile Ala Val Gly Thr Ser His Gly
    210                 215                 220

gcg tac aaa ttc acc cgt ccg ccc aca ggc gac gtg ttg cgt atc gac 720
Ala Tyr Lys Phe Thr Arg Pro Pro Thr Gly Asp Val Leu Arg Ile Asp
225                 230                 235                 240

cgc atc aaa gaa atc cac caa gcc ctg ccc aat aca cac atc gtg atg 768

Arg Ile Lys Glu Ile His Gln Ala Leu Pro Asn Thr His Ile Val Met
                245                 250                 255

cac ggc tcc agc tcc gtt ccg caa gaa tgg ctg aaa gtc atc aac gaa 816
His Gly Ser Ser Ser Val Pro Gln Glu Trp Leu Lys Val Ile Asn Glu
            260                 265                 270

tac ggc ggc aat atc ggc gaa acc tac ggc gtg ccg gtt gaa gaa atc 864
Tyr Gly Gly Asn Ile Gly Glu Thr Tyr Gly Val Pro Val Glu Glu Ile
        275                 280                 285

gtc gaa ggc atc aaa cac ggc gtg cgt aaa gtc aac atc gat acc gac 912
Val Glu Gly Ile Lys His Gly Val Arg Lys Val Asn Ile Asp Thr Asp
    290                 295                 300

ttg cgc ctt gct tcc acc ggc gcg gta cgc cgc tac ctt gcc gaa aac 960
Leu Arg Leu Ala Ser Thr Gly Ala Val Arg Arg Tyr Leu Ala Glu Asn
305                 310                 315                 320

ccg tcc gac ttc gat ccg cgc aaa tat ttg agc aaa acc att gaa gcg 1008
Pro Ser Asp Phe Asp Pro Arg Lys Tyr Leu Ser Lys Thr Ile Glu Ala
                325                 330                 335

atg aag caa atc tgc ctc gac cgc tac ctc gcg ttc ggt tgc gaa ggt 1056
Met Lys Gln Ile Cys Leu Asp Arg Tyr Leu Ala Phe Gly Cys Glu Gly
            340                 345                 350

cag gca ggc aaa atc aaa ccg gtt tcc ttg gaa aaa atg gca aac cgt 1104
Gln Ala Gly Lys Ile Lys Pro Val Ser Leu Glu Lys Met Ala Asn Arg
        355                 360                 365

tat gcc aag ggc gaa ttg aac caa atc gtc aaa 1137
Tyr Ala Lys Gly Glu Leu Asn Gln Ile Val Lys
    370                 375

<210> 126
<211> 379
<212> PRT
<213> Neisseria meningitidis

<400> 126
Met Ser Arg Leu Trp Phe Phe Ala Ala Lys Asn Ile Ile Ile Arg Leu
1               5                   10                  15

Ile Tyr Leu Leu Pro Lys Glu Thr Gln Met Ala Leu Val Ser Met Arg
            20                  25                  30

Gln Leu Leu Asp His Ala Ala Glu Asn Ser Tyr Gly Leu Pro Ala Phe
        35                  40                  45

Asn Val Asn Asn Leu Glu Gln Met Arg Ala Ile Met Glu Ala Ala Asp
    50                  55                  60

Gln Val Asn Ala Pro Val Ile Val Gln Ala Ser Ala Gly Ala Arg Lys
65                  70                  75                  80

Tyr Ala Gly Ala Pro Phe Leu Arg His Leu Ile Leu Ala Ala Val Glu
                85                  90                  95

Glu Phe Pro His Ile Pro Val Val Met His Gln Asp His Gly Ala Ser
            100                 105                 110

Pro Asp Val Cys Gln Arg Ser Ile Gln Leu Gly Phe Ser Ser Val Met
        115                 120                 125

Met Asp Gly Ser Leu Met Glu Asp Gly Lys Thr Pro Ser Ser Tyr Glu
    130                 135                 140

Tyr Asn Val Asn Ala Thr Arg Thr Val Val Asn Phe Ser His Ala Cys
145                 150                 155                 160

Gly Val Ser Val Glu Gly Glu Ile Gly Val Leu Gly Asn Leu Glu Thr
                165                 170                 175

Gly Glu Ala Gly Glu Glu Asp Gly Val Gly Ala Val Gly Lys Leu Ser
            180                 185                 190

His Asp Gln Met Leu Thr Ser Val Glu Asp Ala Val Arg Phe Val Lys
        195                 200                 205

Asp Thr Gly Val Asp Ala Leu Ala Ile Ala Val Gly Thr Ser His Gly
    210                 215                 220

Ala Tyr Lys Phe Thr Arg Pro Pro Thr Gly Asp Val Leu Arg Ile Asp
225                 230                 235                 240

Arg Ile Lys Glu Ile His Gln Ala Leu Pro Asn Thr His Ile Val Met
                245                 250                 255

His Gly Ser Ser Ser Val Pro Gln Glu Trp Leu Lys Val Ile Asn Glu
            260                 265                 270

Tyr Gly Gly Asn Ile Gly Glu Thr Tyr Gly Val Pro Val Glu Glu Ile
        275                 280                 285

Val Glu Gly Ile Lys His Gly Val Arg Lys Val Asn Ile Asp Thr Asp
    290                 295                 300

Leu Arg Leu Ala Ser Thr Gly Ala Val Arg Arg Tyr Leu Ala Glu Asn
305                 310                 315                 320

Pro Ser Asp Phe Asp Pro Arg Lys Tyr Leu Ser Lys Thr Ile Glu Ala
                325                 330                 335

Met Lys Gln Ile Cys Leu Asp Arg Tyr Leu Ala Phe Gly Cys Glu Gly
            340                 345                 350

Gln Ala Gly Lys Ile Lys Pro Val Ser Leu Glu Lys Met Ala Asn Arg
        355                 360                 365

Tyr Ala Lys Gly Glu Leu Asn Gln Ile Val Lys
    370                 375

<210> 127
<211> 444
<212> DNA
<213> Neisseria meningitidis

<220>
<221> CDS
<222> (1)..(444)

<400> 127
gtg gat gct gca gca acg ctc ctg aca tac aaa tgc ccg gcg gaa aac    48
Val Asp Ala Ala Ala Thr Leu Leu Thr Tyr Lys Cys Pro Ala Glu Asn
  1               5                  10                  15

cgc cca aac aca aaa aaa att ggt aat ttt tcc tat tca agg cta caa    96
Arg Pro Asn Thr Lys Lys Ile Gly Asn Phe Ser Tyr Ser Arg Leu Gln
             20                  25                  30

ttc gac acg cac agg gca tct gcc cga ttt cac aat cat aat agc gga    144
Phe Asp Thr His Arg Ala Ser Ala Arg Phe His Asn His Asn Ser Gly
         35                  40                  45

gtt aaa aat atg aca gct aaa gga caa atg ttg caa gat ccc ttc ctg    192
Val Lys Asn Met Thr Ala Lys Gly Gln Met Leu Gln Asp Pro Phe Leu
     50                  55                  60

aac gcc ctg cgt aaa gag cat gtt ccg gtt tcg att tac tta gtt aac    240
Asn Ala Leu Arg Lys Glu His Val Pro Val Ser Ile Tyr Leu Val Asn

65                  70                  75                  80

ggt atc aaa ttg caa ggt cag gtt gag tct ttc gat caa tac gtt gtt     288
Gly Ile Lys Leu Gln Gly Gln Val Glu Ser Phe Asp Gln Tyr Val Val
                85                  90                  95

ctc ctg aga aac act tcc gtc acc caa atg gtt tac aaa cac gcc att     336
Leu Leu Arg Asn Thr Ser Val Thr Gln Met Val Tyr Lys His Ala Ile
            100                 105                 110

tcc acc atc gta ccg gca cgc tcc gtc aac cta caa cat gaa aac aga     384
Ser Thr Ile Val Pro Ala Arg Ser Val Asn Leu Gln His Glu Asn Arg
        115                 120                 125

ccc caa gcc gca ccg act tcg acc ctc gtc caa gtg gaa acc gtc cag     432
Pro Gln Ala Ala Pro Thr Ser Thr Leu Val Gln Val Glu Thr Val Gln
    130                 135                 140

cag cct gcc gaa                                                     444
Gln Pro Ala Glu
145

<210> 128
<211> 148
<212> PRT
<213> Neisseria meningitidis

<400> 128
Val Asp Ala Ala Ala Thr Leu Leu Thr Tyr Lys Cys Pro Ala Glu Asn
1               5                   10                  15

Arg Pro Asn Thr Lys Lys Ile Gly Asn Phe Ser Tyr Ser Arg Leu Gln
            20                  25                  30

Phe Asp Thr His Arg Ala Ser Ala Arg Phe His Asn His Asn Ser Gly
        35                  40                  45

Val Lys Asn Met Thr Ala Lys Gly Gln Met Leu Gln Asp Pro Phe Leu
    50                  55                  60

Asn Ala Leu Arg Lys Glu His Val Pro Val Ser Ile Tyr Leu Val Asn
65                  70                  75                  80

Gly Ile Lys Leu Gln Gly Gln Val Glu Ser Phe Asp Gln Tyr Val Val
                85                  90                  95

Leu Leu Arg Asn Thr Ser Val Thr Gln Met Val Tyr Lys His Ala Ile

100                 105                 110

Ser Thr Ile Val Pro Ala Arg Ser Val Asn Leu Gln His Glu Asn Arg
        115                 120                 125

Pro Gln Ala Ala Pro Thr Ser Thr Leu Val Gln Val Glu Thr Val Gln
    130                 135                 140

Gln Pro Ala Glu
145

<210> 129
<211> 1308
<212> DNA
<213> Neisseria meningitidis

<220>
<221> CDS
<222> (1)..(1308)

<400> 129
atg tac gct aaa aaa ggc ggt ttg gga ctg gtt aaa agc cgc cgt ttc    48
Met Tyr Ala Lys Lys Gly Gly Leu Gly Leu Val Lys Ser Arg Arg Phe
  1               5                  10                  15

gca cct ctt ttc gct acg cag ttt ctc ggc gcg ttc aac gac aat gtg    96
Ala Pro Leu Phe Ala Thr Gln Phe Leu Gly Ala Phe Asn Asp Asn Val
             20                  25                  30

ttc aaa acc gcg ctg ttt gtg atg att ggg ttt tac ggt ttg ggg caa    144
Phe Lys Thr Ala Leu Phe Val Met Ile Gly Phe Tyr Gly Leu Gly Gln
         35                  40                  45

aac ggc ttc ctg cct gcc gga cag atg ttg aac ttg ggc gcg ttg ctg    192
Asn Gly Phe Leu Pro Ala Gly Gln Met Leu Asn Leu Gly Ala Leu Leu
     50                  55                  60

ttt att ttg ccg tat ttc ctg ttt tcc tcg ctg tcg ggg cag ttg ggt    240
Phe Ile Leu Pro Tyr Phe Leu Phe Ser Ser Leu Ser Gly Gln Leu Gly
 65                  70                  75                  80

aac aaa ttc gac aag gcc gtt ttg gcg cgt tgg gcc aag gtg ctg gaa    288
Asn Lys Phe Asp Lys Ala Val Leu Ala Arg Trp Ala Lys Val Leu Glu
                 85                  90                  95

atg atc att atg gcg gtg gcg gca tac ggg ttt tat atc cgg tct gcc    336

Met Ile Ile Met Ala Val Ala Ala Tyr Gly Phe Tyr Ile Arg Ser Ala
            100                 105                 110

ccg ctg ctt ttg gcg tgt ctg ttt tgc atg ggc gcg caa tcg acg ctg 384
Pro Leu Leu Leu Ala Cys Leu Phe Cys Met Gly Ala Gln Ser Thr Leu
        115                 120                 125

ttc ggg ccg ctg aaa tac gcc atc ctg ccc gat tat ctc gac gac aaa 432
Phe Gly Pro Leu Lys Tyr Ala Ile Leu Pro Asp Tyr Leu Asp Asp Lys
    130                 135                 140

gag ttg atg atg ggc aac agc ctg att gaa tcg ggt acg ttt gtc gcc 480
Glu Leu Met Met Gly Asn Ser Leu Ile Glu Ser Gly Thr Phe Val Ala
145                 150                 155                 160

atc ctg ttc ggt cag ata ctg ggg act gcg gtg gca ggt gta ccg cct 528
Ile Leu Phe Gly Gln Ile Leu Gly Thr Ala Val Ala Gly Val Pro Pro
                165                 170                 175

tat att gtc ggg ata ctg gtt ttg ctg gtc gcc gta gga ggc acg gtc 576
Tyr Ile Val Gly Ile Leu Val Leu Leu Val Ala Val Gly Gly Thr Val
            180                 185                 190

ggc agc ctg ttt atg ccg tcc gta ccc gcc aag gct gcc gat aca caa 624
Gly Ser Leu Phe Met Pro Ser Val Pro Ala Lys Ala Ala Asp Thr Gln
        195                 200                 205

att gag tgg aat att gtc cgg ggt aca aaa tcc ctg ctg cgt gaa acg 672
Ile Glu Trp Asn Ile Val Arg Gly Thr Lys Ser Leu Leu Arg Glu Thr
    210                 215                 220

gtg cgg cac aag ccc gtt ttt acc gcc att atc ggt att tcg tgg ttt 720
Val Arg His Lys Pro Val Phe Thr Ala Ile Ile Gly Ile Ser Trp Phe
225                 230                 235                 240

tgg ttt gtc ggc gcg gtt tat acc acg caa ctg ccg acc ttt acc caa 768
Trp Phe Val Gly Ala Val Tyr Thr Thr Gln Leu Pro Thr Phe Thr Gln
                245                 250                 255

atc cat cta ggc ggc aac gac aat gtt ttc aac ctg atg ctt gcc ctg 816
Ile His Leu Gly Gly Asn Asp Asn Val Phe Asn Leu Met Leu Ala Leu
            260                 265                 270

ttt tcc atc ggt att gcc gcc ggt tcg gta ctg tgt gcc aag ttc agc 864
Phe Ser Ile Gly Ile Ala Ala Gly Ser Val Leu Cys Ala Lys Phe Ser
        275                 280                 285

agg gaa cgg ctg agg ttg gct tgg gta acg gtt ggt gcg ttg ggt ttg 912

Arg Glu Arg Leu Arg Leu Ala Trp Val Thr Val Gly Ala Leu Gly Leu
    290                 295                 300

acg gtt tgc ggc ttg gtt ttg gtg tgg ctg acg cac gga cac cgt ttt    960
Thr Val Cys Gly Leu Val Leu Val Trp Leu Thr His Gly His Arg Phe
305                 310                 315                 320

gaa ggg ctg aac ggc att ttt tgg ttt tta tcg caa gga tgg gca tat    1008
Glu Gly Leu Asn Gly Ile Phe Trp Phe Leu Ser Gln Gly Trp Ala Tyr
                325                 330                 335

ccc gtg atg gcg gtg atg acg ctg atc ggc ttt ttc ggc gga ttt ttc    1056
Pro Val Met Ala Val Met Thr Leu Ile Gly Phe Phe Gly Gly Phe Phe
            340                 345                 350

tcc gtt ccg ctc tat acc tgg ctg caa acc gcc agt agc gag act ttc    1104
Ser Val Pro Leu Tyr Thr Trp Leu Gln Thr Ala Ser Ser Glu Thr Phe
        355                 360                 365

cgc gcc cgc gcc gtt gcc gcc aac aat atc gtt aac ggt att ttt atg    1152
Arg Ala Arg Ala Val Ala Ala Asn Asn Ile Val Asn Gly Ile Phe Met
    370                 375                 380

gtt tcc gct gcc gtt ttg agc gcg gtg ttg ctg ttt ttg ttt gac agc    1200
Val Ser Ala Ala Val Leu Ser Ala Val Leu Leu Phe Leu Phe Asp Ser
385                 390                 395                 400

att tcc ttg ttg tat ctg att gtc gct ttg ggc aat att ccg ttg tcg    1248
Ile Ser Leu Leu Tyr Leu Ile Val Ala Leu Gly Asn Ile Pro Leu Ser
                405                 410                 415

gta ttt ttg att aag cgc gaa agg cgg ttt tta ggc gcg gcg gca atc    1296
Val Phe Leu Ile Lys Arg Glu Arg Arg Phe Leu Gly Ala Ala Ala Ile
            420                 425                 430

agg aaa aaa cct                                                    1308
Arg Lys Lys Pro
        435

<210> 130
<211> 436
<212> PRT
<213> Neisseria meningitidis

<400> 130
Met Tyr Ala Lys Lys Gly Gly Leu Gly Leu Val Lys Ser Arg Arg Phe
  1               5                  10                  15

Ala Pro Leu Phe Ala Thr Gln Phe Leu Gly Ala Phe Asn Asp Asn Val
            20                  25                  30

Phe Lys Thr Ala Leu Phe Val Met Ile Gly Phe Tyr Gly Leu Gly Gln
        35                  40                  45

Asn Gly Phe Leu Pro Ala Gly Gln Met Leu Asn Leu Gly Ala Leu Leu
    50                  55                  60

Phe Ile Leu Pro Tyr Phe Leu Phe Ser Ser Leu Ser Gly Gln Leu Gly
65                  70                  75                  80

Asn Lys Phe Asp Lys Ala Val Leu Ala Arg Trp Ala Lys Val Leu Glu
                85                  90                  95

Met Ile Ile Met Ala Val Ala Ala Tyr Gly Phe Tyr Ile Arg Ser Ala
            100                 105                 110

Pro Leu Leu Leu Ala Cys Leu Phe Cys Met Gly Ala Gln Ser Thr Leu
        115                 120                 125

Phe Gly Pro Leu Lys Tyr Ala Ile Leu Pro Asp Tyr Leu Asp Asp Lys
    130                 135                 140

Glu Leu Met Met Gly Asn Ser Leu Ile Glu Ser Gly Thr Phe Val Ala
145                 150                 155                 160

Ile Leu Phe Gly Gln Ile Leu Gly Thr Ala Val Ala Gly Val Pro Pro
                165                 170                 175

Tyr Ile Val Gly Ile Leu Val Leu Leu Val Ala Val Gly Gly Thr Val
            180                 185                 190

Gly Ser Leu Phe Met Pro Ser Val Pro Ala Lys Ala Ala Asp Thr Gln
        195                 200                 205

Ile Glu Trp Asn Ile Val Arg Gly Thr Lys Ser Leu Leu Arg Glu Thr
    210                 215                 220

Val Arg His Lys Pro Val Phe Thr Ala Ile Ile Gly Ile Ser Trp Phe
225                 230                 235                 240

Trp Phe Val Gly Ala Val Tyr Thr Thr Gln Leu Pro Thr Phe Thr Gln
                245                 250                 255

Ile His Leu Gly Gly Asn Asp Asn Val Phe Asn Leu Met Leu Ala Leu
            260                 265                 270

Phe Ser Ile Gly Ile Ala Ala Gly Ser Val Leu Cys Ala Lys Phe Ser
        275                 280                 285

Arg Glu Arg Leu Arg Leu Ala Trp Val Thr Val Gly Ala Leu Gly Leu
    290                 295                 300

Thr Val Cys Gly Leu Val Leu Val Trp Leu Thr His Gly His Arg Phe
305                 310                 315                 320

Glu Gly Leu Asn Gly Ile Phe Trp Phe Leu Ser Gln Gly Trp Ala Tyr
                325                 330                 335

Pro Val Met Ala Val Met Thr Leu Ile Gly Phe Phe Gly Gly Phe Phe
            340                 345                 350

Ser Val Pro Leu Tyr Thr Trp Leu Gln Thr Ala Ser Ser Glu Thr Phe
        355                 360                 365

Arg Ala Arg Ala Val Ala Ala Asn Asn Ile Val Asn Gly Ile Phe Met
    370                 375                 380

Val Ser Ala Ala Val Leu Ser Ala Val Leu Leu Phe Leu Phe Asp Ser
385                 390                 395                 400

Ile Ser Leu Leu Tyr Leu Ile Val Ala Leu Gly Asn Ile Pro Leu Ser
                405                 410                 415

Val Phe Leu Ile Lys Arg Glu Arg Arg Phe Leu Gly Ala Ala Ala Ile
            420                 425                 430

Arg Lys Lys Pro
        435

<210> 131
<211> 876
<212> DNA
<213> Neisseria meningitidis

<220>
<221> CDS
<222> (1)..(876)

<400> 131
atg aat tac gca aaa gaa atc aat gcg tta aat aac agc ctt tcc gat    48
Met Asn Tyr Ala Lys Glu Ile Asn Ala Leu Asn Asn Ser Leu Ser Asp

1                 5                   10                  15

ttg aaa ggc gac atc aac gtt tcg ttt gaa ttt ttt cca ccg aaa aac    96
Leu Lys Gly Asp Ile Asn Val Ser Phe Glu Phe Phe Pro Pro Lys Asn
            20                  25                  30

gag caa atg gaa acg atg ctg tgg gat tcc atc cac cgt ctg caa acc    144
Glu Gln Met Glu Thr Met Leu Trp Asp Ser Ile His Arg Leu Gln Thr
        35                  40                  45

ctg cat ccc aag ttc gta tcc gta acc tac ggc gca aac tcc ggc gaa    192
Leu His Pro Lys Phe Val Ser Val Thr Tyr Gly Ala Asn Ser Gly Glu
    50                  55                  60

cgc gac cgc acg cac ggc atc gtc aaa cgc atc aaa cag gaa acc ggc    240
Arg Asp Arg Thr His Gly Ile Val Lys Arg Ile Lys Gln Glu Thr Gly
65                  70                  75                  80

ttg gaa gca gca ccg cac ctg acc ggc atc gac gca tcc ccc gac gaa    288
Leu Glu Ala Ala Pro His Leu Thr Gly Ile Asp Ala Ser Pro Asp Glu
                85                  90                  95

ttg cgc caa atc gcc aaa gac tat tgg gac agc ggc atc cgc cgc att    336
Leu Arg Gln Ile Ala Lys Asp Tyr Trp Asp Ser Gly Ile Arg Arg Ile
            100                 105                 110

gtc gcc ctg cgt ggc gac gag ccg ccc ggt tat gag aaa aaa ccg ttt    384
Val Ala Leu Arg Gly Asp Glu Pro Pro Gly Tyr Glu Lys Lys Pro Phe
        115                 120                 125

tac gcc gaa gac ttg gtt aag cta tta cgc tcc gtc gcc gac ttc gac    432
Tyr Ala Glu Asp Leu Val Lys Leu Leu Arg Ser Val Ala Asp Phe Asp
    130                 135                 140

atc tct gtg gcg gca tat ccc gaa gtg cat ccc gaa gcc aaa tcc gca    480
Ile Ser Val Ala Ala Tyr Pro Glu Val His Pro Glu Ala Lys Ser Ala
145                 150                 155                 160

caa gcc gat ctg att aat ctg aag cgc aaa atc gat gcg ggt gca aac    528
Gln Ala Asp Leu Ile Asn Leu Lys Arg Lys Ile Asp Ala Gly Ala Asn
                165                 170                 175

cac gtc atc acc caa ttt ttc ttt gac gta gaa cgc tac ctg cgc ttc    576
His Val Ile Thr Gln Phe Phe Phe Asp Val Glu Arg Tyr Leu Arg Phe
            180                 185                 190

cgc gac cgc tgc gtg atg ttg ggt atc gat gtg gaa atc gtc cct ggt    624
Arg Asp Arg Cys Val Met Leu Gly Ile Asp Val Glu Ile Val Pro Gly

195                 200                 205

att ttg cct gtt acc aac ttc aag cag ctc ggc aaa atg gcg caa gta     672
Ile Leu Pro Val Thr Asn Phe Lys Gln Leu Gly Lys Met Ala Gln Val
    210                 215                 220

acc aac gtc aaa atc cca agc tgg ctg tcg caa atg tat gaa ggt ttg     720
Thr Asn Val Lys Ile Pro Ser Trp Leu Ser Gln Met Tyr Glu Gly Leu
225                 230                 235                 240

gac gac gac caa ggc acg cgc aac ctc gtc gcc gcc agt atc gcc atc     768
Asp Asp Asp Gln Gly Thr Arg Asn Leu Val Ala Ala Ser Ile Ala Ile
                245                 250                 255

gat atg gtc aaa gtc ctg tcc cgc gaa ggc gtg aaa gat ttc cac ttc     816
Asp Met Val Lys Val Leu Ser Arg Glu Gly Val Lys Asp Phe His Phe
            260                 265                 270

tac acg ctc aac cgc agc gag ctg act tac gcc atc tgc cat att tta     864
Tyr Thr Leu Asn Arg Ser Glu Leu Thr Tyr Ala Ile Cys His Ile Leu
        275                 280                 285

ggc gtg cgc cct                                                     876
Gly Val Arg Pro
    290

<210> 132
<211> 292
<212> PRT
<213> Neisseria meningitidis

<400> 132
Met Asn Tyr Ala Lys Glu Ile Asn Ala Leu Asn Asn Ser Leu Ser Asp
1               5                   10                  15

Leu Lys Gly Asp Ile Asn Val Ser Phe Glu Phe Phe Pro Pro Lys Asn
            20                  25                  30

Glu Gln Met Glu Thr Met Leu Trp Asp Ser Ile His Arg Leu Gln Thr
        35                  40                  45

Leu His Pro Lys Phe Val Ser Val Thr Tyr Gly Ala Asn Ser Gly Glu
    50                  55                  60

Arg Asp Arg Thr His Gly Ile Val Lys Arg Ile Lys Gln Glu Thr Gly
65                  70                  75                  80

Leu Glu Ala Ala Pro His Leu Thr Gly Ile Asp Ala Ser Pro Asp Glu
                85                  90                  95

Leu Arg Gln Ile Ala Lys Asp Tyr Trp Asp Ser Gly Ile Arg Arg Ile
            100                 105                 110

Val Ala Leu Arg Gly Asp Glu Pro Pro Gly Tyr Glu Lys Lys Pro Phe
        115                 120                 125

Tyr Ala Glu Asp Leu Val Lys Leu Leu Arg Ser Val Ala Asp Phe Asp
    130                 135                 140

Ile Ser Val Ala Ala Tyr Pro Glu Val His Pro Glu Ala Lys Ser Ala
145                 150                 155                 160

Gln Ala Asp Leu Ile Asn Leu Lys Arg Lys Ile Asp Ala Gly Ala Asn
                165                 170                 175

His Val Ile Thr Gln Phe Phe Phe Asp Val Glu Arg Tyr Leu Arg Phe
            180                 185                 190

Arg Asp Arg Cys Val Met Leu Gly Ile Asp Val Glu Ile Val Pro Gly
        195                 200                 205

Ile Leu Pro Val Thr Asn Phe Lys Gln Leu Gly Lys Met Ala Gln Val
    210                 215                 220

Thr Asn Val Lys Ile Pro Ser Trp Leu Ser Gln Met Tyr Glu Gly Leu
225                 230                 235                 240

Asp Asp Asp Gln Gly Thr Arg Asn Leu Val Ala Ala Ser Ile Ala Ile
                245                 250                 255

Asp Met Val Lys Val Leu Ser Arg Glu Gly Val Lys Asp Phe His Phe
            260                 265                 270

Tyr Thr Leu Asn Arg Ser Glu Leu Thr Tyr Ala Ile Cys His Ile Leu
        275                 280                 285

Gly Val Arg Pro
    290

<210> 133
<211> 1371
<212> DNA
<213> Neisseria meningitidis

<220>
<221> CDS
<222> (1)..(1371)

<400> 133
atg agt aat cca ttt tcc tct tta ggt ttg ggt acg gaa ctc gtt tcc     48
Met Ser Asn Pro Phe Ser Ser Leu Gly Leu Gly Thr Glu Leu Val Ser
1               5                   10                  15

gca ctg acc gcg caa ggt tac gaa aac ccg aca ccc atc caa gcc gcc     96
Ala Leu Thr Ala Gln Gly Tyr Glu Asn Pro Thr Pro Ile Gln Ala Ala
            20                  25                  30

gcc att ccc aaa gca ctc gcc ggt cat gat ttg cta gcc gcc gcg caa     144
Ala Ile Pro Lys Ala Leu Ala Gly His Asp Leu Leu Ala Ala Ala Gln
        35                  40                  45

acc ggc aca ggc aaa acc gcc gcc ttt atg ctg ccc agt ctg gaa cgc     192
Thr Gly Thr Gly Lys Thr Ala Ala Phe Met Leu Pro Ser Leu Glu Arg
    50                  55                  60

ctc aaa cgt tac gcc acc gcc agc acc tcg ccc gcg atg cac ccc gtg     240
Leu Lys Arg Tyr Ala Thr Ala Ser Thr Ser Pro Ala Met His Pro Val
65                  70                  75                  80

cgt atg ctc gtc ctc acc ccc acg cgc gaa ctt gcc gac caa atc gac     288
Arg Met Leu Val Leu Thr Pro Thr Arg Glu Leu Ala Asp Gln Ile Asp
                85                  90                  95

caa aac gtg cag ggc tac atc aaa aac ctg ccg ctg cgc cac acc gtc     336
Gln Asn Val Gln Gly Tyr Ile Lys Asn Leu Pro Leu Arg His Thr Val
            100                 105                 110

ttg ttc ggc ggt atg aat atg gac aaa cag acc gcc gac ctg cgt gcc     384
Leu Phe Gly Gly Met Asn Met Asp Lys Gln Thr Ala Asp Leu Arg Ala
        115                 120                 125

ggc tgc gaa atc gtc gtc gcc acc gtc gga cgg ctg ctc gac cac gtg     432
Gly Cys Glu Ile Val Val Ala Thr Val Gly Arg Leu Leu Asp His Val
    130                 135                 140

aaa cag aaa aac atc cat ttg aac aaa gtc gaa atc gtc gtt ttg gac     480
Lys Gln Lys Asn Ile His Leu Asn Lys Val Glu Ile Val Val Leu Asp
145                 150                 155                 160

gaa gcc gac cgt atg ctg gat atg ggt ttt atc gac gac atc cgc aaa     528
Glu Ala Asp Arg Met Leu Asp Met Gly Phe Ile Asp Asp Ile Arg Lys

                165                 170                 175

atc atg cag atg ctg ccc cgc caa cgc caa acc ctg ctc ttt tcc gcc 576
Ile Met Gln Met Leu Pro Arg Gln Arg Gln Thr Leu Leu Phe Ser Ala
            180                 185                 190

acc ttc tcc gcc ccg ata cgc aaa ctg gcg caa gac ttc atg aac gcg 624
Thr Phe Ser Ala Pro Ile Arg Lys Leu Ala Gln Asp Phe Met Asn Ala
        195                 200                 205

ccc gaa acc gtc gaa gtc gcc gcg caa gac acc acc aac gcc aac gtc 672
Pro Glu Thr Val Glu Val Ala Ala Gln Asp Thr Thr Asn Ala Asn Val
    210                 215                 220

gag cag cac atc atc gcc gtc gat acc att cag aag cgc aac ctg ctc 720
Glu Gln His Ile Ile Ala Val Asp Thr Ile Gln Lys Arg Asn Leu Leu
225                 230                 235                 240

gaa cgg ctg att gtc gat ttg cat atg aac cag gtc atc gtg ttc tgc 768
Glu Arg Leu Ile Val Asp Leu His Met Asn Gln Val Ile Val Phe Cys
                245                 250                 255

aaa acc aaa caa agc gtc gac cgc gta acg cgc gaa ctg gtg cgc cgc 816
Lys Thr Lys Gln Ser Val Asp Arg Val Thr Arg Glu Leu Val Arg Arg
            260                 265                 270

aac ctg tcc gca cag gcg ata cac ggc gac cgt tcc caa caa agc cgg 864
Asn Leu Ser Ala Gln Ala Ile His Gly Asp Arg Ser Gln Gln Ser Arg
        275                 280                 285

ctc gaa aca ctc aac gcc ttc aaa gac ggc aac ctg cgc gtc ctc gtc 912
Leu Glu Thr Leu Asn Ala Phe Lys Asp Gly Asn Leu Arg Val Leu Val
    290                 295                 300

gcc acc gat atc gcc gcg cgg ggg ctg gat att gcc gaa ctg ccc ttc 960
Ala Thr Asp Ile Ala Ala Arg Gly Leu Asp Ile Ala Glu Leu Pro Phe
305                 310                 315                 320

gtc atc aat tac gaa atg ccc gcc cag ccc gaa gac tac gtc cac cgc 1008
Val Ile Asn Tyr Glu Met Pro Ala Gln Pro Glu Asp Tyr Val His Arg
                325                 330                 335

atc ggg cgc acg ggg cgc gcg ggc gcg gac ggc gtg gcg att tcc ctg 1056
Ile Gly Arg Thr Gly Arg Ala Gly Ala Asp Gly Val Ala Ile Ser Leu
            340                 345                 350

atg gac gaa tcc gaa cag aaa atg ttt gaa tcc att aaa gag ctg acc 1104
Met Asp Glu Ser Glu Gln Lys Met Phe Glu Ser Ile Lys Glu Leu Thr

355                 360                 365

ggc aac aag ctg ctc atc gag cgc atc gag ggc ttc gag ccg caa tgg     1152
Gly Asn Lys Leu Leu Ile Glu Arg Ile Glu Gly Phe Glu Pro Gln Trp
    370                 375                 380

tgg gaa cag ggc ggc gca aaa ccg gaa aaa ccc gaa atg cgc gaa cca     1200
Trp Glu Gln Gly Gly Ala Lys Pro Glu Lys Pro Glu Met Arg Glu Pro
385                 390                 395                 400

aga caa cgc aac cgc tac gaa tcc gcc aaa gcg caa cgc gaa aaa aac     1248
Arg Gln Arg Asn Arg Tyr Glu Ser Ala Lys Ala Gln Arg Glu Lys Asn
                405                 410                 415

acc cgg ccg gaa aat gcg gca aac gat gcg ggc gcg gct tgc gga aaa     1296
Thr Arg Pro Glu Asn Ala Ala Asn Asp Ala Gly Ala Ala Cys Gly Lys
            420                 425                 430

att gcc gga cgc agc cgc cga agc cgc cgg gaa cac cgg acg tgc gcc     1344
Ile Ala Gly Arg Ser Arg Arg Ser Arg Arg Glu His Arg Thr Cys Ala
        435                 440                 445

ctg ctc caa ccg cgt tac ggc gta aaa                                 1371
Leu Leu Gln Pro Arg Tyr Gly Val Lys
    450                 455

<210> 134
<211> 457
<212> PRT
<213> Neisseria meningitidis

<400> 134
Met Ser Asn Pro Phe Ser Ser Leu Gly Leu Gly Thr Glu Leu Val Ser
1               5                   10                  15

Ala Leu Thr Ala Gln Gly Tyr Glu Asn Pro Thr Pro Ile Gln Ala Ala
            20                  25                  30

Ala Ile Pro Lys Ala Leu Ala Gly His Asp Leu Leu Ala Ala Ala Gln
        35                  40                  45

Thr Gly Thr Gly Lys Thr Ala Ala Phe Met Leu Pro Ser Leu Glu Arg
    50                  55                  60

Leu Lys Arg Tyr Ala Thr Ala Ser Thr Ser Pro Ala Met His Pro Val
65                  70                  75                  80

Arg Met Leu Val Leu Thr Pro Thr Arg Glu Leu Ala Asp Gln Ile Asp
                85                  90                  95

Gln Asn Val Gln Gly Tyr Ile Lys Asn Leu Pro Leu Arg His Thr Val
            100                 105                 110

Leu Phe Gly Gly Met Asn Met Asp Lys Gln Thr Ala Asp Leu Arg Ala
        115                 120                 125

Gly Cys Glu Ile Val Val Ala Thr Val Gly Arg Leu Leu Asp His Val
    130                 135                 140

Lys Gln Lys Asn Ile His Leu Asn Lys Val Glu Ile Val Val Leu Asp
145                 150                 155                 160

Glu Ala Asp Arg Met Leu Asp Met Gly Phe Ile Asp Asp Ile Arg Lys
                165                 170                 175

Ile Met Gln Met Leu Pro Arg Gln Arg Gln Thr Leu Leu Phe Ser Ala
            180                 185                 190

Thr Phe Ser Ala Pro Ile Arg Lys Leu Ala Gln Asp Phe Met Asn Ala
        195                 200                 205

Pro Glu Thr Val Glu Val Ala Ala Gln Asp Thr Thr Asn Ala Asn Val
    210                 215                 220

Glu Gln His Ile Ile Ala Val Asp Thr Ile Gln Lys Arg Asn Leu Leu
225                 230                 235                 240

Glu Arg Leu Ile Val Asp Leu His Met Asn Gln Val Ile Val Phe Cys
                245                 250                 255

Lys Thr Lys Gln Ser Val Asp Arg Val Thr Arg Glu Leu Val Arg Arg
            260                 265                 270

Asn Leu Ser Ala Gln Ala Ile His Gly Asp Arg Ser Gln Gln Ser Arg
        275                 280                 285

Leu Glu Thr Leu Asn Ala Phe Lys Asp Gly Asn Leu Arg Val Leu Val
    290                 295                 300

Ala Thr Asp Ile Ala Ala Arg Gly Leu Asp Ile Ala Glu Leu Pro Phe
305                 310                 315                 320

Val Ile Asn Tyr Glu Met Pro Ala Gln Pro Glu Asp Tyr Val His Arg
                325                 330                 335

Ile Gly Arg Thr Gly Arg Ala Gly Ala Asp Gly Val Ala Ile Ser Leu
            340                 345                 350

Met Asp Glu Ser Glu Gln Lys Met Phe Glu Ser Ile Lys Glu Leu Thr
        355                 360                 365

Gly Asn Lys Leu Leu Ile Glu Arg Ile Glu Gly Phe Glu Pro Gln Trp
    370                 375                 380

Trp Glu Gln Gly Gly Ala Lys Pro Glu Lys Pro Glu Met Arg Glu Pro
385                 390                 395                 400

Arg Gln Arg Asn Arg Tyr Glu Ser Ala Lys Ala Gln Arg Glu Lys Asn
                405                 410                 415

Thr Arg Pro Glu Asn Ala Ala Asn Asp Ala Gly Ala Ala Cys Gly Lys
            420                 425                 430

Ile Ala Gly Arg Ser Arg Arg Ser Arg Arg Glu His Arg Thr Cys Ala
        435                 440                 445

Leu Leu Gln Pro Arg Tyr Gly Val Lys
    450                 455

<210> 135
<211> 1374
<212> DNA
<213> Neisseria meningitidis

<220>
<221> CDS
<222> (1)..(1374)

<400> 135
atg aaa cac atc cac att atc ggt atc ggc ggc acg ttt atg ggt ggg      48
Met Lys His Ile His Ile Ile Gly Ile Gly Gly Thr Phe Met Gly Gly
  1               5                  10                  15

att gcc gcc att gcc aaa gaa gca ggg ttt gaa gtc agc ggt tgc gat      96
Ile Ala Ala Ile Ala Lys Glu Ala Gly Phe Glu Val Ser Gly Cys Asp
             20                  25                  30

gcg aag atg tat ccg ccg atg agc acc cag ctc gaa gcc ttg ggc ata     144
Ala Lys Met Tyr Pro Pro Met Ser Thr Gln Leu Glu Ala Leu Gly Ile
         35                  40                  45

ggc gtg tat gaa ggc ttc gac acc gcg cag ttg gac gaa ttt aaa gcc 192
Gly Val Tyr Glu Gly Phe Asp Thr Ala Gln Leu Asp Glu Phe Lys Ala
    50                  55                  60

gac gtt tac gtt atc ggc aat gtc gcc aag cgc ggg atg gat gtg gtt 240
Asp Val Tyr Val Ile Gly Asn Val Ala Lys Arg Gly Met Asp Val Val
65                  70                  75                  80

gaa gcg att ttg aac cgt ggg ctg cct tat att tcc ggc ccg caa tgg 288
Glu Ala Ile Leu Asn Arg Gly Leu Pro Tyr Ile Ser Gly Pro Gln Trp
                85                  90                  95

ctg gct gaa aac gtg ctg cac cat cat tgg gta ctc ggc gtg gcg ggg 336
Leu Ala Glu Asn Val Leu His His His Trp Val Leu Gly Val Ala Gly
            100                 105                 110

acg cac ggc aaa acg acc acc gcg tct atg ctc gcg tgg gtt ttg gaa 384
Thr His Gly Lys Thr Thr Thr Ala Ser Met Leu Ala Trp Val Leu Glu
        115                 120                 125

tat gcc gga ctc gca ccg ggc ttc ctt atc ggc ggc gta ccg gaa aac 432
Tyr Ala Gly Leu Ala Pro Gly Phe Leu Ile Gly Gly Val Pro Glu Asn
    130                 135                 140

ttc agc gtt tcc gcc cgc ctg ccg caa acg ccg cgc caa gac ccg aac 480
Phe Ser Val Ser Ala Arg Leu Pro Gln Thr Pro Arg Gln Asp Pro Asn
145                 150                 155                 160

agc caa tcg ccg ttt ttc gtc att gaa gcc gac gaa tac gac acc gcg 528
Ser Gln Ser Pro Phe Phe Val Ile Glu Ala Asp Glu Tyr Asp Thr Ala
                165                 170                 175

ttt ttc gac aaa cgc tcc aaa ttc gtg cat tac cgt ccg cgt acc gcc 576
Phe Phe Asp Lys Arg Ser Lys Phe Val His Tyr Arg Pro Arg Thr Ala
            180                 185                 190

gtg ttg aac aat ctg gaa ttc gac cac gcc gac atc ttc gcc gat ttg 624
Val Leu Asn Asn Leu Glu Phe Asp His Ala Asp Ile Phe Ala Asp Leu
        195                 200                 205

ggc gcg ata cag acc cag ttc cac cac ctc gtg cgt acc gtg ccg tct 672
Gly Ala Ile Gln Thr Gln Phe His His Leu Val Arg Thr Val Pro Ser
    210                 215                 220

gaa ggc ctc atc gtc tgc aac gga cgg cag caa agc ctg caa gac act 720
Glu Gly Leu Ile Val Cys Asn Gly Arg Gln Gln Ser Leu Gln Asp Thr
225                 230                 235                 240

ttg gac aaa ggc tgc tgg acg ccg gtg gaa aaa ttc ggc acg gaa cac 768
Leu Asp Lys Gly Cys Trp Thr Pro Val Glu Lys Phe Gly Thr Glu His
                245                 250                 255

ggc tgg cag gcc ggc gaa gcc aat gcc gat ggc tcg ttc gac gtg ttg 816
Gly Trp Gln Ala Gly Glu Ala Asn Ala Asp Gly Ser Phe Asp Val Leu
            260                 265                 270

ctt gac ggc aaa aaa gcc gga cac gtc gct tgg agt ttg atg ggc gga 864
Leu Asp Gly Lys Lys Ala Gly His Val Ala Trp Ser Leu Met Gly Gly
        275                 280                 285

cac aac cgc atg aac gcg ctc gcc gtc atc gcc gcc gcg cgt cat gcc 912
His Asn Arg Met Asn Ala Leu Ala Val Ile Ala Ala Ala Arg His Ala
    290                 295                 300

gga gtc gac att cag acg gcc tgc gaa gcc ttg agc acg ttt aaa aac 960
Gly Val Asp Ile Gln Thr Ala Cys Glu Ala Leu Ser Thr Phe Lys Asn
305                 310                 315                 320

gtc aaa cgc cgc atg gaa atc aaa ggc acg gca aac ggt atc acc gtt 1008
Val Lys Arg Arg Met Glu Ile Lys Gly Thr Ala Asn Gly Ile Thr Val
                325                 330                 335

tac gac gac ttc gcc cac cat ccg acc gct atc gaa acc acg att caa 1056
Tyr Asp Asp Phe Ala His His Pro Thr Ala Ile Glu Thr Thr Ile Gln
            340                 345                 350

ggt ttg cgc cag cgc gtc ggc ggc gcg cgc atc ctc gcc gtc ctc gaa 1104
Gly Leu Arg Gln Arg Val Gly Gly Ala Arg Ile Leu Ala Val Leu Glu
        355                 360                 365

ccg cgt tcc aat acg atg aag ctg ggt acg atg aaa gcc gcc ctg ccc 1152
Pro Arg Ser Asn Thr Met Lys Leu Gly Thr Met Lys Ala Ala Leu Pro
    370                 375                 380

gca agc ctc aaa gaa gcc gac caa gtg ttc tgc tac gcc ggc ggc gcg 1200
Ala Ser Leu Lys Glu Ala Asp Gln Val Phe Cys Tyr Ala Gly Gly Ala
385                 390                 395                 400

gac tgg gac gtt gcc gaa gcc ctc gcg cct ttg ggc ggc agg ctg cac 1248
Asp Trp Asp Val Ala Glu Ala Leu Ala Pro Leu Gly Gly Arg Leu His
                405                 410                 415

gtc ggc aaa gac ttc gat gcc ttc gtt gcc gaa atc gtg aaa aac gcc 1296
Val Gly Lys Asp Phe Asp Ala Phe Val Ala Glu Ile Val Lys Asn Ala
            420                 425                 430

gaa gca ggc gac cat att ttg gtg atg agc aac ggc ggt ttc ggc gga    1344
Glu Ala Gly Asp His Ile Leu Val Met Ser Asn Gly Gly Phe Gly Gly
        435                 440                 445

ata cac acc aaa ctg ctg gac gct ttg aga                            1374
Ile His Thr Lys Leu Leu Asp Ala Leu Arg
    450                 455

<210> 136
<211> 458
<212> PRT
<213> Neisseria meningitidis

<400> 136
Met Lys His Ile His Ile Ile Gly Ile Gly Gly Thr Phe Met Gly Gly
1               5                   10                  15

Ile Ala Ala Ile Ala Lys Glu Ala Gly Phe Glu Val Ser Gly Cys Asp
            20                  25                  30

Ala Lys Met Tyr Pro Pro Met Ser Thr Gln Leu Glu Ala Leu Gly Ile
        35                  40                  45

Gly Val Tyr Glu Gly Phe Asp Thr Ala Gln Leu Asp Glu Phe Lys Ala
    50                  55                  60

Asp Val Tyr Val Ile Gly Asn Val Ala Lys Arg Gly Met Asp Val Val
65                  70                  75                  80

Glu Ala Ile Leu Asn Arg Gly Leu Pro Tyr Ile Ser Gly Pro Gln Trp
                85                  90                  95

Leu Ala Glu Asn Val Leu His His His Trp Val Leu Gly Val Ala Gly
            100                 105                 110

Thr His Gly Lys Thr Thr Thr Ala Ser Met Leu Ala Trp Val Leu Glu
        115                 120                 125

Tyr Ala Gly Leu Ala Pro Gly Phe Leu Ile Gly Gly Val Pro Glu Asn
    130                 135                 140

Phe Ser Val Ser Ala Arg Leu Pro Gln Thr Pro Arg Gln Asp Pro Asn
145                 150                 155                 160

Ser Gln Ser Pro Phe Phe Val Ile Glu Ala Asp Glu Tyr Asp Thr Ala
                165                 170                 175

Phe Phe Asp Lys Arg Ser Lys Phe Val His Tyr Arg Pro Arg Thr Ala
            180                 185                 190

Val Leu Asn Asn Leu Glu Phe Asp His Ala Asp Ile Phe Ala Asp Leu
        195                 200                 205

Gly Ala Ile Gln Thr Gln Phe His His Leu Val Arg Thr Val Pro Ser
    210                 215                 220

Glu Gly Leu Ile Val Cys Asn Gly Arg Gln Gln Ser Leu Gln Asp Thr
225                 230                 235                 240

Leu Asp Lys Gly Cys Trp Thr Pro Val Glu Lys Phe Gly Thr Glu His
                245                 250                 255

Gly Trp Gln Ala Gly Glu Ala Asn Ala Asp Gly Ser Phe Asp Val Leu
            260                 265                 270

Leu Asp Gly Lys Lys Ala Gly His Val Ala Trp Ser Leu Met Gly Gly
        275                 280                 285

His Asn Arg Met Asn Ala Leu Ala Val Ile Ala Ala Ala Arg His Ala
    290                 295                 300

Gly Val Asp Ile Gln Thr Ala Cys Glu Ala Leu Ser Thr Phe Lys Asn
305                 310                 315                 320

Val Lys Arg Arg Met Glu Ile Lys Gly Thr Ala Asn Gly Ile Thr Val
                325                 330                 335

Tyr Asp Asp Phe Ala His His Pro Thr Ala Ile Glu Thr Thr Ile Gln
            340                 345                 350

Gly Leu Arg Gln Arg Val Gly Gly Ala Arg Ile Leu Ala Val Leu Glu
        355                 360                 365

Pro Arg Ser Asn Thr Met Lys Leu Gly Thr Met Lys Ala Ala Leu Pro
    370                 375                 380

Ala Ser Leu Lys Glu Ala Asp Gln Val Phe Cys Tyr Ala Gly Gly Ala
385                 390                 395                 400

Asp Trp Asp Val Ala Glu Ala Leu Ala Pro Leu Gly Gly Arg Leu His
                405                 410                 415

Val Gly Lys Asp Phe Asp Ala Phe Val Ala Glu Ile Val Lys Asn Ala
            420                 425                 430

Glu Ala Gly Asp His Ile Leu Val Met Ser Asn Gly Gly Phe Gly Gly
        435                 440                 445

Ile His Thr Lys Leu Leu Asp Ala Leu Arg
    450                 455

<210> 137
<211> 876
<212> DNA
<213> Neisseria meningitidis

<220>
<221> CDS
<222> (1)..(876)

<400> 137
ttg acc gtc cga acg aaa aag acg gcg cat tat acc cta ttc cat tcc      48
Leu Thr Val Arg Thr Lys Lys Thr Ala His Tyr Thr Leu Phe His Ser
  1               5                  10                  15

gac cga aaa ccg aac atg act act ctc aaa ccc gcc ctg ccc gct tat      96
Asp Arg Lys Pro Asn Met Thr Thr Leu Lys Pro Ala Leu Pro Ala Tyr
             20                  25                  30

ctg gac aac atc cgc atc atc ctc acg cgc acc agc cat ccc gcc aac     144
Leu Asp Asn Ile Arg Ile Ile Leu Thr Arg Thr Ser His Pro Ala Asn
         35                  40                  45

atc ggc tct gcc gcg cgc gcg atg aaa aca atg ggt ctg cac aaa ctg     192
Ile Gly Ser Ala Ala Arg Ala Met Lys Thr Met Gly Leu His Lys Leu
     50                  55                  60

acc atc gtc gcc cca aat ctg atg gca acg ccg atg acg gaa aac ccg     240
Thr Ile Val Ala Pro Asn Leu Met Ala Thr Pro Met Thr Glu Asn Pro
 65                  70                  75                  80

ccc gtg ttt gac ccg gag cat cct caa tcg ttt aaa tta ccg gaa gaa     288
Pro Val Phe Asp Pro Glu His Pro Gln Ser Phe Lys Leu Pro Glu Glu
                 85                  90                  95

agc ttc atc ctc gct tcc ggc gcg gca gac gtt ttg gaa aat gcc acc     336
Ser Phe Ile Leu Ala Ser Gly Ala Ala Asp Val Leu Glu Asn Ala Thr
            100                 105                 110

att gcc gct tct ttg gac gaa gcc ctt gcc gac acc acc atc gcc tgc     384
Ile Ala Ala Ser Leu Asp Glu Ala Leu Ala Asp Thr Thr Ile Ala Cys

        115                 120                 125

gcc ctg acc agc cgc cgc cgc gaa att act gcg ccg ctg caa acc ccg 432
Ala Leu Thr Ser Arg Arg Arg Glu Ile Thr Ala Pro Leu Gln Thr Pro
    130                 135                 140

cgc gat ttg gta tcc gaa tta ctg cag acc gca aac cga ggc gag aaa 480
Arg Asp Leu Val Ser Glu Leu Leu Gln Thr Ala Asn Arg Gly Glu Lys
145                 150                 155                 160

gtg gca ctg gtt ttc ggc aac gag act ttc ggc ttg agc atc gaa gaa 528
Val Ala Leu Val Phe Gly Asn Glu Thr Phe Gly Leu Ser Ile Glu Glu
                165                 170                 175

gtc caa gcc tgc aac cga ctg atg acc atc aac ggc aat ccc gac tat 576
Val Gln Ala Cys Asn Arg Leu Met Thr Ile Asn Gly Asn Pro Asp Tyr
            180                 185                 190

ttc tcg ctc aac ctc gcc caa gcc gtg cag gtc gtg tgc tac gaa atc 624
Phe Ser Leu Asn Leu Ala Gln Ala Val Gln Val Val Cys Tyr Glu Ile
        195                 200                 205

ttc agc caa acc ggt tcg ccc atg acc cat ctt caa caa gaa gac cac 672
Phe Ser Gln Thr Gly Ser Pro Met Thr His Leu Gln Gln Glu Asp His
    210                 215                 220

gct gcg acc cac gag caa atc aaa ggc atg gtc gcc cac atg gaa agc 720
Ala Ala Thr His Glu Gln Ile Lys Gly Met Val Ala His Met Glu Ser
225                 230                 235                 240

gtg atg aac gac atc ggc ttt ttc aac cgc cgc aac ggc gag cgt ctg 768
Val Met Asn Asp Ile Gly Phe Phe Asn Arg Arg Asn Gly Glu Arg Leu
                245                 250                 255

atg cgc cgt atg cag agc ctg ttc gga cgc gcc aac acg caa acc gaa 816
Met Arg Arg Met Gln Ser Leu Phe Gly Arg Ala Asn Thr Gln Thr Glu
            260                 265                 270

gac atc gat atc ctg cgc ggt ttt ttc aat acc gtc agc cat cgt atc 864
Asp Ile Asp Ile Leu Arg Gly Phe Phe Asn Thr Val Ser His Arg Ile
        275                 280                 285

cat aaa aaa gac 876
His Lys Lys Asp
    290

<210> 138

<211> 292
<212> PRT
<213> Neisseria meningitidis

<400> 138
Leu Thr Val Arg Thr Lys Lys Thr Ala His Tyr Thr Leu Phe His Ser
1               5                   10                  15

Asp Arg Lys Pro Asn Met Thr Thr Leu Lys Pro Ala Leu Pro Ala Tyr
            20                  25                  30

Leu Asp Asn Ile Arg Ile Ile Leu Thr Arg Thr Ser His Pro Ala Asn
        35                  40                  45

Ile Gly Ser Ala Ala Arg Ala Met Lys Thr Met Gly Leu His Lys Leu
    50                  55                  60

Thr Ile Val Ala Pro Asn Leu Met Ala Thr Pro Met Thr Glu Asn Pro
65                  70                  75                  80

Pro Val Phe Asp Pro Glu His Pro Gln Ser Phe Lys Leu Pro Glu Glu
                85                  90                  95

Ser Phe Ile Leu Ala Ser Gly Ala Ala Asp Val Leu Glu Asn Ala Thr
            100                 105                 110

Ile Ala Ala Ser Leu Asp Glu Ala Leu Ala Asp Thr Thr Ile Ala Cys
        115                 120                 125

Ala Leu Thr Ser Arg Arg Arg Glu Ile Thr Ala Pro Leu Gln Thr Pro
    130                 135                 140

Arg Asp Leu Val Ser Glu Leu Leu Gln Thr Ala Asn Arg Gly Glu Lys
145                 150                 155                 160

Val Ala Leu Val Phe Gly Asn Glu Thr Phe Gly Leu Ser Ile Glu Glu
                165                 170                 175

Val Gln Ala Cys Asn Arg Leu Met Thr Ile Asn Gly Asn Pro Asp Tyr
            180                 185                 190

Phe Ser Leu Asn Leu Ala Gln Ala Val Gln Val Val Cys Tyr Glu Ile
        195                 200                 205

Phe Ser Gln Thr Gly Ser Pro Met Thr His Leu Gln Gln Glu Asp His
    210                 215                 220

Ala Ala Thr His Glu Gln Ile Lys Gly Met Val Ala His Met Glu Ser

225                 230                 235                 240

Val Met Asn Asp Ile Gly Phe Phe Asn Arg Arg Asn Gly Glu Arg Leu
                245                 250                 255

Met Arg Arg Met Gln Ser Leu Phe Gly Arg Ala Asn Thr Gln Thr Glu
            260                 265                 270

Asp Ile Asp Ile Leu Arg Gly Phe Phe Asn Thr Val Ser His Arg Ile
        275                 280                 285

His Lys Lys Asp
    290

<210> 139
<211> 708
<212> DNA
<213> Neisseria meningitidis

<220>
<221> CDS
<222> (1)..(708)

<400> 139
ttg cgg cag ctt gga tgt ttg att ggt ttt ttc agt gtt gga atc att     48
Leu Arg Gln Leu Gly Cys Leu Ile Gly Phe Phe Ser Val Gly Ile Ile
  1               5                  10                  15

atg aat ttg aaa tta gtg ttt gaa tcg ggc gat ccc gtc ctg atc ggt     96
Met Asn Leu Lys Leu Val Phe Glu Ser Gly Asp Pro Val Leu Ile Gly
             20                  25                  30

gtg ttt gtg ttg atg ctg ttg atg agt atc gta acg tgg tgt ttg gtt    144
Val Phe Val Leu Met Leu Leu Met Ser Ile Val Thr Trp Cys Leu Val
         35                  40                  45

gtc ttg cgc tgc atc aag ctg tat cgg gcg cgc aaa ggg aat gcc gcc    192
Val Leu Arg Cys Ile Lys Leu Tyr Arg Ala Arg Lys Gly Asn Ala Ala
     50                  55                  60

gtc aaa cgg cat atg cgc gat act ttg tcg ctg aac gac gcg gtc gaa    240
Val Lys Arg His Met Arg Asp Thr Leu Ser Leu Asn Asp Ala Val Glu
 65                  70                  75                  80

aaa gtg cgc gcc gtc gat gcg cct ttg tcc aaa ctg gcg caa gag gca    288
Lys Val Arg Ala Val Asp Ala Pro Leu Ser Lys Leu Ala Gln Glu Ala

                85                  90                  95

ttg cag tct tac cgc aac tac cgc cga aac gaa gcg tcc gaa ctg gcg 336
Leu Gln Ser Tyr Arg Asn Tyr Arg Arg Asn Glu Ala Ser Glu Leu Ala
            100                 105                 110

cag gct ttg ccg ttg aac gag tat ttg gtc att caa atc cgc aac agt 384
Gln Ala Leu Pro Leu Asn Glu Tyr Leu Val Ile Gln Ile Arg Asn Ser
        115                 120                 125

atg gcg cag att atg cgc cgg ttt gat tac ggg atg acc gcg ctt gcc 432
Met Ala Gln Ile Met Arg Arg Phe Asp Tyr Gly Met Thr Ala Leu Ala
    130                 135                 140

tcc atc ggc gcg acc gcg ccg ttt atc ggg ttg ttc ggc acg gtt tgg 480
Ser Ile Gly Ala Thr Ala Pro Phe Ile Gly Leu Phe Gly Thr Val Trp
145                 150                 155                 160

ggg att tac cac gcc ctg atc aat atc ggg caa agc ggg cag atg agt 528
Gly Ile Tyr His Ala Leu Ile Asn Ile Gly Gln Ser Gly Gln Met Ser
                165                 170                 175

att gcg gcg gtt gcc ggc ccg att ggc gag gca ctg gtg gcg acg gcg 576
Ile Ala Ala Val Ala Gly Pro Ile Gly Glu Ala Leu Val Ala Thr Ala
            180                 185                 190

gcg ggt ttg ttc gtg gcg att ccg gcg gtg ttg gca tac aac ttc ctc 624
Ala Gly Leu Phe Val Ala Ile Pro Ala Val Leu Ala Tyr Asn Phe Leu
        195                 200                 205

aat cgc ggc aca aaa ata ctg acc cag gat ttg gat gcg atg gcg cac 672
Asn Arg Gly Thr Lys Ile Leu Thr Gln Asp Leu Asp Ala Met Ala His
    210                 215                 220

gat ttg cac gtc cgc ctg ctt aat caa aag gat agc 708
Asp Leu His Val Arg Leu Leu Asn Gln Lys Asp Ser
225                 230                 235

<210> 140
<211> 236
<212> PRT
<213> Neisseria meningitidis

<400> 140
Leu Arg Gln Leu Gly Cys Leu Ile Gly Phe Phe Ser Val Gly Ile Ile
1               5                   10                  15

Met Asn Leu Lys Leu Val Phe Glu Ser Gly Asp Pro Val Leu Ile Gly
            20                  25                  30

Val Phe Val Leu Met Leu Leu Met Ser Ile Val Thr Trp Cys Leu Val
        35                  40                  45

Val Leu Arg Cys Ile Lys Leu Tyr Arg Ala Arg Lys Gly Asn Ala Ala
    50                  55                  60

Val Lys Arg His Met Arg Asp Thr Leu Ser Leu Asn Asp Ala Val Glu
65                  70                  75                  80

Lys Val Arg Ala Val Asp Ala Pro Leu Ser Lys Leu Ala Gln Glu Ala
                85                  90                  95

Leu Gln Ser Tyr Arg Asn Tyr Arg Arg Asn Glu Ala Ser Glu Leu Ala
            100                 105                 110

Gln Ala Leu Pro Leu Asn Glu Tyr Leu Val Ile Gln Ile Arg Asn Ser
        115                 120                 125

Met Ala Gln Ile Met Arg Arg Phe Asp Tyr Gly Met Thr Ala Leu Ala
    130                 135                 140

Ser Ile Gly Ala Thr Ala Pro Phe Ile Gly Leu Phe Gly Thr Val Trp
145                 150                 155                 160

Gly Ile Tyr His Ala Leu Ile Asn Ile Gly Gln Ser Gly Gln Met Ser
                165                 170                 175

Ile Ala Ala Val Ala Gly Pro Ile Gly Glu Ala Leu Val Ala Thr Ala
            180                 185                 190

Ala Gly Leu Phe Val Ala Ile Pro Ala Val Leu Ala Tyr Asn Phe Leu
        195                 200                 205

Asn Arg Gly Thr Lys Ile Leu Thr Gln Asp Leu Asp Ala Met Ala His
    210                 215                 220

Asp Leu His Val Arg Leu Leu Asn Gln Lys Asp Ser
225                 230                 235

<210> 141
<211> 812
<212> DNA
<213> Neisseria meningitidis

<220>
<221> CDS
<222> (1)..(810)

<400> 141
atg act atg cac gcc ctc ccc cgc tac gcc gtt ttt ggc aac ccc gtc     48
Met Thr Met His Ala Leu Pro Arg Tyr Ala Val Phe Gly Asn Pro Val
1               5                   10                  15

gcc cac agc aaa tcg ccg caa att cat caa caa ttt gcc ctt cag gaa     96
Ala His Ser Lys Ser Pro Gln Ile His Gln Gln Phe Ala Leu Gln Glu
            20                  25                  30

ggc gtt gac att gaa tac gaa cgc att tgc gcc gac atc ggc ggt ttc     144
Gly Val Asp Ile Glu Tyr Glu Arg Ile Cys Ala Asp Ile Gly Gly Phe
        35                  40                  45

gcg cag gcg gtt tcg aca ttt ttt gaa aca ggc ggt tgc ggg gca aac     192
Ala Gln Ala Val Ser Thr Phe Phe Glu Thr Gly Gly Cys Gly Ala Asn
    50                  55                  60

gtt acc gta ccg ttc aag cag gaa gcg ttt cat ctg gcg gac gag cat     240
Val Thr Val Pro Phe Lys Gln Glu Ala Phe His Leu Ala Asp Glu His
65                  70                  75                  80

tct gaa cgc gca ttg gct gcc ggc gcg gtc aac acg ctg att ttt ctg     288
Ser Glu Arg Ala Leu Ala Ala Gly Ala Val Asn Thr Leu Ile Phe Leu
                85                  90                  95

aaa aac gga aaa ctg cgc ggc gac aat acc gac ggt atc ggt ttg gcc     336
Lys Asn Gly Lys Leu Arg Gly Asp Asn Thr Asp Gly Ile Gly Leu Ala
            100                 105                 110

aac gac atc acg cag gtc aaa aac att gcc atc gaa ggc aaa acc atc     384
Asn Asp Ile Thr Gln Val Lys Asn Ile Ala Ile Glu Gly Lys Thr Ile
        115                 120                 125

ttg ctt ttg ggc gcg ggc ggc gcg gtg cgc ggc gtg att cct gtt ttg     432
Leu Leu Leu Gly Ala Gly Gly Ala Val Arg Gly Val Ile Pro Val Leu
    130                 135                 140

aaa gaa cac cgt cct gcc cgt atc gtc att gcc aac cgt acc cgc gcc     480
Lys Glu His Arg Pro Ala Arg Ile Val Ile Ala Asn Arg Thr Arg Ala
145                 150                 155                 160

aaa gcc gag gaa ttg gcg cag ctt ttc ggc att gaa gcc gtc ccg atg     528
Lys Ala Glu Glu Leu Ala Gln Leu Phe Gly Ile Glu Ala Val Pro Met

                165                 170                 175

gcg gat gtg aac ggc ggt ttt gat atc atc atc aac ggc acg tcg ggc 576
Ala Asp Val Asn Gly Gly Phe Asp Ile Ile Ile Asn Gly Thr Ser Gly
            180                 185                 190

ggt cta aac ggt cag att ccc gat att ccg ccc gat att ttt caa aac 624
Gly Leu Asn Gly Gln Ile Pro Asp Ile Pro Pro Asp Ile Phe Gln Asn
        195                 200                 205

tgc gcg ctt gcc tac gat atg gtg tac ggc tgc gcg gca aaa ccg ttt 672
Cys Ala Leu Ala Tyr Asp Met Val Tyr Gly Cys Ala Ala Lys Pro Phe
    210                 215                 220

tta gat ttt gca cga caa tcg ggt gcg aaa aaa act gcc gac gga ctg 720
Leu Asp Phe Ala Arg Gln Ser Gly Ala Lys Lys Thr Ala Asp Gly Leu
225                 230                 235                 240

ggt atg cta gtc ggt caa gcg gcg gct tcc tac gcc ctc tgg cgc gga 768
Gly Met Leu Val Gly Gln Ala Ala Ala Ser Tyr Ala Leu Trp Arg Gly
                245                 250                 255

ttt acg ccc gat atc cgc ccc gtt atc gaa tac atg aaa gcc ct 812
Phe Thr Pro Asp Ile Arg Pro Val Ile Glu Tyr Met Lys Ala
            260                 265                 270

<210> 142
<211> 270
<212> PRT
<213> Neisseria meningitidis

<400> 142
Met Thr Met His Ala Leu Pro Arg Tyr Ala Val Phe Gly Asn Pro Val
1               5                   10                  15

Ala His Ser Lys Ser Pro Gln Ile His Gln Gln Phe Ala Leu Gln Glu
            20                  25                  30

Gly Val Asp Ile Glu Tyr Glu Arg Ile Cys Ala Asp Ile Gly Gly Phe
        35                  40                  45

Ala Gln Ala Val Ser Thr Phe Phe Glu Thr Gly Gly Cys Gly Ala Asn
    50                  55                  60

Val Thr Val Pro Phe Lys Gln Glu Ala Phe His Leu Ala Asp Glu His
65                  70                  75                  80

Ser Glu Arg Ala Leu Ala Ala Gly Ala Val Asn Thr Leu Ile Phe Leu
                85                  90                  95

Lys Asn Gly Lys Leu Arg Gly Asp Asn Thr Asp Gly Ile Gly Leu Ala
            100                 105                 110

Asn Asp Ile Thr Gln Val Lys Asn Ile Ala Ile Glu Gly Lys Thr Ile
        115                 120                 125

Leu Leu Leu Gly Ala Gly Gly Ala Val Arg Gly Val Ile Pro Val Leu
    130                 135                 140

Lys Glu His Arg Pro Ala Arg Ile Val Ile Ala Asn Arg Thr Arg Ala
145                 150                 155                 160

Lys Ala Glu Glu Leu Ala Gln Leu Phe Gly Ile Glu Ala Val Pro Met
                165                 170                 175

Ala Asp Val Asn Gly Gly Phe Asp Ile Ile Ile Asn Gly Thr Ser Gly
            180                 185                 190

Gly Leu Asn Gly Gln Ile Pro Asp Ile Pro Pro Asp Ile Phe Gln Asn
        195                 200                 205

Cys Ala Leu Ala Tyr Asp Met Val Tyr Gly Cys Ala Ala Lys Pro Phe
    210                 215                 220

Leu Asp Phe Ala Arg Gln Ser Gly Ala Lys Lys Thr Ala Asp Gly Leu
225                 230                 235                 240

Gly Met Leu Val Gly Gln Ala Ala Ala Ser Tyr Ala Leu Trp Arg Gly
                245                 250                 255

Phe Thr Pro Asp Ile Arg Pro Val Ile Glu Tyr Met Lys Ala
            260                 265                 270

<210> 143
<211> 1515
<212> DNA
<213> Neisseria meningitidis

<220>
<221> CDS
<222> (1)..(1515)

<400> 143

atg ttg tat ttc aga tac ggt ttt ttg gtt gtt tgg tgt gcg gca ggt    48
Met Leu Tyr Phe Arg Tyr Gly Phe Leu Val Val Trp Cys Ala Ala Gly
1               5                   10                  15

gtt tct gcc gcc tat ggg gcg gat gcg ccc gcg att ttg gat gac aag    96
Val Ser Ala Ala Tyr Gly Ala Asp Ala Pro Ala Ile Leu Asp Asp Lys
            20                  25                  30

gca ttg ttg cag gtg cag cgg tcg gtg tcg gat aag tgg gcg gaa tcg    144
Ala Leu Leu Gln Val Gln Arg Ser Val Ser Asp Lys Trp Ala Glu Ser
        35                  40                  45

gat tgg aaa gtt gac aat gat gcc ccg cgc gtg gtt gac ggg gat ttt    192
Asp Trp Lys Val Asp Asn Asp Ala Pro Arg Val Val Asp Gly Asp Phe
    50                  55                  60

ttg ttg gcg cat ccg aaa atg ttg gaa cat agt ttg cgc gac gtg ctc    240
Leu Leu Ala His Pro Lys Met Leu Glu His Ser Leu Arg Asp Val Leu
65                  70                  75                  80

aac ggc aat cag gcg gat ttg atc gct tcg ttg gcg gat ttg tat gcc    288
Asn Gly Asn Gln Ala Asp Leu Ile Ala Ser Leu Ala Asp Leu Tyr Ala
                85                  90                  95

aag ctg ccg gat tat gac gcg gtt ttg tac ggc agg gcg cgg gct ttg    336
Lys Leu Pro Asp Tyr Asp Ala Val Leu Tyr Gly Arg Ala Arg Ala Leu
            100                 105                 110

ctg gcg aaa ttg gcg gga agg ccg gcg gag gcg gtg gcg cgg tat cgg    384
Leu Ala Lys Leu Ala Gly Arg Pro Ala Glu Ala Val Ala Arg Tyr Arg
        115                 120                 125

gaa ctg cac ggg gaa aat gcg gca gac gag cgg att ttg ctg gat ttg    432
Glu Leu His Gly Glu Asn Ala Ala Asp Glu Arg Ile Leu Leu Asp Leu
    130                 135                 140

gcg gcg gcg gag ttt gac gat ttc cgg ctg aag tcg gca gaa agg cat    480
Ala Ala Ala Glu Phe Asp Asp Phe Arg Leu Lys Ser Ala Glu Arg His
145                 150                 155                 160

ttt gcc gag gcg gaa aaa ttg gat ttg ccg gcg ccg gtt ttg gaa aat    528
Phe Ala Glu Ala Glu Lys Leu Asp Leu Pro Ala Pro Val Leu Glu Asn
                165                 170                 175

gtg ggg cgt ttt cgg aaa aaa gcg gag ggg ctg acg ggc tgg cgt ttt    576
Val Gly Arg Phe Arg Lys Lys Ala Glu Gly Leu Thr Gly Trp Arg Phe
            180                 185                 190

tcg ggc ggc atc agt ccg gcg gtc aat aga aat gcc aat aat gcc gcg    624
Ser Gly Gly Ile Ser Pro Ala Val Asn Arg Asn Ala Asn Asn Ala Ala
        195                 200                 205

ccg cag tat tgc cgg caa aac gga ggc cgg cag ata tgc agt gtc agc    672
Pro Gln Tyr Cys Arg Gln Asn Gly Gly Arg Gln Ile Cys Ser Val Ser
    210                 215                 220

cgg gcg gag cgg gcg gca ggc ttg aat tat gaa atc gag gcg gaa aaa    720
Arg Ala Glu Arg Ala Ala Gly Leu Asn Tyr Glu Ile Glu Ala Glu Lys
225                 230                 235                 240

ctg acg gcg ttg gca gat aat cat tat ttg ttg ttc cgt tcc aat atc    768
Leu Thr Ala Leu Ala Asp Asn His Tyr Leu Leu Phe Arg Ser Asn Ile
                245                 250                 255

ggc ggc acg agc tat tat ttc agt aaa aaa tca gct tat gac gac ggg    816
Gly Gly Thr Ser Tyr Tyr Phe Ser Lys Lys Ser Ala Tyr Asp Asp Gly
            260                 265                 270

ttc ggc aga gcg tat ttg ggt tgg cag tat aaa aat gca cgg cag acg    864
Phe Gly Arg Ala Tyr Leu Gly Trp Gln Tyr Lys Asn Ala Arg Gln Thr
        275                 280                 285

gcg ggg att ttg ccg ttt tat cag gtg cag ttg tcg ggc agc gac ggc    912
Ala Gly Ile Leu Pro Phe Tyr Gln Val Gln Leu Ser Gly Ser Asp Gly
    290                 295                 300

ttt gat gcg aaa aca aaa cgg gta aac aac cgc cgc ctg ccg ccg tat    960
Phe Asp Ala Lys Thr Lys Arg Val Asn Asn Arg Arg Leu Pro Pro Tyr
305                 310                 315                 320

atg ctg gcg cac gga gtc ggc gtg cag ttg tcc cat act tac cgc cca    1008
Met Leu Ala His Gly Val Gly Val Gln Leu Ser His Thr Tyr Arg Pro
                325                 330                 335

aac ccg gga tgg caa ttt tcg gtc gcg ctg gaa cat tac cgc caa cgc    1056
Asn Pro Gly Trp Gln Phe Ser Val Ala Leu Glu His Tyr Arg Gln Arg
            340                 345                 350

tac cgc gaa cag gat agg gcg gaa tac aat aac ggt cgg cag gac ggg    1104
Tyr Arg Glu Gln Asp Arg Ala Glu Tyr Asn Asn Gly Arg Gln Asp Gly
        355                 360                 365

ttt tat gtt tcg tcg gca aaa cgt ttg ggc gaa tcg gca act gtg ttc    1152
Phe Tyr Val Ser Ser Ala Lys Arg Leu Gly Glu Ser Ala Thr Val Phe
    370                 375                 380

ggc ggc tgg cag ttt gtg cgg ttt gtg ccg aaa cgc gaa acg gtg ggc 1200
Gly Gly Trp Gln Phe Val Arg Phe Val Pro Lys Arg Glu Thr Val Gly
385                 390                 395                 400

ggc gcg gtc aat aat gcc gcc tac cgg cgc aac ggt gtt tat gcc ggc 1248
Gly Ala Val Asn Asn Ala Ala Tyr Arg Arg Asn Gly Val Tyr Ala Gly
                405                 410                 415

tgg gcg cag gag tgg cgg cag ttg ggc ggt ttg aac agt cgg gtt tcc 1296
Trp Ala Gln Glu Trp Arg Gln Leu Gly Gly Leu Asn Ser Arg Val Ser
            420                 425                 430

gcg tct tat gcc cgc cgc aac tat aag ggc gtt gcg gct ttc tcg aca 1344
Ala Ser Tyr Ala Arg Arg Asn Tyr Lys Gly Val Ala Ala Phe Ser Thr
        435                 440                 445

gag gcg caa cgc aac cgc gaa tgg aat gtc tcg ctg gct ttg agc cac 1392
Glu Ala Gln Arg Asn Arg Glu Trp Asn Val Ser Leu Ala Leu Ser His
    450                 455                 460

gac aag ttg tcg tac aaa ggt atc gtg ccc gcg ttg aat tat cgt ttc 1440
Asp Lys Leu Ser Tyr Lys Gly Ile Val Pro Ala Leu Asn Tyr Arg Phe
465                 470                 475                 480

ggc agg acg gaa agt aat gtg ccg tat gcg aaa cgc cgc aac agc gag 1488
Gly Arg Thr Glu Ser Asn Val Pro Tyr Ala Lys Arg Arg Asn Ser Glu
                485                 490                 495

gtg ttt gtg tcg gcg gat tgg cgg ttt 1515
Val Phe Val Ser Ala Asp Trp Arg Phe
            500                 505

<210> 144
<211> 505
<212> PRT
<213> Neisseria meningitidis

<400> 144
Met Leu Tyr Phe Arg Tyr Gly Phe Leu Val Val Trp Cys Ala Ala Gly
1               5                   10                  15

Val Ser Ala Ala Tyr Gly Ala Asp Ala Pro Ala Ile Leu Asp Asp Lys
            20                  25                  30

Ala Leu Leu Gln Val Gln Arg Ser Val Ser Asp Lys Trp Ala Glu Ser
        35                  40                  45

Asp Trp Lys Val Asp Asn Asp Ala Pro Arg Val Val Asp Gly Asp Phe
    50                  55                  60

Leu Leu Ala His Pro Lys Met Leu Glu His Ser Leu Arg Asp Val Leu
65                  70                  75                  80

Asn Gly Asn Gln Ala Asp Leu Ile Ala Ser Leu Ala Asp Leu Tyr Ala
                85                  90                  95

Lys Leu Pro Asp Tyr Asp Ala Val Leu Tyr Gly Arg Ala Arg Ala Leu
            100                 105                 110

Leu Ala Lys Leu Ala Gly Arg Pro Ala Glu Ala Val Ala Arg Tyr Arg
        115                 120                 125

Glu Leu His Gly Glu Asn Ala Ala Asp Glu Arg Ile Leu Leu Asp Leu
    130                 135                 140

Ala Ala Ala Glu Phe Asp Asp Phe Arg Leu Lys Ser Ala Glu Arg His
145                 150                 155                 160

Phe Ala Glu Ala Glu Lys Leu Asp Leu Pro Ala Pro Val Leu Glu Asn
                165                 170                 175

Val Gly Arg Phe Arg Lys Lys Ala Glu Gly Leu Thr Gly Trp Arg Phe
            180                 185                 190

Ser Gly Gly Ile Ser Pro Ala Val Asn Arg Asn Ala Asn Asn Ala Ala
        195                 200                 205

Pro Gln Tyr Cys Arg Gln Asn Gly Gly Arg Gln Ile Cys Ser Val Ser
    210                 215                 220

Arg Ala Glu Arg Ala Ala Gly Leu Asn Tyr Glu Ile Glu Ala Glu Lys
225                 230                 235                 240

Leu Thr Ala Leu Ala Asp Asn His Tyr Leu Leu Phe Arg Ser Asn Ile
                245                 250                 255

Gly Gly Thr Ser Tyr Tyr Phe Ser Lys Lys Ser Ala Tyr Asp Asp Gly
            260                 265                 270

Phe Gly Arg Ala Tyr Leu Gly Trp Gln Tyr Lys Asn Ala Arg Gln Thr
        275                 280                 285

Ala Gly Ile Leu Pro Phe Tyr Gln Val Gln Leu Ser Gly Ser Asp Gly
    290                 295                 300

Phe Asp Ala Lys Thr Lys Arg Val Asn Asn Arg Arg Leu Pro Pro Tyr
305                 310                 315                 320

Met Leu Ala His Gly Val Gly Val Gln Leu Ser His Thr Tyr Arg Pro
                325                 330                 335

Asn Pro Gly Trp Gln Phe Ser Val Ala Leu Glu His Tyr Arg Gln Arg
            340                 345                 350

Tyr Arg Glu Gln Asp Arg Ala Glu Tyr Asn Asn Gly Arg Gln Asp Gly
        355                 360                 365

Phe Tyr Val Ser Ser Ala Lys Arg Leu Gly Glu Ser Ala Thr Val Phe
    370                 375                 380

Gly Gly Trp Gln Phe Val Arg Phe Val Pro Lys Arg Glu Thr Val Gly
385                 390                 395                 400

Gly Ala Val Asn Asn Ala Ala Tyr Arg Arg Asn Gly Val Tyr Ala Gly
                405                 410                 415

Trp Ala Gln Glu Trp Arg Gln Leu Gly Gly Leu Asn Ser Arg Val Ser
            420                 425                 430

Ala Ser Tyr Ala Arg Arg Asn Tyr Lys Gly Val Ala Ala Phe Ser Thr
        435                 440                 445

Glu Ala Gln Arg Asn Arg Glu Trp Asn Val Ser Leu Ala Leu Ser His
    450                 455                 460

Asp Lys Leu Ser Tyr Lys Gly Ile Val Pro Ala Leu Asn Tyr Arg Phe
465                 470                 475                 480

Gly Arg Thr Glu Ser Asn Val Pro Tyr Ala Lys Arg Arg Asn Ser Glu
                485                 490                 495

Val Phe Val Ser Ala Asp Trp Arg Phe
            500                 505

<210> 145
<211> 840
<212> DNA
<213> Neisseria meningitidis

<220>
<221> CDS

<222> (1)..(840)

<400> 145
atg gat aaa gaa cga att tta acc ccc gca gtc gtg ttt tcc gtc gca    48
Met Asp Lys Glu Arg Ile Leu Thr Pro Ala Val Val Phe Ser Val Ala
1               5                   10                  15

ctg ctg cat ttg gca atg gtg gca ttg ctc tgg cag gcg cac aag ctg    96
Leu Leu His Leu Ala Met Val Ala Leu Leu Trp Gln Ala His Lys Leu
            20                  25                  30

ccc gtg ata gag tca ggc aat gtt att gaa ttt gtc gat ttg ggc gat    144
Pro Val Ile Glu Ser Gly Asn Val Ile Glu Phe Val Asp Leu Gly Asp
        35                  40                  45

ttt ggc gga ggg gac ggc gca ccc gaa ggt gca ggc gcg cct gcc gcg    192
Phe Gly Gly Gly Asp Gly Ala Pro Glu Gly Ala Gly Ala Pro Ala Ala
    50                  55                  60

ccc gaa ccg caa ccc gtg ccc gag ccg ccc aaa cct gtc gag ccg ccc    240
Pro Glu Pro Gln Pro Val Pro Glu Pro Pro Lys Pro Val Glu Pro Pro
65                  70                  75                  80

aag ccg gtt ttg aag ccg gtg gtt acg aaa aag gcg gat gcg gat att    288
Lys Pro Val Leu Lys Pro Val Val Thr Lys Lys Ala Asp Ala Asp Ile
                85                  90                  95

cag cag cct aag gaa gag ccg aaa cct gaa gaa aag ccg aaa ccc gaa    336
Gln Gln Pro Lys Glu Glu Pro Lys Pro Glu Glu Lys Pro Lys Pro Glu
            100                 105                 110

gaa aaa ccg aaa cca gag cct aag ccg gaa gcg aag cct gtc ccg aaa    384
Glu Lys Pro Lys Pro Glu Pro Lys Pro Glu Ala Lys Pro Val Pro Lys
        115                 120                 125

ccg gcg gaa aaa ccg gtc gag aag ccg tct gaa aaa cct gcc gaa cat    432
Pro Ala Glu Lys Pro Val Glu Lys Pro Ser Glu Lys Pro Ala Glu His
    130                 135                 140

ccc ggc aat gct tct gcc aaa gca gac agc gag cag ggc aat ggg gaa    480
Pro Gly Asn Ala Ser Ala Lys Ala Asp Ser Glu Gln Gly Asn Gly Glu
145                 150                 155                 160

gat aag gga acc ggt atc aaa gga gac gga acg ggg cgc gga gaa ggc    528
Asp Lys Gly Thr Gly Ile Lys Gly Asp Gly Thr Gly Arg Gly Glu Gly
                165                 170                 175

agc ggt aaa ggt agc ggc ggt gtc aaa ggc gaa cac ggg gaa gga gcc    576

Ser Gly Lys Gly Ser Gly Gly Val Lys Gly Glu His Gly Glu Gly Ala
            180                 185                 190

ggc agc agc aaa ggc aat cct tta cgc gcc aac ggc agc att ccg cgc     624
Gly Ser Ser Lys Gly Asn Pro Leu Arg Ala Asn Gly Ser Ile Pro Arg
        195                 200                 205

ccg gct tat ccc acg ctt tct atg gag aat gat gag cag ggt acg gtt     672
Pro Ala Tyr Pro Thr Leu Ser Met Glu Asn Asp Glu Gln Gly Thr Val
    210                 215                 220

gtt ttg agc gtt ttg gtt tct ccg ggc ggt cat gtt gag tcc gtt aaa     720
Val Leu Ser Val Leu Val Ser Pro Gly Gly His Val Glu Ser Val Lys
225                 230                 235                 240

atc gtg aaa agc agt ggt ttt tcc cgg ttg gac aat gcg gca cgc aag     768
Ile Val Lys Ser Ser Gly Phe Ser Arg Leu Asp Asn Ala Ala Arg Lys
                245                 250                 255

gcg gcg caa aac ggg cat ttt caa gcc aat gcc tgg acg gag ttt aaa     816
Ala Ala Gln Asn Gly His Phe Gln Ala Asn Ala Trp Thr Glu Phe Lys
            260                 265                 270

gtc ccc gtc aag ttt gaa ttg aat                                     840
Val Pro Val Lys Phe Glu Leu Asn
        275                 280

<210> 146
<211> 280
<212> PRT
<213> Neisseria meningitidis

<400> 146
Met Asp Lys Glu Arg Ile Leu Thr Pro Ala Val Val Phe Ser Val Ala
1               5                   10                  15

Leu Leu His Leu Ala Met Val Ala Leu Leu Trp Gln Ala His Lys Leu
            20                  25                  30

Pro Val Ile Glu Ser Gly Asn Val Ile Glu Phe Val Asp Leu Gly Asp
        35                  40                  45

Phe Gly Gly Gly Asp Gly Ala Pro Glu Gly Ala Gly Ala Pro Ala Ala
    50                  55                  60

Pro Glu Pro Gln Pro Val Pro Glu Pro Pro Lys Pro Val Glu Pro Pro
65                  70                  75                  80

Lys Pro Val Leu Lys Pro Val Val Thr Lys Lys Ala Asp Ala Asp Ile
                85                  90                  95

Gln Gln Pro Lys Glu Glu Pro Lys Pro Glu Glu Lys Pro Lys Pro Glu
            100                 105                 110

Glu Lys Pro Lys Pro Glu Pro Lys Pro Glu Ala Lys Pro Val Pro Lys
        115                 120                 125

Pro Ala Glu Lys Pro Val Glu Lys Pro Ser Glu Lys Pro Ala Glu His
    130                 135                 140

Pro Gly Asn Ala Ser Ala Lys Ala Asp Ser Glu Gln Gly Asn Gly Glu
145                 150                 155                 160

Asp Lys Gly Thr Gly Ile Lys Gly Asp Gly Thr Gly Arg Gly Glu Gly
                165                 170                 175

Ser Gly Lys Gly Ser Gly Gly Val Lys Gly Glu His Gly Glu Gly Ala
            180                 185                 190

Gly Ser Ser Lys Gly Asn Pro Leu Arg Ala Asn Gly Ser Ile Pro Arg
        195                 200                 205

Pro Ala Tyr Pro Thr Leu Ser Met Glu Asn Asp Glu Gln Gly Thr Val
    210                 215                 220

Val Leu Ser Val Leu Val Ser Pro Gly Gly His Val Glu Ser Val Lys
225                 230                 235                 240

Ile Val Lys Ser Ser Gly Phe Ser Arg Leu Asp Asn Ala Ala Arg Lys
                245                 250                 255

Ala Ala Gln Asn Gly His Phe Gln Ala Asn Ala Trp Thr Glu Phe Lys
            260                 265                 270

Val Pro Val Lys Phe Glu Leu Asn
        275                 280

<210> 147
<211> 1572
<212> DNA
<213> Neisseria meningitidis

<220>

<221> CDS
<222> (1)..(1572)

<400> 147
atg ttc aga cgg tat ctt ccg aac aga cag atg aat atg gtt tcc aaa    48
Met Phe Arg Arg Tyr Leu Pro Asn Arg Gln Met Asn Met Val Ser Lys
1               5                   10                  15

ctg gac aaa tac tgg cag cac ccc gcc ctc tac tgg cct ttg ctc atc    96
Leu Asp Lys Tyr Trp Gln His Pro Ala Leu Tyr Trp Pro Leu Leu Ile
            20                  25                  30

ctt ttt gcc gcc gcc acc ccc ttt acc ttc gca ccc tac tac cac ttt    144
Leu Phe Ala Ala Ala Thr Pro Phe Thr Phe Ala Pro Tyr Tyr His Phe
        35                  40                  45

tgg ctg atg ccc ttg att ttc ggt gcc ttc gtc cgc ctc atc gaa ctg    192
Trp Leu Met Pro Leu Ile Phe Gly Ala Phe Val Arg Leu Ile Glu Leu
    50                  55                  60

cgt ccg cgt ttt gct gtc tct tcc gcc tac ctg ttc ggc ctg acc gca    240
Arg Pro Arg Phe Ala Val Ser Ser Ala Tyr Leu Phe Gly Leu Thr Ala
65                  70                  75                  80

tac acg aca cag ttc tac tgg ata cac acc gcc ctg cac gac gtt tcc    288
Tyr Thr Thr Gln Phe Tyr Trp Ile His Thr Ala Leu His Asp Val Ser
                85                  90                  95

ggc ctg ccc gac ctc tat gcc gta ccg ctg acc ttc cta ctc ccc gcc    336
Gly Leu Pro Asp Leu Tyr Ala Val Pro Leu Thr Phe Leu Leu Pro Ala
            100                 105                 110

tac ctt gcc ctt tat ccg gca ctg tgt ttc tgg ctg tgg aaa aaa ttt    384
Tyr Leu Ala Leu Tyr Pro Ala Leu Cys Phe Trp Leu Trp Lys Lys Phe
        115                 120                 125

acc ctg cct cgg ggc ata aaa atc ggt ttg gta ctg ccc atc ctg tgg    432
Thr Leu Pro Arg Gly Ile Lys Ile Gly Leu Val Leu Pro Ile Leu Trp
    130                 135                 140

aca ctg acc gag ttt gcc cgc gaa cgt ttc ctg acc gga ttc ggc tgg    480
Thr Leu Thr Glu Phe Ala Arg Glu Arg Phe Leu Thr Gly Phe Gly Trp
145                 150                 155                 160

ggc gca atc ggc tac tcc caa atc acc ccg gac agc ccg ctc gcc ggc    528
Gly Ala Ile Gly Tyr Ser Gln Ile Thr Pro Asp Ser Pro Leu Ala Gly
                165                 170                 175

ttt gcc cca ttt ggc ggc atc cac atg gtt aca ctg gca acc gcc ttt     576
Phe Ala Pro Phe Gly Gly Ile His Met Val Thr Leu Ala Thr Ala Phe
            180                 185                 190

ctc ggt gtc tgg ctg gtt ttg gcg agt gat aac acc gca cgt tcg ggc     624
Leu Gly Val Trp Leu Val Leu Ala Ser Asp Asn Thr Ala Arg Ser Gly
        195                 200                 205

aaa cgc ctg ctt cca att atc ctg att gcc gcc ctg ctt gcc gca ggc     672
Lys Arg Leu Leu Pro Ile Ile Leu Ile Ala Ala Leu Leu Ala Ala Gly
    210                 215                 220

tac acc gcc cga caa acc gac ttc acc cgc ccc gac ggc agc cgc agc     720
Tyr Thr Ala Arg Gln Thr Asp Phe Thr Arg Pro Asp Gly Ser Arg Ser
225                 230                 235                 240

acc gtc gcc ctg ctt caa ggc aac atc gac caa acc ctc aaa tgg cgt     768
Thr Val Ala Leu Leu Gln Gly Asn Ile Asp Gln Thr Leu Lys Trp Arg
                245                 250                 255

gaa gac caa gtc atc ccg acc ata cag aaa tat tac gaa cag gtc ggc     816
Glu Asp Gln Val Ile Pro Thr Ile Gln Lys Tyr Tyr Glu Gln Val Gly
            260                 265                 270

aaa acc act gcc gac atc gtc atc ctg ccc gaa acc gcc atc ccc gtc     864
Lys Thr Thr Ala Asp Ile Val Ile Leu Pro Glu Thr Ala Ile Pro Val
        275                 280                 285

atg cgc caa aac ctg ccg gaa aac ata ctg gca aaa ttt gcc gaa cag     912
Met Arg Gln Asn Leu Pro Glu Asn Ile Leu Ala Lys Phe Ala Glu Gln
    290                 295                 300

gcg caa aac aac ggc agc gcg ctc gcc gtc ggc atc agc caa tac act     960
Ala Gln Asn Asn Gly Ser Ala Leu Ala Val Gly Ile Ser Gln Tyr Thr
305                 310                 315                 320

tcg gac ggc aac ggt tac gaa aac gcc gtc atc aac ctg acc ggt tat     1008
Ser Asp Gly Asn Gly Tyr Glu Asn Ala Val Ile Asn Leu Thr Gly Tyr
                325                 330                 335

cag gaa aac aat cag gac ggc atc ccc tac tac gcc aaa aac cac ctc     1056
Gln Glu Asn Asn Gln Asp Gly Ile Pro Tyr Tyr Ala Lys Asn His Leu
            340                 345                 350

gtc ccc ttc ggc gaa tac aaa ccg ctg ccc ttc ctg acc acg ccg ctt     1104
Val Pro Phe Gly Glu Tyr Lys Pro Leu Pro Phe Leu Thr Thr Pro Leu
        355                 360                 365

tac aaa atg atg aat atg ccc ctt tcc gac ttc cgc aaa ggc ggc ggt 1152
Tyr Lys Met Met Asn Met Pro Leu Ser Asp Phe Arg Lys Gly Gly Gly
    370                 375                 380

aag caa tcc gcc ctg ctg atg aaa aac caa aaa atc gcc ttc aac atc 1200
Lys Gln Ser Ala Leu Leu Met Lys Asn Gln Lys Ile Ala Phe Asn Ile
385                 390                 395                 400

tgt tac gaa gac gga ttc ggc gac gaa ctg att gcc gcc gcc aaa gat 1248
Cys Tyr Glu Asp Gly Phe Gly Asp Glu Leu Ile Ala Ala Ala Lys Asp
                405                 410                 415

gcc aca ctg ctt gcc aat gcc agc aat atg gcg tgg tac gga aaa tcc 1296
Ala Thr Leu Leu Ala Asn Ala Ser Asn Met Ala Trp Tyr Gly Lys Ser
            420                 425                 430

aac gcc atg tac cag cac ctc caa caa tcg cag gcg cgg gct atg gaa 1344
Asn Ala Met Tyr Gln His Leu Gln Gln Ser Gln Ala Arg Ala Met Glu
        435                 440                 445

ctc gga cgc tat atg gtc cgt gcc acc aac acc ggc gca acc gcc atc 1392
Leu Gly Arg Tyr Met Val Arg Ala Thr Asn Thr Gly Ala Thr Ala Ile
    450                 455                 460

atc tcc ccc aaa ggc aac atc atc gcc caa gcc caa ccc gat acg gaa 1440
Ile Ser Pro Lys Gly Asn Ile Ile Ala Gln Ala Gln Pro Asp Thr Glu
465                 470                 475                 480

acc gta ttg gaa gga cac atc aaa ggc tat gtc ggc gaa acg ccc tat 1488
Thr Val Leu Glu Gly His Ile Lys Gly Tyr Val Gly Glu Thr Pro Tyr
                485                 490                 495

atg aaa acc ggc agt tca tgg tgg ttg atg ggc ata ttg acc cta gcc 1536
Met Lys Thr Gly Ser Ser Trp Trp Leu Met Gly Ile Leu Thr Leu Ala
            500                 505                 510

gca ctg att ctt ttc atc ttc cga aac aaa gaa cac 1572
Ala Leu Ile Leu Phe Ile Phe Arg Asn Lys Glu His
        515                 520

<210> 148
<211> 524
<212> PRT
<213> Neisseria meningitidis

<400> 148
Met Phe Arg Arg Tyr Leu Pro Asn Arg Gln Met Asn Met Val Ser Lys

1                   5                   10                  15

Leu Asp Lys Tyr Trp Gln His Pro Ala Leu Tyr Trp Pro Leu Leu Ile
                20                  25                  30

Leu Phe Ala Ala Ala Thr Pro Phe Thr Phe Ala Pro Tyr Tyr His Phe
            35                  40                  45

Trp Leu Met Pro Leu Ile Phe Gly Ala Phe Val Arg Leu Ile Glu Leu
    50                  55                  60

Arg Pro Arg Phe Ala Val Ser Ser Ala Tyr Leu Phe Gly Leu Thr Ala
65                  70                  75                  80

Tyr Thr Thr Gln Phe Tyr Trp Ile His Thr Ala Leu His Asp Val Ser
                85                  90                  95

Gly Leu Pro Asp Leu Tyr Ala Val Pro Leu Thr Phe Leu Leu Pro Ala
            100                 105                 110

Tyr Leu Ala Leu Tyr Pro Ala Leu Cys Phe Trp Leu Trp Lys Lys Phe
        115                 120                 125

Thr Leu Pro Arg Gly Ile Lys Ile Gly Leu Val Leu Pro Ile Leu Trp
    130                 135                 140

Thr Leu Thr Glu Phe Ala Arg Glu Arg Phe Leu Thr Gly Phe Gly Trp
145                 150                 155                 160

Gly Ala Ile Gly Tyr Ser Gln Ile Thr Pro Asp Ser Pro Leu Ala Gly
                165                 170                 175

Phe Ala Pro Phe Gly Gly Ile His Met Val Thr Leu Ala Thr Ala Phe
            180                 185                 190

Leu Gly Val Trp Leu Val Leu Ala Ser Asp Asn Thr Ala Arg Ser Gly
        195                 200                 205

Lys Arg Leu Leu Pro Ile Ile Leu Ile Ala Ala Leu Leu Ala Ala Gly
    210                 215                 220

Tyr Thr Ala Arg Gln Thr Asp Phe Thr Arg Pro Asp Gly Ser Arg Ser
225                 230                 235                 240

Thr Val Ala Leu Leu Gln Gly Asn Ile Asp Gln Thr Leu Lys Trp Arg
                245                 250                 255

Glu Asp Gln Val Ile Pro Thr Ile Gln Lys Tyr Tyr Glu Gln Val Gly

            260                 265                 270

Lys Thr Thr Ala Asp Ile Val Ile Leu Pro Glu Thr Ala Ile Pro Val
        275                 280                 285

Met Arg Gln Asn Leu Pro Glu Asn Ile Leu Ala Lys Phe Ala Glu Gln
    290                 295                 300

Ala Gln Asn Asn Gly Ser Ala Leu Ala Val Gly Ile Ser Gln Tyr Thr
305                 310                 315                 320

Ser Asp Gly Asn Gly Tyr Glu Asn Ala Val Ile Asn Leu Thr Gly Tyr
                325                 330                 335

Gln Glu Asn Asn Gln Asp Gly Ile Pro Tyr Tyr Ala Lys Asn His Leu
            340                 345                 350

Val Pro Phe Gly Glu Tyr Lys Pro Leu Pro Phe Leu Thr Thr Pro Leu
        355                 360                 365

Tyr Lys Met Met Asn Met Pro Leu Ser Asp Phe Arg Lys Gly Gly Gly
    370                 375                 380

Lys Gln Ser Ala Leu Leu Met Lys Asn Gln Lys Ile Ala Phe Asn Ile
385                 390                 395                 400

Cys Tyr Glu Asp Gly Phe Gly Asp Glu Leu Ile Ala Ala Ala Lys Asp
                405                 410                 415

Ala Thr Leu Leu Ala Asn Ala Ser Asn Met Ala Trp Tyr Gly Lys Ser
            420                 425                 430

Asn Ala Met Tyr Gln His Leu Gln Gln Ser Gln Ala Arg Ala Met Glu
        435                 440                 445

Leu Gly Arg Tyr Met Val Arg Ala Thr Asn Thr Gly Ala Thr Ala Ile
    450                 455                 460

Ile Ser Pro Lys Gly Asn Ile Ile Ala Gln Ala Gln Pro Asp Thr Glu
465                 470                 475                 480

Thr Val Leu Glu Gly His Ile Lys Gly Tyr Val Gly Glu Thr Pro Tyr
                485                 490                 495

Met Lys Thr Gly Ser Ser Trp Trp Leu Met Gly Ile Leu Thr Leu Ala
            500                 505                 510

Ala Leu Ile Leu Phe Ile Phe Arg Asn Lys Glu His

515                 520

<210> 149
<211> 558
<212> DNA
<213> Neisseria meningitidis

<220>
<221> CDS
<222> (1)..(558)

<400> 149
ttg gat ctc tac aaa ctc ggt tac aaa caa cat acc cgt acc gat gcg     48
Leu Asp Leu Tyr Lys Leu Gly Tyr Lys Gln His Thr Arg Thr Asp Ala
1               5                   10                  15

gaa ggc tat atc gag aaa ctg cac att acc ccc gcc aat gcc cat gag     96
Glu Gly Tyr Ile Glu Lys Leu His Ile Thr Pro Ala Asn Ala His Glu
            20                  25                  30

tgc aaa cac ctg ccg ccg ttg ttg gaa gga ctg ccc aaa ggt acg acc     144
Cys Lys His Leu Pro Pro Leu Leu Glu Gly Leu Pro Lys Gly Thr Thr
        35                  40                  45

gtc tat gcc gac aaa ggc tac gac agt gcg gaa aac cgg caa cat ctg     192
Val Tyr Ala Asp Lys Gly Tyr Asp Ser Ala Glu Asn Arg Gln His Leu
    50                  55                  60

gaa gaa cat cag ttg ttg gac ggc att atg cgc aaa gcc tgc cgc aac     240
Glu Glu His Gln Leu Leu Asp Gly Ile Met Arg Lys Ala Cys Arg Asn
65                  70                  75                  80

cgt ccg ctg tcg gaa acg caa acc aaa cgc aac cgg tat ttg tcg aag     288
Arg Pro Leu Ser Glu Thr Gln Thr Lys Arg Asn Arg Tyr Leu Ser Lys
                85                  90                  95

acc cgt tat agt gga tta aat tta aat cag gac aag gcg acg aag ccg     336
Thr Arg Tyr Ser Gly Leu Asn Leu Asn Gln Asp Lys Ala Thr Lys Pro
            100                 105                 110

cag aca gta caa ata gta cgg caa ggc gag gca acg ccg tac tgg ttt     384
Gln Thr Val Gln Ile Val Arg Gln Gly Glu Ala Thr Pro Tyr Trp Phe
        115                 120                 125

aaa ttt aat cca cta tat gtg gtc gaa cag agc ttc ggt acg ctg cac     432
Lys Phe Asn Pro Leu Tyr Val Val Glu Gln Ser Phe Gly Thr Leu His

130                 135                 140

cgt aaa ttc cgc tat gcg cgg gca gcc tat ttc gga ctg att aaa gtg     480
Arg Lys Phe Arg Tyr Ala Arg Ala Ala Tyr Phe Gly Leu Ile Lys Val
145                 150                 155                 160

agt gcg caa agc cat ctg aag gcg atg tgt ttg aac ctg ttg aaa gcc     528
Ser Ala Gln Ser His Leu Lys Ala Met Cys Leu Asn Leu Leu Lys Ala
                165                 170                 175

gcc aac aag cta agt gcg ccc gct gcc gcc                             558
Ala Asn Lys Leu Ser Ala Pro Ala Ala Ala
            180                 185

<210> 150
<211> 186
<212> PRT
<213> Neisseria meningitidis

<400> 150
Leu Asp Leu Tyr Lys Leu Gly Tyr Lys Gln His Thr Arg Thr Asp Ala
1               5                   10                  15

Glu Gly Tyr Ile Glu Lys Leu His Ile Thr Pro Ala Asn Ala His Glu
            20                  25                  30

Cys Lys His Leu Pro Pro Leu Leu Glu Gly Leu Pro Lys Gly Thr Thr
        35                  40                  45

Val Tyr Ala Asp Lys Gly Tyr Asp Ser Ala Glu Asn Arg Gln His Leu
    50                  55                  60

Glu Glu His Gln Leu Leu Asp Gly Ile Met Arg Lys Ala Cys Arg Asn
65                  70                  75                  80

Arg Pro Leu Ser Glu Thr Gln Thr Lys Arg Asn Arg Tyr Leu Ser Lys
                85                  90                  95

Thr Arg Tyr Ser Gly Leu Asn Leu Asn Gln Asp Lys Ala Thr Lys Pro
            100                 105                 110

Gln Thr Val Gln Ile Val Arg Gln Gly Glu Ala Thr Pro Tyr Trp Phe
        115                 120                 125

Lys Phe Asn Pro Leu Tyr Val Val Glu Gln Ser Phe Gly Thr Leu His
    130                 135                 140

Arg Lys Phe Arg Tyr Ala Arg Ala Ala Tyr Phe Gly Leu Ile Lys Val
145                 150                 155                 160

Ser Ala Gln Ser His Leu Lys Ala Met Cys Leu Asn Leu Leu Lys Ala
                165                 170                 175

Ala Asn Lys Leu Ser Ala Pro Ala Ala Ala
            180                 185

<210> 151
<211> 1332
<212> DNA
<213> Neisseria meningitidis

<220>
<221> CDS
<222> (1)..(1332)

<400> 151
atg act gac ctg aac acc ctg ttt gcc aac ctc aaa caa cgc aat ccc      48
Met Thr Asp Leu Asn Thr Leu Phe Ala Asn Leu Lys Gln Arg Asn Pro
1               5                   10                  15

aat cag gag ccg ttc cat cag gcg gtt gaa gaa gtc ttc atg agt ctc      96
Asn Gln Glu Pro Phe His Gln Ala Val Glu Glu Val Phe Met Ser Leu
            20                  25                  30

gat ccg ttt ttg gca aaa aat ccg aaa tac acc cag caa agc ctg ctg      144
Asp Pro Phe Leu Ala Lys Asn Pro Lys Tyr Thr Gln Gln Ser Leu Leu
        35                  40                  45

gaa cgc atc gtc gaa ccc gaa cgc gtc gtg atg ttc cgc gta acc tgg      192
Glu Arg Ile Val Glu Pro Glu Arg Val Val Met Phe Arg Val Thr Trp
    50                  55                  60

cag gac gat aaa ggg caa gtc caa gtc aac cgg ggc tac cgc gtg caa      240
Gln Asp Asp Lys Gly Gln Val Gln Val Asn Arg Gly Tyr Arg Val Gln
65                  70                  75                  80

atg agt tcc gcc atc ggt cct tac aaa ggc ggc ctg cgc ttc cat ccg      288
Met Ser Ser Ala Ile Gly Pro Tyr Lys Gly Gly Leu Arg Phe His Pro
                85                  90                  95

acc gtc gat ttg ggc gta ttg aaa ttc ctc gct ttt gaa caa gtg ttc      336
Thr Val Asp Leu Gly Val Leu Lys Phe Leu Ala Phe Glu Gln Val Phe
            100                 105                 110

aaa aac gcc ttg acc acc ctg cct atg ggc ggc ggc aaa ggc ggt tcc 384
Lys Asn Ala Leu Thr Thr Leu Pro Met Gly Gly Gly Lys Gly Gly Ser
        115                 120                 125

gac ttc gac ccc aaa ggc aaa tcc gat gcc gaa gta atg cgc ttc tgc 432
Asp Phe Asp Pro Lys Gly Lys Ser Asp Ala Glu Val Met Arg Phe Cys
    130                 135                 140

caa gcc ttt atg acc gaa ctc tac cgc cac atc ggc gcg gac acc gat 480
Gln Ala Phe Met Thr Glu Leu Tyr Arg His Ile Gly Ala Asp Thr Asp
145                 150                 155                 160

gtt ccg gcc ggc gac atc ggc gta ggc ggg cgc gaa atc ggc tac ctg 528
Val Pro Ala Gly Asp Ile Gly Val Gly Gly Arg Glu Ile Gly Tyr Leu
                165                 170                 175

ttc gga caa tac aaa aaa atc cgc aac gag ttt tct tcc gtc ctg acc 576
Phe Gly Gln Tyr Lys Lys Ile Arg Asn Glu Phe Ser Ser Val Leu Thr
            180                 185                 190

ggc aaa ggt ttg gaa tgg ggc ggc agc ctc atc cgt ccc gaa gcg acc 624
Gly Lys Gly Leu Glu Trp Gly Gly Ser Leu Ile Arg Pro Glu Ala Thr
        195                 200                 205

ggc tac ggc tgc gtc tat ttc gcc caa gcg atg ctg caa acc cgc aac 672
Gly Tyr Gly Cys Val Tyr Phe Ala Gln Ala Met Leu Gln Thr Arg Asn
    210                 215                 220

gat agt ttt gaa ggc aaa cgc gtc ctg att tcc ggt tcc ggc aac gtg 720
Asp Ser Phe Glu Gly Lys Arg Val Leu Ile Ser Gly Ser Gly Asn Val
225                 230                 235                 240

gcg caa tac gcc gcc gaa aaa gcc atc caa ctg ggc gcg aaa gtg ctg 768
Ala Gln Tyr Ala Ala Glu Lys Ala Ile Gln Leu Gly Ala Lys Val Leu
                245                 250                 255

acc gtt tcc gac tcc aac ggc ttc gtc ctc ttc ccc gac agc ggt atg 816
Thr Val Ser Asp Ser Asn Gly Phe Val Leu Phe Pro Asp Ser Gly Met
            260                 265                 270

agc gaa gcg caa ctc gcc gcc ttg atc gaa ttg aaa gaa gtc cgc cgc 864
Ser Glu Ala Gln Leu Ala Ala Leu Ile Glu Leu Lys Glu Val Arg Arg
        275                 280                 285

gaa cgg gtt gcc acc tac gcc aaa gag caa ggt ctg caa tac ttt gaa 912
Glu Arg Val Ala Thr Tyr Ala Lys Glu Gln Gly Leu Gln Tyr Phe Glu
    290                 295                 300

aac caa aaa ccg tgg ggc gtc gcc gcc gaa atc gcc ctg ccc tgc gcg    960
Asn Gln Lys Pro Trp Gly Val Ala Ala Glu Ile Ala Leu Pro Cys Ala
305                 310                 315                 320

acc cag aac gaa ttg gac gaa gaa gcc gcc aaa acc ctg ttg gca aac    1008
Thr Gln Asn Glu Leu Asp Glu Glu Ala Ala Lys Thr Leu Leu Ala Asn
                325                 330                 335

ggc tgc tac gtc gtt gcc gaa ggt gcg aat atg ccg tcg act ttg ggc    1056
Gly Cys Tyr Val Val Ala Glu Gly Ala Asn Met Pro Ser Thr Leu Gly
            340                 345                 350

gcg gtc gag caa ttt atc aaa gcc ggc atc ctc tac gcc ccg ggc aaa    1104
Ala Val Glu Gln Phe Ile Lys Ala Gly Ile Leu Tyr Ala Pro Gly Lys
        355                 360                 365

gcc tcc aac gcc ggc ggc gta gca act tca ggt ttg gaa atg agc caa    1152
Ala Ser Asn Ala Gly Gly Val Ala Thr Ser Gly Leu Glu Met Ser Gln
    370                 375                 380

aac gcc atc cgc ctg tct tgg act cgt gaa gaa gtc gac caa cgc ctg    1200
Asn Ala Ile Arg Leu Ser Trp Thr Arg Glu Glu Val Asp Gln Arg Leu
385                 390                 395                 400

ttc ggc atc atg caa agc atc cac gaa tcc tgt ctg aaa tac ggc aaa    1248
Phe Gly Ile Met Gln Ser Ile His Glu Ser Cys Leu Lys Tyr Gly Lys
                405                 410                 415

gtc ggc gac aca gta aac tac gtc aat ggt gcg aac att gcc ggt ttc    1296
Val Gly Asp Thr Val Asn Tyr Val Asn Gly Ala Asn Ile Ala Gly Phe
            420                 425                 430

gtc aaa gtt gcc gat gcg atg ctg gcg caa ggc ttc                    1332
Val Lys Val Ala Asp Ala Met Leu Ala Gln Gly Phe
        435                 440

<210> 152
<211> 444
<212> PRT
<213> Neisseria meningitidis

<400> 152
Met Thr Asp Leu Asn Thr Leu Phe Ala Asn Leu Lys Gln Arg Asn Pro
  1               5                  10                  15

Asn Gln Glu Pro Phe His Gln Ala Val Glu Glu Val Phe Met Ser Leu

            20                  25                  30

Asp Pro Phe Leu Ala Lys Asn Pro Lys Tyr Thr Gln Gln Ser Leu Leu
        35                  40                  45

Glu Arg Ile Val Glu Pro Glu Arg Val Val Met Phe Arg Val Thr Trp
    50                  55                  60

Gln Asp Asp Lys Gly Gln Val Gln Val Asn Arg Gly Tyr Arg Val Gln
65                  70                  75                  80

Met Ser Ser Ala Ile Gly Pro Tyr Lys Gly Gly Leu Arg Phe His Pro
                85                  90                  95

Thr Val Asp Leu Gly Val Leu Lys Phe Leu Ala Phe Glu Gln Val Phe
            100                 105                 110

Lys Asn Ala Leu Thr Thr Leu Pro Met Gly Gly Gly Lys Gly Gly Ser
        115                 120                 125

Asp Phe Asp Pro Lys Gly Lys Ser Asp Ala Glu Val Met Arg Phe Cys
    130                 135                 140

Gln Ala Phe Met Thr Glu Leu Tyr Arg His Ile Gly Ala Asp Thr Asp
145                 150                 155                 160

Val Pro Ala Gly Asp Ile Gly Val Gly Gly Arg Glu Ile Gly Tyr Leu
                165                 170                 175

Phe Gly Gln Tyr Lys Lys Ile Arg Asn Glu Phe Ser Ser Val Leu Thr
            180                 185                 190

Gly Lys Gly Leu Glu Trp Gly Gly Ser Leu Ile Arg Pro Glu Ala Thr
        195                 200                 205

Gly Tyr Gly Cys Val Tyr Phe Ala Gln Ala Met Leu Gln Thr Arg Asn
    210                 215                 220

Asp Ser Phe Glu Gly Lys Arg Val Leu Ile Ser Gly Ser Gly Asn Val
225                 230                 235                 240

Ala Gln Tyr Ala Ala Glu Lys Ala Ile Gln Leu Gly Ala Lys Val Leu
                245                 250                 255

Thr Val Ser Asp Ser Asn Gly Phe Val Leu Phe Pro Asp Ser Gly Met
            260                 265                 270

Ser Glu Ala Gln Leu Ala Ala Leu Ile Glu Leu Lys Glu Val Arg Arg

275                 280                 285

Glu Arg Val Ala Thr Tyr Ala Lys Glu Gln Gly Leu Gln Tyr Phe Glu
    290                 295                 300

Asn Gln Lys Pro Trp Gly Val Ala Ala Glu Ile Ala Leu Pro Cys Ala
305                 310                 315                 320

Thr Gln Asn Glu Leu Asp Glu Glu Ala Ala Lys Thr Leu Leu Ala Asn
                325                 330                 335

Gly Cys Tyr Val Val Ala Glu Gly Ala Asn Met Pro Ser Thr Leu Gly
            340                 345                 350

Ala Val Glu Gln Phe Ile Lys Ala Gly Ile Leu Tyr Ala Pro Gly Lys
        355                 360                 365

Ala Ser Asn Ala Gly Gly Val Ala Thr Ser Gly Leu Glu Met Ser Gln
    370                 375                 380

Asn Ala Ile Arg Leu Ser Trp Thr Arg Glu Glu Val Asp Gln Arg Leu
385                 390                 395                 400

Phe Gly Ile Met Gln Ser Ile His Glu Ser Cys Leu Lys Tyr Gly Lys
                405                 410                 415

Val Gly Asp Thr Val Asn Tyr Val Asn Gly Ala Asn Ile Ala Gly Phe
            420                 425                 430

Val Lys Val Ala Asp Ala Met Leu Ala Gln Gly Phe
        435                 440

<210> 153
<211> 867
<212> DNA
<213> Neisseria meningitidis

<220>
<221> CDS
<222> (1)..(867)

<400> 153
atg aaa ccc ata cgg aaa gcc gtc ttc ccc gtc gca ggg atg gga aca    48
Met Lys Pro Ile Arg Lys Ala Val Phe Pro Val Ala Gly Met Gly Thr
1               5                   10                  15

cgc ttc ctg ccc gcc acc aag gca agc ccg aaa gaa atg ctg ccc atc    96
Arg Phe Leu Pro Ala Thr Lys Ala Ser Pro Lys Glu Met Leu Pro Ile
            20                  25                  30

gtc gac aag ccg ctg atc caa tac gcc gta gaa gaa gcc gtg gaa gcc    144
Val Asp Lys Pro Leu Ile Gln Tyr Ala Val Glu Glu Ala Val Glu Ala
        35                  40                  45

ggc tgc acg gaa atg gtg ttt gtt acc gga cgc aac aaa cgc agc atc    192
Gly Cys Thr Glu Met Val Phe Val Thr Gly Arg Asn Lys Arg Ser Ile
    50                  55                  60

gaa gac cat ttc gac aag gca tac gaa ctc gaa acc gag ttg gaa atg    240
Glu Asp His Phe Asp Lys Ala Tyr Glu Leu Glu Thr Glu Leu Glu Met
65                  70                  75                  80

cgc cat aaa gac aaa ttg ttg gaa cac gtc cgc aac atc ctg ccg ccg    288
Arg His Lys Asp Lys Leu Leu Glu His Val Arg Asn Ile Leu Pro Pro
                85                  90                  95

aac att acc tgc ctc tac atc cgt cag gcg gaa gca ctg ggc ttg gga    336
Asn Ile Thr Cys Leu Tyr Ile Arg Gln Ala Glu Ala Leu Gly Leu Gly
            100                 105                 110

cac gcc gtc ttg tgc gcc cgc gcc gcc atc ggc gac gaa ccc ttt gcc    384
His Ala Val Leu Cys Ala Arg Ala Ala Ile Gly Asp Glu Pro Phe Ala
        115                 120                 125

gtg att ttg gct gac gat tta atc gat gcc caa aaa ggc gcg ctc aaa    432
Val Ile Leu Ala Asp Asp Leu Ile Asp Ala Gln Lys Gly Ala Leu Lys
    130                 135                 140

caa atg gtc gaa gtg tac gaa cgc agc ggc aac agc att ttg ggc gta    480
Gln Met Val Glu Val Tyr Glu Arg Ser Gly Asn Ser Ile Leu Gly Val
145                 150                 155                 160

gaa act gtc gaa ccg tcg caa acc ggc tca tac ggc atc gtc gaa acc    528
Glu Thr Val Glu Pro Ser Gln Thr Gly Ser Tyr Gly Ile Val Glu Thr
                165                 170                 175

gaa cag ctc aaa cag ttc caa cgc att acc ggc att gtc gaa aaa ccc    576
Glu Gln Leu Lys Gln Phe Gln Arg Ile Thr Gly Ile Val Glu Lys Pro
            180                 185                 190

aag ccc gaa gac gcg ccc tcc aac ctt gcc gtc gtc gga cgc tac atc    624
Lys Pro Glu Asp Ala Pro Ser Asn Leu Ala Val Val Gly Arg Tyr Ile
        195                 200                 205

ctt acc ccg cgc att ttc gac tta ctg acc gga ctg ccg cgc ggc gcg 672
Leu Thr Pro Arg Ile Phe Asp Leu Leu Thr Gly Leu Pro Arg Gly Ala
    210                 215                 220

ggc aac gaa atc cag ctt aca gac ggc atc gcc aag ctg ctc gat cac 720
Gly Asn Glu Ile Gln Leu Thr Asp Gly Ile Ala Lys Leu Leu Asp His
225                 230                 235                 240

gaa ttt gtc cta gcg cac ccc ttt gaa ggc acg cgc tac gac tgc ggc 768
Glu Phe Val Leu Ala His Pro Phe Glu Gly Thr Arg Tyr Asp Cys Gly
                245                 250                 255

agc aaa ctg ggc tat ctg gaa gcc acc gtc gcc tac ggc ctg aaa cac 816
Ser Lys Leu Gly Tyr Leu Glu Ala Thr Val Ala Tyr Gly Leu Lys His
            260                 265                 270

ccc gaa acc ggc gaa ccc ttc cgc cgc ctt ttg gaa aaa tac cgt acc 864
Pro Glu Thr Gly Glu Pro Phe Arg Arg Leu Leu Glu Lys Tyr Arg Thr
        275                 280                 285

gaa 867
Glu

<210> 154
<211> 289
<212> PRT
<213> Neisseria meningitidis

<400> 154
Met Lys Pro Ile Arg Lys Ala Val Phe Pro Val Ala Gly Met Gly Thr
1               5                   10                  15

Arg Phe Leu Pro Ala Thr Lys Ala Ser Pro Lys Glu Met Leu Pro Ile
            20                  25                  30

Val Asp Lys Pro Leu Ile Gln Tyr Ala Val Glu Glu Ala Val Glu Ala
        35                  40                  45

Gly Cys Thr Glu Met Val Phe Val Thr Gly Arg Asn Lys Arg Ser Ile
    50                  55                  60

Glu Asp His Phe Asp Lys Ala Tyr Glu Leu Glu Thr Glu Leu Glu Met
65                  70                  75                  80

Arg His Lys Asp Lys Leu Leu Glu His Val Arg Asn Ile Leu Pro Pro
                85                  90                  95

Asn Ile Thr Cys Leu Tyr Ile Arg Gln Ala Glu Ala Leu Gly Leu Gly
            100                 105                 110

His Ala Val Leu Cys Ala Arg Ala Ala Ile Gly Asp Glu Pro Phe Ala
        115                 120                 125

Val Ile Leu Ala Asp Asp Leu Ile Asp Ala Gln Lys Gly Ala Leu Lys
    130                 135                 140

Gln Met Val Glu Val Tyr Glu Arg Ser Gly Asn Ser Ile Leu Gly Val
145                 150                 155                 160

Glu Thr Val Glu Pro Ser Gln Thr Gly Ser Tyr Gly Ile Val Glu Thr
                165                 170                 175

Glu Gln Leu Lys Gln Phe Gln Arg Ile Thr Gly Ile Val Glu Lys Pro
            180                 185                 190

Lys Pro Glu Asp Ala Pro Ser Asn Leu Ala Val Val Gly Arg Tyr Ile
        195                 200                 205

Leu Thr Pro Arg Ile Phe Asp Leu Leu Thr Gly Leu Pro Arg Gly Ala
    210                 215                 220

Gly Asn Glu Ile Gln Leu Thr Asp Gly Ile Ala Lys Leu Leu Asp His
225                 230                 235                 240

Glu Phe Val Leu Ala His Pro Phe Glu Gly Thr Arg Tyr Asp Cys Gly
                245                 250                 255

Ser Lys Leu Gly Tyr Leu Glu Ala Thr Val Ala Tyr Gly Leu Lys His
            260                 265                 270

Pro Glu Thr Gly Glu Pro Phe Arg Arg Leu Leu Glu Lys Tyr Arg Thr
        275                 280                 285

Glu

<210> 155
<211> 876
<212> DNA
<213> Neisseria meningitidis

<220>
<221> CDS

<222> (1)..(876)

<400> 155
ttg acc gtc cga acg aaa aag acg gcg cat tat acc cta ttc cat tcc 48
Leu Thr Val Arg Thr Lys Lys Thr Ala His Tyr Thr Leu Phe His Ser
1               5                   10                  15

gac cga aaa ccg aac atg act act ctc aaa ccc gcc ctg ccc gct tat 96
Asp Arg Lys Pro Asn Met Thr Thr Leu Lys Pro Ala Leu Pro Ala Tyr
            20                  25                  30

ctg gac aac atc cgc atc atc ctc acg cgc acc agc cat ccc gcc aac 144
Leu Asp Asn Ile Arg Ile Ile Leu Thr Arg Thr Ser His Pro Ala Asn
        35                  40                  45

atc ggc tct gcc gcg cgc gcg atg aaa aca atg ggt ctg cac aaa ctg 192
Ile Gly Ser Ala Ala Arg Ala Met Lys Thr Met Gly Leu His Lys Leu
    50                  55                  60

acc atc gtc gcc cca aat ctg atg gca acg ccg atg acg gaa aac ccg 240
Thr Ile Val Ala Pro Asn Leu Met Ala Thr Pro Met Thr Glu Asn Pro
65                  70                  75                  80

ccc gtg ttt gac ccg gag cat cct caa tcg ttt aaa tta ccg gaa gaa 288
Pro Val Phe Asp Pro Glu His Pro Gln Ser Phe Lys Leu Pro Glu Glu
                85                  90                  95

agc ttc atc ctc gct tcc ggc gcg gca gac gtt ttg gaa aat gcc acc 336
Ser Phe Ile Leu Ala Ser Gly Ala Ala Asp Val Leu Glu Asn Ala Thr
            100                 105                 110

att gcc gct tct ttg gac gaa gcc ctt gcc gac acc acc atc gcc tgc 384
Ile Ala Ala Ser Leu Asp Glu Ala Leu Ala Asp Thr Thr Ile Ala Cys
        115                 120                 125

gcc ctg acc agc cgc cgc cgc gaa att act gcg ccg ctg caa acc ccg 432
Ala Leu Thr Ser Arg Arg Arg Glu Ile Thr Ala Pro Leu Gln Thr Pro
    130                 135                 140

cgc gat ttg gta tcc gaa tta ctg cag acc gca aac cga ggc gag aaa 480
Arg Asp Leu Val Ser Glu Leu Leu Gln Thr Ala Asn Arg Gly Glu Lys
145                 150                 155                 160

gtg gca ctg gtt ttc ggc aac gag act ttc ggc ttg agc atc gaa gaa 528
Val Ala Leu Val Phe Gly Asn Glu Thr Phe Gly Leu Ser Ile Glu Glu
                165                 170                 175

gtc caa gcc tgc aac cga ctg atg acc atc aac ggc aat ccc gac tat 576

Val Gln Ala Cys Asn Arg Leu Met Thr Ile Asn Gly Asn Pro Asp Tyr
            180                 185                 190

ttc tcg ctc aac ctc gcc caa gcc gtg cag gtc gtg tgc tac gaa atc     624
Phe Ser Leu Asn Leu Ala Gln Ala Val Gln Val Val Cys Tyr Glu Ile
        195                 200                 205

ttc agc caa acc ggt tcg ccc atg acc cat ctt caa caa gaa gac cac     672
Phe Ser Gln Thr Gly Ser Pro Met Thr His Leu Gln Gln Glu Asp His
    210                 215                 220

gct gcg acc cac gag caa atc aaa ggc atg gtc gcc cac atg gaa agc     720
Ala Ala Thr His Glu Gln Ile Lys Gly Met Val Ala His Met Glu Ser
225                 230                 235                 240

gtg atg aac gac atc ggc ttt ttc aac cgc cgc aac ggc gag cgt ctg     768
Val Met Asn Asp Ile Gly Phe Phe Asn Arg Arg Asn Gly Glu Arg Leu
                245                 250                 255

atg cgc cgt atg cag agc ctg ttc gga cgc gcc aac acg caa acc gaa     816
Met Arg Arg Met Gln Ser Leu Phe Gly Arg Ala Asn Thr Gln Thr Glu
            260                 265                 270

gac atc gat atc ctg cgc ggt ttt ttc aat acc gtc agc cat cgt atc     864
Asp Ile Asp Ile Leu Arg Gly Phe Phe Asn Thr Val Ser His Arg Ile
        275                 280                 285

cat aaa aaa gac                                                     876
His Lys Lys Asp
    290

<210> 156
<211> 292
<212> PRT
<213> Neisseria meningitidis

<400> 156
Leu Thr Val Arg Thr Lys Lys Thr Ala His Tyr Thr Leu Phe His Ser
  1               5                  10                  15

Asp Arg Lys Pro Asn Met Thr Thr Leu Lys Pro Ala Leu Pro Ala Tyr
             20                  25                  30

Leu Asp Asn Ile Arg Ile Ile Leu Thr Arg Thr Ser His Pro Ala Asn
         35                  40                  45

Ile Gly Ser Ala Ala Arg Ala Met Lys Thr Met Gly Leu His Lys Leu

50                    55                    60

Thr Ile Val Ala Pro Asn Leu Met Ala Thr Pro Met Thr Glu Asn Pro
65                  70                  75                  80

Pro Val Phe Asp Pro Glu His Pro Gln Ser Phe Lys Leu Pro Glu Glu
                85                  90                  95

Ser Phe Ile Leu Ala Ser Gly Ala Ala Asp Val Leu Glu Asn Ala Thr
            100                 105                 110

Ile Ala Ala Ser Leu Asp Glu Ala Leu Ala Asp Thr Thr Ile Ala Cys
        115                 120                 125

Ala Leu Thr Ser Arg Arg Arg Glu Ile Thr Ala Pro Leu Gln Thr Pro
    130                 135                 140

Arg Asp Leu Val Ser Glu Leu Leu Gln Thr Ala Asn Arg Gly Glu Lys
145                 150                 155                 160

Val Ala Leu Val Phe Gly Asn Glu Thr Phe Gly Leu Ser Ile Glu Glu
                165                 170                 175

Val Gln Ala Cys Asn Arg Leu Met Thr Ile Asn Gly Asn Pro Asp Tyr
            180                 185                 190

Phe Ser Leu Asn Leu Ala Gln Ala Val Gln Val Val Cys Tyr Glu Ile
        195                 200                 205

Phe Ser Gln Thr Gly Ser Pro Met Thr His Leu Gln Gln Glu Asp His
    210                 215                 220

Ala Ala Thr His Glu Gln Ile Lys Gly Met Val Ala His Met Glu Ser
225                 230                 235                 240

Val Met Asn Asp Ile Gly Phe Phe Asn Arg Arg Asn Gly Glu Arg Leu
                245                 250                 255

Met Arg Arg Met Gln Ser Leu Phe Gly Arg Ala Asn Thr Gln Thr Glu
            260                 265                 270

Asp Ile Asp Ile Leu Arg Gly Phe Phe Asn Thr Val Ser His Arg Ile
        275                 280                 285

His Lys Lys Asp
    290

<210> 157
<211> 1008
<212> DNA
<213> Neisseria meningitidis

<220>
<221> CDS
<222> (1)..(1008)

<400> 157
atg tcc atc aaa atc ctg att att tcc ccc agt tgg ata ggc gac tgc    48
Met Ser Ile Lys Ile Leu Ile Ile Ser Pro Ser Trp Ile Gly Asp Cys
1               5                   10                  15

gtg atg acc cag ccc ttg ttc cgc cgt ttg aag gaa ctt cac ccc ggt    96
Val Met Thr Gln Pro Leu Phe Arg Arg Leu Lys Glu Leu His Pro Gly
            20                  25                  30

tgc acg att gat gtg ttc gca ccg aag tgg tcg atg gcg gtg ttt gag    144
Cys Thr Ile Asp Val Phe Ala Pro Lys Trp Ser Met Ala Val Phe Glu
        35                  40                  45

cgt atg ccg gaa gtg aat gag att ctt gaa aat ccg ttc gga cac ggt    192
Arg Met Pro Glu Val Asn Glu Ile Leu Glu Asn Pro Phe Gly His Gly
    50                  55                  60

gcg ttg gag ttg aaa cgc cgt tgg cgg gtc ggt agg gat ttg ggg cgg    240
Ala Leu Glu Leu Lys Arg Arg Trp Arg Val Gly Arg Asp Leu Gly Arg
65                  70                  75                  80

cgc gga tac gat cag gtt atc gtg ttg ccc ggt tct ttg aaa tcg gca    288
Arg Gly Tyr Asp Gln Val Ile Val Leu Pro Gly Ser Leu Lys Ser Ala
                85                  90                  95

atc atc gcg ctg gcg aca ggt atc ggt aaa agg acg ggt tat gtc ggt    336
Ile Ile Ala Leu Ala Thr Gly Ile Gly Lys Arg Thr Gly Tyr Val Gly
            100                 105                 110

gaa agc cgt tat ttt ctg ttg aac gat ata cgc agg ctg gat aag gaa    384
Glu Ser Arg Tyr Phe Leu Leu Asn Asp Ile Arg Arg Leu Asp Lys Glu
        115                 120                 125

cgt ctg cct ttg atg gtg gac aga tat acg gct ctc gcg cat ccg agc    432
Arg Leu Pro Leu Met Val Asp Arg Tyr Thr Ala Leu Ala His Pro Ser
    130                 135                 140

cag gag gat ttt gac ggg cat tcg gga ttc ccc gag ttt tcc att gat    480

Gln Glu Asp Phe Asp Gly His Ser Gly Phe Pro Glu Phe Ser Ile Asp
145                 150                 155                 160

gaa cgg cgg cgg gaa att tct gtc gaa acc ttt ggc ttg gat att gga     528
Glu Arg Arg Arg Glu Ile Ser Val Glu Thr Phe Gly Leu Asp Ile Gly
                165                 170                 175

aag cct gtt ctg gct ttt tgt ccg ggt gcg gaa ttc ggg ccg gca aag     576
Lys Pro Val Leu Ala Phe Cys Pro Gly Ala Glu Phe Gly Pro Ala Lys
            180                 185                 190

cgt tgg ccg aca agg tat ttt gcc gag ttg ggc aaa cat tat tcg gcg     624
Arg Trp Pro Thr Arg Tyr Phe Ala Glu Leu Gly Lys His Tyr Ser Ala
        195                 200                 205

gcg ggt tgg cag gtt tgg ctg ttc ggt tcg caa aaa gat gat gaa att     672
Ala Gly Trp Gln Val Trp Leu Phe Gly Ser Gln Lys Asp Asp Glu Ile
    210                 215                 220

gcc gag gaa atc aac tgc ctt tca gac ggt atg tgt gtc aat ttg tgc     720
Ala Glu Glu Ile Asn Cys Leu Ser Asp Gly Met Cys Val Asn Leu Cys
225                 230                 235                 240

ggc aaa acc gat ttg tcg cag gca atg gat ttg ctg tcg ttg gcg gac     768
Gly Lys Thr Asp Leu Ser Gln Ala Met Asp Leu Leu Ser Leu Ala Asp
                245                 250                 255

acg gtc gtg tgc aac gac agc ggg ctg atg cac ttg gcg gcg gca ttg     816
Thr Val Val Cys Asn Asp Ser Gly Leu Met His Leu Ala Ala Ala Leu
            260                 265                 270

ggc agg aag gtg gtg gcg gtt tac ggt tct tcc agc ccg acg cat acg     864
Gly Arg Lys Val Val Ala Val Tyr Gly Ser Ser Ser Pro Thr His Thr
        275                 280                 285

ccg cct ttg agc gac agg gcg aaa atc gtc agc ctg cac ttg gaa tgt     912
Pro Pro Leu Ser Asp Arg Ala Lys Ile Val Ser Leu His Leu Glu Cys
    290                 295                 300

tcg ccc tgt ttt aag cgg gaa tgt ccg ctg ggg cat acc gac tgc ctc     960
Ser Pro Cys Phe Lys Arg Glu Cys Pro Leu Gly His Thr Asp Cys Leu
305                 310                 315                 320

aac agg ctg tat ccc gag aag att gtg cag gcg gtt gaa gag gcg gta     1008
Asn Arg Leu Tyr Pro Glu Lys Ile Val Gln Ala Val Glu Glu Ala Val
                325                 330                 335

<210> 158
<211> 336
<212> PRT
<213> Neisseria meningitidis

<400> 158
Met Ser Ile Lys Ile Leu Ile Ile Ser Pro Ser Trp Ile Gly Asp Cys
1               5                   10                  15

Val Met Thr Gln Pro Leu Phe Arg Arg Leu Lys Glu Leu His Pro Gly
            20                  25                  30

Cys Thr Ile Asp Val Phe Ala Pro Lys Trp Ser Met Ala Val Phe Glu
        35                  40                  45

Arg Met Pro Glu Val Asn Glu Ile Leu Glu Asn Pro Phe Gly His Gly
    50                  55                  60

Ala Leu Glu Leu Lys Arg Arg Trp Arg Val Gly Arg Asp Leu Gly Arg
65                  70                  75                  80

Arg Gly Tyr Asp Gln Val Ile Val Leu Pro Gly Ser Leu Lys Ser Ala
                85                  90                  95

Ile Ile Ala Leu Ala Thr Gly Ile Gly Lys Arg Thr Gly Tyr Val Gly
            100                 105                 110

Glu Ser Arg Tyr Phe Leu Leu Asn Asp Ile Arg Arg Leu Asp Lys Glu
        115                 120                 125

Arg Leu Pro Leu Met Val Asp Arg Tyr Thr Ala Leu Ala His Pro Ser
    130                 135                 140

Gln Glu Asp Phe Asp Gly His Ser Gly Phe Pro Glu Phe Ser Ile Asp
145                 150                 155                 160

Glu Arg Arg Arg Glu Ile Ser Val Glu Thr Phe Gly Leu Asp Ile Gly
                165                 170                 175

Lys Pro Val Leu Ala Phe Cys Pro Gly Ala Glu Phe Gly Pro Ala Lys
            180                 185                 190

Arg Trp Pro Thr Arg Tyr Phe Ala Glu Leu Gly Lys His Tyr Ser Ala
        195                 200                 205

Ala Gly Trp Gln Val Trp Leu Phe Gly Ser Gln Lys Asp Asp Glu Ile
    210                 215                 220

Ala Glu Glu Ile Asn Cys Leu Ser Asp Gly Met Cys Val Asn Leu Cys
225                 230                 235                 240

Gly Lys Thr Asp Leu Ser Gln Ala Met Asp Leu Leu Ser Leu Ala Asp
                245                 250                 255

Thr Val Val Cys Asn Asp Ser Gly Leu Met His Leu Ala Ala Ala Leu
            260                 265                 270

Gly Arg Lys Val Val Ala Val Tyr Gly Ser Ser Ser Pro Thr His Thr
        275                 280                 285

Pro Pro Leu Ser Asp Arg Ala Lys Ile Val Ser Leu His Leu Glu Cys
    290                 295                 300

Ser Pro Cys Phe Lys Arg Glu Cys Pro Leu Gly His Thr Asp Cys Leu
305                 310                 315                 320

Asn Arg Leu Tyr Pro Glu Lys Ile Val Gln Ala Val Glu Glu Ala Val
                325                 330                 335

<210> 159
<211> 381
<212> DNA
<213> Neisseria meningitidis

<220>
<221> CDS
<222> (1)..(381)

<400> 159
atg gaa cct tcc tcc tac gcg gca gaa aaa aaa gga aaa ggc ggc atc      48
Met Glu Pro Ser Ser Tyr Ala Ala Glu Lys Lys Gly Lys Gly Gly Ile
1               5                   10                  15

agg cgc gtc atc aac gca ttc ggc tat tcg ata gac ggc atc gcc gcc      96
Arg Arg Val Ile Asn Ala Phe Gly Tyr Ser Ile Asp Gly Ile Ala Ala
            20                  25                  30

gcc tac cgt tac gaa gcg gca ttc cgt cag gtt ttg tgg ctg aac gcg      144
Ala Tyr Arg Tyr Glu Ala Ala Phe Arg Gln Val Leu Trp Leu Asn Ala
        35                  40                  45

ctg ctg gtg tgc gcg gca ttt ttt tgg gtt tcc gaa aag tcc ctc cgc      192
Leu Leu Val Cys Ala Ala Phe Phe Trp Val Ser Glu Lys Ser Leu Arg
    50                  55                  60

ctg ccg ttg att atc gcg tct ttt gtg tcg gtc att gtc gaa ctg ttc 240
Leu Pro Leu Ile Ile Ala Ser Phe Val Ser Val Ile Val Glu Leu Phe
65                  70                  75                  80

aac act gcc gtc gaa gcc gcc gtc gat cat act tcg act gaa aaa cac 288
Asn Thr Ala Val Glu Ala Ala Val Asp His Thr Ser Thr Glu Lys His
                85                  90                  95

gag ctg gct aaa cgc gcc aaa gac gca ggt tct gct gca caa ttg ttc 336
Glu Leu Ala Lys Arg Ala Lys Asp Ala Gly Ser Ala Ala Gln Leu Phe
            100                 105                 110

gcg atg ctg atg tta gcg gcg gtt tgg ctg tcc gcc ctg ttc ggg 381
Ala Met Leu Met Leu Ala Ala Val Trp Leu Ser Ala Leu Phe Gly
        115                 120                 125

<210> 160
<211> 127
<212> PRT
<213> Neisseria meningitidis

<400> 160
Met Glu Pro Ser Ser Tyr Ala Ala Glu Lys Lys Gly Lys Gly Gly Ile
1               5                   10                  15

Arg Arg Val Ile Asn Ala Phe Gly Tyr Ser Ile Asp Gly Ile Ala Ala
            20                  25                  30

Ala Tyr Arg Tyr Glu Ala Ala Phe Arg Gln Val Leu Trp Leu Asn Ala
        35                  40                  45

Leu Leu Val Cys Ala Ala Phe Phe Trp Val Ser Glu Lys Ser Leu Arg
    50                  55                  60

Leu Pro Leu Ile Ile Ala Ser Phe Val Ser Val Ile Val Glu Leu Phe
65                  70                  75                  80

Asn Thr Ala Val Glu Ala Ala Val Asp His Thr Ser Thr Glu Lys His
                85                  90                  95

Glu Leu Ala Lys Arg Ala Lys Asp Ala Gly Ser Ala Ala Gln Leu Phe
            100                 105                 110

Ala Met Leu Met Leu Ala Ala Val Trp Leu Ser Ala Leu Phe Gly
        115                 120                 125

<210> 161
<211> 990
<212> DNA
<213> Neisseria meningitidis

<220>
<221> CDS
<222> (1)..(990)

<400> 161
atg aaa aaa gaa agc cgc ccc gcg ttt gac gcg gca gcc gta ttt gac    48
Met Lys Lys Glu Ser Arg Pro Ala Phe Asp Ala Ala Ala Val Phe Asp
1               5                   10                  15

gcg gca gcc gta ccg gta tcc gac agc ggg ttt gcc gcc aat gca aat    96
Ala Ala Ala Val Pro Val Ser Asp Ser Gly Phe Ala Ala Asn Ala Asn
            20                  25                  30

gtc cgc cgt ttt gtg gac gat gaa gtc ggg aaa ggg gat ttt tcc cgg    144
Val Arg Arg Phe Val Asp Asp Glu Val Gly Lys Gly Asp Phe Ser Arg
        35                  40                  45

gcg gaa tgg cag gat ttt ttt gac aaa gcg gct tac aag gcg gac atc    192
Ala Glu Trp Gln Asp Phe Phe Asp Lys Ala Ala Tyr Lys Ala Asp Ile
    50                  55                  60

gtc aag att atg cac cgc ccc tcc aca tcg cgt ccg tgg tat gtg ttc    240
Val Lys Ile Met His Arg Pro Ser Thr Ser Arg Pro Trp Tyr Val Phe
65                  70                  75                  80

cgc acg gga aat tcg ggc aag gcg aaa ttt cgc ggc gcg cgc cgg ttt    288
Arg Thr Gly Asn Ser Gly Lys Ala Lys Phe Arg Gly Ala Arg Arg Phe
                85                  90                  95

tat gcg gaa aac cgc gcg ctt atc gat gat gtg gcg caa aaa tac ggc    336
Tyr Ala Glu Asn Arg Ala Leu Ile Asp Asp Val Ala Gln Lys Tyr Gly
            100                 105                 110

gtg cct gcc gaa ctt atc gtg gcg gtt atc ggg att gaa acg aat tac    384
Val Pro Ala Glu Leu Ile Val Ala Val Ile Gly Ile Glu Thr Asn Tyr
        115                 120                 125

ggc aaa aat acg ggc agt ttc cgt gtg gcg gac gca ttg gcg acc tta    432
Gly Lys Asn Thr Gly Ser Phe Arg Val Ala Asp Ala Leu Ala Thr Leu
    130                 135                 140

ggc ttt gat tac ccc cgc cgc gcc ggg ttt ttc caa aaa gaa ttg gtc 480
Gly Phe Asp Tyr Pro Arg Arg Ala Gly Phe Phe Gln Lys Glu Leu Val
145                 150                 155                 160

gag ctt tta aag ctg gca aaa gaa gaa ggc ggc gat gtt ttc gcc ttt 528
Glu Leu Leu Lys Leu Ala Lys Glu Glu Gly Gly Asp Val Phe Ala Phe
                165                 170                 175

aaa ggc agc tat gcg ggc gca atg ggg atg ccg caa ttt atg cct tcg 576
Lys Gly Ser Tyr Ala Gly Ala Met Gly Met Pro Gln Phe Met Pro Ser
            180                 185                 190

agc tac cgg aaa tgg gcg gtg gat tat gac ggg gac gga cat cgg gac 624
Ser Tyr Arg Lys Trp Ala Val Asp Tyr Asp Gly Asp Gly His Arg Asp
        195                 200                 205

ata tgg ggc aat gtt ggc gat gtc gcg gca tcg att gcc aat tat atg 672
Ile Trp Gly Asn Val Gly Asp Val Ala Ala Ser Ile Ala Asn Tyr Met
    210                 215                 220

aag cag cac ggt tgg cgc acg ggc ggg aaa ata ctg gtg tct gca aca 720
Lys Gln His Gly Trp Arg Thr Gly Gly Lys Ile Leu Val Ser Ala Thr
225                 230                 235                 240

ttg gcg ccg ggt gcg gat gtt cag gca atc att ggc gaa aaa acc gcc 768
Leu Ala Pro Gly Ala Asp Val Gln Ala Ile Ile Gly Glu Lys Thr Ala
                245                 250                 255

ctg acg cgg acg gtg gcg gat ttg aag gcg tac ggc atc atc ccc ggc 816
Leu Thr Arg Thr Val Ala Asp Leu Lys Ala Tyr Gly Ile Ile Pro Gly
            260                 265                 270

gaa gag ctt gcc gat gat gaa aag gcg gtt ttg ttc aaa ctg gaa acc 864
Glu Glu Leu Ala Asp Asp Glu Lys Ala Val Leu Phe Lys Leu Glu Thr
        275                 280                 285

gca ccc ggc gtg ttt gaa tat tat ttg ggc ttg aac aat ttt tat acg 912
Ala Pro Gly Val Phe Glu Tyr Tyr Leu Gly Leu Asn Asn Phe Tyr Thr
    290                 295                 300

gta tgg cag tac aat cac agt cgg atg tat gta acg gcg gtc agg gac 960
Val Trp Gln Tyr Asn His Ser Arg Met Tyr Val Thr Ala Val Arg Asp
305                 310                 315                 320

att gcc aat tcg ctt ggc ggc ccg gga ttg 990
Ile Ala Asn Ser Leu Gly Gly Pro Gly Leu
                325                 330

<210> 162
<211> 330
<212> PRT
<213> Neisseria meningitidis

<400> 162
Met Lys Lys Glu Ser Arg Pro Ala Phe Asp Ala Ala Ala Val Phe Asp
1               5                   10                  15

Ala Ala Ala Val Pro Val Ser Asp Ser Gly Phe Ala Ala Asn Ala Asn
            20                  25                  30

Val Arg Arg Phe Val Asp Asp Glu Val Gly Lys Gly Asp Phe Ser Arg
        35                  40                  45

Ala Glu Trp Gln Asp Phe Phe Asp Lys Ala Ala Tyr Lys Ala Asp Ile
    50                  55                  60

Val Lys Ile Met His Arg Pro Ser Thr Ser Arg Pro Trp Tyr Val Phe
65                  70                  75                  80

Arg Thr Gly Asn Ser Gly Lys Ala Lys Phe Arg Gly Ala Arg Arg Phe
                85                  90                  95

Tyr Ala Glu Asn Arg Ala Leu Ile Asp Asp Val Ala Gln Lys Tyr Gly
            100                 105                 110

Val Pro Ala Glu Leu Ile Val Ala Val Ile Gly Ile Glu Thr Asn Tyr
        115                 120                 125

Gly Lys Asn Thr Gly Ser Phe Arg Val Ala Asp Ala Leu Ala Thr Leu
    130                 135                 140

Gly Phe Asp Tyr Pro Arg Arg Ala Gly Phe Phe Gln Lys Glu Leu Val
145                 150                 155                 160

Glu Leu Leu Lys Leu Ala Lys Glu Glu Gly Gly Asp Val Phe Ala Phe
                165                 170                 175

Lys Gly Ser Tyr Ala Gly Ala Met Gly Met Pro Gln Phe Met Pro Ser
            180                 185                 190

Ser Tyr Arg Lys Trp Ala Val Asp Tyr Asp Gly Asp Gly His Arg Asp
        195                 200                 205

Ile Trp Gly Asn Val Gly Asp Val Ala Ala Ser Ile Ala Asn Tyr Met
    210                 215                 220

Lys Gln His Gly Trp Arg Thr Gly Gly Lys Ile Leu Val Ser Ala Thr
225                 230                 235                 240

Leu Ala Pro Gly Ala Asp Val Gln Ala Ile Ile Gly Glu Lys Thr Ala
                245                 250                 255

Leu Thr Arg Thr Val Ala Asp Leu Lys Ala Tyr Gly Ile Ile Pro Gly
            260                 265                 270

Glu Glu Leu Ala Asp Asp Glu Lys Ala Val Leu Phe Lys Leu Glu Thr
        275                 280                 285

Ala Pro Gly Val Phe Glu Tyr Tyr Leu Gly Leu Asn Asn Phe Tyr Thr
    290                 295                 300

Val Trp Gln Tyr Asn His Ser Arg Met Tyr Val Thr Ala Val Arg Asp
305                 310                 315                 320

Ile Ala Asn Ser Leu Gly Gly Pro Gly Leu
                325                 330

<210> 163
<211> 1773
<212> DNA
<213> Neisseria meningitidis

<220>
<221> CDS
<222> (1)..(1773)

<400> 163
atg agt atc gtg ctg cac ggc gtg gcg gcg ggc aaa ggc att gcc gtc     48
Met Ser Ile Val Leu His Gly Val Ala Ala Gly Lys Gly Ile Ala Val
  1               5                  10                  15

ggt tgc gcc cac ctg att gcg cgc ggt acg gag gaa gtg ccg cag tat     96
Gly Cys Ala His Leu Ile Ala Arg Gly Thr Glu Glu Val Pro Gln Tyr
             20                  25                  30

gat gtt gcg gag gcg gac acc gat gcc gaa gcc gaa cgt ttc gat gcc    144
Asp Val Ala Glu Ala Asp Thr Asp Ala Glu Ala Glu Arg Phe Asp Ala
         35                  40                  45

gcc gtc aaa gcc acg cgc aaa gag ttg gaa cag ctc cgc agc gcg att    192
Ala Val Lys Ala Thr Arg Lys Glu Leu Glu Gln Leu Arg Ser Ala Ile

    50                  55                  60

ccc gaa aac gcc ccg acc gag ttg ggc gcg ttc atc tcg ctg cac ctg 240
Pro Glu Asn Ala Pro Thr Glu Leu Gly Ala Phe Ile Ser Leu His Leu
65                  70                  75                  80

atg ctc ttg acc gat gtt acc ttg tcg cgc gaa ccc gtc gat att tta 288
Met Leu Leu Thr Asp Val Thr Leu Ser Arg Glu Pro Val Asp Ile Leu
                85                  90                  95

agg gaa caa aaa atc aac gcc gag tgg gca ttg aag cag cag agc gac 336
Arg Glu Gln Lys Ile Asn Ala Glu Trp Ala Leu Lys Gln Gln Ser Asp
            100                 105                 110

aaa ctc gcc gcc caa ttc gac aat atg gac gat gcc tat ttg cgc gaa 384
Lys Leu Ala Ala Gln Phe Asp Asn Met Asp Asp Ala Tyr Leu Arg Glu
        115                 120                 125

cgc aag cag gat atg ctg caa gtc gtc cgc cgc atc cac aac aac ctg 432
Arg Lys Gln Asp Met Leu Gln Val Val Arg Arg Ile His Asn Asn Leu
    130                 135                 140

atc ggg cag ggc aac gag ttg gaa gtt gcc gac aac ctg ttt gac gaa 480
Ile Gly Gln Gly Asn Glu Leu Glu Val Ala Asp Asn Leu Phe Asp Glu
145                 150                 155                 160

acc gtt ctg att gca aac gac ctt tcg ccc gcc gac acg gtt ttg ttt 528
Thr Val Leu Ile Ala Asn Asp Leu Ser Pro Ala Asp Thr Val Leu Phe
                165                 170                 175

aaa gag cag cgc att gcc gcc ttc gtt acc gat gcc ggc ggc ccc acc 576
Lys Glu Gln Arg Ile Ala Ala Phe Val Thr Asp Ala Gly Gly Pro Thr
            180                 185                 190

ggg cat acg gcg att ttg ggc agg agc ttg gac atc ccg tcc gtc gtc 624
Gly His Thr Ala Ile Leu Gly Arg Ser Leu Asp Ile Pro Ser Val Val
        195                 200                 205

ggg ctg cac aac gcg cgc aaa ctg att acc gag ggc gaa acg gtc att 672
Gly Leu His Asn Ala Arg Lys Leu Ile Thr Glu Gly Glu Thr Val Ile
    210                 215                 220

gtg gac ggt atc aac ggc gtg ttg att atc gcg ccg gat gag tcg gtg 720
Val Asp Gly Ile Asn Gly Val Leu Ile Ile Ala Pro Asp Glu Ser Val
225                 230                 235                 240

ttg aac gaa tac cgc cgc cgt gcc cgc gaa tac cgc agc cac aaa cgc 768
Leu Asn Glu Tyr Arg Arg Arg Ala Arg Glu Tyr Arg Ser His Lys Arg

                245                 250                 255

gat ttg aac aag ctc aaa aaa acc gcc gcc gcc acc gct gac ggg gtc 816
Asp Leu Asn Lys Leu Lys Lys Thr Ala Ala Ala Thr Ala Asp Gly Val
            260                 265                 270

tgc atc gag ctt gtg ggc aat ata gaa tcc gcc gaa gac gtg aaa ccg 864
Cys Ile Glu Leu Val Gly Asn Ile Glu Ser Ala Glu Asp Val Lys Pro
        275                 280                 285

ctg cac aac ctc ggc gca gac ggc atc ggg ctg ttc cgc agc gag ttt 912
Leu His Asn Leu Gly Ala Asp Gly Ile Gly Leu Phe Arg Ser Glu Phe
    290                 295                 300

ctt tac ctg aac cgc gat acg atg ccg tct gaa gac gag cag tac gaa 960
Leu Tyr Leu Asn Arg Asp Thr Met Pro Ser Glu Asp Glu Gln Tyr Glu
305                 310                 315                 320

gtg tac agc gcg att gtc aaa aaa atg aaa ggc aaa agc gta acg ata 1008
Val Tyr Ser Ala Ile Val Lys Lys Met Lys Gly Lys Ser Val Thr Ile
                325                 330                 335

cgg aca gtc gat ttg ggt gtg gac aaa aac ccg cgc tgg ttc ggg aaa 1056
Arg Thr Val Asp Leu Gly Val Asp Lys Asn Pro Arg Trp Phe Gly Lys
            340                 345                 350

aac agc acg ccc aac ggc agc ctc aac ccc gcg ctg ggc atg acc ggc 1104
Asn Ser Thr Pro Asn Gly Ser Leu Asn Pro Ala Leu Gly Met Thr Gly
        355                 360                 365

atc cgc ctg tgc ctt gcc gaa ccg gtc atg ttc cgc acc cag atg cgc 1152
Ile Arg Leu Cys Leu Ala Glu Pro Val Met Phe Arg Thr Gln Met Arg
    370                 375                 380

gcc atc ctc cgc gcc gcc gcc cac ggc ccc gtg cgg atg atg tgg ccg 1200
Ala Ile Leu Arg Ala Ala Ala His Gly Pro Val Arg Met Met Trp Pro
385                 390                 395                 400

atg att acc tcc gta tcc gaa gtg cgc cag tgc ctc atc cac ctc gac 1248
Met Ile Thr Ser Val Ser Glu Val Arg Gln Cys Leu Ile His Leu Asp
                405                 410                 415

acc gcg caa cgc cag ctt gcc gaa cgc ggc gat gcc ttc ggt aaa gtc 1296
Thr Ala Gln Arg Gln Leu Ala Glu Arg Gly Asp Ala Phe Gly Lys Val
            420                 425                 430

ggc atc ggc tgt atg att gaa att ccg tct gcc gcg ctg acc gtc ggc 1344
Gly Ile Gly Cys Met Ile Glu Ile Pro Ser Ala Ala Leu Thr Val Gly

435                 440                 445

agt att ttg aaa ctg gtc gat ttc atc tcc gtc ggt acc aac gac ctg    1392
Ser Ile Leu Lys Leu Val Asp Phe Ile Ser Val Gly Thr Asn Asp Leu
    450                 455                 460

att caa tac atc ttg tcc gtc gat cgc ggc gac gac agc gtc agc cac    1440
Ile Gln Tyr Ile Leu Ser Val Asp Arg Gly Asp Asp Ser Val Ser His
465                 470                 475                 480

ctc tac cag ccc ggc cat ccc gcc gtg ctg aaa atg ctg caa cac gtc    1488
Leu Tyr Gln Pro Gly His Pro Ala Val Leu Lys Met Leu Gln His Val
                485                 490                 495

atc cgt acc gcc aac cgc atg gac aaa gac gta tcc gta tgc ggc gag    1536
Ile Arg Thr Ala Asn Arg Met Asp Lys Asp Val Ser Val Cys Gly Glu
            500                 505                 510

atg gcg ggc gat acc gcg ttt acc cgc gtt tta ttg ggt atg ggg ctg    1584
Met Ala Gly Asp Thr Ala Phe Thr Arg Val Leu Leu Gly Met Gly Leu
        515                 520                 525

cgc cgt ttt tcc atg aac ccc aac aac atc ctg ccc gtc aaa aac atc    1632
Arg Arg Phe Ser Met Asn Pro Asn Asn Ile Leu Pro Val Lys Asn Ile
    530                 535                 540

att ctg cac agc aat gtc gga cag ctc gaa agc gat att gtg aaa gtc    1680
Ile Leu His Ser Asn Val Gly Gln Leu Glu Ser Asp Ile Val Lys Val
545                 550                 555                 560

atc cgc tgc gaa gac gaa gag aag tcg gaa aag ctg atc aaa cag atg    1728
Ile Arg Cys Glu Asp Glu Glu Lys Ser Glu Lys Leu Ile Lys Gln Met
                565                 570                 575

aac agc gtg tct gtc gag gaa gaa gcc gac ttc aag ggg cgg aaa        1773
Asn Ser Val Ser Val Glu Glu Glu Ala Asp Phe Lys Gly Arg Lys
            580                 585                 590

<210> 164
<211> 591
<212> PRT
<213> Neisseria meningitidis

<400> 164
Met Ser Ile Val Leu His Gly Val Ala Ala Gly Lys Gly Ile Ala Val
1               5                   10                  15

Gly Cys Ala His Leu Ile Ala Arg Gly Thr Glu Glu Val Pro Gln Tyr
            20                  25                  30

Asp Val Ala Glu Ala Asp Thr Asp Ala Glu Ala Glu Arg Phe Asp Ala
        35                  40                  45

Ala Val Lys Ala Thr Arg Lys Glu Leu Glu Gln Leu Arg Ser Ala Ile
    50                  55                  60

Pro Glu Asn Ala Pro Thr Glu Leu Gly Ala Phe Ile Ser Leu His Leu
65                  70                  75                  80

Met Leu Leu Thr Asp Val Thr Leu Ser Arg Glu Pro Val Asp Ile Leu
                85                  90                  95

Arg Glu Gln Lys Ile Asn Ala Glu Trp Ala Leu Lys Gln Gln Ser Asp
            100                 105                 110

Lys Leu Ala Ala Gln Phe Asp Asn Met Asp Asp Ala Tyr Leu Arg Glu
        115                 120                 125

Arg Lys Gln Asp Met Leu Gln Val Val Arg Arg Ile His Asn Asn Leu
    130                 135                 140

Ile Gly Gln Gly Asn Glu Leu Glu Val Ala Asp Asn Leu Phe Asp Glu
145                 150                 155                 160

Thr Val Leu Ile Ala Asn Asp Leu Ser Pro Ala Asp Thr Val Leu Phe
                165                 170                 175

Lys Glu Gln Arg Ile Ala Ala Phe Val Thr Asp Ala Gly Gly Pro Thr
            180                 185                 190

Gly His Thr Ala Ile Leu Gly Arg Ser Leu Asp Ile Pro Ser Val Val
        195                 200                 205

Gly Leu His Asn Ala Arg Lys Leu Ile Thr Glu Gly Glu Thr Val Ile
    210                 215                 220

Val Asp Gly Ile Asn Gly Val Leu Ile Ile Ala Pro Asp Glu Ser Val
225                 230                 235                 240

Leu Asn Glu Tyr Arg Arg Arg Ala Arg Glu Tyr Arg Ser His Lys Arg
                245                 250                 255

Asp Leu Asn Lys Leu Lys Lys Thr Ala Ala Ala Thr Ala Asp Gly Val
            260                 265                 270

Cys Ile Glu Leu Val Gly Asn Ile Glu Ser Ala Glu Asp Val Lys Pro
        275                 280                 285

Leu His Asn Leu Gly Ala Asp Gly Ile Gly Leu Phe Arg Ser Glu Phe
    290                 295                 300

Leu Tyr Leu Asn Arg Asp Thr Met Pro Ser Glu Asp Glu Gln Tyr Glu
305                 310                 315                 320

Val Tyr Ser Ala Ile Val Lys Lys Met Lys Gly Lys Ser Val Thr Ile
                325                 330                 335

Arg Thr Val Asp Leu Gly Val Asp Lys Asn Pro Arg Trp Phe Gly Lys
            340                 345                 350

Asn Ser Thr Pro Asn Gly Ser Leu Asn Pro Ala Leu Gly Met Thr Gly
        355                 360                 365

Ile Arg Leu Cys Leu Ala Glu Pro Val Met Phe Arg Thr Gln Met Arg
    370                 375                 380

Ala Ile Leu Arg Ala Ala Ala His Gly Pro Val Arg Met Met Trp Pro
385                 390                 395                 400

Met Ile Thr Ser Val Ser Glu Val Arg Gln Cys Leu Ile His Leu Asp
                405                 410                 415

Thr Ala Gln Arg Gln Leu Ala Glu Arg Gly Asp Ala Phe Gly Lys Val
            420                 425                 430

Gly Ile Gly Cys Met Ile Glu Ile Pro Ser Ala Ala Leu Thr Val Gly
        435                 440                 445

Ser Ile Leu Lys Leu Val Asp Phe Ile Ser Val Gly Thr Asn Asp Leu
    450                 455                 460

Ile Gln Tyr Ile Leu Ser Val Asp Arg Gly Asp Asp Ser Val Ser His
465                 470                 475                 480

Leu Tyr Gln Pro Gly His Pro Ala Val Leu Lys Met Leu Gln His Val
                485                 490                 495

Ile Arg Thr Ala Asn Arg Met Asp Lys Asp Val Ser Val Cys Gly Glu
            500                 505                 510

Met Ala Gly Asp Thr Ala Phe Thr Arg Val Leu Leu Gly Met Gly Leu
        515                 520                 525

Arg Arg Phe Ser Met Asn Pro Asn Asn Ile Leu Pro Val Lys Asn Ile
530                 535                 540

Ile Leu His Ser Asn Val Gly Gln Leu Glu Ser Asp Ile Val Lys Val
545                 550                 555                 560

Ile Arg Cys Glu Asp Glu Glu Lys Ser Glu Lys Leu Ile Lys Gln Met
                565                 570                 575

Asn Ser Val Ser Val Glu Glu Glu Ala Asp Phe Lys Gly Arg Lys
            580                 585                 590

<210> 165
<211> 381
<212> DNA
<213> Neisseria meningitidis

<220>
<221> CDS
<222> (1)..(381)

<400> 165
atg gaa cct tcc tcc tac gcg gca gaa aaa aaa gga aaa ggc ggc atc    48
Met Glu Pro Ser Ser Tyr Ala Ala Glu Lys Lys Gly Lys Gly Gly Ile
1               5                   10                  15

agg cgc gtc atc aac gca ttc ggc tat tcg ata gac ggc atc gcc gcc    96
Arg Arg Val Ile Asn Ala Phe Gly Tyr Ser Ile Asp Gly Ile Ala Ala
            20                  25                  30

gcc tac cgt tac gaa gcg gca ttc cgt cag gtt ttg tgg ctg aac gcg    144
Ala Tyr Arg Tyr Glu Ala Ala Phe Arg Gln Val Leu Trp Leu Asn Ala
        35                  40                  45

ctg ctg gtg tgc gcg gca ttt ttt tgg gtt tcc gaa aag tcc ctc cgc    192
Leu Leu Val Cys Ala Ala Phe Phe Trp Val Ser Glu Lys Ser Leu Arg
    50                  55                  60

ctg ccg ttg att atc gcg tct ttt gtg tcg gtc att gtc gaa ctg ttc    240
Leu Pro Leu Ile Ile Ala Ser Phe Val Ser Val Ile Val Glu Leu Phe
65                  70                  75                  80

aac act gcc gtc gaa gcc gcc gtc gat cat act tcg act gaa aaa cac    288
Asn Thr Ala Val Glu Ala Ala Val Asp His Thr Ser Thr Glu Lys His
                85                  90                  95

gag ctg gct aaa cgc gcc aaa gac gca ggt tct gct gca caa ttg ttc     336
Glu Leu Ala Lys Arg Ala Lys Asp Ala Gly Ser Ala Ala Gln Leu Phe
            100                 105                 110

gcg atg ctg atg tta gcg gcg gtt tgg ctg tcc gcc ctg ttc ggg         381
Ala Met Leu Met Leu Ala Ala Val Trp Leu Ser Ala Leu Phe Gly
        115                 120                 125

<210> 166
<211> 127
<212> PRT
<213> Neisseria meningitidis

<400> 166
Met Glu Pro Ser Ser Tyr Ala Ala Glu Lys Lys Gly Lys Gly Gly Ile
  1               5                  10                  15

Arg Arg Val Ile Asn Ala Phe Gly Tyr Ser Ile Asp Gly Ile Ala Ala
             20                  25                  30

Ala Tyr Arg Tyr Glu Ala Ala Phe Arg Gln Val Leu Trp Leu Asn Ala
         35                  40                  45

Leu Leu Val Cys Ala Ala Phe Phe Trp Val Ser Glu Lys Ser Leu Arg
     50                  55                  60

Leu Pro Leu Ile Ile Ala Ser Phe Val Ser Val Ile Val Glu Leu Phe
 65                  70                  75                  80

Asn Thr Ala Val Glu Ala Ala Val Asp His Thr Ser Thr Glu Lys His
                 85                  90                  95

Glu Leu Ala Lys Arg Ala Lys Asp Ala Gly Ser Ala Ala Gln Leu Phe
            100                 105                 110

Ala Met Leu Met Leu Ala Ala Val Trp Leu Ser Ala Leu Phe Gly
        115                 120                 125

<210> 167
<211> 1257
<212> DNA
<213> Neisseria meningitidis

<220>
<221> CDS

<222> (1)..(1257)

<400> 167
atg agt atg gca ctt gcc caa aaa ctt gcc gcc gac agc att gcg gcg 48
Met Ser Met Ala Leu Ala Gln Lys Leu Ala Ala Asp Ser Ile Ala Ala
1               5                   10                  15

gtt gcc gaa gga cgc aac ctt cag gac gtg ttg gcg caa atc cgc acc 96
Val Ala Glu Gly Arg Asn Leu Gln Asp Val Leu Ala Gln Ile Arg Thr
            20                  25                  30

gcg cat ccc gac ctt acg gcg cag gaa aac ggc gcg ttg cag gac atc 144
Ala His Pro Asp Leu Thr Ala Gln Glu Asn Gly Ala Leu Gln Asp Ile
        35                  40                  45

gcc tac ggc tgc cag cgt tat ttg ggc agt ttg aaa cat atg ctc gcg 192
Ala Tyr Gly Cys Gln Arg Tyr Leu Gly Ser Leu Lys His Met Leu Ala
    50                  55                  60

cag atg ctg aaa aag ccg att ggc aat ccg cag ctc gaa agt ctg ctt 240
Gln Met Leu Lys Lys Pro Ile Gly Asn Pro Gln Leu Glu Ser Leu Leu
65                  70                  75                  80

ttg gcg gcg ttg tac caa ctg cat tac acg cgc aac gcg ccg cat gct 288
Leu Ala Ala Leu Tyr Gln Leu His Tyr Thr Arg Asn Ala Pro His Ala
                85                  90                  95

gtg gtc aac gaa gct gtt gaa agc atc gcc aaa atc gga cgc ggg cag 336
Val Val Asn Glu Ala Val Glu Ser Ile Ala Lys Ile Gly Arg Gly Gln
            100                 105                 110

tac cgt tcg ttt gcc aac gcg att ttg cgc cgc ttt ttg cgc gaa cgc 384
Tyr Arg Ser Phe Ala Asn Ala Ile Leu Arg Arg Phe Leu Arg Glu Arg
        115                 120                 125

gac aag ctt gcg gct tcc tgc aaa aaa gac gat gtg gcg aaa cac aac 432
Asp Lys Leu Ala Ala Ser Cys Lys Lys Asp Asp Val Ala Lys His Asn
    130                 135                 140

ctg ccg ctg tgg tgg gtg gct tac ttg aaa aac cat tat ccg aaa cac 480
Leu Pro Leu Trp Trp Val Ala Tyr Leu Lys Asn His Tyr Pro Lys His
145                 150                 155                 160

tgg cac aac atc gcc gcc gcg ctg caa tcc cat ccg ccg atg acg ttg 528
Trp His Asn Ile Ala Ala Ala Leu Gln Ser His Pro Pro Met Thr Leu
                165                 170                 175

cgc gtc aac cgc cga cac ggc aac gcc gaa agc tat ttg gaa aaa ctg 576

Arg Val Asn Arg Arg His Gly Asn Ala Glu Ser Tyr Leu Glu Lys Leu
            180                 185                 190

gct gcg gaa ggc att gcg gca aag gcg ttg gac gaa tat gcg gtt acg 624
Ala Ala Glu Gly Ile Ala Ala Lys Ala Leu Asp Glu Tyr Ala Val Thr
        195                 200                 205

ttg gaa gaa gcc gtg ccg gtg aac cgc ctg ccc ggt ttt tca gac ggc 672
Leu Glu Glu Ala Val Pro Val Asn Arg Leu Pro Gly Phe Ser Asp Gly
    210                 215                 220

att gtt tcg gta cag gac ttc ggc gcg cag cag gcg gcg tat ctc ctt 720
Ile Val Ser Val Gln Asp Phe Gly Ala Gln Gln Ala Ala Tyr Leu Leu
225                 230                 235                 240

aac ccg aaa gac ggc gaa cgg att ttg gac gcg tgc gcc gcg ccg ggc 768
Asn Pro Lys Asp Gly Glu Arg Ile Leu Asp Ala Cys Ala Ala Pro Gly
                245                 250                 255

ggc aag acg ggg cat atc ttg gaa ctg gcg gat tgc cgc gtt acc gcc 816
Gly Lys Thr Gly His Ile Leu Glu Leu Ala Asp Cys Arg Val Thr Ala
            260                 265                 270

ttg gac att gat gca ggc cgt ctg aag cgt gtg gag gac aat atc gcc 864
Leu Asp Ile Asp Ala Gly Arg Leu Lys Arg Val Glu Asp Asn Ile Ala
        275                 280                 285

cgt ttg ggc ttt cag acg gca tcg gcg gcg tgt gcc gat gcg cgg gat 912
Arg Leu Gly Phe Gln Thr Ala Ser Ala Ala Cys Ala Asp Ala Arg Asp
    290                 295                 300

ttg gcg gcg tgg tat gat gga aag cct ttc gac aca atc ctt gcc gac 960
Leu Ala Ala Trp Tyr Asp Gly Lys Pro Phe Asp Thr Ile Leu Ala Asp
305                 310                 315                 320

gtg cca tgc acc gct tcc ggc gtg gcg cgg cgc aat ccc gac gtg aaa 1008
Val Pro Cys Thr Ala Ser Gly Val Ala Arg Arg Asn Pro Asp Val Lys
                325                 330                 335

tgg ctg cgc cgt ccg acc gac gcg ctc aaa acc gcc cgc cag cag gaa 1056
Trp Leu Arg Arg Pro Thr Asp Ala Leu Lys Thr Ala Arg Gln Gln Glu
            340                 345                 350

gcc ctg ctg gac gca ctt tgg cag acg ctg aaa cag ggc ggc aga atg 1104
Ala Leu Leu Asp Ala Leu Trp Gln Thr Leu Lys Gln Gly Gly Arg Met
        355                 360                 365

ctg ctt gcc acc tgt tcc gtg ttc gtc gaa gaa aac gac ggg caa ttg 1152

Leu Leu Ala Thr Cys Ser Val Phe Val Glu Glu Asn Asp Gly Gln Leu
    370                 375                 380

caa aaa ttc ctc aac cgc cat gcc gat gcc gaa ccg atc gaa tcg cgg    1200
Gln Lys Phe Leu Asn Arg His Ala Asp Ala Glu Pro Ile Glu Ser Arg
385                 390                 395                 400

gtg ctt tta ccg aac aaa cac caa gat ggc ttt tat tac gcg ctt att    1248
Val Leu Leu Pro Asn Lys His Gln Asp Gly Phe Tyr Tyr Ala Leu Ile
                405                 410                 415

caa aag cat                                                        1257
Gln Lys His

<210> 168
<211> 419
<212> PRT
<213> Neisseria meningitidis

<400> 168
Met Ser Met Ala Leu Ala Gln Lys Leu Ala Ala Asp Ser Ile Ala Ala
1               5                   10                  15

Val Ala Glu Gly Arg Asn Leu Gln Asp Val Leu Ala Gln Ile Arg Thr
            20                  25                  30

Ala His Pro Asp Leu Thr Ala Gln Glu Asn Gly Ala Leu Gln Asp Ile
        35                  40                  45

Ala Tyr Gly Cys Gln Arg Tyr Leu Gly Ser Leu Lys His Met Leu Ala
    50                  55                  60

Gln Met Leu Lys Lys Pro Ile Gly Asn Pro Gln Leu Glu Ser Leu Leu
65                  70                  75                  80

Leu Ala Ala Leu Tyr Gln Leu His Tyr Thr Arg Asn Ala Pro His Ala
                85                  90                  95

Val Val Asn Glu Ala Val Glu Ser Ile Ala Lys Ile Gly Arg Gly Gln
            100                 105                 110

Tyr Arg Ser Phe Ala Asn Ala Ile Leu Arg Arg Phe Leu Arg Glu Arg
        115                 120                 125

Asp Lys Leu Ala Ala Ser Cys Lys Lys Asp Asp Val Ala Lys His Asn
    130                 135                 140

Leu Pro Leu Trp Trp Val Ala Tyr Leu Lys Asn His Tyr Pro Lys His
145                 150                 155                 160

Trp His Asn Ile Ala Ala Ala Leu Gln Ser His Pro Pro Met Thr Leu
                165                 170                 175

Arg Val Asn Arg Arg His Gly Asn Ala Glu Ser Tyr Leu Glu Lys Leu
            180                 185                 190

Ala Ala Glu Gly Ile Ala Ala Lys Ala Leu Asp Glu Tyr Ala Val Thr
        195                 200                 205

Leu Glu Glu Ala Val Pro Val Asn Arg Leu Pro Gly Phe Ser Asp Gly
    210                 215                 220

Ile Val Ser Val Gln Asp Phe Gly Ala Gln Gln Ala Ala Tyr Leu Leu
225                 230                 235                 240

Asn Pro Lys Asp Gly Glu Arg Ile Leu Asp Ala Cys Ala Ala Pro Gly
                245                 250                 255

Gly Lys Thr Gly His Ile Leu Glu Leu Ala Asp Cys Arg Val Thr Ala
            260                 265                 270

Leu Asp Ile Asp Ala Gly Arg Leu Lys Arg Val Glu Asp Asn Ile Ala
        275                 280                 285

Arg Leu Gly Phe Gln Thr Ala Ser Ala Ala Cys Ala Asp Ala Arg Asp
    290                 295                 300

Leu Ala Ala Trp Tyr Asp Gly Lys Pro Phe Asp Thr Ile Leu Ala Asp
305                 310                 315                 320

Val Pro Cys Thr Ala Ser Gly Val Ala Arg Arg Asn Pro Asp Val Lys
                325                 330                 335

Trp Leu Arg Arg Pro Thr Asp Ala Leu Lys Thr Ala Arg Gln Gln Glu
            340                 345                 350

Ala Leu Leu Asp Ala Leu Trp Gln Thr Leu Lys Gln Gly Gly Arg Met
        355                 360                 365

Leu Leu Ala Thr Cys Ser Val Phe Val Glu Glu Asn Asp Gly Gln Leu
    370                 375                 380

Gln Lys Phe Leu Asn Arg His Ala Asp Ala Glu Pro Ile Glu Ser Arg
385                 390                 395                 400

Val Leu Leu Pro Asn Lys His Gln Asp Gly Phe Tyr Tyr Ala Leu Ile
                405                 410                 415

Gln Lys His

<210> 169
<211> 804
<212> DNA
<213> Neisseria meningitidis

<220>
<221> CDS
<222> (1)..(804)

<400> 169
atg aat atg aaa aaa tgg att gcc gcc gcc ctt gcc tgt tcc gcg ctc      48
Met Asn Met Lys Lys Trp Ile Ala Ala Ala Leu Ala Cys Ser Ala Leu
  1               5                  10                  15

gcg ctg tct gcc tgc ggc ggt cag ggt aaa gat gcc gcc gcg ccc gcc      96
Ala Leu Ser Ala Cys Gly Gly Gln Gly Lys Asp Ala Ala Ala Pro Ala
             20                  25                  30

gca aat ccc gac aaa gtg tac cgc gtg gct tcc aac gcc gag ttt gcc     144
Ala Asn Pro Asp Lys Val Tyr Arg Val Ala Ser Asn Ala Glu Phe Ala
         35                  40                  45

ccc ttt gaa tct tta gac tcg aaa ggc aat gtc gaa ggt ttc gat gtg     192
Pro Phe Glu Ser Leu Asp Ser Lys Gly Asn Val Glu Gly Phe Asp Val
     50                  55                  60

gat ttg atg aac gcg atg gcg aag gcg ggc aat ttt aaa atc gaa ttc     240
Asp Leu Met Asn Ala Met Ala Lys Ala Gly Asn Phe Lys Ile Glu Phe
 65                  70                  75                  80

aaa cac cag ccg tgg gac agc ctt ttc ccc gcc ttg aac aac ggc gat     288
Lys His Gln Pro Trp Asp Ser Leu Phe Pro Ala Leu Asn Asn Gly Asp
                 85                  90                  95

gcg gac gtt gtg atg tcg ggc gta acc att acc gac gac cgc aaa cag     336
Ala Asp Val Val Met Ser Gly Val Thr Ile Thr Asp Asp Arg Lys Gln
            100                 105                 110

tct atg gac ttc agc gac ccg tat ttt gaa atc acc caa gtc gtc ctc     384
Ser Met Asp Phe Ser Asp Pro Tyr Phe Glu Ile Thr Gln Val Val Leu

        115                 120                 125

gtt ccg aaa ggc aaa aaa ata tct tct tcc gaa gat ttg aaa aac atg 432
Val Pro Lys Gly Lys Lys Ile Ser Ser Ser Glu Asp Leu Lys Asn Met
    130                 135                 140

aac aaa gtc ggc gtg gta acc ggc tac acg ggc gat ttc tcc gta tcc 480
Asn Lys Val Gly Val Val Thr Gly Tyr Thr Gly Asp Phe Ser Val Ser
145                 150                 155                 160

aaa ctc ttg ggc aac gac aac ccg aaa atc gcg cgc ttt gaa aac gtt 528
Lys Leu Leu Gly Asn Asp Asn Pro Lys Ile Ala Arg Phe Glu Asn Val
                165                 170                 175

ccc ctg att atc aaa gaa ctg gaa aac ggc ggc ttg gat tcc gtg gtc 576
Pro Leu Ile Ile Lys Glu Leu Glu Asn Gly Gly Leu Asp Ser Val Val
            180                 185                 190

agc gac agc gca gtc atc gcc aat tat gtg aaa aac aat ccg acc aaa 624
Ser Asp Ser Ala Val Ile Ala Asn Tyr Val Lys Asn Asn Pro Thr Lys
        195                 200                 205

ggg atg gac ttc gtt acc ctg ccc gac ttc acc acc gaa cac tac ggc 672
Gly Met Asp Phe Val Thr Leu Pro Asp Phe Thr Thr Glu His Tyr Gly
    210                 215                 220

atc gcg gta cgc aaa ggc gac gaa gca acc gtc aaa atg ctg aac gat 720
Ile Ala Val Arg Lys Gly Asp Glu Ala Thr Val Lys Met Leu Asn Asp
225                 230                 235                 240

gcg ttg aaa aaa gta cgc gaa agc ggc gaa tac gac aaa atc tac gcc 768
Ala Leu Lys Lys Val Arg Glu Ser Gly Glu Tyr Asp Lys Ile Tyr Ala
                245                 250                 255

aaa tat ttt gca aaa gaa gac gga cag gcc gca aaa 804
Lys Tyr Phe Ala Lys Glu Asp Gly Gln Ala Ala Lys
            260                 265

<210> 170
<211> 268
<212> PRT
<213> Neisseria meningitidis

<400> 170
Met Asn Met Lys Lys Trp Ile Ala Ala Ala Leu Ala Cys Ser Ala Leu
1               5                   10                  15

Ala Leu Ser Ala Cys Gly Gly Gln Gly Lys Asp Ala Ala Ala Pro Ala
            20                  25                  30

Ala Asn Pro Asp Lys Val Tyr Arg Val Ala Ser Asn Ala Glu Phe Ala
        35                  40                  45

Pro Phe Glu Ser Leu Asp Ser Lys Gly Asn Val Glu Gly Phe Asp Val
    50                  55                  60

Asp Leu Met Asn Ala Met Ala Lys Ala Gly Asn Phe Lys Ile Glu Phe
65                  70                  75                  80

Lys His Gln Pro Trp Asp Ser Leu Phe Pro Ala Leu Asn Asn Gly Asp
                85                  90                  95

Ala Asp Val Val Met Ser Gly Val Thr Ile Thr Asp Asp Arg Lys Gln
            100                 105                 110

Ser Met Asp Phe Ser Asp Pro Tyr Phe Glu Ile Thr Gln Val Val Leu
        115                 120                 125

Val Pro Lys Gly Lys Lys Ile Ser Ser Ser Glu Asp Leu Lys Asn Met
    130                 135                 140

Asn Lys Val Gly Val Val Thr Gly Tyr Thr Gly Asp Phe Ser Val Ser
145                 150                 155                 160

Lys Leu Leu Gly Asn Asp Asn Pro Lys Ile Ala Arg Phe Glu Asn Val
                165                 170                 175

Pro Leu Ile Ile Lys Glu Leu Glu Asn Gly Gly Leu Asp Ser Val Val
            180                 185                 190

Ser Asp Ser Ala Val Ile Ala Asn Tyr Val Lys Asn Asn Pro Thr Lys
        195                 200                 205

Gly Met Asp Phe Val Thr Leu Pro Asp Phe Thr Thr Glu His Tyr Gly
    210                 215                 220

Ile Ala Val Arg Lys Gly Asp Glu Ala Thr Val Lys Met Leu Asn Asp
225                 230                 235                 240

Ala Leu Lys Lys Val Arg Glu Ser Gly Glu Tyr Asp Lys Ile Tyr Ala
                245                 250                 255

Lys Tyr Phe Ala Lys Glu Asp Gly Gln Ala Ala Lys
            260                 265

<210> 171
<211> 864
<212> DNA
<213> Neisseria meningitidis

<220>
<221> CDS
<222> (1)..(864)

<400> 171
atg aaa gca aaa atc ctg act tcc gtt gca ctg ctt gcc tgt tcc ggc     48
Met Lys Ala Lys Ile Leu Thr Ser Val Ala Leu Leu Ala Cys Ser Gly
1               5                   10                  15

agc ctg ttt gcc caa acg ctg gca acc gtc aac ggt cag aaa atc gac     96
Ser Leu Phe Ala Gln Thr Leu Ala Thr Val Asn Gly Gln Lys Ile Asp
            20                  25                  30

agt tcc gtc att gat gcg cag gtt gcc gca ttc cgt gcg gaa aac agc     144
Ser Ser Val Ile Asp Ala Gln Val Ala Ala Phe Arg Ala Glu Asn Ser
        35                  40                  45

cgt gcc gaa gac acg ccg caa ctg cgc caa tcc ctg ctg gaa aac gaa     192
Arg Ala Glu Asp Thr Pro Gln Leu Arg Gln Ser Leu Leu Glu Asn Glu
    50                  55                  60

gtg gtc aac acc gtg gtc gca cag gaa gtg aaa cgc ctg aaa ctc gac     240
Val Val Asn Thr Val Val Ala Gln Glu Val Lys Arg Leu Lys Leu Asp
65                  70                  75                  80

cgg tcg gca gag ttt aaa aat gcg ctt gcc aaa ttg cgt gcc gaa gcg     288
Arg Ser Ala Glu Phe Lys Asn Ala Leu Ala Lys Leu Arg Ala Glu Ala
                85                  90                  95

aaa aag tcg ggc gac gac aag aaa ccg tcc ttc aaa acc gtt tgg cag     336
Lys Lys Ser Gly Asp Asp Lys Lys Pro Ser Phe Lys Thr Val Trp Gln
            100                 105                 110

gcg gta aaa tat ggc ttg aac ggc gag gca tac gcg ctg cat atc gcc     384
Ala Val Lys Tyr Gly Leu Asn Gly Glu Ala Tyr Ala Leu His Ile Ala
        115                 120                 125

aaa acc caa ccg gtt tcc gag cag gaa gta aaa gcc gca tat gac aat     432
Lys Thr Gln Pro Val Ser Glu Gln Glu Val Lys Ala Ala Tyr Asp Asn
    130                 135                 140

atc agc ggt ttt tac aaa ggt acg cag gaa gtc cag ttg ggc gaa atc     480
Ile Ser Gly Phe Tyr Lys Gly Thr Gln Glu Val Gln Leu Gly Glu Ile
145                 150                 155                 160

ctg acc gac aag gaa gaa aat gca aaa aaa gcg gtt gcc gac ttg aag     528
Leu Thr Asp Lys Glu Glu Asn Ala Lys Lys Ala Val Ala Asp Leu Lys
                165                 170                 175

gcg aaa aaa ggt ttc gat gcc gtc ttg aaa caa tat tcc ctc aac gac     576
Ala Lys Lys Gly Phe Asp Ala Val Leu Lys Gln Tyr Ser Leu Asn Asp
            180                 185                 190

cgt acc aaa cag acc ggt gcg ccg gtc gga tat gtg ccg ctg aaa gat     624
Arg Thr Lys Gln Thr Gly Ala Pro Val Gly Tyr Val Pro Leu Lys Asp
        195                 200                 205

ttg gaa cag ggt gtt ccg ccg ctt tat cag gca att aag gac ttg aaa     672
Leu Glu Gln Gly Val Pro Pro Leu Tyr Gln Ala Ile Lys Asp Leu Lys
    210                 215                 220

aaa ggc gaa ttt acg gca acg ccg ctg aaa aac ggc gat ttc tac ggc     720
Lys Gly Glu Phe Thr Ala Thr Pro Leu Lys Asn Gly Asp Phe Tyr Gly
225                 230                 235                 240

gtt tat tat gtc aac gac agc cgc gag gta aaa gtg cct tct ttt gat     768
Val Tyr Tyr Val Asn Asp Ser Arg Glu Val Lys Val Pro Ser Phe Asp
                245                 250                 255

gaa atg aaa gga cag att gcg ggc aac ctt cag gcg gaa cgg att gac     816
Glu Met Lys Gly Gln Ile Ala Gly Asn Leu Gln Ala Glu Arg Ile Asp
            260                 265                 270

cgt gcc gtc ggt gca ctg ttg ggc aag gca aac atc aaa cct gca aaa     864
Arg Ala Val Gly Ala Leu Leu Gly Lys Ala Asn Ile Lys Pro Ala Lys
        275                 280                 285

<210> 172
<211> 288
<212> PRT
<213> Neisseria meningitidis

<400> 172
Met Lys Ala Lys Ile Leu Thr Ser Val Ala Leu Leu Ala Cys Ser Gly
  1               5                  10                  15

Ser Leu Phe Ala Gln Thr Leu Ala Thr Val Asn Gly Gln Lys Ile Asp
             20                  25                  30

Ser Ser Val Ile Asp Ala Gln Val Ala Ala Phe Arg Ala Glu Asn Ser
        35                  40                  45

Arg Ala Glu Asp Thr Pro Gln Leu Arg Gln Ser Leu Leu Glu Asn Glu
    50                  55                  60

Val Val Asn Thr Val Val Ala Gln Glu Val Lys Arg Leu Lys Leu Asp
65                  70                  75                  80

Arg Ser Ala Glu Phe Lys Asn Ala Leu Ala Lys Leu Arg Ala Glu Ala
                85                  90                  95

Lys Lys Ser Gly Asp Asp Lys Lys Pro Ser Phe Lys Thr Val Trp Gln
            100                 105                 110

Ala Val Lys Tyr Gly Leu Asn Gly Glu Ala Tyr Ala Leu His Ile Ala
        115                 120                 125

Lys Thr Gln Pro Val Ser Glu Gln Glu Val Lys Ala Ala Tyr Asp Asn
    130                 135                 140

Ile Ser Gly Phe Tyr Lys Gly Thr Gln Glu Val Gln Leu Gly Glu Ile
145                 150                 155                 160

Leu Thr Asp Lys Glu Glu Asn Ala Lys Lys Ala Val Ala Asp Leu Lys
                165                 170                 175

Ala Lys Lys Gly Phe Asp Ala Val Leu Lys Gln Tyr Ser Leu Asn Asp
            180                 185                 190

Arg Thr Lys Gln Thr Gly Ala Pro Val Gly Tyr Val Pro Leu Lys Asp
        195                 200                 205

Leu Glu Gln Gly Val Pro Pro Leu Tyr Gln Ala Ile Lys Asp Leu Lys
    210                 215                 220

Lys Gly Glu Phe Thr Ala Thr Pro Leu Lys Asn Gly Asp Phe Tyr Gly
225                 230                 235                 240

Val Tyr Tyr Val Asn Asp Ser Arg Glu Val Lys Val Pro Ser Phe Asp
                245                 250                 255

Glu Met Lys Gly Gln Ile Ala Gly Asn Leu Gln Ala Glu Arg Ile Asp
            260                 265                 270

Arg Ala Val Gly Ala Leu Leu Gly Lys Ala Asn Ile Lys Pro Ala Lys
        275                 280                 285

<210> 173
<211> 1794
<212> DNA
<213> Neisseria meningitidis

<220>
<221> CDS
<222> (1)..(1794)

<400> 173
ttg ccc cga att gcc atg ccc tat ttc gcc ctg ttt gac gat gcc gta     48
Leu Pro Arg Ile Ala Met Pro Tyr Phe Ala Leu Phe Asp Asp Ala Val
1               5                   10                  15

agc ggt cgc gca aaa cgc tat caa aat cat gtg gaa agc cgt ttt ttc     96
Ser Gly Arg Ala Lys Arg Tyr Gln Asn His Val Glu Ser Arg Phe Phe
            20                  25                  30

cgt ccc gaa gaa ctc gat gct ttg gac ggc gcg ctg caa tcg ggc tgg     144
Arg Pro Glu Glu Leu Asp Ala Leu Asp Gly Ala Leu Gln Ser Gly Trp
        35                  40                  45

caa aaa ggg ctg cat gcc gtg ttg ttt gca gac tac gga ttc ggt ttg     192
Gln Lys Gly Leu His Ala Val Leu Phe Ala Asp Tyr Gly Phe Gly Leu
    50                  55                  60

ccg ctg acg ggg gtc gag tcc gaa cgc ggc ggc aac ctt gcc ctg cac     240
Pro Leu Thr Gly Val Glu Ser Glu Arg Gly Gly Asn Leu Ala Leu His
65                  70                  75                  80

tgg ttt gcc gac tgc gcc gac acc gat gcc gca agc tgg ctt gcc cga     288
Trp Phe Ala Asp Cys Ala Asp Thr Asp Ala Ala Ser Trp Leu Ala Arg
                85                  90                  95

cac tca gac ggc ctc ccc gcc ggc att tcc acg ccg caa tcc tcc gta     336
His Ser Asp Gly Leu Pro Ala Gly Ile Ser Thr Pro Gln Ser Ser Val
            100                 105                 110

tcc gaa gcc gat tac ctc gac cat atc cgc caa atc cac gaa gcc atc     384
Ser Glu Ala Asp Tyr Leu Asp His Ile Arg Gln Ile His Glu Ala Ile
        115                 120                 125

cga cgc ggc gac acc tat caa atc aac tac act acc cgc ctg cac ctg     432
Arg Arg Gly Asp Thr Tyr Gln Ile Asn Tyr Thr Thr Arg Leu His Leu
    130                 135                 140

caa gcc tac ggc aat ccc gtc aaa ctt tac caa cgc ctg cgc cag ccc 480
Gln Ala Tyr Gly Asn Pro Val Lys Leu Tyr Gln Arg Leu Arg Gln Pro
145                 150                 155                 160

gtc ccc tat gcc gtt ttg tcc cac ctg ccc gat gca caa ggg caa tcc 528
Val Pro Tyr Ala Val Leu Ser His Leu Pro Asp Ala Gln Gly Gln Ser
                165                 170                 175

gcg tgg acg ctg tgt ttc tcg ccc gaa ctc ttc ctc aaa atc ggt tcg 576
Ala Trp Thr Leu Cys Phe Ser Pro Glu Leu Phe Leu Lys Ile Gly Ser
            180                 185                 190

gac ggc acc atc agc acc gaa ccg atg aaa ggc acc gcg ccg att ttg 624
Asp Gly Thr Ile Ser Thr Glu Pro Met Lys Gly Thr Ala Pro Ile Leu
        195                 200                 205

ggc gac gga caa gac gaa cgc cgc gcc gcc gag ttg caa gca gac ccg 672
Gly Asp Gly Gln Asp Glu Arg Arg Ala Ala Glu Leu Gln Ala Asp Pro
    210                 215                 220

aaa aac cgc gcc gaa aac gtg atg att gtc gat ttg ctg cgt aac gac 720
Lys Asn Arg Ala Glu Asn Val Met Ile Val Asp Leu Leu Arg Asn Asp
225                 230                 235                 240

ctc ggc aaa atc gcc caa acc ggc aca gta tgc gta ccc gaa ccg ttt 768
Leu Gly Lys Ile Ala Gln Thr Gly Thr Val Cys Val Pro Glu Pro Phe
                245                 250                 255

aaa gta tcg cgt ttc ggc agc gtt tgg cag atg acc agc acc atc caa 816
Lys Val Ser Arg Phe Gly Ser Val Trp Gln Met Thr Ser Thr Ile Gln
            260                 265                 270

gcc caa gcc ttg ccg cac acc tcg ttc gcc gac atc ctc cgc gcc gcc 864
Ala Gln Ala Leu Pro His Thr Ser Phe Ala Asp Ile Leu Arg Ala Ala
        275                 280                 285

ttc ccc tgc ggc agc atc acc ggc gcg ccc aaa aaa atg agt atg cag 912
Phe Pro Cys Gly Ser Ile Thr Gly Ala Pro Lys Lys Met Ser Met Gln
    290                 295                 300

att atc gaa tcg ctc gaa gcc gaa gcg cgc gga ctt tat acg ggc agc 960
Ile Ile Glu Ser Leu Glu Ala Glu Ala Arg Gly Leu Tyr Thr Gly Ser
305                 310                 315                 320

atc ggc tat ttg aac ccg tgt tcc ggc ggc ttg ggg ttt gaa ggc acg 1008
Ile Gly Tyr Leu Asn Pro Cys Ser Gly Gly Leu Gly Phe Glu Gly Thr
                325                 330                 335

ttc aac gtc gtt atc cgc acc ttg tcg ctc acg ccg ctt tca gac ggc 1056
Phe Asn Val Val Ile Arg Thr Leu Ser Leu Thr Pro Leu Ser Asp Gly
            340                 345                 350

att tat caa ggc gtg tac ggt gtc ggt tcc ggc atc gtc atc gac agc 1104
Ile Tyr Gln Gly Val Tyr Gly Val Gly Ser Gly Ile Val Ile Asp Ser
        355                 360                 365

gac cct gcc gcc gaa tat cgg gaa tgc ggc tgg aaa gcc cgt ttc ctc 1152
Asp Pro Ala Ala Glu Tyr Arg Glu Cys Gly Trp Lys Ala Arg Phe Leu
    370                 375                 380

aac gaa ttg cgc ccc gac ttc ggc att ttt gaa acc ctg cgc gtg gaa 1200
Asn Glu Leu Arg Pro Asp Phe Gly Ile Phe Glu Thr Leu Arg Val Glu
385                 390                 395                 400

aac gga cgc tgc gcc ctg ctc gac cgc cac cta tgc cgt ctg aaa acc 1248
Asn Gly Arg Cys Ala Leu Leu Asp Arg His Leu Cys Arg Leu Lys Thr
                405                 410                 415

tcc gcc caa gcc ctc aac ctg ccc ctg ccc gac ggt tgc gaa aat caa 1296
Ser Ala Gln Ala Leu Asn Leu Pro Leu Pro Asp Gly Cys Glu Asn Gln
            420                 425                 430

atc aaa caa tac atc gca cgc ttg ccc gac ggc gcg ttc cgc atc aaa 1344
Ile Lys Gln Tyr Ile Ala Arg Leu Pro Asp Gly Ala Phe Arg Ile Lys
        435                 440                 445

gcc ctg ctc gct tca gac ggc atc agc ctg tcc cgc gcc gtt tta aac 1392
Ala Leu Leu Ala Ser Asp Gly Ile Ser Leu Ser Arg Ala Val Leu Asn
    450                 455                 460

cgt ctg acc gac aaa caa cgc gtc atc att tcg ccg acc atc ctg ccc 1440
Arg Leu Thr Asp Lys Gln Arg Val Ile Ile Ser Pro Thr Ile Leu Pro
465                 470                 475                 480

gca caa aac tac ctg cgc cgc ttc aaa acc acc tgc cgc acc gtc ttc 1488
Ala Gln Asn Tyr Leu Arg Arg Phe Lys Thr Thr Cys Arg Thr Val Phe
                485                 490                 495

gac caa gcg tgg caa acc gcc gaa aca caa ggc gcg ttc gac agc ctg 1536
Asp Gln Ala Trp Gln Thr Ala Glu Thr Gln Gly Ala Phe Asp Ser Leu
            500                 505                 510

ttt ttc aat tca gac ggc atc ctg ctc gaa ggc ggc aga agc aac gtg 1584
Phe Phe Asn Ser Asp Gly Ile Leu Leu Glu Gly Gly Arg Ser Asn Val
        515                 520                 525

ttc gtc aaa cat cgc gga caa tgg ctc acg ccc tct tta gat tta gac    1632
Phe Val Lys His Arg Gly Gln Trp Leu Thr Pro Ser Leu Asp Leu Asp
    530                 535                 540

att tta aac ggc ata atg cgc caa gcc gtg ttg gac gaa ccg caa aaa    1680
Ile Leu Asn Gly Ile Met Arg Gln Ala Val Leu Asp Glu Pro Gln Lys
545                 550                 555                 560

tat ttg caa aca aat caa gta atc gaa aca cac atc aca caa aaa aca    1728
Tyr Leu Gln Thr Asn Gln Val Ile Glu Thr His Ile Thr Gln Lys Thr
                565                 570                 575

ctg caa gaa gcc gaa gaa atc cgc ctc tcc aac gcc ttg cgc ggc gta    1776
Leu Gln Glu Ala Glu Glu Ile Arg Leu Ser Asn Ala Leu Arg Gly Val
            580                 585                 590

ttt gcc gcc gcc ctt gcc                                            1794
Phe Ala Ala Ala Leu Ala
        595

<210> 174
<211> 598
<212> PRT
<213> Neisseria meningitidis

<400> 174
Leu Pro Arg Ile Ala Met Pro Tyr Phe Ala Leu Phe Asp Asp Ala Val
 1               5                  10                  15

Ser Gly Arg Ala Lys Arg Tyr Gln Asn His Val Glu Ser Arg Phe Phe
            20                  25                  30

Arg Pro Glu Glu Leu Asp Ala Leu Asp Gly Ala Leu Gln Ser Gly Trp
        35                  40                  45

Gln Lys Gly Leu His Ala Val Leu Phe Ala Asp Tyr Gly Phe Gly Leu
    50                  55                  60

Pro Leu Thr Gly Val Glu Ser Glu Arg Gly Gly Asn Leu Ala Leu His
65                  70                  75                  80

Trp Phe Ala Asp Cys Ala Asp Thr Asp Ala Ala Ser Trp Leu Ala Arg
                85                  90                  95

His Ser Asp Gly Leu Pro Ala Gly Ile Ser Thr Pro Gln Ser Ser Val
            100                 105                 110

Ser Glu Ala Asp Tyr Leu Asp His Ile Arg Gln Ile His Glu Ala Ile
        115                 120                 125

Arg Arg Gly Asp Thr Tyr Gln Ile Asn Tyr Thr Thr Arg Leu His Leu
    130                 135                 140

Gln Ala Tyr Gly Asn Pro Val Lys Leu Tyr Gln Arg Leu Arg Gln Pro
145                 150                 155                 160

Val Pro Tyr Ala Val Leu Ser His Leu Pro Asp Ala Gln Gly Gln Ser
                165                 170                 175

Ala Trp Thr Leu Cys Phe Ser Pro Glu Leu Phe Leu Lys Ile Gly Ser
            180                 185                 190

Asp Gly Thr Ile Ser Thr Glu Pro Met Lys Gly Thr Ala Pro Ile Leu
        195                 200                 205

Gly Asp Gly Gln Asp Glu Arg Arg Ala Ala Glu Leu Gln Ala Asp Pro
    210                 215                 220

Lys Asn Arg Ala Glu Asn Val Met Ile Val Asp Leu Leu Arg Asn Asp
225                 230                 235                 240

Leu Gly Lys Ile Ala Gln Thr Gly Thr Val Cys Val Pro Glu Pro Phe
                245                 250                 255

Lys Val Ser Arg Phe Gly Ser Val Trp Gln Met Thr Ser Thr Ile Gln
            260                 265                 270

Ala Gln Ala Leu Pro His Thr Ser Phe Ala Asp Ile Leu Arg Ala Ala
        275                 280                 285

Phe Pro Cys Gly Ser Ile Thr Gly Ala Pro Lys Lys Met Ser Met Gln
    290                 295                 300

Ile Ile Glu Ser Leu Glu Ala Glu Ala Arg Gly Leu Tyr Thr Gly Ser
305                 310                 315                 320

Ile Gly Tyr Leu Asn Pro Cys Ser Gly Gly Leu Gly Phe Glu Gly Thr
                325                 330                 335

Phe Asn Val Val Ile Arg Thr Leu Ser Leu Thr Pro Leu Ser Asp Gly
            340                 345                 350

Ile Tyr Gln Gly Val Tyr Gly Val Gly Ser Gly Ile Val Ile Asp Ser
        355                 360                 365

Asp Pro Ala Ala Glu Tyr Arg Glu Cys Gly Trp Lys Ala Arg Phe Leu
    370                 375                 380

Asn Glu Leu Arg Pro Asp Phe Gly Ile Phe Glu Thr Leu Arg Val Glu
385                 390                 395                 400

Asn Gly Arg Cys Ala Leu Leu Asp Arg His Leu Cys Arg Leu Lys Thr
                405                 410                 415

Ser Ala Gln Ala Leu Asn Leu Pro Leu Pro Asp Gly Cys Glu Asn Gln
            420                 425                 430

Ile Lys Gln Tyr Ile Ala Arg Leu Pro Asp Gly Ala Phe Arg Ile Lys
        435                 440                 445

Ala Leu Leu Ala Ser Asp Gly Ile Ser Leu Ser Arg Ala Val Leu Asn
    450                 455                 460

Arg Leu Thr Asp Lys Gln Arg Val Ile Ile Ser Pro Thr Ile Leu Pro
465                 470                 475                 480

Ala Gln Asn Tyr Leu Arg Arg Phe Lys Thr Thr Cys Arg Thr Val Phe
                485                 490                 495

Asp Gln Ala Trp Gln Thr Ala Glu Thr Gln Gly Ala Phe Asp Ser Leu
            500                 505                 510

Phe Phe Asn Ser Asp Gly Ile Leu Leu Glu Gly Gly Arg Ser Asn Val
        515                 520                 525

Phe Val Lys His Arg Gly Gln Trp Leu Thr Pro Ser Leu Asp Leu Asp
    530                 535                 540

Ile Leu Asn Gly Ile Met Arg Gln Ala Val Leu Asp Glu Pro Gln Lys
545                 550                 555                 560

Tyr Leu Gln Thr Asn Gln Val Ile Glu Thr His Ile Thr Gln Lys Thr
                565                 570                 575

Leu Gln Glu Ala Glu Glu Ile Arg Leu Ser Asn Ala Leu Arg Gly Val
            580                 585                 590

Phe Ala Ala Ala Leu Ala
        595

<210> 175
<211> 279
<212> DNA
<213> Neisseria meningitidis

<220>
<221> CDS
<222> (1)..(279)

<400> 175
ttg caa aac ttt cgg aaa ccc aat atg cag acc gtt acc atg tac aca    48
Leu Gln Asn Phe Arg Lys Pro Asn Met Gln Thr Val Thr Met Tyr Thr
1               5                   10                  15

ggt ccg ttt tgc ccc tac tgc acg atg gcg aaa agg ctg ctg cac gcg    96
Gly Pro Phe Cys Pro Tyr Cys Thr Met Ala Lys Arg Leu Leu His Ala
            20                  25                  30

gca ggt gtc gga cat atc gac gaa atc cgt gtc gat gca agt ccc gaa    144
Ala Gly Val Gly His Ile Asp Glu Ile Arg Val Asp Ala Ser Pro Glu
        35                  40                  45

gcc ttt gcc gaa atg cag cag ctt tcc gga cag cgc agc gtg ccg cag    192
Ala Phe Ala Glu Met Gln Gln Leu Ser Gly Gln Arg Ser Val Pro Gln
    50                  55                  60

att ttc atc ggc gaa acg cac gtc ggc gga ttt acc gac ctc tac cgc    240
Ile Phe Ile Gly Glu Thr His Val Gly Gly Phe Thr Asp Leu Tyr Arg
65                  70                  75                  80

ctc cag cag gaa ggc ggg ctg gac gga ctg ctg aac cct                279
Leu Gln Gln Glu Gly Gly Leu Asp Gly Leu Leu Asn Pro
                85                  90

<210> 176
<211> 93
<212> PRT
<213> Neisseria meningitidis

<400> 176
Leu Gln Asn Phe Arg Lys Pro Asn Met Gln Thr Val Thr Met Tyr Thr
1               5                   10                  15

Gly Pro Phe Cys Pro Tyr Cys Thr Met Ala Lys Arg Leu Leu His Ala
            20                  25                  30

Ala Gly Val Gly His Ile Asp Glu Ile Arg Val Asp Ala Ser Pro Glu

35                  40                  45

Ala Phe Ala Glu Met Gln Gln Leu Ser Gly Gln Arg Ser Val Pro Gln
    50                  55                  60

Ile Phe Ile Gly Glu Thr His Val Gly Gly Phe Thr Asp Leu Tyr Arg
65                  70                  75                  80

Leu Gln Gln Glu Gly Gly Leu Asp Gly Leu Leu Asn Pro
                85                  90

<210> 177
<211> 2274
<212> DNA
<213> Neisseria meningitidis

<220>
<221> CDS
<222> (1)..(2274)

<400> 177
atg aca aca tta cat ttc tca ggc ttc ccg cgt gtc ggt gcc ttc cgc    48
Met Thr Thr Leu His Phe Ser Gly Phe Pro Arg Val Gly Ala Phe Arg
1               5                   10                  15

gaa ttg aaa ttc gca caa gaa aaa tac tgg cgc aaa gaa atc agc gag    96
Glu Leu Lys Phe Ala Gln Glu Lys Tyr Trp Arg Lys Glu Ile Ser Glu
            20                  25                  30

caa gaa ttg ctg gct gtt gct aaa gac ttg cgc gag aaa aac tgg aaa    144
Gln Glu Leu Leu Ala Val Ala Lys Asp Leu Arg Glu Lys Asn Trp Lys
        35                  40                  45

cac cag gcc gct gcc aac gcc gat tac gtt gcc gta ggc gat ttc act    192
His Gln Ala Ala Ala Asn Ala Asp Tyr Val Ala Val Gly Asp Phe Thr
    50                  55                  60

ttc tac gac cac atc ctc gac ctg caa gtc gcc acc ggc gcg att ccc    240
Phe Tyr Asp His Ile Leu Asp Leu Gln Val Ala Thr Gly Ala Ile Pro
65                  70                  75                  80

gcc cgc ttc ggc ttc gac agc caa aac cta tct ttg gaa caa ttc ttc    288
Ala Arg Phe Gly Phe Asp Ser Gln Asn Leu Ser Leu Glu Gln Phe Phe
                85                  90                  95

caa ctg gcg cgc ggt aac aaa gac caa ttc gct atc gaa atg acc aaa    336

Gln Leu Ala Arg Gly Asn Lys Asp Gln Phe Ala Ile Glu Met Thr Lys
            100                 105                 110

tgg ttc gac acc aac tac cac tac ttg gtg cct gaa ttc cac gcc gat 384
Trp Phe Asp Thr Asn Tyr His Tyr Leu Val Pro Glu Phe His Ala Asp
        115                 120                 125

acc gaa ttt aaa gcc aat gcc aaa cac tac gtt caa cag ctg caa gaa 432
Thr Glu Phe Lys Ala Asn Ala Lys His Tyr Val Gln Gln Leu Gln Glu
    130                 135                 140

gcc caa gct ttg ggc ctg aaa gcc aaa ccg acc gtt gtg ggt ccg ttg 480
Ala Gln Ala Leu Gly Leu Lys Ala Lys Pro Thr Val Val Gly Pro Leu
145                 150                 155                 160

act ttc ctg tgg gtc ggt aaa gaa aaa ggc gcc gtt gaa ttc gac cgt 528
Thr Phe Leu Trp Val Gly Lys Glu Lys Gly Ala Val Glu Phe Asp Arg
                165                 170                 175

ctg agc ctg ttg cct aaa ctg ttg cct gtt tac gtt gaa atc ctg act 576
Leu Ser Leu Leu Pro Lys Leu Leu Pro Val Tyr Val Glu Ile Leu Thr
            180                 185                 190

gct ttg gtt gaa gcc ggt gcc gag tgg att caa atc gac gag cct gct 624
Ala Leu Val Glu Ala Gly Ala Glu Trp Ile Gln Ile Asp Glu Pro Ala
        195                 200                 205

ttg act gtt gac ctg cct aaa gaa tgg gta gaa gca tac aaa gac gtt 672
Leu Thr Val Asp Leu Pro Lys Glu Trp Val Glu Ala Tyr Lys Asp Val
    210                 215                 220

tac gcc act ttg agc aaa gta agt gcc aaa atc ctg ctg agc act tac 720
Tyr Ala Thr Leu Ser Lys Val Ser Ala Lys Ile Leu Leu Ser Thr Tyr
225                 230                 235                 240

ttc ggt tct gtt gcc gaa cac gcc gca ttg ttg aaa tcc ctg cct gtt 768
Phe Gly Ser Val Ala Glu His Ala Ala Leu Leu Lys Ser Leu Pro Val
                245                 250                 255

gac ggc ctg cac atc gac ttg gta cgc gct cct gaa caa ctg gat gcg 816
Asp Gly Leu His Ile Asp Leu Val Arg Ala Pro Glu Gln Leu Asp Ala
            260                 265                 270

ttc gcc gac tac gac aaa gtt ctg tct gcc ggc gtc att gac ggc cgc 864
Phe Ala Asp Tyr Asp Lys Val Leu Ser Ala Gly Val Ile Asp Gly Arg
        275                 280                 285

aat att tgg cgc gcc aac ctg aac aaa gtt ttg gaa act gtt gag ctt 912

Asn Ile Trp Arg Ala Asn Leu Asn Lys Val Leu Glu Thr Val Glu Leu
    290                 295                 300

ctg caa gcc aaa ctg ggc gac cgt ttg tgg att tcc agc tct tgc tcg 960
Leu Gln Ala Lys Leu Gly Asp Arg Leu Trp Ile Ser Ser Ser Cys Ser
305                 310                 315                 320

ctg ctg cac act ccg ttt gac ttg tca gtt gaa gaa aaa ctg aaa gcc 1008
Leu Leu His Thr Pro Phe Asp Leu Ser Val Glu Glu Lys Leu Lys Ala
                325                 330                 335

aac aaa ccc gac ctg tac tct tgg ttg gca ttc acc ctg caa aaa acc 1056
Asn Lys Pro Asp Leu Tyr Ser Trp Leu Ala Phe Thr Leu Gln Lys Thr
            340                 345                 350

caa gaa ttg cgc gtc ctg aaa gcc gca ttg aac gaa ggc cgt gat tct 1104
Gln Glu Leu Arg Val Leu Lys Ala Ala Leu Asn Glu Gly Arg Asp Ser
        355                 360                 365

gtt gcc gaa gaa ctg gct gcc agc caa gct gct gcc gac tcc cgt gcc 1152
Val Ala Glu Glu Leu Ala Ala Ser Gln Ala Ala Ala Asp Ser Arg Ala
    370                 375                 380

aac agc agc gaa atc cac cgt gca gac gtt gcc aaa cgc ctg gcc gat 1200
Asn Ser Ser Glu Ile His Arg Ala Asp Val Ala Lys Arg Leu Ala Asp
385                 390                 395                 400

ttg cct gcc aac gca gac caa cgc aaa tcc cca ttt gcc gac cgt atc 1248
Leu Pro Ala Asn Ala Asp Gln Arg Lys Ser Pro Phe Ala Asp Arg Ile
                405                 410                 415

aaa gcg caa caa gca tgg ttg aac ctg cct ctg ctg ccg acg acc aac 1296
Lys Ala Gln Gln Ala Trp Leu Asn Leu Pro Leu Leu Pro Thr Thr Asn
            420                 425                 430

atc ggt tct ttc ccg caa act acc gaa atc cgc caa gca cgc gca gcc 1344
Ile Gly Ser Phe Pro Gln Thr Thr Glu Ile Arg Gln Ala Arg Ala Ala
        435                 440                 445

ttc aaa aaa ggc gaa ctg tct gcc gcc gat tac gaa gcc gcg atg aaa 1392
Phe Lys Lys Gly Glu Leu Ser Ala Ala Asp Tyr Glu Ala Ala Met Lys
    450                 455                 460

aaa gaa atc gcc ttg gtg gtt gaa gag caa gaa aaa ctg gac ttg gac 1440
Lys Glu Ile Ala Leu Val Val Glu Glu Gln Glu Lys Leu Asp Leu Asp
465                 470                 475                 480

gta ctg gta cac ggc gaa gcc gag cgt aac gac atg gtt gaa tac ttc 1488

Val Leu Val His Gly Glu Ala Glu Arg Asn Asp Met Val Glu Tyr Phe
                485                 490                 495

ggc gaa ttg ttg agc ggt ttt gca ttc acc caa tat ggc tgg gta caa 1536
Gly Glu Leu Leu Ser Gly Phe Ala Phe Thr Gln Tyr Gly Trp Val Gln
            500                 505                 510

agc tac ggt tct cgc tgc gtt aaa cca ccg att atc ttc ggt gac gta 1584
Ser Tyr Gly Ser Arg Cys Val Lys Pro Pro Ile Ile Phe Gly Asp Val
        515                 520                 525

agc cgt cct gaa gcc atg acc gta gct tgg tct act tac gca caa agc 1632
Ser Arg Pro Glu Ala Met Thr Val Ala Trp Ser Thr Tyr Ala Gln Ser
    530                 535                 540

ctg acc aaa cgc ccg atg aaa ggt atg ttg acc ggc cct gta acc att 1680
Leu Thr Lys Arg Pro Met Lys Gly Met Leu Thr Gly Pro Val Thr Ile
545                 550                 555                 560

ctg caa tgg tct ttc gtc cgc aac gat att cct cgc tct acc gtg tgt 1728
Leu Gln Trp Ser Phe Val Arg Asn Asp Ile Pro Arg Ser Thr Val Cys
                565                 570                 575

aaa caa atc gca ctg gct ctg aac gac gaa gta ttg gat ctg gaa aaa 1776
Lys Gln Ile Ala Leu Ala Leu Asn Asp Glu Val Leu Asp Leu Glu Lys
            580                 585                 590

gca ggc atc aaa gtc atc caa atc gac gaa cct gcc atc cgc gaa ggc 1824
Ala Gly Ile Lys Val Ile Gln Ile Asp Glu Pro Ala Ile Arg Glu Gly
        595                 600                 605

ctg cct ttg aaa cgt gcc gat tgg gat gcc tac ctg aac tgg gcc ggc 1872
Leu Pro Leu Lys Arg Ala Asp Trp Asp Ala Tyr Leu Asn Trp Ala Gly
    610                 615                 620

gaa tct ttc cgc ctg tcc tct acc ggt tgc gaa gac agc act caa atc 1920
Glu Ser Phe Arg Leu Ser Ser Thr Gly Cys Glu Asp Ser Thr Gln Ile
625                 630                 635                 640

cac acc cat atg tgc tac tct gag ttc aac gac atc ctg cct gcg att 1968
His Thr His Met Cys Tyr Ser Glu Phe Asn Asp Ile Leu Pro Ala Ile
                645                 650                 655

gct gca atg gat gcg gac gtg atc acc atc gag act tca cgt tcc gac 2016
Ala Ala Met Asp Ala Asp Val Ile Thr Ile Glu Thr Ser Arg Ser Asp
            660                 665                 670

atg gaa ctc ttg acc gcg ttc ggc gaa ttc aaa tac ccg aac gac atc 2064

Met Glu Leu Leu Thr Ala Phe Gly Glu Phe Lys Tyr Pro Asn Asp Ile
        675                 680                 685

ggc ccg ggg gtt tac gac atc cac agc ccg cgc gta ccg aca gaa gcc     2112
Gly Pro Gly Val Tyr Asp Ile His Ser Pro Arg Val Pro Thr Glu Ala
    690                 695                 700

gaa gtg gag cac ctg ttg cgc aaa gcc atc gag gtt gta ccg gtt gaa     2160
Glu Val Glu His Leu Leu Arg Lys Ala Ile Glu Val Val Pro Val Glu
705                 710                 715                 720

cgt ctg tgg gtt aac ccg gac tgc ggc ctg aaa aca cgc ggc tgg aaa     2208
Arg Leu Trp Val Asn Pro Asp Cys Gly Leu Lys Thr Arg Gly Trp Lys
                725                 730                 735

gaa act ctg gaa caa ctc caa gtg atg atg aac gta acc cac aaa ttg     2256
Glu Thr Leu Glu Gln Leu Gln Val Met Met Asn Val Thr His Lys Leu
            740                 745                 750

cgt gcc gaa ttg gcg aaa                                             2274
Arg Ala Glu Leu Ala Lys
        755

<210> 178
<211> 758
<212> PRT
<213> Neisseria meningitidis

<400> 178
Met Thr Thr Leu His Phe Ser Gly Phe Pro Arg Val Gly Ala Phe Arg
1               5                   10                  15

Glu Leu Lys Phe Ala Gln Glu Lys Tyr Trp Arg Lys Glu Ile Ser Glu
            20                  25                  30

Gln Glu Leu Leu Ala Val Ala Lys Asp Leu Arg Glu Lys Asn Trp Lys
        35                  40                  45

His Gln Ala Ala Ala Asn Ala Asp Tyr Val Ala Val Gly Asp Phe Thr
    50                  55                  60

Phe Tyr Asp His Ile Leu Asp Leu Gln Val Ala Thr Gly Ala Ile Pro
65                  70                  75                  80

Ala Arg Phe Gly Phe Asp Ser Gln Asn Leu Ser Leu Glu Gln Phe Phe
                85                  90                  95

Gln Leu Ala Arg Gly Asn Lys Asp Gln Phe Ala Ile Glu Met Thr Lys
            100                 105                 110

Trp Phe Asp Thr Asn Tyr His Tyr Leu Val Pro Glu Phe His Ala Asp
        115                 120                 125

Thr Glu Phe Lys Ala Asn Ala Lys His Tyr Val Gln Gln Leu Gln Glu
    130                 135                 140

Ala Gln Ala Leu Gly Leu Lys Ala Lys Pro Thr Val Val Gly Pro Leu
145                 150                 155                 160

Thr Phe Leu Trp Val Gly Lys Glu Lys Gly Ala Val Glu Phe Asp Arg
                165                 170                 175

Leu Ser Leu Leu Pro Lys Leu Leu Pro Val Tyr Val Glu Ile Leu Thr
            180                 185                 190

Ala Leu Val Glu Ala Gly Ala Glu Trp Ile Gln Ile Asp Glu Pro Ala
        195                 200                 205

Leu Thr Val Asp Leu Pro Lys Glu Trp Val Glu Ala Tyr Lys Asp Val
    210                 215                 220

Tyr Ala Thr Leu Ser Lys Val Ser Ala Lys Ile Leu Leu Ser Thr Tyr
225                 230                 235                 240

Phe Gly Ser Val Ala Glu His Ala Ala Leu Leu Lys Ser Leu Pro Val
                245                 250                 255

Asp Gly Leu His Ile Asp Leu Val Arg Ala Pro Glu Gln Leu Asp Ala
            260                 265                 270

Phe Ala Asp Tyr Asp Lys Val Leu Ser Ala Gly Val Ile Asp Gly Arg
        275                 280                 285

Asn Ile Trp Arg Ala Asn Leu Asn Lys Val Leu Glu Thr Val Glu Leu
    290                 295                 300

Leu Gln Ala Lys Leu Gly Asp Arg Leu Trp Ile Ser Ser Ser Cys Ser
305                 310                 315                 320

Leu Leu His Thr Pro Phe Asp Leu Ser Val Glu Glu Lys Leu Lys Ala
                325                 330                 335

Asn Lys Pro Asp Leu Tyr Ser Trp Leu Ala Phe Thr Leu Gln Lys Thr
            340                 345                 350

Gln Glu Leu Arg Val Leu Lys Ala Ala Leu Asn Glu Gly Arg Asp Ser
        355                 360                 365

Val Ala Glu Glu Leu Ala Ala Ser Gln Ala Ala Ala Asp Ser Arg Ala
    370                 375                 380

Asn Ser Ser Glu Ile His Arg Ala Asp Val Ala Lys Arg Leu Ala Asp
385                 390                 395                 400

Leu Pro Ala Asn Ala Asp Gln Arg Lys Ser Pro Phe Ala Asp Arg Ile
                405                 410                 415

Lys Ala Gln Gln Ala Trp Leu Asn Leu Pro Leu Leu Pro Thr Thr Asn
            420                 425                 430

Ile Gly Ser Phe Pro Gln Thr Thr Glu Ile Arg Gln Ala Arg Ala Ala
        435                 440                 445

Phe Lys Lys Gly Glu Leu Ser Ala Ala Asp Tyr Glu Ala Ala Met Lys
    450                 455                 460

Lys Glu Ile Ala Leu Val Val Glu Glu Gln Glu Lys Leu Asp Leu Asp
465                 470                 475                 480

Val Leu Val His Gly Glu Ala Glu Arg Asn Asp Met Val Glu Tyr Phe
                485                 490                 495

Gly Glu Leu Leu Ser Gly Phe Ala Phe Thr Gln Tyr Gly Trp Val Gln
            500                 505                 510

Ser Tyr Gly Ser Arg Cys Val Lys Pro Pro Ile Ile Phe Gly Asp Val
        515                 520                 525

Ser Arg Pro Glu Ala Met Thr Val Ala Trp Ser Thr Tyr Ala Gln Ser
    530                 535                 540

Leu Thr Lys Arg Pro Met Lys Gly Met Leu Thr Gly Pro Val Thr Ile
545                 550                 555                 560

Leu Gln Trp Ser Phe Val Arg Asn Asp Ile Pro Arg Ser Thr Val Cys
                565                 570                 575

Lys Gln Ile Ala Leu Ala Leu Asn Asp Glu Val Leu Asp Leu Glu Lys
            580                 585                 590

Ala Gly Ile Lys Val Ile Gln Ile Asp Glu Pro Ala Ile Arg Glu Gly
        595                 600                 605

Leu Pro Leu Lys Arg Ala Asp Trp Asp Ala Tyr Leu Asn Trp Ala Gly
    610                 615                 620

Glu Ser Phe Arg Leu Ser Ser Thr Gly Cys Glu Asp Ser Thr Gln Ile
625                 630                 635                 640

His Thr His Met Cys Tyr Ser Glu Phe Asn Asp Ile Leu Pro Ala Ile
                645                 650                 655

Ala Ala Met Asp Ala Asp Val Ile Thr Ile Glu Thr Ser Arg Ser Asp
            660                 665                 670

Met Glu Leu Leu Thr Ala Phe Gly Glu Phe Lys Tyr Pro Asn Asp Ile
        675                 680                 685

Gly Pro Gly Val Tyr Asp Ile His Ser Pro Arg Val Pro Thr Glu Ala
    690                 695                 700

Glu Val Glu His Leu Leu Arg Lys Ala Ile Glu Val Val Pro Val Glu
705                 710                 715                 720

Arg Leu Trp Val Asn Pro Asp Cys Gly Leu Lys Thr Arg Gly Trp Lys
                725                 730                 735

Glu Thr Leu Glu Gln Leu Gln Val Met Met Asn Val Thr His Lys Leu
            740                 745                 750

Arg Ala Glu Leu Ala Lys
        755

<210> 179
<211> 867
<212> DNA
<213> Neisseria meningitidis

<220>
<221> CDS
<222> (1)..(867)

<400> 179
atg aaa ccc ata cgg aaa gcc gtc ttc ccc gtc gca ggg atg gga aca 48
Met Lys Pro Ile Arg Lys Ala Val Phe Pro Val Ala Gly Met Gly Thr
1               5                   10                  15

cgc ttc ctg ccc gcc acc aag gca agc ccg aaa gaa atg ctg ccc atc 96
Arg Phe Leu Pro Ala Thr Lys Ala Ser Pro Lys Glu Met Leu Pro Ile

            20                  25                  30

gtc gac aag ccg ctg atc caa tac gcc gta gaa gaa gcc gtg gaa gcc 144
Val Asp Lys Pro Leu Ile Gln Tyr Ala Val Glu Glu Ala Val Glu Ala
        35                  40                  45

ggc tgc acg gaa atg gtg ttt gtt acc gga cgc aac aaa cgc agc atc 192
Gly Cys Thr Glu Met Val Phe Val Thr Gly Arg Asn Lys Arg Ser Ile
    50                  55                  60

gaa gac cat ttc gac aag gca tac gaa ctc gaa acc gag ttg gaa atg 240
Glu Asp His Phe Asp Lys Ala Tyr Glu Leu Glu Thr Glu Leu Glu Met
65                  70                  75                  80

cgc cat aaa gac aaa ttg ttg gaa cac gtc cgc aac atc ctg ccg ccg 288
Arg His Lys Asp Lys Leu Leu Glu His Val Arg Asn Ile Leu Pro Pro
                85                  90                  95

aac att acc tgc ctc tac atc cgt cag gcg gaa gca ctg ggc ttg gga 336
Asn Ile Thr Cys Leu Tyr Ile Arg Gln Ala Glu Ala Leu Gly Leu Gly
            100                 105                 110

cac gcc gtc ttg tgc gcc cgc gcc gcc atc ggc gac gaa ccc ttt gcc 384
His Ala Val Leu Cys Ala Arg Ala Ala Ile Gly Asp Glu Pro Phe Ala
        115                 120                 125

gtg att ttg gct gac gat tta atc gat gcc caa aaa ggc gcg ctc aaa 432
Val Ile Leu Ala Asp Asp Leu Ile Asp Ala Gln Lys Gly Ala Leu Lys
    130                 135                 140

caa atg gtc gaa gtg tac gaa cgc agc ggc aac agc att ttg ggc gta 480
Gln Met Val Glu Val Tyr Glu Arg Ser Gly Asn Ser Ile Leu Gly Val
145                 150                 155                 160

gaa act gtc gaa ccg tcg caa acc ggc tca tac ggc atc gtc gaa acc 528
Glu Thr Val Glu Pro Ser Gln Thr Gly Ser Tyr Gly Ile Val Glu Thr
                165                 170                 175

gaa cag ctc aaa cag ttc caa cgc att acc ggc att gtc gaa aaa ccc 576
Glu Gln Leu Lys Gln Phe Gln Arg Ile Thr Gly Ile Val Glu Lys Pro
            180                 185                 190

aag ccc gaa gac gcg ccc tcc aac ctt gcc gtc gtc gga cgc tac atc 624
Lys Pro Glu Asp Ala Pro Ser Asn Leu Ala Val Val Gly Arg Tyr Ile
        195                 200                 205

ctt acc ccg cgc att ttc gac tta ctg acc gga ctg ccg cgc ggc gcg 672
Leu Thr Pro Arg Ile Phe Asp Leu Leu Thr Gly Leu Pro Arg Gly Ala

210                 215                 220

ggc aac gaa atc cag ctt aca gac ggc atc gcc aag ctg ctc gat cac     720
Gly Asn Glu Ile Gln Leu Thr Asp Gly Ile Ala Lys Leu Leu Asp His
225                 230                 235                 240

gaa ttt gtc cta gcg cac ccc ttt gaa ggc acg cgc tac gac tgc ggc     768
Glu Phe Val Leu Ala His Pro Phe Glu Gly Thr Arg Tyr Asp Cys Gly
                245                 250                 255

agc aaa ctg ggc tat ctg gaa gcc acc gtc gcc tac ggc ctg aaa cac     816
Ser Lys Leu Gly Tyr Leu Glu Ala Thr Val Ala Tyr Gly Leu Lys His
            260                 265                 270

ccc gaa acc ggc gaa ccc ttc cgc cgc ctt ttg gaa aaa tac cgt acc     864
Pro Glu Thr Gly Glu Pro Phe Arg Arg Leu Leu Glu Lys Tyr Arg Thr
        275                 280                 285

gaa                                                                 867
Glu

<210> 180
<211> 289
<212> PRT
<213> Neisseria meningitidis

<400> 180
Met Lys Pro Ile Arg Lys Ala Val Phe Pro Val Ala Gly Met Gly Thr
1               5                   10                  15

Arg Phe Leu Pro Ala Thr Lys Ala Ser Pro Lys Glu Met Leu Pro Ile
            20                  25                  30

Val Asp Lys Pro Leu Ile Gln Tyr Ala Val Glu Glu Ala Val Glu Ala
        35                  40                  45

Gly Cys Thr Glu Met Val Phe Val Thr Gly Arg Asn Lys Arg Ser Ile
    50                  55                  60

Glu Asp His Phe Asp Lys Ala Tyr Glu Leu Glu Thr Glu Leu Glu Met
65                  70                  75                  80

Arg His Lys Asp Lys Leu Leu Glu His Val Arg Asn Ile Leu Pro Pro
                85                  90                  95

Asn Ile Thr Cys Leu Tyr Ile Arg Gln Ala Glu Ala Leu Gly Leu Gly
            100                 105                 110

His Ala Val Leu Cys Ala Arg Ala Ala Ile Gly Asp Glu Pro Phe Ala
        115                 120                 125

Val Ile Leu Ala Asp Asp Leu Ile Asp Ala Gln Lys Gly Ala Leu Lys
    130                 135                 140

Gln Met Val Glu Val Tyr Glu Arg Ser Gly Asn Ser Ile Leu Gly Val
145                 150                 155                 160

Glu Thr Val Glu Pro Ser Gln Thr Gly Ser Tyr Gly Ile Val Glu Thr
                165                 170                 175

Glu Gln Leu Lys Gln Phe Gln Arg Ile Thr Gly Ile Val Glu Lys Pro
            180                 185                 190

Lys Pro Glu Asp Ala Pro Ser Asn Leu Ala Val Val Gly Arg Tyr Ile
        195                 200                 205

Leu Thr Pro Arg Ile Phe Asp Leu Leu Thr Gly Leu Pro Arg Gly Ala
    210                 215                 220

Gly Asn Glu Ile Gln Leu Thr Asp Gly Ile Ala Lys Leu Leu Asp His
225                 230                 235                 240

Glu Phe Val Leu Ala His Pro Phe Glu Gly Thr Arg Tyr Asp Cys Gly
                245                 250                 255

Ser Lys Leu Gly Tyr Leu Glu Ala Thr Val Ala Tyr Gly Leu Lys His
            260                 265                 270

Pro Glu Thr Gly Glu Pro Phe Arg Arg Leu Leu Glu Lys Tyr Arg Thr
        275                 280                 285

Glu

<210> 181
<211> 603
<212> DNA
<213> Neisseria meningitidis

<220>
<221> CDS
<222> (1)..(603)

<400> 181
atg aca ata atg aat atc aat act tcc gaa aat aaa gat gct gtt gcc    48
Met Thr Ile Met Asn Ile Asn Thr Ser Glu Asn Lys Asp Ala Val Ala
1               5                   10                  15

gaa cac acc gga caa tgg ttg gaa aaa gcc gtc atc ggt ctg aac ctg    96
Glu His Thr Gly Gln Trp Leu Glu Lys Ala Val Ile Gly Leu Asn Leu
            20                  25                  30

tgt ccc ttt gcc aaa gcc ccc cac gtt aaa aac ctt gtc cgc atc gca    144
Cys Pro Phe Ala Lys Ala Pro His Val Lys Asn Leu Val Arg Ile Ala
        35                  40                  45

atc agc gaa gcc aaa cac ctt gac agt ttt ttg gaa gac ttg gac gaa    192
Ile Ser Glu Ala Lys His Leu Asp Ser Phe Leu Glu Asp Leu Asp Glu
    50                  55                  60

gaa ctg cag cga ctg ggc aat aca ccc gcc acc gaa ctg gaa act acc    240
Glu Leu Gln Arg Leu Gly Asn Thr Pro Ala Thr Glu Leu Glu Thr Thr
65                  70                  75                  80

ctg ctg gtt cac ccg acc cta ttc ccc gat ttc gac gta ttc aac gat    288
Leu Leu Val His Pro Thr Leu Phe Pro Asp Phe Asp Val Phe Asn Asp
                85                  90                  95

atg ctc gac att gcc gat gcc gcc gtt gtc gaa aac ggc ttg gaa ggc    336
Met Leu Asp Ile Ala Asp Ala Ala Val Val Glu Asn Gly Leu Glu Gly
            100                 105                 110

atc atc caa atc gcc ccg ttt cat ccc gat ttc caa ttt gaa ggc acg    384
Ile Ile Gln Ile Ala Pro Phe His Pro Asp Phe Gln Phe Glu Gly Thr
        115                 120                 125

gat tca gac gac atc ggc aac tac acc aac cgt tct ccc tat ccg acg    432
Asp Ser Asp Asp Ile Gly Asn Tyr Thr Asn Arg Ser Pro Tyr Pro Thr
    130                 135                 140

ctg cac ctc atc cgc gaa gac agc att gcc aaa gcc gca caa gcc ttt    480
Leu His Leu Ile Arg Glu Asp Ser Ile Ala Lys Ala Ala Gln Ala Phe
145                 150                 155                 160

ccc gac gct tcg gca ata ttc gaa cgc aat atc gcc ctg ctg gaa aaa    528
Pro Asp Ala Ser Ala Ile Phe Glu Arg Asn Ile Ala Leu Leu Glu Lys
                165                 170                 175

atg gga cat gag ggc tgg gca aaa ctc ggt atc aca tcc tgc cct tat    576
Met Gly His Glu Gly Trp Ala Lys Leu Gly Ile Thr Ser Cys Pro Tyr
            180                 185                 190

ccg cac aat aag aaa aat att tca aaa                                    603
Pro His Asn Lys Lys Asn Ile Ser Lys
        195                 200

<210> 182
<211> 201
<212> PRT
<213> Neisseria meningitidis

<400> 182
Met Thr Ile Met Asn Ile Asn Thr Ser Glu Asn Lys Asp Ala Val Ala
  1               5                  10                  15

Glu His Thr Gly Gln Trp Leu Glu Lys Ala Val Ile Gly Leu Asn Leu
             20                  25                  30

Cys Pro Phe Ala Lys Ala Pro His Val Lys Asn Leu Val Arg Ile Ala
         35                  40                  45

Ile Ser Glu Ala Lys His Leu Asp Ser Phe Leu Glu Asp Leu Asp Glu
     50                  55                  60

Glu Leu Gln Arg Leu Gly Asn Thr Pro Ala Thr Glu Leu Glu Thr Thr
 65                  70                  75                  80

Leu Leu Val His Pro Thr Leu Phe Pro Asp Phe Asp Val Phe Asn Asp
                 85                  90                  95

Met Leu Asp Ile Ala Asp Ala Ala Val Val Glu Asn Gly Leu Glu Gly
            100                 105                 110

Ile Ile Gln Ile Ala Pro Phe His Pro Asp Phe Gln Phe Glu Gly Thr
        115                 120                 125

Asp Ser Asp Asp Ile Gly Asn Tyr Thr Asn Arg Ser Pro Tyr Pro Thr
    130                 135                 140

Leu His Leu Ile Arg Glu Asp Ser Ile Ala Lys Ala Ala Gln Ala Phe
145                 150                 155                 160

Pro Asp Ala Ser Ala Ile Phe Glu Arg Asn Ile Ala Leu Leu Glu Lys
                165                 170                 175

Met Gly His Glu Gly Trp Ala Lys Leu Gly Ile Thr Ser Cys Pro Tyr
            180                 185                 190

Pro His Asn Lys Lys Asn Ile Ser Lys
        195                 200

<210> 183
<211> 1338
<212> DNA
<213> Neisseria meningitidis

<220>
<221> CDS
<222> (1)..(1338)

<400> 183
ttg cac acc ctt cta gct ttt atc ttc gcc atc ctg att ttg gtc agc    48
Leu His Thr Leu Leu Ala Phe Ile Phe Ala Ile Leu Ile Leu Val Ser
  1               5                  10                  15

ctg cac gaa ttc gga cac tac atc gtc gcc aga ttg tgc ggc gtc aag    96
Leu His Glu Phe Gly His Tyr Ile Val Ala Arg Leu Cys Gly Val Lys
             20                  25                  30

gtt gtg cgt ttt tcc gtc ggc ttc ggc aaa ccg ttt ttc acc cga aag    144
Val Val Arg Phe Ser Val Gly Phe Gly Lys Pro Phe Phe Thr Arg Lys
         35                  40                  45

cgc ggc gac acc gaa tgg tgc ctc gcc ccg att ccg ttg ggc ggt tac    192
Arg Gly Asp Thr Glu Trp Cys Leu Ala Pro Ile Pro Leu Gly Gly Tyr
     50                  55                  60

gtc aaa atg gtc gac acg cgc gaa ggc gaa gta tca gaa gcc gat tta    240
Val Lys Met Val Asp Thr Arg Glu Gly Glu Val Ser Glu Ala Asp Leu
 65                  70                  75                  80

ccc tac gct ttt gac aaa caa cac ccc gcc aag cgc atc gcc atc gtc    288
Pro Tyr Ala Phe Asp Lys Gln His Pro Ala Lys Arg Ile Ala Ile Val
                 85                  90                  95

gcc gcc ggc ccg ctg acc aac ctc gca ctg gcg gtt ttg ctg tac gga    336
Ala Ala Gly Pro Leu Thr Asn Leu Ala Leu Ala Val Leu Leu Tyr Gly
            100                 105                 110

ctg agc ttt tcc ttc ggc gtt acc gaa ctg cgc ccc tat gtc ggc aca    384
Leu Ser Phe Ser Phe Gly Val Thr Glu Leu Arg Pro Tyr Val Gly Thr
        115                 120                 125

gtc gaa ccc gac acc att gcc gcc cgc gcc ggc ttc caa agc ggc gac    432

Val Glu Pro Asp Thr Ile Ala Ala Arg Ala Gly Phe Gln Ser Gly Asp
    130                 135                 140

aaa ata caa tcc gtc aac ggc aca ccc gtt gca gat tgg ggc agc gcg     480
Lys Ile Gln Ser Val Asn Gly Thr Pro Val Ala Asp Trp Gly Ser Ala
145                 150                 155                 160

caa acc gaa atc gtc ctc aac ctc gaa gcc ggc aaa gtc gcc gtc ggc     528
Gln Thr Glu Ile Val Leu Asn Leu Glu Ala Gly Lys Val Ala Val Gly
                165                 170                 175

gtt cag acg gca tcg ggc gcg caa acc gtc cgc acc atc gat gcc gca     576
Val Gln Thr Ala Ser Gly Ala Gln Thr Val Arg Thr Ile Asp Ala Ala
            180                 185                 190

ggc acg ccg gaa gcc ggt aaa atc gca aaa aac caa ggc tac atc gga     624
Gly Thr Pro Glu Ala Gly Lys Ile Ala Lys Asn Gln Gly Tyr Ile Gly
        195                 200                 205

ctg atg ccc ttt aaa atc aca acc gtt gcc ggc ggc gtg gaa aaa ggc     672
Leu Met Pro Phe Lys Ile Thr Thr Val Ala Gly Gly Val Glu Lys Gly
    210                 215                 220

agc ccc gcc gaa aaa gca ggc ctg aaa ccg ggc gac agg ctg act gcc     720
Ser Pro Ala Glu Lys Ala Gly Leu Lys Pro Gly Asp Arg Leu Thr Ala
225                 230                 235                 240

gcc gac ggc aaa ccc atc gcc tca tgg caa gaa tgg gca aac ctg acc     768
Ala Asp Gly Lys Pro Ile Ala Ser Trp Gln Glu Trp Ala Asn Leu Thr
                245                 250                 255

cgc caa agc ccc ggc aaa aaa atc acc ctg acc tac gaa cgc gcc gga     816
Arg Gln Ser Pro Gly Lys Lys Ile Thr Leu Thr Tyr Glu Arg Ala Gly
            260                 265                 270

caa acc cat acc gcc gac atc cgc ccc gat act gtc gaa cag ccc gac     864
Gln Thr His Thr Ala Asp Ile Arg Pro Asp Thr Val Glu Gln Pro Asp
        275                 280                 285

cac acc ctg atc ggg cgc gtc ggc ctc cgt ccg cag ccg gac agg gcg     912
His Thr Leu Ile Gly Arg Val Gly Leu Arg Pro Gln Pro Asp Arg Ala
    290                 295                 300

tgg gac gcg caa atc cgc cgc agc tac cgt ccg tct gtt gtc cgc gca     960
Trp Asp Ala Gln Ile Arg Arg Ser Tyr Arg Pro Ser Val Val Arg Ala
305                 310                 315                 320

ttc ggc atg ggc tgg gaa aaa acc gtt tcc cac tcg tgg aca acc ctc     1008

Phe Gly Met Gly Trp Glu Lys Thr Val Ser His Ser Trp Thr Thr Leu
                325                 330                 335

aaa ttt ttc ggc aaa cta atc agc ggc aac gcc tcc gtc agc cat att     1056
Lys Phe Phe Gly Lys Leu Ile Ser Gly Asn Ala Ser Val Ser His Ile
            340                 345                 350

tcc ggt ccg ctg acc att gcc gat att gcc gga cag tcc gcc gaa ctc     1104
Ser Gly Pro Leu Thr Ile Ala Asp Ile Ala Gly Gln Ser Ala Glu Leu
        355                 360                 365

ggc ttg caa agt tat ttg gaa ttt ttg gca ctg gtc agc atc agc ctc     1152
Gly Leu Gln Ser Tyr Leu Glu Phe Leu Ala Leu Val Ser Ile Ser Leu
    370                 375                 380

ggc gtg ctg aac ctg ctg ccc gtc ccc gtt ttg gac ggc ggc cac ctc     1200
Gly Val Leu Asn Leu Leu Pro Val Pro Val Leu Asp Gly Gly His Leu
385                 390                 395                 400

gtg ttt tat act gcc gaa tgg ata cgc ggc aaa cct ttg ggc gaa cgc     1248
Val Phe Tyr Thr Ala Glu Trp Ile Arg Gly Lys Pro Leu Gly Glu Arg
                405                 410                 415

gtc caa aac atc ggt ttg cgc ttc ggg ctt gcc ctc atg atg ctg atg     1296
Val Gln Asn Ile Gly Leu Arg Phe Gly Leu Ala Leu Met Met Leu Met
            420                 425                 430

atg gcg gtc gcc ttc ttc aac gac gtt acc cgg ctg ctc ggt             1338
Met Ala Val Ala Phe Phe Asn Asp Val Thr Arg Leu Leu Gly
        435                 440                 445

<210> 184
<211> 446
<212> PRT
<213> Neisseria meningitidis

<400> 184
Leu His Thr Leu Leu Ala Phe Ile Phe Ala Ile Leu Ile Leu Val Ser
1               5                   10                  15

Leu His Glu Phe Gly His Tyr Ile Val Ala Arg Leu Cys Gly Val Lys
            20                  25                  30

Val Val Arg Phe Ser Val Gly Phe Gly Lys Pro Phe Phe Thr Arg Lys
        35                  40                  45

Arg Gly Asp Thr Glu Trp Cys Leu Ala Pro Ile Pro Leu Gly Gly Tyr

50                  55                  60

Val Lys Met Val Asp Thr Arg Glu Gly Glu Val Ser Glu Ala Asp Leu
65                  70                  75                  80

Pro Tyr Ala Phe Asp Lys Gln His Pro Ala Lys Arg Ile Ala Ile Val
                85                  90                  95

Ala Ala Gly Pro Leu Thr Asn Leu Ala Leu Ala Val Leu Leu Tyr Gly
            100                 105                 110

Leu Ser Phe Ser Phe Gly Val Thr Glu Leu Arg Pro Tyr Val Gly Thr
        115                 120                 125

Val Glu Pro Asp Thr Ile Ala Ala Arg Ala Gly Phe Gln Ser Gly Asp
    130                 135                 140

Lys Ile Gln Ser Val Asn Gly Thr Pro Val Ala Asp Trp Gly Ser Ala
145                 150                 155                 160

Gln Thr Glu Ile Val Leu Asn Leu Glu Ala Gly Lys Val Ala Val Gly
                165                 170                 175

Val Gln Thr Ala Ser Gly Ala Gln Thr Val Arg Thr Ile Asp Ala Ala
            180                 185                 190

Gly Thr Pro Glu Ala Gly Lys Ile Ala Lys Asn Gln Gly Tyr Ile Gly
        195                 200                 205

Leu Met Pro Phe Lys Ile Thr Thr Val Ala Gly Gly Val Glu Lys Gly
    210                 215                 220

Ser Pro Ala Glu Lys Ala Gly Leu Lys Pro Gly Asp Arg Leu Thr Ala
225                 230                 235                 240

Ala Asp Gly Lys Pro Ile Ala Ser Trp Gln Glu Trp Ala Asn Leu Thr
                245                 250                 255

Arg Gln Ser Pro Gly Lys Lys Ile Thr Leu Thr Tyr Glu Arg Ala Gly
            260                 265                 270

Gln Thr His Thr Ala Asp Ile Arg Pro Asp Thr Val Glu Gln Pro Asp
        275                 280                 285

His Thr Leu Ile Gly Arg Val Gly Leu Arg Pro Gln Pro Asp Arg Ala
    290                 295                 300

Trp Asp Ala Gln Ile Arg Arg Ser Tyr Arg Pro Ser Val Val Arg Ala

305                 310                 315                 320

Phe Gly Met Gly Trp Glu Lys Thr Val Ser His Ser Trp Thr Thr Leu
                325                 330                 335

Lys Phe Phe Gly Lys Leu Ile Ser Gly Asn Ala Ser Val Ser His Ile
            340                 345                 350

Ser Gly Pro Leu Thr Ile Ala Asp Ile Ala Gly Gln Ser Ala Glu Leu
        355                 360                 365

Gly Leu Gln Ser Tyr Leu Glu Phe Leu Ala Leu Val Ser Ile Ser Leu
    370                 375                 380

Gly Val Leu Asn Leu Leu Pro Val Pro Val Leu Asp Gly Gly His Leu
385                 390                 395                 400

Val Phe Tyr Thr Ala Glu Trp Ile Arg Gly Lys Pro Leu Gly Glu Arg
                405                 410                 415

Val Gln Asn Ile Gly Leu Arg Phe Gly Leu Ala Leu Met Met Leu Met
            420                 425                 430

Met Ala Val Ala Phe Phe Asn Asp Val Thr Arg Leu Leu Gly
        435                 440                 445

<210> 185
<211> 1476
<212> DNA
<213> Neisseria meningitidis

<220>
<221> CDS
<222> (1)..(1476)

<400> 185
atg aaa tac aaa gac ctg cgc gac ttc atc gcc atg ctc gag cag cag     48
Met Lys Tyr Lys Asp Leu Arg Asp Phe Ile Ala Met Leu Glu Gln Gln
1               5                   10                  15

ggc aaa ctc aaa cgc atc gcg cac ccc gtt tcc ccg cat ttg gaa atg     96
Gly Lys Leu Lys Arg Ile Ala His Pro Val Ser Pro His Leu Glu Met
            20                  25                  30

acc gaa atc gcc gac cgc gtg ctg cgc gcc gaa ggg ccg gcg ttg ttg     144
Thr Glu Ile Ala Asp Arg Val Leu Arg Ala Glu Gly Pro Ala Leu Leu

35                    40                    45

ttt gaa cac cca gtt aag ccc gac ggt acg cgc tat gat tat ccc gtg     192
Phe Glu His Pro Val Lys Pro Asp Gly Thr Arg Tyr Asp Tyr Pro Val
    50                  55                  60

ttg gca aac ctg ttc ggc acg ccc gaa cgt gtg gcg atg ggc atg ggc     240
Leu Ala Asn Leu Phe Gly Thr Pro Glu Arg Val Ala Met Gly Met Gly
65                  70                  75                  80

gcg gac agc gtg tcc aag ctg cgc gaa atc ggg cag acg ctg gcg tat     288
Ala Asp Ser Val Ser Lys Leu Arg Glu Ile Gly Gln Thr Leu Ala Tyr
                85                  90                  95

ttg aaa gaa ccc gaa ccg ccc aaa ggc att aaa gac gcg ttt tcc aaa     336
Leu Lys Glu Pro Glu Pro Pro Lys Gly Ile Lys Asp Ala Phe Ser Lys
            100                 105                 110

ctg ccg ctc ttg aaa gac att tgg agc atg gcg ccg aac gtg gtg aaa     384
Leu Pro Leu Leu Lys Asp Ile Trp Ser Met Ala Pro Asn Val Val Lys
        115                 120                 125

aat gcg ccg tgt cag gaa atc gta tgg gaa ggc gaa gac gtt gat ttg     432
Asn Ala Pro Cys Gln Glu Ile Val Trp Glu Gly Glu Asp Val Asp Leu
    130                 135                 140

tat caa ctt ccg att cag cat tgc tgg ccg gaa gac gtt gcg ccg ctg     480
Tyr Gln Leu Pro Ile Gln His Cys Trp Pro Glu Asp Val Ala Pro Leu
145                 150                 155                 160

gta acg tgg ggc ttg acc gtc acg cgc ggg ccg cac aaa aaa cgc caa     528
Val Thr Trp Gly Leu Thr Val Thr Arg Gly Pro His Lys Lys Arg Gln
                165                 170                 175

aat ctc ggc att tac cgc caa caa tta atc ggc ata aac aag ctg att     576
Asn Leu Gly Ile Tyr Arg Gln Gln Leu Ile Gly Ile Asn Lys Leu Ile
            180                 185                 190

atg cgc tgg ctg tcg cat cgc ggc ggc gcg ctg gat tat cag gaa ttc     624
Met Arg Trp Leu Ser His Arg Gly Gly Ala Leu Asp Tyr Gln Glu Phe
        195                 200                 205

cgc aaa ctc aat ccc gat acg ccg tat ccc gtc gcc gtc gtg ctc ggc     672
Arg Lys Leu Asn Pro Asp Thr Pro Tyr Pro Val Ala Val Val Leu Gly
    210                 215                 220

tgc gac cct gcc acc att ttg ggc gcg gta acg ccc gtt ccc gat acc     720
Cys Asp Pro Ala Thr Ile Leu Gly Ala Val Thr Pro Val Pro Asp Thr

225                 230                 235                 240

ttg agc gaa tac cag ttt gcc gga ctg ctg cgc ggt tcg cgg acg gaa     768
Leu Ser Glu Tyr Gln Phe Ala Gly Leu Leu Arg Gly Ser Arg Thr Glu
                245                 250                 255

ctg gtg aaa tgt atc ggc aac gat ttg caa gtg cct gcc cgc gca gaa     816
Leu Val Lys Cys Ile Gly Asn Asp Leu Gln Val Pro Ala Arg Ala Glu
            260                 265                 270

atc gtg ttg gaa ggc gtc atc cat cca aac gaa acc gcg ttg gaa ggt     864
Ile Val Leu Glu Gly Val Ile His Pro Asn Glu Thr Ala Leu Glu Gly
        275                 280                 285

cca tac ggc gac cac acg ggc tat tac aac gag cag gac cat ttc ccc     912
Pro Tyr Gly Asp His Thr Gly Tyr Tyr Asn Glu Gln Asp His Phe Pro
    290                 295                 300

gtg ttt acg gtc gaa cgc atc acc atg cgc gaa aac ccg att tac cac     960
Val Phe Thr Val Glu Arg Ile Thr Met Arg Glu Asn Pro Ile Tyr His
305                 310                 315                 320

tct acc tac aca ggc aaa ccg ccc gat gaa ccc gcc gtt ttg ggc gtg     1008
Ser Thr Tyr Thr Gly Lys Pro Pro Asp Glu Pro Ala Val Leu Gly Val
                325                 330                 335

gcg ttg aac gaa gtg ttc gta ccg ctt ttg caa aag cag ttc ccc gaa     1056
Ala Leu Asn Glu Val Phe Val Pro Leu Leu Gln Lys Gln Phe Pro Glu
            340                 345                 350

atc acc gat ttt tac ctg ccg ccc gaa ggc tgc tcc tac cgc atg gcg     1104
Ile Thr Asp Phe Tyr Leu Pro Pro Glu Gly Cys Ser Tyr Arg Met Ala
        355                 360                 365

gtg gtg agc atg aaa aaa cag tac gcc gga cac gcc aag cgc gtg atg     1152
Val Val Ser Met Lys Lys Gln Tyr Ala Gly His Ala Lys Arg Val Met
    370                 375                 380

atg ggc tgc tgg tcg ttc ctg cgc cag ttt atg tac acc aaa ttc atc     1200
Met Gly Cys Trp Ser Phe Leu Arg Gln Phe Met Tyr Thr Lys Phe Ile
385                 390                 395                 400

att gtg gtg gat gac gat gtg gat gtg cgc gac tgg aaa gaa gtc atc     1248
Ile Val Val Asp Asp Asp Val Asp Val Arg Asp Trp Lys Glu Val Ile
                405                 410                 415

tgg gcg gta acc acg cgc atg gac ccc gtg cgc gat acc gtt ttg atg     1296
Trp Ala Val Thr Thr Arg Met Asp Pro Val Arg Asp Thr Val Leu Met

                420                 425                 430

gaa aac acg ccc atc gac tac ctc gac ttc gcc agc ccc gtc agc gga     1344
Glu Asn Thr Pro Ile Asp Tyr Leu Asp Phe Ala Ser Pro Val Ser Gly
        435                 440                 445

ctt ggc ggc aaa atg ggt ttg gat gcg acc aac aag tgg ccg ggc gaa     1392
Leu Gly Gly Lys Met Gly Leu Asp Ala Thr Asn Lys Trp Pro Gly Glu
    450                 455                 460

acc gac cgc gaa tgg gga cgg gtg att aaa aaa gac cct gcg gtt acg     1440
Thr Asp Arg Glu Trp Gly Arg Val Ile Lys Lys Asp Pro Ala Val Thr
465                 470                 475                 480

gct aag att gat gag att tgg gag gaa ttg ggg ttg                     1476
Ala Lys Ile Asp Glu Ile Trp Glu Glu Leu Gly Leu
                485                 490

<210> 186
<211> 492
<212> PRT
<213> Neisseria meningitidis

<400> 186
Met Lys Tyr Lys Asp Leu Arg Asp Phe Ile Ala Met Leu Glu Gln Gln
1               5                   10                  15

Gly Lys Leu Lys Arg Ile Ala His Pro Val Ser Pro His Leu Glu Met
            20                  25                  30

Thr Glu Ile Ala Asp Arg Val Leu Arg Ala Glu Gly Pro Ala Leu Leu
        35                  40                  45

Phe Glu His Pro Val Lys Pro Asp Gly Thr Arg Tyr Asp Tyr Pro Val
    50                  55                  60

Leu Ala Asn Leu Phe Gly Thr Pro Glu Arg Val Ala Met Gly Met Gly
65                  70                  75                  80

Ala Asp Ser Val Ser Lys Leu Arg Glu Ile Gly Gln Thr Leu Ala Tyr
                85                  90                  95

Leu Lys Glu Pro Glu Pro Pro Lys Gly Ile Lys Asp Ala Phe Ser Lys
            100                 105                 110

Leu Pro Leu Leu Lys Asp Ile Trp Ser Met Ala Pro Asn Val Val Lys
        115                 120                 125

Asn Ala Pro Cys Gln Glu Ile Val Trp Glu Gly Glu Asp Val Asp Leu
    130                 135                 140

Tyr Gln Leu Pro Ile Gln His Cys Trp Pro Glu Asp Val Ala Pro Leu
145                 150                 155                 160

Val Thr Trp Gly Leu Thr Val Thr Arg Gly Pro His Lys Lys Arg Gln
                165                 170                 175

Asn Leu Gly Ile Tyr Arg Gln Gln Leu Ile Gly Ile Asn Lys Leu Ile
            180                 185                 190

Met Arg Trp Leu Ser His Arg Gly Gly Ala Leu Asp Tyr Gln Glu Phe
        195                 200                 205

Arg Lys Leu Asn Pro Asp Thr Pro Tyr Pro Val Ala Val Val Leu Gly
    210                 215                 220

Cys Asp Pro Ala Thr Ile Leu Gly Ala Val Thr Pro Val Pro Asp Thr
225                 230                 235                 240

Leu Ser Glu Tyr Gln Phe Ala Gly Leu Leu Arg Gly Ser Arg Thr Glu
                245                 250                 255

Leu Val Lys Cys Ile Gly Asn Asp Leu Gln Val Pro Ala Arg Ala Glu
            260                 265                 270

Ile Val Leu Glu Gly Val Ile His Pro Asn Glu Thr Ala Leu Glu Gly
        275                 280                 285

Pro Tyr Gly Asp His Thr Gly Tyr Tyr Asn Glu Gln Asp His Phe Pro
    290                 295                 300

Val Phe Thr Val Glu Arg Ile Thr Met Arg Glu Asn Pro Ile Tyr His
305                 310                 315                 320

Ser Thr Tyr Thr Gly Lys Pro Pro Asp Glu Pro Ala Val Leu Gly Val
                325                 330                 335

Ala Leu Asn Glu Val Phe Val Pro Leu Leu Gln Lys Gln Phe Pro Glu
            340                 345                 350

Ile Thr Asp Phe Tyr Leu Pro Pro Glu Gly Cys Ser Tyr Arg Met Ala
        355                 360                 365

Val Val Ser Met Lys Lys Gln Tyr Ala Gly His Ala Lys Arg Val Met
    370                 375                 380

Met Gly Cys Trp Ser Phe Leu Arg Gln Phe Met Tyr Thr Lys Phe Ile
385                 390                 395                 400

Ile Val Val Asp Asp Asp Val Asp Val Arg Asp Trp Lys Glu Val Ile
                405                 410                 415

Trp Ala Val Thr Thr Arg Met Asp Pro Val Arg Asp Thr Val Leu Met
            420                 425                 430

Glu Asn Thr Pro Ile Asp Tyr Leu Asp Phe Ala Ser Pro Val Ser Gly
        435                 440                 445

Leu Gly Gly Lys Met Gly Leu Asp Ala Thr Asn Lys Trp Pro Gly Glu
    450                 455                 460

Thr Asp Arg Glu Trp Gly Arg Val Ile Lys Lys Asp Pro Ala Val Thr
465                 470                 475                 480

Ala Lys Ile Asp Glu Ile Trp Glu Glu Leu Gly Leu
                485                 490

<210> 187
<211> 1191
<212> DNA
<213> Neisseria meningitidis

<220>
<221> CDS
<222> (1)..(1191)

<400> 187
atg ttc ttc aag cac atc gaa gcc gcc ccc gcc gat ccg att ctc ggt      48
Met Phe Phe Lys His Ile Glu Ala Ala Pro Ala Asp Pro Ile Leu Gly
  1               5                  10                  15

ttg ggc gaa gca ttc aaa gcc gaa acc cgc ccc gaa aaa gtc aac ctc      96
Leu Gly Glu Ala Phe Lys Ala Glu Thr Arg Pro Glu Lys Val Asn Leu
             20                  25                  30

ggc atc ggc gtg tac aaa gac gca tcc ggc gcg aca cct att gtc aaa     144
Gly Ile Gly Val Tyr Lys Asp Ala Ser Gly Ala Thr Pro Ile Val Lys
         35                  40                  45

gcc gtc aaa gaa gcc gaa aaa cgc ctg ttg gaa agc gaa aca act aaa     192
Ala Val Lys Glu Ala Glu Lys Arg Leu Leu Glu Ser Glu Thr Thr Lys

50                  55                  60

aac tac ctc acc atc gac ggc gtt gcc gac tac aac gag caa acc caa    240
Asn Tyr Leu Thr Ile Asp Gly Val Ala Asp Tyr Asn Glu Gln Thr Gln
65                  70                  75                  80

att ctg ctg ttc ggc aaa gac cac gaa atc atc gcc agc cgt cgc gcc    288
Ile Leu Leu Phe Gly Lys Asp His Glu Ile Ile Ala Ser Arg Arg Ala
                85                  90                  95

aaa aca gcg caa agc ctt ggc ggt aca ggc gca ttg cgt att gcg gcc    336
Lys Thr Ala Gln Ser Leu Gly Gly Thr Gly Ala Leu Arg Ile Ala Ala
            100                 105                 110

gag ttt gcc aaa cgt cag ttg aac gcg caa acc atc tgg att tcc aat    384
Glu Phe Ala Lys Arg Gln Leu Asn Ala Gln Thr Ile Trp Ile Ser Asn
        115                 120                 125

ccg act tgg ccc aac cac aac gcc atc gcc aaa gcg gtc ggt atc caa    432
Pro Thr Trp Pro Asn His Asn Ala Ile Ala Lys Ala Val Gly Ile Gln
    130                 135                 140

gac caa cct tat cgc tac tat gat gcc gcc aaa cac ggt ttg gat tgg    480
Asp Gln Pro Tyr Arg Tyr Tyr Asp Ala Ala Lys His Gly Leu Asp Trp
145                 150                 155                 160

gac ggt atg att gaa gac ttg agc caa gcg caa aaa ggc gac atc gtc    528
Asp Gly Met Ile Glu Asp Leu Ser Gln Ala Gln Lys Gly Asp Ile Val
                165                 170                 175

ctg ctg cac ggc tgc tgc cac aac cct acc ggt atc gac cct acg ccc    576
Leu Leu His Gly Cys Cys His Asn Pro Thr Gly Ile Asp Pro Thr Pro
            180                 185                 190

gaa caa tgg gaa act ctg gca aaa ctt tct gcc gaa aaa ggc tgg tta    624
Glu Gln Trp Glu Thr Leu Ala Lys Leu Ser Ala Glu Lys Gly Trp Leu
        195                 200                 205

ccg ctg ttt gac ttt gcc tac caa ggc ttc ggc aat ggt ttg gaa gaa    672
Pro Leu Phe Asp Phe Ala Tyr Gln Gly Phe Gly Asn Gly Leu Glu Glu
    210                 215                 220

gat gcc tac ggc ctg cgc gtg ttc ttg aaa cac aat aca gaa ttg ctg    720
Asp Ala Tyr Gly Leu Arg Val Phe Leu Lys His Asn Thr Glu Leu Leu
225                 230                 235                 240

att gcc agc tct tat tcc aaa aac ttc ggt atg tac aac gag cgc gtc    768
Ile Ala Ser Ser Tyr Ser Lys Asn Phe Gly Met Tyr Asn Glu Arg Val

245                 250                 255

ggc gcg ttc act ttg gtg gcc gaa gat gaa gca acc gcc gcc cgc gcc     816
Gly Ala Phe Thr Leu Val Ala Glu Asp Glu Ala Thr Ala Ala Arg Ala
            260                 265                 270

cac agc caa gtc aaa acc att atc cgt acc ttg tat tcc aac cct gct     864
His Ser Gln Val Lys Thr Ile Ile Arg Thr Leu Tyr Ser Asn Pro Ala
        275                 280                 285

tca cac ggc gcg aac act att gcg ctg gtg ttg aaa aat gat gat ttg     912
Ser His Gly Ala Asn Thr Ile Ala Leu Val Leu Lys Asn Asp Asp Leu
    290                 295                 300

aaa gca caa tgg att gcc gaa ctc gat gaa atg cgc ggc cgt atc aaa     960
Lys Ala Gln Trp Ile Ala Glu Leu Asp Glu Met Arg Gly Arg Ile Lys
305                 310                 315                 320

gcc atg cgc caa aaa ttt gtc gag ttg ctc aaa gcc aaa ggt gca act     1008
Ala Met Arg Gln Lys Phe Val Glu Leu Leu Lys Ala Lys Gly Ala Thr
                325                 330                 335

caa gac ttt gat ttc att atc gaa caa aac ggc atg ttc tcc ttc agc     1056
Gln Asp Phe Asp Phe Ile Ile Glu Gln Asn Gly Met Phe Ser Phe Ser
            340                 345                 350

ggc ttg act ccc gaa caa gtc gac cgt tta aaa aac gag ttt gcc att     1104
Gly Leu Thr Pro Glu Gln Val Asp Arg Leu Lys Asn Glu Phe Ala Ile
        355                 360                 365

tat gcc gtc cgc tcc ggc cgc atc aac gtc gcc ggc att acc gac gac     1152
Tyr Ala Val Arg Ser Gly Arg Ile Asn Val Ala Gly Ile Thr Asp Asp
    370                 375                 380

aac atc gat tat ctg tgc gaa agt atc gta aaa gta ctg                 1191
Asn Ile Asp Tyr Leu Cys Glu Ser Ile Val Lys Val Leu
385                 390                 395

<210> 188
<211> 397
<212> PRT
<213> Neisseria meningitidis

<400> 188
Met Phe Phe Lys His Ile Glu Ala Ala Pro Ala Asp Pro Ile Leu Gly
1               5                   10                  15

Leu Gly Glu Ala Phe Lys Ala Glu Thr Arg Pro Glu Lys Val Asn Leu
            20                  25                  30

Gly Ile Gly Val Tyr Lys Asp Ala Ser Gly Ala Thr Pro Ile Val Lys
        35                  40                  45

Ala Val Lys Glu Ala Glu Lys Arg Leu Leu Glu Ser Glu Thr Thr Lys
    50                  55                  60

Asn Tyr Leu Thr Ile Asp Gly Val Ala Asp Tyr Asn Glu Gln Thr Gln
65                  70                  75                  80

Ile Leu Leu Phe Gly Lys Asp His Glu Ile Ile Ala Ser Arg Arg Ala
                85                  90                  95

Lys Thr Ala Gln Ser Leu Gly Gly Thr Gly Ala Leu Arg Ile Ala Ala
            100                 105                 110

Glu Phe Ala Lys Arg Gln Leu Asn Ala Gln Thr Ile Trp Ile Ser Asn
        115                 120                 125

Pro Thr Trp Pro Asn His Asn Ala Ile Ala Lys Ala Val Gly Ile Gln
    130                 135                 140

Asp Gln Pro Tyr Arg Tyr Tyr Asp Ala Ala Lys His Gly Leu Asp Trp
145                 150                 155                 160

Asp Gly Met Ile Glu Asp Leu Ser Gln Ala Gln Lys Gly Asp Ile Val
                165                 170                 175

Leu Leu His Gly Cys Cys His Asn Pro Thr Gly Ile Asp Pro Thr Pro
            180                 185                 190

Glu Gln Trp Glu Thr Leu Ala Lys Leu Ser Ala Glu Lys Gly Trp Leu
        195                 200                 205

Pro Leu Phe Asp Phe Ala Tyr Gln Gly Phe Gly Asn Gly Leu Glu Glu
    210                 215                 220

Asp Ala Tyr Gly Leu Arg Val Phe Leu Lys His Asn Thr Glu Leu Leu
225                 230                 235                 240

Ile Ala Ser Ser Tyr Ser Lys Asn Phe Gly Met Tyr Asn Glu Arg Val
                245                 250                 255

Gly Ala Phe Thr Leu Val Ala Glu Asp Glu Ala Thr Ala Ala Arg Ala
            260                 265                 270

His Ser Gln Val Lys Thr Ile Ile Arg Thr Leu Tyr Ser Asn Pro Ala
        275                 280                 285

Ser His Gly Ala Asn Thr Ile Ala Leu Val Leu Lys Asn Asp Asp Leu
    290                 295                 300

Lys Ala Gln Trp Ile Ala Glu Leu Asp Glu Met Arg Gly Arg Ile Lys
305                 310                 315                 320

Ala Met Arg Gln Lys Phe Val Glu Leu Leu Lys Ala Lys Gly Ala Thr
                325                 330                 335

Gln Asp Phe Asp Phe Ile Ile Glu Gln Asn Gly Met Phe Ser Phe Ser
            340                 345                 350

Gly Leu Thr Pro Glu Gln Val Asp Arg Leu Lys Asn Glu Phe Ala Ile
        355                 360                 365

Tyr Ala Val Arg Ser Gly Arg Ile Asn Val Ala Gly Ile Thr Asp Asp
    370                 375                 380

Asn Ile Asp Tyr Leu Cys Glu Ser Ile Val Lys Val Leu
385                 390                 395

<210> 189
<211> 462
<212> DNA
<213> Neisseria meningitidis

<220>
<221> CDS
<222> (1)..(462)

<400> 189
ttg ctt tgc ccg gaa aaa atg tcg ggg atg gcg gga cag tat ccg tac    48
Leu Leu Cys Pro Glu Lys Met Ser Gly Met Ala Gly Gln Tyr Pro Tyr
1               5                   10                  15

ggc gtc cgg tcg ggt ttg cgg agg aac ggc ttg aaa ctt tgg gat att    96
Gly Val Arg Ser Gly Leu Arg Arg Asn Gly Leu Lys Leu Trp Asp Ile
            20                  25                  30

cat ttt aga atg acc cgt ttt atc gtc gca aga tgc ggt tta ttg ttt    144
His Phe Arg Met Thr Arg Phe Ile Val Ala Arg Cys Gly Leu Leu Phe
        35                  40                  45

gca acc ctt aaa gga aaa acc atg aag aaa atg ttc gtg ctg ttc tgt     192
Ala Thr Leu Lys Gly Lys Thr Met Lys Lys Met Phe Val Leu Phe Cys
     50                  55                  60

atg ctg ttc tcc tgc gcc ttc tcc ctt gcg gcg gta aac atc aat gcg     240
Met Leu Phe Ser Cys Ala Phe Ser Leu Ala Ala Val Asn Ile Asn Ala
 65                  70                  75                  80

gct tcg cag cag gag ctg gag gcg ctg ccg ggc ata ggc cct gcg aag     288
Ala Ser Gln Gln Glu Leu Glu Ala Leu Pro Gly Ile Gly Pro Ala Lys
                 85                  90                  95

gcg aag gcc att gcg gaa tac cgt gcg caa aac ggt gcg ttc aag tct     336
Ala Lys Ala Ile Ala Glu Tyr Arg Ala Gln Asn Gly Ala Phe Lys Ser
            100                 105                 110

gta gac gat ttg acc aag gta aag ggc atc ggc cct gcg gtg ctg gcg     384
Val Asp Asp Leu Thr Lys Val Lys Gly Ile Gly Pro Ala Val Leu Ala
        115                 120                 125

aag ctg aag gat cag gct tct gtc ggt gcg ccc gca cca aaa ggc ccg     432
Lys Leu Lys Asp Gln Ala Ser Val Gly Ala Pro Ala Pro Lys Gly Pro
    130                 135                 140

gca aaa ccg gct ctg ccc gca gct aaa aaa                             462
Ala Lys Pro Ala Leu Pro Ala Ala Lys Lys
145                 150

<210> 190
<211> 154
<212> PRT
<213> Neisseria meningitidis

<400> 190
Leu Leu Cys Pro Glu Lys Met Ser Gly Met Ala Gly Gln Tyr Pro Tyr
  1               5                  10                  15

Gly Val Arg Ser Gly Leu Arg Arg Asn Gly Leu Lys Leu Trp Asp Ile
             20                  25                  30

His Phe Arg Met Thr Arg Phe Ile Val Ala Arg Cys Gly Leu Leu Phe
         35                  40                  45

Ala Thr Leu Lys Gly Lys Thr Met Lys Lys Met Phe Val Leu Phe Cys
     50                  55                  60

Met Leu Phe Ser Cys Ala Phe Ser Leu Ala Ala Val Asn Ile Asn Ala

65                  70                  75                  80

Ala Ser Gln Gln Glu Leu Glu Ala Leu Pro Gly Ile Gly Pro Ala Lys
                85                  90                  95

Ala Lys Ala Ile Ala Glu Tyr Arg Ala Gln Asn Gly Ala Phe Lys Ser
            100                 105                 110

Val Asp Asp Leu Thr Lys Val Lys Gly Ile Gly Pro Ala Val Leu Ala
        115                 120                 125

Lys Leu Lys Asp Gln Ala Ser Val Gly Ala Pro Ala Pro Lys Gly Pro
    130                 135                 140

Ala Lys Pro Ala Leu Pro Ala Ala Lys Lys
145                 150

<210> 191
<211> 684
<212> DNA
<213> Neisseria meningitidis

<220>
<221> CDS
<222> (1)..(684)

<400> 191
atg ttt gct ttt tta gaa gcc ttt ttt gtc gaa tac ggt tat gcg gct    48
Met Phe Ala Phe Leu Glu Ala Phe Phe Val Glu Tyr Gly Tyr Ala Ala
1               5                   10                  15

gtt ttt ttt gta ttg gtc atc tgc ggt ttc ggc gtg ccg att ccc gag    96
Val Phe Phe Val Leu Val Ile Cys Gly Phe Gly Val Pro Ile Pro Glu
            20                  25                  30

gat ttg acc ttg gta aca ggc ggc gtg att tcg ggt atg ggt tat acc    144
Asp Leu Thr Leu Val Thr Gly Gly Val Ile Ser Gly Met Gly Tyr Thr
        35                  40                  45

aat ccg cat att atg ttt gca gtc ggt atg ctc ggc gta ttg gtc ggg    192
Asn Pro His Ile Met Phe Ala Val Gly Met Leu Gly Val Leu Val Gly
    50                  55                  60

gac ggc atc atg ttc gcc gcc gga cga att tgg ggg cag aaa atc cta    240
Asp Gly Ile Met Phe Ala Ala Gly Arg Ile Trp Gly Gln Lys Ile Leu
65                  70                  75                  80

agg ttc aaa cct att gcg cgc atc atg acg ccg aaa cgt tat gag cag     288
Arg Phe Lys Pro Ile Ala Arg Ile Met Thr Pro Lys Arg Tyr Glu Gln
                85                  90                  95

gtt cag gaa aaa ttc gac aaa tac ggt aac tgg gtc tta ttt gtc gcc     336
Val Gln Glu Lys Phe Asp Lys Tyr Gly Asn Trp Val Leu Phe Val Ala
            100                 105                 110

cgt ttc ctg ccc ggt ttg aga acg gcc gta ttt gtt aca gcc ggt atc     384
Arg Phe Leu Pro Gly Leu Arg Thr Ala Val Phe Val Thr Ala Gly Ile
        115                 120                 125

agc cgc aag gtt tca tac ttg cgt ttt atc att atg gat gga ctg gcc     432
Ser Arg Lys Val Ser Tyr Leu Arg Phe Ile Ile Met Asp Gly Leu Ala
    130                 135                 140

gca ctg att tcc gtc cct att tgg att tat ctg ggc gaa tac ggt gcg     480
Ala Leu Ile Ser Val Pro Ile Trp Ile Tyr Leu Gly Glu Tyr Gly Ala
145                 150                 155                 160

cac aac atc gat tgg ctg atg gcg aaa atg cac agc ctg caa tcg ggt     528
His Asn Ile Asp Trp Leu Met Ala Lys Met His Ser Leu Gln Ser Gly
                165                 170                 175

att ttt gtt atc ttg ggt ata ggt gcg acc gtt gtc gct tgg att tgg     576
Ile Phe Val Ile Leu Gly Ile Gly Ala Thr Val Val Ala Trp Ile Trp
            180                 185                 190

tgg aaa aaa cgc caa cgt atc cag ttt tac cgc agc aaa ttg aaa gaa     624
Trp Lys Lys Arg Gln Arg Ile Gln Phe Tyr Arg Ser Lys Leu Lys Glu
        195                 200                 205

aag cgg gcg caa cgc aaa gcc gcc aag gca gcc aaa aaa gcc gcg caa     672
Lys Arg Ala Gln Arg Lys Ala Ala Lys Ala Ala Lys Lys Ala Ala Gln
    210                 215                 220

agc aaa caa taa                                                     684
Ser Lys Gln
225

<210> 192
<211> 227
<212> PRT
<213> Neisseria meningitidis

<400> 192

Met Phe Ala Phe Leu Glu Ala Phe Phe Val Glu Tyr Gly Tyr Ala Ala
1               5                   10                  15

Val Phe Phe Val Leu Val Ile Cys Gly Phe Gly Val Pro Ile Pro Glu
            20                  25                  30

Asp Leu Thr Leu Val Thr Gly Gly Val Ile Ser Gly Met Gly Tyr Thr
        35                  40                  45

Asn Pro His Ile Met Phe Ala Val Gly Met Leu Gly Val Leu Val Gly
    50                  55                  60

Asp Gly Ile Met Phe Ala Ala Gly Arg Ile Trp Gly Gln Lys Ile Leu
65                  70                  75                  80

Arg Phe Lys Pro Ile Ala Arg Ile Met Thr Pro Lys Arg Tyr Glu Gln
                85                  90                  95

Val Gln Glu Lys Phe Asp Lys Tyr Gly Asn Trp Val Leu Phe Val Ala
            100                 105                 110

Arg Phe Leu Pro Gly Leu Arg Thr Ala Val Phe Val Thr Ala Gly Ile
        115                 120                 125

Ser Arg Lys Val Ser Tyr Leu Arg Phe Ile Ile Met Asp Gly Leu Ala
    130                 135                 140

Ala Leu Ile Ser Val Pro Ile Trp Ile Tyr Leu Gly Glu Tyr Gly Ala
145                 150                 155                 160

His Asn Ile Asp Trp Leu Met Ala Lys Met His Ser Leu Gln Ser Gly
                165                 170                 175

Ile Phe Val Ile Leu Gly Ile Gly Ala Thr Val Val Ala Trp Ile Trp
            180                 185                 190

Trp Lys Lys Arg Gln Arg Ile Gln Phe Tyr Arg Ser Lys Leu Lys Glu
        195                 200                 205

Lys Arg Ala Gln Arg Lys Ala Ala Lys Ala Ala Lys Lys Ala Ala Gln
    210                 215                 220

Ser Lys Gln
225

<210> 193

<211> 1089
<212> DNA
<213> Neisseria meningitidis

<220>
<221> CDS
<222> (1)..(1089)

<400> 193
atg aat gac tac acg cag cag ctt caa ggt aaa aaa gac tac ctt aaa 48
Met Asn Asp Tyr Thr Gln Gln Leu Gln Gly Lys Lys Asp Tyr Leu Lys
1               5                   10                  15

acc ctt ttt gca ggt ttg gat gtt cct gag tgg gaa gtg tac gaa tct 96
Thr Leu Phe Ala Gly Leu Asp Val Pro Glu Trp Glu Val Tyr Glu Ser
            20                  25                  30

ccg gac aaa cat tac cgt atg cgt gcc gag ttc cgt att tgg cac gaa 144
Pro Asp Lys His Tyr Arg Met Arg Ala Glu Phe Arg Ile Trp His Glu
        35                  40                  45

ggc ggg gaa atg ttt tat gcc atg ttt gaa aaa ggg cag aaa gcc agc 192
Gly Gly Glu Met Phe Tyr Ala Met Phe Glu Lys Gly Gln Lys Ala Ser
    50                  55                  60

ggc gca agc atg ata cgc tgc gac cgt ttt gaa gca gct tcc gag gct 240
Gly Ala Ser Met Ile Arg Cys Asp Arg Phe Glu Ala Ala Ser Glu Ala
65                  70                  75                  80

gtc aac cgc ctc atg ccc gag ctg atc gcc gcc gcc gcg caa tcc ccc 288
Val Asn Arg Leu Met Pro Glu Leu Ile Ala Ala Ala Ala Gln Ser Pro
                85                  90                  95

gaa ctc aaa aaa cgc tgg tat gcc gtc gaa ttt ctg tcc acg ctc agc 336
Glu Leu Lys Lys Arg Trp Tyr Ala Val Glu Phe Leu Ser Thr Leu Ser
            100                 105                 110

ggg gaa atg ctg gtt acc atg att tac cac aaa agg ctt gat gct gag 384
Gly Glu Met Leu Val Thr Met Ile Tyr His Lys Arg Leu Asp Ala Glu
        115                 120                 125

tgg atg cag gcg gcg caa gcg tta cag caa cag ttg gat att tcc gtt 432
Trp Met Gln Ala Ala Gln Ala Leu Gln Gln Gln Leu Asp Ile Ser Val
    130                 135                 140

att ggg cgg agc agg gga cag aaa ata gtc tta aaa cag gac tat gta 480
Ile Gly Arg Ser Arg Gly Gln Lys Ile Val Leu Lys Gln Asp Tyr Val
145                 150                 155                 160

acg gaa act ttg aag gtc ggc aac cgg gat ttc cgt tat cgg caa att 528
Thr Glu Thr Leu Lys Val Gly Asn Arg Asp Phe Arg Tyr Arg Gln Ile
                165                 170                 175

gaa ggc agt ttt acc caa ccg aat gcc gcc gtg tgt cag aaa atg ctt 576
Glu Gly Ser Phe Thr Gln Pro Asn Ala Ala Val Cys Gln Lys Met Leu
            180                 185                 190

gaa tgg gcg tgc cgc acg gcg gaa ggc ttg ggc agc gac ctg ctc gaa 624
Glu Trp Ala Cys Arg Thr Ala Glu Gly Leu Gly Ser Asp Leu Leu Glu
        195                 200                 205

ctg tat tgc gga aac ggc aat ttc acc ctg ccg ctg tcc cga tat ttc 672
Leu Tyr Cys Gly Asn Gly Asn Phe Thr Leu Pro Leu Ser Arg Tyr Phe
    210                 215                 220

cga cag gtt ttg gca acc gaa atc tcc aaa acc tcc gtc agc gcg gcg 720
Arg Gln Val Leu Ala Thr Glu Ile Ser Lys Thr Ser Val Ser Ala Ala
225                 230                 235                 240

caa tgg aat atc gaa gcc aac cgc atc ggc aac atc aaa atc gcc cgt 768
Gln Trp Asn Ile Glu Ala Asn Arg Ile Gly Asn Ile Lys Ile Ala Arg
                245                 250                 255

ctg tct gct gaa gaa ttt acc gaa gcc tat acc gga aaa cgt gag ttt 816
Leu Ser Ala Glu Glu Phe Thr Glu Ala Tyr Thr Gly Lys Arg Glu Phe
            260                 265                 270

aaa cgc ctt aag gat ggc ggt att gct ttg acg gat tac gca ttt tcc 864
Lys Arg Leu Lys Asp Gly Gly Ile Ala Leu Thr Asp Tyr Ala Phe Ser
        275                 280                 285

act att ttc gtc gat ccg ccg cgt gcc ggt atc gat gag gaa act ttg 912
Thr Ile Phe Val Asp Pro Pro Arg Ala Gly Ile Asp Glu Glu Thr Leu
    290                 295                 300

aag ctg gtg tcg cag ttt gac aac atc atc tac att tcc tgc aat ccc 960
Lys Leu Val Ser Gln Phe Asp Asn Ile Ile Tyr Ile Ser Cys Asn Pro
305                 310                 315                 320

gaa acg ttg cgc gcc aat ttg gac acc ttg gcg gaa acc cat gcg gtc 1008
Glu Thr Leu Arg Ala Asn Leu Asp Thr Leu Ala Glu Thr His Ala Val
                325                 330                 335

gaa cgt gcc gcc ctg ttc gac caa ttc ccg ttt acg cac cat atc gaa 1056
Glu Arg Ala Ala Leu Phe Asp Gln Phe Pro Phe Thr His His Ile Glu
            340                 345                 350

agt ggt gta ttg ttg aaa aag aaa atc ctt tga 1089
Ser Gly Val Leu Leu Lys Lys Lys Ile Leu
        355                 360

<210> 194
<211> 362
<212> PRT
<213> Neisseria meningitidis

<400> 194
Met Asn Asp Tyr Thr Gln Gln Leu Gln Gly Lys Lys Asp Tyr Leu Lys
1               5                   10                  15

Thr Leu Phe Ala Gly Leu Asp Val Pro Glu Trp Glu Val Tyr Glu Ser
            20                  25                  30

Pro Asp Lys His Tyr Arg Met Arg Ala Glu Phe Arg Ile Trp His Glu
        35                  40                  45

Gly Gly Glu Met Phe Tyr Ala Met Phe Glu Lys Gly Gln Lys Ala Ser
    50                  55                  60

Gly Ala Ser Met Ile Arg Cys Asp Arg Phe Glu Ala Ala Ser Glu Ala
65                  70                  75                  80

Val Asn Arg Leu Met Pro Glu Leu Ile Ala Ala Ala Ala Gln Ser Pro
                85                  90                  95

Glu Leu Lys Lys Arg Trp Tyr Ala Val Glu Phe Leu Ser Thr Leu Ser
            100                 105                 110

Gly Glu Met Leu Val Thr Met Ile Tyr His Lys Arg Leu Asp Ala Glu
        115                 120                 125

Trp Met Gln Ala Ala Gln Ala Leu Gln Gln Gln Leu Asp Ile Ser Val
    130                 135                 140

Ile Gly Arg Ser Arg Gly Gln Lys Ile Val Leu Lys Gln Asp Tyr Val
145                 150                 155                 160

Thr Glu Thr Leu Lys Val Gly Asn Arg Asp Phe Arg Tyr Arg Gln Ile
                165                 170                 175

Glu Gly Ser Phe Thr Gln Pro Asn Ala Ala Val Cys Gln Lys Met Leu
            180                 185                 190

Glu Trp Ala Cys Arg Thr Ala Glu Gly Leu Gly Ser Asp Leu Leu Glu
        195                 200                 205

Leu Tyr Cys Gly Asn Gly Asn Phe Thr Leu Pro Leu Ser Arg Tyr Phe
    210                 215                 220

Arg Gln Val Leu Ala Thr Glu Ile Ser Lys Thr Ser Val Ser Ala Ala
225                 230                 235                 240

Gln Trp Asn Ile Glu Ala Asn Arg Ile Gly Asn Ile Lys Ile Ala Arg
                245                 250                 255

Leu Ser Ala Glu Glu Phe Thr Glu Ala Tyr Thr Gly Lys Arg Glu Phe
            260                 265                 270

Lys Arg Leu Lys Asp Gly Gly Ile Ala Leu Thr Asp Tyr Ala Phe Ser
        275                 280                 285

Thr Ile Phe Val Asp Pro Pro Arg Ala Gly Ile Asp Glu Glu Thr Leu
    290                 295                 300

Lys Leu Val Ser Gln Phe Asp Asn Ile Ile Tyr Ile Ser Cys Asn Pro
305                 310                 315                 320

Glu Thr Leu Arg Ala Asn Leu Asp Thr Leu Ala Glu Thr His Ala Val
                325                 330                 335

Glu Arg Ala Ala Leu Phe Asp Gln Phe Pro Phe Thr His His Ile Glu
            340                 345                 350

Ser Gly Val Leu Leu Lys Lys Lys Ile Leu
        355                 360

<210> 195
<211> 2145
<212> DNA
<213> Neisseria meningitidis

<220>
<221> CDS
<222> (1)..(2145)

<400> 195
atg aat acc cca ttg ttc cgt ctc agc ctg ctc tcg ctt acc ctg gcg 48
Met Asn Thr Pro Leu Phe Arg Leu Ser Leu Leu Ser Leu Thr Leu Ala
1               5                   10                  15

gca ggt ttt gcc cat gcg gca gaa aat aat gcc aag gtc gta ctg gat 96
Ala Gly Phe Ala His Ala Ala Glu Asn Asn Ala Lys Val Val Leu Asp
            20                  25                  30

acc gtt acc gta aaa ggc gac cgc caa ggc agc aaa atc cgt acc aac 144
Thr Val Thr Val Lys Gly Asp Arg Gln Gly Ser Lys Ile Arg Thr Asn
        35                  40                  45

atc gtt acg ctg caa caa aaa gac gaa agc acc gca acc gat atg cgc 192
Ile Val Thr Leu Gln Gln Lys Asp Glu Ser Thr Ala Thr Asp Met Arg
    50                  55                  60

gaa ctc tta aaa gaa gag ccc tcc atc gat ttc ggc ggc ggc aac ggc 240
Glu Leu Leu Lys Glu Glu Pro Ser Ile Asp Phe Gly Gly Gly Asn Gly
65                  70                  75                  80

acg tcc caa ttc ctg acg ctg cgc ggc atg ggt caa aac tct gtc gac 288
Thr Ser Gln Phe Leu Thr Leu Arg Gly Met Gly Gln Asn Ser Val Asp
                85                  90                  95

atc aag gtg gac aac gcc tat tcc gac agc caa atc ctt tac cac caa 336
Ile Lys Val Asp Asn Ala Tyr Ser Asp Ser Gln Ile Leu Tyr His Gln
            100                 105                 110

ggc aga ttt att gtc gat ccc gct ttg gtt aaa gtc gtt tcc gta caa 384
Gly Arg Phe Ile Val Asp Pro Ala Leu Val Lys Val Val Ser Val Gln
        115                 120                 125

aaa ggc gcg ggt tcc gcc tct gcc ggt atc ggc gcg acc aac ggc gcg 432
Lys Gly Ala Gly Ser Ala Ser Ala Gly Ile Gly Ala Thr Asn Gly Ala
    130                 135                 140

atc atc acc aaa acc gtc gat gcc caa gac ctg ctc aaa ggc ttg gat 480
Ile Ile Thr Lys Thr Val Asp Ala Gln Asp Leu Leu Lys Gly Leu Asp
145                 150                 155                 160

aaa aac tgg ggc gtg cgc ctc aac agc ggc ttt gcc agc aac gaa ggc 528
Lys Asn Trp Gly Val Arg Leu Asn Ser Gly Phe Ala Ser Asn Glu Gly
                165                 170                 175

gta agc tac ggc gca agc gta ttc ggg aaa gag ggc aac ttc gac ggc 576
Val Ser Tyr Gly Ala Ser Val Phe Gly Lys Glu Gly Asn Phe Asp Gly
            180                 185                 190

ttg ttc tct tac aac cgc aac aat gaa aaa gat tac gaa gca ggt aaa 624
Leu Phe Ser Tyr Asn Arg Asn Asn Glu Lys Asp Tyr Glu Ala Gly Lys
        195                 200                 205

ggc ttc cgt aat aat ttc aac ggc ggc aaa acc gta ccg tac agc gcg 672
Gly Phe Arg Asn Asn Phe Asn Gly Gly Lys Thr Val Pro Tyr Ser Ala
    210                 215                 220

ctg gac aaa cgc agc tac ctc gcc aaa atc gga aca agc ttc ggc gac 720
Leu Asp Lys Arg Ser Tyr Leu Ala Lys Ile Gly Thr Ser Phe Gly Asp
225                 230                 235                 240

ggc gac cac cgc atc gta ttg agc cat atg aaa gac cag cac cgg ggc 768
Gly Asp His Arg Ile Val Leu Ser His Met Lys Asp Gln His Arg Gly
                245                 250                 255

atc cgt acc gtc cgt gaa gaa ttt acc gtc ggc ggc gat aaa gag cga 816
Ile Arg Thr Val Arg Glu Glu Phe Thr Val Gly Gly Asp Lys Glu Arg
            260                 265                 270

ata agt atg gaa cgc caa gcc cct gct tac cgc gaa acc aca caa tcc 864
Ile Ser Met Glu Arg Gln Ala Pro Ala Tyr Arg Glu Thr Thr Gln Ser
        275                 280                 285

aac acc aat ttg gcg tac acg ggt aaa aac ctg ggc ttt gtc gaa aaa 912
Asn Thr Asn Leu Ala Tyr Thr Gly Lys Asn Leu Gly Phe Val Glu Lys
    290                 295                 300

ctg gat gcc aac gcc tat gtg ttg gaa aaa gaa cgc tat tcc gcc gat 960
Leu Asp Ala Asn Ala Tyr Val Leu Glu Lys Glu Arg Tyr Ser Ala Asp
305                 310                 315                 320

gac agc ggc acc ggt tac gca ggc aat gta aaa ggc ccc aac cat acc 1008
Asp Ser Gly Thr Gly Tyr Ala Gly Asn Val Lys Gly Pro Asn His Thr
                325                 330                 335

caa atc acc act cgg ggt atg aac ttc aac ttc gac agc cgc ctt gcc 1056
Gln Ile Thr Thr Arg Gly Met Asn Phe Asn Phe Asp Ser Arg Leu Ala
            340                 345                 350

gaa caa acc ctg ctg aaa tac ggt atc aac tac cgc cat cag gaa atc 1104
Glu Gln Thr Leu Leu Lys Tyr Gly Ile Asn Tyr Arg His Gln Glu Ile
        355                 360                 365

aaa ccg caa gcg ttt ttg aat tca caa ttt aaa att gaa gat aaa gaa 1152
Lys Pro Gln Ala Phe Leu Asn Ser Gln Phe Lys Ile Glu Asp Lys Glu
    370                 375                 380

aaa gca act gat gaa gag aaa aat aag aac cgt gaa aat gaa aaa att 1200
Lys Ala Thr Asp Glu Glu Lys Asn Lys Asn Arg Glu Asn Glu Lys Ile
385                 390                 395                 400

gcc aaa gcc tac cgt ctg acc aac ccg acc aaa acc gat acc ggc gcg 1248
Ala Lys Ala Tyr Arg Leu Thr Asn Pro Thr Lys Thr Asp Thr Gly Ala
                405                 410                 415

tat atc gaa gcc att cac gag att gac ggc ttt acc ctg acc ggc ggg 1296
Tyr Ile Glu Ala Ile His Glu Ile Asp Gly Phe Thr Leu Thr Gly Gly
            420                 425                 430

ctg cgt tac gac cgc ttc aag gtg aaa acc cac gac ggc aaa acc gtt 1344
Leu Arg Tyr Asp Arg Phe Lys Val Lys Thr His Asp Gly Lys Thr Val
        435                 440                 445

tca agc aac aac ctt aac ccg agt ttc ggc gtg att tgg cag ccg cac 1392
Ser Ser Asn Asn Leu Asn Pro Ser Phe Gly Val Ile Trp Gln Pro His
    450                 455                 460

gaa cac tgg agc ttc agc gcg agc cac aac tac gcc agc cgc agc ccg 1440
Glu His Trp Ser Phe Ser Ala Ser His Asn Tyr Ala Ser Arg Ser Pro
465                 470                 475                 480

cgc ctg tat gac gcg ctg caa acc cac ggc aaa cgc ggc atc atc tcg 1488
Arg Leu Tyr Asp Ala Leu Gln Thr His Gly Lys Arg Gly Ile Ile Ser
                485                 490                 495

att gcc gac ggc acg aaa gcc gaa cgc gcg cgc aat acc gaa atc ggc 1536
Ile Ala Asp Gly Thr Lys Ala Glu Arg Ala Arg Asn Thr Glu Ile Gly
            500                 505                 510

ttc aac tac aac gac ggc acg ttt gcc gca aac ggc agc tac ttc tgg 1584
Phe Asn Tyr Asn Asp Gly Thr Phe Ala Ala Asn Gly Ser Tyr Phe Trp
        515                 520                 525

cag acc atc aaa gac gcg ctt gcc aat ccg caa aac cgc cac gac tct 1632
Gln Thr Ile Lys Asp Ala Leu Ala Asn Pro Gln Asn Arg His Asp Ser
    530                 535                 540

gtc gcc gtc cgt gaa gcc gtc aat gcc ggt tac atc aaa aac cac ggt 1680
Val Ala Val Arg Glu Ala Val Asn Ala Gly Tyr Ile Lys Asn His Gly
545                 550                 555                 560

tac gaa ttg ggc gcg tcc tac cgc acc ggc ggc ctg act gcc aaa gtc 1728
Tyr Glu Leu Gly Ala Ser Tyr Arg Thr Gly Gly Leu Thr Ala Lys Val
                565                 570                 575

ggc gta agc cac agc aaa ccg cgc ttt tac gat acg cac aaa gac aag 1776
Gly Val Ser His Ser Lys Pro Arg Phe Tyr Asp Thr His Lys Asp Lys
            580                 585                 590

ctg ttg agc gcg aat cct gaa ttt ggc gca caa gtc ggc cgc act tgg 1824
Leu Leu Ser Ala Asn Pro Glu Phe Gly Ala Gln Val Gly Arg Thr Trp
        595                 600                 605

acg gct tcc ctt gcc tac cgc ttc caa aac ccg aat ctg gaa atc ggc 1872
Thr Ala Ser Leu Ala Tyr Arg Phe Gln Asn Pro Asn Leu Glu Ile Gly
    610                 615                 620

tgg cgc ggc cgt tat gtt caa aaa gcc gtg ggt tcg ata ttg gtg gca 1920
Trp Arg Gly Arg Tyr Val Gln Lys Ala Val Gly Ser Ile Leu Val Ala
625                 630                 635                 640

ggt caa aaa gac cgc aac ggc aaa ttg gaa aac gtt gta cgc aaa ggt 1968
Gly Gln Lys Asp Arg Asn Gly Lys Leu Glu Asn Val Val Arg Lys Gly
                645                 650                 655

ttc ggt gtg aac gat gtc ttc gcc aac tgg aaa ccg ctg ggc aaa gac 2016
Phe Gly Val Asn Asp Val Phe Ala Asn Trp Lys Pro Leu Gly Lys Asp
            660                 665                 670

acg ctc aat gtt aat ctt tcg gtt aac aac gtg ttc aac acg ttc tac 2064
Thr Leu Asn Val Asn Leu Ser Val Asn Asn Val Phe Asn Thr Phe Tyr
        675                 680                 685

tat ccg cac agc caa cga tgg acc aat acc ctg ccg ggc gtg gga cgt 2112
Tyr Pro His Ser Gln Arg Trp Thr Asn Thr Leu Pro Gly Val Gly Arg
    690                 695                 700

gat gta cgc ttg ggc gtg aac tac aag ttc taa 2145
Asp Val Arg Leu Gly Val Asn Tyr Lys Phe
705 710 715

<210> 196
<211> 714
<212> PRT
<213> Neisseria meningitidis

<400> 196
Met Asn Thr Pro Leu Phe Arg Leu Ser Leu Leu Ser Leu Thr Leu Ala
1               5                   10                  15

Ala Gly Phe Ala His Ala Ala Glu Asn Asn Ala Lys Val Val Leu Asp
            20                  25                  30

Thr Val Thr Val Lys Gly Asp Arg Gln Gly Ser Lys Ile Arg Thr Asn
        35                  40                  45

Ile Val Thr Leu Gln Gln Lys Asp Glu Ser Thr Ala Thr Asp Met Arg
50                  55                  60

Glu Leu Leu Lys Glu Glu Pro Ser Ile Asp Phe Gly Gly Gly Asn Gly
65                  70                  75                  80

Thr Ser Gln Phe Leu Thr Leu Arg Gly Met Gly Gln Asn Ser Val Asp
                85                  90                  95

Ile Lys Val Asp Asn Ala Tyr Ser Asp Ser Gln Ile Leu Tyr His Gln
            100                 105                 110

Gly Arg Phe Ile Val Asp Pro Ala Leu Val Lys Val Val Ser Val Gln
        115                 120                 125

Lys Gly Ala Gly Ser Ala Ser Ala Gly Ile Gly Ala Thr Asn Gly Ala
    130                 135                 140

Ile Ile Thr Lys Thr Val Asp Ala Gln Asp Leu Leu Lys Gly Leu Asp
145                 150                 155                 160

Lys Asn Trp Gly Val Arg Leu Asn Ser Gly Phe Ala Ser Asn Glu Gly
                165                 170                 175

Val Ser Tyr Gly Ala Ser Val Phe Gly Lys Glu Gly Asn Phe Asp Gly
            180                 185                 190

Leu Phe Ser Tyr Asn Arg Asn Asn Glu Lys Asp Tyr Glu Ala Gly Lys
        195                 200                 205

Gly Phe Arg Asn Asn Phe Asn Gly Gly Lys Thr Val Pro Tyr Ser Ala
    210                 215                 220

Leu Asp Lys Arg Ser Tyr Leu Ala Lys Ile Gly Thr Ser Phe Gly Asp
225                 230                 235                 240

Gly Asp His Arg Ile Val Leu Ser His Met Lys Asp Gln His Arg Gly
                245                 250                 255

Ile Arg Thr Val Arg Glu Glu Phe Thr Val Gly Gly Asp Lys Glu Arg
            260                 265                 270

Ile Ser Met Glu Arg Gln Ala Pro Ala Tyr Arg Glu Thr Thr Gln Ser
        275                 280                 285

Asn Thr Asn Leu Ala Tyr Thr Gly Lys Asn Leu Gly Phe Val Glu Lys
    290                 295                 300

Leu Asp Ala Asn Ala Tyr Val Leu Glu Lys Glu Arg Tyr Ser Ala Asp
305                 310                 315                 320

Asp Ser Gly Thr Gly Tyr Ala Gly Asn Val Lys Gly Pro Asn His Thr
                325                 330                 335

Gln Ile Thr Thr Arg Gly Met Asn Phe Asn Phe Asp Ser Arg Leu Ala
            340                 345                 350

Glu Gln Thr Leu Leu Lys Tyr Gly Ile Asn Tyr Arg His Gln Glu Ile
        355                 360                 365

Lys Pro Gln Ala Phe Leu Asn Ser Gln Phe Lys Ile Glu Asp Lys Glu
    370                 375                 380

Lys Ala Thr Asp Glu Glu Lys Asn Lys Asn Arg Glu Asn Glu Lys Ile
385                 390                 395                 400

Ala Lys Ala Tyr Arg Leu Thr Asn Pro Thr Lys Thr Asp Thr Gly Ala
                405                 410                 415

Tyr Ile Glu Ala Ile His Glu Ile Asp Gly Phe Thr Leu Thr Gly Gly
            420                 425                 430

Leu Arg Tyr Asp Arg Phe Lys Val Lys Thr His Asp Gly Lys Thr Val
        435                 440                 445

Ser Ser Asn Asn Leu Asn Pro Ser Phe Gly Val Ile Trp Gln Pro His
    450                 455                 460

Glu His Trp Ser Phe Ser Ala Ser His Asn Tyr Ala Ser Arg Ser Pro
465                 470                 475                 480

Arg Leu Tyr Asp Ala Leu Gln Thr His Gly Lys Arg Gly Ile Ile Ser
                485                 490                 495

Ile Ala Asp Gly Thr Lys Ala Glu Arg Ala Arg Asn Thr Glu Ile Gly
            500                 505                 510

Phe Asn Tyr Asn Asp Gly Thr Phe Ala Ala Asn Gly Ser Tyr Phe Trp
        515                 520                 525

Gln Thr Ile Lys Asp Ala Leu Ala Asn Pro Gln Asn Arg His Asp Ser
    530                 535                 540

Val Ala Val Arg Glu Ala Val Asn Ala Gly Tyr Ile Lys Asn His Gly
545                 550                 555                 560

Tyr Glu Leu Gly Ala Ser Tyr Arg Thr Gly Gly Leu Thr Ala Lys Val
                565                 570                 575

Gly Val Ser His Ser Lys Pro Arg Phe Tyr Asp Thr His Lys Asp Lys
            580                 585                 590

Leu Leu Ser Ala Asn Pro Glu Phe Gly Ala Gln Val Gly Arg Thr Trp
        595                 600                 605

Thr Ala Ser Leu Ala Tyr Arg Phe Gln Asn Pro Asn Leu Glu Ile Gly
    610                 615                 620

Trp Arg Gly Arg Tyr Val Gln Lys Ala Val Gly Ser Ile Leu Val Ala
625                 630                 635                 640

Gly Gln Lys Asp Arg Asn Gly Lys Leu Glu Asn Val Val Arg Lys Gly
                645                 650                 655

Phe Gly Val Asn Asp Val Phe Ala Asn Trp Lys Pro Leu Gly Lys Asp
            660                 665                 670

Thr Leu Asn Val Asn Leu Ser Val Asn Asn Val Phe Asn Thr Phe Tyr
        675                 680                 685

Tyr Pro His Ser Gln Arg Trp Thr Asn Thr Leu Pro Gly Val Gly Arg
    690                 695                 700

Asp Val Arg Leu Gly Val Asn Tyr Lys Phe
705                 710

<210> 197
<211> 2625
<212> DNA
<213> Neisseria meningitidis

<220>
<221> CDS
<222> (1)..(2625)

<400> 197
atg aaa acc tcc gaa ctg cgc caa aaa ttc cta aaa ttt ttt gaa acc      48
Met Lys Thr Ser Glu Leu Arg Gln Lys Phe Leu Lys Phe Phe Glu Thr
  1               5                  10                  15

aaa ggc cac acc gtc gtc cgc tct tcc agc ctc gtg ccg cac gac gac      96

Lys Gly His Thr Val Val Arg Ser Ser Ser Leu Val Pro His Asp Asp
            20                  25                  30

ccg acc ctg ctg ttt acc aac gcg ggc atg aac cag ttt aaa gac gta 144
Pro Thr Leu Leu Phe Thr Asn Ala Gly Met Asn Gln Phe Lys Asp Val
        35                  40                  45

ttc tta ggt ttc gac aaa cgc ccg tac agc cgc gcc acc acc gcg caa 192
Phe Leu Gly Phe Asp Lys Arg Pro Tyr Ser Arg Ala Thr Thr Ala Gln
    50                  55                  60

aaa tgc gta cgc gca ggc ggc aaa cac aac gac ttg gaa aac gtc ggc 240
Lys Cys Val Arg Ala Gly Gly Lys His Asn Asp Leu Glu Asn Val Gly
65                  70                  75                  80

tac acc gcc cgc cac cac acc ttc ttt gaa atg atg ggc aac ttc tcc 288
Tyr Thr Ala Arg His His Thr Phe Phe Glu Met Met Gly Asn Phe Ser
                85                  90                  95

ttc ggc gac tac ttc aaa cgc gac gcc atc cac ttc gct tgg gaa ttt 336
Phe Gly Asp Tyr Phe Lys Arg Asp Ala Ile His Phe Ala Trp Glu Phe
            100                 105                 110

ctg act tcc ccc gaa tgg ctc aac atc cct aaa gac aaa ctg ttg gcg 384
Leu Thr Ser Pro Glu Trp Leu Asn Ile Pro Lys Asp Lys Leu Leu Ala
        115                 120                 125

acc gtt tac gcg gaa gac gac gaa gcc tac aac atc tgg ttg aac gaa 432
Thr Val Tyr Ala Glu Asp Asp Glu Ala Tyr Asn Ile Trp Leu Asn Glu
    130                 135                 140

atc ggt atg ccg tcc gag cgc atc gtc cgc atc ggc gac aac aaa ggc 480
Ile Gly Met Pro Ser Glu Arg Ile Val Arg Ile Gly Asp Asn Lys Gly
145                 150                 155                 160

gcg aaa tac gca tcc gac aac ttc tgg caa atg ggc gac acc ggc cct 528
Ala Lys Tyr Ala Ser Asp Asn Phe Trp Gln Met Gly Asp Thr Gly Pro
                165                 170                 175

tgc ggc ccc tgc tcc gaa att ttc tac gac cac ggc gaa gaa atc tgg 576
Cys Gly Pro Cys Ser Glu Ile Phe Tyr Asp His Gly Glu Glu Ile Trp
            180                 185                 190

ggc ggc att ccc ggc agt ccc gaa gaa gac ggc gac cgc tgg atc gaa 624
Gly Gly Ile Pro Gly Ser Pro Glu Glu Asp Gly Asp Arg Trp Ile Glu
        195                 200                 205

att tgg aac tgc gta ttt atg cag ttc aac cgc gac gaa caa ggc aat 672

Ile Trp Asn Cys Val Phe Met Gln Phe Asn Arg Asp Glu Gln Gly Asn
    210                 215                 220

atg aac ccg ctt ccc aaa cct tcc gtc gat acc ggt atg ggc ttg gaa    720
Met Asn Pro Leu Pro Lys Pro Ser Val Asp Thr Gly Met Gly Leu Glu
225                 230                 235                 240

cgc ata gcc gcc gtc atg cag cat gtt cac agc aac tac gaa atc gac    768
Arg Ile Ala Ala Val Met Gln His Val His Ser Asn Tyr Glu Ile Asp
                245                 250                 255

ttg ttc caa gac ctg ctc aaa gcc gtt gcc cgc gaa acc ggc gcg ccg    816
Leu Phe Gln Asp Leu Leu Lys Ala Val Ala Arg Glu Thr Gly Ala Pro
            260                 265                 270

ttc aga atg gaa gaa ccc agc ctg aaa gtc atc gcc gac cac atc cgc    864
Phe Arg Met Glu Glu Pro Ser Leu Lys Val Ile Ala Asp His Ile Arg
        275                 280                 285

tcc tgc tcg ttc ctg att gca gac ggc gtc ttg cct tcc aac gaa ggc    912
Ser Cys Ser Phe Leu Ile Ala Asp Gly Val Leu Pro Ser Asn Glu Gly
    290                 295                 300

cgc ggc tac gta ttg cgc cgc att atc cgc cgc gcc gtg cgc cac ggt    960
Arg Gly Tyr Val Leu Arg Arg Ile Ile Arg Arg Ala Val Arg His Gly
305                 310                 315                 320

tac aaa ctg ggt caa agc aaa ccg ttc ttc cac aaa ctc gtt gcc gat    1008
Tyr Lys Leu Gly Gln Ser Lys Pro Phe Phe His Lys Leu Val Ala Asp
                325                 330                 335

ttg gtc aaa gag atg ggc ggt gcc tac cct gaa ttg aaa gaa aaa caa    1056
Leu Val Lys Glu Met Gly Gly Ala Tyr Pro Glu Leu Lys Glu Lys Gln
            340                 345                 350

gcc caa atc gaa gaa gca ttg aaa aac gaa gaa agc cgt ttt gcc caa    1104
Ala Gln Ile Glu Glu Ala Leu Lys Asn Glu Glu Ser Arg Phe Ala Gln
        355                 360                 365

acg ctg gaa acc ggt atg gct ttg ttg gaa aac gcg ctg gtc aaa ggc    1152
Thr Leu Glu Thr Gly Met Ala Leu Leu Glu Asn Ala Leu Val Lys Gly
    370                 375                 380

ggc aaa aca ctc ggc ggc gaa atc atc ttc aaa ctc tac gat acc tac    1200
Gly Lys Thr Leu Gly Gly Glu Ile Ile Phe Lys Leu Tyr Asp Thr Tyr
385                 390                 395                 400

ggt ttc cca tac gac ttg act gcc gac atc tgc cgc gaa cgc aat atc    1248

Gly Phe Pro Tyr Asp Leu Thr Ala Asp Ile Cys Arg Glu Arg Asn Ile
                405                 410                 415

gaa ccg gac gaa gca ggc ttc gag cgc gaa atg gaa gcc caa cgc gca 1296
Glu Pro Asp Glu Ala Gly Phe Glu Arg Glu Met Glu Ala Gln Arg Ala
            420                 425                 430

cgc gca cgc gcc gcc caa agc ttc aaa gcc aac gcc caa ctg cct tat 1344
Arg Ala Arg Ala Ala Gln Ser Phe Lys Ala Asn Ala Gln Leu Pro Tyr
        435                 440                 445

gac ggt caa gac acc gag ttt aaa ggt tat agc gaa cgc caa acc gaa 1392
Asp Gly Gln Asp Thr Glu Phe Lys Gly Tyr Ser Glu Arg Gln Thr Glu
    450                 455                 460

tcc aaa gtc ctc gcc ctc tac aaa gac ggc gag caa gtc aac gaa ttg 1440
Ser Lys Val Leu Ala Leu Tyr Lys Asp Gly Glu Gln Val Asn Glu Leu
465                 470                 475                 480

aac gaa ggc gac agc ggc gca gtc gtc atc gac ttt acc ccg ttc tat 1488
Asn Glu Gly Asp Ser Gly Ala Val Val Ile Asp Phe Thr Pro Phe Tyr
                485                 490                 495

gca gaa tcc ggc ggc caa gtc ggc gat gtc ggc tat atc ttc tca ggc 1536
Ala Glu Ser Gly Gly Gln Val Gly Asp Val Gly Tyr Ile Phe Ser Gly
            500                 505                 510

gaa aac cgc ttt gaa gta cgc gat acc caa aaa atc aaa gcg gcc gta 1584
Glu Asn Arg Phe Glu Val Arg Asp Thr Gln Lys Ile Lys Ala Ala Val
        515                 520                 525

ttc ggt caa ttc ggc gta caa act tca ggc cgt ctg aaa gtc ggc gac 1632
Phe Gly Gln Phe Gly Val Gln Thr Ser Gly Arg Leu Lys Val Gly Asp
    530                 535                 540

agc gtt acc gcc aaa gtg gac gac gaa atc cgc aat gcc aat atg cgc 1680
Ser Val Thr Ala Lys Val Asp Asp Glu Ile Arg Asn Ala Asn Met Arg
545                 550                 555                 560

aac cac agc gca acc cac ttg atg cac aaa gcc ctg cgc gat gta ttg 1728
Asn His Ser Ala Thr His Leu Met His Lys Ala Leu Arg Asp Val Leu
                565                 570                 575

ggc aga cac gtc gaa caa aaa ggc tct ttg gtt acc gcc gaa tcc acc 1776
Gly Arg His Val Glu Gln Lys Gly Ser Leu Val Thr Ala Glu Ser Thr
            580                 585                 590

cgt ttc gac att tcc cat ccc caa gcg gta act gcc gaa gaa att gcc 1824

Arg Phe Asp Ile Ser His Pro Gln Ala Val Thr Ala Glu Glu Ile Ala
        595                 600                 605

gaa gta gaa cgc cgc gtc aac gaa gcc att ttg gcg aac gtt gcc gtc 1872
Glu Val Glu Arg Arg Val Asn Glu Ala Ile Leu Ala Asn Val Ala Val
    610                 615                 620

aat gca gcc att atg agc atg gaa gac gcg caa aaa acc ggc gcg atg 1920
Asn Ala Ala Ile Met Ser Met Glu Asp Ala Gln Lys Thr Gly Ala Met
625                 630                 635                 640

atg ctc ttc ggc gaa aaa tac ggc gaa gaa gtg cgc gta ctg caa atg 1968
Met Leu Phe Gly Glu Lys Tyr Gly Glu Glu Val Arg Val Leu Gln Met
                645                 650                 655

ggc ggt ttc tct acc gaa ttg tgc ggc ggc aca cac gtt tca cgc acc 2016
Gly Gly Phe Ser Thr Glu Leu Cys Gly Gly Thr His Val Ser Arg Thr
            660                 665                 670

ggc gac atc ggc ctc ttc aaa atc atc agc gaa ggc ggt att gcc gca 2064
Gly Asp Ile Gly Leu Phe Lys Ile Ile Ser Glu Gly Gly Ile Ala Ala
        675                 680                 685

ggc gtg cgc cgt atc gaa gcc atc acc ggc ctg aac gca ctc aaa tgg 2112
Gly Val Arg Arg Ile Glu Ala Ile Thr Gly Leu Asn Ala Leu Lys Trp
    690                 695                 700

gcg caa gag caa gag cgt ttg gtg aaa gac att att gcc gaa acc aaa 2160
Ala Gln Glu Gln Glu Arg Leu Val Lys Asp Ile Ile Ala Glu Thr Lys
705                 710                 715                 720

gcc caa acc gaa aaa gac gta ctg gca aaa atc caa gca ggc gcg gca 2208
Ala Gln Thr Glu Lys Asp Val Leu Ala Lys Ile Gln Ala Gly Ala Ala
                725                 730                 735

cac gcc aaa gca ttg gaa aaa gaa ttg gca cgc gcc aaa gcc gaa ctc 2256
His Ala Lys Ala Leu Glu Lys Glu Leu Ala Arg Ala Lys Ala Glu Leu
            740                 745                 750

gcc gtc cac gca ggc gcc aaa ctc ttg gac gat gca aaa gac ttg ggc 2304
Ala Val His Ala Gly Ala Lys Leu Leu Asp Asp Ala Lys Asp Leu Gly
        755                 760                 765

gca gcc aaa ctc gtt gcc gcc caa atc gaa gcc gac gca gcc gcc ctg 2352
Ala Ala Lys Leu Val Ala Ala Gln Ile Glu Ala Asp Ala Ala Ala Leu
    770                 775                 780

cgc gaa atc gtt acc gat tta acc ggt aaa tcc gac aac gcc gtg att 2400

Arg Glu Ile Val Thr Asp Leu Thr Gly Lys Ser Asp Asn Ala Val Ile
785                 790                 795                 800

ctt tta gcg gca gta aac gac ggc aaa gtc tcc ctg tgc gcc ggc gta    2448
Leu Leu Ala Ala Val Asn Asp Gly Lys Val Ser Leu Cys Ala Gly Val
                805                 810                 815

tcc aaa ccg ttg acc ggc aaa gtg aaa gca ggc gat ctg gtt aaa ttt    2496
Ser Lys Pro Leu Thr Gly Lys Val Lys Ala Gly Asp Leu Val Lys Phe
            820                 825                 830

gca gcc gaa caa gtc ggc ggc aaa ggc ggc ggc aga cca gat ttg gcg    2544
Ala Ala Glu Gln Val Gly Gly Lys Gly Gly Gly Arg Pro Asp Leu Ala
        835                 840                 845

caa gcc ggc ggc acg gat gcc gac aaa ttg ccc gcc gtg ttg gat agc    2592
Gln Ala Gly Gly Thr Asp Ala Asp Lys Leu Pro Ala Val Leu Asp Ser
    850                 855                 860

gtg aaa gac tgg gtc ggc gcg aag ctg gtt tga                        2625
Val Lys Asp Trp Val Gly Ala Lys Leu Val
865                 870                 875

<210> 198
<211> 874
<212> PRT
<213> Neisseria meningitidis

<400> 198
Met Lys Thr Ser Glu Leu Arg Gln Lys Phe Leu Lys Phe Phe Glu Thr
  1               5                  10                  15

Lys Gly His Thr Val Val Arg Ser Ser Ser Leu Val Pro His Asp Asp
             20                  25                  30

Pro Thr Leu Leu Phe Thr Asn Ala Gly Met Asn Gln Phe Lys Asp Val
         35                  40                  45

Phe Leu Gly Phe Asp Lys Arg Pro Tyr Ser Arg Ala Thr Thr Ala Gln
     50                  55                  60

Lys Cys Val Arg Ala Gly Gly Lys His Asn Asp Leu Glu Asn Val Gly
 65                  70                  75                  80

Tyr Thr Ala Arg His His Thr Phe Phe Glu Met Met Gly Asn Phe Ser
                 85                  90                  95

Phe Gly Asp Tyr Phe Lys Arg Asp Ala Ile His Phe Ala Trp Glu Phe
            100                 105                 110

Leu Thr Ser Pro Glu Trp Leu Asn Ile Pro Lys Asp Lys Leu Leu Ala
        115                 120                 125

Thr Val Tyr Ala Glu Asp Asp Glu Ala Tyr Asn Ile Trp Leu Asn Glu
    130                 135                 140

Ile Gly Met Pro Ser Glu Arg Ile Val Arg Ile Gly Asp Asn Lys Gly
145                 150                 155                 160

Ala Lys Tyr Ala Ser Asp Asn Phe Trp Gln Met Gly Asp Thr Gly Pro
                165                 170                 175

Cys Gly Pro Cys Ser Glu Ile Phe Tyr Asp His Gly Glu Glu Ile Trp
            180                 185                 190

Gly Gly Ile Pro Gly Ser Pro Glu Glu Asp Gly Asp Arg Trp Ile Glu
        195                 200                 205

Ile Trp Asn Cys Val Phe Met Gln Phe Asn Arg Asp Glu Gln Gly Asn
    210                 215                 220

Met Asn Pro Leu Pro Lys Pro Ser Val Asp Thr Gly Met Gly Leu Glu
225                 230                 235                 240

Arg Ile Ala Ala Val Met Gln His Val His Ser Asn Tyr Glu Ile Asp
                245                 250                 255

Leu Phe Gln Asp Leu Leu Lys Ala Val Ala Arg Glu Thr Gly Ala Pro
            260                 265                 270

Phe Arg Met Glu Glu Pro Ser Leu Lys Val Ile Ala Asp His Ile Arg
        275                 280                 285

Ser Cys Ser Phe Leu Ile Ala Asp Gly Val Leu Pro Ser Asn Glu Gly
    290                 295                 300

Arg Gly Tyr Val Leu Arg Arg Ile Ile Arg Arg Ala Val Arg His Gly
305                 310                 315                 320

Tyr Lys Leu Gly Gln Ser Lys Pro Phe Phe His Lys Leu Val Ala Asp
                325                 330                 335

Leu Val Lys Glu Met Gly Gly Ala Tyr Pro Glu Leu Lys Glu Lys Gln
            340                 345                 350

Ala Gln Ile Glu Glu Ala Leu Lys Asn Glu Glu Ser Arg Phe Ala Gln
        355                 360                 365

Thr Leu Glu Thr Gly Met Ala Leu Leu Glu Asn Ala Leu Val Lys Gly
    370                 375                 380

Gly Lys Thr Leu Gly Gly Glu Ile Ile Phe Lys Leu Tyr Asp Thr Tyr
385                 390                 395                 400

Gly Phe Pro Tyr Asp Leu Thr Ala Asp Ile Cys Arg Glu Arg Asn Ile
                405                 410                 415

Glu Pro Asp Glu Ala Gly Phe Glu Arg Glu Met Glu Ala Gln Arg Ala
            420                 425                 430

Arg Ala Arg Ala Ala Gln Ser Phe Lys Ala Asn Ala Gln Leu Pro Tyr
        435                 440                 445

Asp Gly Gln Asp Thr Glu Phe Lys Gly Tyr Ser Glu Arg Gln Thr Glu
    450                 455                 460

Ser Lys Val Leu Ala Leu Tyr Lys Asp Gly Glu Gln Val Asn Glu Leu
465                 470                 475                 480

Asn Glu Gly Asp Ser Gly Ala Val Val Ile Asp Phe Thr Pro Phe Tyr
                485                 490                 495

Ala Glu Ser Gly Gly Gln Val Gly Asp Val Gly Tyr Ile Phe Ser Gly
            500                 505                 510

Glu Asn Arg Phe Glu Val Arg Asp Thr Gln Lys Ile Lys Ala Ala Val
        515                 520                 525

Phe Gly Gln Phe Gly Val Gln Thr Ser Gly Arg Leu Lys Val Gly Asp
    530                 535                 540

Ser Val Thr Ala Lys Val Asp Asp Glu Ile Arg Asn Ala Asn Met Arg
545                 550                 555                 560

Asn His Ser Ala Thr His Leu Met His Lys Ala Leu Arg Asp Val Leu
                565                 570                 575

Gly Arg His Val Glu Gln Lys Gly Ser Leu Val Thr Ala Glu Ser Thr
            580                 585                 590

Arg Phe Asp Ile Ser His Pro Gln Ala Val Thr Ala Glu Glu Ile Ala
        595                 600                 605

Glu Val Glu Arg Arg Val Asn Glu Ala Ile Leu Ala Asn Val Ala Val
610                 615                 620

Asn Ala Ala Ile Met Ser Met Glu Asp Ala Gln Lys Thr Gly Ala Met
625                 630                 635                 640

Met Leu Phe Gly Glu Lys Tyr Gly Glu Glu Val Arg Val Leu Gln Met
                645                 650                 655

Gly Gly Phe Ser Thr Glu Leu Cys Gly Gly Thr His Val Ser Arg Thr
            660                 665                 670

Gly Asp Ile Gly Leu Phe Lys Ile Ile Ser Glu Gly Gly Ile Ala Ala
        675                 680                 685

Gly Val Arg Arg Ile Glu Ala Ile Thr Gly Leu Asn Ala Leu Lys Trp
    690                 695                 700

Ala Gln Glu Gln Glu Arg Leu Val Lys Asp Ile Ile Ala Glu Thr Lys
705                 710                 715                 720

Ala Gln Thr Glu Lys Asp Val Leu Ala Lys Ile Gln Ala Gly Ala Ala
                725                 730                 735

His Ala Lys Ala Leu Glu Lys Glu Leu Ala Arg Ala Lys Ala Glu Leu
            740                 745                 750

Ala Val His Ala Gly Ala Lys Leu Leu Asp Asp Ala Lys Asp Leu Gly
        755                 760                 765

Ala Ala Lys Leu Val Ala Ala Gln Ile Glu Ala Asp Ala Ala Ala Leu
    770                 775                 780

Arg Glu Ile Val Thr Asp Leu Thr Gly Lys Ser Asp Asn Ala Val Ile
785                 790                 795                 800

Leu Leu Ala Ala Val Asn Asp Gly Lys Val Ser Leu Cys Ala Gly Val
                805                 810                 815

Ser Lys Pro Leu Thr Gly Lys Val Lys Ala Gly Asp Leu Val Lys Phe
            820                 825                 830

Ala Ala Glu Gln Val Gly Gly Lys Gly Gly Gly Arg Pro Asp Leu Ala
        835                 840                 845

Gln Ala Gly Gly Thr Asp Ala Asp Lys Leu Pro Ala Val Leu Asp Ser
    850                 855                 860

Val Lys Asp Trp Val Gly Ala Lys Leu Val
865                 870

<210> 199
<211> 207
<212> DNA
<213> Neisseria meningitidis

<220>
<221> CDS
<222> (1)..(207)

<400> 199
gtg caa aga ggc cgt ctg aaa ggt ctc gtt tgc cgt agg ttg ggt cgc     48
Val Gln Arg Gly Arg Leu Lys Gly Leu Val Cys Arg Arg Leu Gly Arg
1               5                   10                  15

gac cca aca aat ttt gtg aag tat aaa aat gtt ggt cat gac cca acc     96
Asp Pro Thr Asn Phe Val Lys Tyr Lys Asn Val Gly His Asp Pro Thr
            20                  25                  30

tac ctg cct ttt tgt aca aag agg cta tct gaa agg cct tgt ttg ccg     144
Tyr Leu Pro Phe Cys Thr Lys Arg Leu Ser Glu Arg Pro Cys Leu Pro
        35                  40                  45

tat ggt ggg tcg cga ccc agc aga ttt tta tta ggg tat gac cca agc     192
Tyr Gly Gly Ser Arg Pro Ser Arg Phe Leu Leu Gly Tyr Asp Pro Ser
    50                  55                  60

tac ttg cta cga taa                                                 207
Tyr Leu Leu Arg
65

<210> 200
<211> 68
<212> PRT
<213> Neisseria meningitidis

<400> 200
Val Gln Arg Gly Arg Leu Lys Gly Leu Val Cys Arg Arg Leu Gly Arg
1               5                   10                  15

Asp Pro Thr Asn Phe Val Lys Tyr Lys Asn Val Gly His Asp Pro Thr
            20                  25                  30

Tyr Leu Pro Phe Cys Thr Lys Arg Leu Ser Glu Arg Pro Cys Leu Pro
        35                  40                  45

Tyr Gly Gly Ser Arg Pro Ser Arg Phe Leu Leu Gly Tyr Asp Pro Ser
    50                  55                  60

Tyr Leu Leu Arg
65

<210> 201
<211> 2277
<212> DNA
<213> Neisseria meningitidis

<220>
<221> CDS
<222> (1)..(2277)

<400> 201
atg gca caa act aca ctc aaa ccc att gtt tta tca att ctt tta atc      48
Met Ala Gln Thr Thr Leu Lys Pro Ile Val Leu Ser Ile Leu Leu Ile
1               5                   10                  15

aac aca ccc ctc ctc gcc caa gcg cat gaa act gag caa tcg gtg gat      96
Asn Thr Pro Leu Leu Ala Gln Ala His Glu Thr Glu Gln Ser Val Asp
            20                  25                  30

ttg gaa acg gtc agc gtc gtc ggc aaa agc cgt ccg cgc gcc acg tcg     144
Leu Glu Thr Val Ser Val Val Gly Lys Ser Arg Pro Arg Ala Thr Ser
        35                  40                  45

ggg ctg ttg cac act tcg acc gcc tcc gac aaa atc atc tcc ggc gat     192
Gly Leu Leu His Thr Ser Thr Ala Ser Asp Lys Ile Ile Ser Gly Asp
    50                  55                  60

acc ttg cgc caa aaa gcc gtc aac ttg ggc gac gct tta gac ggc gta     240
Thr Leu Arg Gln Lys Ala Val Asn Leu Gly Asp Ala Leu Asp Gly Val
65                  70                  75                  80

ccg ggc atc cac gct tcg caa tac ggc ggc ggc gcg tct gct ccc gtc     288
Pro Gly Ile His Ala Ser Gln Tyr Gly Gly Gly Ala Ser Ala Pro Val
                85                  90                  95

att cgc ggt caa aca ggc agg cgg att aaa gtg ttg aac cat cac ggc     336
Ile Arg Gly Gln Thr Gly Arg Arg Ile Lys Val Leu Asn His His Gly
            100                 105                 110

gaa aca ggc gat atg gcg gat ttt tcg ccc gat cac gcc att atg gta    384
Glu Thr Gly Asp Met Ala Asp Phe Ser Pro Asp His Ala Ile Met Val
        115                 120                 125

gat acc gcc ttg tcg caa cag gtc gaa atc ctg cgc ggg ccg gtt acg    432
Asp Thr Ala Leu Ser Gln Gln Val Glu Ile Leu Arg Gly Pro Val Thr
    130                 135                 140

ctc ttg tac agc tcg ggc aat gtg gcg ggg ctg gtc gat gtt gcc gat    480
Leu Leu Tyr Ser Ser Gly Asn Val Ala Gly Leu Val Asp Val Ala Asp
145                 150                 155                 160

ggc aaa atc ccc gaa aaa atg cct gaa aac ggc gta tcg ggc gaa ctc    528
Gly Lys Ile Pro Glu Lys Met Pro Glu Asn Gly Val Ser Gly Glu Leu
                165                 170                 175

gga ttg cgt ttg agc agc ggc aat ctg gaa aaa ctc acg tcc ggc ggc    576
Gly Leu Arg Leu Ser Ser Gly Asn Leu Glu Lys Leu Thr Ser Gly Gly
            180                 185                 190

atc aat atc ggt ttg ggc aaa aac ttt gta ttg cac acg gaa ggg ctg    624
Ile Asn Ile Gly Leu Gly Lys Asn Phe Val Leu His Thr Glu Gly Leu
        195                 200                 205

tac cgc aaa tcg ggg gat tac gcc gta ccg cgt tac cgc aat ctg aaa    672
Tyr Arg Lys Ser Gly Asp Tyr Ala Val Pro Arg Tyr Arg Asn Leu Lys
    210                 215                 220

cgc ctg ccc gac agc cac gcc gat tcg caa acg ggc agc atc ggg ctg    720
Arg Leu Pro Asp Ser His Ala Asp Ser Gln Thr Gly Ser Ile Gly Leu
225                 230                 235                 240

tct tgg gtt ggc gaa aaa ggt ttt atc ggc gta gcg tac agc gac cgt    768
Ser Trp Val Gly Glu Lys Gly Phe Ile Gly Val Ala Tyr Ser Asp Arg
                245                 250                 255

cgc gac caa tat ggt ctg cct gcc cac agc cac gaa tac gat gat tgc    816
Arg Asp Gln Tyr Gly Leu Pro Ala His Ser His Glu Tyr Asp Asp Cys
            260                 265                 270

cac gcc gac atc atc tgg caa aag agc ttg att aac aaa cgc tat tta    864
His Ala Asp Ile Ile Trp Gln Lys Ser Leu Ile Asn Lys Arg Tyr Leu
        275                 280                 285

cag ctt tat ccg cac ctg ttg acc gaa gaa gac atc gat tac gac aat    912
Gln Leu Tyr Pro His Leu Leu Thr Glu Glu Asp Ile Asp Tyr Asp Asn
    290                 295                 300

ccg ggc ttg agc tgc ggc ttc cac gac gac gat aat gca cac gca cac     960
Pro Gly Leu Ser Cys Gly Phe His Asp Asp Asp Asn Ala His Ala His
305                 310                 315                 320

acc cac agc ggc aga ccg tgg ata gac ctg cgc aac aaa cgc tac gaa     1008
Thr His Ser Gly Arg Pro Trp Ile Asp Leu Arg Asn Lys Arg Tyr Glu
                325                 330                 335

ctc cgt gcc gaa tgg aag caa ccg ttc ccc ggt ttt gaa gcc ctg cgc     1056
Leu Arg Ala Glu Trp Lys Gln Pro Phe Pro Gly Phe Glu Ala Leu Arg
            340                 345                 350

gta cac ctg aac cgc aac gac tac cgc cac gac gaa aaa gca ggc gat     1104
Val His Leu Asn Arg Asn Asp Tyr Arg His Asp Glu Lys Ala Gly Asp
        355                 360                 365

gca gtc gaa aac ttt ttt aac aac caa acg caa aac gcc cgc atc gag     1152
Ala Val Glu Asn Phe Phe Asn Asn Gln Thr Gln Asn Ala Arg Ile Glu
    370                 375                 380

ttg cgc cac caa ccc ata ggt cgt ctg aaa ggc agc tgg ggc gtg caa     1200
Leu Arg His Gln Pro Ile Gly Arg Leu Lys Gly Ser Trp Gly Val Gln
385                 390                 395                 400

tat tta caa caa aaa tcc agt gct tta tct gcc ata tcc gaa gcg gtt     1248
Tyr Leu Gln Gln Lys Ser Ser Ala Leu Ser Ala Ile Ser Glu Ala Val
                405                 410                 415

aaa caa ccg atg ctg ctt gac aac aaa gtg caa cat tac agc ttt ttc     1296
Lys Gln Pro Met Leu Leu Asp Asn Lys Val Gln His Tyr Ser Phe Phe
            420                 425                 430

ggt gta gaa cag gca aac tgg gac aac ttc acg ctt gaa gga ggc gta     1344
Gly Val Glu Gln Ala Asn Trp Asp Asn Phe Thr Leu Glu Gly Gly Val
        435                 440                 445

cgc gtg gaa aaa caa aaa gcc tcc att cag tac gac aaa gca ttg att     1392
Arg Val Glu Lys Gln Lys Ala Ser Ile Gln Tyr Asp Lys Ala Leu Ile
    450                 455                 460

gat cgg gaa aac tac tac aac cac ccc ctg ccc gac ctc ggc gcg cac     1440
Asp Arg Glu Asn Tyr Tyr Asn His Pro Leu Pro Asp Leu Gly Ala His
465                 470                 475                 480

cgc caa acc gcc cgc tca ttc gca ctt tcg ggc aac tgg tat ttc acg     1488
Arg Gln Thr Ala Arg Ser Phe Ala Leu Ser Gly Asn Trp Tyr Phe Thr
                485                 490                 495

cca caa cac aaa ctc agc ctg acc gcc tcc cat cag gaa cgc ctg ccg    1536
Pro Gln His Lys Leu Ser Leu Thr Ala Ser His Gln Glu Arg Leu Pro
            500                 505                 510

tca acg caa gag ctg tac gca cac ggc aaa cac gtc gcc acc aac acc    1584
Ser Thr Gln Glu Leu Tyr Ala His Gly Lys His Val Ala Thr Asn Thr
        515                 520                 525

ttt gaa gtc ggc aac aaa cac ctc aac aaa gag cgt tcc aac aat atc    1632
Phe Glu Val Gly Asn Lys His Leu Asn Lys Glu Arg Ser Asn Asn Ile
    530                 535                 540

gaa ctc gcg ctg ggc tac gaa ggc gac cgc tgg caa tac aat ctg gca    1680
Glu Leu Ala Leu Gly Tyr Glu Gly Asp Arg Trp Gln Tyr Asn Leu Ala
545                 550                 555                 560

ctc tac cgc aac cgc ttc ggt aac tac att tac gcc caa acc tta aac    1728
Leu Tyr Arg Asn Arg Phe Gly Asn Tyr Ile Tyr Ala Gln Thr Leu Asn
                565                 570                 575

gac gga cgc ggc ccc aaa tcc atc gaa gac gac agc gaa atg aag ctc    1776
Asp Gly Arg Gly Pro Lys Ser Ile Glu Asp Asp Ser Glu Met Lys Leu
            580                 585                 590

gtg cgc tac aac caa tcc ggc gcc gac ttc tac ggc gcg gaa ggc gaa    1824
Val Arg Tyr Asn Gln Ser Gly Ala Asp Phe Tyr Gly Ala Glu Gly Glu
        595                 600                 605

atc tac ttc aaa ccg aca ccg cgc tac cgc atc ggc gtt tcc ggc gac    1872
Ile Tyr Phe Lys Pro Thr Pro Arg Tyr Arg Ile Gly Val Ser Gly Asp
    610                 615                 620

tat gta cga ggc cgt ctg aaa aac ctg cct tcc cta ccc ggc aga gaa    1920
Tyr Val Arg Gly Arg Leu Lys Asn Leu Pro Ser Leu Pro Gly Arg Glu
625                 630                 635                 640

gat gcc tac ggc aac cgt cct ttc atc gca cag gac gac caa aat gcc    1968
Asp Ala Tyr Gly Asn Arg Pro Phe Ile Ala Gln Asp Asp Gln Asn Ala
                645                 650                 655

ccc cgt gtt ccg gct gcg cgc ctc ggc ttc cac ctg aaa gcc tcg ctg    2016
Pro Arg Val Pro Ala Ala Arg Leu Gly Phe His Leu Lys Ala Ser Leu
            660                 665                 670

acc gac cgt atc gat gcc aat ttg gac tac tac cgc gtg ttc gcc caa    2064
Thr Asp Arg Ile Asp Ala Asn Leu Asp Tyr Tyr Arg Val Phe Ala Gln
        675                 680                 685

aac aaa ctc gcc cgc tac gaa acg cgc acg ccc gga cac cat atg ctc    2112
Asn Lys Leu Ala Arg Tyr Glu Thr Arg Thr Pro Gly His His Met Leu
    690                 695                 700

aac ctc ggc gca aac tac cgc cgc aat acg cgc tat ggc gag tgg aat    2160
Asn Leu Gly Ala Asn Tyr Arg Arg Asn Thr Arg Tyr Gly Glu Trp Asn
705                 710                 715                 720

tgg tac gtc aaa gcc gac aac ctg ctc aac caa tcc gtt tac gcc cac    2208
Trp Tyr Val Lys Ala Asp Asn Leu Leu Asn Gln Ser Val Tyr Ala His
                725                 730                 735

agc agc ttt ctc tct gat acg ccg caa atg ggc cgc agc ttt acc ggc    2256
Ser Ser Phe Leu Ser Asp Thr Pro Gln Met Gly Arg Ser Phe Thr Gly
            740                 745                 750

ggc gtg aac gtg aag ttt taa                                        2277
Gly Val Asn Val Lys Phe
        755

<210> 202
<211> 758
<212> PRT
<213> Neisseria meningitidis

<400> 202
Met Ala Gln Thr Thr Leu Lys Pro Ile Val Leu Ser Ile Leu Leu Ile
1               5                   10                  15

Asn Thr Pro Leu Leu Ala Gln Ala His Glu Thr Glu Gln Ser Val Asp
            20                  25                  30

Leu Glu Thr Val Ser Val Val Gly Lys Ser Arg Pro Arg Ala Thr Ser
        35                  40                  45

Gly Leu Leu His Thr Ser Thr Ala Ser Asp Lys Ile Ile Ser Gly Asp
    50                  55                  60

Thr Leu Arg Gln Lys Ala Val Asn Leu Gly Asp Ala Leu Asp Gly Val
65                  70                  75                  80

Pro Gly Ile His Ala Ser Gln Tyr Gly Gly Gly Ala Ser Ala Pro Val
                85                  90                  95

Ile Arg Gly Gln Thr Gly Arg Arg Ile Lys Val Leu Asn His His Gly
            100                 105                 110

Glu Thr Gly Asp Met Ala Asp Phe Ser Pro Asp His Ala Ile Met Val
        115                 120                 125

Asp Thr Ala Leu Ser Gln Gln Val Glu Ile Leu Arg Gly Pro Val Thr
    130                 135                 140

Leu Leu Tyr Ser Ser Gly Asn Val Ala Gly Leu Val Asp Val Ala Asp
145                 150                 155                 160

Gly Lys Ile Pro Glu Lys Met Pro Glu Asn Gly Val Ser Gly Glu Leu
                165                 170                 175

Gly Leu Arg Leu Ser Ser Gly Asn Leu Glu Lys Leu Thr Ser Gly Gly
            180                 185                 190

Ile Asn Ile Gly Leu Gly Lys Asn Phe Val Leu His Thr Glu Gly Leu
        195                 200                 205

Tyr Arg Lys Ser Gly Asp Tyr Ala Val Pro Arg Tyr Arg Asn Leu Lys
    210                 215                 220

Arg Leu Pro Asp Ser His Ala Asp Ser Gln Thr Gly Ser Ile Gly Leu
225                 230                 235                 240

Ser Trp Val Gly Glu Lys Gly Phe Ile Gly Val Ala Tyr Ser Asp Arg
                245                 250                 255

Arg Asp Gln Tyr Gly Leu Pro Ala His Ser His Glu Tyr Asp Asp Cys
            260                 265                 270

His Ala Asp Ile Ile Trp Gln Lys Ser Leu Ile Asn Lys Arg Tyr Leu
        275                 280                 285

Gln Leu Tyr Pro His Leu Leu Thr Glu Glu Asp Ile Asp Tyr Asp Asn
    290                 295                 300

Pro Gly Leu Ser Cys Gly Phe His Asp Asp Asp Asn Ala His Ala His
305                 310                 315                 320

Thr His Ser Gly Arg Pro Trp Ile Asp Leu Arg Asn Lys Arg Tyr Glu
                325                 330                 335

Leu Arg Ala Glu Trp Lys Gln Pro Phe Pro Gly Phe Glu Ala Leu Arg
            340                 345                 350

Val His Leu Asn Arg Asn Asp Tyr Arg His Asp Glu Lys Ala Gly Asp
        355                 360                 365

Ala Val Glu Asn Phe Phe Asn Asn Gln Thr Gln Asn Ala Arg Ile Glu
    370                 375                 380

Leu Arg His Gln Pro Ile Gly Arg Leu Lys Gly Ser Trp Gly Val Gln
385                 390                 395                 400

Tyr Leu Gln Gln Lys Ser Ser Ala Leu Ser Ala Ile Ser Glu Ala Val
                405                 410                 415

Lys Gln Pro Met Leu Leu Asp Asn Lys Val Gln His Tyr Ser Phe Phe
            420                 425                 430

Gly Val Glu Gln Ala Asn Trp Asp Asn Phe Thr Leu Glu Gly Gly Val
        435                 440                 445

Arg Val Glu Lys Gln Lys Ala Ser Ile Gln Tyr Asp Lys Ala Leu Ile
    450                 455                 460

Asp Arg Glu Asn Tyr Tyr Asn His Pro Leu Pro Asp Leu Gly Ala His
465                 470                 475                 480

Arg Gln Thr Ala Arg Ser Phe Ala Leu Ser Gly Asn Trp Tyr Phe Thr
                485                 490                 495

Pro Gln His Lys Leu Ser Leu Thr Ala Ser His Gln Glu Arg Leu Pro
            500                 505                 510

Ser Thr Gln Glu Leu Tyr Ala His Gly Lys His Val Ala Thr Asn Thr
        515                 520                 525

Phe Glu Val Gly Asn Lys His Leu Asn Lys Glu Arg Ser Asn Asn Ile
    530                 535                 540

Glu Leu Ala Leu Gly Tyr Glu Gly Asp Arg Trp Gln Tyr Asn Leu Ala
545                 550                 555                 560

Leu Tyr Arg Asn Arg Phe Gly Asn Tyr Ile Tyr Ala Gln Thr Leu Asn
                565                 570                 575

Asp Gly Arg Gly Pro Lys Ser Ile Glu Asp Asp Ser Glu Met Lys Leu
            580                 585                 590

Val Arg Tyr Asn Gln Ser Gly Ala Asp Phe Tyr Gly Ala Glu Gly Glu
        595                 600                 605

Ile Tyr Phe Lys Pro Thr Pro Arg Tyr Arg Ile Gly Val Ser Gly Asp
    610                 615                 620

Tyr Val Arg Gly Arg Leu Lys Asn Leu Pro Ser Leu Pro Gly Arg Glu
625                 630                 635                 640

Asp Ala Tyr Gly Asn Arg Pro Phe Ile Ala Gln Asp Asp Gln Asn Ala
                645                 650                 655

Pro Arg Val Pro Ala Ala Arg Leu Gly Phe His Leu Lys Ala Ser Leu
            660                 665                 670

Thr Asp Arg Ile Asp Ala Asn Leu Asp Tyr Tyr Arg Val Phe Ala Gln
        675                 680                 685

Asn Lys Leu Ala Arg Tyr Glu Thr Arg Thr Pro Gly His His Met Leu
    690                 695                 700

Asn Leu Gly Ala Asn Tyr Arg Arg Asn Thr Arg Tyr Gly Glu Trp Asn
705                 710                 715                 720

Trp Tyr Val Lys Ala Asp Asn Leu Leu Asn Gln Ser Val Tyr Ala His
                725                 730                 735

Ser Ser Phe Leu Ser Asp Thr Pro Gln Met Gly Arg Ser Phe Thr Gly
            740                 745                 750

Gly Val Asn Val Lys Phe
        755

<210> 203
<211> 225
<212> DNA
<213> Neisseria meningitidis

<220>
<221> CDS
<222> (1)..(225)

<400> 203
atg agg ctg gca acc aag gat ttg gcg gaa gcc att agg aaa gga cag     48
Met Arg Leu Ala Thr Lys Asp Leu Ala Glu Ala Ile Arg Lys Gly Gln
1               5                   10                  15

gtt cgc aaa tca agc ttt aac aca gaa caa tta agg gca att gaa aaa     96
Val Arg Lys Ser Ser Phe Asn Thr Glu Gln Leu Arg Ala Ile Glu Lys
            20                  25                  30

gga gaa tct aaa ata ccg gat tac act tgg cat cat cat caa gat aca    144
Gly Glu Ser Lys Ile Pro Asp Tyr Thr Trp His His His Gln Asp Thr
        35                  40                  45

gga agg atg caa ttg att cgt gaa ggc ttg cat cat gat acc ggc cat    192
Gly Arg Met Gln Leu Ile Arg Glu Gly Leu His His Asp Thr Gly His
    50                  55                  60

att ggt tgg gaa gca atg aac aaa gga agg taa                        225
Ile Gly Trp Glu Ala Met Asn Lys Gly Arg
65                  70                  75

<210> 204
<211> 74
<212> PRT
<213> Neisseria meningitidis

<400> 204
Met Arg Leu Ala Thr Lys Asp Leu Ala Glu Ala Ile Arg Lys Gly Gln
1               5                   10                  15

Val Arg Lys Ser Ser Phe Asn Thr Glu Gln Leu Arg Ala Ile Glu Lys
            20                  25                  30

Gly Glu Ser Lys Ile Pro Asp Tyr Thr Trp His His His Gln Asp Thr
        35                  40                  45

Gly Arg Met Gln Leu Ile Arg Glu Gly Leu His His Asp Thr Gly His
    50                  55                  60

Ile Gly Trp Glu Ala Met Asn Lys Gly Arg
65                  70

<210> 205
<211> 1176
<212> DNA
<213> Neisseria meningitidis

<220>
<221> CDS
<222> (1)..(1176)

<400> 205
atg gca aaa atg atg aaa tgg gcg gct gtt gcg gcg gtc gcg gcg gca    48
Met Ala Lys Met Met Lys Trp Ala Ala Val Ala Ala Val Ala Ala Ala

1                 5                   10                  15

gcg gtt tgg ggc gga tgg tct tat ctg aag ccc gag ccg cag gct gct    96
Ala Val Trp Gly Gly Trp Ser Tyr Leu Lys Pro Glu Pro Gln Ala Ala
                20                  25                  30

tat att acg gaa acg gtc agg cgc ggc gac atc agc cgg acg gtt tct    144
Tyr Ile Thr Glu Thr Val Arg Arg Gly Asp Ile Ser Arg Thr Val Ser
            35                  40                  45

gca aca ggg gag att tcg ccg tcc aac ctg gta tcg gtc ggc gcg cag    192
Ala Thr Gly Glu Ile Ser Pro Ser Asn Leu Val Ser Val Gly Ala Gln
    50                  55                  60

gca tcg ggg cag att aag aaa ctt tat gtc aaa ctc ggg caa cag gtt    240
Ala Ser Gly Gln Ile Lys Lys Leu Tyr Val Lys Leu Gly Gln Gln Val
65                  70                  75                  80

aaa aag ggc gat ttg att gcg gaa atc aat tcg acc tcg cag acc aat    288
Lys Lys Gly Asp Leu Ile Ala Glu Ile Asn Ser Thr Ser Gln Thr Asn
                85                  90                  95

acg ctc aat acg gaa aaa tcc aaa ttg gaa acg tat cag gcg aag ctg    336
Thr Leu Asn Thr Glu Lys Ser Lys Leu Glu Thr Tyr Gln Ala Lys Leu
            100                 105                 110

gtg tcg gca cag att gca ttg ggc agc gcg gag aag aaa tat aag cgt    384
Val Ser Ala Gln Ile Ala Leu Gly Ser Ala Glu Lys Lys Tyr Lys Arg
        115                 120                 125

cag gcg gcg ttg tgg aag gat gat gcg acc gct aaa gaa gat ttg gaa    432
Gln Ala Ala Leu Trp Lys Asp Asp Ala Thr Ala Lys Glu Asp Leu Glu
    130                 135                 140

agc gca cag gat gcg ctt gcc gcc gcc aaa gcc aat gtt gcc gag ctg    480
Ser Ala Gln Asp Ala Leu Ala Ala Ala Lys Ala Asn Val Ala Glu Leu
145                 150                 155                 160

aag gct cta atc aga cag agc aaa att tcc atc aat acc gcc gag tcg    528
Lys Ala Leu Ile Arg Gln Ser Lys Ile Ser Ile Asn Thr Ala Glu Ser
                165                 170                 175

gaa ttg ggc tac acg cgc att acc gca acg atg gac ggc acg gtg gtg    576
Glu Leu Gly Tyr Thr Arg Ile Thr Ala Thr Met Asp Gly Thr Val Val
            180                 185                 190

gcg att ctc gtg gaa gag ggg cag act gtg aac gcg gcg cag tct acg    624
Ala Ile Leu Val Glu Glu Gly Gln Thr Val Asn Ala Ala Gln Ser Thr

195                 200                 205

ccg acg att gtc caa ttg gcg aat ctg gat atg atg ttg aac aaa atg     672
Pro Thr Ile Val Gln Leu Ala Asn Leu Asp Met Met Leu Asn Lys Met
    210                 215                 220

cag att gcc gag ggc gat att acc aag gtg aag gcg ggg cag gat att     720
Gln Ile Ala Glu Gly Asp Ile Thr Lys Val Lys Ala Gly Gln Asp Ile
225                 230                 235                 240

tcg ttt acg att ttg tcc gaa ccg gat acg ccg att aag gcg aag ctc     768
Ser Phe Thr Ile Leu Ser Glu Pro Asp Thr Pro Ile Lys Ala Lys Leu
                245                 250                 255

gac agc gtc gac ccc ggg ctg acc acg atg tcg tcg ggc ggc tac aac     816
Asp Ser Val Asp Pro Gly Leu Thr Thr Met Ser Ser Gly Gly Tyr Asn
            260                 265                 270

agc agt acg gat acg gct tcc aat gcg gtc tac tat tat gcc cgt tcg     864
Ser Ser Thr Asp Thr Ala Ser Asn Ala Val Tyr Tyr Tyr Ala Arg Ser
        275                 280                 285

ttt gtg ccg aat ccg gac ggc aaa ctc gcc acg ggg atg acg acg cag     912
Phe Val Pro Asn Pro Asp Gly Lys Leu Ala Thr Gly Met Thr Thr Gln
    290                 295                 300

aat acg gtt gaa atc gac ggt gtg aaa aat gtg ctg att att ccg tcg     960
Asn Thr Val Glu Ile Asp Gly Val Lys Asn Val Leu Ile Ile Pro Ser
305                 310                 315                 320

ctg acc gtg aaa aat cgc ggc ggc agg gcg ttt gtg cgc gtg ttg ggt     1008
Leu Thr Val Lys Asn Arg Gly Gly Arg Ala Phe Val Arg Val Leu Gly
                325                 330                 335

gca gac ggc aag gcg gcg gaa cgc gaa atc cgg acc ggt atg aga gac     1056
Ala Asp Gly Lys Ala Ala Glu Arg Glu Ile Arg Thr Gly Met Arg Asp
            340                 345                 350

agt atg aat acc gaa gta aaa agc ggg ttg aaa gag ggg gac aaa gtg     1104
Ser Met Asn Thr Glu Val Lys Ser Gly Leu Lys Glu Gly Asp Lys Val
        355                 360                 365

gtc atc tcc gaa ata acc gcc gcc gag cag cag gaa agc ggc gaa cgc     1152
Val Ile Ser Glu Ile Thr Ala Ala Glu Gln Gln Glu Ser Gly Glu Arg
    370                 375                 380

gcc cta ggc ggc ccg ccg cgc cga                                     1176
Ala Leu Gly Gly Pro Pro Arg Arg

385                 390

<210> 206
<211> 392
<212> PRT
<213> Neisseria meningitidis

<400> 206
Met Ala Lys Met Met Lys Trp Ala Ala Val Ala Ala Val Ala Ala Ala
1               5                   10                  15

Ala Val Trp Gly Gly Trp Ser Tyr Leu Lys Pro Glu Pro Gln Ala Ala
            20                  25                  30

Tyr Ile Thr Glu Thr Val Arg Arg Gly Asp Ile Ser Arg Thr Val Ser
        35                  40                  45

Ala Thr Gly Glu Ile Ser Pro Ser Asn Leu Val Ser Val Gly Ala Gln
    50                  55                  60

Ala Ser Gly Gln Ile Lys Lys Leu Tyr Val Lys Leu Gly Gln Gln Val
65                  70                  75                  80

Lys Lys Gly Asp Leu Ile Ala Glu Ile Asn Ser Thr Ser Gln Thr Asn
                85                  90                  95

Thr Leu Asn Thr Glu Lys Ser Lys Leu Glu Thr Tyr Gln Ala Lys Leu
            100                 105                 110

Val Ser Ala Gln Ile Ala Leu Gly Ser Ala Glu Lys Lys Tyr Lys Arg
        115                 120                 125

Gln Ala Ala Leu Trp Lys Asp Asp Ala Thr Ala Lys Glu Asp Leu Glu
    130                 135                 140

Ser Ala Gln Asp Ala Leu Ala Ala Ala Lys Ala Asn Val Ala Glu Leu
145                 150                 155                 160

Lys Ala Leu Ile Arg Gln Ser Lys Ile Ser Ile Asn Thr Ala Glu Ser
                165                 170                 175

Glu Leu Gly Tyr Thr Arg Ile Thr Ala Thr Met Asp Gly Thr Val Val
            180                 185                 190

Ala Ile Leu Val Glu Glu Gly Gln Thr Val Asn Ala Ala Gln Ser Thr
        195                 200                 205

Pro Thr Ile Val Gln Leu Ala Asn Leu Asp Met Met Leu Asn Lys Met
    210                 215                 220

Gln Ile Ala Glu Gly Asp Ile Thr Lys Val Lys Ala Gly Gln Asp Ile
225                 230                 235                 240

Ser Phe Thr Ile Leu Ser Glu Pro Asp Thr Pro Ile Lys Ala Lys Leu
                245                 250                 255

Asp Ser Val Asp Pro Gly Leu Thr Thr Met Ser Ser Gly Gly Tyr Asn
            260                 265                 270

Ser Ser Thr Asp Thr Ala Ser Asn Ala Val Tyr Tyr Tyr Ala Arg Ser
        275                 280                 285

Phe Val Pro Asn Pro Asp Gly Lys Leu Ala Thr Gly Met Thr Thr Gln
    290                 295                 300

Asn Thr Val Glu Ile Asp Gly Val Lys Asn Val Leu Ile Ile Pro Ser
305                 310                 315                 320

Leu Thr Val Lys Asn Arg Gly Gly Arg Ala Phe Val Arg Val Leu Gly
                325                 330                 335

Ala Asp Gly Lys Ala Ala Glu Arg Glu Ile Arg Thr Gly Met Arg Asp
            340                 345                 350

Ser Met Asn Thr Glu Val Lys Ser Gly Leu Lys Glu Gly Asp Lys Val
        355                 360                 365

Val Ile Ser Glu Ile Thr Ala Ala Glu Gln Gln Glu Ser Gly Glu Arg
    370                 375                 380

Ala Leu Gly Gly Pro Pro Arg Arg
385                 390

<210> 207
<211> 342
<212> DNA
<213> Neisseria meningitidis

<220>
<221> CDS
<222> (1)..(342)

<400> 207

ttg ttt gta tgc ttt gaa aaa tgt ttg ttt ccg gat ttt gcc att ccc    48
Leu Phe Val Cys Phe Glu Lys Cys Leu Phe Pro Asp Phe Ala Ile Pro
1               5                   10                  15

atc cgg ttt tgc gct gta cga tgt gtt tta gcg cgg act tgc tca aaa    96
Ile Arg Phe Cys Ala Val Arg Cys Val Leu Ala Arg Thr Cys Ser Lys
            20                  25                  30

tcg gat gtg gtt ccg gta ttt ggg gct ttg att agg gat gcg gac ttt    144
Ser Asp Val Val Pro Val Phe Gly Ala Leu Ile Arg Asp Ala Asp Phe
        35                  40                  45

caa tat att ttt ctc agc tac aac aac gaa ggc ttg atg tct gtc ggg    192
Gln Tyr Ile Phe Leu Ser Tyr Asn Asn Glu Gly Leu Met Ser Val Gly
    50                  55                  60

cag gta agg gag att ttt gag cgt ttc ggc aaa tat aat ttg gtt caa    240
Gln Val Arg Glu Ile Phe Glu Arg Phe Gly Lys Tyr Asn Leu Val Gln
65                  70                  75                  80

acg gaa tat cgg cgt ttt aag gca gat aag aca gaa aac cgt aat cat    288
Thr Glu Tyr Arg Arg Phe Lys Ala Asp Lys Thr Glu Asn Arg Asn His
                85                  90                  95

aag gca aat tcg ata ttc gaa tat ctg cat gag acc ttt gca aaa ata    336
Lys Ala Asn Ser Ile Phe Glu Tyr Leu His Glu Thr Phe Ala Lys Ile
            100                 105                 110

gtc tgt                                                            342
Val Cys

<210> 208
<211> 114
<212> PRT
<213> Neisseria meningitidis

<400> 208
Leu Phe Val Cys Phe Glu Lys Cys Leu Phe Pro Asp Phe Ala Ile Pro
1               5                   10                  15

Ile Arg Phe Cys Ala Val Arg Cys Val Leu Ala Arg Thr Cys Ser Lys
            20                  25                  30

Ser Asp Val Val Pro Val Phe Gly Ala Leu Ile Arg Asp Ala Asp Phe
        35                  40                  45

Gln Tyr Ile Phe Leu Ser Tyr Asn Asn Glu Gly Leu Met Ser Val Gly

50                  55                  60

Gln Val Arg Glu Ile Phe Glu Arg Phe Gly Lys Tyr Asn Leu Val Gln
65                  70                  75                  80

Thr Glu Tyr Arg Arg Phe Lys Ala Asp Lys Thr Glu Asn Arg Asn His
                85                  90                  95

Lys Ala Asn Ser Ile Phe Glu Tyr Leu His Glu Thr Phe Ala Lys Ile
            100                 105                 110

Val Cys

<210> 209
<211> 24
<212> DNA
<213> Artificial Sequence

<220>
<223> Description of Artificial Sequence:Primer Sequence

<400> 209
ttggttaatt ggttgtaaca ctgg                                        24

<210> 210
<211> 19
<212> DNA
<213> Artificial Sequence

<220>
<223> Description of Artificial Sequence:Primer Sequence

<400> 210
attctcatgt ttgacagcg                                              19

<210> 211
<211> 18
<212> DNA
<213> Artificial Sequence

<220>
<223> Description of Artificial Sequence:Primer Sequence

<400> 211
atcctacaac ctcaagct                                                    18

<210> 212
<211> 18
<212> DNA
<213> Artificial Sequence

<220>
<223> Description of Artificial Sequence:Primer Sequence

<400> 212
atcccattct aaccaagc                                                    18

<210> 213
<211> 19
<212> DNA
<213> Artificial Sequence

<220>
<223> Description of Artificial Sequence:Primer Sequence

<400> 213
aagagattac gcgcagacc                                                   19

<210> 214
<211> 18
<212> DNA
<213> Artificial Sequence

<220>
<223> Description of Artificial Sequence:Primer Sequence

<400> 214
aatacgcaac cgcctctc                                                    18

## Description

### Field of the Invention

This invention relates to virulence genes and proteins, and their use. More particularly, it relates to genes and proteins/peptides obtained from *Neisseria meningitidis,* and their use in therapy and in screening for drugs.

### Background of the Invention

*Neisseria meningitidis* is a Gram-negative bacterial pathogen that is implicated in septic shock and bacterial meningitis. This bacterium is a leading cause of bacterial meningitis in developed countries, and causes large-scale epidemics in Africa and China. In the UK, *Neisseria meningitidis* is the leading cause of death in childhood apart from road traffic accidents. The bacterium naturally inhabits the human naso-pharynx and then gains access to the blood stream from where it causes severe septicaemia or meningitis. Although current anti-microbials are effective in eliminating the bacterium from the body, the mortalilty from menigococcal septicaemia remains substantial. It would be desirable to provide means for treating or preventing conditions caused by *Neisseria meningitidis,* e.g. by immunisation.

### Summary of the Invention

The present invention is based on the discovery of virulence genes in *Neisseria meningitidis.*

According to a first aspect of the invention, a peptide of the invention is encoded by an operon including any of the nucleotide sequences identified in claim 1, or a homologue thereof in a Gram-negative bacterium, or a functional fragment thereof, for therapeutic or diagnostic use.

The peptides may have many therapeutic uses for treating *Neisseria* infections, including use in vaccines for prophylactic application.

According to a second aspect, a polynucleotide encoding a peptide defined above, may also be useful for therapy or diagnosis.

According to a third aspect, the genes that encode the peptides may be utilised to prepare attenuated microorganisms. The attenuated microorganisms will usually have a mutation that disrupts the expression of one or more of the genes identified herein, to provide a strain that lacks virulence. These microorganisms will also have use in therapy and diagnosis.

According to a fourth aspect, the peptides, genes and attenuated microorganisms according to the invention may be used in the treatment or prevention of a condition associated with infection by *Neisseria* or Gram-negative bacteria.

### Description of the Invention

The present invention is based on the discovery of genes encoding peptides which are implicated in virulence. The peptides and genes of the invention are therefore useful for the preparation of therapeutic agents to treat infection. It should be understood that references to therapy also include preventative treatments, e.g. vaccination. Furthermore, while the products of the invention are intended primarily for treatment of infections in human patients, veterinary applications are also considered to be within the scope of the invention.

The present invention is described with reference to *Neisseria meningitidis.* However, all the *Neisseria* strains, and many other Gram-negative bacterial strains are likely to include related peptides or proteins having amino acid sequence identity or similarity to those identified herein. Organisms likely to contain the peptides include, but are not limited to the genera *Salmonella, Enterobacter, Klebsiella, Shigella* and *Yersinia.*

The experiments carried out to identify the virulence genes of the invention utilised *N. meningitidis* strain B. Homology searches were performed on the strain A database, however the proteins and genes from strain B are preferred.

Preferably, the peptides that may be useful in the various aspects of the invention have greater than a 40% similarity with the peptides identified herein. More preferably, the peptides have greater than 60% sequence similarity. Most preferably, the peptides have greater than 80% sequence similarity, e.g. 95% similarity. With regard to the polynucleotide sequences identified herein, related polynucleotides that may be useful in the various aspects of the invention may have greater than 40% identity with the sequences identified herein. More preferably, the polynucleotide sequences have greater than 60% sequence identity. Most preferably, the polynucleotide sequences have greater than 80% sequence identity, e.g. 95% identity.

The terms "similarity" and "identity" are known in the art. The use of the term "identity" refers to a sequence comparison based on identical matches between correspondingly identical positions in the sequences being compared. The term "similarity" refers to a comparison between amino acid sequences, and takes into account not only identical amino acids in corresponding positions, but also functionally similar amino acids in corresponding positions. Thus similarity between polypeptide sequences indicates functional similarity, in addition to sequence similarity.

Levels of identity between gene sequences and levels of identity or similarity between amino acid sequences can be calculated using known methods. In relation to the present invention, publicly available computer based methods for determining identity and similarity include the BLASTP, BLASTN and FASTA (Atschul et al., J. Molec. Biol., 1990; 215:403-410), the BLASTX program available from NCBI, and the Gap program from Genetics Computer Group, Madison WI. The levels of similarity and identity provided herein, were obtained using the Gap program, with a Gap penalty of 12 and a Gap length penalty of 4 for determining the amino acid sequence comparisons, and a Gap penalty of 50 and a Gap length penalty of 3 for the polynucleotide sequence comparisons.

Having characterised a gene according to the invention, it is possible to use the gene sequence to search for related genes or peptides in other microorganisms. This may be carried out by searching in existing databases, e.g. EMBL or GenBank.

Peptides or proteins according to the invention may be purified and isolated by methods known in the art. In particular, having identified the gene sequence, it will be possible to use recombinant techniques to express the genes in a suitable host. Active fragments and related molecules can be identified and may be useful in therapy. For example, the peptides or their active fragments may be used as antigenic determinants in a vaccine, to elicit an immune response. They may also be used in the preparation of antibodies, for passive immunisation, or diagnostic applications. Suitable antibodies include monoclonal antibodies, or fragments thereof, including single chain Fv fragments. Methods for the preparation of antibodies will be apparent to those skilled in the art.

Active fragments of the peptides are those that retain the biological function of the peptide. For example, when used to elicit an immune response, the fragment will be of sufficient size, such that antibodies generated from the fragment will discriminate between that peptide and other peptides on the bacterial microorganism. Typically, the fragment will be at least 30 nucleotides (10 amino acids) in size, preferably 60 nucleotides (20 amino acids) and most preferably greater than 90 nucleotides (30 amino acids) in size.

It should also be understood, that in addition to related molecules from other microorganisms, the invention encompasses modifications made to the peptides and polynucleotides identified herein which do not significantly alter the biological function. It will be apparent to the skilled person that the degeneracy of the genetic code can result in polynucleotides with minor base changes from those specified herein, but which nevertheless encode the same peptides. Complementary polynucleotides are also within the invention. Conservative replacements at the amino acid level are also envisaged, i.e. different acidic or basic amino acids may be substituted without substantial loss of function.

The preparation of vaccines based on attenuated microorganisms is known to those skilled in the art. Vaccine compositions can be formulated with suitable carriers or adjuvants, e.g. alum, as necessary or desired, to provide effective immunisation against infection. The preparation of vaccine formulations will be apparent to the skilled person. The attenuated microorganisms may be prepared with a mutation that disrupts the expression of any of the genes identified herein. The skilled person will be aware of methods for disrupting expression of particular genes. Techniques that may be used include insertional inactivation or gene deletion techniques. Attenuated microorganisms according to the invention may also comprise additional mutations in other genes, for example in a second gene identified herein or in a separate gene required for growth of the microorganism, e.g. an *aro* mutation or, with regard to *Salmonella,* in a gene located in the SPI2 region identified in WO-A-96/17951.

Attenuated microorganisms may also be used as carrier systems for the delivery of heterologous antigens, therapeutic proteins or nucleic acids (DNA or RNA). In this embodiment, the attenuated microorganisms are used to deliver a heterologous antigen, protein or nucleic acid to a particular site *in vivo.* Introduction of a heterologous antigen, peptide or nucleic acid into an attenuated microorganism can be carried out by conventional techniques, including the use of recombinant constructs, e.g. vectors, which comprise polynucleotides that express the heterologous antigen or therapeutic protein, and also include suitable promoter sequences. Alternatively, the gene that encodes the heterologous antigen or protein may be incorporated into the genome of the organism and the endogenous promoters used to control expression.

More generally, and as is well known to those skilled in the art, a suitable amount of an active component of the invention can be selected, for therapeutic use, as can suitable carriers or excipients, and routes of administration. These factors would be chosen or determined according to known criteria such as the nature/severity of the condition to be treated, the type and/or health of the subject etc.

In a separate embodiment, the products of the invention may be used in screening assays for the identification of potential antimicrobial drugs or for the detection for virulence. Routine screening assays are known to those skilled in the art, and can be adapted using the products of the invention in the appropriate way. For example, the products of the invention may be used as the target for a potential drug, with the ability of the drug to inactivate or bind to the target indicating its potential antimicrobial activity.

The various products of the invention may also be used in veterinary applications.

The following is a brief overview of the experimental procedure used to identify the virulence genes.

Signature-tagged mutagenesis (STM) (Hensel et al., Science, 1995; 269: 400-403) was used to identify genes in *N. meningitidis* that are essential for septicemic infection. In STM, individual mutants are tagged with unique sequence identifiers, allowing large numbers of mutants to be analyzed simultaneously. However, it is necessary to construct libraries of insertional mutants, so far a limitation in studying N. *meningitidis.* Mutagenesis was accomplished successfully using a method in which *Neisseria* DNA is modified *in vitro* using purified components of Tn *10* transposition. As *N. meningitidis* efficiently takes up exogenous DNA, the modified alleles are then introduced into *N. meningitidis* by transformation. The mutants can then be screened for their ability to cause systemic infection.

The vector pSTM115 (Sun et al. Nature Medicine, 2000; 6(11):1269-1273) was used as the transposon donor for *in vitro* mutagenesis. 96 pSTM115 derivatives, each containing unique signature tags, were included in 96 separate transposition reactions. The modified genomic DNA was repaired, and returned to the host by transformation. To determine whether Tn*10* insertion occurs at diverse sites, 40 transformants were assessed from a single transposition reaction by Southern blot analysis. Each had a single, distinct Tn 10 insertion. To establish whether Tn 10 integration was stable during systemic infection of infant rats, the hydridization patterns of six mutants before and after passage through rats were compared. Identical hybridization patterns before and after infection were obtained.

The experimental conditions used were as follows:

### Bacterial strains and growth:

C311+ is an ET-5, serogroup B *N. meningitidis* isolate from a patient with invasive meningococcal infection (Virji et al., Mol. Microbiol., 1991; 5: 1831-1841). *N. meningitidis* was grown on brain-heart infusion medium with 5% Levinthal's supplement. *E. coli* strains were propagated on Luria Bertani media. Kanamycin was added to solid media as required at concentrations of 75 and 50 µg/ml for *N. meningitidis* and *E. coli,* respectively.

### In vitro transposition and insertion site characterization:

The pACYC184 origin of replication in pSTM115 was used to isolate the insertion site by marker rescue. Nucleotide sequencing was carried out using the dye-termination method (Perkin Elmer, Norwalk, Connecticut) with primers NG62 (5'-TTGGTTAATTGGTTGTAACACTGG-3') (SEQ ID NO. 209) or NG99 (5'-ATTCTCATGTTTGACAGCG-3') (SEQ ID NO. 210). Homology searches were performed against protein databases (http://www.ncbi.nim.nih.gov/), including the serogroup A and B *N. meningitidis* and the *N. gonorrhoeae* genome sequences (http://www.sanger.ac.uk/Projects/N_meningitidis and http://www.tigr.org, and http://dna1.chem.ou.edu/gono.html, respectively).

### Tag amplification, cloning and Southern blot analyses:

Hybridizations and preparations of dot blots were performed as described in Hensel *et a*/*., supra,* except that tags were amplified with primers NG13 (5'-ATCCTACAACCTCAAGCT-3') (SEQ ID NO. 211) and NG14 (5'-ATCCCATTCTAACCAAGC-3') (SEQ ID NO. 212), and PCR products, rather than plasmid DNA, were fixed onto membranes. Oligonucleotides S1 (5'-AAGAGATTACGCGCAGACC-3') (SEQ ID NO. 213) and S2 (5'-AATACGCAACCGCCTCTC-3') (SEQ ID NO. 214) anneal to sequences in pSTM115 flanking the 'signature tags' and were used to amplify a 367-base-pair product from each pSTM115 derivative. For Southern blot analysis, the kanamycin-resistance cassette from pSTM115 was labelled using the random primers method (NEB), and was used as a probe against genomic DNA digested with *Cl*al.

### Animal model:

For screening the STM pools, mutants were grown individually for 18 h in microtiter plates. The bacteria were pooled, then re-suspended in PBS. Wistar rats (5 days old) were inoculated intraperitoneally with 100 µl of the suspension, and were monitored for 48 h. To establish the competitive index of a mutant, wild-type and mutant bacteria were grown for 18 h on solid media and collected into PBS, and rats were inoculated with a 1:1 ratio of mutant to wild-type cells in a total inoculum of 5 x 10⁶ CFU. The proportion of mutant (kanamycin-resistant) to wild-type (kanamycin-sensitive) bacteria was determined by plating replicate samples to media with or without added antibiotic.

The results of the homology searches are shown in Table 1.

**Table 1**

| SEQ ID NO. | PROTEIN | ACCESSION NO. | ORGANISM |
|---|---|---|---|
| 3 & 4 | 3'dehydroquinate synthase | NMB0647 | N. gonorrhoeae |
| 25 & 26 | Glycosyl transferase | LS12 | N. gonorrheae |
| 35 & 36 | Polyribonucleotide nucleotidyl transferase | NMB0758 | N. meningitidis |
| 37 & 38 | Phosphoribosyl formyl glycinamide synthase | PURL | E. coli |
| 43 & 44 | Shikimate dehydrogenase | AROE | N. meningitidis |
| 47 & 48 | Hypothetical 21.7 kD protein | NMB0673 | N. meningitidis |
| 53 & 54 | Putative cell binding factor | NMB0345 | N. meningitidis |
| 57 & 58 | Hypothetical protein | HI0633 | H. influenzae |
| 61 & 62 | Na+/H+ antiporter | NMBN0536 | N. memingitidis |
| 63 & 64 | Chorismate synthase | AROC | V. anguillarum |
| 67 & 68 | Paraquat-inducible protein B | PQ15B | E. coli |
| 71 & 72 | 5'-methyltetrahydropteroylyl triglutamate-homocysteine methyl transferase | NMB0944 | N. meningitidis |
| 79 & 80 | α-1,2 N-acetylglucosamine transferase | RFAK | N. meningitidis |
| 83 & 84 | Putative RNA methylase | NMB1348 | N. meningitidis |
| 89 & 90 | L-lactate permease | NMB0543 | N. meningitidis |
| 91 & 92 | ABC transporter | NMB1240 | N. meningitidis |
| 103 & 104 | Probable GTP-binding protein | HI0393 | H. influenzae |
| 109 & 110 | Capsule polysaccharide modification protein | LIPB | N. meningitidis |
| 113 & 114 | Hypothetical 17.8 kD protein | NMB0734 | N. meningitidis |
| 115 & 116 | *E. coli* hypothetical protein | YIGC | S. typhimurium |
| 119 & 120 | Ribonuclease III | NMB0686 | N. meningitidis |
| 121 & 122 | AMPD protein | NMB0668 | N. meningitidis |
| 123 & 124 | 5-methyltetrahydropteroylyl triglutamate-homocysteine methyl transferase | NMB0944 | N. meningitidis |
| 133 & 134 | Putative ATP-depenent RNA helicase | NMB1422 | N. meningitidis |
| 137 & 138 | Putative RNA methylase | NMB1348 | N. meningitidis |
| 141 & 142 | Shikimate dehydrogenase | AROE | N. meningitidis |
| 143 & 144 | Putative outer membrane protein | OMPU | N. meningitidis |
| 145 & 146 | TONB protein | TONB | N. meningitidis |
| 147 & 148 | Putative apolipoprotein N-acyl transferase | NMB0713 | N. meningitidis |
| 149 & 150 | Transposase | NMB0991 | N. meningitidis |
| 153 & 154 | UTP-glucose-1-phosphate uridylyltransferase | NMB0638 | N. meningitidis |
| 157 & 158 | ADP Heptose-LPS heptosyl transferase II | NMB1527 | N. meningitidis |
| 161 & 162 | Putative membrane-bound lytic murein transglycosylase B | NMB1279 | N. meningitidis |
| 171 & 172 | Putative cell-binding factor | NMB0345 | N. meningitidis |
| 173 & 174 | P-amino benzoate synthetase | PABB | H. pylori J99 |
| 177 & 178 | 5'-methyltetrahydropteroylyl triglutamate-homocysteine methyl transferase | NMB0944 | N. meningitidis |
| 183 & 184 | Conserved hypothetical protein | NMB0183 | N. meningitidis |
| 185 & 186 | *E. coli* hypothetical protein | YIGC | S. typhi LT2 |

For the remaining sequences identified herein, no homology results were obtained.

The gene products were used to produce polyclonal antibodies that were tested in an Elisa assay against various *N. meningitidis* strains to evaluate their effectiveness as a vaccine candidate. The strains used were:
*Neisseria meningitidis* (B) Type 1000
*Neisseria meningitidis* (B) Type NGE31
*Neisseria meningitidis* (B) Type NGH15
*Neisseria meningitidis* (B) Type SW2 107
*Neisseria meningitidis* (B) Type NHG38
*Neisseria meningitidis* (B) Type NGE28
*Neisseria meningitidis* (B) Type 2996

This provides information as to the variety of *N. meningitidis* strains that are recognised by these antibodies.

*N. Meningitidis* was grown on Columbia agar with chocolated horse blood (Oxoid) for 14 hours at 37°C in 5% CO₂. The cells were scraped from agar plates and resuspended in 20ml PBS in a 50ml tube. The cell suspension was heated for 30 minutes at 56°C to kill the bacteria.

A 50 µl sample of the heat-killed *N. Meningitidis* was spread on the Columbia agar with the chocolated horse blood and incubated for 18 hours at 37C, 5%CO₂. This allows confirmation that all *N. Meningitidis* cells have been killed. The OD₆₂₀ of the suspension is adjusted to 0.1 OD units versus PBS. Elisa with heat killed *N. meningitidis*

Elisa assays were carried out using the heat-killed *N. meningitidis* using the following protocol. Elisa plates were coated overnight with heat-killed cells (50µl of killed bacteria in PBS to each well of 96 well plate and incubated 4°C).

Standard Elisa protocols were followed, with all incubations at 37°C for 1 hour. PBS/3% BSA blocking solution, PBS/Tween 0.1% wash solution, anti-rabbit AP conjugate secondary antibody (Sigma) and Sigma Fast P Nitrophenyl phosphate detection reagent (Sigma) were utilised. The data was read at 405nm using an appropriate micro-titre plate reader. The sera used was that available seven days after the first booster vaccination (day 35 after first vaccination).

The antibodies tested were those raised against the gene products identified as SEQ ID NOS. 8, 102, 140, 158 and 202. In each case, the results showed that the antisera recognised several different strains of *N. meningitidis* B.

### Ex vivo/in vitro screening.

Protection against meningococcal disease in humans has been associated with the presence of bacteriocidal antibodies against *N. meningitidis* (Goldscheider et al. J. Exp. Med., 1969; 129: 1307-1326). There is also evidence to suggest a correlation between the presence of detectable bacteriocidal activity and protection in an *in vivo* model (reference Martin, J. Bacteriol., 2000; 83: 27-31). Therefore, the antiserum generated was used to evaluate the bacteriocidal activity of the antibodies generated.

The bactericidal assays were performed with pre-immune sera and the corresponding rabbit antiserum raised against the candidate antigens. Commercially available rabbit serum was used as the complement source following pre-screening to eliminate complement only killing. Dulbecco's PBS (Gibco) was used as a buffer where necessary. The *N. meningitidis* strain MC58, was grown at 37°C (5% CO₂) for 14 hours prior to use in the assay.

200-400 CFU of MC58 in a 50µl volume were incubated in the presence of complement (50µl) with 100µl of serial dilutions of heat-inactivated serum. Samples at time zero were plated to Columbia agar with chocolated horse blood (Oxoid). After incubation for 60 minutes, the number of surviving bacteria was evaluated by plating to Columbia agar with chocolated horse blood (Oxoid). The bacteriocidal activity was expressed in terms of percentage of bacteria surviving after 60 minutes. All samples were tested in duplicate and plated in triplicate. All appropriate positive and negative controls were utilised. In each test sample, the bacteriocidal activity was substantially greater than that for the pre-immune sera.

### In vivo screening.

To evaluate the protective efficacy of vaccine candidates, adult mice were immunised with the recombinant proteins identified herein as SEQ ID NOS. 102 and 108 and the protective response determined by live bacterial challenge. For each vaccine candidate 15 six week old mice (6 week old balb/C mice) were vaccinated (subcutaneously) with 25µg of antigen on two separate occasions at three week intervals.

One week after the end of the immunisation schedule, the group was challenged with the homologous bacterial strain MC58. The bacteria were inoculated intraperiponeally in a volume of 500µl in Brain Heart Infusion/ 0.5% iron dextran media at a dose of 10⁷ cfu. Previous results have shown that iron is required for initiation of bacteraemic disease in these animals. This model has previously been used to demonstrate the protective efficacy of vaccination (Lissolo et al., Infect. Immujn., 1995; 63: 884-890).

Control groups included animals vaccinated with adjuvant alone (negative control) or with adjuvant combined with purified PorA (positive control). PorA is an outer membrane protein expressed exclusively by *N. meningitidis* and is the principal target for bactericidal antibodies induced by outer membrane vesicle vaccines. Monoclonal antibodies against PorA have also been shown to passively protect animals in the infant rat model. PorA however varies considerably between strains and so while it elicits some protection when challenged with a homologous strain, it is not an ideal vaccine candidate. Survival was monitored following challenge. The negative control showed no survival after 48 hours. Those vaccinated with PorA showed 6 survivors at 72 hours. Those vaccinated with the proteins of SEQ ID NOS. 102 and 108 showed 5 and 3 survivors, respectively.

## Claims

1. A peptide encoded by any of the nucleotide sequences identified herein as SEQ ID NOS. 87, 1, 3, 5, 7, 9, 11, 13, 15, 17, 19, 21, 23, 25, 27, 29, 31, 33, 37, 39, 41, 43, 45, 47, 49, 51, 53, 55, 57, 59, 61, 63, 65, 67, 69, 71, 73, 75, 77, 79, 81, 83, 85, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, of *N. meningitidis,* or a related molecule having at least 40% sequence similarity or identity at the peptide or nucleotide level in a Gram-negative bacterium, or a functional fragment thereof, for therapeutic or diagnostic use.

2. A peptide according to claim 1, wherein the sequence similarity or identity is at least 60%.

3. A peptide according to claim 1 or claim 2, wherein the sequence similarity or identity is at least 90%.

4. A peptide according to claim 1, comprising the amino acid sequence identified herein as SEQ ID NOS. 88, 2, 4, 6, 8, 10, 12, 14, 16, 18, 20, 22, 24, 26, 28, 30, 32, 34, 38, 40, 42, 44, 46, 48, 50, 52, 54, 56, 58, 60, 62, 64, 66, 68, 70, 72, 74, 76, 78, 80, 82, 84, 86, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206 and 208.

5. A polynucleotide encoding a peptide according to any preceding claim, for therapeutic or diagnostic use.

6. A host transformed to express a peptide according to any of claims 1 to 4.

7. An attenuated microorganism comprising a mutation that disrupts the expression of any of the nucleotide sequences defined in claim 1.

8. A microorganism according to claim 7, wherein the mutation is insertional inactivation or a gene deletion.

9. A microorganism according to claim 7 or claim 8, wherein the microorganism is *Neisseria meningitidis.*

10. A microorganism according to any of claims 7 to 9, comprising a second mutation in a second nucleotide sequence.

11. A microorganism according to any of claims 7 to 10, for therapeutic or diagnostic use.

12. A microorganism according to any of claims 7 to 11, comprising a heterologous antigen, therapeutic peptide or nucleic acid.

13. A vaccine comprising a peptide according to any of claims 1 to 4, or the means for its expression.

14. A vaccine comprising a microorganism according to any of claims 7 to 12.

15. An antibody raised against a peptide according to any of claims 1 to 4.

16. Use of a product according to any of claims 1 to 12, for the manufacture of a medicament for use in the treatment or prevention of a condition associated with infection by *Neisseria* or Gram-negative bacteria.

17. Use according to claim 16, wherein the condition is meningitis.

18. Use according to claim 16 or claim 17, for veterinary treatment.

19. Use of a product according to any of claims 1 to 12, in a screening assay for the identification of an antimicrobial drug.
